(19) Europäisches Patentamt
European Patent Office
Office européen des brevets

(11) EP 2 145 888 A1

(12) EUROPEAN PATENT APPLICATION

(43) Date of publication:
**20.01.2010 Bulletin 2010/03**

(51) Int Cl.:
***C07D 487/04*** *(2006.01)* ***A61K 31/519*** *(2006.01)*

(21) Application number: **09157018.4**

(22) Date of filing: **20.09.2004**

(84) Designated Contracting States:
**AT BE BG CH CY CZ DE DK EE ES FI FR GB GR HU IE IT LI LU MC NL PL PT RO SE SI SK TR**
Designated Extension States:
**AL HR LT LV MK**

(30) Priority: **18.09.2003 US 504135 P**
**26.07.2004 US 591467 P**

(62) Document number(s) of the earlier application(s) in accordance with Art. 76 EPC:
**04788954.8 / 1 670 802**

(71) Applicant: **Conforma Therapeutics Corporation**
**San Diego, CA 92121 (US)**

(72) Inventors:
- **Kasibhatla, Srinivas R.**
  **San Diego, CA 92130 (US)**
- **Boehm, Marcus F.**
  **San Diego, CA 92104 (US)**
- **Hong, Kevin D.**
  **San Diego, CA 92105 (US)**
- **Biamonte, Marco A.**
  **San Diego, CA 92124 (US)**
- **Shi, Jiandong**
  **San Diego, CA 92130 (US)**
- **Le Brazidec, Jean-Yves**
  **San Diego, CA 92121 (US)**
- **Zhang, Lin**
  **San Diego, CA 92129 (US)**
- **Hurst, David**
  **Santee, CA 92071 (US)**

(74) Representative: **Pohlman, Sandra M.**
**df-mp**
**Fünf Höfe**
**Theatinerstrasse 16**
**80333 München (DE)**

Remarks:
This application was filed on 31-03-2009 as a divisional application to the application mentioned under INID code 62.

(54) **Deazapurine derivatives as HSP90-Inhibitors**

(57) Heterobicyclic compounds of formula I are described and demonstrated to have utility as Heat Shock Protein 90 (HSP90) inhibiting agent. Method of synthesis and use of such compounds are also described.

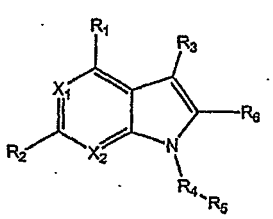

I

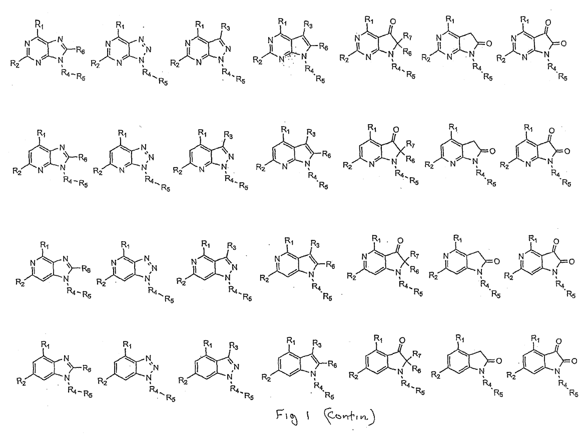

Fig 1 (Contin)

EP 2 145 888 A1

## Description

FIELD OF THE INVENTION

The invention relates in general to heterocyclics and related compounds and their broad-spectrum utility, *e.g.,* in inhibiting heat shock protein 90 (HSP90) to thereby treat or prevent HSP90-mediated diseases.

BACKGROUND

HSP90s are ubiquitous chaperone proteins that are involved in folding, activation and assembly of a wide range of proteins, including key proteins involved in signal transduction, cell cycle control and transcriptional regulation. Researchers have reported that HSP90 chaperone proteins are associated with important signaling proteins, such as steroid hormone receptors and protein kinases, including, e.g., Raf-1, EGFR, v-Src family kinases, Cdk4, and ErbB-2 ( Buchner J. TIBS 1999, 24, 136-141; Stepanova, L. et al. Genes Dev. 1996, 10, 1491-502; Dai, K. et al. J. Biol. Chem. 1996, 271, 22030-4). Studies further indicate that certain co-chaperones, *e.g.,* HSP70, p60/Hop/Sti1, Hip, Bag1, HSP40/Hdj2/Hsj1, immunophilins, p23, and p50, may assist HSP90 in its function (see, *e.g.*, Caplan, A. Trends in Cell Biol.1999, 9, 262-68).

Ansamycin antibiotics, e.g., herbimycin A (HA), geldanamycin (GM), and 17-allylaminogeldanamycin (17-AAG) are thought to exert their anticancerous effects by tight binding of the N-terminus pocket of HSP90, thereby destabilizing substrates that normally interact with HSP90 (Stebbins, C. et al. Cell1997, 89, 239-250). This pocket is highly conserved and has weak homology to the ATP-binding site of DNA gyrase (Stebbins, C. et *al., supra;* Grenert, J.P. et al. J. Biol. Chem. 1997,272,23843-50). Further, ATP and ADP have both been shown to bind this pocket with low affinity and to have weak ATPase activity (Proromou, C. et al. Cell 1997, 90, 65-75; Panaretou, B. et al. EMBO J. 1998, 17; 4829-36). *In vitro* and *in vivo* studies have demonstrated that occupancy of this N-terminal pocket by ansamycins and other HSP90 inhibitors alters HSP90 function and inhibits protein folding. At high concentrations, ansamycins and other HSP90 inhibitors have been shown to prevent binding of protein substrates to HSP90 (Scheibel, T.H. et al. Proc. Natl. Acad. Sci. USA 1999, 96, 1297-302; Schulte, T. W. et al. J. Biol. Chem. 1995, 270, 24585-8; Whitesell, L., et al. Proc. Natl. Acad. Sci. USA 1994, 91, 8324-8328). Ansamycins have also been demonstrated to inhibit the ATP-dependent release of chaperone-associated protein substrates (Schneider, C.L. et al. Proc. Natl. Acad. Sci., USA 1996, 93, 14536-41; Sepp-Lorenzino et al. J Biol. Chem. 1995, 270, 16580-16587). In either event, the substrates are degraded by a ubiquitin-dependent process in the proteasome (Schneider, C. L., *supra;* Sepp-Lorenzino, L., et al. J. Biol. Chem. 1995, 270, 16580-16587; Whitesell, L. et al. Proc. Natl. Acad. Sci. USA 1994, 91, 8324-8328).

HSP90 substrate destabilization occurs in tumor and non-transformed cells alike and has been shown to be especially effective on a subset of signaling regulators, e.g., Raf (Schulte, T. W. et al. Biochem. Biophys. Res. Commun. 1997, 239, 655-9; Schulte, T. W., et al. J. Biol. Chem. 1995, 270, 24585-8), nuclear steroid receptors (Segnitz, B.; U. Gehring J. Biol. Chem. 1997, 272,18694-18701; Smith, D. F. et al. Mol. Cell. Biol. 1995, 15, 6804-12), v-Src (Whitesell, L., et al. Proc. Natl. Acad. Sci. USA 1994, 91, 8324-8328) and certain transmembrane tyrosine kinases (Sepp-Lorenzino, L. et al. J. Biol.Chem. 1995, 270, 16580-16587) such as EGF receptor (EGFR) and HER2/Neu (Hartmann, F., et al. Int. J. Cancer 1997, 70, 221-9*;* Miller, P. et al. Cancer Res. 1994, 54, 2724-2730; Mimnaugh, E. G., et al. J. Biol. Chem. 1996, 271, 22796-801; Schnur, R. et al. J. Med. Chem. 1995, 38, 3806-3812), CDK4, and mutant p53. Erlichman et al. Proc. AACR 2001, 42, abstract 4474. The ansamycin-induced loss of these proteins leads to the selective disruption of certain regulatory pathways and results in growth arrest at specific phases of the cell cycle (Muise-Heimericks, R. C. et al. J. Biol. Chem. 1998, 273, 29864-72), and apoptosis, and/or differentiation of cells so treated (Vasilevskaya, A. et al. Cancer Res., 1999, 59, 3935-40). Ansamycins thus hold great promise for the treatment and/or prevention of many types of cancers and proliferative disorders, and also hold promise as traditional antibiotics. However, their relative insolubility makes them difficult to formulate and administer, and they are not easily synthesized and currently must, at least in part, be generated through fermentation. Further, the dose limiting toxicity of ansamyins is hepatic.

In addition to anti-cancer and antitumorgenic activity, HSP90 inhibitors have also been implicated in a wide variety of other utilities, including use as anti-inflammation agents, anti-infectious disease agents, agents for treating autoimmunity, agents for treating stroke, ischemia, multiple sclerosis, cardiac disorders, central nervous system related disorders and agents useful in promoting nerve regeneration (See, *e.g.,* Rosen et al. WO 02/09696 (PCT/US01/23640); Degranco et al. WO 99/51223 (PCT/US99/07242); Gold, U.S. Patent 6,210,974 B1; DeFranco et al., US Patent 6,174,875. Overlapping somewhat with the above, there are reports in the literature that fibrogenetic disorders including but not limited to scleroderma, polymyositis, systemic lupus, rheumatoid arthritis, liver cirrhosis, keloid formation, interstitial nephritis, and pulmonary fibrosis also may be treatable with HSP90 inhibitors. Strehlow, WO 02/02123 (PCT/US01/20578). Still further HSP90 modulation, modulators and uses thereof are reported in Application Nos. PCT/US03/04283, PCT/US02/35938, PCT/US02/16287, PCT/US02/06518, PCT/US98/09805, PCT/US00/09512, PCT/US01/09512, PCT/US01l23640, PCT/US01/46303, PCT/US01/46304, PCT/US02/06518, PCT/US02/29715,

PCT/US02/35069, PCT/US02/35938, PCT/US02/39993, 60/293,246, 60/371,668, 60/335,391, 60/128,593, 60/337,919, 60/340,762, 60/359,484 and 60/331,893.

Recently, purine derivatives showing HSP90 inhibitory activity have been reported, e.g., in PCT/US02/35069 and PCT/US02/36075. Purine moieties are well accepted bioisosteres for a variety of ATP-dependent molecular targets, see, JP 10025294; US Patent 4,748,177; US Patent 4,772,606; US Patent 6,369,092; WO 00/06573; WO 02/055521; WO 02/055082; WO 02/055083; European Patent 0178178; Eur. J Med. Chem, 1994, 29(1), 3-9; and J. Het. Chem. 1990, 27(5), 1409. However, compounds having the desired potency, selectivity and pharmaceutical properties required for effective HSP90 inhibition *in vivo* have not been reported. Therefore, a need remains for additional novel and potent HSP90 inhibitors that meet the demanding biological and pharmaceutical criteria required to proceed towards human clinical trials.

SUMMARY OF THE INVENTION

The present invention is directed towards heterocyclic compounds, particularly heterocyclic compounds and related analogs that show broad utility, *e.g.,* by inhibiting HSP90 and treating and preventing diseases that are HSP90-dependent.

In one aspect, the invention comprises the heterocyclic compounds and related analogs as specified below in Formulae A, I, IA-E, II, IIA-D, III, IIIA-B, and IV and IVA and heterocyclic compounds that are produced by a process of the invention. Also included in the scope of the present invention are stereoisomeric forms, including the individual enantiomers and diastereomers, racemic mixtures, and diastereomeric mixtures, as well as polymorphs, solvates, esters, tautomers, pharmaceutically acceptable salts and prodrugs of these compounds. Stereoisomers of the compounds of the present invention may be isolated by standard resolution techniques such as, for example, fractional crystallization and chiral column chromatography.

In one aspect, the invention provides compounds of Formula A, or a polymorph, solvate, ester, tautomer, diastereomer, enantiomer, pharmaceutically acceptable salt or prodrug thereof, which show utility for inhibiting HSP90 and/or treating and preventing diseases that are HSP90-dependent.

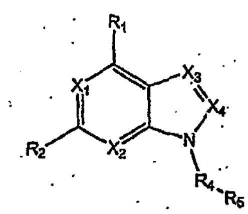

Formula A

Various genuses and subgenuses of compounds of Formula A that are within the scope of the invention are illustrated in FIGURE 1. Particularly, the invention provides compounds of Formula 1-IV.

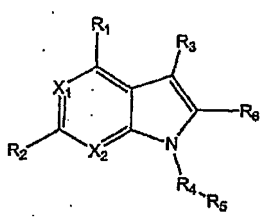

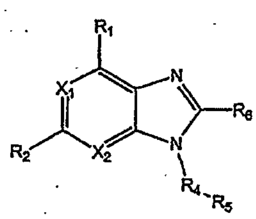

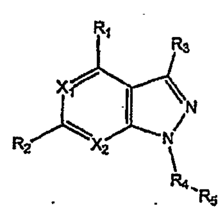

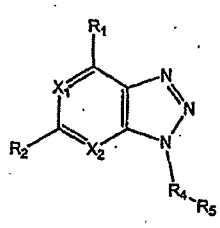

I II III IV

In another aspect, the invention provides compounds, or a polymorph, solvate, ester, tautomer, diastereomer, enantiomer, pharmaceutically acceptable salt or prodrug thereof, which show utility for inhibiting HSP90 and/or treating and preventing diseases that are HSP90-dependent, that are prepared by the process comprising:

reacting a compound of Formula Y and a compound of Formula Z, wherein:

Y is represented by any of the following formulae:

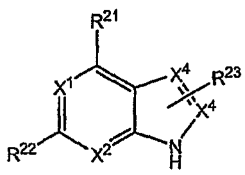
,
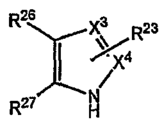
and
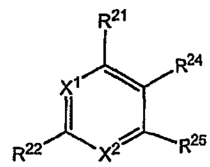
, and

Z is $L^1$-$R^4$-$R^5$.

In another aspect, the present invention is directed to pharmaceutical compositions comprising the compounds of the invention, in particular, the compounds of Formulae A, I, II, III and IV and related analogs, and compounds formed by the process of the invention, and their polymorphs, solvates, esters, tautomers, diastereomer, enantiomers, pharmaceutically acceptable salts and prodrugs thereof, and one or more pharmaceutical excipients, for use in treatment or prevention of diseases that are HSP90-dependent.

In another aspect, the invention features a method of treating an individual having an HSP90-mediated disorder by administering to the individual a pharmaceutical composition that comprises a pharmaceutically effective amount of a compound of Formulae A, I, II, III or IV, or a polymorph, solvate, ester, tautomer, diastereomer, enantiomer, enantiomers pharmaceutically acceptable salt or prodrug thereof.

In one embodiment, the invention provides a method for treating an individual having a disorder selected from the group of inflammatory diseases, infections, autoimmune disorders, stroke, ischemia, cardiac disorders, neurological disorders, fibrogenetic disorders, proliferative disorders, tumors, leukemias, neoplasms, cancers, carcinomas, metabolic diseases, and malignant diseases.

In yet another embodiment, the invention provides a method for treating an individual having a fibrogenetic disorder, such as, for example, scleroderma, polymyositis, systemic lupus, rheumatoid arthritis, liver cirrhosis, keloid formation, interstitial nephritis and pulmonary fibrosis.

In another embodiment, the invention provides a combination therapy comprising the administration of a pharmaceutically effective amount of a compound of Formulae A, I, II, III or IV and related analogs, or a polymorph, solvate, ester, tautomer, diastereomer, enantiomer, pharmaceutically acceptable salt and prodrug thereof, according to any of the preceding aspects or embodiments, and at least one therapeutic agent selected from the group of cytotoxic agents, anti-angiogenesis agents and anti-neoplastic agents. The anti-neoplastic agent may be selected from the group of alkylating agents, anti-metabolites, epidophyllotoxins antineoplastic enzymes, topoisomerase inhibitors, procarbazines, mitoxantrones, platinum coordination complexes, biological response modifiers and growth inhibitors, hormonal/anti-hormonal therapeutic agents, and haematopoietic growth factors.

Any of the above described aspects and embodiments of the invention can be combined where practical.

The individual compounds, methods and compositions prescribed do not preclude the utilization of other, unspecified steps and agents, and those of ordinary skill in the art will appreciate that additional steps and compounds may also be combined usefully within the spirit of various aspects and embodiments of the invention.

Advantages of the invention depend on the specific aspect and embodiment and may include one or more of: ease of synthesis and/or formulation, solubility, and $IC_{50}$ relative to previously existing compounds in the same or different classes of HSP90 inhibitors.

BRIEF DESCRIPTION OF THE FIGURES

FIGURE 1 shows various illustrative genuses and subgenuses of Formula A.

DETAILED DESCRIPTION OF THE PREFERRED EMBODIMENTS

## I. DEFINITIONS

A "pharmaceutically acceptable derivative or prodrug" means any pharmaceutically acceptable salt, ester, salt of an ester or other derivative of a compound of this invention, which, upon administration to a recipient, is capable of providing, either directly or indirectly, a compound of this invention or a pharmaceutically active metabolite or residue thereof. Particularly favored derivatives or prodrugs are those that increase the bioavailability of the compounds of this invention when such compounds are administered to a patient (e.g., by allowing orally administered compound to be more readily absorbed into blood) or which enhance delivery of the parent compound to a biological compartment (*e.g.,* the brain or lymphatic system).

A "pharmaceutically acceptable salt" may be prepared for any compound of the invention having a functionality capable of forming a salt, for example, an acid or base functionality. Pharmaceutically acceptable salts may be derived from organic or inorganic acids and bases. Compounds of the invention that contain one or more basic functional groups, e.g., amino or alkylamino, are capable of forming pharmaceutically acceptable salts with pharmaceutically acceptable organic and inorganic acids. These salts can be prepared *in situ* during the final isolation and purification of the compounds of the invention, or by separately reacting a purified compound of the invention in its free base form with a suitable organic or inorganic acid, and isolating the salt thus formed. Examples of suitable acid salts include acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, formate, fumarate, glucoheptanoate, glycerophosphate, glycolate, hemisulfate, heptanoate, hexanoate, hydrochloride, hydrobromide, hydroiodide, 2-hydroxyethanesulfonate, lactate, maleate, malonate, methanesulfonate, 2-napthalenesulfonate, nicotinate, nitrate, oxalate, palmoate, pectinate, persulfate, 3-phenylpropionate, phosphate, picrate, pivalate, propionate, salicylate, succinate, sulfate, tartrate, thiocyanate, tosylate and undecampate. Other acids, such as oxalic, while not in themselves pharmaceutically acceptable, may be employed in the preparation of salts useful as intermediates in obtaining the compounds of the invention and their pharmaceutically acceptable acid addition salts. See, e.g., Berge et al. "Pharmaceutical Salts", J. Pharm. Sci. 1977, 66:1-19.

Compounds of the present invention that contain one or more acidic functional groups are capable of forming pharmaceutically acceptable salts with pharmaceutically acceptable bases. The term "pharmaceutically acceptable salts" in these instances refers to the relatively non-toxic, inorganic and organic base addition salts of compounds of the present invention. These salts can likewise be prepared *in situ* during the final isolation and purification of the compounds, or by separately reacting the purified compound in its free acid form with a suitable base, such as the hydroxide, carbonate or bicarbonate of a pharmaceutically acceptable metal cation, with ammonia, or with a pharmaceutically acceptable organic primary, secondary or tertiary amine. Representative alkali or alkaline earth salts include the lithium, sodium, potassium, calcium, magnesium, and aluminum salts and the like. Illustrative examples of some of the bases that can be used include sodium hydroxide, potassium hydroxide, choline hydroxide, sodium carbonate, $N^+(C_{1-4}\text{ alkyl})_4$, and the like. Representative organic amines useful for the formation of base addition salts include ethylamine, diethylamine, ethylenediamine, ethanolamine, diethanolamine, piperazine and the like. This invention also envisions the quaternization of any basic nitrogen-containing groups of the compounds disclosed herein. Water or oil-soluble or dispersible products may be obtained by such quaternization. See, for example, Berge et al., *supra.*

Pharmaceutically acceptable prodrugs of the compounds of this invention include, but are not limited to, esters, carbonates, thiocarbonates, N-acyl derivatives, N-acyloxyalkyl derivatives, quaternary derivatives of tertiary amines, N-Mannich bases, Schiff bases, aminoacid conjugates, phosphate esters, metal salts and sulfonate esters.

Suitable positions for derivatization of the compounds of the invention to create "prodrugs" include but are not limited, 2-amino substitution. Those of ordinary skill in the art have the knowledge and means to accomplish this without undue experimentation. Various forms of prodrugs are well known in the art. For examples of such prodrug derivatives, see, *e.g.,*

a) Design of Prodrugs, Bundgaard, A. Ed., Elseview, 1985 *and* Method in Enzymology, Widder, K. et al., Ed.; Academic, 1985, vol. 42, p. 309-396;
b) Bundgaard, H. "Design and Application of Prodrugs" in A Textbook of Drug Design and Development, Krosgaard-Larsen and H. Bundgaard, Ed., 1991, Chapter 5, p. 113-191; and
c) Bundgaard, H., Advanced Drug Delivery Review, 1992, 8, 1-38. Each of which is incorporated herein by reference.

The term "prodrugs" as employed herein includes, but is not limited to, the following groups and combinations of these groups:

Amine prodrugs:

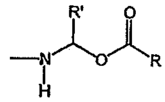
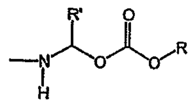
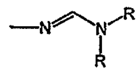
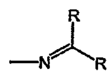
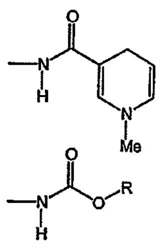
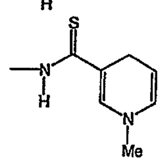
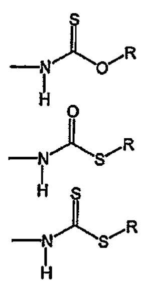
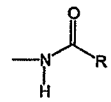
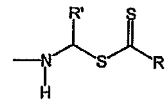
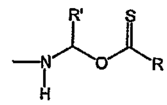
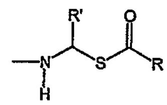
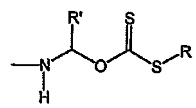
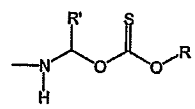
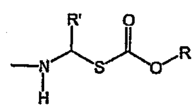
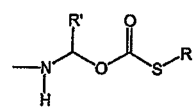
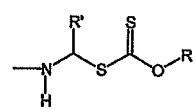
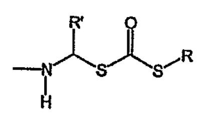

Hydroxy prodrugs:

Acyloxyalkyl esters;
Alkoxycarbonyloxyalkyl esters;
Alkyl esters;
Aryl esters; and
Disulfide containing esters.

The term "alkyl," alone or in combination, refers to an optionally substituted straight-chain, or optionally substituted branched-chain saturated hydrocarbon radical having from one to about thirty carbons, more preferably one to twelve carbons. Examples of alkyl radicals include methyl, ethyl, n-propyl, isopropyl, n-butyl, isobutyl, sec-butyl, tert-butyl, tert-amyl, pentyl, hexyl, heptyl, octyl and the like. The term "cycloalkyl" embraces cyclic alkyl radicals which include monocyclic, bicyclic, tricyclic, and higher multicyclic alkyl radicals wherein each cyclic moiety has from three to about eight carbon atoms. Examples of cycloalkyl radicals include cyclopropyl, cyclobutyl, cyclopentyl, cyclohexyl and the like.

A "lower alkyl" is a shorter alkyl, e.g., one containing from one to about six carbon atoms.

The term "alkenyl," alone or in combination, refers to an optionally substituted straight-chain, or optionally substituted branched-chain hydrocarbon radical having one or more carbon-carbon double-bonds and having from two to about thirty carbon atoms, more preferably two to about eighteen carbons. Examples of alkenyl radicals include ethenyl, propenyl, butenyl, 1,3-butadienyl and the like. The term "cylcoalkenyl" refers to cyclic alkenyl radicals which include monocyclic, bicyclic, tricyclic, and higher multicyclic alkenyl radicals wherein each cyclic moiety has from three to about eight carbon atoms. A "lower alkenyl" refers to an alkenyl having from two to about six carbons.

The term "alkynyl," alone or in combination, refers to an optionally substituted straight-chain or optionally substituted branched-chain hydrocarbon radical having one or more carbon-carbon triple-bonds and having from two to about thirty carbon atoms, more preferably from two to about tweove carbon atoms, from two to about six carbon atoms as well as those having from two to about four carbon atoms. Examples of alkynyl radicals include ethynyl, 2-propynyl, 2-butynyl, 1,3-butadiynyl and the like. The term "cycloalkynyl" refers to cyclic alkynyl radicals which include monocyclic, bicyclic, tricyclic, and higher multicyclic alkynyl radicals wherein each cyclic moiety has from three to about eight carbon atoms. A "lower alkynyl" refers to an alkynyl having from two to about six carbons.

The terms "heteroalkyl, heteroalkenyl and heteroalkynyl" include optionally substituted alkyl, alkenyl and alkynyl structures, as described above, and which have one or more skeletal chain atoms selected from an atom other than carbon, *e,g.,* oxygen, nitrogen, sulfur, phosphorous or combinations thereof.

The term "carbon chain" embraces any alkyl, alkenyl, alkynyl, or heteroalkyl, heteroalkenyl, or heteroalkynyl group, which are linear, cyclic, or any combination thereof. If the chain is part of a linker and that linker comprises one or more rings as part of the core backbone, for purposes of calculating chain length, the "chain" only includes those carbon atoms that compose the bottom or top of a given ring and not both, and where the top and bottom of the ring(s) are not equivalent in length, the shorter distance shall be used in determining the chain length. If the chain contains heteroatoms as part of the backbone, those atoms are not calculated as part of the carbon chain length.

The term "membered ring" can embrace any cyclic structure, including aromatic, heteroaromatic, alicyclic, heterocyclic and polycyclic fused ring systems as described below. The term "membered" is meant to denote the number of skeletal atoms that constitute the ring. Thus, for example, pyridine, pyran, and pyrimidine are six-membered rings and pyrrole, tetrahydrofuran, and thiophene are five-membered rings.

The term "aryl," alone or in combination, refers to an optionally substituted aromatic hydrocarbon radical of six to about twenty ring atoms, and includes mono-aromatic rings and fused aromatic ring. A fused aromatic ring radical contains from two to four fused rings where the ring of attachment is an aromatic ring, and the other individual rings within the fused ring may be aromatic, heteroaromatic, alicyclic or heterocyclic. Further, the term aryl includes mono-aromatic ring and fused aromatic rings containing from six to about twelve carbon atoms, as well as those containing from six to about ten carbon atoms. Examples of aryl groups include, without limitation, phenyl, naphthyl, anthryl, chrysenyl, and benzopyrenyl ring systems. The term "lower aryl" refers to an aryl having six to about ten skeletal ring carbons, *e.g.,* phenyl and naphthyl ring systems.

The term "heteroaryl" refers to optionally substituted aromatic radicals containing from about five to about twenty skeletal ring atoms and where one or more of the ring atoms is a heteroatom such as, for example, oxygen, nitrogen, sulfur, selenium and phosphorus. The term heteroaryl includes optionally substituted mono-heteroaryl radicals and fused heteroaryl radicals having at least one heteroatom (*e.g.*, quinoline, benzothiazole). A fused heteroaryl radical may contain from two to four fused rings and where the ring of attachment is a heteroaromatic ring, the other individual rings within the fused ring system may be aromatic, heteroaromatic, alicyclic or heterocyclic. The term heteroaryl also includes mono-heteroaryls or fused heteroaryls having from five to about twelve skeletal ring atoms, as well as those having from five to about ten skeletal ring atoms. Examples of heteroaryls include, without limitation, furanyl, benzofuranyl, chromenyl, pyridyl, pyrrolyl, indolyl, quinolinyl, pyridyl-N-oxide, pyrimidyl, pyrazinyl, imidazolyl, pyrazolyl, oxazolyl, isoxazolyl, benzothiozole, benzimidazole, benzoxazoles, benzothiadiazole, benzoxadiazole, benzotriazole, quinolines, isoquinolines, indoles, purinyl, indolizinyl, thienyl and the like and their oxides. The term "lower heteroaryl" refers to a heteroaryl having five to about ten skeletal ring atoms, *e.g.,* pyridyl, thienyl, pyrimidyl, pyrazinyl, pyrrolyl, or furanyl.

The term "alicyclic" alone or in combination, refers to an optionally substituted saturated or unsaturated nonaromatic hydrocarbon ring system containing from three to about twenty ring atoms. The term alicyclic includes mono-alicyclic and fused alicyclic radicals. A fused alicyclic may contain from two to four fused rings where the ring of attachment is an alicyclic ring, and the other individual rings within the fused-alicyclic radical may be aromatic, heteroaromatic, alicyclic and heterocyclic. The term alicyclic also includes mono-alicyclic and fused alicyclic radicals containing from three to about twelve carbon atoms, as well as those containing from three to about ten carbon atoms. Examples of alicyclics include, without limitation, cyclopropyl, cyclopropenyl, cyclobutyl, cyclopentyl, cyclodecyl, cyclododecyl, cyclopentadienyl, indanyl, and cyclooctatetraenyl ring systems. The term "lower alicyclic" refers to an alicyclic having three to about ten skeletal ring carbons, *e.g.,* cyclopropyl, cyclopropenyl, cyclobutyl, cyclopentyl, decalinyl, and cyclohexyl.

The term "heterocyclic" refers to optionally substituted saturated or unsaturated nonaromatic ring radicals containing from five to about twenty ring atoms where one or more of the ring atoms are heteroatoms such as, for

example, oxygen, nitrogen, sulfur, and phosphorus. The term alicyclic includes mono-heterocyclic and fused heterocyclic ring radicals. A fused heterocyclic radical may contain from two to four fused rings where the attaching ring is a heterocyclic, and the other individual rings within the fused heterocyclic radical may be aromatic, heteroaromatic, alicyclic or heterocyclic. The term heterocyclic also includes mono-heterocrclic and fused alicyclic radicals having from five to about twelve skeletal ring atoms, as well as those having from five to about ten skeletal ring atoms. Example of heterocyclics include without limitation, tetrahydrofuranyl, benzodiazepinyl, tetrahydroindazoleyl, dihyroquinolinyl, and the like. The term "lower heterocyclic" refers to a heterocyclic ring system having five to about ten skeletal ring atoms, *e.g.,* dihydropyranyl, pyrrolidinyl, indolyl, piperidinyl, piperazinyl, and the like.

The term "alkylaryl," or "araalkyl," alone or in combination, refers to an aryl radical as defined above in which one H atom is replaced by an alkyl radical as defined above, such as, for example, tolyl, xylyl and the like.

The term "arylalkyl," alone or in combination, refers to an alkyl radical as defined above in which one H atom is replaced by an aryl radical as defined above, such as, for example, benzyl, 2-phenylethyl and the like.

The term "heteroarylalkyl" refers to an alkyl radical as defined above in which one H atom is replaced by a heteroaryl radical as defined above, each of which may be optionally substituted.

The term "alkoxy," alone or in combination, refers to an alkyl ether radical, alkyl-O-, wherein the term alkyl is defined as above. Examples of alkoxy radicals include methoxy, ethoxy, n-propoxy, isopropoxy, n-butoxy, iso-butoxy, sec-butoxy, tert-butoxy and the like.

The term "aryloxy," alone or in combination, refers to an aryl ether radical
wherein the term aryl is defined as above. Examples of aryloxy radicals include phenoxy, benzyloxy and the like.

The term "alkylthio," alone or in combination, refers to an alkyl thio radical, alkyl-S-, wherein the term alkyl is as defined above.

The term "arylthio," alone or in combination, refers to an aryl thio radical, aryl-S-, wherein the term aryl is as defined above.

The term "heteroarylthio" refers to the group heteroaryl-S-, wherein the term heteroaryl is as defined above.

The term "acyl" refers to a radical-C(O)R where R includes alkyl, alkenyl, alkynyl, aryl, heteroaryl, alicyclic, heterocylic, arylalkyl or heteroarylalkyl wherein the alkyl, alkenyl, alkynyl, aryl, heteroryl, alicylic, heterocylic, arylalkyl or heteroaryl alkyl groups may be optionally substituted.

The term "acyloxy" refers to the ester group -OC(O)R, where R is H, alkyl, alkenyl, alkynyl, aryl, heteroaryl, alicyclic, heterocyclic, arylalkyl, or heteroarylalkyl
wherein the alkyl, alkenyl, alkynyl, aryl, heteroaryl, alicyclic, heterocyclic, arylalkyl or heteroarylalkyl may be optionally substituted.

The term "carboxy esters" refers to -C(O)OR where R is alkyl, aryl or arylalkyl,
wherein the alkyl, aryl and arylalkyl groups may be optionally substituted.

The term "carboxamido" refers to

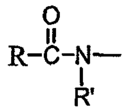

where each of R and R' are independently selected from the group consisting of H, alkyl, aryl, heteroaryl, alicyclic, heterocyclic, arylalkyl and heteroarylalkyl, wherein the alkyl, aryl, heteroaryl, alicyclic, heterocyclic, or arylalkyl groups may be optionally substituted.

The term "oxo" refers to =O.

The term "halogen" includes F, Cl, Br and I.

The terms "haloalkyl, haloalkenyl, haloalkynyl and haloalkoxy" include alkyl, alkenyl, alkynyl and alkoxy structures, as described above, that are substituted with one or more fluorines, chlorines, bromines or iodines, or with combinations thereof.

The terms "perhaloalkyl, perhaloalkyloxy and perhaloacyl" refer to alkyl, alkyloxy and acyl radicals as described above, that all the H atoms are substituted with fluorines, chlorines, bromines or iodines, or combinations thereof.

The terms "cycloalkyl, arylalkyl, aryl, heteroaryl, alicyclic, heterocyclic, alkyl, alkynyl, alkenyl, haloalkyl, and heteroalkyl" include optionally substituted cycloalkyl, arylalkyl, aryl, heteroaryl, alicyclic, heterocyclic, alkyl, alkynyl, alkenyl, haloalkyl and heteroalkyl groups.

The terms "alkylamino", refers to the group -NHR where R is independently selected from alkyl.

The terms "dialkylamino", refers to the group -NRR' where R and R' are alkyls.

The term "sulfide" refers to a sulfur atom covalently linked to two atoms; the formal oxidation state of said sulfur is (II). The term "thioether" may be used interchangebly with the term "sulfide."

The term "sulfoxide" refers to a sulfur atom covalently linked to three atoms, at least one of which is an oxygen atom; the formal oxidation state of said sulfur atom is (IV).

The term "sulfone" refers to a sulfur atom covalently linked to four atoms, at least two of which are oxygen atoms; the formal oxidation state of said sulfur atom is (VI).

The terms "optional" or "optionally" mean that the subsequently described event or circumstance may but need not occur, and that the description includes instances where the event or circumstance occurs and instances in which it does not. For example, "aryl optionally mono- or di-substituted with an alkyl" means that the alkyl may but need not be present, or either one alkyl or two may be present, and the description includes situations where the aryl is substituted with one or two alkyls and situations where the aryl is not substituted with an alkyl.

"Optionally substituted" groups may be substituted or unsubstituted. The substituents of an "optionally substituted" group may include, without limitation, one or more substituents independently selected from the following groups or designated subsets thereof: lower alkyl, lower alkenyl, lower alkynyl, lower aryl, heteroaryl, alicyclic, heterocyclic, arylalkyl, heteroarylalkyl, lower alkoxy, lower aryloxy, amino, alkylamino, dialkylamino, diarylalkylamino, alkylthio, arylthio, heteroarylthio, oxo, oxa, carbonyl (-C(O)), carboxyesters (-C(O)OR), carboxamido ($-C(O)NH_2$), carboxy, acyloxy, -H, halo, -CN, $-NO_2$, $-N_3$, -SH, -OH, $-C(O)CH_3$, perhaloalkyl, perhaloalkoxy, perhaloacyl, guanidine, pyridinyl, thiophene, furanyl, indole, indazole, esters, amides, phosphonates, phosphonic acid, phosphates, phosphoramides, sulfonates, sulfones, sulfates, sulphonamides, carbamates, ureas, thioureas, thioamides, thioalkyls. An optionally substituted group may be unsubstituted (*e.g.,* $-CH_2CH_3$), fully substituted (e.g., $-CF_2CF_3$), monosubstituted (e.g., $-CH_2CH_2F$) or substituted at a level anywhere in-between fully substituted and monosubstututed (*e.g.,* $-CH_2CF_3$).

The term "pyridine-1-oxy" also means "pyridine-N-oxy."

Some of the compounds of the present invention may contain one or more chiral centers and therefore may exist in enantiomeric and diastereomeric forms. The scope of the present invention is intended to cover all isomers *per se,* as well as mixtures of *cis* and *trans* isomers, mixtures of diastereomers and racemic mixtures of enantiomers (optical isomers) as well. Further, it is possible using well known techniques to separate the various forms, and some embodiments of the invention may feature purified or enriched species of a given enantiomer or diasteriomer.

A "pharmacological composition" refers to a mixture of one or more of the compounds described herein, or pharmaceutically acceptable salts thereof, with other chemical components, such as pharmaceutically acceptable carriers and/or excipients. The purpose of a pharmacological composition is to facilitate administration of a compound to an organism.

The phrase "pharmaceutically acceptable carrier" as used herein means a pharmaceutically-acceptable material, composition or vehicle, such as a liquid or solid filler, diluent, excipient, solvent or encapsulating material, involved in carrying or transporting the subject agent from one organ, or portion of the body, to another organ, or portion of the body. Each carrier must be "acceptable" in the sense of being compatible with the other ingredients of the formulation and not injurious to the patient. Some examples of materials which can serve as pharmaceutically-acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) phosphate buffer solutions; and (21) other non-toxic compatible substances employed in pharmaceutical formulations. A physiologically acceptable carrier should not cause significant irritation to an organism and does not abrogate the biological activity and properties of the administered compound.

An "excipient" refers to an inert substance added to a pharmacological composition to further facilitate administration of a compound. Examples of excipients include but are not limited to calcium carbonate, calcium phosphate, various sugars and types of starch, cellulose derivatives, gelatin, vegetable oils and polyethylene glycols.

A "pharmaceutically effective amount" means an amount which is capable of providing a therapeutic and/or prophylactic effect. The specific dose of compound administered according to this invention to obtain therapeutic and/or prophylactic effect will, of course, be determined by the particular circumstances surrounding the case, including, for example, the specific compound administered, the route of administration, the condition being treated, and the individual being treated. A typical daily dose (administered in single or divided doses) will contain a dosage level of from about 0.01 mg/kg to about 50-100 mg/kg of body weight of an active compound of the invention. Preferred daily doses generally will be from about 0.05 mg/kg to about 20 mg/kg and ideally from about 0.1 mg/kg to about 10 mg/kg. Factors such as clearance rate, half-life and maximum tolerated dose (MTD) have yet to be determined but one of ordinary skill in the art can determine these using standard procedures.

In some method embodiments, the preferred therapeutic effect is the inhibition, to some extent, of the growth of cells characteristic of a proliferative disorder, *e.g.,* breast cancer. A therapeutic effect will also normally, but need not,

relieve to some extent one or more of the symptoms other than cell growth or size of cell mass. A therapeutic effect may include, for example, one or more of 1) a reduction in the number of cells; 2) a reduction in cell size; 3) inhibition (*i.e.,* slowing to some extent, preferably stopping) of cell infiltration into peripheral organs, *e.g.,* in the instance of cancer metastasis; 3) inhibition (*i.e.,* slowing to some extent, preferably stopping) of tumor metastasis; 4) inhibition, to some extent, of cell growth; and/or 5) relieving to some extent one or more of the symptoms associated with the disorder.

As used herein, the term $IC_{50}$ refers to an amount, concentration or dosage of a particular test compound that achieves a 50% inhibition of a maximal response in an assay that measures such response. In some method embodiments of the invention, the "$IC_{50}$" value of a compound of the invention can be greater for normal cells than for cells exhibiting a proliferative disorder, *e.g.*, breast cancer cells. The value depends on the assay used.

By a "standard" is meant a positive or negative control. A negative control in the context of HER2 expression levels is, *e.g.,* a sample possessing an amount of HER2 protein that correlates with a normal cell. A negative control may also include a sample that contains no HER2 protein. By contrast, a positive control does contain HER2 protein, preferably of an amount that correlates with overexpression as found in proliferative disorders, *e.g.,* breast cancers. The controls may be from cell or tissue samples, or else contain purified ligand (or absent ligand), immobilized or otherwise. In some embodiments, one or more of the controls may be in the form of a diagnostic "dipstick."

By "selectively targeting" is meant affecting one type of cell to a greater extent than another, *e.g.*, in the case of cells with high as opposed to relatively low or normal HER2 levels.

## II. COMPOUNDS OF THE INVENTION

Compounds of the invention and their polymorphs, solvates, esters, tautomers, diastereomers, enantiomers, pharmaceutically acceptable salts or prodrugs show utility for inhibiting HSP90 and treating and preventing diseases that are HSP90-dependent.

One embodiment of the compounds of the invention is of Formula A:

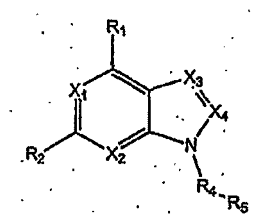

A

or a polymorph, solvate, ester, tautomer, diastereomer, enantiomer, pharmaceutically acceptable salt or prodrug thereof, wherein:

$X^1$ and $X^2$ are the same or different and each is nitrogen or $-CR^6$;
$X^3$ is nitrogen or $-CR^3$ wherein $R^3$ is hydrogen, OH, a keto tautomer, $-OR^8$, -CN, halogen, lower alkyl, or $-C(O)R^9$;
$X^4$ is nitrogen or a group $CR^6$ when $X^3$ is nitrogen, and $X_4$ is $-CR^6R^7$ when $X_3$ is - $CR^3$;
$R^1$ is halogen, $-OR^8$, $-SR^8$, or lower alkyl;
$R^2$ is $-NR^8R^{10}$;
$R^4$ is $-(CH_2)_n-$ wherein n = 0-3, -C(O), -C(S), $-SO_2-$, or $-SO_2N-$; and
$R^5$ is alkyl, aryl, heteroaryl, alicyclic, or heterocyclic, each of which is optionally bi-or tricyclic, and optionally substituted with H, halogen, lower alkyl, lower alkenyl, lower alkynyl, lower aryl, lower alicyclic, araalkyl, aryloxyalkyl, alkoxyalkyl, perhaloalkyl, perhaloalkyloxy, perhaloacyl, $-N_3$, $-SR^8$, $-OR^8$, -CN, $-CO_2R^9$, $-NO_2$, or - $NR^8R^{10}$;
with the provisos that:

the compound is not one found or described in one or more of JP 10025294; US Patent 4,748,177; US Patent 4,748,177; US Patent 6,369,092; WO 00/06573; WO 02/055521; WO 02/055082; WO 02/055083; Eur. J. Med. Chem., 1994, 29(1), 3-9; and J. Het. Chem. 1990, 27(5), 1409;
$-R^4R^5$ is not a ribose or derivative thereof, or a sugar or derivative thereof;
$-R^4R^5$ is not a phosphonate or phosphonic acid, or a group substituted with a phosphonate or phosphonic acid; and
when $R^4$ is $(CH_2)_n$ where n= 0 or 1, then $R^4$ and $R^5$ are not connected with 'O', e.g., $-CH_2-O-CH_2-$ or $-CH_2-CH_2-O-CH_2-$.

In one embodiment of the compound, tautomer, pharmaceutically acceptable salt thereof, or prodrug thereof of Formula A, $X_1$ and $X_2$ are the same or different and each is nitrogen or $-CR^6$; $R^1$ is halogen, $-OR^8$, $-SR^8$, or lower alkyl; $R^2$ is $-NR^8R^{10}$; R3 is hydrogen, -OH or keto tautomer, $-OR^8$, halogen, -CN, lower alkyl, or -C(O)R9; $R^4$ is - $(CH_2)_n$- wherein n = 0-3, -C(O), -C(S), $-SO_2-$, or $-SO_2N-$; and $R^5$ is alkyl, aromatic, heteroaromatic, alicyclic, heterocyclic, each of which is optionally bi- or tricyclic, and optionally substituted with H, halogen, lower alkyl, $-SR^8$, $-OR^8$, -CN, $-CO_2R^9$, $-NO_2$ or $-NR^8R^{10}$; $R^8$ is hydrogen, lower alkyl, lower aryl or $-(CO)R^9$; $R^9$ is lower alkyl, lower aryl, lower heteroaryl, $-NR^8R^{10}$ or $OR^{11}$; $R^{11}$ is lower alkyl or lower aryl; and $R^{10}$ is hydrogen or lower alkyl.

In one embodiment, the compound, tautomer, pharmaceutically acceptable salt thereof, or prodrug thereof of Formula A, $R^1$ is selected from halogen, hydroxyl, lower alkoxy, lower thioalkyl and $C_{1-4}$ alkyl; and $R^2$ is $-NH_2$; R3 is hydrogen.

In another embodiment, $R^4$ is $-(CH_2)_n-$, where n = 0-3.

In another embodiment, $R^1$ is selected from halogen, hydroxyl, lower alkoxy, lower thioalkyl or $C_{1-4}$ alkyl; optionally, $R^2$ is $NH_2$.

In another embodiment, $R^4$ is $-CH_2-$.

In another embodiment, $R^4$ is $-(CH_2)_n-$, wherein n = 0-3, $R^1$ is selected from halogen, hydroxyl, lower alkoxy, lower thioalkyl, and $C_{1-4}$ alkyl, and $R^2$ is optionally $NH_2$.

In another embodiment, $R^1$ is halogen, hydroxyl, lower alkoxy, lower thioalkyl, or $C_{1-4}$ alkyl; and $R^2$ is optionally $NH_2$, $R^4$ is $-(CH_2)-$, and $R^5$ is phenyl, benzyl, or pyridyl, all optionally substituted with H, halogen, lower alkyl, $-SR^8$, $-OR^8$ (or cyclic ethers such as methylenedioxy), -CN, $-CO_2R^9$, $NO_2$, or $-NR^8R^{10}$; $R^8$ is hydrogen, lower alkyl, lower aryl or $-(CO)R^9$; $R^9$ is lower alkyl, lower aryl, lower heteroaryl, $-NR^8R^{10}$ or $-OR^{11}$; $R^{11}$ is lower alkyl or lower aryl; and $R^{10}$ is hydrogen or lower alkyl.

In another embodiment $R^1$ is halogen, $R^2$ is $-NH_2$, $R^4$ is $-CH_2-$, $R^6$ is H or halogen, and $R^5$ is phenyl optionally substituted with H, halogen, $C_{1-4}$ alkyl, $C_{1-4}$ alkoxy, $C_{1-4}$ alkylthio, perhaloalkyl, perhaloalkyloxy, -CN, $-NO_2$, $-NH_2$ or $-CO_2R^{11}$.

In another embodiment, $R^1$ is halogen, $R^2$ is $-NH_2$, $R^4$ is $-CH_2-$, $R^6$ is H, and $R^5$ is 2-halo-3, 5-dimethoxyphenyl optionally substituted with H, halogen, $C_{1-4}$ alkyl, $C_{1-4}$ alkoxy, $C_{1-4}$ alkylthio, perhaloalkyl, perhaloalkyloxy, -CN, $-NO_2$, $-NH_2$, or $-CO_2R^{11}$ at the para (4-) position.

In another embodiment, $R^1$ is chloro, $R^2$ is $-NH_2$, $R^4$ is $-CH_2-$, $R^6$ is H and $R^5$ is 2-chloro-3, 4,5-trimethoxyphenyl.

In another embodiment, $R^1$ is chloro, $R^2$ is $-NH_2$, $R^4$ is $-CH_2-$, $R^6$ is H and $R^5$ is 2-bromo-3, 4,5-trimethoxyphenyl. In other embodiments, $R^5$ is selected from 2-iodo-3,4,5-trimethoxyphenyl, 2-fluoro-3,4,5-trimethoxyphenyl, or 2-bromo-3,4,5-trimethoxyphenyl.

Any of the foregoing embodiments can be combined where feasible and appropriate.

In one aspect, the invention provides compounds of Formula I:

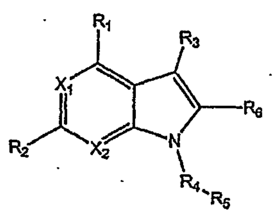

I

or tautomer, pharmaceutically acceptable salt, or prodrug thereof, wherein:

$X^1$ and $X^2$ are the same or different and each is nitrogen or $-CR_6$;
$R_1$ is halogen, $-OR_8$, $-SR_8$ or lower alkyl;
$R_2$ is $-NR_8R_{10}$;
$R_3$ is hydrogen, OH or a keto tautomer, $-OR_8$, halogen, -CN, lower alkyl or $-C(O)R_9$;
$R_4$ is $-(CH_2)_n-$ where n = 0-3, -C(O), -C(S), $-SO_2-$ or $-SO_2N-$;
$R_6$ is hydrogen, halogen, lower alkyl, $-SR_8$, $-OR_8$, $-NR_8R_{10}$, $-N_3$, or $-C(O)R_9$;
$R_5$ is alkyl, aromatic, heteroaromatic, alicyclic, heterocyclic, all optionally bi- or tricyclic, and all optionally substituted with H, halogen, lower alkyl, $-SR_8$, $-OR_8$, -CN, $-CO_2R_9$, $-NO_2$, or $-NR_8R_{10}$;
$R_8$ is hydrogen, lower alkyl, lower aryl, or $-(CO)R_9$;
$R_9$ is lower alkyl, lower aryl, lower heteroaryl, $-NR_8R_{10}$ or $-OR_{11}$;
$R_{10}$ is hydrogen or lower alkyl; and
$R_{11}$ is lower alkyl or lower aryl.

In one embodiment of the compounds of Formula I, or a tautomer, pharmaceutically acceptable salt, or prodrug thereof, $R_1$ is halogen, hydroxyl; lower alkoxy, lower thioalkyl, or $C_{1-4}$ alkyl; and $R^2$ is $NH_2$.

In another embodiment of the compounds of Formula I, or a tautomer, pharmaceutically acceptable salt, or prodrug thereof, $R^4$ is-$(CH_2)_n$-, wherein n = 0-3.

In another embodiment of the compounds of Formula I, or a tautomer, pharmaceutically acceptable salt, or prodrug thereof, $R_1$ is halogen, hydroxyl, lower alkoxy, lower thioalkyl, or $C_{1-4}$ alkyl; and $R^2$ is $NH_2$; $R^4$ is-$(CH_2)_n$-, wherein n = 0-3.

In another embodiment of the compounds of Formula I, or a tautomer, pharmaceutically acceptable salt, or prodrug thereof, $R_1$ is halogen; $R^2$ is $NH_2$; and $R^4$ is - $CH_2$-.

In another aspect, the invention provides compounds of Formula IA:

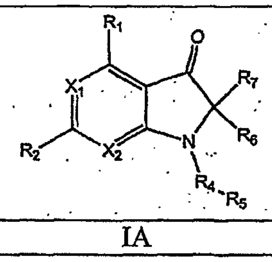

IA

or tautomer, pharmaceutically acceptable salt, or prodrug thereof, wherein:

$X_1$ and $X_2$ are the same or different and each is nitrogen or a group -$CR^6$;
$R^1$ is halogen, -$OR^8$, -$SR^8$, or lower alkyl;
$R^2$ is -$NR^8R^{10}$;
$R^4$ is -$(CH_2)_n$- where n = 0-3, -C(O), -C(S), -$SO_2$- or -$SO_2N$-;
$R^5$ is alkyl, aromatic, heteroaromatic, alicyclic, heterocyclic, all optionally bi- or tricyclic, and all optionally substituted with H, halogen, lower alkyl, -$SR^8$, -$OR^8$, -CN,
-$CO_2R^9$, -$NO_2$, or -$NR^8R^{10}$;
$R^6$ is hydrogen, halogen, lower alkyl, -$SR^8$, -$OR^8$, -$NR^8R^{10}$, -$N_3$, -CN, -C(O)$R^9$, or taken together with $R^7$ is carbonyl (C=O);
$R^7$ is independently selected from hydrogen, lower alkyl or taken together with $R^6$ is
-C(O);
$R^8$ is hydrogen, lower alkyl, lower aryl, or -(CO)$R^9$;
$R^9$ is lower alkyl, lower aryl, lower heteroaryl, -$NR^8R^{10}$ or-$OR^{11}$;
$R^{10}$ is hydrogen or lower alkyl, and
$R^{11}$ is lower alkyl or lower aryl.

In one embodiment of the compounds of Formula IA, or a tautomer, pharmaceutically acceptable salt, or prodrug thereof, $R_1$ is halogen, hydroxyl, lower alkoxy, lower thioalkyl, or $C_{1-4}$ alkyl; and $R^2$ is $NH_2$.

In another embodiment of the compounds of Formula IA, or a tautomer, pharmaceutically acceptable salt thereof, or prodrug thereof, $R^4$ is-$(CH_2)_n$-, where n = 0-3.

In another embodiment of the compounds of Formula IA, or a tautomer, pharmaceutically acceptable salt, or prodrug thereof, $R_1$ is halogen, hydroxyl, lower alkoxy, lower thioalkyl, or $C_{1-4}$ alkyl; and $R^2$ is $NH_2$; $R^4$ is-$(CH_2)_n$-, and wherein n = 0-3.

In another embodiment of the compounds of Formula IA, or a tautomer, pharmaceutically acceptable salt thereof, $R_1$ is halogen; $R^2$ is $NH_2$, $R^4$ is -$CH_2$-.

In one embodiment, the invention provides compounds of Formula IB:

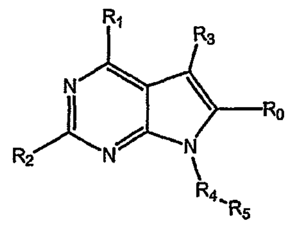

IB

or a polymorph, solvate, ester, tautomer, diastereomer, enantiomer, pharmaceutically acceptable salt or prodrug thereof, wherein:

$R^0$ is selected from hydrogen, halogen, lower alkyl, -$SR^8$, -$OR^8$, -CN, and -$NHR^8$,
$R^1$ is halogen, -$OR^{11}$, -$SR^{11}$ or lower alkyl;
$R^2$ is -$NHR^8$;
$R^3$ is selected from the group consisting of hydrogen, halogen, -$SR^8$, -$OR^8$, -CN, - C(O)$R^9$,
-C(O)OH, -$NO_2$, -$NR^8R^{10}$, lower alkyl, lower alkenyl, lower alkynyl, lower perhaloalkyl, aryl, heteroaryl, alicyclic and heterocyclic, all optionally substituted, wherein:

the aryl, heteroaryl, alicyclic and heterocyclic groups are optionally mono-, bi- or tri-cyclic,
$R^8$ and $R^{10}$ taken together optionally form a ring of 3-7 ring atoms and optionally 1-3 of the ring atoms are heteroatoms selected from the group of O, S and N, and
the optional substituents on $R^3$ are selected from the group consisting of halogen, lower alkyl, lower alkenyl, lower alkynyl, -$SR^8$, -$OR^8$, - CN, -C(O)OH,
-C(O)$R^9$, -$NO_2$, -$NR^8R^{10}$, lower aryl, heteroaryl, alicyclic, lower heterocyclic, arylalkyl, heteroarylalkyl, amino, alkylamino, dialkylamino, diarylalkylamino, oxo, oxa, perhaloalkyl, perhaloalkoxy, perhaloacyl, guanidine, pyridinyl, thiophene, furanyl, indole, indazole, phosphonates, phosphates, phosphoramides, sulfonates, sulfones, sulfates, sulphonamides, carbamates, ureas, thioureas and thioamides, wherein $R^8$ and $R^{10}$ taken together optionally form a ring of 3-7 ring atoms and optionally 1-3 of the ring atoms are heteroatoms selected from the group of O, S and N;

$R^0$ or $R^3$ is -OH or -SH, the compound may exist as the corresponding (thio)keto tautomer or a mixture of keto-enol tautomers;
$R^4$ is -$CHR^{12}$-, -C(O)-, -C(S)-, -S(O)- or -$SO_2$-;
$R^5$ is aryl, heteroaryl, alicyclic, or heterocyclic, wherein
the aryl group is substituted with 3 to 5 substituents, the heteroaryl group is substituted with 2 to 5 substituents, the alicyclic group is substituted with 3 to 5 substituents, the heterocyclic group is substituted with 3 to 5 substituents, and the substituents are selected from the group consisting of halogen, lower
alkyl, lower alkenyl, lower alkynyl, -$SR^8$, -$OR^8$, -CN, -C(O)OH, - C(O)$R^9$, -$NO_2$, -$NR^8R^{10}$, lower aryl, heteroaryl, alicyclic, lower heterocyclic, arylalkyl, heteroarylalkyl, amino, alkylamino, dialkylamino, diarylalkylamino, oxo, oxa, perhaloalkyl, perhaloalkoxy, perhaloacyl, guanidine, pyridinyl, thiophene, furanyl, indole, indazole, phosphonates, phosphates, phosphoramides, sulfonates, sulfones, sulfates, sulphonamides, carbamates, ureas, thioureas and thioamides, wherein $R^8$ and $R^{10}$ taken together optionally form a ring of 3-7 ring atoms and optionally 1-3 of the ring atoms are heteroatoms selected from the group of O, S and N;
$R^8$ is hydrogen, lower alkyl, lower alkenyl, or lower alkynyl, lower aryl, lower heteroaryl, or -C(O)$R^9$;
$R^9$ is H, lower alkyl, lower alkenyl, lower alkynyl, lower aryl, lower heteroaryl, - $NR^{10}R^{10}$, or -$OR^{11}$, wherein $R^{10}$ and $R^{10}$ taken together optionally form a ring of 3-7 ring atoms and optionally 1-3 of the ring atoms are heteroatoms selected from the group of O, S and N;
$R^{10}$ is hydrogen, lower alkyl, lower alkenyl, lower alkynyl, lower aryl or lower heteroaryl;
$R^{11}$ is lower alkyl, lower alkenyl, or lower alkynyl, lower heteroaryl or lower aryl; and
$R^{12}$ is hydrogen or lower alkyl.

In one embodiment of the compounds of Formula IB, or a polymorph, solvate, ester, tautomer, enantiomer, pharmaceutically acceptable salt or prodrug thereof, each of the aryl, heteroaryl, alicyclic or heterocyclic group is monocyclic or bicyclic.

In another embodiment of the compounds of Formula IB, or a polymorph, solvate, ester, tautomer, enantiomer,

pharmaceutically acceptable salt or prodrug thereof, $R^0$ is hydrogen, halogen, -SH, -OH, or-CN; $R^1$ is halogen; and $R^2$ is $-NHR^8$, where $R^8$ is hydrogen or $-C(O)R^9$,

In another embodiment of the compounds of Formula IB, or a polymorph, solvate, ester, tautomer, enantiomer, pharmaceutically acceptable salt or prodrug thereof, $R^1$ is chloro or bromo, $R^2$ is $-NHR^8$, where $R^8$ is hydrogen or -C (O)$R^9$; $R^3$ is hydrogen, halogen, $OR^8$, $SR^8$, $NR^8R^{10}$, lower alkyl, lower alkenyl, or lower alkynyl, lower perhaloalkyl, lower aryl, or lower heteroaryl.

In another embodiment of the compounds of Formula IB, or a polymorph, solvate, ester, tautomer, enantiomer, pharmaceutically acceptable salt or prodrug thereof, $R^0$ is hydrogen, halogen or -CN; $R^2$ is $-NHR^8$, where $R^8$ is hydrogen or $-C(O)R^9$; and $R^4$ is $-CH_2-$.

In another embodiment of the compounds of Formula IB, or a polymorph, solvate, ester, tautomer, enantiomer, pharmaceutically acceptable salt or prodrug thereof, $R^0$ is hydrogen, halogen, -SH, -OH or-CN; $R^1$ is halogen; $R^2$ is $-NH_2$, $R^3$ is hydrogen, halogen, $-OR^8$, $-SR^8$,
$-NR^8R^{10}$, lower alkyl, lower alkenyl, lower alkynyl, perhaloalkyl, lower aryl, or lower heteroaryl, wherein $R^8$ is hydrogen, lower alkyl, lower aryl, or -C (O)$R^9$; $R^4$ is $-CH_2-$; and $R^5$ is aryl or heteroaryl, wherein each of said aryl and heteroaryl is monocyclic or bicyclic and is substituted with 3 to 5 substituents.

In another embodiment of the compounds of Formula IB, or a polymorph, solvate, ester, tautomer, enantiomer, pharmaceutically acceptable salt or prodrug thereof, $R^1$ is chloro or bromo, $R^2$ is $-NH_2$, and $R^5$ is a phenyl having at least three substituents.

In another embodiment of the compounds of Formula IB, or a polymorph, solvate, ester, tautomer, enantiomer, pharmaceutically acceptable salt or prodrug thereof, $R^1$ is chloro or bromo, $R^2$ is $-NH_2$ and $R^5$ is a pyridyl having at least two substituents.

In another embodiment of the compounds of Formula IB, or a polymorph, solvate, ester, tautomer, enantiomer, pharmaceutically acceptable salt or prodrug thereof, $R^1$ is chloro or bromo, $R^2$ is $-NH_2$, and $R^5$ is 1-oxy-pyridyl (N-oxy-pyridyl) having at least two substituents.

Another embodiment of the invention is compounds of Formula IC:

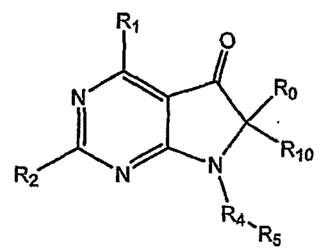

IC

or a polymorph, solvate, ester, tautomer, enantiomer, pharmaceutically acceptable salt or prodrug thereof, wherein:

- $R^0$ is hydrogen, halogen, lower alkyl, $-SR^8$, $-OR^8$, -CN or $-NHR^8$;
- $R^1$ is halogen, $-OR^{11}$, $-SR^{11}$ or lower alkyl;
- $R^2$ is $-NH_2$;
- $R^4$ is $-CHR^{12}-$, -C(O)-, -C(S)-, -S(O)- or $-SO_2-$;
- $R^5$ is aryl, heteroaryl, alicyclic, or heterocyclic, wherein:

  the aryl group is substituted with 3 to 5 substituents,
  the heteroaryl group is substituted with 2 to 5 substituents,
  the alicyclic group is substituted with 3 to 5 substituents,
  the heterocyclic group is substituted with 3 to 5 substituents, and
  the substituents on $R^5$ are selected from the group consisting of halogen, lower alkyl, lower alkenyl, lower alkynyl, $-SR^8$, $-OR^8$, -CN, - C(O)OH, $-C(O)R^9$, $-NO_2$, $-NR^8R^{10}$, lower aryl, heteroaryl, alicyclic, lower heterocyclic, arylalkyl, heteroarylalkyl, amino, alkylamino, dialkylamino, diarylalkylamino, oxo, oxa, perhaloalkyl, perhaloalkoxy, perhaloacyl, guanidine, pyridinyl, thiophene, furanyl, indole, indazole, phosphonates, phosphates, phosphoramides, sulfonates, sulfones, sulfates, sulphonamides, carbamates, ureas, thioureas and thioamides, wherein when $R^8$ and $R^{10}$ taken together optionally form a ring of 3-7 ring atoms and optionally 1-3 of the ring atoms are heteroatoms selected from the group of O, S and N;

$R^8$ is hydrogen, lower alkyl, lower alkenyl, lower alkynyl, lower aryl, lower heteroaryl, or -C(O)$R^9$;
$R^9$ is H, lower alkyl, lower aryl, lower heteroaryl, -$NR^{10}R^{10}$, or -$OR^{11}$, wherein $R^{10}$ and $R^{10}$ taken together optionally form a ring of 3-7 ring atoms and optionally 1-3 of the ring atoms are heteroatoms selected from the group of O, S and N;
$R^{10}$ is hydrogen, lower alkyl, lower heteroaryl, lower aryl, lower alkenyl, or lower alkynyl;
$R^{11}$ is lower alkyl, lower alkenyl, or lower alkynyl, lower heteroaryl or lower aryl;
$R^{12}$ is hydrogen or lower alkyl; and
$R^0$ and $R^{10}$ when taken together optionally form an exocyclic double bond which is optionally substituted, or optionally form a ring of 3-7 ring atoms and optionally
1-3 of the ring atoms are heteroatoms selected from the group of O, S and N.

In one embodiment of the compounds of Formula IC, or a polymorph, solvate, ester, tautomer, enantiomer, pharmaceutically acceptable salt or prodrug thereof, $R^1$ is halogen or lower alkyl; $R^4$ is -$CHR^{12}$-; $R^5$ is aryl or heteroaryl, wherein each of said aryl and heteroaryl is monocyclic or bicyclic and is substituted with 3 to 5 substituents.

In another embodiment of the compounds of Formula IC, or a polymorph, solvate, ester, tautomer, enantiomer, pharmaceutically acceptable salt or prodrug thereof, $R^0$ is hydrogen or -$NHR_8$, $R^1$ is halogen, -$OR^{11}$, -$SR^{11}$ or lower alkyl; $R^{10}$ is hydrogen or lower alkyl.

In another embodiment of the compounds of Formula IC, or a polymorph, solvate, ester, tautomer, enantiomer, pharmaceutically acceptable salt or prodrug thereof, $R^0$ is hydrogen; $R^1$ is halogen; $R^4$ is -$CH_2$-; $R^5$ is aryl or heteroaryl, wherein each of said aryl and heteroaryl is monocyclic or bicyclic and is substituted with 3 to 5 substituents; and $R^{10}$ is hydrogen.

In another embodiment of the compounds of Formula IC, or a polymorph, solvate, ester, tautomer, enantiomer, pharmaceutically acceptable salt or prodrug thereof, wherein $R^1$ is chloro or bromo, $R^5$ is phenyl, pyridyl or 1-oxy-pyridyl (N-oxy-pyridyl) each of $R^5$ has at least two substituents.

Another embodiment of the invention is compounds represented by Formula ID:

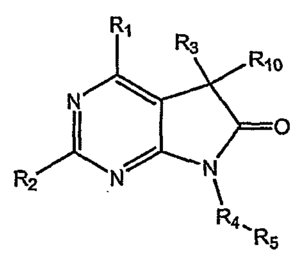

ID

or a polymorph, solvate, ester, tautomer, enantiomer, pharmaceutically acceptable salt or prodrug thereof:wherein:

$R^1$ is halogen, -$OR^{11}$, -$SR^{11}$ or lower alkyl;
$R^2$ is -$NH_2$;
$R^3$ is selected from the group consisting of hydrogen, halogen, -$SR^8$, -$OR^8$, -CN, - C(O)$R^9$, -C(O)OH, -$NO_2$, -$NR^8R^{10}$, lower alkyl, lower alkenyl, lower alkynyl, lower perhaloalkyl, aryl, heteroaryl, alicyclic, heterocyclic, all optionally substituted, wherein:

the aryl, heteroaryl, alicyclic and heterocyclic groups are optionally mono-, bi- or tri-cyclic,
$R^8$ and $R^{10}$ taken together optionally form a ring of 3-7 ring atoms and optionally 1-3 of the ring atoms are heteroatoms selected from the group of O, S and N, and
the optional substituents on $R^3$ are selected from the group consisting of halogen, lower alkyl, lower alkenyl, lower alkynyl, -$SR^8$, -$OR^8$,-CN, -C(O)OH, -C(O)$R^9$, -$NO_2$, -$NR^8R^{10}$, lower aryl, heteroaryl, alicyclic, lower heterocyclic, arylalkyl, heteroarylalkyl, amino, alkylamino, dialkylamino, diarylalkylamino, oxo, oxa, perhaloalkyl, perhaloalkoxy, perhaloacyl, guanidine, pyridinyl, thiophene, furanyl, indole, indazole, phosphonates, phosphates, phosphoramides, sulfonates, sulfones, sulfates, sulphonamides, carbamates, ureas, thioureas and thioamides, wherein $R^8$ and $R^{10}$ taken together optionally form a ring of 3-7 ring atoms and optionally 1-3 of the ring atoms are heteroatoms selected from the group of O, S and N;

$R^4$ is -$CHR^{12}$-, -C(O)-, -C(S)-, -S(O)- or -$SO_2$-;

$R^5$ is aryl, heteroaryl, alicyclic, or heterocyclic, wherein
the aryl group is substituted with 3 to 5 substituents,
the heteroaryl group is substituted with 2 to 5 substituents,
the alicyclic group is substituted with 3 to 5 substituents,
the heterocyclic group is substituted with 3 to 5 substituents, and
the substituents on $R^5$ are selected from the group consisting of halogen, lower alkyl, lower alkenyl, lower alkynyl, $-SR^8$, $-OR^8$, -CN, - C(O)OH, $-C(O)R^9$, $-NO_2$, $-NR^8R^{10}$, lower aryl, heteroaryl, alicyclic, lower heterocyclic, arylalkyl, heteroarylalkyl, amino, alkylamino, dialkylamino, diarylalkylamino, oxo, oxa, perhaloalkyl, perhaloalkoxy, perhaloacyl, guanidine, pyridinyl, thiophene, furanyl, indole, indazole, phosphonates, phosphates, phosphoramides, sulfonates, sulfones, sulfates, sulphonamides, carbamates, ureas, thioureas and thioamides, wherein $R^8$ and $R^{10}$ taken together optionally form a ring of 3-7 ring atoms and optionally 1-3 of the ring atoms are heteroatoms selected from the group of O, S and N;
$R^8$ is hydrogen, lower alkyl, lower alkenyl, lower alkynyl, lower aryl, lower heteroaryl, or $-C(O)R^9$.
$R^9$ is H, lower alkyl, lower aryl, lower heteroaryl, $-NR^{10}R^{10}$. or $-OR^{11}$, wherein when $R^{10}$ and $R^{10}$ taken together optionally form a ring of 3-7 ring atoms and optionally 1-3 of the ring atoms are heteroatoms selected from the group of O, S and N;
$R^{10}$ is hydrogen, lower alkyl, lower heteroaryl, lower aryl, lower alkenyl, or lower alkynyl,
$R^{11}$ is lower alkyl, lower alkenyl, lower alkynyl, lower heteroaryl or lower aryl;
$R^{12}$ is hydrogen or lower alkyl; and
$R^3$ and $R^{10}$ taken together optionally form an exocyclic double bond which is optionally substituted, or optionally form a ring of 3-7 ring atoms and optionally 1-3 of the ring atoms are heteroatoms selected from the group of O, S and N.

In one embodiment of the compounds of Formula ID, or a polymorph, solvate, ester, tautomer, enantiomers, pharmaceutically acceptable salt or prodrug thereof, $R^1$ is halogen; $R^3$ is hydrogen, halogen, $-OR^8$, $-SR^8$, $-NR^8R^8$, lower alkyl, lower alkenyl, or lower alkynyl, lower perhaloalkyl, lower aryl, or lower heteroaryl, wherein $R^8$ is hydrogen, lower alkyl, lower alkenyl, or lower alkynyl, lower aryl, lower heteroaryl, or - C (O)$R^9$; $R^4$ is $-CH_2-$; $R^5$ is aryl or heteroaryl, wherein each of said aryl and heteroaryl is monocyclic or bicyclic and is substituted with 3 to 5 substituents, and $R^{10}$ is hydrogen or lower alkyl.

In another embodiment of the compounds of Formula ID, or a polymorph, solvate, ester, tautomer, enantiomer, pharmaceutically acceptable salt or prodrug thereof, $R^1$ is halogen; $R^4$ is $-CH_2-$; $R^5$ is aryl or heteroaryl, wherein each of said aryl and heteroaryl is monocyclic or bicyclic and is substituted with 3 to 5 substituents; and $R^{10}$ is hydrogen.

In another embodiment of the compounds of Formula ID, or a polymorph, solvate, ester, tautomer, enantiomer, pharmaceutically acceptable salt or prodrug thereof, $R^1$ is halogen; $R^3$ is hydrogen; $R^4$ is $-CH_2-$; $R^5$ is aryl or heteroaryl, wherein each of said aryl and heteroaryl is monocyclic or bicyclic and is substituted with 3 to 5 substituents; and $R^{10}$ is hydrogen.

In another embodiment of the compounds of Formula ID, or a polymorph, solvate, ester, tautomer, enantiomer, pharmaceutically acceptable salt or prodrug thereof, wherein $R^1$ is chloro or bromo, $R^5$ is phenyl, pyridyl or 1-oxy-pyridyl (N-oxy-pyridyl), each of which has at least two substituents.

Another embodiment of the invention is a compounds represented by Formula IE:

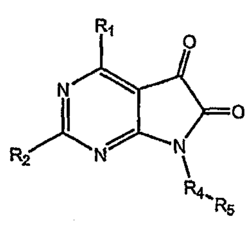
IE

or a polymorph, solvate, ester, tautomer, enantiomer, pharmaceutically acceptable salt or prodrug thereof, wherein:

$R^1$ is halogen, $-OR^{11}$, $-SR^{11}$ or lower alkyl;
$R^2$ is $-NH_2$;
$R^3$ is selected from the group consisting of hydrogen, halogen, $-SR^8$, $-OR^8$, -CN,$-C(O)R^9$, -C(O)OH, $-NO_2$, $-NR^8R^{10}$, lower alkyl, lower alkenyl, lower alkynyl, lower perhaloalkyl, aryl, heteroaryl, alicyclic, heterocyclic, all optionally substituted, wherein:

the aryl, heteroaryl, alicyclic and heterocyclic groups are optionally mono-, bi- or tri-cyclic,
$R^8$ and $R^{10}$ taken together optionally form a ring of 3-7 ring atoms and optionally 1-3 of the ring atoms are heteroatoms selected from the group of O, S and N, and
the optional substituents on $R^3$ are selected from the group consisting of halogen, lower alkyl, lower alkenyl, lower alkynyl, $-SR^8$, $-OR^8$,-CN, -C(O)OH,
$-C(O)R^9$, $-NO_2$, $-NR^8R^{10}$, lower aryl, heteroaryl, alicyclic, lower heterocyclic, arylalkyl, heteroarylalkyl, amino, alkylamino, dialkylamino, diarylakylamino, oxo, oxa, perhaloalkyl, perhaloalkoxy, perhaloacyl, guanidine, pyridinyl, thiophene, furanyl, indole, indazole, phosphonates, phosphates, phosphoramides, sulfonates, sulfones, sulfates, sulphonamides, carbamates, ureas, thioureas and thioamides, wherein $R^8$ and $R^{10}$ taken together optionally form a ring of 3-7 ring atoms and optionally 1-3 of the ring atoms are heteroatoms selected from the group of O, S and N;

$R^4$ is $-CHR^{12}$-, -C(O)-, -C(S)-, -S(O)- or $-SO_2$-;
$R^5$ is aryl, heteroaryl, alicyclic, or heterocyclic, wherein
the aryl group is substituted with 3 to 5 substituents,
the heteroaryl group is substituted with 2 to 5 substituents,
the alicyclic group is substituted with 3 to 5 substituents,
the heterocyclic group is substituted with 3 to 5 substituents, and
the substituents on $R^5$ are selected from the group consisting of halogen, lower alkyl, lower alkenyl, lower alkynyl, $-SR^8$, $-OR^8$, -CN,-C(O)OH, $-C(O)R^9$,
$-NO_2$ and $-NR^8R^{10}$, lower aryl, heteroaryl, alicyclic, lower heterocyclic, arylalkyl, heteroarylalkyl, amino, alkylamino, dialkylamino, diarylalkylamino, oxo, oxa, perhaloalkyl, perhaloalkoxy, perhaloacyl, guanidine, pyridinyl, thiophene, furanyl, indole, indazole, phosphonates, phosphates, phosphoramides, sulfonates, sulfones, sulfates, sulphonamides, carbamates, ureas, thioureas and thioamides, wherein $R^8$ and $R^{10}$ taken together optionally form a ring of 3-7 ring atoms and optionally 1-3 of the ring atoms are heteroatoms selected from the group of O, S and N;
$R^8$ is hydrogen, lower alkyl, lower alkenyl, lower alkynyl, lower aryl, lower heteroaryl, or $-C(O)R^9$;
$R^9$ is H, lower alkyl, lower aryl, lower heteroaryl, $-NR^{10}R^{10}$, or $-OR^{11}$, wherein $R^{10}$ and $R^{10}$ taken together optionally form a ring of 3-7 ring atoms and optionally 1-3 of the ring atoms are heteroatoms selected from the group of O, S and N;
$R^{10}$ is hydrogen, lower alkyl, lower heteroaryl, lower aryl, lower alkenyl, or lower alkynyl,
$R^{11}$ is lower alkyl, lower alkenyl, lower alkynyl, lower heteroaryl or lower aryl;
$R^{12}$ is hydrogen or lower alkyl; and
$R^3$ and $R^{10}$ taken together optionally form an exocyclic double bond which is optionally substituted, or optionally form a ring of 3-7 ring atoms and optionally 1-3 of the ring atoms are heteroatoms selected from the group of O, S and N.

In one embodiment of the compounds of Formula IE, or a polymorph, solvate, ester, tautomer, enantiomer, pharmaceutically acceptable salt or prodrug thereof, $R^1$ is halogen; $R^4$ is
$-CH_2$-; $R^5$ is aryl or heteroaryl, wherein each of said aryl and heteroaryl is monocyclic or bicyclic and is substituted with 3 to 5 substituents.

In another embodiment of the compounds of Formula IE, or a polymorph, solvate, ester, tautomer, enantiomer, pharmaceutically acceptable salt or prodrug thereof, $R^1$ is chloro or bromo, $R^5$ is phenyl, pyridyl or 1-oxy-pyridyl (N-oxy-pyridyl), each of which has at least two substituents.

It should be understood that any of the foregoing embodiments can be combined where feasible and appropriate.

Illustrative species of the compounds of Formula IB are described in TABLE 1. Prodrugs which can be employed with the compound of the invention include, but are not limited to, those listed in the Definition section above.

### B. Compounds of Formula II

In one aspect, the invention provides compounds of Formula II:

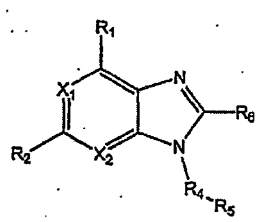

II

or a polymorph, solvate, ester, tautomer, diastercomer, enantiomer, pharmaceutically acceptable salt or prodrug thereof, wherein:

$X_1$ and $X_2$ are the same or different and each is nitrogen or $-CR_6$;
$R_1$ is halogen, $-OR_8$, $-SR_8$, or lower alkyl;
$R_2$ is $-NR_8R_{10}$;
$R_4$ is $-(CH_2)_n-$ wherein = 0-3, -C(O), -C(S), $-SO_2-$, or $-SO_2N-$;
$R_6$ is hydrogen, halogen, lower alkyl, $-SR_8$, $-OR_8$, $-NR_8R_{10}$, $-N_3$, -CN, or - $C(O)R_9$;
$R_5$ is alkyl, aryl, heteroaryl, alicyclic, or heterocyclic, all optionally bi- or tri-cyclic, and all optionally substituted with H, halogen, lower alkyl, $-SR_8$,$-OR_8$, -CN, - $CO_2R_9$ $-NO_2$, or $-NR_8R_{10}$;
$R_8$ is hydrogen, lower alkyl, lower aryl, or $-(CO)R_9$;
$R_9$ is lower alkyl, lower aryl, lower heteroaryl, $-NR_8R_{10}$ or $-OR_{11}$;
$R_{10}$ is hydrogen or lower alkyl,
$R_{11}$ is lower alkyl or lower aryl.

In one embodiment of the compounds of Formula II, or tautomer, pharmaceutically acceptable salt, or prodrug thereof, $R_1$ is halogen, hydroxyl, lower alkoxy, lower thioalkyl, or $C_{1-4}$ alkyl; and $R^2$ is $NH_2$.
In another embodiment of the compounds of Formula II, or tautomer, pharmaceutically acceptable salt, or prodrug thereof, $R^4$ is$-(CH_2)_n-$, where n = 0-3.
In another embodiment of the compounds of Formula II, or tautomer, pharmaceutically acceptable salt, or prodrug thereof, $R_1$ is halogen, hydroxyl, lower alkoxy, lower thioalkyl, or $C_{1-4}$ alkyl; and $R^2$ is $NH_2$; $R^4$ is$-(CH_2)_n-$, wherein n = 0-3.
In another embodiment of the compounds of Formula II, or tautomer, pharmaceutically acceptable salt thereof, $R_1$ is halogen; $R^2$ is $NH_2$, $R^4$ is $-CH_2-$.
In another embodiment of the compounds of Formula II, or tautomer, pharmaceutically acceptable salt, or prodrug thereof, $R^4$ is $-(CH_2)-$; $R^5$ is phenyl, benzyl, or pyridyl, and is independently optionally substituted with H, halogen, lower alkyl, - $SR^8$, $-OR^8$ (or cyclic ethers such as methylenedioxy), -CN, $-CO_2R^9$, $-NO_2$, or - $NR^8R^{10}$; $R^8$ is hydrogen, lower alkyl, lower aryl or $-(CO)R^9$; $R^9$ is lower alkyl, lower aryl, lower heteroaryl, $-NR^8R^{10}$ or $-OR^{11}$; $R^{11}$ is lower alkyl or lower aryl; and $R^{10}$ is hydrogen or lower alkyl.
In another embodiment of the compounds of Formula II, or tautomer, pharmaceutically acceptable salt, or prodrug thereof, $R^1$ is halogen, $R^2$ is $-NH_2$, $R^4$ is - $CH_2-$, $R^6$ is H or halogen, $R^5$ is phenyl optionally substituted with H, halogen, $C_{1-4}$ alkyl, $C_{1-4}$ alkoxy, $C_{1-4}$ alkylthio, perhaloalkyl, perhaloalkyloxy, -CN, $-NO_2$, $-NH_2$ or $-CO_2R^1$.
In another embodiment of the compounds of Formula II, or tautomer, pharmaceutically acceptable salt, or prodrug thereof, $R^1$ is halogen, $R^2$ is $-NH_2$, $R^4$ is - $CH_2-$, $R^6$ is H, wherein $R^5$ is 2-halo-3,5dimethoxyphenyl optionally substituted with H, halogen, $C_{1-4}$ alkyl, $C_{1-4}$ alkoxy, $C_{1-4}$ alkylthio, perhaloalkyl, perhaloalkyloxy, -CN, $-NO_2$, $NH_2$, or $-CO_2R^{11}$ at the para (4-) position.
In another embodiment of the compounds of Formula II, or tautomer, pharmaceutically acceptable salt, or prodrug thereof, $R^1$ is chloro, $R^2$ is $-NH_2$, $R^4$ is - $CH_2-$, $R^6$ is H and $R^5$ is 2-chloro-3,4,5-trimethoxyphenyl.
In another embodiment of the compounds of Formula II, or tautomer, pharmaceutically acceptable salt, or prodrug thereof, $R^1$ is chloro, $R^2$ is $-NH_2$, $R^4$ is - $CH_2-$, $R^6$ is H and $R^5$ is 2-bromo-3,4,5-trimethoxyphenyl.
In another embodiment of the compounds of Formula II, or tautomer, pharmaceutically acceptable salt, or prodrug thereof, $R_1$ is chloro, $R^2$ is $-NH_2$, $R^4$ is $-CH_2-$, $R^6$ is H and $R^5$ is 2-iodo-3,4,5-trimethoxyphenyl.
In another embodiment of the compounds of Formula II, or tautomer, pharmaceutically acceptable salt, or prodrug thereof, $R_1$ is chloro, $R^2$ is $-NH_2$, $R^4$ is $-CH_2-$, $R^6$ is H and $R^5$ is 2-fluoro-3,4,5-trimethoxyphenyl.
In another embodiment of the compounds of Formula II, or tautomer, pharmaceutically acceptable salt, or prodrug thereof, $R_1$ is bromo, $R^2$ is $-NH_2$, $R^4$ is $-CH_2-$, $R^6$ is H, and $R^5$ is 2-bromo-3,4,5-trimethoxyphenyl.
In another embodiment of the compounds of Formula II, or tautomer, pharmaceutically acceptable salt, or prodrug thereof, $R_1$ is bromo, $R^2$ is $-NH_2$, $R^4$ is $-CH_2-$, $R^6$ is H, and $R^5$ is 2-iodo-3,4,5-trimethoxyphenyl.
In another embodiment of the compounds of Formula II, or tautomer, pharmaceutically acceptable salt, or prodrug thereof, $R_1$ is bromo, $R^2$ is $-NH_2$, $R^4$ is $-CH_2-$, $R^6$ is H and $R^5$ is 2-iodo-3,4,5-trimethoxyphenyl.
In another embodiment of the compounds of Formula II, or tautomer, pharmaceutically acceptable salt, or prodrug thereof, $R^1$ is bromo, $R^2$ is $-NH_2$, $R^4$ is $-CH_2-$, $R^6$ is H and $R^5$ is 2-fluoro-3,4,5-trimethoxyphenyl.
In another embodiment of the compounds of Formula II, or tautomer, pharmaceutically acceptable salt, or prodrug thereof, $R^1$ is chloro, $R^2$ is $-NH_2$, $R^4$ is $-CH_2-$, $R^6$ is halo and $R^5$ is 2-chloro-3,4,5-trimethoxyphenyl.
In another embodiment of the compounds of Formula II, or tautomer, pharmaceutically acceptable salt, or prodrug thereof, $R^1$ is chloro, $R^2$ is $NH_2$, $R^4$ is $-CH_2-$, $R^6$ is halo, and $R^5$ is 2-bromo-3,4,5-trimethoxyphenyl.
In another embodiment of the compounds of Formula II, or tautomer, pharmaceutically acceptable salt, or prodrug thereof, $R^1$ is bromo, $R^2$ is $-NH_2$, $R^4$ is $-CH_2-$, $R^6$ is halo and $R^5$ is 2-chloro-3,4,5-trimethoxyphenyl.

In another embodiment of the compounds of Formula II, or tautomer, pharmaceutically acceptable salt, or prodrug thereof, $R^1$ is bromo, $R^2$ is $-NH_2$, $R^4$ is $-CH_2-$, $R^6$ is halo and $R^5$ is 2-bromo-3,4,5-trimethoxyphenyl.

In one embodiment, the invention provides compounds of Formula IIA:

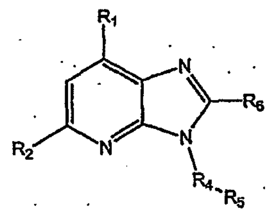

IIA

or a polymorph, solvate, ester, tautomer, pharmaceutically acceptable salt or prodrug thereof, wherein:

$R_1$ is halogen, $-OR_8$, $-SR_8$ or lower alkyl;
$R_2$ is $-NR_8R_{10}$;
$R_4$ is $-(CH_2)_n-$ where n = 0-3, -C(O), -C(S), $-SO_2-$ or $-SO_2N-$;
$R_6$ is hydrogen, halogen, lower alkyl, $-SR_8$, $-OR_8$, $-NR_8R_{10}$, $-N_3$, -CN or $C(O)R_9$;
$R_5$ is alkyl, aromatic, heteroaromatic, alicyclic, heterocyclic, all optionally bi- or tricyclic, and all optionally substituted with H, halogen, lower alkyl, $-SR_8$, $-OR_8$, -CN,
$-CO_2R_9$ $-NO_2$ or $-NR_8R_{10}$;
$R_8$ is hydrogen, lower alkyl, lower aryl, or $-(CO)R_9$;
$R_9$ is lower alkyl, lower aryl, lower heteroaryl, $-NR_8R_{10}$ or $OR_{11}$;
$R_{10}$ is hydrogen or lower alkyl; and
$R_{11}$ is lower alkyl or lower aryl.

In one embodiment of the compounds of Formula IIA, or a tautomer, pharmaceutically acceptable salt, or prodrug thereof, $R_1$ is halogen, hydroxyl, lower alkoxy, lower thioalkyl, or $C_{1-4}$ alkyl; and $R^2$ is $NH_2$.

In another embodiment of the compounds of Formula IIA, or a tautomer, pharmaceutically acceptable salt, or prodrug thereof, $R^4$ is-$(CH_2)_n$-, where n = 0-3.

In another embodiment of the compounds of Formula IIA, or a tautomer, pharmaceutically acceptable salt, or prodrug thereof, $R_1$ is halogen, hydroxyl, lower alkoxy, lower thioalkyl, or $C_{1-4}$ alkyl; and $R^2$ is $NH_2$; $R^4$ is-$(CH_2)_n$-, wherein n = 0-3.

In another embodiment of the compounds of Formula IIA, or a tautomer, pharmaceutically acceptable salt thereof, $R_1$ is halogen; $R^2$ is $NH_2$, $R^4$ is $-CH_2-$.

In one embodiment, the invention provides compounds of Formula IIB:

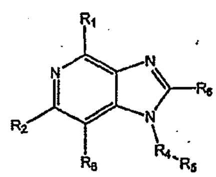

VII IIB

wherein:

$R_1$ is halogen, $-OR_8$, $-SR_8$ or lower alkyl;
$R_2$ is $-NR_8R_{10}$;
$R_4$ is $-(CH_2)_n-$, where n = 0-3, -C(O), -C(S), $-SO_2-$ or $-SO_2N-$;
$R_6$ is hydrogen, halogen, lower alkyl, $-SR_8$, $-OR_8$, $-NR_8R_{10}$, $-N_3$, -CN or $-C(O)R_9$;
$R_5$ is alkyl, aryl, heteroaromatic, alicyclic, heterocyclic, all optionally bi- or tricyclic, and all optionally substituted with H, halogen, lower alkyl, $-SR_8$, $-OR_8$, -CN, - $CO_2R_9$,
$-NO_2$, or $-NR_8R_{10}$;
$R_8$ is hydrogen, lower alkyl, lower aryl, or $-(CO)R_9$;
$R_9$ is lower alkyl, lower aryl, lower heteroaryl, $-NR_8R_{10}$ or $-OR_{11}$;

$R_{10}$ is hydrogen or lower alkyl,
$R_{11}$ is lower alkyl or lower aryl.

In one embodiment of the compounds of Formula IIB, or a tautomer, pharmaceutically acceptable salt, or prodrug thereof, $R^1$ is halogen, hydroxyl, lower alkoxy, lower thioalkyl, or $C_{1-4}$ alkyl; and $R^2$ is $-NH_2$.

In another embodiment of the compounds of Formula IIB, or tautomer, pharmaceutically acceptable salt thereof, or prodrug thereof, $R^4$ is-$(CH_2)_n$-, where n = 0-3.

In another embodiment of the compounds of Formula IIB, or a tautomer, pharmaceutically acceptable salt thereof, or prodrug thereof, $R^1$ is halogen, hydroxyl, lower alkoxy, lower thioalkyl, or $C_{1-4}$ alkyl; and $R^2$ is $NH_2$; $R^4$ is-$(CH_2)_n$-, wherein n = 0-3.

In another embodiment of the compounds of Formula IIB, or a tautomer, pharmaceutically acceptable salt, or prodrug thereof, $R_1$ is halogen; $R^2$ is $-NH_2$; and $R^4$ is $-CH_2-$.

In one embodiment, the invention provides compounds of Formula IIC:

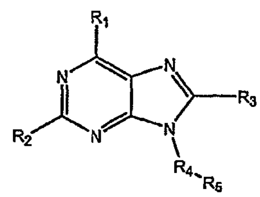

IIC

or a polymorph, solvate, ester, tautomer, pharmaceutically acceptable salt or prodrug thereof, wherein:

$R^1$ is halogen or lower alkyl;
$R^2$ is $-NR^8R^{10}$;
$R^4$ is $-CHR^{12}-$;
$R^3$ is hydrogen, halogen, or -CN;
$R^5$ is aryl, heteroaryl, alicyclic, or heterocyclic, wherein

the aryl group is substituted with 3 to 5 substituents,
the heteroaryl group is substituted with 2 to 5 substituents, wherein when the heteroaryl is substituted with only two substituents, the two substituents must form part of an optionally substituted fused ring,
the alicyclic group is substituted with 3 to 5 substituents,
the heterocyclic group is substituted with 3 to 5 substituents, and
the subsituents are selected from the group of halogen, lower alkyl, lower alkenyl, lower alkynyl; lower aryl, lower alicyclic, arylalkyl, aryloxy, axyloxyalkyl, alkoxyalkyl, perhaloalkyl, perhaloalkyloxy, perhaloacyl, $-N_3$, $-SR^8$, $-OR^8$, -CN, $-C(O)R^9$, $-NO_2$ $-NR^8R^{10}$, phosphonate and phosphonic acid;

$R^8$ is hydrogen, lower alkyl, lower aryl, or $-C(O)R^9$;
$R^9$ is lower alkyl, lower aryl, lower heteroaryl, $-NW^{10}R^{10}$ or $-OR^{11}$;
$R^{10}$ is independently hydrogen or lower alkyl;
$R^{11}$ is lower alkyl, lower aryl or lower heteroaryl;
$R^{12}$ is hydrogen or lower alkyl;
provided that
when $R^5$ is aryl, $R^5$ is not an organo-metallic cyclopentadiene;
when $R^5$ is phenyl, the substituents are not 3,5 di-halo;
when $R^5$ is alicyclic, the ring system does not contain any tetra-substituted $sp^3$ ring carbons;
when $R^5$ is heterocyclic, the ring system does not contain any tetra-substituted $sp^3$ ring carbons or the ring system is not a tetra-substituted pyrrolidine.

In another embodiment, the invention provides compounds of Formula IID, which are compounds of Formula IIC where $R_4$ is $-CH_2-$,

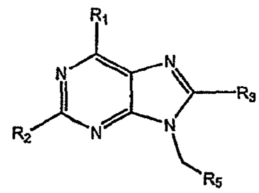

IID

or a polymorph, solvate, ester, tautomer, enantiomer, diastereomer, pharmaceutically acceptable salt or prodrug thereof, wherein:

$R^1$ is halogen or lower alkyl;
$R^2$ is $-NR^8R^{10}$;
$R^3$ is hydrogen, halogen, or -CN;
$R^5$ is aryl, heteroaryl, alicyclic, or heterocyclic, wherein

the aryl group is substituted with 3 to 5 substituents,
the heteroaryl group is substituted with 2 to 5 substituents, wherein when the heteroaryl is substituted with only two substituents, the two substituents must form part of an optionally substituted fused ring,
the alicyclic group is substituted with 3 to 5 substituents,
the heterocyclic group is substituted with 3 to 5 substituents, and
the subsituents are selected from the group of halogen, lower alkyl, lower alkenyl, lower alkynyl, lower aryl, lower alicyclic, arylalkyl, aryloxy, aryloxyalkyl, alkoxyalkyl, perhaloalkyl, perhaloalkyloxy, perhaloacyl, $-N_3$, $-SR^8$, $-OR^8$, -CN, $-C(O)R^9$, $-NO_2$, $-NR^8R^{10}$, phosphonate and phosphonic acid;

$R^8$ is hydrogen, lower alkyl, lower aryl, or $-C(O)R^9$;
$R^9$ is lower alkyl, lower aryl, lower heteroaryl, $-NR^{10}R^{10}$ or $-OR^{11}$;
$R^{10}$ is independently hydrogen or lower alkyl;
$R^{11}$ is lower alkyl, lower aryl or lower heteroaryl;
provided that
when $R^5$ is aryl, $R^5$ is not an organo-metallic cyclopentadiene;
when $R^5$ is phenyl, the substituents are not 3,5 di-halo;
when $R^5$ is alicyclic, the ring system does not contain any tetra-substituted $sp^3$ ring carbons;
when $R^5$ is heterocyclic, the ring system does not contain any tetra-substituted $sp^3$ ring carbons or the ring system is not a tetra-substituted pyrrolidine.

In one embodiment of the compounds of Formula IID, or a polymorph, solvate, ester, tautomer, pharmaceutically acceptable salt or prodrug thereof, $R^1$ is halogen or methyl; and $R^2$ is
$-NHR^8$, where $R^8$ is hydrogen or $-C(O)R^9$.
In another embodiment of the compounds of Formula IID, or a polymorph, solvate, ester, tautomer, pharmaceutically acceptable salt or prodrug thereof, $R^1$ is halogen.
In another embodiment of the compounds of Formula IID, or a polymorph, solvate, ester, tautomer, pharmaceutically acceptable salt or prodrug thereof, $R^2$ is $-NH_2$ and $R^3$ is hydrogen.
In another embodiment of the compounds of Formula IID, or a polymorph, solvate, ester, tautomer, pharmaceutically acceptable salt or prodrug thereof, $R^1$ is halogen; $R^2$ is $-NH_2$; $R^3$ is hydrogen; $R^5$ is aryl or heteroaryl, wherein each of said aryl and heteroaryl groups is monocyclic or bicyclic, the aryl group is substituted with 4 to 5 substituents, the heteroaryl group is substituted with 2 to 5 substituents, wherein when the heteroaryl is substituted with only two substituents, the two substituents must form part of an optionally substituted fused ring.
In another embodiment of the compounds of Formula IID, or a polymorph, solvate, ester, tautomer, pharmaceutically acceptable salt or prodrug thereof, $R^1$ is chloro or bromo, $R^2$ is $-NH_2$, and $R^5$ is a phenyl having 3 to 5 substituents.
In another embodiment of the compounds of Formula IID, or a polymorph, solvate, ester, tautomer, pharmaceutically acceptable salt or prodrug thereof, $R^1$ is chloro or bromo, $R^2$ is $-NH_2$, and $R^5$ is a pyridyl having 3 to 5 substituents.
In another embodiment of the compounds of Formula IID, or a polymorph, solvate, ester, tautomer, pharmaceutically acceptable salt or prodrug thereof, $R^1$ is chloro or bromo, $R^2$ is $-NH_2$, and $R^5$ is an 1-oxy-pyridyl (N-oxy-pyridyl) having 3 to 5 substituents.
In another embodiments of the compounds of Formula IID, or a polymorph, solvate, ester, tautomer, pharmaceutically acceptable salt or prodrug thereof, each of the aryl, heteroaryl, alicyclic or heterocyclic group is monocyclic

or bicyclic.

In another embodiment of the compounds of Formula IID, or a polymorph, solvate, ester, tautomer, enantiomer, pharmaceutically acceptable salt or prodrug thereof, $R^1$ is halogen or methyl; and $R^2$ is $-NHR^8$, where $R^8$ is hydrogen or $-C(O)R^9$,

In another embodiment of the compounds of Formula IID, or a polymorph, solvate, ester, tautomer, enantiomer, pharmaceutically acceptable salt or prodrug thereof, wherein $R^1$ is halogen.

In another embodiment of the compounds of Formula IID, or a polymorph, solvate, ester, tautomer, enantiomer, pharmaceutically acceptable salt or prodrug thereof, wherein $R^2$ is $-NH_2$ and $R^3$ is hydrogen.

In another embodiment of the compounds of Formula IID, or a polymorph, solvate, ester, tautomer, pharmaceutically acceptable salt or prodrug thereof, $R^1$ is halogen; $R^2$ is $-NH_2$; $R^3$ is hydrogen; $R^5$ is aryl or heteroaryl, wherein each of the aryl and heteroaryl groups is monocyclic or bicyclic, the aryl group is substituted with 4 to 5 substituents, the heteroaryl group is substituted with 2 to 5 substituents, wherein when the heteroaryl is substituted with only two substituents, the two substituents must form part of an optionally substituted fused ring.

In another embodiment of the compounds of Formula IID, or a polymorph, solvate, ester, tautomer, pharmaceutically acceptable salt or prodrug thereof, $R^1$ is chloro or bromo, $R^2$ is $-NH_2$, and $R^5$ is a phenyl having 3 to 5 substituents.

In another embodiment of the compounds of Formula IID, or a polymorph, solvate, ester, tautomer, enantiomer, pharmaceutically acceptable salt or prodrug thereof, $R^1$ is chloro or bromo, $R^2$ is $-NH_2$, and $R^5$ is a pyridyl having 3 to 5 substituents.

In another embodiment of the compounds of Formula IID, or a polymorph, solvate, ester, tautomer, enantiomer, pharmaceutically acceptable salt or prodrug thereof,
wherein $R^1$ is chloro or bromo, $R^2$ is $-NH_2$, and $R^5$ is an 1-oxy-pyridyl (N-oxy-pyridyl) having 3 to 5 substituents.

It should be understood that any of the foregoing embodiments can be combined where feasible and appropriate.

In another embodiment, the invention provides compounds, or polymorphs, solvates, esters, tautomers, pharmaceutically acceptable salts or prodrugs thereof, prepared by the process comprising:

reacting a compound of formula Y and a compound of formula Z, wherein:

Y is represented by any one of the following formulae:

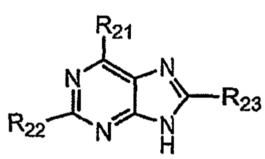 , 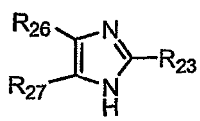 and 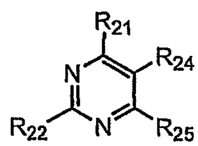 ,

and

Z is $L^1-R^4-R^5$; wherein:

$L^1$ is halogen, $NR^8R^{10}$, triflate, tosylate, or mesylate;

$R^4$ is $-CHR^{12}-$,

$R^5$ is aryl, heteroaryl, alicyclic, or heterocyclic, wherein:

the aryl group is substituted with 3 to 5 substituents,

the heteroaryl group is substituted with 2 to 5 substituents,

wherein when the heteroaryl is substituted with only two substituents, the two substituents must form part of an optionally substituted fused ring,

the alicyclic group is substituted with 3 to 5 substituents,

the heterocyclic group is substituted with 3 to 5 substituents, and

the substituents are selected from the group of halogen, lower alkyl, lower alkenyl, lower alkynyl, lower aryl, lower alicyclic, arylalkyl, aryloxy, aryloxyalkyl, alkoxyalkyl, perhaloalkyl, perhaloalkyloxy, perhaloacyl, $-N_3$, $-SR^8$, $-OR^8$, -CN, $-C(O)R^9$, $-NO_2$, $-NR^8R^{10}$, phosphonate and phosphonic acid;

$R^8$ is hydrogen, lower alkyl, lower aryl, or $-C(O)R^9$;

$R^9$ is lower alkyl, lower aryl, lower heteroaryl, $-NR^{10}R^{10}$ or $-OR^{11}$;

$R^{10}$ is independently hydrogen or lower alkyl;

$R^{11}$ is lower alkyl, lower aryl or lower heteroaryl;

$R^{12}$ is independently hydrogen or lower alkyl;
$R^{21}$ is halogen, lower alkyl or -OH;
$R^{22}$ is $-NR^8R^{10}$;
$R^{23}$ is hydrogen, halogen, or -CN;
$R^{24}$ is $-NH_2$, $-NO_2$ or -NO;
$R^{25}$ is halogen or -OH;
$R^{26}$ is $-C(O)NH_2$ or C(O)OEt; and
$R^{27}$ is $-NH_2$, -OH or halogen;

provided that:

when $R^5$ is aryl, $R^5$ is not an organo-metallic cyclopentadiene;
when $R^5$ is phenyl, the substituents are not 3,5 di-halo;
when $R^5$ is alicyclic, the ring system does not contain any tetra-substituted $sp^3$ ring carbons; or
when $R^5$ is heterocyclic, the ring system does not contain any tetra-substituted $sp^3$ ring carbons or the ring system is not a tetra-substituted pyrrolidine.

In one embodiment of the compounds prepared by the process of the invention, or a polymorph, solvate, ester, tautomer, pharmaceutically acceptable salt or prodrug thereof, $R^5$ is aryl, heteroaryl, alicyclic, or heterocyclic, optionally mono- or bicyclic.

In another embodiment of the compounds prepared by the process of the invention, or a polymorph, solvate, ester, tautomer, pharmaceutically acceptable salt or prodrug thereof, $R^4$ is $-CH_2-$.

In another embodiment of the compounds prepared by the process of the invention, or a polymorph, solvate, ester, tautomer, pharmaceutically acceptable salt or prodrug thereof, $L^1$ is -Cl, -Br or $NH_2$; $R^5$ is aryl or hetertoaryl, wherein the aryl group is substituted with 4 to 5 substituents.

In another embodiment of the compounds prepared by the process of the invention, or a polymorph, solvate, ester, tautomer, pharmaceutically acceptable salt or prodrug thereof, Y is a substituted purine.

In another embodiment of the compounds prepared by the process of the invention, or a polymorph, solvate, ester, tautomer, pharmaceutically acceptable salt or prodrug thereof, the reaction is performed in a solvent comprising a member selected from the group of DMF, THF and DMSO.

In another embodiment of the compounds prepared by the process of the invention, or a polymorph, solvate, ester, tautomer, pharmaceutically acceptable salt or prodrug thereof, the reaction is performed in a solvent that comprises DMF.

Compounds for use in the method of treatment are inhibitors of HSP90 of the following Formula IIC, or a polymorph, solvate, ester, tautomer, enantiomer, pharmaceutically acceptable salt or prodrug thereof,

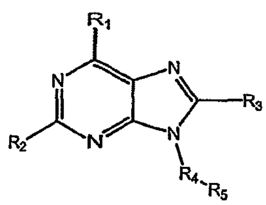

IIC

wherein:

$R^1$ is halogen, $-OR^{11}$, $-SR^{11}$, $-NHR^8$, hydrogen, or lower alkyl;
$R^2$ is $-NR^8R^{10}$;
$R^3$ is hydrogen, halogen, $-N_3$, or -CN;
$R^4$ is $-(CHR^{12})_n-$ where n = 0, 1 or 2, -C(O)-, -C(S)-, or -S(O)-;
$R^5$ is alkyl, aryl, heteroaryl, alicyclic, or heterocyclic, all optionally substituted with halogen, lower alkyl, lower alkenyl, lower alkynyl, lower aryl, lower alicyclic, arylalkyl, aryloxy, aryloxyalkyl, alkoxyalkyl, perhaloalkyl, perhaloalkyloxy, perhaloacyl, $-N_3$, $-SR^8$, $-OR^8$, -CN, $-C(O)R^9$, $-NO_2$, or $-NR^8R^{10}$;
$R^8$ is hydrogen, lower alkyl, lower aryl, or $-C(O)R^9$;
$R^9$ is lower alkyl, lower aryl, lower heteroaryl, $-NR^{10}R^{10}$, or $-OR^{11}$;
$R^{10}$ is independently hydrogen or lower alkyl;
$R^{11}$ is lower alkyl, lower aryl or lower heteroaryl;

$R^{12}$ is hydrogen or lower alkyl;
provided that:

$R^4R^5$ is not a ribose or derivative thereof, or a sugar or derivative thereof;
$-R^4R^5$ is not a phosphonate or phosphonic acid, or substituted with phosphonate or phosphonic acid; and
when $R^4$ is $-(CH_2)_n-$ where n=1 or 2, then $R^4$ and $R^5$ are not connected through an ether linkage.

In one embodiment of the compounds of Formula IIC for the method of the invention, $R^3$ is hydrogen, halogen or -CN; $R^5$ is aryl, heteroaryl, alicyclic, or heterocyclic, all optionally substituted with halogen, lower alkyl, lower alkenyl, lower alkynyl, lower aryl, lower alicyclic, arylalkyl, aryloxy, aryloxyalkyl, alkoxyalkyl, perhaloalkyl, perhaloalkyloxy, perhaloacyl, $-N_3$, $-SR^8$, $-OR^8$, -CN, $-C(O)R^9$, $-NO_2$, - $NR^8R^{10}$, phosphonate, or phosphonic acid.

In another embodiment of the compounds of Formula IIC for the method of the invention, or a polymorph, solvate, ester, tautomer, pharmaceutically acceptable salt or prodrug thereof, $R^1$ is halogen or methyl and $R^2$ is $-NHR^8$, where $R^8$ is hydrogen or $-C(O)R^9$.

In another embodiment of the compounds of Formula IIC for the method of the invention, $R^2$ is $-NH_2$ and $R^3$ is hydrogen.

In another embodiment of the compounds of Formula IIC for the method of the invention, $R^4$ is $-CH_2-$.

In another embodiment of the compounds of Formula IIC for the method of the invention, wherein $R^1$ is halogen; $R^2$ is $-NH_2$; $R^3$ is hydrogen; $R^4$ is $-CH_2-$; $R^5$ is aryl and heteroaryl, the aryl and heteroaryl groups are monocyclic or bicyclic, the aryl group is substituted with 4 to 5 substituents, the heteroaryl group is substituted with 2 to 5 substituents, wherein when the heteroaryl is substituted with two substituents, the two substituents must form part of an optionally substituted fused ring.

In another embodiment of the compounds of Formula IIC for the method of the invention, $R^1$ is chloro or bromo, $R^2$ is $-NH_2$, and $R^5$ is a phenyl having 3 to 5 substituents.

In another embodiment of the compounds of Formula IIC for the method of the invention, $R^1$ is chloro or bromo, $R^2$ is $-NH_2$, and $R^5$ is a pyridyl having 3 to 5 substituents.

In another embodiment of the compounds of Formula IIC for the method of the invention, $R^1$ is chloro or bromo, $R^2$ is $-NH_2$, and $R^5$ is an 1-oxy-pyridyl (N-oxy-pyridyl) having 3 to 5 substituents.

It should be understood that any of the foregoing embodiments can be combined where feasible and appropriate.

Compounds of the invention that are based on the following Formula IIC have illustrative species as described in TABLE II. Suitable prodrugs which can be employed by the compounds include, but are not limited to, those listed in the Definition section.

C. COMPOUNDS OF FORMULA III

In another aspect, the invention provides compounds of Formula III:

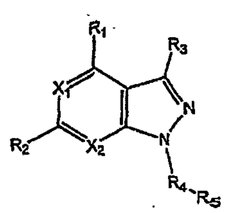
III

or a polymorph, solvate, ester, tautomer, enantiomer, pharmaceutically acceptable salt or prodrug thereof, wherein

$X_1$ and $X_2$ are the same or different and each is nitrogen or $CR_6$;
$R_1$ is halogen, $-OR_8$, $-SR_8$ or lower alkyl;
$R_2$ is $-NR_8R_{10}$;
$R_3$ is hydrogen, -OH or keto tautomer, $-OR_8$, halogen, -CN, lower alkyl or - $C(O)R_9$;
$R_4$ is $-(CH_2)_n$ where n = 0-3, -C(O), -C(S), $-SO_2$ or $-SO_2N-$;
$R_5$ is alkyl, aryl, heteroaryl, alicyclic, heterocyclic, all optionally bi- or tricyclic, and all optionally substituted with H, halogen, lower alkyl, $-SR_8$, $-OR_8$, - CN, $-CO_2R_9$, $-NO_2$ or $-NR_8R_{10}$;
$R_8$ is hydrogen, lower alkyl, lower aryl or $-(CO)R_9$;
$R_9$ is lower alkyl, lower aryl, lower heteroaryl, $-NR_8R_{10}$ or $OR_{11}$;
$R_{11}$ is lower alkyl or lower aryl; and

$R_{10}$ is hydrogen or lower alkyl.

In one embodiment of the compounds of Formula III, or a tautomer, pharmaceutically acceptable salt, or prodrug thereof, $R_1$ is halogen, hydroxyl, lower alkoxy, lower thioalkyl, or $C_{1-4}$ alkyl; and $R^2$ is $NH_2$.

In another embodiment of the compounds of Formula III, or a tautomer, pharmaceutically acceptable salt, or prodrug thereof, $R^4$ is-$(CH_2)_n$-, where n = 0-3.

In another embodiment of the compounds of Formula III, or a tautomer, pharmaceutically acceptable salt, or prodrug thereof, $R_1$ is halogen, hydroxyl, lower alkoxy, lower thioalkyl, or $C_{1-4}$ alkyl; and $R^2$ is $NH_2$; and $R^4$ is-$(CH_2)_n$-, wherein n = 0-3.

In another embodiment of the compounds of Formula III, or a tautomer, pharmaceutically acceptable salt thereof, $R_1$ is halogen; $R^2$ is $NH_2$, and $R^4$ is -$CH_2$-.

One embodiment of the compounds based on Formula III are compounds of Formula IIIA:

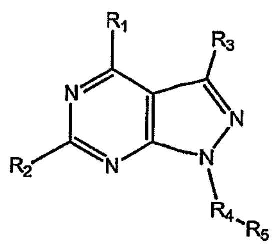

IIIA

or a polymorph, solvate, ester, tautomer, diasteremer, enantiomer, pharmaceutically acceptable salt or prodrug thereof, wherein:

$R^1$ is halogen, -$OR^{11}$ -$SR^{11}$ or lower alkyl;
$R^2$ is -$NHR^8$;
$R^3$ is selected from the group consisting of hydrogen, halogen, -$SR^8$, -$OR^8$, -CN, -C(O)$R^9$, -$CO_2$H, -$NO_2$, -$NR^8R^{10}$, lower alkyl, lower alkenyl, lower alkynyl, lower perhaloalkyl, aryl, heteroaryl, acyclic and heterocyclic, all optionally substituted, wherein:

the aryl, heteroaryl, alicyclic and heterocyclic groups are optionally mono-, bi- or tri-cyclic;
$R^8$ and $R^{10}$ taken together optionally form a ring of 3-7 ring atoms and optionally 1-2 of the ring atoms are heteroatoms selected from the group of O, S and N, and the optional substituents on $R^3$ are selected from the group consisting of halogen, lower alkyl, lower alkenyl, lower alkynyl, -$SR^8$, -$OR^8$, -CN, -C(O)$R^9$, -C(O)OH, -$NO_2$ and -$NR^8R^{10}$, lower aryl, lower heteroaryl, lower alicyclic, lower heterocyclic, arylalkyl, heteroarylalkyl, amino, alkylamino, dialkylamino, diarylalkylamino, oxo, oxa, perhaloalkyl, perhaloalkoxy, perhaloacyl, guanidine, pyridinyl, thiophene, furanyl, indole, indazole, phosphonates, phosphates, phosphoramides, sulfonates, sulfones, sulfates, sulphonamides, arbamates, ureas, thioureas, and thioamides, wherein $R^8$ and $R^{10}$ taken together optionally form a ring of 3-7 ring atoms and optionally 1-3 of the ring atoms are heteroatoms selected from the group of O, S and N;

$R^4$ is -$CHR^{12}$-, -C(O), -C(S), -S(O)-, or -$SO_2$-;
$R^5$ is aryl, heteroaryl, alicyclic, or heterocyclic, wherein
the aryl group is substituted with 3 to 5 substituents,
the heteroaryl group is substituted with 2 to 5 substituents,
the alicyclic group is substituted with 3 to 5 substituents,
the heterocyclic group is substituted with 3 to 5 substituents, and
the substituents on $R^5$ are selected from the group consisting of halogen, lower alkyl, lower alkenyl, lower alkynyl, -$SR^8$, -$OR^8$, -CN, -C(O)OH, -C(O)$R^9$, -$NO_2$ and -$NR^8R^{10}$, lower aryl, heteroaryl, alicyclic, lower heterocyclic, arylalkyl, heteroarylalkyl, amino, alkylamino, dialkylamino, diarylalkylamino, oxo, oxa, perhaloalkyl, perhaloalkoxy, perhaloacyl, guanidine, pyridinyl, thiophene, furanyl, indole, indazole, phosphonates, phosphates, phosphoramides, sulfonates, sulfones, sulfates, sulphonamides, carbamates, ureas, thioureas, and thioamides, wherein $R^8$ and $R^{10}$ taken together optionally form a ring of 3-7 ring atoms and optionally 1-3 of the ring atoms are heteroatoms selected from the group of O, S and N;
$R^8$ is hydrogen, lower alkyl, lower alkenyl, lower alkynyl, lower aryl, lower heteroaryl or -C(O)$R^9$;

$R^9$ is hydrogen, lower alkyl, lower alkenyl, lower alkynyl, lower aryl, lower heteroaryl, $-NR^{10}R^{10}$ or $-OR^{11}$ $R^{10}$ and $R^{10}$ taken together optionally form a ring of 3-7 ring atoms and optionally 1-3 of the ring atoms are heteroatoms selected from the group of O, S and N;

$R^{10}$ is hydrogen, lower alkyl, lower alkenyl, lower alkynyl, lower aryl or lower heteroaryl;

$R^{11}$ is lower alkyl, lower alkenyl, lower alkynyl, lower aryl or lower heteroaryl;

and

$R^{12}$ is hydrogen or lower alkyl.

In one embodiment of the compounds of Formula IIIA, or a polymorph, solvate, ester, tautomer, enantiomer, pharmaceutically acceptable salt or prodrug thereof, $R^1$ is halogen or lower alkyl; $R^2$ is $-NHR^8$, where $R^8$ is hydrogen or $-C(O)R^9$; $R^5$ is aryl or heteroaryl, each of said aryl and heteroaryl groups is monocyclic or bicyclic.

In another embodiment of the compounds of Formula IIIA, or a polymorph, solvate, ester, tautomer, enantiomer, pharmaceutically acceptable salt or prodrug thereof, $R^2$ is $-NH_2$; $R^3$ is selected from hydrogen, halogen, $-SR^8$, $-OR^8$, -CN, $-NR^8R^{10}$, lower alkyl, lower alkenyl, lower alkynyl, lower perhaloalkyl, lower aryl, lower heteroaryl, lower alicyclic, lower heterocyclic, $R^8$ is hydrogen, lower alkyl, lower alkenyl, lower alkynyl, lower aryl or lower heteroaryl, and $R^8$ and $R^{10}$ taken together optionally form a ring of 3-7 ring atoms and optionally 1-3 of the ring atoms are heteroatoms selected from the group of O, S and N; and $R^5$ is aryl or heteroaryl, each of said aryl and heteroaryl groups is monocyclic or bicyclic.

In another embodiment of the compounds of Formula IIIA, or a polymorph, solvate, ester, tautomer, enantiomer, pharmaceutically acceptable salt or prodrug thereof, $R^1$ is halogen or lower alkyl; $R^2$ is $-NH_2$; $R^4$ is $-(CH_2)-$; $R^5$ is aryl, heteroaryl, alicyclic or heterocyclic, each of said aryl, heteroaryl alicyclic or heterocyclic groups is monocyclic or bicyclic.

In another embodiment of the compounds of Formula IIIA, or a polymorph, solvate, ester, tautomer, enantiomer, pharmaceutically acceptable salt or prodrug thereof, $R^1$ is halogen; $R^2$ is $-NH_2$; $R^3$ is hydrogen, halogen, $-SR^8$, $-OR^8$, lower alkyl, lower aryl, lower heteroaryl, or $-NR^8R^{10}$, $R^8$ and $R^{10}$ taken together optionally form a ring of 3-7 ring atoms and optionally 1-3 of the ring atoms are heteroatoms selected from the group of O, S and N; $R^4$ is $-CH_2-$; $R^5$ is aryl or heteroaryl, wherein each of said aryl and heteroaryl groups is monocyclic or bicyclic.

In another embodiment of the compounds of Formula IIIA, or a polymorph, solvate, ester, tautomer, enantiomer, pharmaceutically acceptable salt or prodrug thereof, $R^1$ is chloro or bromo, and $R^5$ is a phenyl having 3 to 5 substituents.

In another embodiment of the compounds of Formula IIIA, or a polymorph, solvate, ester, tautomer, enantiomer, pharmaceutically acceptable salt or prodrug thereof, $R^1$ is chloro or bromo, and $R^5$ is a pyridyl having 3 to 5 substituents.

In another embodiment of the compounds of Formula IIIA, or a polymorph, solvate, ester, tautomer, enantiomer, pharmaceutically acceptable salt or prodrug thereof, $R^1$ is chloro or bromo, and $R^5$ is an 1-oxy-pyridyl (N-oxy-pyridyl) having 3 to 5 substituents.

It should be understood that any of the foregoing embodiments can be combined where feasible and appropriate.

Other embodiments of compounds based on Formula III are compounds of Formula IIIB:

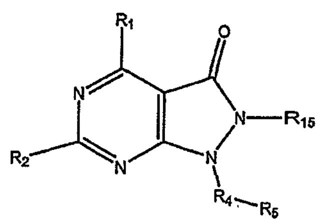

IIIB

or a polymorph, solvate, ester, tautomer, pharmaceutically acceptable salt or prodrug thereof, wherein:

$R^1$ is halogen, $-OR^{11}$, $-SR^{11}$ or lower alkyl;

$R^2$ is $-NHR^8$;

$R^4$ is $-CHR^{12}-$, -C(O), -C(S), -S(O)-, or $-SO_2-$;

$R^5$ is aryl, heteroaryl, alicyclic, or heterocyclic, wherein
the aryl group is substituted with 3 to 5 substituents,
the heteroaryl group is substituted with 2 to 5 substituents,
the alicyclic group is substituted with 3 to 5 substituents,
the heterocyclic group is substituted with 3 to 5 substituents, and
the substituents on $R^5$ are selected from the group consisting of halogen, lower alkyl, lower alkenyl, lower alkynyl, $-SR^8$, $-OR^8$, -CN, -C(O)OH, $-C(O)R^9$, $-NO_2$ and $-NR^8R^{10}$, lower aryl, heteroaryl, alicyclic, lower heterocyclic, arylalkyl, heteroarylalkyl, amino, alkylamino, dialkylamino, diarylalkylamino, oxo, oxa, perhaloalkyl, perhaloalkoxy, perhaloacyl, guanidine, pyridinyl, thiophene, furanyl, indole, indazole, phosphonates, phosphates, phosphoramides, sulfonates, sulfones, sulfates, sulphonamides, carbamates, ureas, thioureas, and hioamides, wherein $R^8$ and $R^{10}$ taken together optionally form a ring of 3-7 ring atoms and optionally 1-3 of the ring atoms are heteroatoms selected from the group of O, S and N;
$R^8$ is hydrogen, lower alkyl, lower alkenyl, lower alkynyl, lower aryl, lower heteroaryl or $-C(O)R^9$;
$R^9$ is hydrogen, lower alkyl, lower alkenyl, lower alkynyl, lower aryl, lower heteroaryl, $-NR^{10}R^{10}$ or $-OR^{11}$, $R^{10}$ and $R^{10}$ taken together optionally form a ring of 3-7 ring atoms and optionally 1-3 of the ring atoms are heteroatoms selected from the group of O, S and N;
$R^{10}$ is hydrogen, lower alkyl, lower alkenyl, lower alkynyl, lower aryl or lower heteroaryl;
$R^{11}$ is lower alkyl, lower alkenyl, lower alkynyl, lower aryl or lower heteroaryl;
and
$R^{12}$ is hydrogen or lower alkyl; and
$R^{15}$ is hydrogen, lower alkyl, lower alkenyl or lower alknyl.

In one embodiment of the compounds of Formula IIIB, or a polymorph, solvate, ester, tautomer, pharmaceutically acceptable salt or prodrug thereof, $R^2$ is $-NHR^8$, where $R^8$ is hydrogen or $-C(O)R^9$; $R^5$ is aryl, heteroaryl, alicyclic, or heterocyclic, all optionally mono-, bi- or tri-cyclic; and $R^9$ is lower alkyl, lower alkenyl, lower alkynl, lower aryl or lower heteroaryl.

In another embodiment of the compounds of Formula IIIB, or a polymorph, solvate, ester, tautomer, pharmaceutically acceptable salt or prodrug thereof, $R^1$ is halogen or lower alkyl; $R^2$ is $-NHR^8$, where $R^8$ is hydrogen or $-C(O)R^9$; $R^4$ is $-CH_2-$, $R^5$ is aryl, heteroaryl, alicyclic, or heterocyclic, all optionally mono-, bi- or tri-cyclic.

In another embodiment of the compounds of Formula IIIB, or a polymorph, solvate, ester, tautomer, enantiomer, pharmaceutically acceptable salt or prodrug thereof, wherein $R^1$ is chloro or bromo, $R^2$ is $-NH_2$, $R^5$ is a phenyl having 3 to 5 substituents.

In another embodiment of the compounds of Formula IIIB, or a polymorph, solvate, ester, tautomer, enantiomer, pharmaceutically acceptable salt or prodrug thereof, wherein $R^1$ is chloro or bromo, $R^2$ is $-NH_2$, $R^5$ is a pyridyl having 3 to 5 substituents.

In another embodiment of the compounds of Formula IIIB, or a polymorph, solvate, ester, tautomer, enantiomer, pharmaceutically acceptable salt or prodrug thereof,
wherein $R^1$ is chloro or bromo, $R^2$ is $-NH_2$, $R^5$ is an 1-oxy-pyridyl (N-oxy-pyridyl) having 3 to 5 substituents.

It should be understood that any of the foregoing embodiments can be combined where feasible and appropriate.

Other embodiments of the compounds of the invention based on Formula III are the compounds, or polymorphs, solvates, esters, tautomers, pharmaceutically acceptable salts or prodrugs thereof, prepared by the process comprising:

reacting a compound of Formula Y and a compound of Formula Z, wherein:
Y is represented by any one of the following formulae:

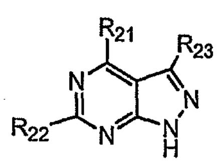 , 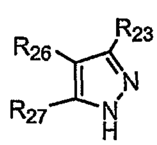 and 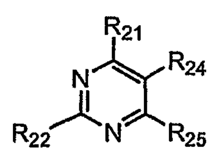 , and

Z is $L^1-R^4-R^5$; wherein:

$L^1$ is halogen, $-NR^8R^{10}$, triflate, tosylate, or mesylate;
$R^4$ is $-(CHR^{12})-$, $-C(O)$, $-C(S)$, $-S(O)-$, or $-SO_2-$;
$R^5$ is aryl, heteroaryl, alicyclic, or heterocyclic, wherein
the aryl group is substituted with 3 to 5 substituents,
the heteroaryl group is substituted with 2 to 5 substituents,
the alicyclic group is substituted with 3 to 5 substituents,
the heterocyclic group is substituted with 3 to 5 substituents, and
the substituents on $R^5$ are selected from the group consisting of halogen, lower alkyl, lower alkenyl, lower alkynyl, $-SR^8$, $-OR^8$, $-CN$, $-C(O)OH$, $-C(O)R^9$, $-NO_2$ and $-NR^8R^{10}$, lower aryl, heteroaryl, alicyclic, lower heterocyclic, arylalkyl, heteroarylalkyl, amino, alkylamino, dialkylamino, diarylalkylamino, oxo, oxa, perhaloalkyl, perhaloalkoxy, perhaloacyl, guanidine, pyridinyl, thiophene, furanyl, indole, indazole, phosphonates, phosphates, phosphoramides, sulfonates, sulfones, sulfates, sulphonamides, carbamates, ureas, thioureas, and thioamides, wherein $R^8$ and $R^{10}$ taken together optionally form a ring of 3-7 ring atoms and optionally 1-3 of the ring atoms are heteroatoms selected from the group of O, S and N;

$R^8$ is hydrogen, lower alkyl, lower alkenyl, lower alkynyl, lower aryl, lower heteroaryl or $-C(O)R^9$;

$R^9$ is hydrogen, lower alkyl, lower alkenyl, lower alkynyl, lower aryl, lower heteroaryl, $-NR^{10}R^{10}$ or $-OR^{11}$, $R^{10}$ and $R^{10}$ taken together optionally form a ring of 3-7 ring atoms and optionally 1-3 of the ring atoms are heteroatoms selected from the group of O, S and N;

$R^{10}$ is hydrogen, lower alkyl, lower alkenyl, lower alkynyl, lower aryl or lower heteroaryl;

$R^{11}$ is lower alkyl, lower alkenyl, lower alkynyl, lower aryl or lower heteroaryl;

$R^{12}$ is hydrogen or lower alkyl;

$R^{21}$ is halogen, $-OR^8$, $-SR^8$ or lower alkyl;

$R^{22}$ is $-NR^8R^{10}$;

$R^{23}$ is hydrogen, -OH or its keto tautomer, $-OR^8$, halogen, -CN, lower alkyl, lower aryl or $-C(O)R^9$;

$R^{24}$ is -CHO, $-NH_2$, $-NO_2$ or -NO;

$R^{25}$ is halogen or -OH;

$R^{26}$ is $-C(O)NH_2$ or C(O)OEt; and

$R^{27}$ is $-NH_2$, -OH or halogen.

In one embodiment of the compounds prepared by the process of the invention, or a polymorph, solvate, ester, tautomer, pharmaceutically acceptable salt or prodrug thereof, $R^5$ is aryl, heteroaryl, alicyclic, or heterocyclic, optionally mono- or bicyclic.

In another embodiment of the compounds of the invention which are prepared by the process of the invention, or a polymorph, solvate, ester, tautomer, pharmaceutically acceptable salt or prodrug thereof, $L^1$ is -Cl, -Br or $-NH_2$; $R^4$ is $-CH_2-$; and $R^5$ is aryl or heteroaryl.

In another embodiment of the compounds of the invention which are prepared by the process of the invention, or a polymorph, solvate, ester, tautomer, pharmaceutically acceptable salt or prodrug thereof, Y is a pyrazolopyrimidine.

In another embodiment of the compounds of the invention which are prepared by the process of the invention, or a polymorph, solvate, ester, tautomer, pharmaceutically acceptable salt or prodrug thereof, said reaction is performed in a solvent comprising a member selected from the group of DMF, THF and DMSO.

In another embodiment of the compounds of the invention which are prepared by the process of the invention, or a polymorph, solvate, ester, tautomer, pharmaceutically acceptable salt or prodrug thereof, the said reaction is performed in a solvent that comprises DMF.

It should be understood that any of the foregoing embodiments can be combined where feasible and appropriate.

Illustrative species of the compounds of the invention that are based on Formula IIIA, where $R^2 = -NH_2$ are described in TABLE III. Prodrugs which can be employed by those compounds include, but are not limited to, those listed in the Definition section.

### D. Compounds of Formula IV

In another aspect, the invention provides compounds of Formula IV:

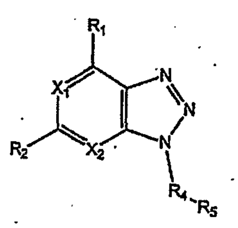

or a polymorph, solvate, ester, tautomer, diasteremer, enantiomer, pharmaceutically acceptable salt or prodrug thereof, wherein:

$X_1$ and $X_2$ are the same or different and each is nitrogen or $CR_6$;

$R_1$ is halogen, $-OR_8$, $-SR_8$, or lower alkyl;

$R_2$ is $-NR_8R_{10}$;

$R_4$ is $-CH_{2n},-$ where n = 0-3, -C(O), -C(S), $-SO_2-$, or $-SO_2N-$;

$R_5$ is alkyl, aryl, heteroaryl, alicyclic, or heterocyclic, all optionally bi-or tricyclic, and all optionally substituted with H, halogen, lower alkyl, $-SR_8$, $-OR_8$, -CN,
$-CO_2R_9$, $-NO_2$, or $-NR_8R_{10}$;
$R_8$ is hydrogen, lower alkyl, lower aryl or $-(CO)R_9$;
$R_9$ is lower alkyl, lower aryl, lower heteroaryl, $-NR_8R_{10}$ or $-OR_{11}$; $R_{11}$ is lower alkyl or lower aryl; and
$R_{10}$ is hydrogen or lower alkyl.

In one embodiment of the compounds of Formula IV, a tautomer, pharmaceutically acceptable salt thereof, or prodrug thereof, $R^1$ is halogen, hydroxyl, lower alkoxy, lower thioalkyl, or $C_{1-4}$ alkyl; and $R^2$ is $-NH_2$.

In one embodiment of the compounds of Formula IV, a tautomer, pharmaceutically acceptable salt thereof, or prodrug thereof, $R^1$ is halogen, hydroxyl, lower alkoxy, lower thioalkyl, or $C_{1-4}$ alkyl; and $R^2$ is $-NH_2$; R4 is $-CH_2-$, -C(O), -C(S), - $SO_2$-.

In one embodiment of the compounds of Formula IV, a tautomer, pharmaceutically acceptable salt thereof, or prodrug thereof, $R^1$ is halogen or $C_{1-4}$alkyl; and $R^2$ is $NH_2$, $R_4$ is $-CH_2-$.

In one embodiment of the compounds of Formula IV, a tautomer, pharmaceutically acceptable salt thereof, or prodrug thereof, $R^1$ is halogen, hydroxyl, lower alkoxy, lower thioalkyl, or $C_{1-4}$ alkyl; and $R^2$ is $NH_2$, R4 is $-(CH_2)_n-$, where n = 0-3.

In one embodiment of the compounds of Formula IV, a tautomer, pharmaceutically acceptable salt, or prodrug thereof, $R^4$ is -C(O) or $-CH_2-$; $R^1$ is halogen, lower alkoxy or $C_{1-4}$ alkyl; and $R^2$ is $NH_2$.

In another embodiment of compounds based on Formula IV are compounds of Formula IVA:

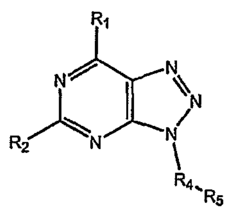

IVA

or a polymorph, solvate, ester, tautomer, enantiomer, diastereomer, pharmaceutically acceptable salt or prodrug thereof, wherein:

$R^1$ is halogen, $-OR^{11}$, $-SR^{11}$ or lower alkyl;
$R^2$ is $-NHR^8$;
$R^4$ is $-CHR^{12}-$, -C(O)-, -C(S)-, -S(O)- or $-SO_2-$;
$R^5$ is aryl, heteroaryl, alicyclic, or heterocyclic, wherein:

the aryl group is substituted with 3 to 5 substituents,
the heteroaryl group is substituted with 2 to 5 substituents,
the alicyclic group is substituted with 3 to 5 substituents,
the heterocyclic group is substituted with 3 to 5 substituents, and
the substituents are selected from the group consisting of halogen, lower alkyl, lower alkenyl, lower alkynyl, $-SR^8$, $-OR^8$, -CN, -C(O)OH, - $C(O)R^9$, $-NO_2$ and $-NR^8R^{10}$, lower aryl, heteroaryl, alicyclic, lower heterocyclic, arylalkyl, heteroarylalkyl, amino, alkylamino, dialkylamino, diarylalkylamino, oxo, oxa, perhaloalkyl, perhaloalkoxy, perhaloacyl, guanidine, pyridinyl, thiophene, furanyl, indole, indazole, phosphonates, phosphates, phosphoramides, sulfonates, sulfones, sulfates, sulphonamides, carbamates, ureas, thioureas, and thioamides, wherein $R^8$ and $R^{10}$ taken together optionally form a ring of 3-7 ring atoms and optionally 1-3 of the ring atoms are heteroatoms selected from the group of O, S and N;

$R^8$ is hydrogen, lower alkyl, lower alkenyl, lower alkynyl, lower aryl, lower heteroaryl, or
$-C(O)R^9$;
$R^9$ is H, lower alkyl, lower alkenyl, or lower alkynyl, lower aryl, lower heteroaryl,
$-NR^{10}R^{10}$, or $-OR^{11}$, wherein $R^{10}$ and $R^{10}$ taken together optionally form a ring of 3-7 ring atoms and optionally 1-3 of the ring atoms are heteroatoms selected from the group of O, S and N;
$R^{10}$ is hydrogen, lower alkyl, lower heteroaryl, lower aryl, lower alkenyl, or lower alkynyl,
$R^{11}$ is lower alkyl, lower alkenyl, or lower alkynyl, lower heteroaryl or lower aryl; and $R_4$ is $-CHR^{12}-$, -C(O)-, -C(S)-, -S(O)- or $-SO_2-$; and

$R^{12}$ is hydrogen or lower alkyl;
provided that when $R^5$ is alicyclic, the ring system does not contain any tetra-substituted $sp^3$ ring carbons.

In one embodiment of the compounds of Formula IVA, or a polymorph, solvate, ester, tautomer, enantiomer, diastereomer, pharmaceutically acceptable salt or prodrug thereof, each of the aryl, heteroaryl, alicyclic or heterocyclic group is monocyclic or bicyclic.

In another embodiment of the compounds of Formula IVA, or a polymorph, solvate, ester, tautomer, enantiomer, diastereomer, pharmaceutically acceptable salt or prodrug thereof, $R^1$ is halogen; and $R^2$ is $-NHR^8$, where $R^8$ is hydrogen or $-C(O)R^9$.

In another embodiment of the compounds of Formula IVA, or a polymorph, solvate, ester, tautomer, enantiomer, pharmaceutically acceptable salt or prodrug thereof, $R^1$ is chloro or bromo, $R^2$ is $-NHR^8$, where $R^8$ is hydrogen or $-C(O)R^9$; and $R^4$ is lower alkyl.

In another embodiment of the compounds of Formula IVA, or a polymorph, solvate, ester, tautomer, enantiomer, pharmaceutically acceptable salt or prodrug thereof, $R^2$ is $-NHR^8$, where $R^8$ is hydrogen or $-C(O)R^9$; and $R^4$ is $-CH_2-$.

In another embodiment of the compounds of Formula IVA, or a polymorph, solvate, ester, tautomer, enantiomer, pharmaceutically acceptable salt or prodrug thereof, $R^1$ is halogen; $R^2$ is $-NH_2$, $R^4$ is $-CH_2-$; and $R^5$ is aryl or heteroaryl, wherein each of the aryl and heteroaryl is monocyclic or bicyclic and is substituted with 3 to 5 substituents.

In another embodiment of the compounds of Formula IVA, or a polymorph, solvate, ester, tautomer, enantiomer, pharmaceutically acceptable salt or prodrug thereof, $R^1$ is chloro or bromo, $R^2$ is $-NH_2$, and $R^5$ is a phenyl having at least three substituent.

In another embodiment of the compounds of Formula IVA, or a polymorph, solvate, ester, tautomer, enantiomer, pharmaceutically acceptable salt or prodrug thereof, $R^1$ is chloro or bromo, $R^2$ is $-NH_2$ and $R^5$ is a pyridyl having at least two substituent.

In another embodiment of the compounds of Formula IVA, or a polymorph, solvate, ester, tautomer, enantiomer, pharmaceutically acceptable salt or prodrug thereof, $R^1$ is chloro or bromo, $R^2$ is $-NH_2$, and $R^5$ is 1-oxy-pyridyl (N-oxy-pyridyl) having at least two substituent.

It should be understood that any of the foregoing embodiments can be combined where feasible and appropriate.

In another embodiment, the invention provides compounds, or polymorphs, solvates, esters, tautomers, pharmaceutically acceptable salts or prodrugs thereof, prepared by the process comprising:

reacting a compound of formula Y and a compound of formula Z, wherein:
Y is a represented by any one of the following formulae:

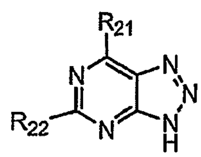 , 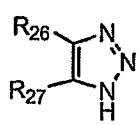 and 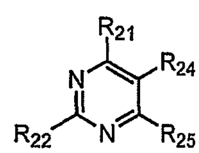 ,

Z is $L^1-R^4-R^5$; wherein:

$L^1$ is halogen, $-NR^8R^{10}$, triflate, tosylate, or mesylate;
$R^4$ is $-CHR^{12}-$, $-C(O)-$, $-C(S)-$, $-S(O)-$ or $-SO_2-$;
$R^5$ is aryl, heteroaryl, alicyclic, or heterocyclic, wherein:

the aryl group is substituted with 3 to 5 substituents,
the heteroaryl group is substituted with 2 to 5 substituents,
the alicyclic group is substituted with 3 to 5 substituents,
the heterocyclic group is substituted with 3 to 5 substituents, and
the substituents are selected from the group consisting of halogen, lower alkyl, lower alkenyl, lower alkynyl, $-SR^8$, $-OR^8$, -CN, -C(O)OH, $-C(O)R^9$, $-NO_2$ and $-NR^8R^{10}$, lower aryl, heteroaryl, alicyclic, lower heterocyclic, arylalkyl, heteroarylalkyl, amino, alkylamino, dialkylamino, diarylalkylamino, oxo, oxa, perhaloalkyl, perhaloalkoxy, perhaloacyl, guanidine, pyridinyl, thiophene, furanyl, indole, indazole, phosphonates, phosphates, phosphoramides, sulfonates, sulfones, sulfates, sulphonamides, carbamates, ureas, thioureas, thioamides, wherein $R^8$ and $R^{10}$ taken together optionally form a ring of 3-7 ring atoms and optionally 1-3 of the ring atoms are heteroatoms selected from the group of O, S and N;

$R^8$ is hydrogen, lower alkyl, lower alkenyl, or lower alkynyl, lower aryl, lower heteroaryl, or -C(O)$R^9$;
$R^9$ is H, lower alkyl, lower alkenyl, or lower alkynyl, lower aryl, lower heteroaryl, -$NR^{10}R^{10}$, or -$OR^{11}$, wherein $R^{10}$ and $R^{10}$ taken together optionally form a ring of 3-7 ring atoms and optionally 1-3 of the ring atoms are heteroatoms selected from the group of O, S and N;
$R^{10}$ is hydrogen, lower alkyl, lower heteroaryl, lower aryl, lower alkenyl, or lower alkynyl,
$R^{11}$ is lower alkyl, lower alkenyl, or lower alkynyl, lower heteroaryl or lower aryl;
$R^{12}$ is hydrogen or lower alkyl;
$R^{21}$ is halogen, -$OR^8$, -$SR^8$ or lower alkyl;
$R^{22}$ is -$NR^8R^{10}$;
$R^{24}$ is -$NH_2$, -$NO_2$ or -NO;
$R^{25}$ is halogen or -OH;
$R^{26}$ is -C(O)$NH_2$ or C(O)OEt; and
$R^{27}$ is $NH_2$, -OH or halogen;
provided that when $R^5$ is alicyclic, the ring system does not contain any tetra-substituted $sp^3$ ring carbons.

In one embodiment of the compounds prepared by the process of the invention, or a polymorph, solvate, ester, tautomer, pharmaceutically acceptable salt or prodrug thereof, $L^1$ is -Cl, -Br or -$NH_2$; $R^5$ is aryl or heteroaryl.

In another embodiment of the compounds prepared by the process of the invention, or a polymorph, solvate, ester, tautomer, pharmaceutically acceptable salt or prodrug thereof, $R^4$ is -$CH_2$-.

In another embodiment of the compounds prepared by the process of the invention, or a polymorph, solvate, ester, tautomer, pharmaceutically acceptable salt or prodrug thereof, $R^5$ is aryl, heteroaryl, alicyclic, or heterocyclic, optionally mono- or bicyclic.

In another embodiment of the compounds prepared by the process of the invention, or a polymorph, solvate, ester, tautomer, pharmaceutically acceptable salt or prodrug thereof, $L_1$ is -Cl, -Br or -$NH_2$; $R^4$ is -$CH_2$-; and $R^5$ is aryl or heteroaryl.

In another embodiment of the compounds prepared by the process of the invention, or a polymorph, solvate, ester, tautomer, pharmaceutically acceptable salt or prodrug thereof, Y is a triazolopyrimidine.

In another embodiment of the compounds prepared by the process of the invention, or a polymorph, solvate, ester, tautomer, pharmaceutically acceptable salt or prodrug thereof, Y is a triazole.

In another embodiment of the compounds prepared by the process of the invention, or a polymorph, solvate, ester, tautomer, pharmaceutically acceptable salt or prodrug thereof, Y is a pyrimidine.

In another embodiment of the compounds prepared by the process of the invention, or a polymorph, solvate, ester, tautomer, pharmaceutically acceptable salt or prodrug thereof, the reaction is performed in a solvent comprising a member selected from the group of DMF, THF and DMSO.

In another embodiment of the compounds prepared by the process of the invention, or a polymorph, solvate, ester, tautomer, pharmaceutically acceptable salt or prodrug thereof, the reaction is performed in a solvent that comprises DMF.

It should be understood that any of the foregoing embodiments can be combined where feasible and appropriate.

Illustrative species of the compounds of the invention that are based on Formula IVA are described in TABLE 4. Prodrugs which can be employed by these compounds include, but are not limited to, those listed in the Definition section.

**TABLE I: Exemplary Compounds based on Formula IA**

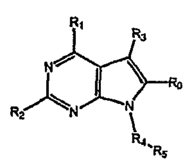

IA

| No. | Ex | $R^1$ | $R^2$ | $R^3$ | $R^4$ | $R^5$ | $R^0$ |
|---|---|---|---|---|---|---|---|
| 1 | 9 | Cl | $NH_2$ | H | $CH_2$ | 3,4,5-Trimethoxyphenyl | H |
| 2 | | Cl | $NH_2$ | H | $CH_2$ | 2-Chloro-3,4,5-trimethoxyphenyl | H |
| 3 | 6 | Cl | $NH_2$ | H | $CH_2$ | 2-Bromo-3,4,5-trimethoxyphenyl | H |

(continued)

| No. | Ex | $R^1$ | $R^2$ | $R^3$ | $R^4$ | $R^5$ | $R^0$ |
|---|---|---|---|---|---|---|---|
| 4 | 7 | Cl | $NH_2$ | H | $CH_2$ | 2-Iodo-3,4,5-trimethoxyphenyl | H |
| 5 | | Cl | $NH_2$ | H | $CH_2$ | 2-Fluoro-3,4,5-trimethoxyphenyl | H |
| 6 | | Cl | $NH_2$ | H | $CH_2$ | 3,4,5-Trimethylphenyl | H |
| 7 | | Cl | $NH_2$ | H | $CH_2$ | 2-Chloro-3,4,5-trimethylphenyl | H |
| 8 | | Cl | $NH_2$ | H | $CH_2$ | 2-Bromo-3,4,5-trimethylphenyl | H |
| 9 | | Cl | $NH_2$ | H | $CH_2$ | 2-lodo-3,4,5-trimethylphenyl | H |
| 10 | | Cl | $NH_2$ | H | $CH_2$ | 2-Fluoro-3,4,5-trimethylphenyl | H |
| 11 | | Cl | $NH_2$ | H | $CH_2$ | 3,5-Dimethoxy-4-methylphenyl | H |
| 12 | | Cl | $NH_2$ | H | $CH_2$ | 2-Chloro-3,5-dimethoxy-4-methylphenyl | H |
| 13 | | Cl | $NH_2$ | H | $CH_2$ | 2-Bromo-3,5-dimethoxy-4-methylphenyl | H |
| 14 | | Cl | $NH_2$ | H | $CH_2$ | 2-Iodo-3,5-dimethoxy-4-methylphenyl | H |
| 15 | | Cl | $NH_2$ | H | $CH_2$ | 2-Fluoro-3,5-dimethoxy-4-methylphenyl | H |
| 16 | | Cl | $NH_2$ | i-pr | $CH_2$ | 3,4,5-Trimethoxyphenyl | H |
| 17 | | Cl | $NH_2$ | i-pr | $CH_2$ | 2-Chloro-3,4,5-trimethoxyphenyl | H |
| 18 | | Cl | $NH_2$ | i-pr | $CH_2$ | 2-Bromo-3,4,5-trimethoxyphenyl | H |
| 19 | | Cl | $NH_2$ | i-pr | $CH_2$ | 2-Iodo-3,4,5-trimethoxyphenyl | H. |
| 20 | | Cl | $NH_2$ | i-pr | $CH_2$ | 2-Fluoro-3,4,5-trimethoxyphenyl | H |
| 21 | | Cl | $NH_2$ | i-pr | $CH_2$ | 3,4,5-Trimethylphenyl | H |
| 22 | | Cl | $NH_2$ | i-pr | $CH_2$ | 2-Chloro-3,4,5-trimethylphenyl | H |
| 23 | | Cl | $NH_2$ | i-pr | $CH_2$ | 2-Bromo-3,4,5-trimethylphenyl | H |
| 24 | | Cl | $NH_2$ | i-pr | $CH_2$ | 2-Iodo-3,4,5-trimethylphenyl | H |
| 25 | | Cl | $NH_2$ | i-pr | $CH_2$ | 2-Fluoro-3,4,5-trimethylphenyl | H |
| 26 | | Cl | $NH_2$ | i-pr | $CH_2$ | 3,5-Dimethoxy-4-methylphenyl | H |
| 27 | | Cl | $NH_2$ | i-pr | $CH_2$ | 2-Chloro-3,5-dimethoxy-4-methylphenyl | H |
| 28 | | Cl | $NH_2$ | i-pr | $CH_2$ | 2-Bromo-3,5-dimethoxy-4-methylphenyl | H |
| 29 | | Cl | $NH_2$ | i-pr | $CH_2$ | 2-Iodo-3,5-dimethoxy-4-methylphenyl | H |
| 30 | | Cl | $NH_2$ | i-pr | $CH_2$ | 2-Fluoro-3,5-dimethoxy-4-methylphenyl | H |
| 31 | | Cl | $NH_2$ | Et | $CH_2$ | 3,4,5-Trimethoxyphenyl | H |
| 32 | | Cl | $NH_2$ | Et | $CH_2$ | 2-Chloro-3,4,5-trimethoxyphenyl | H |
| 33 | | Cl | $NH_2$ | Et | $CH_2$ | 2-Bromo-3,4,5-trimethoxyphenyl | H |
| 34 | | Cl | $NH_2$ | Et | $CH_2$ | 2-Iodo-3,4,5-trimethoxyphenyl | H |
| 35 | | Cl | $NH_2$ | Et | $CH_2$ | 2-Fluoro-3,4,5-trimethoxyphenyl | H |
| 36 | | Cl | $NH_2$ | Et | $CH_2$ | 3,4,5-Trimethylphenyl | H |
| 37 | | Cl | $NH_2$ | Et | $CH_2$ | 2-Chloro-3,4,5-trimethylphenyl | H |
| 38 | | Cl | $NH_2$ | Et | $CH_2$ | 2-Bromo-3,4,5-trimethylphenyl | H |
| 39 | | Cl | $NH_2$ | Et | $CH_2$ | 2-Iodo-3,4,5-trimethylphenyl | H |
| 40 | | Cl | $NH_2$ | Et | $CH_2$ | 2,Fluoro-3,4,5-trimethylphenyl | H |
| 41 | | Cl | $NH_2$ | Et | $CH_2$ | 3,5-Dimethoxy-4-methylphenyl | H |

(continued)

| No. | Ex | $R^1$ | $R^2$ | $R^3$ | $R^4$ | $R^5$ | $R^0$ |
|---|---|---|---|---|---|---|---|
| 42 | | Cl | $NH_2$ | Et | $CH_2$ | 2-Chloro-3,5-dimethoxy-4-methylphenyl | H |
| 43 | | Cl | $NH_2$ | Et | $CH_2$ | 2-Bromo-3,5-dimethoxy-4-methylphenyl | H |
| 44 | | Cl | $NH_2$ | Et | $CH_2$ | 2-Iodo-3,5-dimethoxy-4-methylphenyl | H |
| 45 | | Cl | $NH_2$ | Et | $CH_2$ | 2-Fluoro-3,5-dimethoxy-4-methylphenyl | H |
| 46 | | Cl | $NH_2$ | Me | $CH_2$ | 3,4,5-Trimethoxyphenyl | H |
| 47 | | Cl | $NH_2$ | Me | $CH_2$ | 2-Chloro-3,4,5-trimethoxyphenyl | H |
| 48 | | Cl | $NH_2$ | Me | $CH_2$ | 2-Bromo-3,4,5-trimethoxyphenyl | H |
| 49 | | Cl | $NH_2$ | Me | $CH_2$ | 2-Iodo-3,4,5-trimethoxyphenyl | H |
| 50 | | Cl | $NH_2$ | Me | $CH_2$ | 2-Fluoro-3,4,5-trimethoxyphenyl | H |
| 51 | | Cl | $NH_2$ | Me | $CH_2$ | 3,4,5-Trimethylphenyl | H |
| 52 | | Cl | $NH_2$ | Me | $CH_2$ | 2-Chloro-3,4,5-trimethylphenyl | H |
| 53 | | Cl | $NH_2$ | Me | $CH_2$ | 2-Bromo-3,4,5-trimethylphenyl | H |
| 54 | | Cl | $NH_2$ | Me | $CH_2$ | 2-Iodo-3,4,5-trimethylphenyl | H |
| 55 | | Cl | $NH_2$ | Me | $CH_2$ | 2-Fluoro-3,4,5-trimethylphenyl | H |
| 56 | | Cl | $NH_2$ | Me | $CH_2$ | 3,5-Dimethoxy-4-methylphenyl | H |
| 57 | | Cl | $NH_2$ | Me | $CH_2$ | 2-Chloro-3,5-dimethoxy-4-methylphenyl | H |
| 58 | | Cl | $NH_2$ | Me | $CH_2$ | 2-Bromo-3,5-dimethoxy-4-methylphenyl | H |
| 59 | | Cl | $NH_2$ | Me | $CH_2$ | 2-Iodo-3,5-dimethoxy-4-methylphenyl | H |
| 60 | | Cl | $NH_2$ | Me | $CH_2$ | 2-Fluoro-3,5-dimethoxy-4-methylphenyl | H |
| 61 | | Cl | $NH_2$ | Ph | $CH_2$ | 3,4,5-Trimethoxyphenyl | H |
| 62 | | Cl | $NH_2$ | Ph | $CH_2$ | 2-Chloro-3,4,5-trimethoxyphenyl | H |
| 63 | | Cl | $NH_2$ | Ph | $CH_2$ | 2-Bromo-3,4,5-trimethoxyphenyl | H |
| 64 | | Cl | $NH_2$ | Ph | $CH_2$ | 2-Iodo-3,4,5-trimethoxyphenyl | H |
| 65 | | Cl | $NH_2$ | Ph | $CH_2$ | 2-Fluoro-3,4,5-trimethoxyphenyl | H |
| 66 | | Cl | $NH_2$ | Ph | $CH_2$ | 3,4,5-Trimethylphenyl | H |
| 67 | | Cl | $NH_2$ | Ph | $CH_2$ | 2-Chloro-3,4,5-trimethylphenyl | H |
| 68 | | Cl | $NH_2$ | Ph | $CH_2$ | 2-Bromo-3,4,5-trimethylphenyl | H |
| 69 | | Cl | $NH_2$ | Ph | $CH_2$ | 2-Iodo-3,4,5-trimethylphenyl | H |
| 70 | | Cl | $NH_2$ | Ph | $CH_2$ | 2-Fluoro-3,4,5-trimethylphenyl | H |
| 71 | | Cl | $NH_2$ | Ph | $CH_2$ | 3,5-Dimethoxy-4-methylphenyl | H |
| 72 | | Cl | $NH_2$ | Ph | $CH_2$ | 2-Chloro-3,5-dimethoxy-4-methylphenyl | H |
| 73 | | Cl | $NH_2$ | Ph | $CH_2$ | 2-Bromo-3,5-dimethoxy-4-methylphenyl | H |
| 74 | | Cl | $NH_2$ | Ph | $CH_2$ | 2-lodo-3,5-dimethoxy-4-methylphenyl | H |
| 75 | | Cl | $NH_2$ | Ph | $CH_2$ | 2-Fluoro-3,5-dimethoxy-4-methylphenyl | H |
| 76 | | Cl | $NH_2$ | 2-Py | $CH_2$ | 3,4,5-Trimethoxyphenyl | H |
| 77 | | Cl | $NH_2$ | 2-Py | $CH_2$ | 2-Chloro-3,4,5-trimethoxyphenyl | H |
| 78 | | Cl | $NH_2$ | 2-Py | $CH_2$ | 2-Bromo-3,4,5-trimethoxyphenyl | H |
| 79 | | Cl | $NH_2$ | 2-Py | $CH_2$ | 2-Iodo-3,4,5-trimethoxyphenyl | H |

(continued)

| No. | Ex | $R^1$ | $R^2$ | $R^3$ | $R^4$ | $R^5$ | $R^0$ |
|---|---|---|---|---|---|---|---|
| 80 | | Cl | $NH_2$ | 2-Py | $CH_2$ | 2-Fluoro-3,4,5-trimethoxyphenyl | H |
| 81 | | Cl | $NH_2$ | 2-Py | $CH_2$ | 3,4,5-Trimethylphenyl | H |
| 82 | | Cl | $NH_2$ | 2-Py | $CH_2$ | 2-Chloro-3,4,5-trimethylphenyl | H |
| 83 | | Cl | $NH_2$ | 2-Py | $CH_2$ | 2-Bromo-3,4,5-trimethylphenyl | H |
| 84 | | Cl | $NH_2$ | 2-Py | $CH_2$ | 2-Iodo-3,4,5-trimethylphenyl | H |
| 85 | | Cl | $NH_2$ | 2-Py | $CH_2$ | 2-Fluoro-3,4,5-trimethylphenyl | H |
| 86 | | Cl | $NH_2$ | 2-Py | $CH_2$ | 3,5-Dimethoxy-4-methylphenyl | H |
| 87 | | Cl | $NH_2$ | 2-Py | $CH_2$ | 2-Chloro-3,5-dimethoxy-4-methylphenyl | H |
| 88 | | Cl | $NH_2$ | 2-Py | $CH_2$ | 2-Bromo-3,5-dimethoxy-4-methylphenyl | H |
| 89 | | Cl | $NH_2$ | 2-Py | $CH_2$ | 2-Iodo-3,5-dimethoxy-4-methylphenyl | H |
| 90 | | Cl | $NH_2$ | 2-Py | $CH_2$ | 2-Fluoro-3,5-dimethoxy-4-methylphenyl | H |
| 91 | | Cl | $NH_2$ | 4-Py | $CH_2$ | 3,4,5-Trimethoxyphenyl | H |
| 92 | | Cl | $NH_2$ | 4-Py | $CH_2$ | 2-Chloro-3,4,5-trimethoxyphenyl | H |
| 93 | | Cl | $NH_2$ | 4-Py | $CH_2$ | 2-Bromo-3,4,5-trimethoxyphenyl | H. |
| 94 | | Cl | $NH_2$ | 4-Py | $CH_2$ | 2-Iodo-3,4,5-trimethoxyphenyl | H |
| 95 | | Cl | $NH_2$ | 4-Py | $CH_2$ | 2-Fluoro-3,4,5-trimethoxyphenyl | H |
| 96 | | Cl | $NH_2$ | Ph | $CH_2$ | 3,4,5-Trimethylphenyl | H |
| 97 | | Cl | $NH_2$ | Ph | $CH_2$ | 2-Chloro-3,4,5-trimethylphenyl | H |
| 98 | | Cl | $NH_2$ | Ph | $CH_2$ | 2-Bromo-3,4,5-trimethylphenyl | H |
| 99 | | Cl | $NH_2$ | Ph | $CH_2$ | 2-Iodo-3,4,5-trimethylphenyl | H |
| 100 | | Cl | $NH_2$ | Ph | $CH_2$ | 2-Fluoro-3,4,5-trimethylphenyl | H |
| 101 | | Cl | $NH_2$ | Ph | $CH_2$ | 3,5-Dimethoxy-4-methylphenyl | H |
| 102 | | Cl | $NH_2$ | Ph | $CH_2$ | 2-Chloro-3,5-dimethoxy-4-methylphenyl | H |
| 103 | | Cl | $NH_2$ | Ph | $CH_2$ | 2-Bromo-3,5-dimethoxy-4-methylphenyl | H |
| 104 | | Cl | $NH_2$ | Ph | $CH_2$ | 2-Iodo-3,5-dimethoxy-4-methylphenyl | H |
| 105 | | Cl | $NH_2$ | Pr | $CH_2$ | 2-Fluoro-3,5-dimethoxy-4-methylphenyl | H |
| 106 | | Cl | $NH_2$ | Pr | $CH_2$ | 3,5-Dimethoxy-4-methylphenyl | H. |
| 107 | | Cl | $NH_2$ | Pr | $CH_2$ | 2-Chloro-3,5-dimethoxy-4-methylphenyl | H |
| 108 | | Cl | $NH_2$ | Pr | $CH_2$ | 2-Bromo-3,5-dimethoxy-4-methylphenyl | H |
| 109 | | Cl | $NH_2$ | Pr | $CH_2$ | 2-Iodo-3,5-dimethoxy-4-methylphenyl | H |
| 110 | | Cl | $NH_2$ | Pr | $CH_2$ | 2-Fluoro-3,5-dimethoxy-4-methylphenyl | H |
| 111 | | Cl | $NH_2$ | Pr | $CH_2$ | 3,4,5-Trimethoxyphenyl | H |
| 112 | | Cl | $NH_2$ | Pr | $CH_2$ | 2-Chloro-3,4,5-trimethoxyphenyl | H |
| 113 | | Cl | $NH_2$ | Pr | $CH_2$ | 2-Bromo-3,4,5-trimethoxyphenyl | H |
| 114 | | Cl | $NH_2$ | Pr | $CH_2$ | 2-Iodo-3,4,5-trimethoxyphenyl | H |
| 115 | | Cl | $NH_2$ | Pr | $CH_2$ | 2-Fluoro-3,4,5-trimethoxyphenyl | H |
| 116 | | Cl | $NH_2$ | Pr | $CH_2$ | 3,4,5-Trimethylphenyl | H |
| 117 | | Cl | $NH_2$ | Pr | $CH_2$ | 2-Chloro-3,4,5-trimethylphenyl | H |

(continued)

| No. | Ex | $R^1$ | $R^2$ | $R^3$ | $R^4$ | $R^5$ | $R^0$ |
|---|---|---|---|---|---|---|---|
| 118 | | Cl | $NH_2$ | Pr | $CH_2$ | 2-Bromo-3,4,5-trimethylphenyl | H |
| 119 | | Cl | $NH_2$ | Pr | $CH_2$ | 2-Iodo-3,4,5-trimethylphenyl | H |
| 120 | | Cl | $NH_2$ | Pr | $CH_2$ | 2-Fluoro-3,4,5-trimethylphenyl | H. |
| 121 | | Cl | $NH_2$ | Pr | $CH_2$ | 3,5-Dimethoxy-4-methylphenyl | H |
| 122 | | Cl | $NH_2$ | Pr | $CH_2$ | 2-Chloro-3,5-dimethoxy-4-methylphenyl | H |
| 123 | | Cl | $NH_2$ | Pr | $CH_2$ | 2-Bromo-3,5-dimethoxy-4-methylphenyl | H |
| 124 | | Cl | $NH_2$ | Pr | $CH_2$ | 2-Iodo-3,5-dimethoxy-4-methylphenyl | H |
| 125 | | Cl | $NH_2$ | Pr | $CH_2$ | 2-Fluoro-3,5-dimethoxy-4-methylphenyl | H |
| 126 | | Br | $NH_2$ | H | $CH_2$ | 3,4,5-Trimethoxyphenyl | H |
| 127 | | Br | $NH_2$ | H | $CH_2$ | 2-Chloro-3,4,5-trimethoxyphenyl | H |
| 128 | | Br | $NH_2$ | H | $CH_2$ | 2-Bromo-3,4,5-trimethoxyphenyl | H |
| 129 | | Br | $NH_2$ | H | $CH_2$ | 2-Iodo-3,4,5-trimethoxyphenyl | H |
| 130 | | Br | $NH_2$ | H | $CH_2$ | 2-Fluoro-3,4,5-trimethoxyphenyl | H |
| 131 | | Br | $NH_2$ | H | $CH_2$ | 3,4,5-Trimethylphenyl | H |
| 132 | | Br | $NH_2$ | H | $CH_2$ | 2-Chloro-3,4,5-trimethylphenyl | H |
| 133 | | Br | $NH_2$ | H | $CH_2$ | 2-Bromo-3,4,5-trimethylphenyl | H. |
| 134 | | Br | $NH_2$ | H | $CH_2$ | 2-Iodo-3,4,5-trimethylphenyl | H |
| 135 | | Br | $NH_2$ | H | $CH_2$ | 2-Fluoro-3,4,5-trimethylphenyl | H |
| 136 | | Br | $NH_2$ | H | $CH_2$ | 3,5-Dimethoxy-4-methylphenyl | H |
| 137 | | Br | $NH_2$ | H | $CH_2$ | 2-Chloro-3,5-dimethoxy-4-methylphenyl | H |
| 138 | | Br | $NH_2$ | H | $CH_2$ | 2-Bromo-3,5-dimethoxy-4-methylphenyl | H |
| 139 | | Br | $NH_2$ | H | $CH_2$ | 2-Iodo-3,5-dimethoxy-4-methylphenyl | H |
| 140 | | Br | $NH_2$ | H | $CH_2$ | 2-Fluoro-3,5-dimethoxy-4-methylphenyl | H |
| 141 | | Cl | $NH_2$ | i-Bu | $CH_2$ | 3,4,5-Trimethoxyphenyl | H |
| 142 | | Cl | $NH_2$ | i-Bu | $CH_2$ | 2-Chloro-3,4,5-trimethoxyphenyl | H |
| 143 | | Cl | $NH_2$ | i-Bu | $CH_2$ | 2-Bromo-3,4,5-trimethoxyphenyl | H |
| 144 | | Cl | $NH_2$ | i-Bu | $CH_7$ | 2-Iodo-3,4,5-trimethoxyphenyl | H |
| 145 | | Cl | $NH_2$ | i-Bu | $CH_2$ | 2-Fluoro-3,4,5-trimethoxyphenyl | H |
| 146 | | Cl | $NH_2$ | i-Bu | $CH_2$ | 3,4,5-Trimethylphenyl | H |
| 147 | | Cl | $NH_2$ | i-Bu | $CH_2$ | 2-Chloro-3,4,5-trimethylphenyl | H- |
| 148 | | Cl | $NH_2$ | i-Bu | $CH_2$ | 2-Bromo-3,4,5-trimethylphenyl | H |
| 149 | | Cl | $NH_2$ | i-Bu | $CH_2$ | 2-Iodo-3,4,5-trimethylphenyl | H |
| 150 | | Cl | $NH_2$ | i-Bu | $CH_2$ | 2-Fluoro-3,4,5-trimethylphenyl | H |
| 151 | | Cl | $NH_2$ | i-Bu | $CH_2$ | 3,5-Dimethoxy-4-methylphenyl | H |
| 152 | | Cl | $NH_2$ | i-Bu | $CH_2$ | 2-Chloro-3,5-dimethoxy-4-methylphenyl | H |
| 153 | | Cl | $NH_2$ | i-Bu | $CH_2$ | 2-Bromo-3,5-dimethoxy-4-methylphenyl | H |
| 154 | | Cl | $NH_2$ | i-Bu | $CH_2$ | 2-Iodo-3,5-dimethoxy-4-methylphenyl | H |
| 155 | | Cl | $NH_2$ | i-Bu | $CH_2$ | 2-Fluoro-3,5-dimethoxy-4-methylphenyl | H |

(continued)

| No. | Ex | $R^1$ | $R^2$ | $R^3$ | $R^4$ | $R^5$ | $R^0$ |
|---|---|---|---|---|---|---|---|
| 156 | | Cl | $NH_2$ | CN | $-CH_2$ | 3,4,5-Trimethoxyphenyl | H |
| 157 | | Cl | $NH_2$ | CN | $CH_2$ | 2-Chloro-3,4,5-trimethoxyphenyl | H |
| 158 | | Cl | $NH_2$ | CN | $CH_2$ | 2-Bromo-3,4,5-trimethoxyphenyl | H |
| 159 | | Cl | $NH_2$ | CN | $CH_2$ | 2-Iodo-3,4,5-trimethoxyphenyl | H |
| 160 | | Cl | $NH_2$ | CN | $CH_2$ | 3,4,5-Trimethoxyphenyl | H |
| 161 | | Cl | $NH_2$ | CN | $CH_2$ | 2-Chloro-3,4,5-trimethoxyphenyl | H |
| 162 | | Cl | $NH_2$ | CN | $CH_2$ | 2-Bromo-3,4,5-trimethoxyphenyl | H |
| 163 | | Cl | $NH_2$ | CN | $CH_2$ | 2-Iodo-3,4,5-trimethoxyphenyl | H |
| 164 | | Cl | $NH_2$ | CN | $CH_2$ | 2-Fluoro-3,4,5-trimethoxyphenyl | H |
| 165 | | Cl | $NH_2$ | CN | $CH_2$ | 3,4,5-Trimethylphenyl | H |
| 166 | | Cl | $NH_2$ | CN | $CH_2$ | 2-Chloro-3,4,5-trimethylphenyl | H |
| 167 | | Cl | $NH_2$ | CN | $CH_2$ | 2-Bromo-3,4,5-trimethylphenyl | H |
| 168 | | Cl | $NH_2$ | CN | $CH_2$ | 2-Iodo-3,4,5-trimethylphenyl | H |
| 169 | | Cl | $NH_2$ | CN | $CH_2$ | 2-Fluoro-3,4,5-trimethylphenyl | H |
| 170 | | Cl | $NH_2$ | CN | $CH_2$ | 3,5-Dimethoxy-4-methylphenyl | H |
| 171 | | Cl | $NH_2$ | CN | $CH_2$ | 2-Chloro-3,5-dimethoxy-4-methylphenyl | H |
| 172 | | Cl | $NH_2$ | CN | $CH_2$ | 2-Bromo-3,5-dimethoxy-4-methylphenyl | H |
| 173 | | Cl | $NH_2$ | CN | $CH_2$ | 2-Iodo-3,5-dimethoxy-4-methylphenyl | H |
| 174 | | Cl | $NH_2$ | CN | $CH_2$ | 2-Fluoro-3, 5-dimethoxy-4-methylphenyl | H |
| 175 | | Cl | $NH_2$ | Cl | $CH_2$ | 3,4,5-Trimethoxyphenyl | H |
| 176 | | Cl | $NH_2$ | Cl | $CH_2$ | 2-Chloro-3,4,5-trimethoxyphenyl | H |
| 177 | | Cl | $NH_2$ | Cl | $CH_2$ | 2-Bromo-3,4,5-trimethoxyphenyl | H |
| 178 | | Cl | $NH_2$ | Cl | $CH_2$ | 2-Iodo-3,4,5-trimethoxyphenyl | H |
| 179 | | Cl | $NH_2$ | Cl | $CH_2$ | 2-Fluoro-3,4,5-trimetlnoxyphenyl | H |
| 180 | | Cl | $NH_2$ | Cl | $CH_2$ | 3,4,5-Trimethylphenyl | H |
| 181 | | Cl | $NH_2$ | Cl | $CH_2$ | 2-Chloro-3,4,5-trimethylphenyl | H |
| 182 | | Cl | $NH_2$ | Cl | $CH_2$ | 2-Bromo-3,4,5-trimethylphenyl | H |
| 183 | | Cl | $NH_2$ | Cl | $CH_2$ | 2-Iodo-3,4,5-trimethylphenyl | H |
| 184 | | Cl | $NH_2$ | Cl | $CH_2$ | 2-Fluoro-3,4,5-trimethylphenyl | H |
| 185 | | Cl | $NH_2$ | Cl | $CH_2$ | 3,5-Dimethoxy-4-methylphenyl | H |
| 186 | | Cl | $NH_2$ | Cl | $CH_2$ | 2-Chloro-3,5-dimethoxy-4-methylphenyl | H |
| 187 | | Cl | $NH_2$ | Cl | $CH_2$ | 2-Bromo-3,5-dimethoxy-4-methylphenyl | H |
| 188 | | Cl | $NH_2$ | Cl | $CH_2$ | 2-Iodo-3,5-dimethoxy-4-methylphenyl | H |
| 189 | | Cl | $NH_2$ | Cl | $CH_2$ | 2-Fluoro-3,5-dimethoxy-4-methylphenyl | H |
| 190 | | Cl | $NH_2$ | Br | $CH_2$ | 3,4,5-Trimethoxyphenyl | H |
| 191 | | Cl | $NH_2$ | Br | $CH_2$ | 2-Chloro-3,4,5-trimethoxyphenyl | H |
| 192 | | Cl | $NH_2$ | Br | $CH_2$ | 2-Bromo-3,4,5-trimethoxyphenyl | H |
| 193 | | Cl | $NH_2$ | Br | $CH_2$ | 2-Iodo-3,4,5-trimethoxyphenyl | H |

(continued)

| No. | Ex | $R^1$ | $R^2$ | $R^3$ | $R^4$ | $R^5$ | $R^0$ |
|---|---|---|---|---|---|---|---|
| 194 | | Cl | $NH_2$ | Br | $CH_2$ | 2-Fluoro-3,4,5-trimethoxyphenyl | H |
| 195 | | Cl | $NH_2$ | Br | $CH_2$ | 3,4,5-Trimethylphenyl | H |
| 196 | | Cl | $NH_2$ | Br | $CH_2$ | 2-Chloro-3,4,5-trimethylphenyl | H |
| 197 | | Cl | $NH_2$ | Br | $CH_2$ | 2-Bromo-3,4,5-trimethylphenyl | H |
| 198 | | Cl | $NH_2$ | Br | $CH_2$ | 2-Iodo-3,4,5-trimethylphenyl | H |
| 199 | | Cl | $NH_2$ | Br | $CH_2$ | 2-Fluoro-3,4,5-trimethylphenyl | H |
| 200 | | Cl | $NH_2$ | Br | $CH_2$ | 3,5-Dimethoxy-4-methylphenyl | H |
| 201 | | Cl | $NH_2$ | Br | $CH_2$ | 2-Chloro-3,5-dimethoxy-4-methylphenyl | H |
| 202 | | Cl | $NH_2$ | Br | $CH_2$ | 2-Bromo-3,5-dimethoxy-4-methylphenyl | H |
| 203 | | Cl | $NH_2$ | Br | $CH_2$ | 2-Iodo-3,5-dimethoxy-4-methylphenyl | H |
| 204 | | Cl | $NH_2$ | Br | $CH_2$ | 2-Fluoro-3,5-dimethoxy-4-methylphenyl | H |
| 205 | | Cl | $NH_2$ | I | $CH_2$ | 3,4,5-Trimethoxyphenyl | H |
| 206 | | Cl | $NH_2$ | I | $CH_2$ | 2-Chloro-3,4,5-trimethoxyphenyl | H |
| 207 | | Cl | $NH_2$ | I | $CH_2$ | 2-Bromo-3,4,5-trimethoxyphenyl | H |
| 208 | | Cl | $NH_2$ | I | $CH_2$ | 2-Iodo-3,4,5-trimethoxyphenyl | H |
| 209 | | Cl | $NH_2$ | I | $CH_2$ | 2-Fluoro-3,4,5-trimethoxyphenyl | H |
| 210 | | Cl | $NH_2$ | I | $CH_2$ | 3,4,5-Trimethylphenyl | H |
| 211 | | Cl | $NH_2$ | I | $CH_2$ | 2-Chloro-3,4,5-trimethylphenyl | H |
| 212 | | Cl | $NH_2$ | I | $CH_2$ | 2-Bromo-3,4,5-trimethylphenyl | H |
| 213 | | Cl | $NH_2$ | I | $CH_2$ | 2-Iodo-3,4,5-trimethylphenyl | H |
| 214 | | Cl | $NH_2$ | I | $CH_2$ | 2-Fluoro-3,4,5-trimethylphenyl | H |
| 215 | | Cl | $NH_2$ | I | $CH_2$ | 3,5-Dimethoxy-4-methylphenyl | H |
| 216 | | Cl | $NH_2$ | I | $CH_2$ | 2-Chloro-3,5-dimethoxy-4-methylphenyl | H |
| 217 | | Cl | $NH_2$ | I | $CH_2$ | 2-Bromo-3,5-dimethoxy-4-methylphenyl | H |
| 218 | | Cl | $NH_2$ | I | $CH_2$ | 2-Iodo-3,5-dimethoxy-4-methylphenyl | H |
| 219 | | Cl | $NH_2$ | I | $CH_2$ | 2-Fluoro-3,5-dimethoxy-4-methylphenyl | H |
| 220 | | Cl | $NH_2$ | $CH_2$-$NMe_2$ | $CH_2$ | 3,4,5-Trimethoxyphenyl | H |
| 221 | | Cl | $NH_2$ | $CH_2$-$NMe_2$ | | 2-Chloro-3,4,5-trimethoxyphenyl | H |
| 222 | | Cl | $NH_2$ | $CH_2$-$NMe_2$ | $CH_2$ | 2-Bromo-3,4,5-trimethoxyphenyl | H |
| 223 | | Cl | $NH_2$ | $CH_2$-$NMe_2$ | $CH_2$ | 2-Iodo-3,4,5-trimethoxyphenyl | H |
| 224 | | Cl | NH2 | $CH_2$- $NMe_2$ | $CH_2$ | 2-Fluoro-3,4,5-trimethoxyphenyl | H |
| 225 | | Cl | $NH_2$ | $CH_2$- $NMe_2$ | $CH_2$ | 3,4,5-Trimethylphenyl | H |
| 226 | | Cl | $NH_2$ | $CH_2$- $NMe_2$ | $CH_2$ | 2-Chloro-3,4,5-trimethylphenyl | H |
| 227 | | Cl | $NH^2$ | $CH_2$- $NMe_2$ | $CH_2$ | 2-Bromo-3,4,5-trimethylphenyl | H |
| 228 | | Cl | $NH^2$ | $CH_2$- $NMe_2$ | $CH_2$ | 2-Iodo-3,4,5-trimethylphenyl | H |
| 229 | | Cl | $NH_2$ | $CH_2$- $NMe_2$ | $CH_2$ | 2-Fluoro-3,4,5-trimethylphenyl | H |
| 230 | | Cl | $NH_2$ | $CH_2$- $NMe_2$ | $CH_2$ | 3,5-Dimethoxy-4-methylphenyl | H |
| 231 | | Cl | $NH_2$ | $CH_2$-$NMe_2$ | $CH_2$ | 2-Chloro-3,5-dimethoxy-4-methylphenyl | H |

(continued)

| No. | Ex | $R^1$ | $R^2$ | $R^3$ | $R^4$ | $R^5$ | $R^0$ |
|---|---|---|---|---|---|---|---|
| 232 | | Cl | $NH_2$ | $CH_2$-$NMe_2$ | $CH_2$ | 2-Bromo-3,5-dimethoxy-4-methylphenyl | H |
| 233 | | Cl | $NH_2$ | $CH_2$-$NMe_2$ | $CH_2$ | 2-Iodo-3,5-dimethoxy-4-methylphenyl | H |
| 234 | | Cl | $NH_2$ | $CH_2$-$NMe_2$ | $CH_2$ | 2-Fluoro-3,5-dimethoxy-4-methylphenyl | H |
| 235 | | Cl | $NH_2$ | 3-Py | $CH_2$ | 3,4,5-Trimethoxyphenyl | H |
| 236 | | Cl | $NH_2$ | 3-Py | $CH_2$ | 2-Chlolo-3,4,5-trimethoxyphenyl | H |
| 237 | | Cl | $NH_2$ | 3-Py | $CH_2$ | 2-Bromo-3,4,5-trimethoxyphenyl | H |
| 238 | | Cl | $NH_2$ | 3-Py | $CH_2$ | 2-Iodo-3,4,5-trimethoxyphenyl | H |
| 239 | | Cl | $NH_2$ | 3-Py | $CH_2$ | 2-Fluoro-3,4,5-trimethoxyphenyl | H |
| 240 | 5 | Cl | $NH_2$ | H | $CH_2$ | 3,5-Dimethyl-4-methoxypyridin-2-yl | H |
| 241 | 8 | Cl | $NH_2$ | H | $CH_2$ | 3,5-Dimethyl-4-methoxy-1-oxypyridin-2-yl | H |
| 242 | | Cl | $NH_2$ | H | $CH_2$ | 6-Bromo-3,5-dimethyl-4-methoxypyridin-2-yl | H |
| 243 | 10 | Cl | $NH_2$ | H | $CH_2$ | 6-Chloro-3,5-dimethyl-4-methoxypyridin-2-yl | H |
| 244 | 13 | Cl | $NH_2$ | H | $CH_2$ | 3,5-Dimethyl-4-bromopyridin-2-yl | H |
| 245 | 15 | Cl | $NH_2$ | H | $CH_2$ | 3,5-Dimethyl-4-bromo-l-oxypyridin-2-yl | H. |
| 246 | 11 | Cl | $NH_2$ | H | $CH_2$ | 3,5-Dimethyl-4-chloropyridin-2-yl | H |
| 247 | 14 | Cl | $NH_2$ | H | $CH_2$ | 3,5-Dimethyl-4-chloro-1-oxypyridin-2-yl | H |
| 248 | | Cl | $NH_2$ | H | $CH_2$ | 3,5-Dimethyl-4-iodopyridin-2-yl | H |
| 249 | | Cl | $NH_2$ | H | $CH_2$ | 3,5-Dimethyl-4-iodo-1-oxypyridin-2-yl | H |
| 250 | | Cl | $NH_2$ | H | $CH_2$ | 3,5-Dimethyl-4-thiomethyl-pyridin-2-yl | H |
| 251 | | Cl | $NH_2$ | H | $CH_2$ | 3,5-Dimethyl-4-thiomethyl-1-oxypyridin-2-yl | H |
| 252 | | Cl | $NH_2$ | H | $CH_2$ | 3,4,5-Trimethyl-pyridin-2-yl | H |
| 253 | | Cl | $NH_2$ | H | $CH_2$ | 3,4,5-Trimethyl-1-oxypyridin-2-yl | H |
| 254 | | Cl | $NH_2$ | H | $CH_2$ | 4,5,6-Trimethoxypyridin-2-yl | H |
| 255 | | Cl | $NH_2$ | H | $CH_2$ | 4,5,6-Trimethoxy-1-oxypyridin-2-yl | H |
| 256 | | Cl | $NH_2$ | H | $CH_2$ | 3-Bxomo-4,5,6-trimethoxypyridin-2-yl | H |
| 257 | | Cl | $NH_2$ | H | $CH_2$ | 3-Chloro-4,5,6-trimethoxypyridin-2-yl | H |
| 258 | | Cl | $NH_2$ | H | $CH_2$ | 3,4,5-Trimethoxy-pyridin-2-yl | H |
| 259 | | Cl | $NH_2$ | H | $CH_2$ | 3,4,5-Trimethoxy-1-oxypyridin-2-yl | H. |
| 260 | | Cl | $NH_2$ | H | $CH_2$ | 3-Bromo-3,4,5-trimethoxy-pyridin-2-yl | H |
| 261 | | Cl | $NH_2$ | H | $CH_2$ | 3-Chloro-3,4,5-trimethoxy-pyridin-2-yl | H |
| 262 | | Cl | $NH_2$ | H | $CH_2$ | 4,5,6-Trimethyl-pyridin-2-yl | H |
| 263 | | Cl | $NH_2$ | H | $CH_2$ | 4,5,6-Trimethyl-1-oxypyridin-2-yl | H |
| 264 | | Cl | $NH_2$ | H | $CH_2$ | 4,6-Dimethyl-5-methoxy-pyridin-2-yl | H |
| 265 | | Cl | $NH_2$ | H | $CH_2$ | 4,6-Dimethyl-5-methoxypyridin-3-yl | H |
| 266 | | Cl | $NH_2$ | H | $CH_2$ | 4,6-Dimethyl-5-methoxy-1-oxypyridin-3-yl | H |
| 267 | | Cl | $NH_2$ | H | $CH_2$ | 4,6-Dimethyl-5-bromopyridin-3-yl | H |
| 268 | | Cl | $NH_2$ | H | $CH_2$ | 4,6-Dimethyl-5-chloropyridin-3-yl | H |
| 269 | | Cl | $NH_2$ | H | $CH_2$ | 5,6-Dimethyl-4-bromopyridin-3-yl | H |

(continued)

| No. | Ex | $R^1$ | $R^2$ | $R^3$ | $R^4$ | $R^5$ | $R^0$ |
|---|---|---|---|---|---|---|---|
| 270 | | Cl | $NH_2$ | H | $CH_2$ | 5,6-Dimethyl-4-chloropyridin-3-yl | H |
| 271 | | Cl | $NH_2$ | H | $CH_2$ | 2,6-Dimethyl-3-methoxypyridin-4-yl | H |
| 272 | | Cl | $NH_2$ | H | $CH_2$ | 2,6-Dimethyl-pyridin-4-yl | H. |
| 273 | | Cl | $NH_2$ | H | $CH_2$ | 2,3,6-Tzimethyl-pyridin-4-yl | H |
| 274 | | Cl | $NH_2$ | H | $CH_2$ | 2,3,6-Trimethoxy-pyridin-4-yl | H |
| 275 | | Cl | $NH_2$ | H | $CH_2$ | 2,6-Dimethyl-3-bromopyridin-4-yl | H |
| 276 | | Cl | $NH_2$ | H | $CH_2$ | 2,6-Dimethyl-3-chlaropyridin-4-yl | H |
| 277 | | Cl | $NH_2$ | H | $CH_2$ | 2,6-Dimethyl-3-methoxy-1-oxy pyridin-4-yl | H |
| 278 | | Cl | $NH_2$ | H | $CH_2$ | 2,6-Dimethyl-1-oxy-pyridin-4-yl | H |
| 279 | | Cl | $NH_2$ | H | $CH_2$ | 2,3,6-Trimethyl-1-oxy-pyridin-4-yl | H |
| 280 | | Cl | $NH_2$ | H | $CH_2$ | 2,3,6-Trimethoxy-1-oxy-pyridin-4-yl | H |
| 281 | | Cl | $NH_2$ | H | $CH_2$ | 2,6-Dimethyl-3-bromol-oxy-pyridin-4-yl | H |
| 282 | | Cl | $NH_2$ | H | $CH_2$ | 2,6-Dimethyl-3-chlorol-oxy-pyridin-4-yl | H |
| 283 | | Cl | $NH_2$ | H | $CH_2$ | 4,6-Dimethyl-5-iodopyridin-3-yl | H |
| 284 | | Cl | $NH_2$ | H | $CH_2$ | 3,5-Dimethyl-4-aminopyridin-2-yl | H |
| 285 | | Cl | $NH_2$ | i-pr | $CH_2$ | 3,5-Dimethyl-4-methoxypyridin-2-yl | H |
| 286 | | Cl | $NH_2$ | i-pr | $CH_2$ | 3,5-Dimethyl-4-mthoxy-1-oxypyridin-2-yl | H |
| 287 | | Cl | $NH_2$ | i-pr | $CH_2$ | 6-Bromo-3,5-dimethyl-4-methoxypyridin-2-yl | H |
| 288 | | Cl | $NH_2$ | i-pr | $CH_2$ | 6-Chloro-3,5-dimethyl-4-methoxypyridin-2-yl | H |
| 289 | | Cl | $NH_2$ | i-pr | $CH_2$ | 3,5-Dimethyl-4-bromopyridin-2-yl | H |
| 290 | | Cl | $NH_2$ | i-pr | $CH_2$ | 3,5-Dimethyl-4-bromo-1-oxypyridin-2-yl | H |
| 291 | | Cl | $NH_2$ | i-pr | $CH_2$ | 3,5-Dimethyl-4-chloropyridin-2-yl | H |
| 292 | | Cl | $NH_2$ | i-pr | $CH_2$ | 3,5-Dimethyl-4-chloro-1-oxypyridin-2-yl | H |
| 293 | | Cl | NH2 | i-pr | $CH_2$ | 3,5-Dimethyl-4-iodopyridin-2-yl | H |
| 294 | | Cl | $NH_2$ | i-pr | $CH_2$ | 3,5-Dimethyl-4-iodo-1-oxypyridin-2-yl | H |
| 295 | | Cl | $NH_2$ | i-pr | $CH_2$ | 3,5-Dimethyl-4-thiomethyl-pyridin-2-yl | H |
| 296 | | Cl | $NH_2$ | i-pr | $CH_2$ | 3,5-Dimethyl-4-thiomethyl-1-oxypyridin-2-yl | H |
| 297 | | Cl | $NH_2$ | i-pr | $CH_2$ | 3,4,5-Trimethyl-pyridin-2-yl | H |
| 298 | | Cl | $NH_2$ | i-pr | $CH_2$ | 3,4,5-Trimethyl-1-oxypyridin-2-yl | H |
| 299 | | Cl | $NH_2$ | i-pr | $CH_2$ | 4,5,6-Trimethoxypyridin-2-yl | H. |
| 300 | | Cl | $NH_2$ | i-pr | $CH_2$ | 4,5,6-Trimethoxy-1-oxypyridin-2-yl | H |
| 301 | | Cl | $NH_2$ | i-pr | $CH_2$ | 3-Bromo-4,5,6-trimethoxypyridin-2-yl | H |
| 302 | | Cl | $NH_2$ | i-pr | $CH_2$ | 3-Chloro-4,5,6-trinnethoxypyridin-2-yl | H |
| 303 | | Cl | $NH_2$ | i-pr | $CH_2$ | 3,4,5-Trimethoxy-pyridin-2-yl | H |
| 304 | | Cl | $NH_2$ | i-pr | $CH_2$ | 3,4,5-Trimethoxy-1-oxypyridin-2-yl | H |
| 305 | | Cl | $NH_2$ | i-pr | $CH_2$ | 3-Bromo-3,4,5-trimethoxy-pyridin-2-yl | H |
| 306 | | Cl | $NH_2$ | i-pr | $CH_2$ | 3-Chloro-3,4,5-trimethoxy-pyridin-2-yl | H |
| 307 | | Cl | $NH_2$ | i-pr | $CH_2$ | 4,5,6-Trimethyl-pyridin-2-yl | H |

(continued)

| No. | Ex | $R^1$ | $R^2$ | $R^3$ | $R^4$ | $R^5$ | $R^0$ |
|---|---|---|---|---|---|---|---|
| 308 | | Cl | $NH_2$ | i-pr | $CH_2$ | 4,5,6-Trimethyl-1-oxypyridin-2-yl | H |
| 309 | | Cl | $NH_2$ | i-pr | $CH_2$ | 4,6-Dimethyl-5-methoxy-pyridin-2-yl | H |
| 310 | | Cl | $NH_2$ | i-pr | $CH_2$ | 4,6-Dimethyl-5-methoxypyridin-3-yl | H |
| 311 | | Cl | $NH_2$ | i-pr | $CH_2$ | 4,6-Dimethyl-5-methoxy-1-oxypyridin-3-yl | H |
| 312 | | Cl | $NH_2$ | i-pr | $CH_2$ | 4,6-Dimethyl-5-bromopyridin-3-yl | H |
| 313 | | Cl | $NH_2$ | i-pr | $CH_2$ | 4,6-Dimethyl-5-chloropyridin-3-yl | H. |
| 314 | | Cl | $NH_2$ | i-pr | $CH_2$ | 5,6-Dimethyl-4-bromopyridin-3-yl | H |
| 315 | | Cl | $NH_2$ | i-pr | $CH_2$ | 5,6-Dimethyl-4-chloropyridin-3-yl | H |
| 316 | | Cl | $NH_2$ | i-pr | $CH_2$ | 2,6-Dimethyl-3-methoxypyridin-4-yl | H |
| 317 | | Cl | $NH_2$ | i-pr | $CH_2$ | 2,6-Dimethyl-pyridin-4-yl | H |
| 318 | | Cl | $NH_2$ | i-pr | $CH_2$ | 2,3,6-Trimethyl-pyridin-4-yl | H |
| 319 | | Cl | $NH_2$ | i-pr | $CH_2$ | 2,3,6-Trimethoxy-pyridin-4-yl | H |
| 320 | | Cl | $NH_2$ | i-pr | $CH_2$ | 2,6-Dimethyl-3-bromopyridin-4-yl | H |
| 321 | | Cl | $NH_2$ | i-pr | $CH_2$ | 2,6-Dimethyl-3-chloropyridin-4-yl | H |
| 322 | | Cl | $NH_2$ | i-pr | $CH_2$ | 2,6-Dimethyl-3-methoxy-1-oxy-pyridin-4-yl | H |
| 323 | | Cl | $NH_2$ | i-pr | $CH_2$ | 2,6-Dimethyl-1-oxy-pyridin-4-yl | H |
| 324 | | Cl | $NH_2$ | i-pr | $CH_2$ | 2,3,6-Trimethyl-1-oxy-pyridin-4-yl | H |
| 325 | | Cl | $NH_2$ | i-pr | $CH_2$ | 2,3,6-Trimethoxy-1-oxy-pyridin-4-yl | H |
| 326 | | Cl | $NH_2$ | i-pr | $CH_2$ | 2,6-Dimethyl-3-bromol-oxy-pyridin-4-yl | H |
| 327 | | Cl | $NH_2$ | i-pr | $CH_2$ | 2,6-Dimethyl-3-chlorl-oxy-pyridin-4-yl | H |
| 328 | | Cl | $NH_2$ | i-pr | $CH_2$ | 4,6-Dimethyl-5-iodopyridin-3-yl | H |
| 329 | | Cl | $NH_2$ | i-pr | $CH_2$ | 3,5-Dimethyl-4-aminopyridin-2-yl | H |
| 330 | | Cl | $NH_2$ | Me | $CH_2$ | 3,5-Dimethyl-4-methoxypyridin-2-yl | H |
| 331 | | Cl | $NH_2$ | Me | $CH_2$ | 3,5-Dimethyl-4-methoxy-1-oxypyridin-2-yl | H |
| 332 | | Cl | $NH_2$ | Me | $CH_2$ | 3,5-Dimethyl-4-bromopyridin-2-yl | H |
| 333 | | Cl | $NH_2$ | Me | $CH_2$ | 3,5-Dimethyl-4-bromo-1-oxypyridin-2-yl | H |
| 334 | | Cl | $NH_2$ | Me | $CH_2$ | 3,5-Dimethyl-4-chloropyridin-2-yl | H |
| 335 | | Cl | $NH_2$ | Me | $CH_2$ | 3,5-Dimethyl-4-chloro-1-oxypyridin-2-yl | H |
| 336 | | Cl | $NH_2$ | Me | $CH_2$ | 3,5-Dimethyl-4-iodopyridin-2-yl | H |
| 337 | | Cl | $NH_2$ | Me | $CH_2$ | 3,5-Dimethyl-4-iodo-1-oxypyridin-2-yl | H |
| 338 | | Cl | $NH_2$ | Me | $CH_2$ | 3,5-Dimethyl-4-thiomethyl-pyridin-2-yl | H |
| 339 | | Cl | $NH_2$ | Me | $CH_2$ | 3,5-Dimethyl-4-thiomethyl-1-oxypyridin-2-yl | H |
| 340 | | Cl | $NH_2$ | Me | $CH_2$ | 3,4,5-Trimethyl-pyridin-2-yl | H |
| 341 | | Cl | $NH_2$ | Me | $CH_2$ | 3,4,5-Trimethyl-1-oxypyridin-2-yl | H |
| 342 | | Cl | $NH_2$ | Me | $CH_2$ | 4,5,6-Trimethoxypyridin-2-yl | H |
| 343 | | Cl | $NH_2$ | Me | $CH_2$ | 4,5,6-Trimethoxy-1-oxypyridin-2-yi | H |
| 344 | | Cl | $NH_2$ | Me | $CH_2$ | 3,4,5-Trimethoxy-pyridin-2-yl | H |
| 345 | | Cl | $NH_2$ | Me | $CH_2$ | 3,4,5-Trimethoxy-1-oxypyzidin-2-yl | H |

(continued)

| No. | Ex | $R^1$ | $R^2$ | $R^3$ | $R^4$ | $R^5$ | $R^0$ |
|---|---|---|---|---|---|---|---|
| 346 | | Cl | $NH_2$ | Me | $CH_2$ | 4,5,6-Trimethyl-pyridin-2-yl | H |
| 347 | | Cl | $NH_2$ | Me | $CH_2$ | 4,5,6-Trimethyl-1-oxypyridin-2-yl | H |
| 348 | | Cl | $NH_2$ | Me | $CH_2$ | 4,6-Dhnethyl-5-methoxy-pyridin-2-yl | H |
| 349 | | Cl | $NH_2$ | Me | $CH_2$ | 4,6-Dimethyl-5-methoxypyridin-3-yl | H |
| 350 | | Cl | $NH_2$ | Me | $CH_2$ | 4,6-Dimethyl-5-methoxy-1-oxypyridin-3-yl | H |
| 351 | | Cl | $NH_2$ | Me | $CH_2$ | 4,6-Dimethyl-5-bromopyridin-3-yl | H |
| 352 | | Cl | $NH_2$ | Me | $CH_2$ | 4,6-Dimethyl-5-chloropyridin-3-yl | H |
| 353 | | Cl | $NH_2$ | Me | $CH_2$ | 5,6-Dimethyl-4-bromopyridin-3-yl | H |
| 354 | | Cl | $NH_2$ | Me | $CH_2$ | 5,6-Dimethyl-4-chloropyridin-3-yl | H. |
| 355 | | Cl | $NH_2$ | Me | $CH_2$ | 2,6-Dimethyl-3-methoxypyridin-4-yl | H |
| 356 | | Cl | $NH_2$ | Me | $CH_2$ | 2,6-Dimethyl-pyridin-4-yl | H |
| 357 | | Cl | $NH_2$ | Me | $CH_2$ | 2,3,6-Trhnethyl-pyridin-4-yl | H |
| 358 | | Cl | $NH_2$ | Me | $CH_2$ | 2,3,6-Trimethoxy-pyridin-4-yl | H |
| 359 | | Cl | $NH_2$ | Me | $CH_2$ | 2,6-Dimethyl-3-bromopyridin-4-yl | H |
| 360 | | Cl | $NH_2$ | Me | $CH_2$ | 2,6-Dimethyl-3-chloropyridin-4-yl | H |
| 361 | | Cl | $NH_2$ | Me | $CH_2$ | 4,6-Dixnethyl-5-iodopyridin-3 yl | H |
| 362 | | Cl | $NH_2$ | Me | $CH_2$ | 3,5-Dimethyl-4-aminopyridin-2-yl | H |
| 363 | | Cl | $NH_2$ | Et | $CH_2$ | 3,5-Dimethyl-4-methoxypyridin-2-yl | H |
| 364 | | Cl | $NH_2$ | Et | $CH_2$ | 3,5-Dimethyl-4-methoxy-1-oxypyridin-2-yl | H |
| 365 | | Cl | $NH_2$ | Et | $CH_2$ | 3,5-Dimethyl-4-bromopyridin-2-yl | H |
| 366 | | Cl | $NH_2$ | Et | $CH_2$ | 3,5-Dimethyl-4-bromo-1-oxypyridin-2-yl | H |
| 367 | | Cl | $NH_2$ | Et | $CH_2$ | 3,5-Dimethyl-4-chloropyridin-2-yl | H |
| 368 | | Cl | $NH_2$ | Et | $CH_2$ | 3,5-Dimethyl-4-chloro-1-oxypyridin-2-yl | H |
| 369 | | Cl | $NH_2$ | Et | $CH_2$ | 3,5-Dimethyl-4-iodopyridin-2-yl | H |
| 370 | | Cl | $NH_2$ | Et | $CH_2$ | 3,5-Dimethyl-4-iodo-1-oxypyridin-2-yl | H |
| 371 | | Cl | $NH_2$ | Et | $CH_2$ | 3,5-Dimethyl-4-thiomethyl-pyridin-2-yl | H |
| 372 | | Cl | $NH_2$ | Et | $CH_2$ | 3,5-Dimethyl-4-thiomethyl-1-oxypyndin-2-yl | H |
| 373 | | Cl | $NH_2$ | Et | $CH_2$ | 3,4,5-Trimethyl-pyridin-2-yl | H |
| 374 | | Cl | $NH_2$ | Et | $CH_2$ | 3,4,5-Trimethyl-1-oxypyridin-2-yl | H |
| 375 | | Cl | $NH_2$ | Et | $CH_2$ | 4,5,6-Trimethoxypyridin-2-yl | H |
| 376 | | Cl | $NH_2$ | Et | $CH_2$ | 4,5,6-Trimethoxy-1-oxypyridin-2-yl | H |
| 377 | | Cl | $NH_2$ | Et | $CH_2$ | 3,4,5-Trimethoxy-pyridin-2-yl | H |
| 378 | | Cl | $NH_2$ | Et | $CH_2$ | 3,4,S-Trimethoxy-1-oxypyridin-2-yl | H |
| 379 | | Cl | $NH_2$ | Et | $CH_2$ | 4,5,6-Trimethyl-pyridin-2-yl | H |
| 380 | | Cl | $NH_2$ | Et | $CH_2$ | 4,5,6-Trimethyl-1-oxypyridin-2-yl | H |
| 381 | | Cl | $NH_2$ | Et | $CH_2$ | 4,6-Dimethyl-5-methoxy-pyidin-2-yl | H |
| 382 | | Cl | $NH_2$ | Et | $CH_2$ | 4,6-Dimethyl-5-methoxypyridin-3-yl | H |
| 383 | | Cl | $NH_2$ | Et | $CH_2$ | 4,6-Dimethyl-5-methoxy-1-oxypyridin-3-yl | H |

(continued)

| No. | Ex | $R^1$ | $R^2$ | $R^3$ | $R^4$ | $R^5$ | $R^0$ |
|---|---|---|---|---|---|---|---|
| 384 | | Cl | $NH_2$ | Et | $CH_2$ | 4,6-Dimethyl-5-bromopyridin-3-yl | H |
| 385 | | Cl | $NH_2$ | Et | $CH_2$ | 4,6-Dimethyl-5-chloropyridin-3-yl | H |
| 386 | | Cl | $NH_2$ | Et | $CH_2$ | 5,6-Dimethyl-4-bromopyridin-3-yl | H |
| 387 | | Cl | $NH_2$ | Et | $CH_2$ | 5,6-Dimethyl-4-chloropyridin-3-yl | H |
| 388 | | Cl | $NH_2$ | Et | $CH_2$ | 2,6-Dimethyl-3-methoxypyridin-4-yl | H |
| 389 | | Cl | $NH_2$ | Et | $CH_2$ | 2,6-Dimethyl-pyridin-4-yl | H |
| 390 | | Cl | $NH_2$ | Et | $CH_2$ | 2,3,6-Trimethyl-pyridin-4-yl | H |
| 391 | | Cl | $NH_2$ | Et | $CH_2$ | 2,3,6-Trimethoxy-pyridin-4-yl | H |
| 392 | | Cl | $NH_2$ | Et | $CH_2$ | 2,6-Dimethyl-3-bromopyridin-4-yl | H |
| 393 | | Cl | $NH_2$ | Et | $CH_2$ | 2,6-Dimethyl-3-chloropyridin-4-yl | H |
| 394 | | Cl | $NH_2$ | Et | $CH_2$ | 4,6-Dimethyl-5-iodopyridin-3-yl | H |
| 395 | | Cl | $NH_2$ | Et | $CH_2$ | 3,5-Dimethyl-4-aminopyridin-2-yl | H |
| 396 | | Cl | $NH_2$ | 2-Py | $CH_2$ | 3,5-Dimethyl-4-methoxypyridin-2-yl | H |
| 397 | | Cl | $NH_2$ | 2-Py | $CH_2$ | 3,5-Dimethyl-4-methoxy-1-oxypyridin-2-yl | H |
| 398 | | Cl | $NH_2$ | 2-Py | $CH_2$ | 3,5-Dimethyl-4-bromopyridin-2-yl | H |
| 399 | | Cl | $NH_2$ | 2-Py | $CH_2$ | 3,5-Dimethyl-4-bromo-1-oxypyridin-2-yl | H |
| 400 | | Cl | $NH_2$ | 2-Py | $CH_2$ | 3,5-Dimethyl-4-chloropyridin-2-yl | H |
| 401 | | Cl | $NH_2$ | 2-Py | $CH_2$ | 3,5-Dimethyl-4-chloro-1-oxypyridin-2-yl | H |
| 402 | | Cl | $NH_2$ | 2-Py | $CH_2$ | 3,5-Dimethyl-4-iodopyridin-2-yl | H |
| 403 | | Cl | $NH_2$ | 2-Py | $CH_2$ | 3,5-Dimethyl-4-iodo-1-oxypyridin-2-yl | H |
| 404 | | Cl | $NH_2$ | 2-Py | $CH_2$ | 3,5-Dimethyl-4-thiomethyl-pyridin-2-yl | H |
| 405 | | Cl | $NH_2$ | 2-Py | $CH_2$ | 3,5-Dimethyl-4-thiomethyl-1-oxypyridin-2-yl | H |
| 406 | | Cl | $NH_2$ | 2-Py | $CH_2$ | 3,4,5-Trimethyl-pyridin-2-yl | H |
| 407 | | Cl | $NH_2$ | 2-Py | $CH_2$ | 3,4,5-Trimethyl-1-oxypyridin-2-yl | H |
| 408 | | Cl | $NH_2$ | 2-Py | $CH_2$ | 4,5,6-Trimethoxypyridin-2-yl | H. |
| 409 | | Cl | $NH_2$ | 2-Py | $CH_2$ | 4,5,6-Trimethoxy-1-oxypyridin-2-yl | H |
| 410 | | Cl | $NH_2$ | 2-Py | $CH_2$ | 3,4,5-Trimethoxy-pyridin-2-yl | H |
| 411 | | Cl | $NH_2$ | 2-Py | $CH_2$ | 3,4,5-Trimethoxy-1-oxypyridin-2-yl | H |
| 412 | | Cl | $NH_2$ | 2-Py | $CH_2$ | 4,5,6-Trimethyl-pyridin-2-yl | H |
| 413 | | Cl | $NH_2$ | 2-Py | $CH_2$ | 4,5,6-Trimethyl-1-oxypyridin-2-yl | H |
| 414 | | Cl | $NH_2$ | 2-Py | $CH_2$ | 4,6-Dimethyl-5-methoxy-pyridin-2-yl | H |
| 415 | | Cl | $NH_2$ | 2-Py | $CH_2$ | 4,6-Dimethyl-5-methoxypyridin-3-yl | H |
| 416 | | Cl | $NH_2$ | 2-Py | $CH_2$ | 4,6-Dimethyl-5-methoxy-1-oxypyridin-3-yl | H |
| 417 | | Cl | $NH_2$ | 2-Py | $CH_2$ | 4,6-Dimethyl-5-bromopyridin-3-yl | H |
| 418 | | Cl | $NH_2$ | 2-Py | $CH_2$ | 4,6-Dimethyl-5-chloropyridin-3-yl | H |
| 419 | | Cl | $NH_2$ | 2-Py | $CH_2$ | 5,6-Dimethyl-4-bromopyridin-3-yl | H |
| 420 | | Cl | $NH_2$ | 2-Py | $CH_2$ | 5,6-Dimethyl-4-chloropyridin-3-yl | H |
| 421 | | Cl | $NH_2$ | 2-Py | $CH_2$ | 2,6-Dimethyl-3-methoxypyridin-4-yl | H. |

(continued)

| No. | Ex | $R^1$ | $R^2$ | $R^3$ | $R^4$ | $R^5$ | $R^0$ |
|---|---|---|---|---|---|---|---|
| 422 | | Cl | $NH_2$ | 2-Py | $CH_2$ | 2,6-Dimethyl-pyridin-4-yl | H |
| 423 | | Cl | $NH_2$ | 2-Py | $CH_2$ | 2,3,6-Trimethyl-pyridin-4-yl | H |
| 424 | | Cl | $NH_2$ | 2-Py | $CH_2$ | 2,3,6-Trimethoxy-pyridin-4-yl | H |
| 425 | | Cl | $NH_2$ | 2-Py | $CH_2$ | 2,6-Dimethyl-3-bromopyridin-4-yl | H |
| 426 | | Cl | $NH_2$ | 2-Py | $CH_2$ | 2,6-Dimethyl-3-chloropyridin-4-yl | H |
| 427 | | Cl | $NH_2$ | 2-Py | $CH_2$ | 4,6-Dimethyl-5-iodopyridin-3-yl | H |
| 428 | | Cl | $NH_2$ | 2-Py | $CH_2$ | 3,5-Dimethyl-4-aminopyridin-2-yl | H |
| 429 | | Cl | $NH_2$ | Ph | $CH_2$ | 3,5-Dimethyl-4-methoxypyridin-2-yl | H |
| 430 | | Cl | $NH_2$ | Ph | $CH_2$ | 3,5-Dimethyl-4-methoxy-1-oxypyridin-2-yl | H |
| 431 | | Cl | $NH_2$ | Ph | $CH_2$ | 3,5-Dimethyl-4-bromopyridin-2-yl | H |
| 432 | | Cl | $NH_2$ | Ph | $CH_2$ | 3,5-Dimethyl-4-bromo-1-oxypyridin-2-yl | H |
| 433 | | Cl | $NH_2$ | Ph | $CH_2$ | 3,5-Dimethyl-4-chloropyridin-2-yl | H |
| 434 | | Cl | $NH_2$ | Ph | $CH_2$ | 3,5-Dimethyl-4-chloro-1-oxypyridin-2-yl | H |
| 435 | | Cl | $NH_2$ | Ph | $CH_2$ | 3,5-Dimethyl-4-iodopyridin-2-yl | H |
| 436 | | Cl | $NH_2$ | Ph | $CH_2$ | 3,5-Dimethyl-4-iodo-1-oxypyridin-2-yl | H |
| 437 | | Cl | $NH_2$ | Ph | $CH_2$ | 3,5-Dimethyl-4-thiomethyl-pyridin-2-yl | H |
| 438 | | Cl | $NH_2$ | Ph | $CH_2$ | 3,5-Dimethyl-4-thiomethyl-1-oxypyridin-2-yl | H |
| 439 | | Cl | $NH_2$ | Ph | $CH_2$ | 3,4,5-Trimethyl-pyridin-2-yl | H |
| 440 | | Cl | $NH_2$ | Ph | $CH_2$ | 3,4,5-Trimethyl-1-oxypyridin-2-yl | H |
| 441 | | Cl | $NH_2$ | Ph | $CH_2$ | 4,5,6-Trimethoxypyridin-2-yl | H |
| 442 | | Cl | $NH_2$ | Ph | $CH_2$ | 4,5,6-Trimethoxy-1-oxypyridin-2-yl | H |
| 443 | | Cl | $NH_2$ | Ph | $CH_2$ | 3,4,5-Trimethoxy-pyridin-2-yl | H |
| 444 | | Cl | $NH_2$ | Ph | $CH_2$ | 3,4,5-Trimethoxy-1-oxypyridin-2-yl | H |
| 445 | | Cl | $NH_2$ | Ph | $CH_2$ | 4,5,6-Trimethyl-pyridin-2-yl | H |
| 446 | | Cl | $NH_2$ | Ph | $CH_2$ | 4,5,6-Trimethyl-1-oxypyridin-2-yl | H |
| 447 | | Cl | $NH_2$ | Ph | $CH_2$ | 4,6-Dimethyl-5-methoxy-pyridin-2-yl | H |
| 448 | | Cl | $NH_2$ | Ph | $CH_2$ | 4,6-Dimethyl-5-methoxypyridin-3-yl | H |
| 449 | | Cl | $NH_2$ | Ph | $CH_2$ | 4,6-Dimethyl-5-methoxy-1-oxypyridin-3-yl | H. |
| 450 | | Cl | $NH_2$ | Ph | $CH_2$ | 4,6-Dimethyl-5-bromopyridin-3-yl | H |
| 451 | | Cl | $NH_2$ | Ph | $CH_2$ | 4,6-Dimethyl-5-chloropyridin-3-yl | H |
| 452 | | Cl | $NH_2$ | Ph | $CH_2$ | 5,6-Dimethyl-4-bromopyridin-3-yl | H |
| 453 | | Cl | $NH_2$ | Ph | $CH_2$ | 5,6-Dimethyl-4-chloropyridin-3-yl | H |
| 454 | | Cl | $NH_2$ | Ph | $CH_2$ | 2,6-Dimethyl-3-methoxypyridin-4-yl | H |
| 455 | | Cl | $NH_2$ | Ph | $CH_2$ | 2,6-Dimethyl-pyridin-4-yl | H |
| 456 | | Cl | $NH_2$ | Ph | $CH_2$ | 2,3,6-Trimethyl-pyridin-4-yl | H |
| 457 | | Cl | $NH_2$ | Ph | $CH_2$ | 2,3,6-Trimethoxy-pyridin-4-yl | H |
| 458 | | Cl | $NH_2$ | Ph | $CH_2$ | 2,6-Dimethyl-3-bromopyridin-4-yl | H |
| 459 | | Cl | $NH_2$ | Ph | $CH_2$ | 2,6-Dimethyl-3-chloropyridin-4-yl | H |

(continued)

| No. | Ex | $R^1$ | $R^2$ | $R^3$ | $R^4$ | $R^5$ | $R^0$ |
|---|---|---|---|---|---|---|---|
| 460 | | Cl | $NH_2$ | Ph | $CH_2$ | 4,6-Dimethyl-5-iodopyridin-3-yl | H |
| 461 | | Cl | $NH_2$ | Ph | $CH_2$ | 3,5-Dimethyl-4-aminopyridin-2-yl | H |
| 462 | | Cl | $NH_2$ | 3-Py | $CH_2$ | 3,5-Dimethyl-4-methoxypyridin-2-yl | H |
| 463 | | Cl | $NH_2$ | 3-Py | $CH_2$ | 3,5-Dimethyl-4-methoxy-1-oxypyridin-2-yl | H |
| 464 | | Cl | $NH_2$ | 3-Py | $CH_2$ | 3,5-Dimethyl-4-bromopyridin-2-yl | H |
| 465 | | Cl | $NH_2$ | 3-Py | $CH_2$ | 3,5-Dimethyl-4-bromo-1-oxypyridin-2-yl | H |
| 466 | | Cl | $NH_2$ | 3-Py | $CH_2$ | 3,5-Dimethyl-4-chloropyridin-2-yl | H |
| 467 | | Cl | $NH_2$ | 3-Py | $CH_2$ | 3,5-Dimethyl-4-chloro-1-oxypyridin-2-yl | H |
| 468 | | Cl | $NH_2$ | 3-Py | $CH_2$ | 3,5-Dimethyl-4-iodopyridin-2-yl | H |
| 469 | | Cl | $NH_2$ | 3-Py | $CH_2$ | 3,5-Dimethyl-4-iodo-1-oxypyridin-2-yl | H |
| 470 | | Cl | $NH_2$ | 3-Py | $CH_2$ | 3,5-Dimethyl-4-thiomthyl-pyridin-2-yl | H |
| 471 | | Cl | $NH_2$ | 3-Py | $CH_2$ | 3,5-Dimethyl-4-thiomethyl-1-oxypyridin-2-yl | H |
| 472 | | Cl | $NH_2$ | 3-Py | $CH_2$ | 3,4,5-Trimethyl-pyridin-2-yl | H |
| 473 | | Cl | $NH_2$ | 3-Py | $CH_2$ | 3,4,5-Trimethyl-1-oxypyridin-2-yl | H |
| 474 | | Cl | $NH_2$ | 3-Py | $CH_2$ | 4,5,6-Trimethoxypyridin-2-yl | H |
| 475 | | Cl | $NH_2$ | 3-Py | $CH_2$ | 4,5,6-Trimethoxy-1-oxypyridin-2-yl | H |
| 476 | | Cl | $NH_2$ | 3-Py | $CH_2$ | 3,4,5-Trimethoxy-pyridin-2-yl | H. |
| 477 | | Cl | $NH_2$ | 3-Py | $CH_2$ | 3,4,5-Trimethoxy-1-oxypyridin-2-yl | H |
| 478 | | Cl | $NH_2$ | 3-Py | $CH_2$ | 4,5,6-Trimethyl-pyridin-2-yl | H |
| 479 | | Cl | $NH_2$ | 3-Py | $CH_2$ | 4,5,6-Trimethyl-1-oxypyridin-2-yl | H |
| 480 | | Cl | $NH_2$ | 3-Py | $CH_2$ | 4,6-Dimethyl-5-methoxy-pyridin-2-yl | H |
| 481 | | Cl | $NH_2$ | 3-Py | $CH_2$ | 4,6-Dimethyl-5-methoxypyridin-3-yl | H |
| 482 | | Cl | $NH_2$ | 3-Py | $CH_2$ | 4,6-Dimethyl-5-methoxy-1-oxypyridin-3-yl | H |
| 483 | | Cl | $NH_2$ | 3-Py | $CH_2$ | 4,6-Dimethyl-5-bromopyridin-3-yl | H |
| 484 | | Cl | $NH_2$ | 3-Py | $CH_2$ | 4,6-Dimethyl-5-chloropyridin-3-yl | H |
| 485 | | Cl | $NH_2$ | 3-Py | $CH_2$ | 5,6-Dimethyl-4-bromopyridin-3-yl | H |
| 486 | | Cl | $NH_2$ | 3-Py | $CH_2$ | 5,6-Dimethyl-4-chloropyridin-3-yl | H |
| 487 | | Cl | $NH_2$ | 3-Py | $CH_2$ | 2,6-Dimethyl-3-methoxypyridin-4-yl | H |
| 488 | | Cl | $NH_2$ | 3-Py | $CH_2$ | 2,6-Dimethyl-pyridin-4-yl | H |
| 489 | | Cl | $NH_2$ | 3-Py | $CH_2$ | 2,3,6-Trimethyl-pyridin-4-yl | H. |
| 490 | | Cl | $NH_2$ | 3-Py | $CH_2$ | 2,3,6-Trimethoxy-pyridin-4-yl | H |
| 491 | | Cl | $NH_2$ | 3-Py | $CH_2$ | 2,6-Dimethyl-3-bromopyridin-4-yl | H |
| 492 | | Cl | $NH_2$ | 3-Py | $CH_2$ | 2,6-Dimethyl-3-chloropyridin-4-yl | H |
| 493 | | Cl | $NH_2$ | 3-Py | $CH_2$ | 4,6-Dimethyl-5-iodopyridin-3-yl | H |
| 494 | | Cl | $NH_2$ | 3-Py | $CH_2$ | 3,5-Dimethyl-4-aminopyridin-2-yl | H |
| 495 | | Cl | $NH_2$ | $DH_2$- $NMe_2$ | $CH_2$ | 3,5-Dimethyl-4-methoxypyridin-2-yl | H |
| 496 | | Cl | $NH_2$ | $CH_2$- $NMe_2$ | $CH_2$ | 3,5-Dimethyl-4-methoxy-1-oxypyridin-2-yl | H |
| 497 | | Cl | NH2 | $CH_2$- $NMe_2$ | $CH_2$ | 3,5-Dimethyl-4-bromopyridin-2-yl | H |

(continued)

| No. | Ex | $R^1$ | $R^2$ | $R^3$ | $R^4$ | $R^5$ | $R^0$ |
|---|---|---|---|---|---|---|---|
| 498 | | Cl | $NH_2$ | $CH_2$- $NMe_2$ | $CH_2$ | 3,5-Dimethyl-4-bromo-1-oxypyridin-2-yl | H |
| 499 | | Cl | $NH_2$ | $CH_2$- $NMe_2$ | $CH_2$ | 3,5-Dimethyl-4-chloropyridin-2-yl | H |
| 500 | | Cl | $NH_2$ | $CH_2$- $NMe_2$ | $CH_2$ | 3,5-Dimethyl-4-chloro-1-oxypyridin-2-yl | H |
| 501 | | Cl | $NH_2$ | $CH_2$- $NMe_2$ | $CH_2$ | 3,5-Dimethyl-4-iodopyridin-2-yl | H |
| 502 | | Cl | $NH_2$ | $CH_2$- $NMe_2$ | $CH_2$ | 3,5-Dimethyl-4-iodo-1-oxypyridin-2-yl | H |
| 503 | | Cl | $NH_2$ | $CH_2$- $NMe_2$ | $CH_2$ | 3,5-Dimethyl-4-thiomethyl-pyridin-2-yl | H |
| 504 | | Cl | $NH_2$ | $CH_2$- $NMe_2$ | $CH_2$ | 3,5-Dimethyl-4-thiomethyl-1-oxypyridin-2-yl | H |
| 505 | | Cl | $NH_2$ | $CH_2$- $NMe_2$ | $CH_2$ | 3,4,5-Trimethyl-pyridin-2-yl | H |
| 506 | | Cl | $NH_2$ | $CH_2$- $NMe_2$ | $CH_2$ | 3,4,5-Trimethyl-1-oxypyridin-2-yl | H |
| 507 | | Cl | $NH_2$ | $CH_2$- $NMe_2$ | $CH_2$ | 4,5,6-Trimethoxypyridin-2-yl | H |
| 508 | | Cl | $NH_2$ | $CH_2$- $NMe_2$ | $CH_2$ | 4,5,6-Trimethoxy-1-oxypyridin-2-yl | H |
| 509 | | Cl | $NH_2$ | $CH_2$- $NMe_2$ | $CH_2$ | 3,4,5-Trimethoxy-pyridin-2-yl | H |
| 510 | | Cl | $NH_2$ | $CH_2$- $NMe_2$ | $CH_2$ | 3,4,5-Trimethoxy-1-oxypyridin-2-yl | H |
| 511 | | Cl | $NH_2$ | $CH_2$- $NMe_2$ | $CH_2$ | 4,5,6-Trimethyl-pyridin-2-yl | H |
| 512 | | Cl | $NH_2$ | $CH_2$- $NMe_2$ | $CH_2$ | 4,5,6-Trimethyl-1-oxypyidin-2-yl | H |
| 513 | | Cl | $NH_2$ | $CH_2$- $NMe_2$ | $CH_2$ | 4,6-Dimethyl-5-methoxy-pyridin-2-yl | H |
| 514 | | Cl | $NH_2$ | $CH_2$- $NMe_2$ | $CH_2$ | 4,6-Dimethyl-5-methoxypyridin-3-yl | H |
| 515 | | Cl | $NH_2$ | $CH_2$- $NMe_2$ | $CH_2$ | 4,6-Dimethyl-5-methoxy-1-oxypyridin-3-yl | H |
| 516 | | Cl | $NH_2$ | $CH_2$- $NMe_2$ | $CH_2$ | 4,6-Dimethyl-5-bromopyridin-3-yl | H |
| 517 | | Cl | $NH_2$ | $CH_2$- $NMe_2$ | $CH_2$ | 4,6-Dimethyl-5-chloropyridin-3-yl | H |
| 518 | | Cl | $NH_2$ | $CH_2$- $NMe_2$ | $CH_2$ | 5,6-Dimethyl-4-bromopyridin-3-yl | H |
| 519 | | Cl | $NH_2$ | $CH_2$- $NMe_2$ | $CH_2$ | 5,6-Dimethyl-4-chloropyridin-3-yl | H |
| 520 | | Cl | $NH_2$ | $CH_2$- $NMe_2$ | $CH_2$ | 2,6-Dimethyl-3-methoxypyridin-4-yl | H |
| 521 | | Cl | $NH_2$ | $CH_2$- $NMe_2$ | $CH_2$ | 2,6-Dimethyl-pyridin-4-yl | H. |
| 522 | | Cl | $NH_2$ | $CH_2$- $NMe_2$ | $CH_2$ | 2,3,6-Trimethyl-pyridin-4-yl | H |
| 523 | | Cl | $NH_2$ | $CH_2$- $NMe_2$ | $CH_2$ | 2,3,6-Trimethoxy-pyridin-4-yl | H |
| 524 | | Cl | $NH_2$ | $CH_2$- $NMe_2$ | $CH_2$ | 2,6-Dimethyl-3-bromopyridin-4-yl | H |
| 525 | | Cl | $NH_2$ | $CH_2$- $NMe_2$ | $CH_2$ | 2,6-Dimethyl-3-chloropyridin-4-yl | H |
| 526 | | Cl | $NH_2$ | $CH_2$- $NMe_2$ | $CH_2$ | 4,6-Dimethyl-5-iodopyridin-3-yl | H |
| 527 | | Cl | $NH_2$ | $CH_2$- $NMe_2$ | $CH_2$ | 3,5-Dimethyl-4-aminopyridin-2-yl | H |
| 528 | | Cl | $NH_2$ | 2- furanyl | $CH_2$ | 3,5-Dimethyl-4-methoxypyridin-2-yl | H |
| 529 | | Cl | $NH_2$ | 2- furanyl | $CH_2$ | 3,5-Dimethyl-4-methoxy-1-oxypyridin-2-yl | H |
| 530 | | Cl | $NH_2$ | 2- furanyl | $CH_2$ | 3,5-Dimethyl-4-bromopyridin-2-yl | H |
| 531 | | Cl | $NH_2$ | 2-furanyl | $CH_2$ | 3,5-Dimethyl-4-bromo-1-oxypyridin-2-yl | H |
| 532 | | Cl | $NH_2$ | 2-furanyl | $CH_2$ | 3,5-Dimethyl-4-chloropyridin-2-yl | H |
| 533 | | Cl | $NH_2$ | 2-furanyl | $CH_2$ | 3,5-Dimethyl-4-chloro-1-oxypyridin-2-yl | H |
| 534 | | Cl | $CH_2$ | 2-furanyl | $CH_2$ | 3,5-Dimethyl-4-iodopyridin-2-yl | H |
| 535 | | Cl | $NH_2$ | 2- furanyl | $CH_2$ | 3,5-Dimethyl-4-iodo-1-oxypyridin-2-yl | H |

(continued)

| No. | Ex | $R^1$ | $R^2$ | $R^3$ | $R^4$ | $R^5$ | $R^0$ |
|---|---|---|---|---|---|---|---|
| 536 | | Cl | $NH_2$ | 2-furanyl | $CH_2$ | 3,5-Dimethyl-4-thiomethyl-pyridin-2-yl | H |
| 537 | | Cl | $NH_2$ | 2- furanyl | $CH_2$ | 3,5-Dimethyl-4-thiomethyl-1-oxypyridin-2-yl | H |
| 538 | | Cl | $NH_2$ | 2-furanyl | $CH_2$ | 3,4,5-Trimethyl-pyridin-2-yl | H |
| 539 | | Cl | $NH_2$ | 2-furanyl | $CH_2$ | 3,4,5-Trimethyl-1-oxypyridin-2-yl | H |
| 540 | | Cl | $NH_2$ | 2-furanyl | $CH_2$ | 4,5,6-Trimethoxypyridin-2-y1 | H |
| 541 | | Cl | $NH_2$ | 2-furanyl | $CH_2$ | 4,5,6-Trimethoxy-1-oxypyridin-2-yl | H |
| 542 | | Cl | $NH_2$ | 2-furanyl | $CH_2$ | 3,4,5-Trimethoxy-pyridin-2-yl | H |
| 543 | | Cl | $NH_2$ | 2- furanyl | $CH_2$ | 3,4,5-Trimethoxy-1-oxypyridin-2-yl | H |
| 544 | | Cl | $NH_2$ | 2- furanyl | $CH_2$ | 4,5,6-Trimethyl-pyridin-2-yl | H |
| 545 | | Cl | $NH_2$ | 2-furanyl | $CH_2$ | 4,5,6-Trimethyl-1-oxypyridin-2-yl | H |
| 546 | | Cl | $NH_2$ | 2-furanyl | $CH_2$ | 4,6-Dimethyl-5-methoxy-pyridin-2-yl | H |
| 547 | | Cl | $NH_2$ | 2- furanyl | $CH_2$ | 4,6-Dimethyl-5-methoxypyridin-3-yl | H |
| 548 | | Cl | $NH_2$ | 2-furanyl | $CH_2$ | 4,6-Dimethyl-5-methoxy-1-oxypyridin-3-yl | H |
| 549 | | Cl | $NH_2$ | 2-furanyl | $CH_2$ | 4,6-Dimethyl-5-bromopyridin-3-yl | H |
| 550 | | Cl | $NH_2$ | 2-furanyl | $CH_2$ | 4,6-Dimethyl-5-chloropyridin-3-yl | H |
| 551 | | Cl | $NH_2$ | 2-furanyl | $CH_2$ | 5,6-Dimethyl-4-bromopyridin-3-yl | H |
| 552 | | Cl | $NH_2$ | 2-furanyl | $CH_2$ | 5,6-Dimethyl-4-chloropyridin-3-yl | H |
| 553 | | Cl | $NH_2$ | 2- furanyl | $CH_2$ | 2,6-Dimethyl-3-methoxypyridin-4-yl | H |
| 554 | | Cl | $NH_2$ | 2- furanyl | $CH_2$ | 2,6-Dimethyl-pyridin-4-yl | H |
| 555 | | Cl | $NH_2$ | 2- furanyl | $CH_2$ | 2,3,6-Trimethyl-pyridin-4-yl | H |
| 556 | | Cl | $NH_2$ | 2-furanyl | $CH_2$ | 2,3,6-Trimethoxy-pyridin-4-yl | H |
| 557 | | Cl | $NH_2$ | 2-furanyl | $CH_2$ | 2,6-Dimethyl-3-bromopyridin-4-yl | H |
| 558 | | Cl | $NH_2$ | 2- furanyl | $CH_2$ | 2,6-Dimethyl-3-chloropyridin-4-yl | H |
| 559 | | Cl | $NH_2$ | 2-furanyl | $CH_2$ | 3,5-Dimethyl-4-aminopyridin-2-yl | H |
| 560 | | Cl | $NH_2$ | Cl | $CH_2$ | 3,5-Dimethyl-4-methoxypyridin-2-yl | H |
| 561 | | Cl | $NH_2$ | Cl | $CH_2$ | 3,5-Dimethyl-4-methoxy-1-oxypyridin-2-yl | H |
| 562 | | Cl | $NH_2$ | Cl | $CH_2$ | 3,5-Dimethyl-4-bromopyridin-2-yl | H. |
| 563 | | Cl | $NH_2$ | Cl | $CH_2$ | 3,5-Dimethyl-4-bromo-1-oxypyridin-2-yl | H |
| 564 | | Cl | $NH_2$ | Cl | $CH_2$ | 3,5-Dimethyl-4-chloropyridin-2-yl | H |
| 565 | | Cl | $NH_2$ | Cl | $CH_2$ | 3,5-Dimethyl-4-chloro-1-oxypyridin-2-yl | H |
| 566 | | Cl | $NH_2$ | Cl | $CH_2$ | 3,5-Dimethyl-4-iodopyridin-2-yl | H |
| 567 | | Cl | $NH_2$ | Cl | $CH_2$ | 3,5-Dimethyl-4-iodo-1-oxypyridin-2-yl | H |
| 568 | | Cl | $NH_2$ | Cl | $CH_2$ | 3,5-Dimethyl-4-thiomethyl-pyridin-2-yl | H |
| 569 | | Cl | $NH_2$ | Cl | $CH_2$ | 3,5-Dimethyl-4-thiomethyl-1-oxypyridin-2-yl | H |
| 570 | | Cl | $NH_2$ | Cl | $CH_2$ | 3,4,5-Trimethyl-pyridin-2-yl | H |
| 571 | | Cl | $NH_2$ | Cl | $CH_2$ | 3,4,5-Trimethyl-1-oxypyridin-2-yl | H |
| 572 | | Cl | $NH_2$ | Cl | $CH_2$ | 4,6-Dimethyl-5-methoxypyridin-3-yl | H |
| 573 | | Cl | $NH_2$ | Cl | $CH_2$ | 4,6-Dimethyl-5-methoxy-1-oxypyridin-3-yl | H |

(continued)

| No. | Ex | $R^1$ | $R^2$ | $R^3$ | $R^4$ | $R^5$ | $R^0$ |
|---|---|---|---|---|---|---|---|
| 574 | | Cl | $NH_2$ | Cl | $CH_2$ | 3,5-Dimethyl-4-aminopyridin-2-yl | H |
| 575 | | Cl | $NH_2$ | Br | $CH_2$ | 3,5-Dimethyl-4-methoxypyridin-2-yl | H |
| 576 | | Cl | $NH_2$ | Br | $CH_2$ | 3,5-Dimethyl-4-methoxy-1-oxypyridin-2-yl | H |
| 577 | | Cl | $NH_2$ | Br | $CH_2$ | 3,5-Dimethyl-4-bromopyridin-2-yl | H |
| 578 | | Br | $NH_2$ | Br | $CH_2$ | 3,5-Dimethyl-4-bromo-1-oxypyridin-2-yl | H |
| 579 | | Cl | $NH_2$ | Br | $CH_2$ | 3,5-Dimethyl-4-chloropyridin-2-yl | H |
| 580 | | Br | $NH_2$ | Br | $CH_2$ | 3,5-Dimethyl-4-chloro-1-oxypyridin-2-yl | H |
| 581 | | Cl | $NH_2$ | Br | $CH_2$ | 3,5-Dimethyl-4-iodopyridin-2-yl | H |
| 582 | | Br | $NH_2$ | Br | $CH_2$ | 3,5-Dimethyl-4-iodo-1-oxypyridin-2-yl | H |
| 583 | | Cl | $NH_2$ | Br | $CH_2$ | 3,5-Dimethyl-4-thiomethyl-pyridin-2-yl | H |
| 584 | | Br | $NH_2$ | Br | $CH_2$ | 3,5-Dimethyl-4-thiomethyl-1-oxypyridin-2-yl | H |
| 585 | | Cl | $NH_2$ | Br | $CH_2$ | 3,4,5-Trimethyl-pyridin-2-yl | H |
| 586 | | Br | $NH_2$ | Br | $CH_2$ | 3,4,5-Trimethyl-1-oxypyridin-2-yl | H |
| 587 | | Cl | $NH_2$ | Br | $CH_2$ | 4,6-Dimethyl-5-methoxypyridin-3-yl | H |
| 588 | | Cl | $NH_2$ | Br | $CH_2$ | 4,6-Dimethyl-5-methoxy-1-oxypyridin-3-yl | H |
| 589 | | Cl | $NH_2$ | Br | $CH_2$ | 3,5-Dimethyl-4-aminopyridin-2-yl | H |
| 590 | | Cl | $NH_2$ | I | $CH_2$ | 3,5-Dimethyl-4-methoxypyridin-2-yl | H |
| 591 | | Cl | $NH_2$ | I | $CH_2$ | 3,5-Dimethyl-4-methoxy-1-oxypyridin-2-yl | H |
| 592 | | Cl | $NH_2$ | I | $CH_2$ | 3,5-Dimethyl-4-bromopyridin-2-yl | H |
| 593 | | Cl | $NH_2$ | I | $CH_2$ | 3,5-Dimethyl-4-bromo-1-oxypyridin-2-yl | H |
| 594 | | Cl | $NH_2$ | I | $CH_2$ | 3,5-Dimethyl-4-chloropyridin-2-yl | H |
| 595 | | Cl | $NH_2$ | I | $CH_2$ | 3,5-Dimethyl-4-chloro-1-oxypyridin-2-yl | H |
| 596 | | Cl | $NH_2$ | I | $CH_2$ | 3,5-Dimethyl-4-iodopyridin-2-yl | H |
| 597 | | Cl | $NH_2$ | I | $CH_2$ | 3,5-Dimethyl-4-iodo-1-oxypyridin-2-yl | H |
| 598 | | Cl | $NH_2$ | I | $CH_2$ | 3,5-Dimethyl-4-thiomethyl-pyridin-2-yl | H |
| 599 | | Cl | $NH_2$ | I | $CH_2$ | 3,5-Dimethyl-4-thiomethyl-1-oxypyridin-2-yl | H |
| 600 | | Cl | $NH_2$ | I | $CH_2$ | 3,4,5-Trimethyl-pyridin-2-yl | H |
| 601 | | Cl | $NH_2$ | I | $CH_2$ | 3,4,5-Trimethyl-1-oxypyridin-2-yl | H |
| 602 | | Cl | $NH_2$ | I | $CH_2$ | 4,6-Dimethyl-5-methoxypyridin-3-yl | H. |
| 603 | | Cl | $NH_2$ | I | $CH_2$ | 4,6-Dimethyl-5-methoxy-1-oxypyridin-3-yl | H |
| 604 | | Cl | $NH_2$ | I | $CH_2$ | 3,5-Dimethyl-4-aminopyridin-2-yl | H |
| 605 | | Cl | $NH_2$ | CN | $CH_2$ | 3,5-Dimethyl-4-methoxypyridin-2-yl | H |
| 606 | | Cl | $NH_2$ | CN | $CH_2$ | 3,5-Dimethyl-4-methoxy-1-oxypyridin-2-yl | H |
| 607 | | Cl | $NH_2$ | CN | $CH_2$ | 3,5-Dimethyl-4-bromopyridin-2-yl | H |
| 608 | | Cl | $NH_2$ | CN | $CH_2$ | 3,5-Dimethyl-4-bromo-1-oxypyridin-2-yl | H |
| 609 | | Cl | $NH_2$ | CN | $CH_2$ | 3,5-Dimethyl-4-chloropyridin-2-yl | H |
| 610 | | Cl | $NH_2$ | CN | $CH_2$ | 3,5-Dimethyl-4-chloro-1-oxypyridin-2-yl | H |
| 611 | | Cl | $NH_2$ | CN | $CH_2$ | 3,5-Dimethyl-4-iodopyridin-2-yl | H |

(continued)

| No. | Ex | $R^1$ | $R^2$ | $R^3$ | $R^4$ | $R^5$ | $R^0$ |
|---|---|---|---|---|---|---|---|
| 612 | | Cl | $NH_2$ | CN | $CH_2$ | 3,5-Dimethyl-4-iodo-1-oxypyridin-2-yl | H |
| 613 | | Cl | $NH_2$ | CN | $CH_2$ | 3,5-Dimethyl-4-thiomethyl-pyridin-2-yl | H |
| 614 | | Cl | $NH_2$ | CN | $CH_2$ | 3,5-Dimethyl-4-thiomethyl-1-oxypyridin-2-yl | H |
| 615 | | Cl | $NH_2$ | CN | $CH_2$ | 3,4,5-Trimethyl-pyridin-2-yl | H. |
| 616 | | Cl | $NH_2$ | CN | $CH_2$ | 3,4,5-Trimethyl-1-oxypyridin-2-yl | H |
| 617 | | Cl | $NH_2$ | CN | $CH_2$ | 4,6-Dimethyl-5-methoxypyridin-3-yl | H |
| 618 | | Cl | $CH_2$ | CN | $CH_2$ | 4,6-Dimethyl-5-methoxy-1-oxypyridin-3-yl | H |
| 619 | | Cl | $NH_2$ | CN | $CH_2$ | 3,5-Dimethyl-4-aminopyridin-2-yl | H |
| 620 | | Cl | $NH_2$ | H | C(O) | 3,5-Dimethyl-4-methoxypyridin-2-yl | H |
| 621 | | Cl | $NH_2$ | H | C(O) | 3,5-Dimethyl-4-methoxy-1-oxypyridin-2-yl | H |
| 622 | | Cl | $NH_2$ | H | C(O) | 3,5-Dimethyl-4-bromopyridin-2-yl | H |
| 623 | | Cl | $NH_2$ | H | C(O) | 3,5-Dimethyl-4-bromo-1-oxypyridin-2-yl | H |
| 624 | | Cl | $NH_2$ | H | C(O) | 3,5-Dimethyl-4-chloropyridin-2-yl | H |
| 625 | | Cl | $NH_2$ | H | C(O) | 3,5-Dimethyl-4-chloro-1-oxypyridin-2-yl | H |
| 626 | | Cl | $NH_2$ | H | C(O) | 3,5-Dimethyl-4-iodopyridin-2-yl | H |
| 627 | | Cl | $NH_2$ | H | C(O) | 3,5-Dimethyl-4-iodo-1-oxypyridin-2-yl | H |
| 628 | | Cl | $NH_2$ | H | C(O) | 3,5-Dimethyl-4-thiomethyl-pyridin-2-yl | H |
| 629 | | Cl | $NH_2$ | H | C(O) | 3,5-Dimethyl-4-thiomethyl-1-oxypyridin-2-yl | H |
| 630 | | Cl | $NH_2$ | H | C(O) | 3,4,5-Trimethyl-pyridin-2-yl | H |
| 631 | | Cl | $NH_2$ | H | C(O) | 3,4,5-Trimethyl-1-oxypyridin-2-yl | H |
| 632 | | Cl | $NH_2$ | H | C(O) | 4,6-Dimethyl-5-methoxypyridin-3-yl | H |
| 633 | | Cl | $NH_2$ | H | C(O) | 4,6-Dimethyl-5-methoxy-1-oxypyridin-3-yl | H |
| 634 | | Cl | $NH_2$ | H | C(O) | 3,5-Dimethyl-4-aminopyridin-2-yl | H |
| 635 | | Cl | $NH_2$ | H | S(O) | 3,5-Dimethyl-4-methoxypyridin-2-yl | H |
| 636 | | Cl | $NH_2$ | H | S(O) | 3,5-Dimethyl-4-methoxy-1-oxypyridin-2-yl | H |
| 637 | | Cl | $NH_2$ | H | S(O) | 3,5-Dimethyl-4-bromopyridin-2-yl | H |
| 638 | | Cl | $NH_2$ | H | S(O) | 3,5-Dimethyl-4-bromo-1-oxypyridin-2-yl | H |
| 639 | | Cl | $NH_2$ | H | S(O) | 3,5-Dimethyl-4-chloropyridin-2-yl | H |
| 640 | | Cl | $NH_2$ | H | S(O) | 3,5-Dimethyl-4-chloro-1-oxypyridin-2-yl | H |
| 641 | | Cl | $NH_2$ | H | S(O) | 3,5-Dimethyl-4-iodopyridin-2-yl | H |
| 642 | | Cl | $NH_2$ | H | S(O) | 3,5-Dimethyl-4-iodo-1-oxypyridin-2-yl | H. |
| 643 | | Cl | $NH_2$ | H | S(O) | 3,5-Dimethyl-4-thiomethyl-pyridin-2-yl | H |
| 644 | | Cl | $NH_2$ | H | S(O) | 3,5-Dimethyl-4-thiomethyl-1-oxypyridin-2-yl | H |
| 645 | | Cl | $NH_2$ | Br | S(O) | 3,4,5-Trimethyl-pyridin-2-yl | H |
| 646 | | Cl | $NH_2$ | H | S(O) | 3,4,5-Trimethyl-1-oxypyridin-2-yl | H |
| 647 | | Cl | $NH_2$ | Br | S(O) | 4,6-Dimethyl-5-methoxypyridin-3-yl | H |
| 648 | | Cl | $NH_2$ | H | S(O) | 4,6-Dimethyl-5-methoxy-1-oxypyridin-3-yl | H |
| 649 | | Cl | $NH_2$ | H | $SO_2$ | 3,5-Dimethyl-4-methoxypyridin-2-yl | H |

(continued)

| No. | Ex | $R^1$ | $R^2$ | $R^3$ | $R^4$ | $R^5$ | $R^0$ |
|---|---|---|---|---|---|---|---|
| 650 | | Cl | $NH_2$ | H | $SO_2$ | 3,5-Dimethyl-4-methoxy-1-oxypyridin-2-yl | H |
| 651 | | Cl | $NH_2$ | H | $SO_2$ | 3,5-Dimethyl-4-bromopyridin-2-yl | H |
| 652 | | Cl | $NH_2$ | H | $SO_2$ | 3,5-Dimethyl-4-bromo-1-oxypyridin-2-yl | H |
| 653 | | Cl | $NH_2$ | Br | $SO_2$ | 3,5-Dimethyl-4-chloropyridin-2-yl | H |
| 654 | | Cl | $NH_2$ | H | $SO_2$ | 3,5-Dimethyl-4-chloro-1-oxypyridin-2-yl | H |
| 655 | | Cl | $NH_2$ | H | $SO_2$ | 3,5-Dimethyl-4-iodopyridin-2-yl | H. |
| 656 | | Cl | $NH_2$ | H | $SO_2$ | 3,5-Dimethyl-4-iodo-1-oxypyridin-2-yl | H |
| 657 | | Cl | $NH_2$ | H | $SO_2$ | 3,5-Dimethyl-4-thiomethyl-pyridin-2-yl | H |
| 658 | | Cl | $NH_2$ | H | $SO_2$ | 3,5-Dimethyl-4-thiomethyl-1-oxypyridin-2-yl | H |
| 659 | | Cl | $NH_2$ | H | $SO_2$ | 3,4,5-Trimethyl-pyridin-2-yl | H |
| 660 | | Cl | $NH_2$ | H | $SO_2$ | 3,4,5-Trimethyl-1-oxypyridin-2-yl | H |
| 661 | | Cl | $NH_2$ | H | $SO_2$ | 4,6-Dimethyl-5-methoxypyridin-3-yl | H |
| 662 | | Cl | $NH_2$ | H | $SO_2$ | 4,6-Dimethyl-5-methoxy-1-oxypyridin-3-yl | H |
| 663 | | Cl | $NH_2$ | i-pr | C(O) | 3,5-Dimethyl-4-methoxypyridin-2-yl | H |
| 664 | | Cl | $NH_2$ | i-pr | C(O) | 3,5-Dimethyl-4-methoxy-1-oxypyridin-2-yl | H |
| 665 | | Cl | $NH_2$ | i-pr | C(O) | 3,5-Dimethyl-4-bromopyridin-2-yl | H |
| 666 | | Cl | $NH_2$ | i-pr | C(O) | 3,5-Dimethyl-4-bromo-1-oxypyridin-2-yl | H |
| 667 | | Cl | $NH_2$ | i-pr | C(O) | 3,5-Dimethyl-4-chloropyridin-2-yl | H |
| 668 | | Cl | $NH_2$ | i-pr | C(O) | 3,5-Dimethyl-4-chloro-1-oxypyridin-2-yl | H |
| 669 | | Cl | $NH_2$ | i-pr | C(O) | 3,5-Dimethyl-4-iodopyridin-2-yl | H |
| 670 | | Cl | $NH_2$ | i-pr | C(O) | 3,5-Dimethyl-4-iodo-1-oxypyridin-2-yl | H |
| 671 | | Cl | $NH_2$ | i-pr | C(O) | 3,5-Dimethyl-4-thiomethyl-pyridin-2-yl | H |
| 672 | | Cl | $NH_2$ | i-pr | C(O) | 3,5-Dimethyl-4-thiomethyl-1-oxypyridin-2-yl | H |
| 673 | | Cl | $NH_2$ | i-pr | C(O) | 3,4,5-Trimethyl-pyridin-2-yl | H |
| 674 | | Cl | $NH_2$ | i-pr | C(O) | 3,4,5-Trimethyl-1-oxypyridin-2-yl | H |
| 675 | | Cl | $NH_2$ | i-pr | C(O) | 4,6-Dimethyl-5-methoxypyridin-3-yl | H |
| 676 | | Cl | $NH_2$ | i-pr | C(O) | 4,6-Dimethyl-5-methoxy-1-oxypyridin-3-yl | H |
| 677 | | Cl | $NH_2$ | i-pr | C(O) | 3,5-Dimethyl-4-aminopyridin-2-yl | H |
| 678 | | Cl | $NH_2$ | i-pr | S(O) | 3,5-Dimethyl-4-methoxypyridin-2-yl | H |
| 679 | | Cl | $NH_2$ | i-pr | S(O) | 3,5-Dimethyl-4-methoxy-1-oxypyridin-2-yl | H |
| 680 | | Cl | $NH_2$ | i-pr | S(O) | 3,5-Dimethyl-4-bromopyridin-2-yl | H |
| 681 | | Cl | $NH_2$ | i-pr | S(O) | 3,5-Dimethyl-4-bromo-1-oxypyridin-2-yl | H |
| 682 | | Cl | $NH_2$ | i-pr | S(O) | 3,5-Dimethyl-4-chloropyridin-2-yl | H |
| 683 | | Cl | $NH_2$ | i-pr | S(O) | 3,5-Dimethyl-4-chloro-1-oxypyridin-2-yl | H |
| 684 | | Cl | $NH_2$ | i-pr | S(O) | 3,5-Dimethyl-4-iodopyridin-2-yl | H |
| 685 | | Cl | $NH_2$ | i-pr | S(O) | 3,5-Dimethyl-4-iodo-1-oxypyridin-2-yl | H |
| 686 | | Cl | $NH_2$ | i-pr | S(O) | 3,5-Dimethyl-4-thiomethyl-pyridin-2-yl | H |
| 687 | | Cl | $NH_2$ | i-pr | S(O) | 3,5-Dimethyl-4-thiomethyl-1-oxypyridin-2-yl | H |

(continued)

| No. | Ex | $R^1$ | $R^2$ | $R^3$ | $R^4$ | $R^5$ | $R^0$ |
|---|---|---|---|---|---|---|---|
| 688 | | Cl | $NH_2$ | i-pr | S(O) | 3,4,5-Trimethyl-pyridin-2-yl | H |
| 689 | | Cl | $NH_2$ | i-pr | S(O) | 3,4,5-Trimethyl-1-oxypyridin-2-yl | H |
| 690 | | Cl | $NH_2$ | i-pr | S(O) | 4,6-Dimethyl-5-methoxypyridin-3-yl | H |
| 691 | | Cl | $NH_2$ | i-pr | S(O) | 4,6-Dimethyl-5-methoxy-1-oxypyridin-3-yl | H |
| 692 | | Cl | $NH_2$ | i-pr | $SO_2$ | 3,5-Dimethyl-4-methoxypyridin-2-yl | H |
| 693 | | Cl | $NH_2$ | i-pr | $SO_2$ | 3,5-Dimethyl-4-methoxy-1-oxypyridin-2-yl | H |
| 694 | | Cl | $NH_2$ | i-pr | $SO_2$ | 3,5-Dimethyl-4-bromopyridin-2-yl | H |
| 695 | | Cl | $NH_2$ | i-pr | $SO_2$ | 3,5-Dimethyl-4-bromo-1-oxypyridin-2-yl | H |
| 696 | | Cl | $NH_2$ | i-pr | $SO_2$ | 3,5-Dimethyl-4-chloropyridin-2-yl | H |
| 697 | | Cl | $NH_2$ | i-pr | $SO_2$ | 3,5-Dimethyl-4-chloro-1-oxypyridin-2-yl | H |
| 698 | | Cl | $NH_2$ | i-pr | $SO_2$ | 3,5-Dimethyl-4-iodopyridin-2-yl | H |
| 699 | | Cl | $NH_2$ | i-pr | $SO_2$ | 3,5-Dimethyl-4-iodo-1-oxypyridin-2-yl | H |
| 700 | | Cl | $NH_2$ | i-pr | $SO_2$ | 3,5-Dimethyl-4-thiomethyl-pyridin-2-yl | H |
| 701 | | Cl | $NH_2$ | i-pr | $SO_2$ | 3,5-Dimethyl-4-thiomethyl-1-oxypyridin-2-yl | H |
| 702 | | Cl | $NH_2$ | i-pr | $SO_2$ | 3,4,5-Trimethyl-pyridin-2-yl | H |
| 703 | | Cl | $NH_2$ | i-pr | $SO_2$ | 3,4,5-Trimethyl-1-oxypyridin-2-yl | H |
| 704 | | Cl | $NH_2$ | i-pr | $SO_2$ | 4,6-Dimethyl-5-methoxypyridin-3-yl | H |
| 705 | | Cl | $NH_2$ | i-pr | $SO_2$ | 4,6-Dimethyl-5-methoxy-1-oxypyridin-3-yl | H |
| 706 | | Cl | $NH_2$ | H | C(O) | 3,4,5-Trimethoxyphenyl | H |
| 707 | | Cl | $NH_2$ | H | C(O) | 2-Chloro-3,4,5-trimethoxyphenyl | H |
| 708 | | Cl | $NH_2$ | H | C(O) | 2-Bromo-3,4,5-trimethoxyphenyl | H |
| 709 | | Cl | $NH_2$ | H | C(O) | 3,5-Dimethyl-4-methoxyphenyl | H |
| 710 | | Cl | $NH_2$ | H | C(O) | 2-Chloro-3,5-Dimethyl-4-methoxyphenyl | H |
| 711 | | Cl | $NH_2$ | H | C(O) | 2-Bromo-3,5-Dimethyl-4-methoxyphenyl | H |
| 712 | | Cl | $NH_2$ | H | $SO_2$ | 3,4,5-Trimethoxyphenyl | H |
| 713 | | Cl | $NH_2$ | H | $SO_2$ | 2-Chloro-3,4,5-trimethoxyphenyl | H |
| 714 | | Cl | $NH_2$ | H | $SO_2$ | 2-Bromo-3,4,5-trimethoxyphenyl | H |
| 715 | | Cl | $NH_2$ | H | $SO_2$ | 3,5-Dimethyl-4-methoxyphenyl | H |
| 716 | | Cl | $NH_2$ | H | $SO_2$ | 2-Chloro-3,5-Dimethyl-4-methoxyphenyl | H |
| 717 | | Cl | $NH_2$ | H | $SO_2$ | 2-Bromo-3,5-Dimethyl-4-methoxyphenyl | H |
| 718 | | Cl | $NH_2$ | H | $CH_2$ | 3,5-Dimethyl-4-methoxypyridin-2-yl | Br |
| 719 | | Cl | $NH_2$ | H | $CH_2$ | 3,5-Dimethyl-4-methoxy-1-oxypyridin-2-yl | Br |
| 720 | | Cl | $NH_2$ | H | $CH_2$ | 3,5-Dimethyl-4-bromopyridin-2-yl | Br |
| 721 | | Cl | $NH_2$ | H | $CH_2$ | 3,5-Dimethyl-4-bromo-1-oxypyridin-2-yl | Br |
| 722 | | Cl | $NH_2$ | H | $CH_2$ | 3,5-Dimethyl-4-chloropyridin-2-yl | Br |
| 723 | | Cl | $NH_2$ | H | $CH_2$ | 3,5-Dimethyl-4-chloro-1-oxypyridin-2-yl | Br |
| 724 | | Cl | $NH_2$ | H | $CH_2$ | 3,5-Dimethyl-4-iodopyridin-2-yl | Br |
| 725 | | Cl | $NH_2$ | H | $CH_2$ | 3,5-Dimethyl-4-iodo-1-oxypyridin-2-yl | Br |

(continued)

| No. | Ex | $R^1$ | $R^2$ | $R^3$ | $R^4$ | $R^5$ | $R^0$ |
|---|---|---|---|---|---|---|---|
| 726 | | Cl | $NH_2$ | H | $CH_2$ | 3,5-Dimethyl-4-thiomethyl-pyridin-2-yl | Br |
| 727 | | Cl | $NH_2$ | H | $CH_2$ | 3,5-Dimethyl-4-thiomethyl-1-oxypyridin-2-yl | Br. |
| 728 | | Cl | $NH_2$ | H | $CH_2$ | 3,4,5-Trimethyl-pyridin-2-yl | Br |
| 729 | | Cl | $NH_2$ | H | $CH_2$ | 3,4,5-Trimethyl-1-oxypyridin-2-yl | Br |
| 730 | | Cl | $NH_2$ | H | $CH_2$ | 4,6-Dimethyl-5-methoxypyridin-3-yl | Br |
| 731 | | Cl | $NH_2$ | H | $CH_2$ | 4,6-Dimethyl-5-methoxy-1-oxypyridin-3-yl | Br |
| 732 | | Cl | $NH_2$ | H | $CH_2$ | 3,5-Dimethyl-4-methoxypyridin-2-yl | Cl |
| 733 | | Cl | $NH_2$ | H | $CH_2$ | 3,5-Dimethyl-4-methoxy-1-oxypyridin-2-yl | Cl |
| 734 | | Cl | $NH_2$ | H | $CH_2$ | 3,5-Dimethyl-4-bromopyridin-2-yl | Cl |
| 735 | | Cl | $NH_2$ | H | $CH_2$ | 3,5-Dimethyl-4-bromo-1-oxypyridin-2-yl | Cl |
| 736 | | Cl | $NH_2$ | H | $CH_2$ | 3,5-Dimethyl-4-chloropyridin-2-yl | Cl |
| 737 | | Cl | $NH_2$ | H | $CH_2$ | 3,5-Dimethyl-4-chloro-1-oxypyridin-2-yl | Cl |
| 738 | | Cl | $NH_2$ | H | $CH_2$ | 3,5-Dimethyl-4-iodopyridin-2-yl | Cl |
| 739 | | Cl | $NH_2$ | H | $CH_2$ | 3,5-Dimethyl-4-iodo-1-oxypyridin-2-yl | Cl |
| 740 | | Cl | $NH_2$ | H | $CH_2$ | 3,5-Dimethyl-4-thiomethyl-pyridin-2-yl | Cl |
| 741 | | Cl | $NH_2$ | H | $CH_2$ | 3,5-Dimethyl-4-thiomethyl-1-oxypyridin-2-yl | Cl |
| 742 | | Cl | $NH_2$ | H | $CH_2$ | 3,4,5-Trimethyl-pyridin-2-yl | Cl |
| 743 | | Cl | $NH_2$ | H | $CH_2$ | 3,4,5-Trimethyl-1-oxypyridin-2-yl | Cl |
| 744 | | Cl | $NH_2$ | H | $CH_2$ | 4,6-Dimethyl-5-methoxypyridin-3-yl | Cl |
| 745 | | Cl | $NH_2$ | H | $CH_2$ | 4,6-Dimethyl-5-methoxy-1-oxypyridin-3-yl | Cl |
| 746 | | Cl | $NH_2$ | H | $CH_2$ | 3,5-Dimethyl-4-methoxypyridin-2-yl | CN |
| 747 | | Cl | $NH_2$ | H | $CH_2$ | 3,5-Dimethyl-4-methoxy-1-oxypyridin-2-yl | CN |
| 748 | | Cl | $NH_2$ | H | $CH_2$ | 3,5-Dimethyl-4-bromopyridin-2-yl | CN |
| 749 | | Cl | $NH_2$ | H | $CH_2$ | 3,5-Dimethyl-4-bromo-1-oxypyridin-2-yl | CN |
| 750 | | Cl | $NH_2$ | H | $CH_2$ | 3,5-Dimethyl-4-chloropyridin-2-yl | CN |
| 751 | | Cl | $NH_2$ | H | $CH_2$ | 3,5-Dimethyl-4-chloro-1-oxypyridin-2-yl | CN |
| 752 | | Cl | $NH_2$ | H | $CH_2$ | 3,5-Dimethyl-4-iodopyridin-2-yl | CN |
| 753 | 25 | Br | $NH_2$ | H | $CH_2$ | 3,5-Dimethyl-4-methoxypyridin-2-yl | H |
| 754 | 20 | Br | $NH_2$ | H | $CH_2$ | 3,5-Dimethyl-4-methoxy-1-oxypyridin-2-yl | H |
| 755 | | Br | $NH_2$ | H | $CH_2$ | 6-Bromo-3,5-dimethyl-4-methoxypyridin-2-yl | H |
| 756 | | Br | $NH_2$ | H | $CH_2$ | 6-Chloro-3,5-dimethyl-4-methoxypyridin-2-yl | H |
| 757 | 23 | Br | $NH_2$ | H | $CH_2$ | 3,5-Dimethyl-4-bromopyridin-2-yl | H |
| 758 | 24 | Br | $NH_2$ | H | $CH_2$ | 3,5-Dimethyl-4-bromo-1-oxypyridin-2-yl | H |
| 759 | 21 | Br | $NH_2$ | H | $CH_2$ | 3,5-Dimethyl-4-chloropyridin-2-yl | H |
| 760 | 22 | Br | $NH_2$ | H | $CH_2$ | 3,5-Dimethyl-4-chloro-1-oxypyridin-2-yl | H |
| 761 | | Br | $NH_2$ | H | $CH_2$ | 3,5-Dimethyl-4-iodopyridin-2-yl | H |
| 762 | | Br | $NH_2$ | H | $CH_2$ | 3,5-Dimethyl-4-iodo-1-oxypyridin-2-yl | H |
| 763 | | Br | $NH_2$ | H | $CH_2$ | 3,5-Dimethyl-4-thiomethyl-pyridin-2-yl | H |

(continued)

| No. | Ex | $R^1$ | $R^2$ | $R^3$ | $R^4$ | $R^5$ | $R^0$ |
|---|---|---|---|---|---|---|---|
| 764 | | Br | $NH_2$ | H | $CH_2$ | 3,5-Dimethyl-4-thiomethyl-1-oxypyridin-2-yl | H |
| 765 | | Br | $NH_2$ | H | $CH_2$ | 3,4,5-Trimethyl-pyridin-2-yl | H |
| 766 | | Br | $NH_2$ | H | $CH_2$ | 3,4,5-Trimethyl-1-oxypyridin-2-yl | H |
| 767 | | Br | $NH_2$ | H | $CH_2$ | 4,5,6-Trimethoxypyridin-2-yl | H |
| 768 | | Br | $NH_2$ | H | $CH_2$ | 4,5,6-Trimethoxy-1-oxypyridin-2-yl | H |
| 769 | | Br | $NH_2$ | H | $CH_2$ | 3-Bromo-4,5,6-trimethoxypyridin-2-yl | H |
| 770 | | Br | $NH_2$ | H | $CH_2$ | 3-Chloro-4,5,6-trimethoxypyridin-2-yl | H |
| 771 | | Br | $NH_2$ | H | $CH_2$ | 3,4,5-Trimethoxy-pyridin-2-yl | H |
| 772 | | Br | $NH_2$ | H | $CH_2$ | 3,4,5-Trimethoxy-1-oxypyridin-2-yl | H |
| 773 | | Br | $NH_2$ | H | $CH_2$ | 3-Bromo-3,4,5-trimethoxy-pyridin-2-yl | H |
| 774 | | Br | $NH_2$ | H | $CH_2$ | 3-Chloro-3,4,5-trimethoxy-pyridin-2-yl | H |
| 775 | | Br | $NH_2$ | H | $CH_2$ | 4,5,6-Trimethyl-pyridin-2-yl | H |
| 776 | | Br | $NH_2$ | H | $CH_2$ | 4,5,6-Trimethyl-l-oxypyridin-2-yl | H |
| 777 | | Br | $NH_2$ | H | $CH_2$ | 4,6-Dimethyl-5-methoxy-pyridin-2-yl | H |
| 778 | | Br | $NH_2$ | H | $CH_2$ | 4,6-Dimethyl-5-methoxypyridin-3-yl | H |
| 779 | | Br | $NH_2$ | H | $CH_2$ | 4,6-Dimethyl-5-methoxy-1-oxypyridin-3-yl | H |
| 780 | | Br | $NH_2$ | H | $CH_2$ | 4,6-Dimethyl-5-bromopyridin-3-yl | H |
| 781 | | Br | $NH_2$ | H | $CH_2$ | 4,6-Dimethyl-5-chloropyridin-3-yl | H |
| 782 | | Br | $NH_2$ | H | $CH_2$ | 5,6-Dimethyl-4-bromopyridin-3-yl | H |
| 783 | | Br | $NH_2$ | H | $CH_2$ | 5,6-Dimethyl-4-chloropyridin-3-yl | H |
| 784 | | Br | $NH_2$ | H | $CH_2$ | 2,6-Dimethyl-3-methoxypyridin-4-yl | H |
| 785 | | Br | $NH_2$ | H | $CH_2$ | 2,6-Dimethyl-pyridin-4-yl | H |
| 786 | | Br | $NH_2$ | H | $CH_2$ | 2,3,6-Trimethyl-pyridin-4-yl | H |
| 787 | | Br | $NH_2$ | H | $CH_2$ | 2,3,6-Trimethoxy-pyridin-4-yl | H |
| 788 | | Br | $NH_2$ | H | $CH_2$ | 2,6-Dimethyl-3-bromopyridin-4-yl | H |
| 789 | | Br | $NH_2$ | H | $CH_2$ | 2,6-Dimethyl-3-chloropyridin-4-yl | H |
| 790 | | Br | $NH_2$ | H | $CH_2$ | 2,6-Dimethyl-3-methoxy-1-oxy-pyridin-4-yl | H |
| 791 | | Br | $NH_2$ | H | $CH_2$ | 2,6-Dimethyl-1-oxy-pyidin-4-yl | H |
| 792 | | Br | $NH_2$ | H | $CH_2$ | 2,3,6-Trimethyl-1-oxy-pyridin-4-yl | H |
| 793 | | Br | $NH_2$ | H | $CH_2$ | 2,3,6-Trimethoxy-1-oxy-pyridin-4-yl | H |
| 794 | | Br | $NH_2$ | H | $CH_2$ | 2,6-Dimethyl-3-bromol-oxy-pyridin-4-yl | H |
| 795 | | Br | $NH_2$ | H | $CH_2$ | 2,6-Dimethyl-3-chlorol-oxy-pyridin-4-yl | H |
| 796 | | Br | $NH_2$ | H | $CH_2$ | 4,6-Dimethyl-5-iodopyridin-3-yl | H |
| 797 | | Br | $NH_2$ | H | $CH_2$ | 3,5-Dimethyl-4-aminopyridin-2-yl | H |
| 798 | | Br | $NH_2$ | i-pr | $CH_2$ | 3,5-Dimethyl-4-methoxypyridin-2-yl | H |
| 799 | | Br | $NH_2$ | i-pr | $CH_2$ | 3,5-Dimethyl-4-methoxy-1-oxypyridin-2-yl | H |
| 800 | | Br | $NH_2$ | i-pr | $CH_2$ | 6-Bromo-3,5-dimethyl-4-methoxypyridin-2-yl | H |
| 801 | | Br | $NH_2$ | i-pr | $CH_2$ | 6-Chloro-3,5-dimethyl-4-methoxypyridin-2-yl | H |

(continued)

| No. | Ex | $R^1$ | $R^2$ | $R^3$ | $R^4$ | $R^5$ | $R^0$ |
|---|---|---|---|---|---|---|---|
| 802 | | Br | $NH_2$ | i-pr | $CH_2$ | 3,5-Dimethyl-4-bromopyridin-2-yl | H |
| 803 | | Br | $NH_2$ | i-pr | $CH_2$ | 3,5-Dimethyl-4-bromo-1-oxypyridin-2-yl | H |
| 804 | | Br | $NH_2$ | i-pr | $CH_2$ | 3,5-Dimethyl-4-chloropyridin-2-yl | H |
| 805 | | Br | $NH_2$ | i-pr | $CH_2$ | 3,5-Dimethyl-4-chloro-1-oxypyridin-2-yl | H |
| 806 | | Br | $NH_2$ | i-pr | $CH_2$ | 3,5-Dimethyl-4-iodopyridin-2-yl | H |
| 807 | | Br | $NH_2$ | i-pr | $CH_2$ | 3,5-Dimethyl-4-iodo-1-oxypyridin-2-yl | H |
| 808 | | Br | $NH_2$ | i-pr | $CH_2$ | 3,5-Dimethyl-4-thiomethyl-pyridin-2-yl | H |
| 809 | | Br | $NH_2$ | i-pr | $CH_2$ | 3,5-Dimethyl-4-thiomethyl-1-oxypyridin-2-yl | H |
| 810 | | Br | $NH_2$ | i-pr | $CH_2$ | 3,4,5-Trimethyl-pyridin-2-yl | H |
| 811 | | Br | $NH_2$ | i-pr | $CH_2$ | 3,4,5-Trimethyl-1-oxypyridin-2-yl | H |
| 812 | | Br | $NH_2$ | i-pr | $CH_2$ | 4,5,6-Trimethoxypyridin-2-yl | H |
| 813 | | Br | $NH_2$ | i-pr | $CH_2$ | 4,5,6-Trimethoxy-1-oxypyridin-2-yl | H |
| 814 | | Br | $NH_2$ | i-pr | $CH_2$ | 3-Bromo-4,5,6-trimethoxypyridin-2-yl | H |
| 815 | | Br | $NH_2$ | i-pr | $CH_2$ | 3-Chloro-4,5,6-trimethoxypyridin-2-yl | H |
| 816 | | Br | $NH_2$ | i-pr | $CH_2$ | 3,4,5-Trimethoxy-pyridin-2-yl | H |
| 817 | | Br | $NH_2$ | i-pr | $CH_2$ | 3,4,5-Trimethoxy-1-oxypyridin-2-yl | H |
| 818 | | Br | $NH_2$ | i-pr | $CH_2$ | 3-Bromo-3,4,5-trimethoxy-pyridin-2-yl | H |
| 819 | | Br | $NH_2$ | i-pr | $CH_2$ | 3-Chloro-3,4,5-trimethoxy-pyridin-2-yl | H |
| 820 | | Br | $NH_2$ | i-pr | $CH_2$ | 4,5,6-Trimethyl-pyridin-2-yl | H |
| 821 | | Br | $NH_2$ | i-pr | $CH_2$ | 4,5,6-Trimethyl-1-oxypyridin-2-yl | H |
| 822 | | Br | $NH_2$ | i-pr | $CH_2$ | 4,6-Dimethyl-5-methoxy-pyridin-2-yl | H |
| 823 | | Br | $NH_2$ | i-pr | $CH_2$ | 4,6-Dimethyl-5-methoxypyridin-3-yl | H |
| 824 | | Br | $NH_2$ | i-pr | $CH_2$ | 4,6-Dimethyl-5-methoxy-1-oxypyridin-3-yl | H |
| 825 | | Br | $NH_2$ | i-pr | $CH_2$ | 4,6-Dimethyl-5-bromopyridin-3-yl | H |
| 826 | | Br | $NH_2$ | i-pr | $CH_2$ | 4,6-Dimethyl-5-chloropyridin-3-yl | H |
| 827 | | Br | $NH_2$ | i-pr | $CH_2$ | 5,6-Dimethyl-4-bromopyridin-3-yl | H |
| 828 | | Br | $NH_2$ | i-pr | $CH_2$ | 5,6-Dimethyl-4-chloropyridin-3-yl | H |
| 829 | | Br | $NH_2$ | i-pr | $CH_2$ | 2,6-Dimethyl-3-methoxypyridin-4-yl | H |
| 830 | | Br | $NH_2$ | i-pr | $CH_2$ | 2,6-Dimethyl-pyridin-4-yl | H |
| 831 | | Br | $NH_2$ | i-pr | $CH_2$ | 2,3,6-Trimethyl-pyridin-4-yl | H |
| 832 | | Br | $NH_2$ | i-pr | $CH_2$ | 2,3,6-Trimethoxy-pyridin-4-yl | H |
| 833 | | Br | $NH_2$ | i-pr | $CH_2$ | 2,6-Dimethyl-3-bromopyridin-4-yl | H |
| 834 | | Br | $NH_2$ | i-pr | $CH_2$ | 2,6-Dimethyl-3-chloropyridin-4-yl | H |
| 835 | | Br | $NH_2$ | i-pr | $CH_2$ | 2,6-Dimethyl-3-methoxy-1-oxypyridin-4-yl | H |
| 836 | | Br | $NH_2$ | i-pr | $CH_2$ | 2,6-Dimethyl-1-oxy-pyridin-4-yl | H |
| 837 | | Br | $NH_2$ | i-pr | $CH_2$ | 2,3,6-Trimethyl-1-oxypyridin-4-yl | H |
| 838 | | Br | $NH_2$ | i-pr | $CH_2$ | 2,3,6-Trimethoxy-1-oxypyridin-4-yl | H |
| 839 | | Br | $NH_2$ | i-pr | $CH_2$ | 2,6-Dimethyl-3-bromol-oxypyridin-4-yl | H |

(continued)

| No. | Ex | $R^1$ | $R^2$ | $R^3$ | $R^4$ | $R^5$ | $R^0$ |
|---|---|---|---|---|---|---|---|
| 840 | | Br | $NH_2$ | i-pr | $CH_2$ | 2,6-Dimethyl-3-chlorol-oxypyridin-4-yl | H |
| 841 | | Br | $NH_2$ | i-pr | $CH_2$ | 4,6-Dimethyl-5-iodopyridin-3-yl | H |
| 842 | | Br | $NH_2$ | i-pr | $CH_2$ | 3,5-Dimethyl-4-aminopyridin-2-yl | H |
| 843 | | Br | $NH_2$ | Ph | $CH_2$ | 3,5-Dimethyl-4-methoxypyridin-2-yl | H |
| 844 | | Br | $NH_2$ | Ph | $CH_2$ | 3,5-Dimethyl-4-methoxy-1-oxypyridin-2-yl | H |
| 845 | | Br | $NH_2$ | Ph | $CH_2$ | 6-Bromo-3,5-dimethyl-4-methoxypyridin-2-yl | H |
| 846 | | Br | $NH_2$ | Ph | $CH_2$ | 6-Chloro-3,5-dimethyl-4-methoxypyridin-2-yl | H |
| 847 | | Br | $NH_2$ | Ph | $CH_2$ | 3,5-Dimethyl-4-bromopyridin-2-yl | H |
| 848 | | Br | $NH_2$ | Ph | $CH_2$ | 3,5-Dimethyl-4-bromo-1-oxypyridin-2-yl | H |
| 849 | | Br | $NH_2$ | Ph | $CH_2$ | 3,5-Dimethyl-4-chloropyridin-2-yl | H |
| 850 | | Br | $NH_2$ | Ph | $CH_2$ | 3,5-Dimethyl-4-chloro-1-oxypyridin-2-yl | H |
| 851 | | Br | $NH_2$ | Ph | $CH_2$ | 3,5-Dimethyl-4-iodopyridin-2-yl | H |
| 852 | | Br | $NH_2$ | Ph | $CH_2$ | 3,5-Dimethyl-4-iodo-1-oxypyridin-2-yl | H |
| 853 | | Br | $NH_2$ | Ph | $CH_2$ | 3,5-Dimethyl-4-thiomethyl-pyridin-2-yl | H |
| 854 | | Br | $NH_2$ | Ph | $CH_2$ | 3,5-Dimethyl-4-thiomethyl-1-oxypyridin-2-yl | H |
| 855 | | Br | $NH_2$ | Ph | $CH_2$ | 3,4,5-Trimethyl-pyridin-2-yl | H |
| 856 | | Br | $NH_2$ | Ph | $CH_2$ | 3,4,5-Trimethyl-1-oxypyridin-2-yl | H |
| 857 | | Br | $NH_2$ | Ph | $CH_2$ | 4,5,6-Trimethoxypyridin-2-yl | H |
| 858 | | Br | $NH_2$ | Ph | $CH_2$ | 4,5,6-Trimethoxy-1-oxypyridin-2-yl | H |
| 859 | | Br | $NH_2$ | Ph | $CH_2$ | 3-Bromo-4,5,6-trimethoxypyridin-2-yl | H |
| 860 | | Br | $NH_2$ | Ph | $CH_2$ | 3-Chloro-4,5,6-trimethoxypyridin-2-yl | H |
| 861 | | Br | $NH_2$ | Ph | $CH_2$ | 4,6-Dimethyl-5-methoxypyridin-3-yl | H |
| 862 | | Br | $NH_2$ | Ph | $CH_2$ | 4,6-Dimethyl-5-methoxy-1-oxypyridin-3-yl | H |
| 863 | | Br | $NH_2$ | Me | $CH_2$ | 3,5-Dimethyl-4-methoxypyridin-2-yl | H |
| 864 | | Br | $NH_2$ | Me | $CH_2$ | 3,5-Dimethyl-4-methoxy-1-oxypyridin-2-yl | H |
| 865 | | Br | $NH_2$ | Me | $CH_2$ | 6-Bromo-3,5-dimethyl-4-methoxypyridin-2-yl | H |
| 866 | | Br | $NH_2$ | Me | $CH_2$ | 6-Chloro-3,5-dimethyl-4-methoxypyridin-2-yl | H |
| 867 | | Br | $NH_2$ | Me | $CH_2$ | 3,5-Dimethyl-4-bromopyridin-2-yl | H |
| 868 | | Br | $NH_2$ | Me | $CH_2$ | 3,5-Dimethyl-4-bromo-1-oxypyridin-2-yl | H |
| 869 | | Br | $NH_2$ | Me | $CH_2$ | 3,5-Dimethyl-4-chloropyridin-2-yl | H |
| 870 | | Br | $NH_2$ | Me | $CH_2$ | 3,5-Dimethyl-4-chloro-1-oxypyridin-2-yl | H |
| 871 | | Br | $NH_2$ | Me | $CH_2$ | 3,5-Dimethyl-4-iodopyridin-2-yl | H |
| 872 | | Br | $NH_2$ | Me | $CH_2$ | 3,5-Dimethyl-4-iodo-1-oxypyridin-2-yl | H |
| 873 | | Br | $NH_2$ | Me | $CH_2$ | 4,6-Dimethyl-5-methoxypyridin-3-yl | H |
| 874 | | Br | $NH_2$ | Me | $CH_2$ | 4,6-Dimethyl-5-methoxy-1-oxypyridin-3-yl | H |
| 875 | 39 | Cl | $NH_2$ | CH2 $(Bn)_2$ | $CH_2$ | 3,5-Dimethyl-4-methoxypyridin-2-yl | H |
| 876 | 12 | Cl | $NH_2$ | H | $CH_2$ | 2-Chloro-4,5-dimethoxylphenyl | H |
| 877 | 16 | Cl | $NH_2$ | H | $CH_2$ | 2-Nitro-4,5-dimethoxylphenyl | H |

(continued)

| No. | Ex | $R^1$ | $R^2$ | $R^3$ | $R^4$ | $R^5$ | $R^0$ |
|---|---|---|---|---|---|---|---|
| 878 | 17 | Cl | $NH_2$ | H | $CH_2$ | 3,4-Dichlorophenyl | H |
| 879 | 18 | Cl | $NH_2$ | H | $CH_2$ | 3,5-Dimethoxylphenyl | H |
| 880 | 19 | Cl | $NH_2$ | H | $CH_2$ | 2,5-Dimethoxylphenyl | H |
| 881 | 26 | Br | $NH_2$ | H | $CH_2$ | 3,5-Dimethoxylphenyl | H |
| 882 | 27 | Cl | $NH_2$ | H | $CH_2$ | 3-Methoxylphenyl | H |
| 883 | 28 | Cl | $NH_2$ | H | $CH_2$ | 4-Methoxylphenyl | H |
| 884 | 29 | Cl | 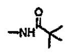 | I | $CH_2$ | 3,5-Dimethyl-4-methoxy-1-oxypyridin-2-yl | H |
| 885 | 30 | Cl | 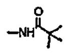 | I | $CH_2$ | 3,5-Dimethyl-4-bromo-1-oxypyridin-2-yl | H |
| 886 | 31 | Cl | 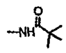 | I | $CH_2$ | 3,5-Dimethyl-4-methoxypyridin-2-yl | H |
| 887 | 32 | Cl | 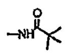 | H | $CH_2$ | 3,5-dimethyl-4-bromo-1-oxypyridin-2-yl | H |
| 888 | 33 | Cl | 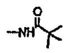 | H | $CH_2$ | 3,5-Dimethyl-4-methoxypyridin-2-yl | H |
| 889 | 34 | Cl | 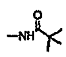 | H | $CH_2$ | 3,5-Dimethyl-4-methoxy-1-oxypyridin-2-yl | H |
| 890 | 35 | Cl | 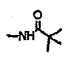 | H | $CH_2$ | 3,5-Dimethyl-4-chloropyridin-2-yl | H |
| 891 | 36 | Cl | 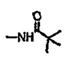 | I | $CH_2$ | 3,5-Dimethyl-4-chloropyridin-2-yl | H |
| 892 | 37 | Cl | 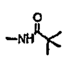 | H | $CH_2$ | 3,5-Dimethyl-4-chloro-1-oxypyridin-2-yl | H |
| 893 | 38 | Cl | 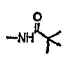 | I | $CH_2$ | 3,5-Dimethyl-4-chloro-1-oxypyridin-2-yl | H |

Compounds of interest in Table 1 are: 2, 3, 17, 18, 27, 28, 62, 63, 77, 78, 92, 93, 129, 130, 238, 239, 242, 243, 245, 246, 247, 248, 249, 250, 251, 252, 253, 267, 268, 287, 288, 291, 292, 293, 294, 295, 296, 297, 298, 312, 313, 332, 333, 334, 335, 336, 337, 338, 339, 351, 352, 365, 366, 384, 385, 398, 399, 400, 401, 402, 403, 404, 405, 417, 418, 431, 432, 433, 434, 435, 436, 437, 438, 450, 451, 464, 465, 483, 484, 497, 498, 530, 531, 549, 550, 562, 563, 574, 575, 577, 578, 589, 59U, 592, 593, 604, 605, 607, 608, 619, 620, 755, 756, 759, 760, 761, 762, 763, 764, 765, 766, 780, 781, 800, 801, 804, 805, 806, 807, 808, 809, 810, 811, 825, 826, 845, 846, 863, 864, 865, 866, 875, and 876 (with those selected being 17, 18, 27, 28, 62, 63, 77, 78, 242, 243, 245, 246, 247, 248, 249, 250, 251, 252, 253, 267, 268, 287, 288, 291, 292, 293, 294, 295, 296, 312, 313, 431, 432, 755, 756, 759, . 760, 761, 762, 763, 764, 800, and 801.)

TABLE 2. EXEMPLARY COMPOUNDS OF THE INVENTION OF FORMULA IIC

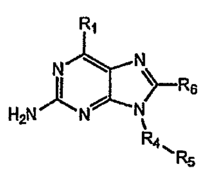

IIC

| No. | Ex. | $R^1$ | $R^3$ | $R^4$ | $R^5$ |
|---|---|---|---|---|---|
| 1 | 48 | Cl | H | $CH_2$ | 3,4,5-Trimethoxyphenyl |

(continued)

| No. | Ex. | $R^1$ | $R^3$ | $R^4$ | $R^5$ |
|---|---|---|---|---|---|
| 2 | 98 | Cl | H | $CH_2$ | 2-Chloro-3,4,5-trimethoxyphenyl |
| 3 | 51 | Cl | H | $CH_2$ | 2-Bromo-3,4,5-trimethoxyphenyl |
| 4 | 106 | Cl | H | $CH_2$ | 2-Iodo-3,4,5-trimethoxyphenyl |
| 5 | | Cl | H | $CH_2$ | 2-Fluoro-3,4,5-trimethoxyphenyl |
| 6 | | Cl | H | $CH_2$ | 3,4,5-Trimethylphenyl |
| 7 | | Cl | H | $CH_2$ | 2-Chloro-3,4,5-trimethylphenyl |
| 8 | | Cl | H | $CH_2$ | 2-Bromo-3,4,5-trimethylphenyl |
| 9 | | Cl | H | $CH_2$ | 2-Iodo-3,4,5-trimethylphenyl |
| 10 | | Cl | H | $CH_2$ | 2-Fluoro-3,4,5-trimethylphenyl |
| 11 | 43 | Cl | H | $CH_2$ | 3,5-Dimethoxy-4-methylphenyl |
| 12 | | Cl | H | $CH_2$ | 2-Chloro-3,5-dimethoxy-4-methylphenyl |
| 13 | 44 | Cl | H | $CH_2$ | 2-Bromo-3,5-dimethoxy-4-methylphenyl |
| 14 | | Cl | H | $CH_2$ | 2-Iodo-3,5-dimethoxy-4-methylphenyl |
| 15 | | Cl | H | $CH_2$ | 2-Fluoro-3,5-dimethoxy-4-methylphenyl |
| 16 | | Cl | H | $CH_2$ | 3,5-Dichloro-4-methylphenyl |
| 17 | | Cl | H | $CH_2$ | 2,3,5-Trichloro-4-methylphenyl |
| 18 | | Cl | H | $CH_2$ | 2-Bromo-3,5-dichloro-4-methylphenyl |
| 19 | | Cl | H | $CH_2$ | 2-Iodo-3,5-dichloro-4-methylphenyl |
| 20 | | Cl | H | $CH_2$ | 2-Fluoro-3,5-dichloro-4-methylphenyl |
| 21 | | Cl | H | $CH_2$ | 3,5-Dibromo-4-methylphenyl |
| 22 | | Cl | H | $CH_2$ | 2-Chloro-3,5-dibromo-4-methylphenyl |
| 23 | | Cl | H | $CH_2$ | 2,3,5-Tribromo-4-methylphenylphenyl |
| 24 | | Cl | H | $CH_2$ | 2-Iodo-3,5-dibromo-4-methylphenyl |
| 25 | | Cl | H | $CH_2$ | 2-Fluoro-3,5-dibromo-4-methylphenyl |
| 26 | | Cl | H | $CH_2$ | 3,5-Dichloro-4-methoxyphenyl |
| 27 | | Cl | H | $CH_2$ | 2,3,5-Trichloro-4-methoxyphenyl |
| 28 | | Cl | H | $CH_2$ | 2-Bromo-3,5-dichloro-4-methoxyphenyl |
| 29 | | Cl | H | $CH_2$ | 2-Iodo-3,5-dichloro-4-methoxyphenyl |
| 30 | | Cl | H | $CH_2$ | 2-Fluoro-3,5-dichloro-4-methoxyphenyl |
| 31 | | Cl | H | $CH_2$ | 3,5-Dibromo-4-methoxyphenyl |
| 32 | | Cl | H | $CH_2$ | 2-Chloro-3,5-dibromo-4-methoxyphenyl |
| 33 | | Cl | H | $CH_2$ | 2,3,5-Tribromo-4-methoxyphenyl |
| 34 | | Cl | H | $CH_2$ | 2-Iodo-3,5-dibromo-4-methoxyphenyl |
| 35 | | Cl | H | $CH_2$ | 2-Fluoro-3,5-dibromo-4-methoxyphenyl |
| 36 | | Cl | H | $CH_2$ | 3-Chloro-5-bromo-4-methylphenyl |
| 37 | | Cl | H | $CH_2$ | 2,3-Dichloro-5-bromo-4-methylphenyl |
| 38 | | Cl | H | $CH_2$ | 2,5-Dibromo-3-chloro-4-methylphenyl |
| 39 | | Cl | H | $CH_2$ | 2-Iodo-3-chloro-5-bromo-4-methylphenyl |

(continued)

| No. | Ex. | $R^1$ | $R^3$ | $R^4$ | $R^5$ |
|---|---|---|---|---|---|
| 40 | | Cl | H | $CH_2$ | 2-Fluoro-3-chloro-5-bromo-4-methylphenyl |
| 41 | | Cl | H | $CH_2$ | 3-Chloro-5-bromo-4-methoxyphenylphenyl |
| 42 | | Cl | H | $CH_2$ | 2,3-Dichloro-5-bromo-4-methoxyphenyl |
| 43 | | Cl | H | $CH_2$ | 2,5-Dibromo-3-chloro-4-methoxyphenyl |
| 44 | | Cl | H | $CH_2$ | 2-Iodo-3-chloro-5-bromo-4-methoxyphenyl |
| 45 | | Cl | H | $CH_2$ | 2-Fluoro-3-chloro-5-bromo-4-methoxyphenyl |
| 46 | | Cl | H | $CH_2$ | 3-Bromo-5-chloro-4-methylphenyl |
| 47 | | Cl | H | $CH_2$ | 2,5-Dichloro-3-bromo-4-methylphenyl |
| 48 | | Cl | H | $CH_2$ | 2,3-Dibromo-5-chloro-4-methylphenyl |
| 49 | | Cl | H | $CH_2$ | 2-Iodo-3-bromo-5-chloro-4-methylphenyl |
| 50 | | Cl | H | $CH_2$ | 2-Fluoro-3-bromo-5-chloro-4-methylphenyl |
| 51 | | Cl | H | $CH_2$ | 3-Bromo-5-chloro-4-methoxyphenyl |
| 52 | | Cl | H | $CH_2$ | 2,5-Dichloro-3-bromo-4-methoxyphenyl |
| 53 | | Cl | H | $CH_2$ | 2,3-Dibromo-5-chloro-4-methoxyphenyl |
| 54 | | Cl | H | $CH_2$ | 2-Iodo-3-bromo-5-chloro-4-methoxyphenyl |
| 55 | | Cl | H | $CH_2$ | 2-Fluoro-3-bromo-5-chloro-4-methoxyphenyl |
| 56 | | Cl | H | $CH_2$ | 3,5-Dimethoxy-4-trifluoromethylphenyl |
| 57 | | Cl | H | $CH_2$ | 2-Chloro-3,5-dimethoxy-4- trifluoromethylphenyl |
| 58 | | Cl | H | $CH_2$ | 2-Bromo-3,5-dimethoxy-4- trifluoromethylphenyl |
| 59 | | Cl | H | $CH_2$ | 2-Iodo-3,5-dimethoxy-4- trifluoromethylphenyl |
| 60 | | Cl | H | $CH_2$ | 2-Fluoro-3,5-dimethoxy-4- trifluoromethylphenyl |
| 61 | | Cl | H | $CH_2$ | 3,5-dibromo-4- trifluoromethoxyphenyl |
| 62 | | Cl | H | $CH_2$ | 2-Chloro-3,5-dibromo-4-trifluoromethoxyphenyl |
| 63 | | Cl | H | $CH_2$ | 2,3,5-Tribromo-4- trifluoromethoxyphenyl |
| 64 | | Cl | H | $CH_2$ | 2-Iodo-3,5-dibromo-4- trifluoromethoxyphenyl |
| 65 | | Cl | H | $CH_2$ | 2-Fluoro-3,5-dibromo-4- trifluoromethoxyphenyl |
| 66 | | Cl | H | $CH_2$ | 3,5-Dimethyl-4-methoxyphenyl |
| 67 | | Cl | H | $CH_2$ | 2-Chloro-3,5-dimethyl-4-methoxyphenyl |
| 68 | | Cl | H | $CH_2$ | 2-Bromo-3,5-dimethyl-4-methoxyphenyl |
| 69 | | Cl | H | $CH_2$ | 2-Iodo-3,5-dimethyl-4-methoxyphenyl |
| 70 | | Cl | H | $CH_2$ | 2-Fluoro-3,5-dimethyl-4-methoxyphenyl |
| 71 | | Cl | H | $CH_2$ | 3,5-Dimethyl-4-bromophenyl |
| 72 | | Cl | H | $CH_2$ | 2-Chloro-3,5-dimethyl-4-bromophenyl |
| 73 | | Cl | H | $CH_2$ | 2,4-Dibromo-3,5-dimethylphenyl |
| 74 | | Cl | H | $CH_2$ | 2-Iodo-3,5-dimethyl-4-bromophenyl |
| 75 | | Cl | H | $CH_2$ | 2-Fluoro-3,5-dimethyl-4-bromophenyl |
| 76 | | Cl | H | $CH_2$ | 3,5-Dimethyl-4-chlorophenyl |
| 77 | | Cl | H | $CH_2$ | 2,4-Dichloro-3,5-dimethylphenyl |

(continued)

| No. | Ex. | $R^1$ | $R^3$ | $R^4$ | $R^5$ |
|---|---|---|---|---|---|
| 78 | | Cl | H | $CH_2$ | 2-Bromo-3,5-dimethyl-4-chlorophenyl |
| 79 | | Cl | H | $CH_2$ | 2-Iodo-3,5-dimethyl-4-chlorophenyl |
| 80 | | Cl | H | $CH_2$ | 2-Fluoro-3,5-dimethyl-4-chlorophenyl |
| 81 | 101 | Br | H | $CH_2$ | 3,4,5-Trimethoxyphenyl |
| 82 | 77 | Br | H | $CH_2$ | 2-Chloro-3,4,5-trimethoxyphenyl |
| 83 | 102 | Br | H | $CH_2$ | 2-Bromo-3,4,5-trimethoxyphenyl |
| 84 | | Br | H | $CH_2$ | 2-Iodo-3,4,5-trimethoxyphenyl |
| 85 | | Br | H | $CH_2$ | 2-Fluoro-3,4,5-trimethoxyphenyl |
| 86 | | Br | H | $CH_2$ | 3,4,5-Trimethylphenyl |
| 87 | | Br | H | $CH_2$ | 2-Chloro-3,4,5-trimethylphenyl |
| 88 | | Br | H | $CH_2$ | 2-Bromo-3,4,5-trimethylphenyl |
| 89 | | Br | H | $CH_2$ | 2-Iodo-3,4,5-trimethylphenyl |
| 90 | | Br | H | $CH_2$ | 2-Fluoro-3,4,5-trimethylphenyl |
| 91 | | Br | H | $CH_2$ | 3,5-Dimethoxy-4-methylphenyl |
| 92 | | Br | H | $CH_2$ | 2-Chloro-3,5-dimethoxy-4-methylphenyl |
| 93 | | Br | H | $CH_2$ | 2-Bromo-3,5-dimethoxy-4-methylphenyl |
| 94 | | Br | H | $CH_2$ | 2-Iodo-3,5-diniethoxy-4-methylphenyl |
| 95 | | Br | H | $CH_2$ | 2-Fluoro-3,5-dimethoxy-4-methylphenyl |
| 96 | | Br | H | $CH_2$ | 3,5-Dichloro-4-methylphenyl |
| 97 | | Br | H | $CH_2$ | 2,3,5-Trichloro-4-methylphenyl |
| 98 | | Br | H | $CH_2$ | 2-Bromo-3,5-dichloro-4-methylphenyl |
| 99 | | Br | H | $CH_2$ | 2-Iodo-3,5-dichloro-4-methylphenyl |
| 100 | | Br | H | $CH_2$ | 2-Fluoro-3,5-dichloro-4-methylphenyl |
| 101 | | Br | H | $CH_2$ | 3,5-Dibromo-4-methylphenyl |
| 102 | | Br | H | $CH_2$ | 2-Chloro-3,5-dibromo-4-methylphenyl |
| 103 | | Br | H | $CH_2$ | 2,3,5-Tribromo-4-methylphenylphenyl |
| 104 | | Br | H | $CH_2$ | 2-Iodo-3,5-dibromo-4-methylphenyl |
| 105 | | Br | H | $CH_2$ | 2-Fluoro-3,5-dibromo-4-methylphenyl |
| 106 | | Br | H | $CH_2$ | 3,5-Dichloro-4-methoxyphenyl |
| 107 | | Br | H | $CH_2$ | 2,3,5-Trichloro-4-methoxyphenyl |
| 108 | | Br | H | $CH_2$ | 2-Bromo-3,5-dichloro-4-methoxyphenyl |
| 109 | | Br | H | $CH_2$ | 2-Iodo-3,5-dichloro-4-methoxyphenyl |
| 110 | | Br | H | $CH_2$ | 2-Fluoro-3,5-dichloro-4-methoxyphenyl |
| 111 | | Br | H | $CH_2$ | 3,5-Dibromo-4-methoxyphenyl |
| 112 | | Br | H | $CH_2$ | 2-Chloro-3,5-dibromo-4-methoxyphenyl |
| 113 | | Br | H | $CH_2$ | 2,3,5-Tribromo-4-methoxyphenyl |
| 114 | | Br | H | $CH_2$ | 2-Iodo-3,5-dibromo-4-methoxyphenyl |
| 115 | | Br | H | $CH_2$ | 2-Fluoro-3,5-dibromo-4-methoxyphenyl |

(continued)

| No. | Ex. | $R^1$ | $R^3$ | $R^4$ | $R^5$ |
|---|---|---|---|---|---|
| 116 | | Br | H | $CH_2$ | 3-Chloro-5-bromo-4-methylphenyl |
| 117 | | Br | H | $CH_2$ | 2,3-Dichloro-5-bromo-4-methylphenyl |
| 118 | | Br | H | $CH_2$ | 2,5-Dibromo-3-chloro-4-methylphenyl |
| 119 | | Br | H | $CH_2$ | 2-Iodo-3-chloro-5-bromo-4-methylphenyl |
| 120 | | Br | H | $CH_2$ | 2-Fluoro-3-chloro-5-bromo-4-methylphenyl |
| 121 | | Br | H | $CH_2$ | 3-Chloro-5-bromo-4-methoxyphenylphenyl |
| 122 | | Br | H | $CH_2$ | 2,3-Dichloro-5-bromo-4-methoxyphenyl |
| 123 | | Br | H | $CH_2$ | 2,5-Dibromo-3-chloro-4-methoxyphenyl |
| 124 | | Br | H | $CH_2$ | 2-Iodo-3-chloro-5-bromo-4-methoxyphenyl |
| 125 | | Br | H | $CH_2$ | 2-Fluoro-3-chloro-5-bromo-4-methoxyphenyl |
| 126 | | Br | H | $CH_2$ | 3-Bromo-5-chloro-4-methylphenyl |
| 127 | | Br | H | $CH_2$ | 2,5-Dichloro-3-bromo-4-methylphenyl |
| 128 | | Br | H | $CH_2$ | 2,3-Dibromo-5-chloro-4-methylphenyl |
| 129 | | Br | H | $CH_2$ | 2-Iodo-3-bromo-5-chloro-4-methylphenyl |
| 130 | | Br | H | $CH_2$ | 2-Fluoro-3-bromo-5-chloro-4-methylphenyl |
| 131 | | Br | H | $CH_2$ | 3-Bromo-5-chloro-4-methoxyphenyl |
| 132 | | Br | H | $CH_2$ | 2,5-Dichloro-3-bromo-4-methoxyphenyl |
| 133 | | Br | H | $CH_2$ | 2,3-Dibromo-5-chloro-4-methoxyphenyl |
| 134 | | Br | H | $CH_2$ | 2-Iodo-3-bromo-5-chloro-4-methoxyphenyl |
| 135 | | Br | H | $CH_2$ | 2-Fluoro-3-bromo-5-chloro-4-methoxyphenyl |
| 136 | | Br | H | $CH_2$ | 3,5-Dimethoxy-4-trifluoromethylphenyl |
| 137 | | Br | H | $CH_2$ | 2-Chloro-3,5-dimethoxy-4- trifluoromethylphenyl |
| 138 | | Br | H | $CH_2$ | 2-Bromo-3,5-dimethoxy-4- trifluoromethylphenyl |
| 139 | | Br | H | $CH_2$ | 2-Iodo-3,5-dimethoxy-4- trifluoromethylphenyl |
| 140 | | Br | H | $CH_2$ | 2-Fluoro-3,5-dimethoxy-4- trifluoromethylphenyl |
| 141 | | Br | H | $CH_2$ | 3,5-Dibromo-4- trifluoromethoxyphenyl |
| 142 | | Br | H | $CH_2$ | 2-Chloro-3,5-dibromo-4-trifluoromethoxyphenyl |
| 143 | | Br | H | $CH_2$ | 2,3,5-Tribromo-4- trifluoromethoxyphenyl |
| 144 | | Br | H | $CH_2$ | 2-Iodo-3,5-dibromo-4- trifluoromethoxyphenyl |
| 145 | | Br | H | $CH_2$ | 2-Fluoro-3,5-dibromo-4- trifluoromethoxyphenyl |
| 146 | | Br | H | $CH_2$ | 3,5-Dimethyl-4-methoxyphenyl |
| 147 | | Br | H | $CH_2$ | 2-Chloro-3,5-dimethyl-4-methoxyphenyl |
| 148 | | Br | H | $CH_2$ | 2-Bromo-3,5-dimethyl-4-methoxyphenyl |
| 149 | | Br | H | $CH_2$ | 2-Iodo-3,5-dimethyl-4-methoxyphenyl |
| 150 | | Br | H | $CH_2$ | 2-Fluoro-3,5-dimethyl-4-methoxyphenyl |
| 151 | | Br | H | $CH_2$ | 3,5-Dimethyl-4-bromophenyl |
| 152 | | Br | H | $CH_2$ | 2-Chloro-3,5-dimethyl-4-bromophenyl |
| 153 | | Br | H | $CH_2$ | 2,4-Dibromo-3,5-dimethylphenyl |

(continued)

| No. | Ex. | $R^1$ | $R^3$ | $R^4$ | $R^5$ |
|---|---|---|---|---|---|
| 154 | | Br | H | $CH_2$ | 2-Iodo-3,5-dimethyl-4-bromophenyl |
| 155 | | Br | H | $CH_2$ | 2-Fluoro-3,5-dimethyl-4-bromophenyl |
| 156 | | Br | H | $CH_2$ | 3,5-Dimethyl-4-chlorophenyl |
| 157 | | Br | H | $CH_2$ | 2,4-Dichloro-3,5-dimethylphenyl |
| 158 | | Br | H | $CH_2$ | 2-Bromo-3,5-dimethyl-4-chlorophenyl |
| 159 | | Br | H | $CH_2$ | 2-Iodo-3,5-dimethyl-4-chlorophenyl |
| 160 | | F | H | $CH_2$ | 3,4,5-Trimethoxyphenyl |
| 161 | | F | H | $CH_2$ | 2-Chloro-3,4,5-trimethoxyphenyl |
| 162 | | F | H | $CH_2$ | 2-Bromo-3,4,5-trimethoxyphenyl |
| 163 | | F | H | $CH_2$ | 2-Iodo-3,4,5-trimethoxyphenyl |
| 164 | | F | H | $CH_2$ | 2-Fluoro-3,4,5-trimethoxyphenyl |
| 165 | | F | H | $CH_2$ | 3,4,5-Trimethylphenyl |
| 166 | | F | H | $CH_2$ | 2-Chloro-3,4,5-trimethylphenyl |
| 167 | | F | H | $CH_2$ | 2-Bromo-3,4,5-trimethylphenyl |
| 168 | | F | H | $CH_2$ | 2-Iodo-3,4,5-trimethylphenyl |
| 169 | | F | H | $CH_2$ | 2-Fluoro-3,4,5-trimethylphenyl |
| 170 | | F | H | $CH_2$ | 3,5-Dimethoxy-4-methylphenyl |
| 171 | | F | H | $CH_2$ | 2-Chloro-3,5-dimethoxy-4-methylphenyl |
| 172 | | F | H | $CH_2$ | 2-Bromo-3,5-dimethoxy-4-methylphenyl |
| 173 | | F | H | $CH_2$ | 2-Iodo-3,5-dimethoxy-4-methylphenyl |
| 174 | | F | H | $CH_2$ | 2-Fluoro-3,5-dimethoxy-4-methylphenyl |
| 175 | | F | H | $CH_2$ | 3,5-Dichloro-4-methylphenyl |
| 176 | | F | H | $CH_2$ | 2,3,5-Trichloro-4-methylphenyl |
| 177 | | F | H | $CH_2$ | 2-Bromo-3,5-dichloro-4-methylphenyl |
| 178 | | F | H | $CH_2$ | 2-Iodo-3,5-dichloro-4-methylphenyl |
| 179 | | F | H | $CH_2$ | 2-Fluoro-3,5-dichloro-4-methylphenyl |
| 180 | | F | H | $CH_2$ | 3,5-Dibromo-4-methylphenyl |
| 181 | | F | H | $CH_2$ | 2-Chloro-3,5-dibromo-4-methylphenyl |
| 182 | | F | H | $CH_2$ | 2,3,5-Tribromo-4-methylphenylphenyl |
| 183 | | F | H | $CH_2$ | 2-Iodo-3,5-dibromo-4-methylphenyl |
| 184 | | F | H | $CH_2$ | 2-Fluoro-3,5-dibromo-4-methylphenyl |
| 185 | | F | H | $CH_2$ | 3,5-Dichloro-4-methoxyphenyl |
| 186 | | F | H | $CH_2$ | 2,3,5-Trichloro-4-methoxyphenyl |
| 187 | | F | H | $CH_2$ | 2-Bromo-3,5-dichloro-4-methoxyphenyl |
| 188 | | F | H | $CH_2$ | 2-Iodo-3,5-dichloro-4-methoxyphenyl |
| 189 | | F | H | $CH_2$ | 2-Fluoro-3,5-dichloro-4-mothoxyphenyl |
| 190 | | F | H | $CH_2$ | 3,5-Dibromo-4-methoxyphenyl |
| 191 | | F | H | $CH_2$ | 2-Chloro-3,5-dibromo-4-methoxyphenyl |

(continued)

| No. | Ex. | $R^1$ | $R^3$ | $R^4$ | $R^5$ |
|---|---|---|---|---|---|
| 192 | | F | H | $CH_2$ | 2,3,5-Tribromo-4-methoxyphenyl |
| 193 | | F | H | $CH_2$ | 2-Iodo-3,5-dibromo-4-methoxyphenyl |
| 194 | | F | H | $CH_2$ | 2-Fluoro-3,5-dibromo-4-methoxyphenyl |
| 195 | | F | H | $CH_2$ | 3-Chloro-5-bromo-4-methylphenyl |
| 196 | | F | H | $CH_2$ | 2,3-Dichloro-5-bromo-4-methylphenyl |
| 197 | | F | H | $CH_2$ | 2,5-Dibromo-3-chloro-4-methylphenyl |
| 198 | | F | H | $CH_2$ | 2-Iodo-3-chloro-5-bromo-4-methylphenyl |
| 199 | | F | H | $CH_2$ | 2-Fluoro-3-chloro-5-bromo-4-methylphenyl |
| 200 | | F | H | $CH_2$ | 3-Chloro-5-bromo-4-methoxyphenylphenyl |
| 201 | | F | H | $CH_2$ | 2,3-Dichloro-5-bromo-4-methoxyphenyl |
| 202 | | F | H | $CH_2$ | 2,5-Dibromo-3-chloro-4-methoxyphenyl |
| 203 | | F | H | $CH_2$ | 2-Iodo-3-chloro-5-bromo-4-methoxyphenyl |
| 204 | | F | H | $CH_2$ | 2-Fluoro-3-chloro-5-bromo-4-methoxyphenyl |
| 205 | | F | H | $CH_2$ | 3-Bromo-5-chloro-4-methylphenyl |
| 206 | | F | H | $CH_2$ | 2,5-Dichloro-3-bromo-4-methylphenyl |
| 207 | | F | H | $CH_2$ | 2,3-Dibromo-5-chloro-4-methylphenyl |
| 208 | | F | H | $CH_2$ | 2-Iodo-3-bromo-5-chloro-4-methylphenyl |
| 209 | | F | H | $CH_2$ | 2-Fluoro-3-bromo-5-chloro-4-methylphenyl |
| 210 | | F | H | $CH_2$ | 3-Bromo-5-chloro-4-methoxyphenyl |
| 211 | | F | H | $CH_2$ | 2,5-Dichloro-3-bromo-4-methoxyphenyl |
| 212 | | F | H | $CH_2$ | 2,3-Dibromo-5-chloro-4-methoxyphenyl |
| 213 | | F | H | $CH_2$ | 2-Iodo-3-bromo-5-chloro-4-methoxyphenyl |
| 214 | | F | H | $CH_2$ | 2-Fluoro-3-bromo-5-chloro-4-methoxyphenyl |
| 215 | | F | H | $CH_2$ | 3,5-Dimethoxy-4-trifluoromethylphenyl |
| 216 | | F | H | $CH_2$ | 2-Chloro-3,5-dimethoxy-4- trifluoromethylphenyl |
| 217 | | F | H | $CH_2$ | 2-Bromo-3,5-dimethoxy-4- trifluoromethylphenyl |
| 218 | | F | H | $CH_2$ | 2-Iodo-3,5-dimethoxy-4- trifluoromethylphenyl |
| 219 | | F | H | $CH_2$ | 2-Fluoro-3,5-dimethoxy-4- trifluoromethylphenyl |
| 220 | | F | H | $CH_2$ | 3,5-Dibromo-4- trifluoromethoxyphenyl |
| 221 | | F | H | $CH_2$ | 2-Chloro-3,5-dibromo-4-trifluoromethoxyphenyl |
| 222 | | F | H | $CH_2$ | 2,3,5-Tribromo-4- trifluoromethoxyphenyl |
| 223 | | F | H | $CH_2$ | 2-Iodo-3,5-dibromo-4- trifluoromethoxyphenyl |
| 224 | | F | H | $CH_2$ | 2-Fluoro-3,5-dibromo-4- trifluoromethoxyphenyl |
| 225 | | F | H | $CH_2$ | 3,5-Dimethyl-4-methoxyphenyl |
| 226 | | F | H | $CH_2$ | 2-Chloro-3,5-dimethyl-4-methoxyphenyl |
| 227 | | F | H | $CH_2$ | 2-Bromo-3,5-dimethyl-4-methoxyphenyl |
| 228 | | F | H | $CH_2$ | 2-Iodo-3,5-dimethyl-4-methoxyphenyl |
| 229 | | F | H | $CH_2$ | 2-Fluoro-3,5-dimethyl-4-methoxyphenyl |

(continued)

| No. | Ex. | $R^1$ | $R^3$ | $R^4$ | $R^5$ |
|---|---|---|---|---|---|
| 230 | | F | H | $CH_2$ | 3,5-Dimethyl-4-bromophenyl |
| 231 | | F | H | $CH_2$ | 2-Chloro-3,5-dimethyl-4-bromophenyl |
| 232 | | F | H | $CH_2$ | 2,4-Dibromo-3,5-dimethylphenyl |
| 233 | | F | H | $CH_2$ | 2-Iodo-3,5-dimethyl-4-bromophenyl |
| 234 | | F | H | $CH_2$ | 2-Fluoro-3,5-dimethyl-4-bromophenyl |
| 235 | | F | H | $CH_2$ | 3,5-Dimethyl-4-chlorophenyl |
| 236 | | F | H | $CH_2$ | 2,4-Dichloro-3,5-dimethylphenyl |
| 237 | | F | H | $CH_2$ | 2-Bromo-3,5-dimethyl-4-chlorophenyl |
| 238 | | F | H | $CH_2$ | 2-Iodo-3,5-dimethyl-4-chlorophenyl |
| 239 | | F | H | $CH_2$ | 2-Fluoro-3,5-dimethyl-4-chlorophenyl |
| 240 | 25 | Cl | H | $CH_2$ | 3,5-Dimethyl-4-methoxypyridin-2-yl |
| 241 | 31 | Cl | H | $CH_2$ | 3,5-Dimethyl-4-methoxy-1-oxypyridin-2-yl |
| 242 | | Cl | H | $CH_2$ | 6-Bromo-3,5-dimethyl-4-methoxypyridin-2-yl |
| 243 | 33 | Cl | H | $CH_2$ | 6-Chloro-3,5-dimethyl-4-methoxypyridin-2-yl |
| 244 | | Cl | H | $CH_2$ | 6-Chloro-3,5-dimethyl-4-methoxy-1-oxypyridin-2-yl |
| 245 | | Cl | H | $CH_2$ | 6-Bromo-3,5-dimethyl-4-methoxy-1-oxypyridin-2-yl |
| 246 | 1 | Cl | H | $CH_2$ | 3,5-Dimethyl-4-bromopyridin-2-yl |
| 247 | 4 | Cl | H | $CH_2$ | 3,5-Dfinethyl-4-bromo-1-oxypyridin-2-yl |
| 248 | | Cl | H | $CH_2$ | 6-Bromo-3,5-dimethyl-4-bromopyridin-2-yl |
| 249 | | Cl | H | $CH_2$ | 6-Chloro-3,5-dimethyl-4-bromopyridin-2-yl |
| 250 | | Cl | H | $CH_2$ | 6-Chloro-3,5-dimethyl-4-bromo-1-oxypyridin-2-yl |
| 251 | | Cl | H | $CH_2$ | 4,6-Dibromo-3,5-dimethyl-1-oxypyridin-2-yl |
| 252 | 18 | Cl | H | $CH_2$ | 3,5-Dimethyl-4-chloropyridin-2-yl |
| 253 | 19 | Cl | H | $CH_2$ | 3,5-Dimethyl-4-chloro-1-oxypyridin-2-yl |
| 254 | | Cl | H | $CH_2$ | 6-Bromo-3,5-dimethyl-4-chloropyridin-2-yl |
| 255 | | Cl | H | $CH_2$ | 6-Chloro-3,5-dimethyl-4-chloropyridin-2-yl |
| 256 | | Cl | H | $CH_2$ | 4,6-Dichloro-3,5-dimethyl-1-oxypyridin-2-yl |
| 257 | | Cl | H | $CH_2$ | 6-Bromo-3,5-dimethyl-4-chloro-1-oxypyridin-2-yl |
| 258 | 111 | Cl | H | $CH_2$ | 3,5-Dimethyl-4-iodopyridin-2-yl |
| 259 | | Cl | H | $CH_2$ | 3,5-Dimethyl-4-iodo-1-oxypyridin-2-yl |
| 260 | | Cl | H | $CH_2$ | 6-Bromo-3,5-dimethyl-4-iodopyridin-2-yl |
| 261 | | Cl | H | $CH_2$ | 6-Chloro-3,5-dimethyl-4-iodopyridin-2-yl |
| 262 | | Cl | H | $CH_2$ | 6-Chloro-3,5-dimethyl-4-iodo-1-oxypyridin-2-yl |
| 263 | | Cl | H | $CH_2$ | 6-Bromo-3,5-dimethyl-4-iodo-1-oxypyridin-2-yl |
| 264 | 115 | Cl | H | $CH_2$ | 3,5-Dimethyl-4-thiomethyl-pyridin-2-yl |
| 265 | | Cl | H | $CH_2$ | 3,5-Dimethyl-4-thiomethyl-1-oxypyridin-2-yl |
| 266 | | Cl | H | $CH_2$ | 6-Bromo-3,5-dimethyl-4-thiomethyl-pyridin-2-yl |
| 267 | | Cl | H | $CH_2$ | 6-Chloro-3,5-dimethyl-4-thiomethyl-pyridin-2-yl |

(continued)

| No. | Ex. | $R^1$ | $R^3$ | $R^4$ | $R^5$ |
|---|---|---|---|---|---|
| 268 | | Cl | H | $CH_2$ | 6-Chloro-3,5-dimethyl-4-thiomethyl-1-oxypyridin-2-yl |
| 269 | | Cl | H | $CH_2$ | 6-Bromo-3,5-dimethyl-4-thiomethyl-1-oxypyridin-2-yl |
| 270 | 117 | Cl | H | $CH_2$ | 3,4,5-Trimethyl-pyridin-2-yl |
| 271 | | Cl | H | $CH_2$ | 3,4,5-Trimethyl-1-oxypyridin-2-yl |
| 272 | | Cl | H | $CH_2$ | 6-Bromo-3,4,5-trimethyl-pyridin-2-yl |
| 273 | | Cl | H | $CH_2$ | 6-Chloro-3,4,5-trimethyl-pyridin-2-yl |
| 274 | | Cl | H | $CH_2$ | 6-Chloro-3,4,5-trimethyl-1-oxypyridin-2-yl |
| 275 | | Cl | H | $CH_2$ | 6-Bromo-3,4,5-trimethyl-1-oxypyridin-2-yl |
| 276 | | Cl | H | $CH_2$ | 4,5,6-Trimethoxypyridin-2-yl |
| 277 | | Cl | H | $CH_2$ | 4,5,6-Trimethoxy-1-oxypyridin-2-yl |
| 278 | | Cl | H | $CH_2$ | 3-Bromo-4,5,6-trimethoxypyridin-2-yl |
| 279 | | Cl | H | $CH_2$ | 3-Chloro-4,5,6-trimethoxypyridin-2-yl |
| 280 | | Cl | H | $CH_2$ | 3-Chloro-4,5,6-trimethoxy-1-oxypyridin-2-yl |
| 281 | | Cl | H | $CH_2$ | 3-Bromo-4,5,6-trimethoxy-1-oxypyridin-2-yl |
| 282 | | Cl | H | $CH_2$ | 3,4,5-Trimethoxy-pyridin-2-yl |
| 283 | | Cl | H | $CH_2$ | 3,4,5-Trimethoxy-1-oxypyridin-2-yl |
| 284 | | Cl | H | $CH_2$ | 3-Bromo-3,4,5-trimethoxy-pyridin-2-yl |
| 285 | | Cl | H | $CH_2$ | 3-Chloro-3,4,5-trimethoxy-pyridin-2-yl |
| 286 | | Cl | H | $CH_2$ | 3-Chloro-3,4,5-trimethoxy-1-oxypyridin-2-yl |
| 287 | | Cl | H | $CH_2$ | 3-Bromo-3,4,5-trimethoxy-1-oxypyridin-2-yl |
| 288 | | Cl | H | $CH_2$ | 4,5,6-Trimethyl-pyridin-2-yl |
| 289 | | Cl | H | $CH_2$ | 4,5,6-Trimethyl-1-oxypyridin-2-yl |
| 290 | | Cl | H | $CH_2$ | 3-Bromo-4,5,6-trimethyl-pyridin-2-yl |
| 291 | | Cl | H | $CH_2$ | 3-Chloro-4,5,6-trimethyl-pyridin-2-yl |
| 292 | | Cl | H | $CH_2$ | 3-Chloro-4,5,6-trimethyl-1-oxypyridin-2-yl |
| 293 | | Cl | H | $CH_2$ | 3-Bromo-4,5,6-trimethyl-1-oxypyridin-2-yl |
| 294 | | Cl | H | $CH_2$ | 4,6-Dimethyl-5-methoxy-pyridin-2-yl |
| 295 | | Cl | H | $CH_2$ | 4,6-Dimethyl-5-methoxy-1-oxypyridin-2-yl |
| 296 | | Cl | H | $CH_2$ | 3-Bromo-4,6-dimethyl-5-methoxy-pyridin-2-yl |
| 297 | | Cl | H | $CH_2$ | 3-Chloro-4,6-dimethyl-5-methoxy-pyridin-2-yl |
| 298 | | Cl | H | $CH_2$ | 3-Chloro-4,6-dimethyl-5-methoxy-1-oxypyridin-2-yl |
| 299 | | Cl | H | $CH_2$ | 3-Bromo-4,6-dimethyl-5-methoxy-1-oxypyridin-2-yl |
| 300 | | Cl | H | $CH_2$ | 4-Bromo-5,6-dimethoxy-pyridin-2-yl |
| 301 | | Cl | H | $CH_2$ | 4-Bromo-5,6-dimethoxy-1-oxypyridin-2-yl |
| 302 | | Cl | H | $CH_2$ | 3,4-Dibromo-5,6-dimethoxy-pyridin-2-yl |
| 303 | | Cl | H | $CH_2$ | 3-Chloro-4-bromo-5,6-dimethoxy-pyridin-2-yl |
| 304 | | Cl | H | $CH_2$ | 3-Chloro-4-bromo-5,6-dimethoxy-1-oxypyridin-2-yl |
| 305 | | Cl | H | $CH_2$ | 3,4-Dibromo-5,6-dimethoxy-1-oxypyridin-2-yl |

(continued)

| No. | Ex. | $R^1$ | $R^3$ | $R^4$ | $R^5$ |
|---|---|---|---|---|---|
| 306 | 41 | Cl | H | $CH_2$ | 4,6-Dimethyl-5-methoxypyridin-3-yl |
| 307 | | Cl | H | $CH_2$ | 4,6-Dimethyl-5-methoxy-1-oxypyridin-3-yl |
| 308 | | Cl | H | $CH_2$ | 4,6-Dimethyl-5-bromopyridin-3-yl |
| 309 | | Cl | H | $CH_2$ | 4,6-Dimethyl-5-chloropyridin-3-yl |
| 310 | | Cl | H | $CH_2$ | 5,6-Dimethyl-4-bromopyridin-3-yl |
| 311 | | Cl | H | $CH_2$ | 5,6-Dimethyl-4-chloropyridin-3-yl |
| 312 | | Cl | H | $CH_2$ | 4,6-Dimethyl-5-bromo-1-oxypyridin-pyridin-3-yl |
| 313 | | Cl | H | $CH_2$ | 4,6-Dimethyl-5-chloro-1-oxypyridin-pyridin-3-yl |
| 314 | | Cl | H | $CH_2$ | 5,6-Dimethyl-4-bromo-1-oxypyridin-pyridin-3-yl |
| 315 | | Cl | H | $CH_2$ | 5,6-Dimethyl-4-chloro-1-oxypyridin-pyridin-3-yl |
| 316 | | Cl | H | $CH_2$ | 2,6-Dimethyl-3-methoxypyridin-4-yl |
| 317 | | Cl | H | $CH_2$ | 2,6-Dimethyl-pyridin-4-yl |
| 318 | | Cl | H | $CH_2$ | 2,3,6-Trimethyl-pyridin-4-yl |
| 319 | | Cl | H | $CH_2$ | 2,3,6-Trimethoxy-pyridin-4-yl |
| 320 | | Cl | H | $CH_2$ | 2,6-Dimethyl-3-bromopyridin-4-yl |
| 321 | | Cl | H | $CH_2$ | 2,6-Dimethyl-3-chloropyridin-4-yl |
| 322 | | Cl | H | $CH_2$ | 2,6-Dichloro-3-bromopyridin-4-yl |
| 323 | | Cl | H | $CH_2$ | 2,6-Dibromo-3-chloropyridin-4-yl |
| 324 | | Cl | H | $CH_2$ | 2,3,6-Trichloro-pyridin-4-yl |
| 325 | | Cl | H | $CH_2$ | 2,3,6-Tribromo-pyridin-4-yl |
| 326 | | Cl | H | $CH_2$ | 2,6-Dimethyl-3-methoxy-1-oxy-pyridin-4-yl |
| 327 | | Cl | H | $CH_2$ | 2,6-Dimethyl-1-oxy-pyridin-4-yl |
| 328 | | Cl | H | $CH_2$ | 2,3,6-Trimethyl-1-oxy-pyridin-4-yl |
| 329 | | Cl | H | $CH_2$ | 2,3,6-Trimethoxy-1-oxy-pyridin-4-yl |
| 330 | | Cl | H | $CH_2$ | 2,6-Dimethyl-3-bromol-oxy-pyridin-4-yl |
| 331 | | Cl | H | $CH_2$ | 2,6-Dimethyl-3-chlorol-oxy-pyridin-4-yl |
| 332 | | Cl | H | $CH_2$ | 2,6-Dichloro-3-bromo-1-oxy-pyridin-4-yl |
| 333 | | Cl | H | $CH_2$ | 2,6-Dibromo-3-chloro-1-oxy-pyridin-4-yl |
| 334 | | Cl | H | $CH_2$ | 2,3,6-Trichloro-1-oxy-pyridin-4-yl |
| 335 | | Cl | H | $CH_2$ | 2,3,6-Tribromo-1-oxy-pyridin-4-yl |
| 336 | | Cl | H | $CH_2$ | 4,6-Dimethyl-5-iodopyridin-3-yl |
| 337 | | Cl | H | $CH_2$ | 5,6-Dimethyl-4-iodopyridin-3-yl |
| 338 | | Cl | H | $CH_2$ | 4,5,6-Trichloropyridin-3-yl |
| 339 | | Cl | H | $CH_2$ | 4,5,6-Tribromopyridin-3-yl |
| 340 | 21 | Br | H | $CH_2$ | 3,5-Dimethyl-4-methoxypyridin-2-yl |
| 341 | 24 | Br | H | $CH_2$ | 3,5-Dimethyl-4-methoxy-1-oxypyridin-2-yl |
| 342 | | Br | H | $CH_2$ | 6-Bromo-3,5-dimethyl-4-methoxypyridin-2-yl |
| 343 | | Br | H | $CH_2$ | 6-Chloro-3,5-dimethyl-4-methoxypyridin-2-yl |

(continued)

| No. | Ex. | $R^1$ | $R^3$ | $R^4$ | $R^5$ |
|---|---|---|---|---|---|
| 344 | | Br | H | $CH_2$ | 6-Chloro-3,5-dimethyl-4-methoxy-1-oxypyridin-2-yl |
| 345 | | Br | H | $CH_2$ | 6-Bromo-3,5-dimethyl-4-methoxy-1-oxypyridin-2-yl |
| 346 | 5 | Br | H | $CH_2$ | 3,5-Dimethyl-4-bromopyridin-2-yl |
| 347 | 6 | Br | H | $CH_2$ | 3,5-Dimethyl-4-bromo-1-oxypyridin-2-yl |
| 348 | | Br | H | $CH_2$ | 6-Bromo-3,5-dimethyl-4-bromopyridin-2-yl |
| 349 | | Br | H | $CH_2$ | 6-Chloro-3,5-dimethyl-4-bromopyridin-2-yl |
| 350 | | Br | H | $CH_2$ | 6-Chloro-3,5-dimethyl-4-bromo-1-oxypyridin-2-yl |
| 351 | | Br | H | $CH_2$ | 4,6-Dibromo-3,5-dimethyl-1-oxypyridin-2-yl |
| 352 | | Br | H | $CH_2$ | 3,5-Dimethyl-4-chloropyridin-2-yl |
| 353 | | Br | H | $CH_2$ | 3,5-Dimethyl-4-chloro-1-oxypyridin-2-yl |
| 354 | | Br | H | $CH_2$ | 6-Bromo-3,5-dimethyl-4-chloropyridin-2-yl |
| 355 | | Br | H | $CH_2$ | 6-Chloro-3,5-dimethyl-4-chloropyridin-2-yl |
| 356 | | Br | H | $CH_2$ | 4,6-Dichloro-3,5-dimethyl-1-oxypyridin-2-yl |
| 357 | | Br | H | $CH_2$ | 6-Bromo-3,5-dimethyl-4-chloro-1-oxypyridin-2-yl |
| 358 | | Br | H | $CH_2$ | 3,5-Dimethyl-4-iodopyridin-2-yl |
| 359 | | Br | H | $CH_2$ | 3,5-Dimethyl-4-iodo-1-oxypyridin-2-yl |
| 360 | | Br | H | $CH_2$ | 6-Bromo-3,5-dimethyl-4-iodopyridin-2-yl |
| 361 | | Br | H | $CH_2$ | 6-Chloro-3,5-dimethyl-4-iodopyridin-2-yl |
| 362 | | Br | H | $CH_2$ | 6-Chloro-3,5-dimethyl-4-iodo-1-oxypyridin-2-yl |
| 363 | | Br | H | $CH_2$ | 6-Bromo-3,5-dimethyl-4-iodo-1-oxypyridin-2-yl |
| 364 | | Br | H | $CH_2$ | 3,5-Dimethyl-4-thiomethyl-pyridin-2-yl |
| 365 | | Br | H | $CH_2$ | 3,5-Dimethyl-4-thiomethyl-1-oxypyridin-2-yl |
| 366 | | Br | H | $CH_2$ | 6-Bromo-3,5-dimethyl-4-thiomethyl-pyridin-2-yl |
| 367 | | Br | H | $CH_2$ | 6-Chloro-3,5-dimethyl-4-thiomethyl-pyridin-2-yl |
| 368 | | Br | H | $CH_2$ | 6-Chloro-3,5-dimethyl-4-thiomethyl-1-oxypyridin-2-yl |
| 369 | | Br | H | $CH_2$ | 6-Bromo-3,5-dimethyl-4-thiomethyl-1-oxypyridin-2-yl |
| 370 | 118 | Br | H | $CH_2$ | 3,4,5-Trimethyl-pyridin-2-yl |
| 371 | 119 | Br | H | $CH_2$ | 3,4,5-Trimethyl-1-oxypyridin-2-yl |
| 372 | | Br | H | $CH_2$ | 6-Bromo-3,4,5-trimethyl-pyridin-2-yl |
| 373 | | Br | H | $CH_2$ | 6-Chloro-3,4,5-trimethyl-pyridin-2-yl |
| 374 | | Br | H | $CH_2$ | 6-Chloro-3,4,5-trimethyl-1-oxypyridin-2-yl |
| 375 | | Br | H | $CH_2$ | 6-Bromo-3,4,5-trimethyl-1-oxypyridin-2-yl |
| 376 | | Br | H | $CH_2$ | 3,4,5-Trimethoxy-pyridin-2-yl |
| 377 | | Br | H | $CH_2$ | 3,4,5-Trimethoxy-l-oxypyridin-2-yl |
| 378 | | Br | H | $CH_2$ | 6-Bromo-3,4,5-trimethoxy-pyridin-2-yl |
| 379 | | Br | H | $CH_2$ | 6-Chloro-3,4,5-trimethoxy-pyridin-2-yl |
| 380 | | Br | H | $CH_2$ | 6-Chloro-3,4,5-trimethoxy-1-oxypyridin-2-yl |
| 381 | | Br | H | $CH_2$ | 6-Bromo-3,4,5-trimethoxy-1-oxypyridin-2-yl |

(continued)

| No. | Ex. | $R^1$ | $R^3$ | $R^4$ | $R^5$ |
|---|---|---|---|---|---|
| 382 | | Br | H | $CH_2$ | 4,5,6-Trimethoxypyridin-2-yl |
| 383 | | Br | H | $CH_2$ | 4,5,6- Trimethoxy-1-oxypyridin-2-yl |
| 384 | | Br | H | $CH_2$ | 3-Bromo-4,5,6-trimethoxypyridin-2-yl |
| 385 | | Br | H | $CH_2$ | 3-Chloro-4,5,6-trimethoxypyridin-2-yl |
| 386 | | Br | H | $CH_2$ | 3-Chloro-4,5,6-trimethoxy-1-oxypyridin-2-yl |
| 387 | | Br | H | $CH_2$ | 3-Bromo-4,5,6-trimethoxy-1-oxypyridin-2-yl |
| 388 | | Br | H | $CH_2$ | 4,5,6-Trimethoxypyridin-2-yl |
| 389 | | Br | H | $CH_2$ | 4,5,6- Trimethoxy-1-oxypyridin-2-yl |
| 390 | | Br | H | $CH_2$ | 3-Bromo-4,5,6-trimethyl-pyridin-2-yl |
| 391 | | Br | H | $CH_2$ | 3-Chloro-4,5,6-trimethyl-pyridin-2-yl |
| 392 | | Br | H | $CH_2$ | 3-Chloro-4,5,6-trimethyl-1-oxypyridin-2-yl |
| 393 | | Br | H | $CH_2$ | 3-Bromo-4,5,6-trimethyl-1-oxypyridin-2-yl |
| 394 | | Br | H | $CH_2$ | 4,6-Dimethyl-5-methoxy-pyridin-2-yl |
| 395 | | Br | H | $CH_2$ | 4,6-Dimethyl-5-methoxy-1-oxypyridin-2-yl |
| 396 | | Br | H | $CH_2$ | 3-Bromo-4,6-dimethyl-5-methoxy-pyridin-2-yl |
| 397 | | Br | H | $CH_2$ | 3-Chloro-4,6-dimethyl-5-methoxy-pyridin-2-yl |
| 398 | | Br | H | $CH_2$ | 3-Chloro-4,6-dimethyl-5-methoxy-1-oxypyridin-2-yl |
| 399 | | Br | H | $CH_2$ | 3-Bromo-4,6-dimethyl-5-methoxy-1-oxypyridin-2-yl |
| 400 | | Br | H | $CH_2$ | 4-Bromo-5,6-dimethoxy-pyridin-2-yl |
| 401 | | Br | H | $CH_2$ | 4-Bromo-5,6-dimethoxy-1-oxypyridin-2-yl |
| 402 | | Br | H | $CH_2$ | 3,4-Dibromo-5,6-dimethoxy-pyridin-2-yl |
| 403 | | Br | H | $CH_2$ | 3-Chloro-4-bromo-5,6-dimethoxy-pyridin-2-yl |
| 404 | | Br | H | $CH_2$ | 3-Chloro-4-bromo-5,6-dimethoxy-1-oxypyridin-2-yl |
| 405 | | Br | H | $CH_2$ | 3,4-Dibromo-5,6-dimethoxy-1-oxypyridin-2-yl |
| 406 | | Br | H | $CH_2$ | 4,6-Dimethyl-5-methoxypyridin-3-yl |
| 407 | | Br | H | $CH_2$ | 4,6-Dimethyl-5-methoxy-1-oxypyridin-3-yl |
| 408 | | Br | H | $CH_2$ | 4,6-Dimethyl-5-bromopyridin-3-yl |
| 409 | | Br | H | $CH_2$ | 4,6-Dimethyl-5-chloropyridin-3-yl |
| 410 | | Br | H | $CH_2$ | 5,6-Dimethyl-4-bromopyridin-3-yl |
| 411 | | Br | H | $CH_2$ | 5,6-Dimethyl-4-chloropyridin-3-yl |
| 412 | | Br | H | $CH_2$ | 4,6-Dimethyl-5-bromo-1-oxypyridin-pyridin-3-yl |
| 413 | | Br | H | $CH_2$ | 4,6-Dimethyl-5-chloro-1-oxypyridin-pyridin-3-yl |
| 414 | | Br | H | $CH_2$ | 5,6-Dimethyl-4-bromo-1-oxypyridin-pyridin-3-yl |
| 415 | | Br | H | $CH_2$ | 5,6-Dimethyl-4-chloro-1-oxypyridin-pyridin-3-yl |
| 416 | | Br | H | $CH_2$ | 2,6-Dimethyl-3-methoxypyridin-4-yl |
| 417 | | Br | H | $CH_2$ | 2,6-Dimethyl-pyridin-4-yl |
| 418 | | Br | H | $CH_2$ | 2,3,6-Trimethyl-pyridin-4-yl |
| 419 | | Br | H | $CH_2$ | 2,3,6-Trimethoxy-pyridin-4-yl |

(continued)

| No. | Ex. | $R^1$ | $R^3$ | $R^4$ | $R^5$ |
|---|---|---|---|---|---|
| 420 | | Br | H | $CH_2$ | 2,6-Dimethyl-3-bromopyridin-4-yl |
| 421 | | Br | H | $CH_2$ | 2,6-Dimethyl-3-chloropyridin-4-yl |
| 422 | | Br | H | $CH_2$ | 2,6-Dichloro-3-bromopyridin-4-yl |
| 423 | | Br | H | $CH_2$ | 2,6-Dibromo-3-chloropyridin-4-yl |
| 424 | | Br | H | $CH_2$ | 2,3,6-Trichloro-pyridin-4-yl |
| 425 | | Br | H | $CH_2$ | 2,3,6-Tribromo-pyridin-4-yl |
| 426 | | Br | H | $CH_2$ | 2,6-Dimethyl-3-methoxy-1-oxy-pyridin-4-yl |
| 427 | | Br | H | $CH_2$ | 2,6-Dimethyl-1-oxy-pyridin-4-yl |
| 428 | | Br | H | $CH_2$ | 2,3,6-Trimethyl-1-oxy-pyridin-4-yl |
| 429 | | Br | H | $CH_2$ | 2,3,6-Trimethoxy-1-oxy-pyridin-4-yl |
| 430 | | Br | H | $CH_2$ | 2,6-Dimethyl-3-bromol-oxy-pyridin-4-yl |
| 431 | | Br | H | $CH_2$ | 2,6-Dimethyl-3-chlorol-oxy-pyridin-4-yl |
| 432 | | Br | H | $CH_2$ | 2,6-Dichloro-3-bromol-oxy-pyridin-4-yl |
| 433 | | Br | H | $CH_2$ | 2,6-Dibromo-3-chlorol-oxy-pyridin-4-yl |
| 434 | | Br | H | $CH_2$ | 2,3,6-Trichloro-1-oxy-pyridin-4-yl |
| 435 | | Br | H | $CH_2$ | 2,3,6-Tribromo-1-oxy-pyridin-4-yl |
| 436 | | Br | H | $CH_2$ | 4,6-Dimethyl-5-iodopyridin-3-yl |
| 437 | | Br | H | $CH_2$ | 5,6-Dimethyl-4-iodopyridin-3-yl |
| 438 | | Br | H | $CH_2$ | 4,5,6-Trichloropyridin-3-yl |
| 439 | | Br | H | $CH_2$ | 4,5,6-Tribromopyridin-3-yl |
| 440 | | F | H | $CH_2$ | 3,5-Dimethyl-4-methoxypyridin-2-yl |
| 441 | | F | H | $CH_2$ | 3,5-Dimethyl-4-methoxy-1-oxypyridin-2-yl |
| 442 | | F | H | $CH_2$ | 6-Bromo-3,5-dimethyl-4-methoxypyridin-2-yl |
| 443 | | F | H | $CH_2$ | 6-Chloro-3,5-dimethyl-4-methoxypyridin-2-yl |
| 444 | | F | H | $CH_2$ | 6-Chloro-3,5-dimethyl-4-methoxy-1-oxypyridin-2-yl |
| 445 | | F | H | $CH_2$ | 6-Bromo-3,5-dimethyl-4-methoxy-1-oxypyridin-2-yl |
| 446 | | F | H | $CH_2$ | 3,5-Dimethyl-4-bromopyridin-2-yl |
| 447 | | F | H | $CH_2$ | 3,5-Dimethyl-4-bromo-1-oxypyridin-2-yl |
| 448 | | F | H | $CH_2$ | 6-Bromo-3,5-dimethyl-4-bromopyridin-2-yl |
| 449 | | F | H | $CH_2$ | 6-Chloro-3,5-dimethyl-4-bromopyridin-2-yl |
| 450 | | F | H | $CH_2$ | 6-Chloro-3,5-dimethyl-4-bromo-1-oxypyridin-2-yl |
| 451 | | F | H | $CH_2$ | 4,6-Dibromo-3,5-dimethyl-1-oxypyridin-2-yl |
| 452 | | F | H | $CH_2$ | 3,5-Dimethyl-4-chloropyridin-2-yl |
| 453 | | F | H | $CH_2$ | 3,5-Dimethyl-4-chloro-1-oxypyridin-2-yl |
| 454 | | F | H | $CH_2$ | 6-Bromo-3,5-dimethyl-4-chloropyridin-2-yl |
| 455 | | F | H | $CH_2$ | 6-Chloro-3,5-dimethyl-4-chloropyridin-2-yl |
| 456 | | F | H | $CH_2$ | 4,6-Dichloro-3,5-dimethyl-1-oxypyridin-2-yl |
| 457 | | F | H | $CH_2$ | 6-Bromo-3,5-dimethyl-4-chloro-1-oxypyridin-2-yl |

(continued)

| No. | Ex. | $R^1$ | $R^3$ | $R^4$ | $R^5$ |
|---|---|---|---|---|---|
| 458 | | F | H | $CH_2$ | 3,5-Dimethyl-4-iodopyridin-2-yl |
| 459 | | F | H | $CH_2$ | 3,5-Dimethyl-4-iodo-1-oxypyridin-2-yl |
| 460 | | F | H | $CH_2$ | 6-Bromo-3,5-dimethyl-4-iodopyridin-2-yl |
| 461 | | F | H | $CH_2$ | 6-Chloro-3,5-dimethyl-4-iodopyridin-2-yl |
| 462 | | F | H | $CH_2$ | 6-Chloro-3,5-dimethyl-4-iodo-1-oxypyridin-2-yl |
| 463 | | F | H | $CH_2$ | 6-Bromo-3,5-dimethyl-4-iodo-1-oxypyridin-2-yl |
| 464 | | F | H | $CH_2$ | 3,5-Dimethyl-4-thiomethyl-pyridin-2-yl |
| 465 | | F | H | $CH_2$ | 3,5-Dimethyl-4-thiomethyl-1-oxypyridin-2-yl |
| 466 | | F | H | $CH_2$ | 6-Bromo-3,5-dimethyl-4-thiomethyl-pyridin-2-yl |
| 467 | | F | H | $CH_2$ | 6-Chloro-3,5-dimethyl-4-thiomethyl-pyridin-2-yl |
| 468 | | F | H | $CH_2$ | 6-Chloro-3,5-dimethyl-4-thiomethyl-1-oxypyridin-2-yl |
| 469 | | F | H | $CH_2$ | 6-Bromo-3,5-dimethyl-4-thiomethyl-1-oxypyidin-2-yl |
| 470 | | F | H | $CH_2$ | 3,4,5-Trimethyl-pyridin-2-yl |
| 471 | | F | H | $CH_2$ | 3,4,5-Trimethyl-1-oxypyridin-2-yl |
| 472 | | F | H | $CH_2$ | 6-Bromo-3,4,5-trimethyl-pyridin-2-yl |
| 473 | | F | H | $CH_2$ | 6-Chloro-3,4,5-trimethyl-pyridin-2-yl |
| 474 | | F | H | $CH_2$ | 6-Chloro-3,4,5-trimethyl-1-oxypyridin-2-yl |
| 475 | | F | H | $CH_2$ | 6-Bromo-3,4,5-trimethyl-1-oxypyridin-2-yl |
| 476 | | F | H | $CH_2$ | 3,4,5-Trimethoxy-pyridin-2-yl |
| 477 | | F | H | $CH_2$ | 3,4,5-Trimethoxy-1-oxypyridin-2-yl |
| 478 | | F | H | $CH_2$ | 6-Bromo-3,4,5-trimethoxy-pyridin-2-yl |
| 479 | | F | H | $CH_2$ | 6-Chloro-3,4,5-trimethoxy-pyridin-2-yl |
| 480 | | F | H | $CH_2$ | 6-Chloro-3,4,5-trimethoxy-1-oxypyridin-2-yl |
| 481 | | F | H | $CH_2$ | 6-Bromo-3,4,5-trimethoxy-1-oxypyridin-2-yl |
| 482 | | F | H | $CH_2$ | 4,5,6-Trimethoxy-pyridin-2-yl |
| 483 | | F | H | $CH_2$ | 4,5,6-Trimethoxy-1-oxypyridin-2-yl |
| 484 | | F | H | $CH_2$ | 3-Bromo-4,5,6-trimethoxy-pyridin-2-yl |
| 485 | | F | H | $CH_2$ | 3-Chloro-4,5,6-trimethoxy-pyridin-2-yl |
| 486 | | F | H | $CH_2$ | 3-Chloro-4,5,6-trimethoxy-1-oxypyridin-2-yl |
| 487 | | F | H | $CH_2$ | 3-Bromo-4,5,6-trimethoxy-1-oxypyridin-2-yl |
| 488 | | F | H | $CH_2$ | 4,5,6-Trimethyl-pyridin-2-yl |
| 489 | | F | H | $CH_2$ | 4,5,6-Trimethyl-1-oxypyridin-2-yl |
| 490 | | F | H | $CH_2$ | 3-Bromo-4,5,6-trimethyl-pyridin-2-yl |
| 491 | | F | H | $CH_2$ | 3-Chloro-4,5,6-trimethyl-pyridin-2-yl |
| 492 | | F | H | $CH_2$ | 3-Chloro-4,5,6-trimethyl-1-oxypyridin-2-yl |
| 493 | | F | H | $CH_2$ | 3-Bromo-4,5,6-trimethyl-1-oxypyridin-2-yl |
| 494 | | F | H | $CH_2$ | 4,6-Dimethyl-5-methoxy-pyridin-2-yl |
| 495 | | F | H | $CH_2$ | 4,6-Dimethyl-5-methoxy-1-oxypyridin-2-yl |

(continued)

| No. | Ex. | $R^1$ | $R^3$ | $R^4$ | $R^5$ |
|---|---|---|---|---|---|
| 496 | | F | H | $CH_2$ | 3-Bromo-4,6-dimethyl-5-methoxy-pyridin-2-yl |
| 497 | | F | H | $CH_2$ | 3-Chloro-4,6-dimethyl-5-methoxy-pyridin-2-yl |
| 498 | | F | H | $CH_2$ | 3-Chloro-4,6-dimethyl-5-methoxy-1-oxypyridin-2-yl |
| 499 | | F | H | $CH_2$ | 3-Bromo-4,6-dimethyl-5-methoxy-1-oxypyridin-2-yl |
| 500 | | F | H | $CH_2$ | 4-Bromo-5,6-dimethoxy-pyridin-2-yl |
| 501 | | F | H | $CH_2$ | 4-Bromo-5,6-dimethoxy-1-oxypyridin-2-yl |
| 502 | | F | H | $CH_2$ | 3,4-Dibromo-5,6-dimethoxy-pyridin-2-yl |
| 503 | | F | H | $CH_2$ | 3-Chloro-4-bromo-5,6-dimethoxy-pyridin-2-yl |
| 504 | | F | H | $CH_2$ | 3-Chloro-4-bromo-5,6-dimethoxy-1-oxypyridin-2-yl |
| 505 | | F | H | $CH_2$ | 3,4-Dibromo-5,6-dimethoxy-1-oxypyridin-2-yl |
| 506 | | F | H | $CH_2$ | 4,6-Dimethyl-5-methoxypyridin-3-yl |
| 507 | | F | H | $CH_2$ | 4,6-Dimethyl-5-methoxy-1-oxypyridin-3-yl |
| 508 | | F | H | $CH_2$ | 4,6-Dimethyl-5-bromopyridin-3-yl |
| 509 | | F | H | $CH_2$ | 4,6-Dimethyl-5-chloropyridin-3-yl |
| 510 | | F | H | $CH_2$ | 5,6-Dimethyl-4-bromopyridin-3-yl |
| 511 | | F | H | $CH_2$ | 5,6-Dimethyl-4-chloropyridin-3-yl |
| 512 | | F | H | $CH_2$ | 4,6-Dimethyl-5-bromo-1-oxypyridin-pyridin-3-yl |
| 513 | | F | H | $CH_2$ | 4,6-Dimethyl-5-chloro-1-oxypyridin-pyridin-3-yl |
| 514 | | F | H | $CH_2$ | 5,6-Dimethyl-4-bromo-1-oxypyridin-pyridin-3-yl |
| 515 | | F | H | $CH_2$ | 5,6-Dimethyl-4-chloro-1-oxypyridin-pyridin-3-yl |
| 516 | | F | H | $CH_2$ | 2,6-Dimethyl-3-methoxypyridin-4-yl |
| 517 | | F | H | $CH_2$ | 2,6-Dimethyl-pyridin-4-yl |
| 518 | | F | H | $CH_2$ | 2,3,6-Trimethyl-pyridin-4-yl |
| 519 | | F | H | $CH_2$ | 2,3,6-Trimethoxy-pyridin-4-yl |
| 520 | | F | H | $CH_2$ | 2,6-Dimethyl-3-bromopyridin-4-yl |
| 521 | | F | H | $CH_2$ | 2,6-Dimethyl-3-chloropyridin-4-yl |
| 522 | | F | H | $CH_2$ | 2,6-Dichloro-3-bromopyridin-4-yl |
| 523 | | F | H | $CH_2$ | 2,6-Dibromo-3-chloropyridin-4-yl |
| 524 | | F | H | $CH_2$ | 2,3,6-Trichloro-pyridin-4-yl |
| 525 | | F | H | $CH_2$ | 2,3,6-Tribromo-pyridin-4-yl |
| 526 | | F | H | $CH_2$ | 2,6-Dimethyl-3-methoxy-1-oxy-pyridin-4-yl |
| 527 | | F | H | $CH_2$ | 2,6-Dimethyl-1-oxy-pyridin-4-yl |
| 528 | | F | H | $CH_2$ | 2,3,6-Trimethyl-1-oxy-pyridin-4-yl |
| 529 | | F | H | $CH_2$ | 2,3,6-Trimethoxy-1-oxy-pyridin-4-yl |
| 530 | | F | H | $CH_2$ | 2,6-Dimethyl-3-bromol-oxy-pyridin-4-yl |
| 531 | | F | H | $CH_2$ | 2,6-Dimethyl-3-chlorol-oxy-pyridin-4-yl |
| 532 | | F | H | $CH_2$ | 2,6-Dichloro-3-bromol-oxy-pyridin-4-yl |
| 533 | | F | H | $CH_2$ | 2,6-Dibromo-3-chlorol-oxy-pyridin-4-yl |

(continued)

| No. | Ex. | $R^1$ | $R^3$ | $R^4$ | $R^5$ |
|---|---|---|---|---|---|
| 534 | | F | H | $CH_2$ | 2,3,6-Trichloro-1-oxy-pyridin-4-yl |
| 536 | | F | H | $CH_2$ | 2,3,6-Tribromo-1-oxy-pyridin-4-yl |
| 537 | | F | H | $CH_2$ | 4,6-Dimethyl-5-iodopyridin-3-yl |
| 538 | | F | H | $CH_2$ | 5,6-Dimethyl-4-iodopyridin-3-yl |
| 539 | | F | H | $CH_2$ | 4,5,6-Trichloropyridin-3-yl |
| 540 | | Cl | Cl | $CH_2$ | 3,5-Dimethyl-4-methoxypyridin-2-yl |
| 541 | | Br | Cl | $CH_2$ | 3,5-Dimethyl-4-methoxypyridin-2-yl |
| 542 | | Cl | Br | $CH_2$ | 3,5-Dimethyl-4-methoxypyridin-2-yl |
| 543 | | Br | Br | $CH_2$ | 3,5-Dimethyl-4-methoxypyridin-2-yl |
| 544 | | Cl | $N_3$ | $CH_2$ | 3,5-Dimethyl-4-methoxypyridin-2-yl |
| 545 | | Br | $N_3$ | $CH_2$ | 3,5-Dimethyl-4-methoxypyridin-2-yl |
| 546 | | F | Cl | $CH_2$ | 3,5-Dimethyl-4-methoxypyridin-2-yl |
| 547 | | F | Br | $CH_2$ | 3,5-Dimethyl-4-methoxypyridin-2-yl |
| 548 | | Cl | CN | $CH_2$ | 3,5-Dimethyl-4-methoxypyridin-2-yl |
| 549 | | Br | CN | $CH_2$ | 3,5-Dimethyl-4-methoxypyridin-2-yl |
| 550 | | Cl | Cl | $CH_2$ | 3,5-Dimethyl-4-bromopyridin-2-yl |
| 551 | | Br | Cl | $CH_2$ | 3,5-Dimethyl-4-bromopyridin-2-yl |
| 552 | | Cl | Br | $CH_2$ | 3,5-Dimethyl-4-bromopyridin-2-yl |
| 553 | | Br | Br | $CH_2$ | 3,5-Dimethyl-4-bromopyridin-2-yl |
| 554 | | Cl | $N_3$ | $CH_2$ | 3,5-Dimethyl-4-bromopyridin-2-yl |
| 555 | | Br | $N_3$ | $CH_2$ | 3,5-Dimethyl-4-bromopyridin-2-yl |
| 556 | | F | Cl | $CH_2$ | 3,5-Dimethyl-4-bromopyridin-2-yl |
| 557 | | F | Br | $CH_2$ | 3,5-Dimethyl-4-bromopyridin-2-yl |
| 558 | | Cl | CN | $CH_2$ | 3,5-Dimethyl-4-bromopyridin-2-yl |
| 559 | | Br | CN | $CH_2$ | 3,5-Dimethyl-4-bromopyridin-2-yl |
| 560 | | Cl | Cl | $CH_2$ | 3,5-Dimethyl-4-chloropyridin-2-yl |
| 561 | | Br | Cl | $CH_2$ | 3,5-Dimethyl-4-chloropyridin-2-yl |
| 562 | | Cl | Br | $CH_2$ | 3,5-Dimethyl-4-chloropyridin-2-yl |
| 563 | | Br | Br | $CH_2$ | 3,5-Dimethyl-4-chloropyridin-2-yl |
| 564 | | Cl | $N_3$ | $CH_2$ | 3,5-Dimethyl-4-chloropyridin-2-yl |
| 565 | | Br | $N_3$ | $CH_2$ | 3,5-Dimethyl-4-chloropyridin-2-yl |
| 566 | | F | Cl | $CH_2$ | 3,5-Dimethyl-4-chloropyridin-2-yl |
| 567 | | F | Br | $CH_2$ | 3,5-Dimethyl-4-chloropyridin-2-yl |
| 568 | | Cl | CN | $CH_2$ | 3,5-Dimethyl-4-chloropyridin-2-yl |
| 569 | | Br | CN | $CH_2$ | 3,5-Dimethyl-4-chloropyridin-2-yl |
| 570 | | Cl | Cl | $CH_2$ | 3,5-Dimethyl-4-iodopyridin-2-yl |
| 571 | | Br | Cl | $CH_2$ | 3,5-Dimethyl-4-iodopyridin-2-yl |
| 572 | | Cl | Br | $CH_2$ | 3,5-Dimethyl-4-iodopyridin-2-yl |

(continued)

| No. | Ex. | $R^1$ | $R^3$ | $R^4$ | $R^5$ |
|---|---|---|---|---|---|
| 573 | | Br | Br | $CH_2$ | 3,5-Dimethyl-4-iodopyridin-2-yl |
| 574 | | Cl | $N_3$ | $CH_2$ | 3,5-Dimethyl-4-iodopyridin-2-yl |
| 575 | | Br | $N_3$ | $CH_2$ | 3,5-Dimethyl-4-iodopyridin-2-yl |
| 576 | | F | Cl | $CH_2$ | 3,5-Dimethyl-4-iodopyridin-2-yl |
| 577 | | F | Br | $CH_2$ | 3,5-Dimethyl-4-iodopyridin-2-yl |
| 578 | | Cl | CN | $CH_2$ | 3,5-Dimethyl-4-iodopyridin-2-yl |
| 579 | | Br | CN | $CH_2$ | 3,5-Dimethyl-4-iodopyridin-2-yl |
| 580 | | Cl | Cl | $CH_2$ | 4,5,6-Trimethoxy-3-chloropyridin-2-yl |
| 581 | | Br | Cl | $CH_2$ | 4,5,6-Trimethoxy-3-chloropyridin-2-yl |
| 582 | | Cl | Br | $CH_2$ | 4,5,6-Trimethoxy-3-chloropyridin-2-yl |
| 583 | | Br | Br | $CH_{2.}$ | 4,5,6-Trimethoxy-3-chloropyridin-2-yl |
| 584 | | Cl | Cl | $CH_2$ | 4,5,6-Trimethoxy-3-bromopyridin-2-yl |
| 585 | | Br | Cl | $CH_2$ | 4,5,6-Trimethoxy-3-bromopyridin-2-yl |
| 586 | | Cl | Br | $CH_2$ | 4,5,6-Trimethoxy-3-bromopyridin-2-yl |
| 587 | | Br | Br | $CH_2$ | 4,5,6-Trimethoxy-3-bromopyridin-2-yl |
| 588 | 7 | Cl | H | $CH_2$ | 3,5-Dimethyl-4-hydroxypyridin-2-yl |
| 589 | 8 | Cl | H | $CH_2$ | 3,5-Dimethyl-4-ethoxypyridin-2-yl |
| 590 | 9 | Cl | H | $CH_2$ | 3,5-Dimethyl-4-allyloxypyridin-2-yl |
| 591 | 10 | Cl | H | $CH_2$ | 3,5-Dimethyl-4-(2-ethoxy-ethoxy)pyridin-2-yl |
| 592 | 11 | Cl | H | $CH_2$ | 3,5-Dimethyl-4isopropoxypyridin-2-yl |
| 593 | 12 | Cl | H | $CH_2$ | 3,5-Dimethyl-4-cyclopropylmethoxypyridin-2-yl |
| 594 | 13 | Cl | H | $CH_2$ | 3,5-Dimethyl-4-(3-methyl-butoxy)pyridin-2-yl |
| 595 | 14 | Cl | H | $CH_2$ | 3,5-Dimethyl-4-isobutoxypyridin-2-yl |
| 596 | 15 | Cl | H | $CH_2$ | 3,5-Dimethyl-4-(2-acetyloxy-ethoxy)pyridin-2-yl |
| 597 | 16 | Cl | H | $CH_2$ | 3,5-Dimethyl-4-(3-acetyloxy-propoxy)pyridin-2-yl |
| 598 | 17 | Cl | H | $CH_2$ | 3,5-Dimethyl-4-propoxypyridin-2-yl |
| 599 | 20 | Cl | H | $CH_2$ | 3,5-Dimethylpyridin-2-yl |
| 600 | 32 | Cl | H | $CH_2$ | 3,5-Dimethyl-4-methoxy-1-methoxypyridin-2-yl |
| 601 | 34 | Cl | H | $CH_2$ | 5-Methoxy-4-methoxymethyl-6-methylpyridin-3-yl |
| 602 | 35 | Cl | H | $CH_2$ | 5-Ethoxy-4-hydroxymethyl-6-methylpyridin-3-yl |
| 604 | 36 | Cl | H | $CH_2$ | 3,5-Dimethyl-4-aminopyridin-2-yl |
| 605 | 37 | Cl | H | $CH_2$ | 3-Methoxy-5-methoxymethyl-4-methylpyridin-2-yl |
| 606 | 38 | Cl | H | $CH_2$ | 5-Chloro-6-methoxypyridin-3-yl |
| 607 | 39 | Cl | H | $CH_2$ | 3,4-Dimethoxypyridin-2-yl |
| 608 | 40 | Cl | H | $CH_2$ | 3-Methoxy-6-methylpyridin-2-yl |
| 609 | 42 | H | H | $CH_2$ | 4-Methoxy-3,5-dimethylpyridin-2-yl |
| 610 | 45 | Cl | Br | $CH_2$ | 2-Bromo-3,5-dimethoxy-4-methylphenyl |
| 611 | 47 | Cl | Bu | $CH_2$ | 3,4,5-Trimethoxyphenyl |

(continued)

| No. | Ex. | $R^1$ | $R^3$ | $R^4$ | $R^5$ |
|---|---|---|---|---|---|
| 612 | 49 | Cl | H | $CH_2$ | 2-Bromo-3,5-dimethoxy-4-acetyloxyphenyl |
| 613 | 50 | Cl | H | $CH_2$ | 2-Bromo-3,5-dimethoxy-4-hydroxyphenyl |
| 614 | 52 | Cl | H | $CH_2$ | 2-Bromo-3,5-dimethoxy-4-allyloxyphenyl |
| 615 | 53 | Cl | H | $CH_2$ | 2-Bromo-3,5-dimethoxy-4-chloromethyloxyphenyl |
| 616 | 54 | Cl | H | $CH_2$ | 2-Bromo-3,5-dimethoxy-4-chloroethyloxyphenyl |
| 617 | 55 | Cl | H | $CH_2$ | 2-Bromo-3,5-dimethoxy-4-cyclopropylmethoxyphenyl |
| 618 | 56 | Cl | H | $CH_2$ | 2-Bromo-3,5-dimethoxy-4-ethoxyphenyl |
| 619 | 57 | Cl | H | $CH_2$ | 2-Bromo-3,5-dimethoxy-4-propoxyphenyl |
| 620 | 58 | Cl | H | $CH_2$ | 2-Bromo-3,5-dimethoxy-4-butoxyphenyl |
| 621 | 59 | Cl | H | $CH_2$ | 3-Methoxymethyloxy-6-imethylpyridin-2-yl |
| 622 | 60 | Cl | H | $CH_2$ | 3H-Benzothiazole-2-thione |
| 623 | 61 | Cl | H | $CH_2$ | 2,5-Dimethylphenyl |
| 624 | 62 | Cl | H | $CH_2$ | Isoquinolin-1-yl |
| 625 | 63 | Cl | H | $CH_2$ | Benzo[1,2,5]thiadiazol-5-yl |
| 626 | 64 | Cl | H | $CH_2$ | 1-Methyl-1H-benzotriazol-5-yl |
| 627 | 65 | Cl | H | $CH_2$ | 6-Chloro-benzo[1,2,5]thiadiazol-5-yl |
| 628 | 66 | Cl | H | $CH_2$ | Benzo[1,2,5]thiadiazol-4-yl |
| 629 | 67 | Cl | H | $CH_2$ | 6-Fluoro-4a,8a-dihydro-4H-benzo[1,3]dioxin-8-yl |
| 630 | 68 | Cl | H | $CH_2$ | 2-Methoxy-4-acetylphenyl |
| 631 | 69 | Cl | H | $CH_2$ | 3-Trifluoromethoxyphenyl |
| 632 | 70 | Cl | H | $CH_2$ | 2-Fluoro-3-trifluoromethylphenyl |
| 633 | 71 | Cl | H | $CH_2$ | 2-Fluoro-4,5-dimethoxyphenyl |
| 634 | 72 | Cl | H | $CH_2$ | 2,3-Dimethoxyphenyl |
| 635 | 73 | Cl | H | $CH_2$ | 3,4-Dimethoxyphenyl |
| 636 | 74 | Me | H | $CH_2$ | 2-Chloro-3,4,5-trimethoxyphenyl |
| 637 | 75 | Cl | H | $CH_2$ | 2-Chloro-4,5-dimethoxyphenyl |
| 638 | 76 | Cl | H | $CH_2$ | 2-Iodo-4,5-dimethoxyphenyl |
| 639 | 78 | Cl | H | $CH_2$ | 6-Chloro-benzo[1,3]dioxol-5-yl |
| 640 | 79 | Cl | H | $CH_2$ | 2,4-Dimethoxy-3-methylphenyl |
| 641 | 80 | Cl | H | $CH_2$ | 2-Chloro-3,4-dimethoxyphenyl |
| 642 | 81 | Cl | H | $CH_2$ | 3-Methoxyphenyl |
| 643 | 82 | Cl | H | $CH_2$ | 2,6-Dibromo-3,5-dimethoxyphenyl |
| 644 | 83 | Cl | H | $CH_2$ | 2-Bromo-3,5-dimethoxyphenyl |
| 645 | 84 | Cl | H | $CH_2$ | 3,5-Dimethoxyphenyl |
| 646 | 86 | Cl | H | $CH_2$ | 2,5-Dimethoxyphenyl |
| 647 | 87 | Cl | Br | $CH_2$ | 2,5-Dimethoxyphenyl |
| 648 | 88 | Cl | H | $CH_2$ | 2-Nitro-4,5-dimethoxyphenyl |
| 649 | 89 | Cl | Br | $CH_2$ | 2-Nitro-4,5-dimethoxyphenyl |

(continued)

| No. | Ex. | $R^1$ | $R^3$ | $R^4$ | $R^5$ |
|---|---|---|---|---|---|
| 650 | 90 | Cl | H | $CH_2$ | 2,5-Dichlorophenyl |
| 651 | 91 | Cl | H | $CH_2$ | 2,3,5-Trifluorophenyl |
| 652 | 92 | Cl | H | C(O) | 3,4,5-Trimethoxyphenyl |
| 653 | 95 | Cl | H | $CH_2$ | 3,5-Dichlorophenyl |
| 654 | 96 | Cl | H | $CH_2$ | 3,4-Dichlorophenyl |
| 655 | 97 | Cl | Br | $CH_2$ | 3,4-Dichlorophenyl |
| 656 | 99 | Cl | H | $CH_2$ | 2,6-Dichloro-3,4,5-trimethoxyphenyl |
| 657 | 100 | OH | H | $CH_2$ | 2-Chloro-3,4,5-trimethoxyphenyl |
| 658 | 103 | SEt | H | $CH_2$ | 2-Bromo-3,4,5-trimethoxyphenyl |
| 659 | 104 | OMe | H | $CH_2$ | 2-Bromo-3,4,5-trimethoxyphenyl |
| 660 | 105 | $NH_2$ | H | $CH_2$ | 2-Bromo-3,4,5-trimethoxyphenyl |
| 661 | 107 | Cl | H | $CH_2$ | 2-Bromo-3,5-dimethoxy-4-methoxymethyloxyphenyl |
| 662 | 108 | Cl | H | $CH_2$ | Benzothiazol-2-yl |
| 663 | 109 | Cl | H | $CH_2$ | 4-Methoxyphenyl |
| 664 | 110 | Cl | H | $CH_2$ | 2-Bromo-4,5-dimethoxyphenyl |
| 665 | 112 | Cl | H | $CH_2$ | 4-Methylquinolin-2-yl |
| 666 | 113 | Br | H | $CH_2$ | 4-Methylquinolin-2-yl |
| 667 | 114 | Br | H | $CH_2$ | 4-Methyl-1-oxy-quinolin-2-yl |
| 668 | 116 | Cl | H | $CH_2$ | 6-Chloro-benzothiazol-2-yl |
| 669 | | Cl | H | $CH_2$ | 4,5,6-Trimethoxy-pyridin-3-yl |
| 670 | | Cl | H | $CH_2$ | 4,5,6-Trimethoxy-1-oxy-pyridin-3-yl |
| 671 | | Cl | H | $CH_2$ | 2-Bromo-4,5,6-trimethoxy-pyridin-3-yl |
| 672 | | Cl | H | $CH_2$ | 2-Chloro-4,5,6-trimethoxy-pyridin-3-yl |
| 673 | | Cl | H | $CH_2$ | 2-Chloro-4,5,6-trimethoxy-1-oxy-pyridin-3-yl |
| 674 | | Cl | H | $CH_2$ | 2-Bromo-4,5,6-trimethoxy-1-oxy-pyridin-3-yl |
| 675 | | Cl | H | $CH_2$ | 4,5,6-Trimethyl-pyridin-3-yl |
| 676 | | Cl | H | $CH_2$ | 4,5,6-Trimethyl-1-oxy-pyridin-3-yl |
| 677 | | Cl | H | $CH_2$ | 2-Bromo-4,5,6-trimethyl-pyridin-3-yl |
| 678 | | Cl | H | $CH_2$ | 2-Chloro-4,5,6-trimethyl-pyridin-3-yl |
| 679 | | Cl | H | $CH_2$ | 2-Chloro-4,5,6-trimethyl-1-oxy-pyridin-3-yl |
| 680 | | Cl | H | $CH_2$ | 2-Bromo-4,5,6-trimethyl-1-oxy-pyridin-3-yl |
| 681 | | Cl | H | $CH_2$ | 2-Iodo-4,5,6-trimethyl-pyridin-3-yl |
| 682 | | Cl | H | $CH_2$ | 2-Iodo-4,5,6-trimethyl-pyridin-3-yl |
| 683 | | Br | H | $CH_2$ | 4,5,6-Trimethoxy-pyridin-3-yl |
| 684 | | Br | H | $CH_2$ | 4,5,6-Trimethoxy-1-oxy-pyridin-3-yl |
| 685 | | Br | H | $CH_2$ | 2-Bromo-4,5,6-trimethoxy-pyridin-3-yl |
| 686 | | Br | H | $CH_2$ | 2-Chloro-4,5,6-trimethoxy-pyridin-3-yl |
| 687 | | Br | H | $CH_2$ | 2-Chloro-4,5,6-trimethoxy-1-oxy-pyridin-3-yl |

(continued)

| No. | Ex. | $R^1$ | $R^3$ | $R^4$ | $R^5$ |
|---|---|---|---|---|---|
| 688 | | Br | H | $CH_2$ | 2-Bromo-4,5,6-trimethoxy-1-oxy-pyridin-3-yl |
| 689 | | Br | H | $CH_2$ | 4,5,6-Trimethyl-pyridin-3-yl |
| 690 | | Br | H | $CH_2$ | 4,5,6-Trimethyl-1-oxy-pyridin-3-yl |
| 691 | | Br | H | $CH_2$ | 2-Bromo-4,5,6-trimethyl-pyridin-3-yl |
| 692 | | Br | H | $CH_2$ | 2-Chloro-4,5,6-trimethyl-pyridin-3-yl |
| 693 | | Br | H | $CH_2$ | 2-Chloro-4,5,6-trimethyl-1-oxy-pyridin-3-yl |
| 694 | | Br | H | $CH_2$ | 2-Bromo-4,5,6-trimethyl-1-oxy-pyridin-3-yl |
| 695 | | I | H | $CH_2$ | 3,5-Dimethyl-4-methoxypyridin-2-yl |
| 696 | | I | H | $CH_2$ | 3,5-Dimethyl-4-methoxy-1-oxypyridin-2-yl |
| 697 | | I | H | $CH_2$ | 3-Bromo-4,5,6-trimethoxypyridin-2-yl |
| 698 | | I | H | $CH_2$ | 4,5,6-Trimethoxypyridin-2-yl |
| 699 | | I | H | $CH_2$ | 6-Chloro-3,5-dimethyl-4-methoxy-pyridin-2-yl |
| 700 | | I | H | $CH_2$ | 3,5-dimethyl-4-thiomethyl-1-oxypyridin-2-yl |
| 701 | | I | H | $CH_2$ | 3,5-Dimethyl-4-bromopyridin-2-yl |
| 702 | | I | H | $CH_2$ | 3,5-Dimethyl-4-bromo-1-oxypyridin-2-yl |
| 703 | | I | H | $CH_2$ | 3-Chloro-4,5,6-trimethoxypyridin-2-yl |
| 704 | | I | H | $CH_2$ | 3,4,5-Trimethoxyphenyl |
| 705 | | I | H | $CH_2$ | 2-Chloro-3,4,5-trimethoxyphenyl |
| 706 | | I | H | $CH_2$ | 2-Bromo-3,4,5-trimethoxyphenyl |
| 707 | | I | H | $CH_2$ | 2-Iodo-3,4,5-trimethoxyphenyl |
| 708 | | I | H | $CH_2$ | 3,4,5-Trimethylpyridin-2-yl |
| 709 | | I | H | $CH_2$ | 4,5,6-Trimethylpyridin-3-yl |
| 710 | | I | H | $CH_2$ | 3,5-dimethyl-4-thiomethylpyridin-2-yl |
| 711 | | I | H | $CH_2$ | 4,6-Dimethyl-5-methoxypyridin-3-yl |
| 712 | | I | H | $CH_2$ | 4,6-Dimethyl-5-methoxy-1-oxypyridin-3-yl |
| 713 | | I | H | $CH_2$ | 3,5-Dimethyl-4-chloropyridin-2-yl |
| 714 | | I | H | $CH_2$ | 3,5-Dimethyl-4-iodopyridin-2-yl |

Compounds of interest in Table 2 are: 2, 3, 11, 44, 82, 83, 242, 243, 245, 248, 249, 254, 255, 260, 266, 272, 273, 278, 279, 284, 286, 287, 308, 309, 343, 348, 349, 354, 366, 367, 372, 373, 671 and 697 (with those selected being 2, 242, 243, 248, 254, 260, 266, 272, 278, 284, 286, 287, 308, 343, 348, 349, 354, 372, 373, 671 and 697.)

**TABLE 3: EXEMPLARY COMPOUNDS OF FORMULA IIIA, $R^2$ is = $NH_2$**

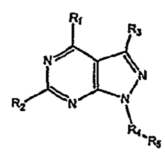

IIIA

(continued)

| No. | Ex | $R^1$ | $R^3$ | $R^4$ | $R^5$ |
|---|---|---|---|---|---|
| 1 | 5 | Cl | H | $CH_2$ | 3,4,5-Trimethoxyphenyl |
| 2 | 6 | Cl | H | $CH_2$ | 2-Chloro-3,4,5-trimethoxyphenyl |
| 3 | | Cl | H | $CH_2$ | 2-Bromo-3,4,5-trimethoxyphenyl |
| 4 | | Cl | H | $CH_2$ | 2-Iodo-3,4,5-trimethoxyphenyl |
| 5 | | Cl | H | $CH_2$ | 2-Fluoro-3,4,5-trimethoxyphenyl |
| 6 | | Cl | H | $CH_2$ | 3,4,5-Trimethylphenyl |
| 7 | | Cl | H | $CH_2$ | 2-Chloro-3,4,5-trimethylphenyl |
| 8 | | Cl | H | $CH_2$ | 2-Bromo-3,4,5-trimethylphenyl |
| 9 | | Cl | H | $CH_2$ | 2-Iodo-3,4,5-trimethylphenyl |
| 10 | | Cl | H | $CH_2$ | 2-Fluoro-3,4,5-trimethylphenyl |
| 11 | | Cl | H | $CH_2$ | 3,5-Dimethoxy-4-methylphenyl |
| 12 | | Cl | H | $CH_2$ | 2-Chloro-3,5-dimethoxy-4-methylphenyl |
| 13 | | Cl | H | $CH_2$ | 2-Bromo-3,5-dimethoxy-4-methylphenyl |
| 14 | | Cl | H | $CH_2$ | 2-Iodo-3,5-dimethoxy-4-methylphenyl |
| 15 | | Cl | H | $CH_2$ | 2-Fluoro-3,5-dimethoxy-4-methylphenyl |
| 16 | | Cl | i-pr | $CH_2$ | 3,4,5-Trimethoxyphenyl |
| 17 | | Cl | i-pr | $CH_2$ | 2-Chloro-3,4,5-trimethoxyphenyl |
| 18 | | Cl | i-pr | $CH_2$ | 2-Bromo-3,4,5-trimethoxyphenyl |
| 19 | | Cl | i-pr | $CH_2$ | 2-Iodo-3,4,5-trimethoxyphenyl |
| 20 | | Cl | i-pr | $CH_2$ | 2-Fluoro-3,4,5-trimethoxyphenyl |
| 21 | | Cl | i-pr | $CH_2$ | 3,4,5-Trimethylphenyl |
| 22 | | Cl | i-pr | $CH_2$ | 2-Chloro-3,4,5-trimethylphenyl |
| 23 | | Cl | i-pr | $CH_2$ | 2-Bromo-3,4,5-trimethylphenyl |
| 24 | | Cl | i-pr | $CH_2$ | 2-Iodo-3,4,5-trimethylphenyl |
| 25 | | Cl | i-pr | $CH_2$ | 2-Fluoro-3,4,5-trimethylphenyl |
| 26 | | Cl | i-pr | $CH_2$ | 3,5-Dimethoxy-4-methylphenyl |
| 27 | | Cl | i-pr | $CH_2$ | 2-Chloro-3,5-dimethoxy-4-methylphenyl |
| 28 | | Cl | i-pr | $CH_2$ | 2-Bromo-3,5-dimethoxy-4-methylphenyl |
| 29 | | Cl | i-pr | $CH_2$ | 2-Iodo-3,5-dimethoxy-4-methylphenyl |
| 30 | | Cl | i-pr | $CH_2$ | 2-Fluoro-3,5-dimethoxy-4-methylphenyl |
| 31 | | Cl | Et | $CH_2$ | 3,4,5-Trimethoxyphenyl |
| 32 | | Cl | Et | $CH_2$ | 2-Chloro-3,4,5-trimethoxyphenyl |
| 33 | | Cl | Et | $CH_2$ | 2-Bromo-3,4,5-trimethoxyphenyl |
| 34 | | Cl | Et | $CH_2$ | 2-Iodo-3,4,5-trimethoxyphenyl |
| 35 | | Cl | Et | $CH_2$ | 2-Fluoro-3,4,5-trimethoxyphenyl |
| 36 | | Cl | Et | $CH_2$ | 3,4,5-Trimethylphenyl |
| 37 | | Cl | Et | $CH_2$ | 2-Chloro-3,4,5-trimethylphenyl |
| 38 | | Cl | Et | $CH_2$ | 2-Bromo-3,4,5-trimethylphenyl |

(continued)

| No. | Ex | $R^1$ | $R^3$ | $R^4$ | $R^5$ |
|---|---|---|---|---|---|
| 39 | | Cl | Et | $CH_2$ | 2-Iodo-3,4,5-trimethylphenyl |
| 40 | | Cl | Et | $CH_2$ | 2-Fluoro-3,4,5-trimethylphenyl |
| 41 | | Cl | Et | $CH_2$ | 3,5-Dimethoxy-4-methylphenyl |
| 42 | | Cl | Et | $CH_2$ | 2-Chloro-3,5-dimethoxy-4-methylphenyl |
| 43 | | Cl | Et | $CH_2$ | 2-Bromo-3,5-dimethoxy-4-methylphenyl |
| 44 | | Cl | Et | $CH_2$ | 2-Iodo-3,5-dimethoxy-4-methylphenyl |
| 45 | | Cl | Et | $CH_2$ | 2-Fluoro-3,5-dimethoxy-4-methylphenyl |
| 46 | 27 | Cl | Me | $CH_2$ | 3,4,5-Trimethoxyphenyl |
| 47 | | Cl | Me | $CH_2$ | 2-Chloro-3,4,5-trimethoxyphenyl |
| 48 | | Cl | Me | $CH_2$ | 2-Bromo-3,4,5-trimethoxyphenyl |
| 49 | | Cl | Me | $CH_2$ | 2-Iodo-3,4,5-trimethoxyphenyl |
| 50 | | Cl | Me | $CH_2$ | 2-Fluoro-3,4,5-trimethoxyphenyl |
| 51 | | Cl | Me | $CH_2$ | 3,4,5-Trimethylphenyl |
| 52 | | Cl | Me | $CH_2$ | 2-Chloro-3,4,5-trimethylphenyl |
| 53 | | Cl | Me | $CH_2$ | 2-Bromo-3,4,5-trimethylphenyl |
| 54 | | Cl | Me | $CH_2$ | 2-Iodo-3,4,5-trimethylphenyl |
| 55 | | Cl | Me | $CH_2$ | 2-Fluoro-3,4,5-trimethylphenyl |
| 56 | | Cl | Me | $CH_2$ | 3,5-Dimethoxy-4-methylphenyl |
| 57 | | Cl | Me | $CH_2$ | 2-Chloro-3,5-dimethoxy-4-methylphenyl |
| 58 | | Cl | Me | $CH_2$ | 2-Bromo-3,5-dimethoxy-4-methylphenyl |
| 59 | | Cl | Me | $CH_2$ | 2-Iodo-3,5-dimethoxy-4-methylphenyl |
| 60 | | Cl | Me | $CH_2$ | 2-Fluoro-3,5-dimethoxy-4-methylphenyl |
| 61 | | Cl | Ph | $CH_2$ | 3,4,5-Trimethoxyphenyl |
| 62 | | Cl | Ph | $CH_2$ | 2-Chloro-3,4,5-trimethoxyphenyl |
| 63 | | Cl | Ph | $CH_2$ | 2-Bromo-3,4,5-trimethoxyphenyl |
| 64 | | Cl | Ph | $CH_2$ | 2-Iodo-3,4,5-trimethoxyphenyl |
| 65 | | Cl | Ph | $CH_2$ | 2-Fluoro-3,4,5-trimethoxyphenyl |
| 66 | | Cl | Ph | $CH_2$ | 3,4,5-Trimethylphenyl |
| 67 | | Cl | Ph | $CH_2$ | 2-Chloro-3,4,5-trimethylphenyl |
| 68 | | Cl | Ph | $CH_2$ | 2-Bromo-3,4,5-trimethylphenyl |
| 69 | | Cl | Ph | $CH_2$ | 2-Iodo-3,4,5-trimethylphenyl |
| 70 | | Cl | Ph | $CH_2$ | 2-Fluoro-3,4,5-trimethylphenyl |
| 71 | | Cl | Ph | $CH_2$ | 3,5-Dimethoxy-4-methylphenyl |
| 72 | | Cl | Ph | $CH_2$ | 2-Chloro-3,5-dimethoxy-4-methylphenyl |
| 73 | | Cl | Ph | $CH_2$ | 2-Bromo-3,5-dimethoxy-4-methylphenyl |
| 74 | | Cl | Ph | $CH_2$ | 2-Iodo-3,5-dimethoxy-4-methylphenyl |
| 75 | | Cl | Ph | $CH_2$ | 2-Fluoro-3,5-dimethoxy-4-methylphenyl |
| 76 | | Cl | 2-Py | $CH_2$ | 3,4,5-Trimethoxyphenyl |

(continued)

| No. | Ex | $R^1$ | $R^3$ | $R^4$ | $R^5$ |
|---|---|---|---|---|---|
| 77 | | Cl | 2-Py | $CH_2$ | 2-Chloro-3,4,5-trimethoxyphenyl |
| 78 | | Cl | 2-Py | $CH_2$ | 2-Bromo-3,4,5-trimethoxyphenyl |
| 79 | | Cl | 2-Py | $CH_2$ | 2-Iodo-3,4,5-trimethoxyphenyl |
| 80 | | Cl | 2-Py | $CH_2$ | 2-Fluoro-3,4,5-trimethoxyphenyl |
| 81 | | Cl | 2-Py | $CH_2$ | 3,4,5-Trimethylphenyl |
| 82 | | Cl | 2-Py | $CH_2$ | 2-Chloro-3,4,5-trimethylphenyl |
| 83 | | Cl | 2-Py | $CH_2$ | 2-Bromo-3,4,5-trimethylphenyl |
| 84 | | Cl | 2-Py | $CH_2$ | 2-Iodo-3,4,5-trimethylphenyl |
| 85 | | Cl | 2-Py | $CH_2$ | 2-Fluoro-3,4,5-trimethylphenyl |
| 86 | | Cl | 2-Py | $CH_2$ | 3,5-Dimethoxy-4-methylphenyl |
| 87 | | Cl | 2-Py | $CH_2$ | 2-Chloro-3,5-dimethoxy-4-methylphenyl |
| 88 | | Cl | 2-Py | $CH_2$ | 2-Bromo-3,5-dimethoxy-4-methylphenyl |
| 89 | | Cl | 2-Py | $CH_2$ | 2-Iodo-3,5-dimethoxy-4-methylphenyl |
| 90 | | Cl | 2-Py | $CH_2$ | 2-Fluoro-3,5-dimethoxy-4-methylphenyl |
| 91 | | Cl | Me | $CH_2$ | 3,4,5-Trimethoxyphenyl |
| 92 | | Cl | Me | $CH_2$ | 2-Chloro-3,4,5-trimethoxyphenyl |
| 93 | | Cl | Me | $CH_2$ | 2-Bromo-3,4,5-trimethoxyphenyl |
| 94 | | Cl | Me | $CH_2$ | 2-Iodo-3,4,5-trimethoxyphenyl |
| 95 | | Cl | Me | $CH_2$ | 2-Fluoro-3,4,5-trimethoxyphenyl |
| 96 | | Cl | Ph | $CH_2$ | 3,4,5-Trimethylphenyl |
| 97 | | Cl | Ph | $CH_2$ | 2-Chloro-3,4,5-trimethylphenyl |
| 98 | | Cl | Ph | $CH_2$ | 2-Bromo-3,4,5-trimethylphenyl |
| 99 | | Cl | Ph | $CH_2$ | 2-Iodo-3,4,5-trimethylphenyl |
| 100 | | Cl | Ph | $CH_2$ | 2-Fluoro-3,4,5-trimethylphenyl |
| 101 | | Cl | Ph | $CH_2$ | 3,5-Dimethoxy-4-methylphenyl |
| 102 | | Cl | Ph | $CH_2$ | 2-Chloro-3,5-dimethoxy-4-methylphenyl |
| 103 | | Cl | Ph | $CH_2$ | 2-Bromo-3,5-dimethoxy-4-methylphenyl |
| 104 | | Cl | Ph | $CH_2$ | 2-Iodo-3,5-dimethoxy-4-methylphenyl |
| 105 | | Cl | Pr | $CH_2$ | 2-Fluoro-3,5-dimethoxy-4-methylphenyl |
| 106 | | Cl | Pr | $CH_2$ | 3,5-Dimethoxy-4-methylphenyl |
| 107 | | Cl | Pr | $CH_2$ | 2-Chloro-3,5-dimethoxy-4-methylphenyl |
| 108 | | Cl | Pr | $CH_2$ | 2-Bromo-3,5-dimethoxy-4-methylphenyl |
| 109 | | Cl | Pr | $CH_2$ | 2-Iodo-3,5-dimethoxy-4-methylphenyl |
| 110 | | Cl | Pr | $CH_2$ | 2-Fluoro-3,5-dimethoxy-4-methylphenyl |
| 111 | | Cl | Pr | $CH_2$ | 3,4,5-Trimethoxyphenyl |
| 112 | | Cl | Pr | $CH_2$ | 2-Chloro-3,4,5-trimethoxyphenyl |
| 113 | | Cl | Pr | $CH_2$ | 2-Bromo-3,4,5-trimethoxyphenyl |
| 114 | | Cl | Pr | $CH_2$ | 2-Iodo-3,4,5-trimethoxyphenyl |

(continued)

| No. | Ex | $R^1$ | $R^3$ | $R^4$ | $R^5$ |
|---|---|---|---|---|---|
| 115 | | Cl | Pr | $CH_2$ | 2-Fluoro-3,4,5-trimethoxyphenyl |
| 116 | | Cl | Pr | $CH_2$ | 3,4,5-Trimethylphenyl |
| 117 | | Cl | Pr | $CH_2$ | 2-Chloro-3,4,5-trimethylphenyl |
| 118 | | Cl | Pr | $CH_2$ | 2-Bromo-3,4,5-trimethylphenyl |
| 119 | | Cl | Pr | $CH_2$ | 2-Iodo-3,4,5-trimethylphenyl |
| 120 | | Cl | Pr | $CH_2$ | 2-Fluoro-3,4,5-trimethylphenyl |
| 121 | | Cl | Pr | $CH_2$ | 3,5-Dimethoxy-4-methylphenyl |
| 122 | | Cl | Pr | $CH_2$ | 2-Chloro-3,5-dimethoxy-4-methylphenyl |
| 123 | | Cl | Pr | $CH_2$ | 2-Bromo-3,5-dimethoxy-4-methylphenyl |
| 124 | | Cl | Pr | $CH_2$ | 2-Iodo-3,5-dimethoxy-4-methylphenyl |
| 125 | | Cl | Pr | $CH_2$ | 2-Fluoro-3,5-dimethoxy-4-methylphenyl |
| 126 | | Br | H | $CH_2$ | 3,4,5-Trimethoxyphenyl |
| 127 | | Br | H | $CH_2$ | 2-Chloro-3,4,5-trimethoxyphenyl |
| 128 | | Br | H | $CH_2$ | 2-Bromo-3,4,5-trimethoxyphenyl |
| 129 | | Br | H | $CH_2$ | 2-Iodo-3,4,5-trimethoxyphenyl |
| 130 | | Br | H | $CH_2$ | 2-Fluoro-3,4,5-trimethoxyphenyl |
| 131 | | Br | H | $CH_2$ | 3,4,5-Trimethylphenyl |
| 132 | | Br | H | $CH_2$ | 2-Chloro-3,4,5-trimethylphenyl |
| 133 | | Br | H | $CH_2$ | 2-Bromo-3,4,5-trimethylphenyl |
| 134 | | Br | H | $CH_2$ | 2-Iodo-3,4,5-trimethylphenyl |
| 135 | | Br | H | $CH_2$ | 2-Fluoro-3,4,5-trimethylphenyl |
| 136 | | Br | H | $CH_2$ | 3,5-Dimethoxy-4-methylphenyl |
| 137 | | Br | H | $CH_2$ | 2-Chloro-3,5-dimethoxy-4-methylphenyl |
| 138 | | Br | H | $CH_2$ | 2-Bromo-3,5-dimethoxy-4-methylphenyl |
| 139 | | Br | H | $CH_2$ | 2-Iodo-3,5-dimethoxy-4-methylphenyl |
| 140 | | Br | H | $CH_2$ | 2-Fluoro-3,5-dimethoxy-4-methylphenyl |
| 141 | | Cl | i-Bu | $CH_2$ | 3,4,5-Trimethoxyphenyl |
| 142 | | Cl | i-Bu | $CH_2$ | 2-Chloro-3,4,5-trimethoxyphenyl |
| 143 | | Cl | i-Bu | $CH_2$ | 2-Bromo-3,4,5-trimethoxyphenyl |
| 144 | | Cl | i-Bu | $CH_2$ | 2-Iodo-3,4,5-trimethoxyphenyl |
| 145 | | Cl | i-Bu | $CH_2$ | 2-Fluoro-3,4,5-trimethoxyphenyl |
| 146 | | Cl | i-Bu | $CH_2$ | 3,4,5-Trimethylphenyl |
| 147 | | Cl | i-Bu | $CH_2$ | 2-Chloro-3,4,5-trimethylphenyl |
| 148 | | Cl | i-Bu | $CH_2$ | 2-Bromo-3,4,5-trimethylphenyl |
| 149 | | Cl | i-Bu | $CH_2$ | 2-Iodo-3,4,5-trimethylphenyl |
| 150 | | Cl | i-Bu | $CH_2$ | 2-Fluoro-3,4,5-trimethylphenyl |
| 151 | | Cl | i-Bu | $CH_2$ | 3,5-Dimethoxy-4-methylphenyl |
| 152 | | Cl | i-Bu | $CH_2$ | 2-Chloro-3,5-dimethoxy-4-methylphenyl |

(continued)

| No. | Ex | $R^1$ | $R^3$ | $R^4$ | $R^5$ |
|---|---|---|---|---|---|
| 153 | | Cl | i-Bu | $CH_2$ | 2-Bromo-3,5-dimethoxy-4-methylphenyl |
| 154 | | Cl | i-Bu | $CH_2$ | 2-Iodo-3,5-dimethoxy-4-methylphenyl |
| 155 | | Cl | i-Bu | $CH_2$ | 2-Fluoro-3,5-dimethoxy-4-methylphenyl |
| 156 | | Cl | CN | $CH_2$ | 3,4,5-Trimethoxyphenyl |
| 157 | | Cl | CN | $CH_2$ | 2-Chloro-3,4,5-trimethoxyphenyl |
| 158 | | Cl | CN | $CH_2$ | 2-Bromo-3,4,5-trimethoxyphenyl |
| 159 | | Cl | CN | $CH_2$ | 2-Iodo-3,4,5-trimethoxyphenyl |
| 160 | | Cl | CN | $CH_2$ | 3,4,5-Trimethoxyphenyl |
| 161 | | Cl | CN | $CH_2$ | 2-Chloro-3,4,5-trimethoxyphenyl |
| 162 | | Cl | CN | $CH_2$ | 2-Bromo-3,4,5-trimethoxyphenyl |
| 163 | | Cl | CN | $CH_2$ | 2-Iodo-3,4,5-trimethoxyphenyl |
| 164 | | Cl | CN | $CH_2$ | 2-Fluoro-3,4,5-trimethoxyphenyl |
| 165 | | Cl | CN | $CH_2$ | 3,4,5-Trimethylphenyl |
| 166 | | Cl | CN | $CH_2$ | 2-Chloro-3,4,5-trimethylphenyl |
| 167 | | Cl | CN | $CH_2$ | 2-Bromo-3,4,5-trimethylphenyl |
| 168 | | Cl | CN | $CH_2$ | 2-Iodo-3,4,5-trimethylphenyl |
| 169 | | Cl | CN | $CH_2$ | 2-Fluoro-3,4,5-trimethylphenyl |
| 170 | | Cl | CN | $CH_2$ | 3,5-Dimethoxy-4-methylphenyl |
| 171 | | Cl | CN | $CH_2$ | 2-Chloro-3,5-dimethoxy-4-methylphenyl |
| 172 | | Cl | CN | $CH_2$ | 2-Bromo-3,5-dimethoxy-4-methylphenyl |
| 173 | | Cl | CN | $CH_2$ | 2-Iodo-3,5-dimethoxy-4-methylphenyl |
| 174 | | Cl | CN | $CH_2$ | 2-Fluoro-3,5-dimethoxy-4-methylphenyl |
| 175 | | Cl | Cl | $CH_2$ | 3,4,5-Trimethoxyphenyl |
| 176 | | Cl | Cl | $CH_2$ | 2-Chloro-3,4,5-trimethoxyphenyl |
| 177 | | Cl | Cl | $CH_2$ | 2-Bromo-3,4,5-trimethoxyphenyl |
| 178 | | Cl | Cl | $CH_2$ | 2-Iodo-3,4,5-trimethoxyphenyl |
| 179 | | Cl | Cl | $CH_2$ | 2-Fluoro-3,4,5-trimethoxyphenyl |
| 180 | | Cl | Cl | $CH_2$ | 3,4,5-Trimethylphenyl |
| 181 | | Cl | Cl | $CH_2$ | 2-Chloro-3,4,5-trimethylphenyl |
| 182 | | Cl | Cl | $CH_2$ | 2-Bromo-3,4,5-trimethylphenyl |
| 183 | | Cl | Cl | $CH_2$ | 2-Iodo-3,4,5-trimethylphenyl |
| 184 | | Cl | Cl | $CH_2$ | 2-Fluoro-3,4,5-trimethylphenyl |
| 185 | | Cl | Cl | $CH_2$ | 3,5-Dimethoxy-4-methylphenyl |
| 186 | | Cl | Cl | $CH_2$ | 2-Chloro-3,5-dimethoxy-4-methylphenyl |
| 187 | | Cl | Cl | $CH_2$ | 2-Bromo-3,5-dimethoxy-4-methylphenyl |
| 188 | | Cl | Cl | $CH_2$ | 2-Iodo-3,5-dimethoxy-4-methylphenyl |
| 189 | | Cl | Cl | $CH_2$ | 2-Fluoro-3,5-dimethoxy-4-methylphenyl |
| 190 | | Cl | Br | $CH_2$ | 3,4,5-Trimethoxyphenyl |

(continued)

| No. | Ex | $R^1$ | $R^3$ | $R^4$ | $R^5$ |
|---|---|---|---|---|---|
| 191 | | Cl | Br | $CH_2$ | 2-Chloro-3,4,5-trimethoxyphenyl |
| 192 | | Cl | Br | $CH_2$ | 2-Bromo-3,4,5-trimethoxyphenyl |
| 193 | | Cl | Br | $CH_2$ | 2-Iodo-3,4,5-trimethoxyphenyl |
| 194 | | Cl | Br | $CH_2$ | 2-Fluoro-3,4,5-trimethoxyphenyl |
| 195 | | Cl | Br | $CH_2$ | 3,4,5-Trimethylphenyl |
| 196 | | Cl | Br | $CH_2$ | 2-Chloro-3,4,5-trimethylphenyl |
| 197 | | Cl | Br | $CH_2$ | 2-Bromo-3,4,5-trimethylphenyl |
| 198 | | Cl | Br | $CH_2$ | 2-Iodo-3,4,5-trimethylphenyl |
| 199 | | Cl | Br | $CH_2$ | 2-Fluoro-3,4,5-trimethylphenyl |
| 200 | | Cl | Br | $CH_2$ | 3,5-Dimethoxy-4-methylphenyl |
| 201 | | Cl | Br | $CH_2$ | 2-Chloro-3,5-dimethoxy-4-methylphenyl |
| 202 | | Cl | Br | $CH_2$ | 2-Bromo-3,5-dimethoxy-4-methylphenyl |
| 203 | | Cl | Br | $CH_2$ | 2-Iodo-3,5-dimethoxy-4-methylphenyl |
| 204 | | Cl | Br | $CH_2$ | 2-Fluoro-3,5-dimethoxy-4-methylphenyl |
| 205 | | Cl | I | $CH_2$ | 3,4,5-Trimethoxyphenyl |
| 206 | | Cl | I | $CH_2$ | 2-Chloro-3,4,5-trimethoxyphenyl |
| 207 | | Cl | I | $CH_2$ | 2-Bromo-3,4,5-trimethoxyphenyl |
| 208 | | Cl | I | $CH_2$ | 2-Iodo-3,4,5-trimethoxyphenyl |
| 209 | | Cl | I | $CH_2$ | 2-Fluoro-3,4,5-trimethoxyphenyl |
| 210 | | Cl | I | $CH_2$ | 3,4,5-Trimethylphenyl |
| 211 | | Cl | I | $CH_2$ | 2-Chloro-3,4,5-trimethylphenyl |
| 212 | | Cl | I | $CH_2$ | 2-Bromo-3,4,5-trimethylphenyl |
| 213 | | Cl | I | $CH_2$ | 2-Iodo-3,4,5-trimethylphenyl |
| 214 | | Cl | I | $CH_2$ | 2-Fluoro-3,4,5-trimethylphenyl |
| 215 | | Cl | I | $CH_2$ | 3,5-Dimethoxy-4-methylphenyl |
| 216 | | Cl | I | $CH_2$ | 2-Chloro-3,5-dimethoxy-4-methylphenyl |
| 217 | | Cl | I | $CH_2$ | 2-Bromo-3,5-dimethoxy-4-methylphenyl |
| 218 | | Cl | I | $CH_2$ | 2-Iodo-3,5-dimethoxy-4-methylphenyl |
| 219 | | Cl | I | $CH_2$ | 2-Fluoro-3,5-dimethoxy-4-methylphenyl |
| 220 | | Cl | $CH_2$-$NMe_2$ | $CH_2$ | 3,4,5-Trimethoxyphenyl |
| 221 | | Cl | $CH_2$-$NMe_2$ | $CH_2$ | 2-Chloro-3,4,5-trimethoxyphenyl |
| 222 | | Cl | $CH_2$-$NMe_2$ | $H_2$ | 2-Bromo-3,4,5-trimethoxyphenyl |
| 223 | | Cl | $CH_2$-$NMe_2$ | $CH_2$ | 2-Iodo-3,4,5-trimethoxyphenyl |
| 224 | | Cl | $CH_2NMe_2$ | $CH_2$ | 2-Fluoro-3,4,5-trimethoxyphenyl |
| 225 | | Cl | $CH_2$-$NMe_2$ | $CH_2$ | 3,4,5-Trimethylphenyl |
| 226 | | Cl | $CH_2$-$NMe_2$ | $CH_2$ | 2-Chloro-3,4,5-trimethylphenyl |
| 227 | | Cl | $CH_2$-$NMe_2$ | $CH_2$ | 2-Bromo-3,4,5-trimethylphenyl |
| 228 | | Cl | $CH_2$-$NMe_2$ | $CH_2$ | 2-Iodo-3,4,5-trimethylphenyl |

(continued)

| No. | Ex | $R^1$ | $R^3$ | $R^4$ | $R^5$ |
|---|---|---|---|---|---|
| 229 | | Cl | $CH_2$-$NMe_2$ | $CH_2$ | 2-Fluoro-3,4,5-trimethylphenyl |
| 230 | | Cl | $CH_2$-$NMe_2$ | $CH_2$ | 3,5-Dimethoxy-4-methylphenyl |
| 231 | | Cl | $CH_2$-$NMe_2$ | $CH_2$ | 2-Chloro-3,5-dimethoxy-4-methylphenyl |
| 232 | | Cl | $CH_2$-$NMe_2$ | $CH_2$ | 2-Bromo-3,5-dimethoxy-4-methylphenyl |
| 233 | | Cl | $CH_2$-$NMe_2$ | $CH_2$ | 2-Iodo-3,5-dimethoxy-4-methylphenyl |
| 234 | | Cl | $CH_2$-$NMe_2$ | $CH_2$ | 2-Fluoro-3,5-dimethoxy-4-methylphenyl |
| 235 | | Cl | 3-Py | $CH_2$ | 3,4,5-Trimethoxyphenyl |
| 236 | | Cl | 3-Py | $CH_2$ | 2-Chloro-3,4,5-trimethoxyphenyl |
| 237 | | Cl | 3-Py | $CH_2$ | 2-Bromo-3,4,5-trimethoxyphenyl |
| 238 | | Cl | 3-Py | $CH_2$ | 2-Iodo-3,4,5-trimethoxyphenyl |
| 239 | | Cl | 3-Py | $CH_2$ | 2-Fluoro-3,4,5-trimethoxyphenyl |
| 240 | 7 | Cl | H | $CH_2$ | 3,5-Dimethyl-4-methoxypyridin-2-yl |
| 241 | 10 | Cl | H | $CH_2$ | 3,5-Dimethyl-4-methoxy-l-oxypyridin-2-yl |
| 242 | | Cl | H | $CH_2$ | 6-Bromo-3,5-dimethyl-4-methoxypyridin-2-yl |
| 243 | 8 | Cl | H | $CH_2$ | 6-Chloro-3,5-dimethyl-4-methoxypyridin-2-yl |
| 244 | 14 | Cl | H | $CH_2$ | 3,5-Dimethyl-4-bromopyridin-2-yl |
| 245 | 15 | Cl | H | $CH_2$ | 3,5-Dimethyl-4-bromo-1-oxypyridin-2-yl |
| 246 | 9 | Cl | H | $CH_2$ | 3,5-Dimethyl-4-chloropyridin-2-yl |
| 247 | | Cl | H | $CH_2$ | 3,5-Dimethyl-4-chloro-1-oxypyridin-2-yl |
| 248 | | Cl | H | $CH_2$ | 3,5-Dimethyl-4-iodopyridin-2-yl |
| 249 | | Cl | H | $CH_2$ | 3,5-Dimethyl-4-iodo-1-oxypyridin-2-yl |
| 250 | | Cl | H | $CH_2$ | 3,5-Dimethyl-4-thiomethyl-pyridin-2-yl |
| 251 | | Cl | H | $CH_2$ | 3,5-Dimethyl-4-thiomethyl-1-oxypyridin-2-yl |
| 252 | | Cl | H | $CH_2$ | 3,4,5-Trimethyl-pyridin-2-yl |
| 253 | | Cl | H | $CH_2$ | 3,4,5-Trimethyl-1-oxypyridin-2-yl |
| 254 | | Cl | H | $CH_2$ | 4,5,6-Trimethoxypyridin-2-yl |
| 255 | | Cl | H | $CH_2$ | 4,5,6-Trimethoxy-1-oxypyridin-2-yl |
| 256 | | Cl | H | $CH_2$ | Bromo-4,5,6-trimethoxypyridin-2-yl |
| 257 | | Cl | H | $CH_2$ | 3-Chloro-4,5,6-trimethoxypyridin-2-yl |
| 258 | | Cl | H | $CH_2$ | 3,4,5-Trimethoxy-pyridin-2-yl |
| 259 | | Cl | H | $CH_2$ | 3,4,5-Trimethoxy-1-oxypyridin-2-yl |
| 260 | | Cl | H | $CH_2$ | 3-Bromo-3,4,5-trimethoxy-pyridin-2-yl |
| 261 | | Cl | H | $CH_2$ | 3-Chloro-3,4,5-trimethoxy-pyridin-2-yl |
| 262 | | Cl | H | $CH_2$ | 4,5,6-Trimethyl-pyridin-2-yl |
| 263 | | Cl | H | $CH_2$ | 4,5,6-Trimethyl-1-oxypyridin-2-yl |
| 264 | | Cl | H | $CH_2$ | 4,6-Dimethyl-5-methoxy-pyridin-2-yl |
| 265 | | Cl | H | $CH_2$ | 4,6-Dimethyl-5-methoxypyridin-3-yl |
| 266 | | Cl | H | $CH_2$ | 4,6-Dimethyl-5-methoxy-1-oxypyridin-3-yl |

(continued)

| No. | Ex | $R^1$ | $R^3$ | $R^4$ | $R^5$ |
|---|---|---|---|---|---|
| 267 | | Cl | H | $CH_2$ | 4,6-Dimethyl-5-bromopyridin-3-yl |
| 268 | | Cl | H | $CH_2$ | 4,6-Dimethyl-5-chloropyridin-3-yl |
| 269 | | Cl | H | $CH_2$ | 5,6-Dimethyl-4-bromopyridin-3-yl |
| 270 | | Cl | H | $CH_2$ | 5,6-Dimethyl-4-chloropyridin-3-yl |
| 271 | | Cl | H | $CH_2$ | 2,6-Dimethyl-3-methoxypyridin-4-yl |
| 272 | | Cl | H | $CH_2$ | 2,6-Dimethyl-pyridin-4-yl |
| 273 | | Cl | H | $CH_2$ | 2,3,6-Trimethyl-pyridin-4-yl |
| 274 | | Cl | H | $CH_2$ | 2,3,6-Trimethoxy-pyridin-4-yl |
| 275 | | Cl | H | $CH_2$ | 2,6-Dimethyl-3-bromopyridin-4-yl |
| 276 | | Cl | H | $CH_2$ | 2,6-Dimethyl-3-chloropyridin-4-yl |
| 277 | | Cl | H | $CH_2$ | 2,6-Dimethyl-3-methoxy-1-oxy-pyridin-4-yl |
| 278 | | Cl | H | $CH_2$ | 2,6-Dimethyl-1-oxy-pyridin4-yl |
| 279 | | Cl | H | $CH_2$ | 2,3,6-Trimethyl-1-oxy-pyridin-4-yl |
| 280 | | Cl | H | $CH_2$ | 2,3,6-Trimethoxy-1-oxy-pyridin-4-yl |
| 281 | | Cl | H | $CH_2$ | 2,6-Dimethyl-3-bromol-oxy-pyridin-4-yl |
| 282 | | Cl | H | $CH_2$ | 2,6-Dimethyl-3-chlorol-oxy-pyridin-4-yl |
| 283 | | Cl | H | $CH_2$ | 4,6-Dimethyl-5-iodopyridin-3-yl |
| 284 | | Cl | H | $CH_2$ | 3,5-Dimethyl-4-aminopyridin-2-yl |
| 285 | 31 | Cl | i-pr | $CH_2$ | 3,5-Dimethyl-4-methoxypyridin-2-yl |
| 286 | | Cl | i-pr | $CH_2$ | 3,5-Dimethyl-4-methoxy-1-oxypyridin-2-yl |
| 287 | | Cl | i-pr | $CH_2$ | 6-Bromo-3,5-dimethyl-4-methoxypyridin-2-yl |
| 288 | | Cl | i-pr | $CH_2$ | 6-Chloro-3,5-dimethyl-4-methoxypyridin-2-yl |
| 289 | | Cl | i-pr | $CH_2$ | 3,5-Dimethyl-4-bromopyridin-2-yl |
| 290 | | Cl | i-pr | $CH_2$ | 3,5-Dimethyl-4-bromo-1-oxypyridin-2-yl |
| 291 | | Cl | i-pr | $CH_2$ | 3,5-Dimethyl-4-chloropyridin-2-yl |
| 292 | | Cl | i-pr | $CH_2$ | 3,5-Dimethyl-4-chloro-1-oxypyridin-2-yl |
| 293 | | Cl | i-pr | $CH_2$ | 3,5-Dimethyl-4-iodopyridin-2-yl |
| 294 | | Cl | i-pr | $CH_2$ | 3,5-Dimethyl-4-iodo-1-oxypyridin-2-yl |
| 295 | | Cl | i-pr | $CH_2$ | 3,5-Dimethyl-4-thiomethyl-pyridin-2-yl |
| 296 | | Cl | i-pr | $CH_2$ | 3,5-Dimethyl-4-thiomethyl-1-oxypyridin-2-yl |
| 297 | | Cl | i-pr | $CH_2$ | 3,4,5-Trimethyl-pyridin-2-yl |
| 298 | | Cl | i-pr | $CH_2$ | 3,4,5-Trimethyl-1-oxypyridin-2-yl |
| 299 | | Cl | i-pr | $CH_2$ | 4,5,6-Trimethoxypyridin-2-yl |
| 300 | | Cl | i-pr | $CH_2$ | 4,5,6-Trimethoxy-1-oxypyridin-2-yl |
| 301 | | Cl | i-pr | $CH_2$ | 3-Bromo-4,5,6-trimethoxypyridin-2-yl |
| 302 | | Cl | i-pr | $CH_2$ | 3-Chloro-4,5,6-trimethoxypyridin-2-yl |
| 303 | | Cl | i-pr | $CH_2$ | 3,4,5-Trimethoxy-pyridin-2-yl |
| 304 | | Cl | i-pr | $CH_2$ | 3,4,5-Trimethoxy-1-oxypyridin-2-yl |

(continued)

| No. | Ex | $R^1$ | $R^3$ | $R^4$ | $R^5$ |
|---|---|---|---|---|---|
| 305 | | Cl | i-pr | $CH_2$ | 3-Bromo-3,4,5-trimethoxy-pyridin-2-yl |
| 306 | | Cl | i-pr | $CH_2$ | 3-Chloro-3,4,5-trimethoxy-pyridin-2-yl |
| 307 | | Cl | i-pr | $CH_2$ | 4,5,6-Trimethyl-pyridin-2-yl |
| 308 | | Cl | i-pr | $CH_2$ | 4,5,6-Trimethyl-1-oxypyridin-2-yl |
| 309 | | Cl | i-pr | $CH_2$ | 4,6-Dimethyl-5-methoxy-pyridin-2-yl |
| 310 | | Cl | i-pr | $CH_2$ | 4,6-Dimethyl-5-methoxypyridin-3-yl |
| 311 | | Cl | i-pr | $CH_2$ | 4,6-Dimethyl-5-methoxy-1-oxypyridin-3-yl |
| 312 | | Cl | i-pr | $CH_2$ | 4,6-Dimethyl-5-bromopyridin-3-yl |
| 313 | | Cl | i-pr | $CH_2$ | 4,6-Dimethyl-5-chloropyridin-3-yl |
| 314 | | Cl | i-pr | $CH_2$ | 5,6-Dimethyl-4-bromopyridin-3-yl |
| 315 | | Cl | i-pr | $CH_2$ | 5,6-Dimethyl-4-chloropyridin-3-yl |
| 316 | | Cl | i-pr | $CH_2$ | 2,6-Dimethyl-3-methoxypyridin-4-yl |
| 317 | | Cl | i-pr | $CH_2$ | 2,6-Dimethyl-pyridin-4-yl |
| 318 | | Cl | i-pr | $CH_2$ | 2,3,6-Trimethyl-pyridin-4-yl |
| 319 | | Cl | i-pr | $CH_2$ | 2,3,6-Trimethoxy-pyridin-4-yl |
| 320 | | Cl | i-pr | $CH_2$ | 2,6-Dimethyl-3-bromopyridin-4-yl |
| 321 | | Cl | i-pr | $CH_2$ | 2,6-Dimethyl-3-chloropyridin-4-yl |
| 322 | | Cl | i-pr | $CH_2$ | 2,6-Dimethyl-3-methoxy-1-oxy-pyridin-4-yl |
| 323 | | Cl | i-pr | $CH_2$ | 2,6-Dimethyl-1-oxy-pyridin-4-yl |
| 324 | | Cl | i-pr | $CH_2$ | 2,3,6-Trimethyl-1-oxy-pyridin-4-yl |
| 325 | | Cl | i-pr | $CH_2$ | 2,3,6-Trimethoxy-1-oxy-pyridin-4-yl |
| 326 | | Cl | i-pr | $CH_2$ | 2,6-Dimethyl-3-bromol-oxy-pyridin-4-yl |
| 327 | | Cl | i-pr | $CH_2$ | 2,6-Dimethyl-3-chlorol-oxy-pyridin-4-yl |
| 328 | | Cl | i-pr | $CH_2$ | 4,6-Dimethyl-5-iodopyridin-3-yl |
| 329 | | Cl | i-pr | $CH_2$ | 3,5-Dimethyl-4-aminopyridin-2-yl |
| 330 | 23 | Cl | Me | $CH_2$ | 3,5-Dimethyl-4-methoxypyridin-2-yl |
| 331 | | Cl | Me | $CH_2$ | 3,5-Dmicthyl-4-methoxy-l-oxypyridm-2-yl |
| 332 | 28 | Cl | Me | $CH_2$ | 3,5-Dimethyl-4-bromopyridin-2-yl |
| 333 | 24 | Cl | Me | $CH_2$ | 3,5-Dimethyl-4-bromo-1-oxypyridin-2-yl |
| 334 | 26 | Cl | Me | $CH_2$ | 3,5-Dimethyl-4-chloropyridin-2-yl |
| 335 | 25 | Cl | Me | $CH_2$ | 3,5-Dimethyl-4-chloro-1-oxypyridin-2-yl |
| 336 | | Cl | Me | $CH_2$ | 3,5-Dimethyl-4-iodopyridin-2-yl |
| 337 | | Cl | Me | $CH_2$ | 3,5-Dimethyl-4-iodo-1-oxypyridin-2-yl |
| 338 | | Cl | Me | $CH_2$ | 3,5-Dimethyl-4-thiomethyl-pyridin-2-yl |
| 339 | | Cl | Me | $CH_2$ | 3,5-Dimethyl-4-thiomethyl-1-oxypyridin-2-yl |
| 340 | | Cl | Me | $CH_2$ | 3,4,5-Trimethyl-pyridin-2-yl |
| 341 | | Cl | Me | $CH_2$ | 3,4,5-Trimethyl-1-oxypyridin-2-yl |
| 342 | | Cl | Me | $CH_2$ | 4,5,6-Trimethoxypyridin-2-yl |

(continued)

| No. | Ex | $R^1$ | $R^3$ | $R^4$ | $R^5$ |
|---|---|---|---|---|---|
| 343 | | Cl | Me | $CH_2$ | 4,5,6-Trimethoxy-1-oxypyridin-2-yl |
| 344 | | Cl | Me | $CH_2$ | 3,4,5-Trimethoxy-pyridin-2-yl |
| 345 | | Cl | Me | $CH_2$ | 3,4,5-Trimethoxy-1-oxypyridin-2-yl |
| 346 | | Cl | Me | $CH_2$ | 4,5,6-Trimethyl-pyridin-2-yl |
| 347 | | Cl | Me | $CH_2$ | 4,5,6-Trimethyl-1-oxypyridin-2-yl |
| 348 | | Cl | Me | $CH_2$ | 4,6-Dimethyl-5-methoxy-pyridin-2-yl |
| 349 | | Cl | Me | $CH_2$ | 4,6-Dimethyl-5-methoxypyridin-3-yl |
| 350 | | Cl | Me | $CH_2$ | 4,6-Dimethyl-5-methoxy-1-oxypyridin-3-yl |
| 351 | | Cl | Me | $CH_2$ | 4,6-Dimethyl-5-bromopyridin-3-yl |
| 352 | | Cl | Me | $CH_2$ | 4,6-Dimethyl-5-chloropyridin-3-yl |
| 353 | | Cl | Me | $CH_2$ | 5,6-Dimethyl-4-bromopyridin-3-yl |
| 354 | | Cl | Me | $CH_2$ | 5,6-Dimethyl-4-chloropyridin-3-yl |
| 355 | | Cl | Me | $CH_2$ | 2,6-Dimethyl-3-methoxypyridin-4-yl |
| 356 | | Cl | Me | $CH_2$ | 2,6-Dimethyl-pyridin-4-yl |
| 357 | | Cl | Me | $CH_2$ | 2,3,6-Trimethyl-pyridin-4-yl |
| 358 | | Cl | Me | $CH_2$ | 2,3,6-Trimethoxy-pyridin-4-yl |
| 359 | | Cl | Me | $CH_2$ | 2,6-Dimethyl-3-bromopyridin-4-yl |
| 360 | | Cl | Me | $CH_2$ | 2,6-Dimethyl-3-chloropyridin-4-yl |
| 361 | | Cl | Me | $CH_2$ | 4,6-Dimethyl-5-iodopyridin-3-yl |
| 362 | | Cl | Me | $CH_2$ | 3,5-Dimethyl-4-aminopyridin-2-yl |
| 363 | 29 | Cl | Et | $CH_2$ | 3,5-Dimethyl-4-methoxypyridin-2-yl |
| 364 | | Cl | Et | $CH_2$ | 3,5-Dimethyl-4-methoxy-1-oxypyridin-2-yl |
| 365 | | Cl | Et | $CH_2$ | 3,5-Dimethyl-4-bromopyridin-2-yl |
| 366 | | Cl | Et | $CH_2$ | 3,5-Dimethyl-4-bromo-1-oxypyridin-2-yl |
| 367 | 30 | Cl | Et | $CH_2$ | 3,5-Dimethyl-4-chloropyridin-2-yl |
| 368 | | Cl | Et | $CH_2$ | 3,5-Dimethyl-4-chloro-1-oxypyridin-2-yl |
| 369 | | Cl | Et | $CH_2$ | 3,5-Dimethyl-4-iodopyridin-2-yl |
| 370 | | Cl | Et | $CH_2$ | 3,5-Dimethyl-4-iodo-1-oxypyridin-2-yl |
| 371 | | Cl | Et | $CH_2$ | 3,5-Dimethyl-4-thiomethyl-pyridin-2-yl |
| 372 | | Cl | Et | $CH_2$ | 3,5-Dimethyl-4-thiomethyl-1-oxypyridin-2-yl |
| 373 | | Cl | Et | $CH_2$ | 3,4,5-Trimethyl-pyridin-2-yl |
| 374 | | Cl | Et | $CH_2$ | 3,4,5-Trimethyl-1-oxypyridin-2-yl |
| 375 | | Cl | Et | $CH_2$ | 4,5,6-Trimethoxypyridin-2-yl |
| 376 | | Cl | Et | $CH_2$ | 4,5,6-Trimethoxy-1-oxypyridin-2-yl |
| 377 | | Cl | Et | $CH_2$ | 3,4,5-Trimethoxy-pyridin-2-yl |
| 378 | | Cl | Et | $CH_2$ | 3,4,5-Trimethoxy-1-oxypyridin-2-yl |
| 379 | | Cl | Et | $CH_2$ | 4,5,6-Trimethyl-pyridin-2-yl |
| 380 | | Cl | Et | $CH_2$ | 4,5,6-Trimethyl-1-oxypyridin-2-yl |

(continued)

| No. | Ex | $R^1$ | $R^3$ | $R^4$ | $R^5$ |
|---|---|---|---|---|---|
| 381 | | Cl | Et | $CH_2$ | 4,6-Dimethyl-5-methoxy-pyridin-2-yl |
| 382 | | Cl | Et | $CH_2$ | 4,6-Dimethyl-5-methoxypyridin-3-yl |
| 383 | | Cl | Et | $CH_2$ | 4,6-Dimeihyl-5-methoxy-1-oxypyridin-3-yl |
| 384 | | Cl | Et | $CH_2$ | 4,6-Dimethyl-5-bromopyridin-3-yl |
| 385 | | Cl | Et | $CH_2$ | 4,6-Dimethyl-5-chloropyridin-3-yl |
| 386 | | Cl | Et | $CH_2$ | 5,6-Dimethyl-4-bromopyridin-3-yl |
| 387 | | Cl | Et | $CH_2$ | 5,6-Dimethyl-4-chloropyridin-3-yl |
| 388 | | Cl | Et | $CH_2$ | 2,6-Dimethyl-3-methoxypyridin-4-yl |
| 389 | | Cl | Et | $CH_2$ | 2,6-Dimethyl-pyridin-4-yl |
| 390 | | Cl | Et | $CH_2$ | 2,3,6-Trimethyl-pyridin-4-yl |
| 391 | | Cl | Et | $CH_2$ | 2,3,6-Trimethoxy-pyridin-4-yl |
| 392 | | Cl | Et | $CH_2$ | 2,6-Dimethyl-3-bromopyridin-4-yl |
| 393 | | Cl | Et | $CH_2$ | 2,6-Dimethyl-3-chloropyridin-4-yl |
| 394 | | Cl | Et | $CH_2$ | 4,6-Dimethyl-5-iodopyridin-3-yl |
| 395 | | Cl | Et | $CH_2$ | 3,5-Dimethyl-4-aminopyridin-2-yl |
| 396 | | Cl | 2-Py | $CH_2$ | 3,5-Dimethyl-4-methoxypyridin-2-yl |
| 397 | | Cl | 2-Py | $CH_2$ | 3,5-Dimethyl-4-methoxy-1-oxypyridin-2-yl |
| 398 | | Cl | 2-Py | $CH_2$ | 3,5-Dimethyl-4-bromopyridin-2-yl |
| 399 | | Cl | 2-Py | $CH_2$ | 3,5-Dimethyl-4-bromo-1-oxypyridin-2-yl |
| 400 | | Cl | 2-Py | $CH_2$ | 3,5-Dimethyl-4-chloropyridin-2-yl |
| 401 | | Cl | 2-Py | $CH_2$ | 3,5-Dimethyl-4-chloro-1-oxypyridin-2-yl |
| 402 | | Cl | 2-Py | $CH_2$ | 3,5-Dimethyl-4-iodopyridin-2-yl |
| 403 | | Cl | 2-Py | $CH_2$ | 3,5-Dimethyl-4-iodo-1-oxypyridin-2-yl |
| 404 | | Cl | 2-Py | $CH_2$ | 3,5-Dimethyl-4-thiomethyl-pyridin-2-yl |
| 405 | | Cl | 2-Py | $CH_2$ | 3,5-Dimethyl-4-thiomethyl-1-oxypyridin-2-yl |
| 406 | | Cl | 2-Py | $CH_2$ | 3,4,5-Trimethyl-pyridin-2-yl |
| 407 | | Cl | 2-Py | $CH_2$ | 3,4,5-Trimethyl-1-oxypyridin-2-yl |
| 408 | | Cl | 2-Py | $CH_2$ | 4,5,6-Trimethoxypyridin-2-yl |
| 409 | | Cl | 2-Py | $CH_2$ | 4,5,6-Trimethoxy-1-oxypyridin-2-yl |
| 410 | | Cl | 2-Py | $CH_2$ | 3,4,5-Trimethoxy-pyridin-2-yl |
| 411 | | Cl | 2-Py | $CH_2$ | 3,4,5-Trimethoxy-1-oxypyridin-2-yl |
| 412 | | Cl | 2-Py | $CH_2$ | 4,5,6-Trimethyl-pyridin-2-yl |
| 413 | | Cl | 2-Py | $CH_2$ | 4,5,6-Trimethyl-1-oxypyridin-2-yl |
| 414 | | Cl | 2-Py | $CH_2$ | 4,6-Dimethyl-5-methoxy-pyridin-2-yl |
| 415 | | Cl | 2-Py | $CH_2$ | 4,6-Dimethyl-5-methoxypyridin-3-yl |
| 416 | | Cl | 2-Py | $CH_2$ | 4,6-Dimethyl-5-methoxy-1-oxypyridin-3-yl |
| 417 | | Cl | 2-Py | $CH_2$ | 4,6-Dimethyl-5-bromopyridin-3-yl |
| 418 | | Cl | 2-Py | $CH_2$ | 4,6-Dimethyl-5-chloropyridin-3-yl |

(continued)

| No. | Ex | $R^1$ | $R^3$ | $R^4$ | $R^5$ |
|---|---|---|---|---|---|
| 419 | | Cl | 2-Py | $CH_2$ | 5,6-Dimethyl-4-bromopyridin-3-yl |
| 420 | | Cl | 2-Py | $CH_2$ | 5,6-Dimethyl-4-chloropyridin-3-yl |
| 421 | | Cl | 2-Py | $CH_2$ | 2,6-Dimethyl-3-methoxypyridin-4-yl |
| 422 | | Cl | 2-Py | $CH_2$ | 2,6-Dimethyl-pyridin-4-yl |
| 423 | | Cl | 2-Py | $CH_2$ | 2,3,6-Trimethyl-pyridin-4-yl |
| 424 | | Cl | 2-Py | $CH_2$ | 2,3,6-Trimethoxy-pyridin-4-yl |
| 425 | | Cl | 2-Py | $CH_2$ | 2,6-Dimethyl-3-bromopyridin-4-yl |
| 426 | | Cl | 2-Py | $CH_2$ | 2,6-Dimethyl-3-chloropyridin-4-yl |
| 427 | | Cl | 2-Py | $CH_2$ | 4,6-Dimethyl-5-iodopyridin-3-yl |
| 428 | | Cl | 2-Py | $CH_2$ | 3,5-Dimethyl-4-aminopyridin-2-yl |
| 429 | 32 | Cl | Ph | $CH_2$ | 3,5-Dimethyl-4-methoxypyridin-2-yl |
| 430 | | Cl | Ph | $CH_2$ | 3,5-Dimethyl-4-methoxy-1-oxypyridin-2-yl |
| 431 | 34 | Cl | Ph | $CH_2$ | 3,5-Dimethyl-4-bromopyridin-2-yl |
| 432 | | Cl | Ph | $CH_2$ | 3,5-Dimethyl-4-bromo-1-oxypyridin-2-yl |
| 433 | 33 | Cl | Ph | $CH_2$ | 3,5-Dimethyl-4-chloropyridin-2-yl |
| 434 | 35 | Cl | Ph | $CH_2$ | 3,5-Dimethyl-4-chloro-1-oxypyridin-2-yl |
| 435 | | Cl | Ph | $CH_2$ | 3,5-Dimethyl-4-iodopyridin-2-yl |
| 436 | | Cl | Ph | $CH_2$ | 3,5-Dimethyl-4-iodo-1-oxypyridin-2-yl |
| 437 | | Cl | Ph | $CH_2$ | 3,5-Dimethyl-4-thiomethyl-pyridin-2-yl |
| 438 | | Cl | Ph | $CH_2$ | 3,5-Dimethyl-4-thiomethyl-1-oxypyridin-2-yl |
| 439 | | Cl | Ph | $CH_2$ | 3,4,5-Trimethyl-pyridin-2-yl |
| 440 | | Cl | Ph | $CH_2$ | 3,4,5-Trimethyl-1-oxypyridin-2-yl |
| 441 | | Cl | Ph | $CH_2$ | 4,5,6-Trimethoxypyridin-2-yl |
| 442 | | Cl | Ph | $CH_2$ | 4,5,6-Trimethoxy-1-oxypyridin-2-yl |
| 443 | | Cl | Ph | $CH_2$ | 3,4,5-Trimethoxy-pyridin-2-yl |
| 444 | | Cl | Ph | $CH_2$ | 3,4,5-Trimethoxy-1-oxypyridin-2-yl |
| 445 | | Cl | Ph | $CH_2$ | 4,5,6-Trimethyl-pyridin-2-yl |
| 446 | | Cl | Ph | $CH_2$ | 4,5,6-Trimethyl-1-oxypyridin-2-yl |
| 447 | | Cl | Ph | $CH_2$ | 4,6-Dimethyl-5-methoxy-pyridin-2-yl |
| 448 | | Cl | Ph | $CH_2$ | 4,6-Dimethyl-5-methoxypyridin-3-yl |
| 449 | | Cl | Ph | $CH_2$ | 4,6-Dimethyl-5-methoxy-1-oxypyridin-3-yl |
| 450 | | Cl | Ph | $CH_2$ | 4,6-Dimethyl-5-bromopyridin-3-yl |
| 451 | | Cl | Ph | $CH_2$ | 4,6-Dimethyl-5-chloropyridin-3-yl |
| 452 | | Cl | Ph | $CH_2$ | 5,6-Dimethyl-4-bromopyridin-3-yl |
| 453 | | Cl | Ph | $CH_2$ | 5,6-Dimethyl-4-chloropyridin-3-yl |
| 454 | | Cl | Ph | $CH_2$ | 2,6-Dimethyl-3-methoxypyridin-4-yl |
| 455 | | Cl | Ph | $CH_2$ | 2,6-Dimethyl-pyridin-4-yl |
| 456 | | Cl | Ph | $CH_2$ | 2,3,6-Trimethyl-pyridin-4-yl |

(continued)

| No. | Ex | $R^1$ | $R^3$ | $R^4$ | $R^5$ |
|---|---|---|---|---|---|
| 457 | | Cl | Ph | $CH_2$ | 2,3,6-Trimethoxy-pyridin-4-yl |
| 458 | | Cl | Ph | $CH_2$ | 2,6-Dimethyl-3-bromopyridin-4-yl |
| 459 | | Cl | Ph | $CH_2$ | 2,6-Dimethyl-3-chloropyridin-4-yl |
| 460 | | Cl | Ph | $CH_2$ | 4,6-Dimethyl-5-iodopyridin-3-yl |
| 461 | | Cl | Ph | $CH_2$ | 3,5-Dimethyl-4-aminopyridin-2-yl |
| 462 | | Cl | 3-Py | $CH_2$ | 3,5-Dimethyl-4-methoxypyridin-2-yl |
| 463 | | Cl | 3-Py | $CH_2$ | 3,5-Dimethyl-4-methoxy-1-oxypyridin-2-yl |
| 464 | | Cl | 3-Py | $CH_2$ | 3,5-Dimethyl-4-bromopyridin-2-yl |
| 465 | | Cl | 3-Py | $CH_2$ | 3,5-Dimethyl-4-bromo-1-oxypyridin-2-yl |
| 466 | | Cl | 3-Py | $CH_2$ | 3,5-Dimethyl-4-chloropyridin-2-yl |
| 467 | | Cl | 3-Py | $CH_2$ | 3,5-Dimethyl-4-chloro-1-oxypyridin-2-yl |
| 468 | | Cl | 3-Py | $CH_2$ | 3,5-Dimethyl-4-iodopyridin-2-yl |
| 469 | | Cl | 3-Py | $CH_2$ | 3,5-Dimethyl-4-iodo-1-oxypyridin-2-yl |
| 470 | | Cl | 3-Py | $CH_2$ | 3,5-Dimethyl-4-thiomethyl-pyridin-2-yl |
| 471 | | Cl | 3-Py | $CH_2$ | 3,5-Dimethyl-4-thiomethyl-1-oxypyridin-2-yl |
| 472 | | Cl | 3-Py | $CH_2$ | 3,4,5-Trimethyl-pyridin-2-yl |
| 473 | | Cl | 3-Py | $CH_2$ | 3,4,5-Trimethyl-1-oxypyridin-2-yl |
| 474 | | Cl | 3-Py | $CH_2$ | 4,5,6-Trimethoxypyridin-2-yl |
| 475 | | Cl | 3-Py | $CH_2$ | 4,5,6-Trimethoxy-1-oxypyridin-2-yl |
| 476 | | Cl | 3-Py | $CH_2$ | 3,4,5-Trimethoxy-pyridin-2-yl |
| 477 | | Cl | 3-Py | $CH_2$ | 3,4,5-Trimethoxy-1-oxypyridin-2-yl |
| 478 | | Cl | 3-Py | $CH_2$ | 4,5,6-Trimethyl-pyridin-2-yl |
| 479 | | Cl | 3-Py | $CH_2$ | 4,5,6-Trimethyl-1-oxypyridin-2-yl |
| 480 | | Cl | 3-Py | $CH_2$ | 4,6-Dimethyl-5-methoxy-pyridin-2-yl |
| 481 | | Cl | 3-Py | $CH_2$ | 4,6-Dimethyl-5-methoxypyridin-3-yl |
| 482 | | Cl | 3-Py | $CH_2$ | 4,6-Dimethyl-5-methoxy-1-oxypyridin-3-yl |
| 483 | | Cl | 3-Py | $CH_2$ | 4,6-Dimethyl-5-bromopyridin-3-yl |
| 484 | | Cl | 3-Py | $CH_2$ | 4,6-Dimethyl-5-chloropyridin-3-yl |
| 485 | | Cl | 3-Py | $CH_2$ | 5,6-Dimethyl-4-bromopyridin-3-yl |
| 486 | | Cl | 3-Py | $CH_2$ | 5,6-Dimethyl-4-chloropyridin-3-yl |
| 487 | | Cl | 3-Py | $CH_2$ | 2,6-Dimethyl-3-methoxypyridin-4-yl |
| 488 | | Cl | 3-Py | $CH_2$ | 2,6-Dimethyl-pyridin-4-yl |
| 489 | | Cl | 3-Py | $CH_2$ | 2,3,6-Trimethyl-pyridin-4-yl |
| 490 | | Cl | 3-Py | $CH_2$ | 2,3,6-Trimethoxy-pyridin-4-yl |
| 491 | | Cl | 3-Py | $CH_2$ | 2,6-Dimethyl-3-bromopyridin-4-yl |
| 492 | | Cl | 3-Py | $CH_2$ | 2,6-Dimethyl-3-chloropyridin-4-yl |
| 493 | | Cl | 3-Py | $CH_2$ | 4,6-Dimethyl-5-iodopyridin-3-yl |
| 494 | | Cl | 3-Py | $CH_2$ | 3,5-Dimethyl-4-aminopyridin-2-yl |

(continued)

| No. | Ex | $R^1$ | $R^3$ | $R^4$ | $R^5$ |
|---|---|---|---|---|---|
| 495 | | Cl | $CH_2$- $NMe_2$ | $CH_2$ | 3,5-Dimethyl-4-methoxypyridin-2-yl |
| 496 | | Cl | $CH_2$- | $CH_2$ | 3,5-Dimethyl-4-methoxy-1-oxypyridin-2-yl |
| 497 | | Cl | $NMe_2$ | $CH_2$ | 3,5-Dimethyl-4-bromopyridin-2-yl |
| 498 | | Cl | $CH_2$- $NMe_2$ | $CH_2$ | 3,5-Dimethyl-4-bromo-1-oxypyridin-2-yl |
| 499 | | Cl | $CH_2$- $NMe_2$ | $CH_2$ | 3,5-Dimethyl-4-chloropyridin-2-yl |
| 500 | | Cl | $CH_2$- $NMe_2$ | $CH_2$ | 3,5-Dimethyl-4-chloro-1-oxypyridin-2-yl |
| 501 | | Cl | $CH_2$- $NMe_2$ | $CH_2$ | 3,5-Dimethyl-4-iodopyridin-2-yl |
| 502 | | Cl | $CH_2$- $NMe_2$ | $CH_2$ | 3,5-Dimethyl-4-iodo-1-oxypyridin-2-yl |
| 503 | | Cl | $CH_2$- $NMe_2$ | $CH_2$ | 3,5-Dimethyl-4-thiomethyl-pyridin-2-yl |
| 504 | | Cl | $CH_2$- $NMe_2$ | $CH_2$ | 3,5-Dimethyl-4-thiomethyl-1-oxypyridin-2-yl |
| 505 | | Cl | $CH_2$- $NMe_2$ | $CH_2$ | 3,4,5-Trimethyl-pyridin-2-yl |
| 506 | | Cl | $CH_2$- $NMe_2$ | $CH_2$ | 3,4,5-Trimethyl-1-oxypyridin-2-yl |
| 507 | | Cl | $CH_2$- $NMe_2$ | $CH_2$ | 4,5,6-Trimethoxypyridin-2-yl |
| 508 | | Cl | $CH_2$- $NMe_2$ | $CH_2$ | 4,5,6-Trimethoxy-1-oxypyridin-2-yl |
| 509 | | Cl | $CH_2$- $NMe_2$ | $CH_2$ | 3,4,5-Trimethoxy-pyridin-2-yl |
| 510 | | Cl | $CH_2$- $NMe_2$ | $CH_2$ | 3,4,5-Trimethoxy-1-oxypyridin-2-yl |
| 511 | | Cl | $CH_2$- $NMe_2$ | $CH_2$ | 4,5,6-Trimethyl-pyridin-2-yl |
| 512 | | Cl | $CH_2$- $NMe_2$ | $CH_2$ | 4,5,6-Trimethyl-1-oxypyridin-2-yl |
| 513 | | Cl | $CH_2$- $NMe_2$ | $CH_2$ | 4,6-Dimethyl-5-methoxy-pyridin-2-yl |
| 514 | | Cl | $CH_2$- $NMe_2$ | $CH_2$ | 4,6-Dimethyl-5-methoxypyridin-3-yl |
| 515 | | Cl | $CH_2$- $NMe_2$ | $CH_2$ | 4,6-Dimethyl-5-methoxy-1-oxypyridin-3-yl |
| 516 | | Cl | $CH_2$- $NMe_2$ | $CH_2$ | 4,6-Dimethyl-5-bromopyridin-3-yl |
| 517 | | Cl | $CH_2$- $NMe_2$ | $CH_2$ | 4,6-Dimethyl-5-chloropyridin-3-yl |
| 518 | | Cl | $CH_2$- $NMe_2$ | $CH_2$ | 5,6-Dimethyl-4-bromopyridin-3-yl |
| 519 | | Cl | $CH_2$- $NMe_2$ | $CH_2$ | 5,6-Dimethyl-4-chloropyridin-3-yl |
| 520 | | Cl | $CH_2$- $NMe_2$ | $CH_2$ | 2,6-Dimethyl-3-methoxypyridin-4-yl |
| 521 | | Cl | $CH_2$- $NMe_2$ | $CH_2$ | 2,6-Dimethyl-pyridin-4-yl |
| 522 | | Cl | $CH_2$- $NMe_2$ | $CH_2$ | 2,3,6-Trimethyl-pyridin-4-yl |
| 523 | | Cl | $CH_2$- $NMe_2$ | $CH_2$ | 2,3,6-Trimethoxy-pyridin-4-yl |
| 524 | | Cl | $CH_2$- $NMe_2$ | $CH_2$ | 2,6-Dimethyl-3-bromopyridin-4-yl |
| 525 | | Cl | $CH_2$- $NMe_2$ | $CH_2$ | 2,6-Dimethyl-3-chloropyridin-4-yl |
| 526 | | Cl | $CH_2$- $NMe_2$ | $CH_2$ | 4,6-Dimethyl-5-iodopyridin-3-yl |
| 527 | | Cl | $CH_2$- $NMe_2$ | $CH_2$ | 3,5-Dimethyl-4-aminopyridin-2-yl |
| 528 | | Cl | 2- furanyl | $CH_2$ | 3,5-Dimethyl-4-methoxypyridin-2-yl |
| 529 | | Cl | 2- furanyl | $CH_2$ | 3,5-Dimethyl-4-methoxy-1-oxypyridin-2-yl |
| 530 | | Cl | 2- furanyl | $CH_2$ | 3,5-Dimethyl-4-bromopyridin-2-yl |
| 531 | | Cl | 2-furanyl | $CH_2$ | 3,5-Dimethyl-4-bromo-1-oxypyridin-2-yl |
| 532 | | Cl | 2-furanyl | $CH_2$ | 3,5-Dimethyl-4-chloropyridin-2-yl |

(continued)

| No. | Ex | $R^1$ | $R^3$ | $R^4$ | $R^5$ |
|---|---|---|---|---|---|
| 533 | | Cl | 2-furanyl | $CH_2$ | 3,5-Dimethyl-4-chloro-1-oxypyridin-2-yl |
| 534 | | Cl | 2- furanyl | $CH_2$ | 3,5-Dimethyl-4-iodopyridin-2-yl |
| 536 | | Cl | 2-furanyl | $CH_2$ | 3,5-Dimethyl-4-iodo-1-oxypyridin-2-yl |
| 537 | | Cl | 2-furanyl | $CH_2$ | 3,5-Dimethyl-4-thiomethyl-pyridin-2-yl |
| 538 | | Cl | 2- | $CH_2$ | 3,5-Dimethyl-4-thiomethyl-1-oxypyridin-2-yl |
| 539 | | Cl | 2-furanyl | $CH_2$ | 3,4,5-Trimethyl-pyridin-2-yl |
| 540 | | Cl | 2-furanyl | $CH_2$ | 3,4,5-Trimethyl-1-oxypyridin-2-yl |
| 541 | | Cl | 2-furanyl | $CH_2$ | 4,5,6-Trimethoxypyridin-2-yl |
| 542 | | Cl | 2- furanyl | $CH_2$ | 4,5,6-Trimethoxy-1-oxypyridin-2-yl |
| 543 | | Cl | 2- furanyl | $CH_2$ | 3,4,5-Trimethoxy-pyridin-2-yl |
| 544 | | Cl | 2-furanyl | $CH_2$ | 3,4,5-Trimethoxy-1-oxypyridin-2-yl |
| 545 | | Cl | 2-furanyl | $CH_2$ | 4,5,6-Trimethyl-pyridin-2-yl |
| 546 | | Cl | 2-furanyl | $CH_2$ | 4,5,6-Trimethyl-1-oxypyridin-2-yl |
| 547 | | Cl | 2-furanyl | $CH_2$ | 4,6-Dimethyl-5-methoxy-pyridin-2-yl |
| 548 | | Cl | 2- furanyl | $CH_2$ | 4,6-Dimethyl-5-methoxypyridin-3-yl |
| 549 | | Cl | 2- furanyl | $CH_2$ | 4,6-Dimethyl-5-methoxy-l-oxypyndin-3-yl |
| 550 | | Cl | 2- furanyl | $CH_2$ | 4,6-Dimethyl-5 -bromopyridin-3-yl |
| 551 | | Cl | 2-furanyl | $CH_2$ | 4,6-Dimethyl-5-chloropyridin-3-yl |
| 552 | | Cl | 2-furanyl | $CH_2$ | 5,6-Dimethyl-4-bromopyridin-3-yl |
| 553 | | Cl | 2- furanyl | $CH_2$ | 5,6-Dimethyl-4-chloropyridin-3-yl |
| 554 | | Cl | 2-furanyl | $CH_2$ | 2,6-Dimethyl-3-methoxypyridin-4-yl |
| 555 | | Cl | 2-furanyl | $CH_2$ | 2,6-Dimethyl-pyridin-4-yl |
| 556 | | Cl | 2-furanyl | $CH_2$ | 2,3,6-Trimethyl-pyridin-4-yl |
| 557 | | Cl | 2-furanyl | $CH_2$ | 2,3,6-Trimethoxy-pyridin-4-yl |
| 558 | | Cl | 2-furanyl | $CH_2$ | 2,6-Dimethyl-3-bromopyridin-4-yl |
| 559 | | Cl | 2- furanyl | $CH_2$ | 2,6-Dimethyl-3-chloropyridin-4-yl |
| 560 | | Cl | 2-furanyl | $CH_2$ | 3,5-Dimethyl-4-aminopyridin-2-yl |
| 561 | | Cl | Cl | $CH_2$ | 3,5-Dimethyl-4-methoxypyridin-2-yl |
| 562 | | Cl | Cl | $CH_2$ | 3,5-Dimethyl-4-methoxy-1-oxypyridin-2-yl |
| 563 | | Cl | Cl | $CH_2$ | 3,5-Dimethyl-4-bromopyridin-2-yl |
| 564 | | Cl | Cl | $CH_2$ | 3,5-Dimethyl-4-bromo-1-oxypyridin-2-yl |
| 565 | | Cl | Cl | $CH_2$ | 3,5-Dimethyl-4-chloropyridin-2-yl |
| 566 | | Cl | Cl | $CH_2$ | 3,5-Dimethyl-4-chloro-1-oxypyridin-2-yl |
| 567 | | Cl | Cl | $CH_2$ | 3,5-Dimethyl-4-iodopyridin-2-yl |
| 568 | | Cl | Cl | $CH_2$ | 3,5-Dimethyl-4-iodo-1-oxypyridin-2-yl |
| 569 | | Cl | Cl | $CH_2$ | 3,5-Dimethyl-4-thiomethyl-pyridin-2-yl |
| 570 | | Cl | Cl | $CH_2$ | 3,5-Dimethyl-4-thiomethyl-1-oxypyridin-2-yl |
| 571 | | Cl | Cl | $CH_2$ | 3,4,5-Trimethyl-pyridin-2-yl |

(continued)

| No. | Ex | $R^1$ | $R^3$ | $R^4$ | $R^5$ |
|---|---|---|---|---|---|
| 572 | | Cl | Cl | $CH_2$ | 3,4,5-Trimethyl-1-oxypyridin-2-yl |
| 573 | | Cl | Cl | $CH_2$ | 4,6-Dimethyl-5-methoxypyridin-3-yl |
| 574 | | Cl | Cl | $CH_2$ | 4,6-Dimethyl-5-methoxy-1-oxypyridin-3-yl |
| 575 | | Cl | Cl | $CH_2$ | 3,5-Dimethyl-4-aminopyridin-2-yl |
| 576 | | Cl | Br | $CH_2$ | 3,5-Dimethyl-4-methoxypyridin-2-yl |
| 577 | | Cl | Br | $CH_2$ | 3,5-Dimethyl-4-methoxy-1-oxypyridin-2-yl |
| 578 | | Cl | Br | $CH_2$ | 3,5-Dimethyl-4-bromopyridin-2-yl |
| 579 | | Br | Br | $CH_2$ | 3,5-Dimethyl-4-bromo-1-oxypyridin-2-yl |
| 580 | | Cl | Br | $CH_2$ | 3,5-Dimethyl-4-chloropyridin-2-yl . |
| 581 | | Br | Br | $CH_2$ | 3,5-Dimethyl-4-chloro-1-oxypyridin-2-yl |
| 582 | | Cl | Br | $CH_2$ | 3,5-Dimethyl-4-iodopyridin-2-yl |
| 583 | | Br | Br | $CH_2$ | 3,5-Dimethyl-4-iodo-1-oxypyridin-2-yl |
| 584 | | Cl | Br | $CH_2$ | 3,5-Dimethyl-4-thiomethyl-pyridin-2-yl |
| 585 | | Br | Br | $CH_2$ | 3,5-Dimethyl-4-thiomethyl-1-oxypyridin-2-yl |
| 586 | | Cl | Br | $CH_2$ | 3,4,5-Trimethyl-pyridin-2-yl |
| 587 | | Br | Br | $CH_2$ | 3,4,5-Trimethyl-1-oxypyridin-2-yl |
| 588 | | Cl | Br | $CH_2$ | 4,6-Dimethyl-5-methoxypyidin-3-yl |
| 589 | | Cl | Br | $CH_2$ | 4,6-Dimethyl-5-methoxy-1-oxypyridin-3-yl |
| 590 | | Cl | Br | $CH_2$ | 3,5-Dimethyl-4-aminopyridin-2-yl |
| 591 | | Cl | I | $CH_2$ | 3,5-Dimethyl-4-methoxypyridin-2-yl |
| 592 | | Cl | I | $CH_2$ | 3,5-Dimethyl-4-methoxy-1-oxypyridin-2-yl |
| 593 | | Cl | I | $CH_2$ | 3,5-Dimethyl-4-bromopyridin-2-yl . |
| 594 | | Cl | I | $CH_2$ | 3,5-Dimethyl-4-bromo-1-oxypyridin-2-yl |
| 595 | | Cl | I | $CH_2$ | 3,5-Dimethyl-4-chloropyridin-2-yl |
| 596 | | Cl | I | $CH_2$ | 3,5-Dimethyl-4-chloro-1-oxypyridin-2-yl |
| 597 | | Cl | I | $CH_2$ | 3,5-Dimethyl-4-iodopyridin-2-yl |
| 598 | | Cl | I | $CH_2$ | 3,5-Dimethyl-4-iodo-1-oxypyridin-2-yl |
| 599 | | Cl | I | $CH_2$ | 3,5-Dimethyl-4-thiomethyl-pyridin-2-yl |
| 600 | | Cl | I | $CH_2$ | 3,5-Dimethyl-4-thiomethyl-1-oxypyridin-2-yl |
| 601 | | Cl | I | $CH_2$ | 3,4,5-Trimethyl-pyridin-2-yl |
| 602 | | Cl | I | $CH_2$ | 3,4,5-Trimethyl-1-oxypyridin-2-yl |
| 603 | | Cl | I | $CH_2$ | 4,6-Dimethyl-5-methoxypyridin-3-yl |
| 604 | | Cl | I | $CH_2$ | 4,6-Dimethyl-5-methoxy-1-oxypyridin-3-yl |
| 605 | | Cl | I | $CH_2$ | 3,5-Dimethyl-4-aminopyridin-2-yl |
| 606 | | Cl | CN | $CH_2$ | 3,5-Dimethyl-4-methoxypyridin-2-yl |
| 607 | | Cl | CN | $CH_2$ | 3,5-Dimethyl-4-methoxy-1-oxypyridin-2-yl |
| 608 | | Cl | CN | $CH_2$ | 3,5-Dimethyl-4-bromopyridin-2-yl |
| 609 | | Cl | CN | $CH_2$ | 3,5-Dimethyl-4-bromo-1-oxypyridin-2-yl |

(continued)

| No. | Ex | $R^1$ | $R^3$ | $R^4$ | $R^5$ |
|---|---|---|---|---|---|
| 610 | | Cl | CN | $CH_2$ | 3,5-Dimethyl-4-chloropyridin-2-yl |
| 611 | | Cl | CN | $CH_2$ | 3,5-Dimethyl-4-chloro-1-oxypyridin-2-yl |
| 612 | | Cl | CN | $CH_2$ | 3,5-Dimethyl-4-iodopyridin-2-yl |
| 613 | | Cl | CN | $CH_2$ | 3,5-Dimethyl-4-iodo-1-oxypyridin-2-yl |
| 614 | | Cl | CN | $CH_2$ | 3,5-Dimethyl-4-thiomethyl-pyridin-2-yl |
| 615 | | Cl | CN | $CH_2$ | 3,5-Dimethyl-4-thiomethyl-1-oxypyridin-2-yl |
| 616 | | Cl | CN | $CH_2$ | 3,4,5-Trimethyl-pyridin-2-yl |
| 617 | | Cl | CN | $CH_2$ | 3,4,5-Trimethyl-1-oxypyridin-2-yl |
| 618 | | Cl | CN | $CH_2$ | 4,6-Dimethyl-5-methoxypyridin-3-yl |
| 619 | | Cl | CN | $CH_2$ | 4,6-Dimethyl-5-methoxy-1-oxypyridin-3-yl |
| 620 | | Cl | CN | $CH_2$ | 3,5-Dimethyl-4-aminopyridin-2-yl |
| 621 | | Cl | H | C(O) | 3,5-Dimethyl-4-methoxypyridin-2-yl |
| 622 | | Cl | H | C(O) | 3,5-Dimethyl-4-methoxy-1-oxypyridin-2-yl |
| 623 | | Cl | H | C(O) | 3,5-Dimethyl-4-bromopyridin-2-yl |
| 624 | | Cl | H | C(O) | 3,5-Dimethyl-4-bromo-1-oxypyridin-2-yl |
| 625 | | Cl | H | C(O) | 3,5-Dimethyl-4-chloropyridin-2-yl |
| 626 | | Cl | H | C(O) | 3,5-Dimethyl-4-chloro-1-oxypyridin-2-yl |
| 627 | | Cl | H | C(O) | 3,5-Dimethyl-4-iodopyridin-2-yl |
| 628 | | Cl | H | C(O) | 3,5-Dimethyl-4-iodo-1-oxypyridin-2-yl |
| 629 | | Cl | H | C(O) | 3,5-Dimethyl-4-thiomethyl-pyridin-2-yl |
| 630 | | Cl | H | C(O) | 3,5-Dimethyl-4-thiomethyl-1-oxypyridin-2-yl |
| 631 | | Cl | H | C(O) | 3,4,5-Trimethyl-pyridin-2-yl |
| 632 | | Cl | H | C(O) | 3,4,5-Trimethyl-1-oxypyridin-2-yl |
| 633 | | Cl | H | C(O) | 4,6-Dimethyl-5-methoxypyridin-3-yl |
| 634 | | Cl | H | C(O) | 4,6-Dimethyl-5-methoxy-1-oxypyridin-3-yl |
| 635 | | Cl | H | C(O) | 3,5-Dimethyl-4-aminopyridin-2-yl |
| 636 | | Cl | H | S(O) | 3,5-Dimethyl-4-methoxypyridin-2-yl |
| 637 | | Cl | H | S(O) | 3,5-Dimethyl-4-methoxy-1-oxypyridin-2-yl |
| 638 | | Cl | H | S(O) | 3,5-Dimethyl-4-bromopyridin-2-yl |
| 639 | | Cl | H | S(O) | 3,5-Dimethyl-4-bromo-1-oxypyridin-2-yl |
| 640 | | Cl | H | S(O) | 3,5-Dimethyl-4-chloropyridin-2-yl |
| 641 | | Cl | H | S(O) | 3,5-Dimethyl-4-chloro-1-oxypyridin-2-yl |
| 642 | | Cl | H | S(O) | 3,5-Dimethyl-4-iodopyridin-2-yl |
| 643 | | Cl | H | S(O) | 3,5-Dimethyl-4-iodo-1-oxypyridin-2-yl |
| 644 | | Cl | H | S(O) | 3,5-Dimethyl-4-thiomethyl-pyridin-2-yl |
| 645 | | Cl | H | S(O) | 3,5-Dimethyl-4-thiomethyl-1-oxypyridin-2-yl |
| 646 | | Cl | Br | S(O) | 3,4,5-Trimethyl-pyridin-2-yl |
| 647 | | Cl | H | S(O) | 3,4,5-Trimethyl-1-oxypyridin-2-yl |

(continued)

| No. | Ex | $R^1$ | $R^3$ | $R^4$ | $R^5$ |
|---|---|---|---|---|---|
| 648 | | Cl | Br | S(O) | 4,6-Dimethyl-5-methoxypyridin-3-yl |
| 649 | | Cl | H | S(O) | 4,6-Dimethyl-5-methoxy-1-oxypyridin-3-yl |
| 650 | | Cl | H | $SO_2$ | 3,5-Dimethyl-4-methoxypyridin-2-yl |
| 651 | | Cl | H | $SO_2$ | 3,5-Dimethyl-4-methoxy-1-oxypyridin-2-yl |
| 652 | | Cl | H | $SO_2$ | 3,5-Dimethyl-4-bromopyridin-2-yl |
| 653 | | Cl | H | $SO_2$ | 3,5-Dimethyl-4-bromo-1-oxypyridin-2-yl |
| 654 | | Cl | Br | $SO_2$ | 3,5-Dimethyl-4-chloropyridin-2-yl |
| 655 | | Cl | H | $SO_2$ | 3,5-Dimethyl-4-chloro-1-oxypyridin-2-yl |
| 656 | | Cl | H | $SO_2$ | 3,5-Dimethyl-4-iodopyridin-2-yl |
| 657 | | Cl | H | $SO_2$ | 3,5-Dimethyl-4-iodo-1-oxypyridin-2-yl |
| 658 | | Cl | H | $SO_2$ | 3,5-Dimethyl-4-thiomethyl-pyridin-2-yl |
| 659 | | Cl | H | $SO_2$ | 3,5-Dimethyl-4-thiomethyl-1-oxypyridin-2-yl |
| 660 | | Cl | H | $SO_2$ | 3,4,5-Trimethyl-pyridin-2-yl |
| 661 | | Cl | H | $SO_2$ | 3,4,5-Trimethyl-1-oxypyridin-2-yl |
| 662 | | Cl | H | $SO_2$ | 4,6-Dimethyl-5-methoxypyridin-3-yl |
| 663 | | Cl | H | $SO_2$ | 4,6-Dimethyl-5-methoxy-1-oxypyridin-3-yl |
| 664 | | Cl | i-pr | C(O) | 3,5-Dimethyl-4-methoxypyridin-2-yl |
| 665 | | Cl | i-pr | C(O) | 3,5-Dimethyl-4-methoxy-1-oxypyridin-2-yl |
| 666 | | Cl | i-pr | C(O) | 3,5-Dimethyl-4-bromopyridin-2-yl |
| 667 | | Cl | i-pr | C(O) | 3,5-Dimethyl-4-bromo-1-oxypyridin-2-yl |
| 668 | | Cl | i-pr | C(O) | 3,5-Dimethyl-4-chloropyridin-2-yl |
| 669 | | Cl | i-pr | C(O) | 3,5-Dimethyl-4-chloro-1-oxypyridin-2-yl |
| 670 | | Cl | i-pr | C(O) | 3,5-Dimethyl-4-iodopyridin-2-yl |
| 671 | | Cl | i-pr | C(O) | 3,5-Dimethyl-4-iodo-1-oxypyridin-2-yl |
| 672 | | Cl | i-pr | C(O) | 3,5-Dimethyl-4-thiomethyl-pyridin-2-yl |
| 673 | | Cl | i-pr | C(O) | 3,5-Dimethyl-4-thiomethyl-1-oxypyridin-2-yl |
| 674 | | Cl | i-pr | C(O) | 3,4,5-Trimethyl-pyridin-2-yl |
| 675 | | Cl | i-pr | C(O) | 3,4,5-Trimethyl-1-oxypyridin-2-yl |
| 676 | | Cl | i-pr | C(O) | 4,6-Dimethyl-5-methoxypyridin-3-yl |
| 677 | | Cl | i-pr | C(O) | 4,6-Dimethyl-5-methoxy-1-oxypyridin-3-yl |
| 678 | | Cl | i-pr | C(O) | 3,5-Dimethyl-4-aminopyridin-2-yl |
| 679 | | Cl | i-pr | S(O) | 3,5-Dimethyl-4-methoxypyridin-2-yl |
| 680 | | Cl | i-pr | S(O) | 3,5-Dimethyl-4-methoxy-1-oxypyridin-2-yl |
| 681 | | Cl | i-pr | S(O) | 3,5-Dimethyl-4-bromopyridin-2-yl |
| 682 | | Cl | i-pr | S(O) | 3,5-Dimethyl-4-bromo-1-oxypyridin-2-yl |
| 683 | | Cl | i-pr | S(O) | 3,5-Dimethyl-4-chloropyridin-2-yl |
| 684 | | Cl | i-pr | S(O) | 3,5-Dimethyl-4-chloro-1-oxypyfidin-2-yl |
| 685 | | Cl | i-pr | S(O) | 3,5-Dimethyl-4-iodopyridin-2-yl |

(continued)

| No. | Ex | $R^1$ | $R^3$ | $R^4$ | $R^5$ |
|---|---|---|---|---|---|
| 686 | | Cl | i-pr | S(O) | 3,5-Dimethyl-4-iodo-1-oxypyridin-2-yl |
| 687 | | Cl | i-pr | S(O) | 3,5-Dimethyl-4-thiomethyl-pyridin-2-yl |
| 688 | | Cl | i-pr | S(O) | 3,5-Dimethyl-4-thiomethyl-1-oxypyridin-2-yl |
| 689 | | Cl | i-pr | S(O) | 3,4,5-Trimethyl-pyridin-2-yl |
| 690 | | Cl | i-pr | S(O) | 3,4,5-Trimethyl-1-oxypyridin-2-yl |
| 691 | | Cl | i-pr | S(O) | 4,6-Dimethyl-5-methoxypyridin-3-yl |
| 692 | | Cl | i-pr | S(O) | 4,6-Dimethyl-5-methoxy-1-oxypyridin-3-yl |
| 693 | | Cl | i-pr | $SO_2$ | 3,5-Dimethyl-4-methoxypyridin-2-yl |
| 694 | | Cl | i-pr | $SO_2$ | 3,5-Dimethyl-4-methoxy-1-oxypyridin-2-yl |
| 695 | | Cl | i-pr | $SO_2$ | 3,5-Dimethyl-4-bromopyridin-2-yl |
| 696 | | Cl | i-pr | $SO_2$ | 3,5-Dimethyl-4-bromo-1-oxypyridin-2-yl |
| 697 | | Cl | i-pr | $SO_2$ | 3,5-Dimethyl-4-chloropyridin-2-yl |
| 698 | | Cl | i-pr | $SO_2$ | 3,5-Dimethyl-4-chloro-1-oxypyridin-2-yl |
| 699 | | Cl | i-pr | $SO_2$ | 3,5-Dimethyl-4-iodopyridin-2-yl |
| 700 | | Cl | i-pr | $SO_2$ | 3,5-Dimethyl-4-iodo-1-oxypyridin-2-yl |
| 701 | | Cl | i-pr | $SO_2$ | 3,5-Dimethyl-4-thiomethyl-pyridin-2-yl |
| 702 | | Cl | i-pr | $SO_2$ | 3,5-Dimethyl-4-thiomethyl-1-oxypyridin-2-yl |
| 703 | | Cl | i-pr | $SO_2$ | 3,4,5-Trimethyl-pyridin-2-yl |
| 704 | | Cl | i-pr | $SO_2$ | 3,4,5-Trimethyl-1-oxypyridin-2-yl |
| 705 | | Cl | i-pr | $SO_2$ | 4,6-Dimethyl-5-methoxypyridin-3-yl |
| 706 | | Cl | i-pr | $SO_2$ | 4,6-Dimethyl-5-methoxy-1-oxypyridin-3-yl |
| 707 | | Cl | H | C(O) | 3,4,5-Trimethoxyphenyl |
| 708 | | Cl | H | C(O) | 2-Chloro-3,4,5-trimethoxyphenyl |
| 709 | | Cl | H | C(O) | 2-Bromo-3,4,5-trimethoxyphenyl |
| 710 | | Cl | H | C(O) | 3,5-Dimethyl-4-methoxyphenyl |
| 711 | | Cl | H | C(O) | 2-Chloro-3,5-Dimethyl-4-methoxyphenyl |
| 712 | | Cl | H | C(O) | 2-Bromo-3,5-Dimethyl-4-methoxyphenyl |
| 713 | | Cl | H | $SO_2$ | 3,4,5-Trimethoxyphenyl |
| 714 | | Cl | H | $SO_2$ | 2-Chloro-3,4,5-trimethoxyphenyl |
| 715 | | Cl | H | $SO_2$ | 2-Bromo-3,4,5-trimethoxyphenyl |
| 716 | | Cl | H | $SO_2$ | 3,5-Dimethyl-4-methoxyphenyl |
| 717 | | Cl | H | $SO_2$ | 2-Chloro-3,5-Dimethyl-4-methoxyphenyl |
| 718 | | Cl | H | $SO_2$ | 2-Bromo-3,5-Dimethyl-4-methoxyphenyl |
| 719 | | Br | H | $CH_2$ | 3,5-Dimethyl-4-methoxypyridin-2-yl |
| 720 | | Br | H | $CH_2$ | 3,5-Dimethyl-4-methoxy-1-oxypyridin-2-yl |
| 721 | | Br | H | $CH_2$ | 6-Bromo-3,5-dimethyl-4-methoxypyridin-2-yl |
| 722 | | Br | H | $CH_2$ | 6-Chloro-3,5-dimethyl-4-methoxypyridin-2-yl |
| 723 | | Br | H | $CH_2$ | 3,5-Dimethyl-4-bromopyridin-2-yl |

(continued)

| No. | Ex | $R^1$ | $R^3$ | $R^4$ | $R^5$ |
|---|---|---|---|---|---|
| 724 | | Br | H | $CH_2$ | 3,5-Dimethyl-4-bromo-1-oxypyridin-2-yl |
| 725 | | Br | H | $CH_2$ | 3,5-Dimethyl-4-chloropyridin-2-yl |
| 726 | | Br | H | $CH_2$ | 3,5-Dimethyl-4-chloro-1-oxypyridin-2-yl |
| 727 | | Br | H | $CH_2$ | 3,5-Dimethyl-4-iodopyridin-2-yl |
| 728 | | Br | H | $CH_2$ | 3,5-Dimethyl-4-iodo-1-oxypyridin-2-yl |
| 729 | | Br | H | $CH_2$ | 3,5-Dimethyl-4-thiomethyl-pyridin-2-yl |
| 730 | | Br | H | $CH_2$ | 3,5-Dimethyl-4-thiomethyl-1-oxypyridin-2-yl |
| 731 | | Br | H | $CH_2$ | 3,4,5-Trimethyl-pyridin-2-yl |
| 732 | | Br | H | $CH_2$ | 3,4,5-Trimethyl-1-oxypyridin-2-yl |
| 733 | | Br | H | $CH_2$ | 4,5,6-Trimethoxypyridin-2-yl |
| 734 | | Br | H | $CH_2$ | 4,5,6-Trimethoxy-1-oxypyridin-2-yl |
| 735 | | Br | H | $CH_2$ | 3-Bromo-4,5,6-trimethoxypyridin-2-yl |
| 736 | | Br | H | $CH_2$ | 3-Chloro-4,5,6-trimethoxypyridin-2-yl |
| 737 | | Br | H | $CH_2$ | 3,4,5-Trimethoxy-pyridin-2-yl |
| 738 | | Br | H | $CH_2$ | 3,4,5-Trimethoxy-1-oxypyridin-2-yl |
| 739 | | Br | H | $CH_2$ | 3-Bromo-3,4,5-trimethoxy-pyridin-2-yl |
| 740 | | Br | H | $CH_2$ | 3-Chloro-3,4,5-trimethoxy-pyridin-2-yl |
| 741 | | Br | H | $CH_2$ | 4,5,6-Trimethyl-pyridin-2-yl |
| 742 | | Br | H | $CH_2$ | 4,5,6-Trimethyl-1-oxypyridin-2-yl |
| 743 | | Br | H | $CH_2$ | 4,6-Dimethyl-5-methoxy-pyridin-2-yl |
| 744 | | Br | H | $CH_2$ | 4,6-Dimethyl-5-methoxypyridin-3-yl |
| 745 | | Br | H | $CH_2$ | 4,6-Dimethyl-5-methoxy-1-oxypyridin-3-yl |
| 746 | | Br | H | $CH_2$ | 4,6-Dimethyl-5-bromopyridin-3-yl |
| 747 | | Br | H | $CH_2$ | 4,6-Dimethyl-5-chloropyridin-3-yl |
| 748 | | Br | H | $CH_2$ | 5,6-Dimethyl-4-bromopyridin-3-yl |
| 749 | | Br | H | $CH_2$ | 5,6-Dimethyl-4-chloropyridin-3-yl |
| 750 | | Br | H | $CH_2$ | 2,6-Dimethyl-3-methoxypyridin-4-yl |
| 751 | | Br | H | $CH_2$ | 2,6-Dimethyl-pyridin-4-yl |
| 752 | | Br | H | $CH_2$ | 2,3,6-Trimethyl-pyridin-4-yl |
| 753 | | Br | H | $CH_2$ | 2,3,6-Trimethoxy-pyridin-4-yl |
| 754 | | Br | H | $CH_2$ | 2,6-Dimethyl-3-bromopyridin-4-yl |
| 755 | | Br | H | $CH_2$ | 2,6-Dimethyl-3-chloropyridin-4-yl |
| 756 | | Br | H | $CH_2$ | 2,6-Dimethyl-3-methoxy-1-oxy-pyridin-4-yl |
| 757 | | Br | H | $CH_2$ | 2,6-Dimethyl-1-oxy-pyridin-4-yl |
| 758 | | Br | H | $CH_2$ | 2,3,6-Trimethyl-1-oxy-pyridin-4-yl |
| 759 | | Br | H | $CH_2$ | 2,3,6-Trimethoxy-1-oxy-pyridin-4-yl |
| 760 | | Br | H | $CH_2$ | 2,6-Dimethyl-3-bromol-oxy-pyridin-4-yl |
| 761 | | Br | H | $CH_2$ | 2,6-Dimethyl-3-chlorol-oxy-pyridin-4-yl |

(continued)

| No. | Ex | $R^1$ | $R^3$ | $R^4$ | $R^5$ |
|---|---|---|---|---|---|
| 762 | | Br | H | $CH_2$ | 4,6-Dimethyl-5-iodopyridin-3-yl |
| 763 | | Br | H | $CH_2$ | 3,5-Dimethyl-4-aminopyridin-2-yl |
| 764 | | Br | i-pr | $CH_2$ | 3,5-Dimethyl-4-methoxypyridin-2-yl |
| 765 | | Br | i-pr | $CH_2$ | 3,5-Dimethyl-4-methoxy-1-oxypyridin-2-yl |
| 766 | | Br | i-pr | $CH_2$ | 6-Bromo-3,5-dimethyl-4-methoxypyridin-2-yl |
| 767 | | Br | i-pr | $CH_2$ | 6-Chloro-3,5-dimethyl-4-methoxypyridin-2-yl |
| 768 | | Br | i-pr | $CH_2$ | 3,5-Dimethyl-4-bromopyridin-2-yl |
| 769 | | Br | i-pr | $CH_2$ | 3,5-Dimethyl-4-bromo-1-oxypyridin-2-yl |
| 770 | | Br | i-pr | $CH_2$ | 3,5-Dimethyl-4-ehloropyridin-2-yl |
| 771 | | Br | i-pr | $CH_2$ | 3,5-Dimethyl-4-chloro-1-oxypyridin-2-yl |
| 772 | | Br | i-pr | $CH_2$ | 3,5-Dimethyl-4-iodopyridin-2-yl |
| 773 | | Br | i-pr | $CH_2$ | 3,5-Dimethyl-4-iodo-1-oxypyridin-2-yl |
| 774 | | Br | i-pr | $CH_2$ | 3,5-Dimethyl-4-thiomethyl-pyridin-2-yl |
| 775 | | Br | i-pr | $CH_2$ | 3,5-Dimethyl-4-thiomethyl-1-oxypyridin-2-yl |
| 776 | | Br | i-pr | $CH_2$ | 3,4,5-Trimethyl-pyridin-2-yl |
| 777 | | Br | i-pr | $CH_2$ | 3,4,5-Trimethyl-1-oxypyridin-2-yl |
| 778 | | Br | i-pr | $CH_2$ | 4,5,6-Trimethoxypyridin-2-yl |
| 779 | | Br | i-pr | $CH_2$ | 4,5,6-Trimethoxy-1-oxypyridin-2-yl |
| 780 | | Br | i-pr | $CH_2$ | 3-Bromo-4,5,6-trimethoxypyridin-2-yl |
| 781 | | Br | i-pr | $CH_2$ | 3-Chloro-4,5,6-trimethoxypyridin-2-yl |
| 782 | | Br | i-pr | $CH_2$ | 3,4,5-Trimethoxy-pyridin-2-yl |
| 783 | | Br | i-pr | $CH_2$ | 3,4,5-Trimethoxy-1-oxypyridin-2-yl |
| 784 | | Br | i-pr | $CH_2$ | 3-Bromo-3,4,5-trimethoxy-pyridin-2-yl |
| 785 | | Br | i-pr | $CH_2$ | 3-Chloro-3,4,5-trimethoxy-pyridin-2-yl |
| 786 | | Br | i-pr | $CH_2$ | 4,5,6-Trimethyl-pyridin-2-yl |
| 787 | | Br | i-pr | $CH_2$ | 4,5,6-Trimethyl-1-oxypyridin-2-yl |
| 788 | | Br | i-pr | $CH_2$ | 4,6-Dimethyl-5-methoxy-pyridin-2-yl |
| 789 | | Br | i-pr | $CH_2$ | 4,6-Dimethyl-5-methoxypyridin-3-yl |
| 790 | | Br | i-pr | $CH_2$ | 4,6-Dimethyl-5-methoxy-1-oxypyridin-3-yl |
| 791 | | Br | i-pr | $CH_2$ | 4,6-Dimethyl-5-bromopyridin-3-yl |
| 792 | | Br | i-pr | $CH_2$ | 4,6-Dimethyl-5-chloropyridin-3-yl |
| 793 | | Br | i-pr | $CH_2$ | 5,6-Dimethyl-4-bromopyridin-3-yl |
| 794 | | Br | i-pr | $CH_2$ | 5,6-Dimethyl-4-chloropyridin-3-yl |
| 795 | | Br | i-pr | $CH_2$ | 2,6-Dimethyl-3-methoxypyridin-4-yl |
| 796 | | Br | i-pr | $CH_2$ | 2,6-Dimethyl-pyridin-4-yl |
| 797 | | Br | i-pr | $CH_2$ | 2,3,6-Trimethyl-pyridin-4-yl |
| 798 | | Br | i-pr | $CH_2$ | 2,3,6-Trimethoxy-pyridin-4-yl |
| 799 | | Br | i-pr | $CH_2$ | 2,6-Dimethyl-3-bromopyridin-4-yl |

(continued)

| No. | Ex | $R^1$ | $R^3$ | $R^4$ | $R^5$ |
|---|---|---|---|---|---|
| 800 | | Br | i-pr | $CH_2$ | 2,6-Dimethyl-3-chloropyridin-4-yl |
| 801 | | Br | i-pr | $CH_2$ | 2,6-Dimethyl-3-methoxy-1-oxy-pyridin-4-yl |
| 802 | | Br | i-pr | $CH_2$ | 2,6-Dimethyl-1-oxy-pyridin-4-yl |
| 803 | | Br | i-pr | $CH_2$ | 2,3,6-Trimethyl-1-oxy-pyridin-4-yl |
| 804 | | Br | i-pr | $CH_2$ | 2,3,6-Trimethoxy-1-oxy-pyridin-4-yl |
| 805 | | Br | i-pr | $CH_2$ | 2,6-Dimethyl-3-bromol-oxy-pyridin-4-yl |
| 806 | | Br | i-pr | $CH_2$ | 2,6-Dimethyl-3-chlorol-oxy-pyridin-4-yl |
| 807 | | Br | i-pr | $CH_2$ | 4,6-Dimethyl-5-iodopyridin-3-yl |
| 808 | | Br | i-pr | $CH_2$ | 3,5-Dimethyl-4-aminopyridin-2-yl |
| 809 | | Br | Ph | $CH_2$ | 3,5-Dimethyl-4-methoxypyridin-2-yl |
| 810 | | Br | Ph | $CH_2$ | 3,5-Dimethyl-4-methoxy-1-oxypyridin-2-yl |
| 811 | | Br | Ph | $CH_2$ | 3,5-Dimethyl-4-bromopyridin-2-yl |
| 812 | | Br | Ph | $CH_2$ | 3,5-Dimethyl-4-bromo-1-oxypyridin-2-yl |
| 813 | | Br | Ph | $CH_2$ | 3,5-Dimethyl-4-chloropyridin-2-yl |
| 814 | | Br | Ph | $CH_2$ | 3,5-Dimethyl-4-chloro-1-oxypyridin-2-yl |
| 815 | | Br | Ph | $CH_2$ | 3,5-Dimethyl-4-iodopyridin-2-yl |
| 816 | | Br | Ph | $CH_2$ | 3,5-Dimethyl-4-iodo-1-oxypyridin-2-yl |
| 817 | | Br | Ph | $CH_2$ | 3,5-Dimethyl-4-thiomethyl-pyridin-2-yl |
| 818 | | Br | Ph | $CH_2$ | 3,5-Dimethyl-4-thiomethyl-1-oxypyridin-2-yl |
| 819 | | Br | Ph | $CH_2$ | 3-Bromo-4,5,6-trimethoxypyridin-2-yl |
| 820 | | Br | Ph | $CH_2$ | 3-Chloro-4,5,6-trimethoxypyridin-2-yl |
| 821 | | Br | Ph | $CH_2$ | 4,6-Dimethyl-5-methoxypyridin-3-yl |
| 822 | | Br | Ph | $CH_2$ | 4,6-Dimethyl-5-methoxy-1-oxypyridin-3-yl |
| 823 | | Br | Et | $CH_2$ | 3,5-Dimethyl-4-methoxypyridin-2-yl |
| 824 | | Br | Et | $CH_2$ | 3,5-Dimethyl-4-methoxy-1-oxypyridin-2-yl |
| 825 | | Br | Et | $CH_2$ | 3,5-Dimethyl-4-bromopyridin-2-yl |
| 826 | | Br | Et | $CH_2$ | 3,5-Dimethyl-4-bromo-1-oxypyridin-2-yl |
| 827 | | Br | Et | $CH_2$ | 3,5-Dimethyl-4-chloropyridin-2-yl |
| 828 | | Br | Et | $CH_2$ | 3,5-Dimethyl-4-chloro-1-oxypyridin-2-yl |
| 829 | | Br | Et | $CH_2$ | 3,5-Dimethyl-4-iodopyridin-2-yl |
| 830 | | Br | Et | $CH_2$ | 3,5-Dimethyl-4-iodo-1-oxypyridin-2-yl |
| 831 | | Br | Et | $CH_2$ | 3,5-Dimethyl-4-thiomethyl-pyridin-2-yl |
| 832 | | Br | Et | $CH_2$ | 3,5-Dimethyl4-thiomethyl-1-oxypyridin-2-yl |
| 833 | | Br | Et | $CH_2$ | 3-Bromo-4,5,6-trimethoxypyridin-2-yl |
| 834 | | Br | Et | $CH_2$ | 3-Chloro-4,5,6-trimethoxypyridin-2-yl |
| 835 | | Br | Et | $CH_2$ | 4,6-Dimethyl-5-methoxypyridin-3-yl |
| 836 | | Br | Et | $CH_2$ | 4,6-Dimethyl-5-methoxy-1-oxypyridin-3-yl |
| 837 | | Br | Me | $CH_2$ | 3,5-Dimethyl-4-methoxypyridin-2-yl |

(continued)

| No. | Ex | $R^1$ | $R^3$ | $R^4$ | $R^5$ |
|---|---|---|---|---|---|
| 838 | | Br | Me | $CH_2$ | 3,5-Dimethyl-4-methoxy-1-oxypyidin-2-yl |
| 839 | | Br | Me | $CH_2$ | 3,5-Dimethyl-4-bromopyridin-2-yl |
| 840 | | Br | Me | $CH_2$ | 3,5-Dimethyl-4-bromo-1-oxypyridin-2-yl |
| 841 | | Br | Me | $CH_2$ | 3,5-Dimethyl-4-chloropyridin-2-yl |
| 842 | | Br | Me | $CH_2$ | 3,5-Dimethyl-4-chloro-1-oxypyridin-2-yl |
| 843 | | Br | Me | $CH_2$ | 3,5-Dimethyl-4-iodopyridin-2-yl |
| 844 | | Br | Me | $CH_2$ | 3,5-Dimethyl-4-iodo-1-oxypyridin-2-yl |
| 845 | | Br | Me | $CH_2$ | 3,5-Dimethyl-4-thiomethyl-pyridin-2-yl |
| 846 | | Br | Me | $CH_2$ | 3,5-Dimethyl-4-thiomethyl-1-oxypyridin-2-yl |
| 847 | | Br | Me | $CH_2$ | 3-Bromo-4,5,6-trimethoxypyridin-2-yl |
| 848 | | Br | Me | $CH_2$ | 3-Chloro-4,5,6-trimethoxypyridin-2-yl |
| 849 | | Br | Me | $CH_2$ | 4,6-Dimethyl-5-methoxypyridin-3-yl |
| 850 | | Br | Me | $CH_2$ | 4,6-Dimethyl-5-methoxy-1-oxypyridin-3-yl |
| 851 | | Br | 2-Py | $CH_2$ | 3,5-Dimethyl4-methoxypyridin-2-yl |
| 852 | | Br | 2-Py | $CH_2$ | 3,5-Dimethyl-4-methoxy-1-oxypyridin-2-yl |
| 853 | | Br | 2-Py | $CH_2$ | 3,5-Dimethyl-4-bromopyridin-2-yl |
| 854 | | Br | 2-Py | $CH_2$ | 3,5-Dimethyl-4-bromo-1-oxypyridin-2-yl |
| 855 | | Br | 2-Py | $CH_2$ | 3,5-Dimethyl-4-chloropyridin-2-yl |
| 856 | | Br | 2-Py | $CH_2$ | 3,5-Dimethyl-4-chloro-1-oxypyridin-2-yl |
| 857 | | Br | 2-Py | $CH_2$ | 3,5-Dimethyl-4-iodopyridin-2-yl |
| 858 | | Br | 2-Py | $CH_2$ | 3,5-Dimethyl-4-iodo-1-oxypyridin-2-yl |
| 859 | | Br | 2-Py | $CH_2$ | 3,5-Dimethyl-4-thiomethyl-pyridin-2-yl |
| 860 | | Br | 2-Py | $CH_2$ | 3,5-Dimethyl-4-thiomethyl-1-oxypyridin-2-yl |
| 861 | | Br | 2-Py | $CH_2$ | 3-Bromo-4,5,6-trimethoxypyridin-2-yl |
| 862 | | Br | 2-Py | $CH_2$ | 3-Chloro-4,5,6-trimethoxypyridin-2-yl |
| 863 | | Br | 2-Py | $CH_2$ | 4,6-Dimethyl-5-methoxypyridin-3-yl |
| 864 | | Br | 2-Py | $CH_2$ | 4,6-Dimethyl-5-methoxy-1-oxypyridin-3-yl |
| 865 | | Br | 3-Py | $CH_2$ | 3,5-Dimethyl-4-methoxypyridin-2-yl |
| 866 | | Br | 3-Py | $CH_2$ | 3,5-Dimethyl-4-methoxy-1-oxypyridin-2-yl |
| 867 | | Br | 3-Py | $CH_2$ | 3,5-Dimethyl-4-bromopyridin-2-yl |
| 868 | | Br | 3-Py | $CH_2$ | 3,5-Dimethyl-4-bromo-1-oxypyridin-2-yl |
| 869 | | Br | 3-Py | $CH_2$ | 3,5-Dimethyl-4-chloropyridin-2-yl |
| 870 | | Br | 3-Py | $CH_2$ | 3,5-Dimethyl-4-chloro-1-oxypyridin-2-yl |
| 871 | | Br | 3-Py | $CH_2$ | 3,5-Dimethyl-4-iodopyridin-2-yl |
| 872 | | Br | 3-Py | $CH_2$ | 3,5-Dhnethyl-4-iodo-1-oxypyridin-2-yl |
| 873 | | Br | 3-Py | $CH_2$ | 3,5-Dimethyl-4-thiomethyl-pyridin-2-yl |
| 874 | | Br | 3-Py | $CH_2$ | 3,5-Dimethyl-4-thiomethyl-1-oxypyridin-2-yl |
| 875 | | Br | 3-Py | $CH_2$ | 3-Bromo-4,5,6-trimethoxypyridin-2-yl |

(continued)

| No. | Ex | $R^1$ | $R^3$ | $R^4$ | $R^5$ |
|---|---|---|---|---|---|
| 876 | | Br | 3-Py | $CH_2$ | 3-Chloro-4,5,6-trimethoxypyridin-2-yl |
| 877 | | Br | 3-Py | $CH_2$ | 4,6-Dimethyl-5-methoxypyridin-3-yl |
| 878 | | Br | 3-Py | $CH_2$ | 4,6-Dimethyl-5-methoxy-1-oxypyridin-3-yl |

Compounds of interest in Table 3 are: 1, 2, 3 16, 17, 18, 27, 28, 47, 48, 62, 63, 77, 78, 92, 93, 97, 98, 129, 130, 242, 243, 245, 246, 247, 248, 249, 250, 251, 252, 253, 267, 268, 287, 288, 289, 290, 291, 292, 293, 294, 295, 29d, 297, 298, 312, 313, 332, 333, 334, 335, 336, 337, 338, 339, 351, 352, 365, 366, 367, 368, 369, 370, 384, 385, 398, 399, 400, 401, 417, 418, 431, 432, 433, 434, 435, 436, 437, 438, 439, 440, 450, 451, 464, 465, 466, 466, 467, 468, 469, 470, 471, 483, 484, 497, 498, 530, 531, 550, 551, 563, 564, 575, 576, 578, 579, 590, 591, 593, 594, 605, 606, 608, 609, 620, 621, 721, 722, 725, 726, 727, 728, 729, 730, 746, 747, 766, 767, 791, 792, 811, 812, 823, 824, 825, 826, 839, 840, 853, 854, 867, and 868, and the more preferred compounds are 242, 243, 245, 246, 247, 248, 267, 268, 287, 288, 312, 313, 332, 333, 334, 335, 336, 337, 338, 339, 365, 369, 398, 417, 431, 432, 433, 434, 435, 436, 437, 438, 450, 451, 464, 465, 483, and 484.

**TABLE 4: EXEMPLARY COMPOUNDS OF FORMULA IV**

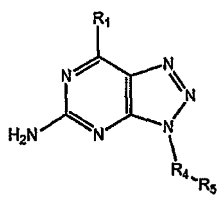

IV

| No. | Ex. | $R^1$ | $R^4$ | $R^5$ |
|---|---|---|---|---|
| 1 | 5 | Cl | $CH_2$ | 3,4,5-Trimethoxyphenyl |
| 2 | 6 | Cl | $CH_2$ | 2-Chloro-3,4,5-trimethoxyphenyl |
| 3 | 8 | Cl | $CH_2$ | 2-Bromo-3,4,5-trimethoxyphenyl |
| 4 | 10 | Cl | $CH_2$ | 2-Iodo-3,4,5-trimethoxyphenyl |
| 5 | | Cl | $CH_2$ | 2-Fluoro-3,4,5-trimethoxyphenyl |
| 6 | | Cl | $CH_2$ | 3,4,5-Trimethylphenyl |
| 7 | | Cl | $CH_2$ | 2-Chloro-3,4,5-trimethylphenyl |
| 8 | | Cl | $CH_2$ | 2-Bromo-3,4,5-trimethylphenyl |
| 9 | | Cl | $CH_2$ | 2-Iodo-3,4,5-trimethylphenyl |
| 10 | | Cl | $CH_2$ | 2-Fluoro-3,4,5-trimethylphenyl |
| 11 | | Cl | $CH_2$ | 3,5-Dimethoxy-4-methylphenyl |
| 12 | | Cl | $CH_2$ | 2-Chloro-3,5-dimethoxy-4-methylphenyl |
| 13 | | Cl | $CH_2$ | 2-Bromo-3,5-dimethoxy-4-methylphenyl |
| 14 | | Cl | $CH_2$ | 2-Iodo-3,5-dimethoxy-4-methylphenyl |
| 15 | | Cl | $CH_2$ | 2-Fluoro-3,5-dimethoxy-4-methylphenyl |
| 16 | | Cl | $CH_2$ | 3,5-Dichloro-4-methylphenyl |
| 17 | | Cl | $CH_2$ | 2,3,5-Trichloro-4-methylphenyl |
| 18 | | Cl | $CH_2$ | 2-Bromo-3,5-dichloro-4-methylphenyl |

(continued)

| No. | Ex. | $R^1$ | $R^4$ | $R^5$ |
|---|---|---|---|---|
| 19 | | Cl | $CH_2$ | 2-Iodo-3,5-dichloro-4-methylphenyl |
| 20 | | Cl | $CH_2$ | 2-Fluoro-3,5-dichloro-4-methylphenyl |
| 21 | | Cl | $CH_2$ | 3,5-Dibromo-4-methylphenyl |
| 22 | | Cl | $CH_2$ | 2-Chloro-3,5-dibromo-4-methylphenyl |
| 23 | | Cl | $CH_2$ | 2,3,5-Tribromo-4-methylphenylphenyl |
| 24 | | Cl | $CH_2$ | 2-Iodo-3,5-dibromo-4-methylphenyl |
| 25 | | Cl | $CH_2$ | 2-Fluoro-3,5-dibromo-4-methylphenyl |
| 26 | | Cl | $CH_2$ | 3,5-Dichloro-4-methoxyphenyl |
| 27 | | Cl | $CH_2$ | 2,3,5-Trichloxo-4-methoxyphenyl |
| 28 | | Cl | $CH_2$ | 2-Bromo-3,5-dichloro-4-methoxyphenyl |
| 29 | | Cl | $CH_2$ | 2-Iodo-3,5-dichloro-4-methoxyphenyl |
| 30 | | Cl | $CH_2$ | 2-Fluoro-3,5-dichloro-4-methoxyphenyl |
| 31 | | Cl | $CH_2$ | 3,5-Dibromo-4-methoxyphenyl |
| 32 | | Cl | $CH_2$ | 2-Chloro-3,5-dibromo-4-methoxyphenyl |
| 33 | | Cl | $CH_2$ | 2,3,5-Tribromo-4-methoxyphenyl |
| 34 | | Cl | $CH_2$ | 2-Iodo-3,5-dibromo-4-methoxyphenyl |
| 35 | | Cl | $CH_2$ | 2-Fluoro-3,5-dibromo-4-methoxyphenyl |
| 36 | | Cl | $CH_2$ | 3-Chloro-5-bromo-4-methylphenyl |
| 37 | | Cl | $CH_2$ | 2,3-Dichloro-5-bromo-4-methylphenyl |
| 38 | | Cl | $CH_2$ | 2,5-Dibromo-3-chloro-4-methylphenyl |
| 39 | | Cl | $CH_2$ | 2-Iodo-3-chloro-5-bromo-4-methylphenyl |
| 40 | | Cl | $CH_2$ | 2-Fluoro-3-chloro-5-bromo-4-methylphenyl |
| 41 | | Cl | $CH_2$ | 3-Chloro-5-bromo-4-methoxyphenylphenyl |
| 42 | | Cl | $CH_2$ | 2,3-Dichloro-5-bromo-4-methoxyphenyl |
| 43 | | Cl | $CH_2$ | 2,5-Dibromo-3-chloro-4-methoxyphenyl |
| 44 | | Cl | $CH_2$ | 2-Iodo-3-chloro-5-bromo-4-methoxyphenyl |
| 45 | | Cl | $CH_2$ | 2-Fluoro-3-chloro-5-bromo-4-methoxyphenyl |
| 46 | | Cl | $CH_2$ | 3-Bromo-5-chloro-4-methylphenyl |
| 47 | | Cl | $CH_2$ | 2,5-Dichloro-3-bromo-4-methylphenyl |
| 48 | | Cl | $CH_2$ | 2,3-Dibromo-5-chloro-4-methylphenyl |
| 49 | | Cl | $CH_2$ | 2-Iodo-3-bromo-5-chloro-4-methylphenyl |
| 50 | | Cl | $CH_2$ | 2-Fluoro-3-bromo-5-chloro-4-methylphenyl |
| 51 | | Cl | $CH_2$ | 3-Bromo-5-chloro-4-methoxyphenyl |
| 52 | | Cl | $CH_2$ | 2,5-Dichloro-3-bromo-4-methoxyphenyl |
| 53 | | Cl | $CH_2$ | 2,3-Dibromo-5-chloro-4-methoxyphenyl |
| 54 | | Cl | $CH_2$ | 2-Iodo-3-bromo-5-chloro-4-methoxyphenyl |
| 55 | | Cl | $CH_2$ | 2-Fluoro-3-bromo-5-chloro-4-methoxyphenyl |
| 56 | | Cl | $CH_2$ | 3,5-Dimethoxy-4-trifluoromethylphenyl |

(continued)

| No. | Ex. | $R^1$ | $R^4$ | $R^5$ |
|---|---|---|---|---|
| 57 | | Cl | $CH_2$ | 2-Chloro-3,5-dimethoxy-4-trifluoromethylphenyl |
| 58 | | Cl | $CH_2$ | 2-Bromo-3,5-dimethoxy-4- trifluoromethylphenyl |
| 59 | | Cl | $CH_2$ | 2-Iodo-3,5-dimethoxy-4- trifluoromethylphenyl |
| 60 | | Cl | $CH_2$ | 2-Fluoro-3,5-dimethoxy-4- trifluoromethylphenyl |
| 61 | | Cl | $CH_2$ | 3,5-dibromo-4- trifluoromethoxyphenyl |
| 62 | | Cl | $CH_2$ | 2-Chloro-3,5-dibromo-4-trifluoromethoxyphenyl |
| 63 | | Cl | $CH_2$ | 2,3,5-Tribromo-4- trifluoromethoxyphenyl |
| 64 | | Cl | $CH_2$ | 2-Iodo-3,5-dibromo-4- trifluoromethoxyphenyl |
| 65 | | Cl | $CH_2$ | 2-Fluoro-3,5-dibromo-4- trifluoromethoxyphenyl |
| 66 | | Cl | $CH_2$ | 3,5-Dimethyl-4-methoxyphenyl |
| 67 | | Cl | $CH_2$ | 2-Chloro-3,5-dimethyl-4-methoxyphenyl |
| 68 | | Cl | $CH_2$ | 2-Bromo-3,5-dimethyl-4-methoxyphenyl |
| 69 | | Cl | $CH_2$ | 2-Iodo-3,5-dimethyl-4-methoxyphenyl |
| 70 | | Cl | $CH_2$ | 2-Fluoro-3,5-dimethyl-4-methoxyphenyl |
| 71 | | Cl | $CH_2$ | 3,5-Dimethyl-4-bromophenyl |
| 72 | | Cl | $CH_2$ | 2-Chloro-3,5-dimethyl-4-bromophenyl |
| 73 | | Cl | $CH_2$ | 2,4-Dibromo-3,5-dimethylphenyl |
| 74 | | Cl | $CH_2$ | 2-Iodo-3,5-dimethyl-4-bromophenyl |
| 75 | | Cl | $CH_2$ | 2-Fluoro-3,5-dimethyl-4-bromophenyl |
| 76 | | Cl | $CH_2$ | 3,5-Dimethyl-4-chlorophenyl |
| 77 | | Cl | $CH_2$ | 2,4-Dichloro-3,5-dimethylphenyl |
| 78 | | Cl | $CH_2$ | 2-Bromo-3,5-dimethyl-4-chlorophenyl |
| 79 | | Cl | $CH_2$ | 2-Iodo-3,5-dimethyl-4-chlorophenyl |
| 80 | | Cl | $CH_2$ | 2-Fluoro-3,5-dimethyl-4-chlorophenyl |
| 81 | | Br | $CH_2$ | 3,4,5-Trimethoxyphenyl |
| 82 | | Br | $CH_2$ | 2-Chloro-3,4,5-trimethoxyphenyl |
| 83 | | Br | $CH_2$ | 2-Bromo-3,4,5-trimethoxyphenyl |
| 84 | | Br | $CH_2$ | 2-Iodo-3,4,5-trimethoxyphenyl |
| 85 | | Br | $CH_2$ | 2-Fluoro-3,4,5-trimethoxyphenyl |
| 86 | | Br | $CH_2$ | 3,4,5-Trimethylphenyl |
| 87 | | Br | $CH_2$ | 2-Chloro-3,4,5-trimethylphenyl |
| 88 | | Br | $CH_2$ | 2-Bromo-3,4,5-trimethylphenyl |
| 89 | | Br | $CH_2$ | 2-Iodo-3,4,5-trimethylphenyl |
| 90 | | Br | $CH_2$ | 2-Fluoro-3,4,5-trimethylphenyl |
| 91 | | Br | $CH_2$ | 3,5-Dimethoxy-4-methylphenyl |
| 92 | | Br | $CH_2$ | 2-Chloro-3,5-dimethoxy-4-methylphenyl |
| 93 | | Br | $CH_2$ | 2-Bromo-3,5-dimethoxy-4-methylphenyl |
| 94 | | Br | $CH_2$ | 2-Iodo-3,5-dimethoxy-4-methylphenyl |

(continued)

| No. | Ex. | $R^1$ | $R^4$ | $R^5$ |
|---|---|---|---|---|
| 95 | | Br | $CH_2$ | 2-Fluoro-3,5-dimethoxy-4-methylphenyl |
| 96 | | Br | $CH_2$ | 3,5-Dichloro-4-methylphenyl |
| 97 | | Br | $CH_2$ | 2,3,5-Trichloro-4-methylphenyl |
| 98 | | Br | $CH_2$ | 2-Bromo-3,5-dichloro-4-methylphenyl |
| 99 | | Br | $CH_2$ | 2-Iodo-3,5-dichloro-4-methylphenyl |
| 100 | | Br | $CH_2$ | 2-Fluoro-3,5-dichloro-4-methylphenyl |
| 101 | | Br | $CH_2$ | 3,5-Dibromo-4-methylphenyl |
| 102 | | Br | $CH_2$ | 2-Chloro-3,5-dibromo-4-methylphenyl |
| 103 | | Br | $CH_2$ | 2,3,5-Tribromo-4-methylphenylphenyl |
| 104 | | Br | $CH_2$ | 2-Iodo-3,5-dibromo-4-methylphenyl |
| 105 | | Br | $CH_2$ | 2-Fluoro-3,5-dibromo-4-methylphenyl |
| 106 | | Br | $CH_2$ | 3,5-Dichloro-4-methoxyphenyl |
| 107 | | Br | $CH_2$ | 2,3,5-Trichloro-4-methoxyphenyl |
| 108 | | Br | $CH_2$ | 2-Bromo-3,5-dichloro-4-methoxyphenyl |
| 109 | | Br | $CH_2$ | 2-Iodo-3,5-dichloro-4-methoxyphenyl |
| 110 | | Br | $CH_2$ | 2-Fluoro-3,5-dichloro-4-methoxyphenyl |
| 111 | | Br | $CH_2$ | 3,5-Dibromo-4-methoxyphenyl |
| 112 | | Br | $CH_2$ | 2-Chloro-3,5-dibromo-4-methoxyphenyl |
| 113 | | Br | $CH_2$ | 2,3,5-Tribromo-4-methoxyphenyl |
| 114 | | Br | $CH_2$ | 2-Iodo-3,5-dibromo-4-methoxyphenyl |
| 115 | | Br | $CH_2$ | 2-Fluoro-3,5-dibromo-4-methoxyphenyl |
| 116 | | Br | $CH_2$ | 3-Chloro-5-bromo-4-methylphenyl |
| 117 | | Br | $CH_2$ | 2,3-Dichloro-5-bromo-4-methylphenyl |
| 118 | | Br | $CH_2$ | 2,5-Dibromo-3-chloro-4-methylphenyl |
| 119 | | Br | $CH_2$ | 2-Iodo-3-chloro-5-bromo-4-methylphenyl |
| 120 | | Br | $CH_2$ | 2-Fluoro-3-chloro-5-bromo-4-methylphenyl |
| 121 | | Br | $CH_2$ | 3-Chloro-5-bromo-4-methoxyphenylphenyl |
| 122 | | Br | $CH_2$ | 2,3-Dichloro-5-bromo-4-methoxyphenyl |
| 123 | | Br | $CH_2$ | 2,5-Dibromo-3-chloro-4-methoxyphenyl |
| 124 | | Br | $CH_2$ | 2-Iodo-3-chloro-5-bromo-4-methoxyphenyl |
| 125 | | Br | $CH_2$ | 2-Fluoro-3-chloro-5-bromo-4-methoxyphenyl |
| 126 | | Br | $CH_2$ | 3-Bromo-5-chloro-4-methylphenyl |
| 127 | | Br | $CH_2$ | 2,5-Dichloro-3-bromo-4-methylphenyl |
| 128 | | Br | $CH_2$ | 2,3-Dibromo-5-chloro-4-methylphenyl |
| 129 | | Br | $CH_2$ | 2-Iodo-3-bromo-5-chloro-4-methylphenyl |
| 130 | | Br | $CH_2$ | 2-Fluoro-3-bromo-5-chloro-4-methylphenyl |
| 131 | | Br | $CH_2$ | 3-Bromo-5-chloro-4-methoxyphenyl |
| 132 | | Br | $CH_2$ | 2,5-Dichloro-3-bromo-4-methoxyphenyl |

(continued)

| No. | Ex. | $R^1$ | $R^4$ | $R^5$ |
|---|---|---|---|---|
| 133 | | Br | $CH_2$ | 2,3-Dibromo-5-chloro-4-methoxyphenyl |
| 134 | | Br | $CH_2$ | 2-Iodo-3-bromo-5-chloro-4-methoxyphenyl |
| 135 | | Br | $CH_2$ | 2-Fluoro-3-bromo-5-chloro-4-methoxyphenyl |
| 136 | | Br | $CH_2$ | 3,5-Dimethoxy-4-trifluoromethylphenyl |
| 137 | | Br | $CH_2$ | 2-Chloro-3,5-dimethoxy-4- trifluoromethylphenyl |
| 138 | | Br | $CH_2$ | 2-Bromo-3,5-dimethoxy-4- trifluoromethylphenyl |
| 139 | | Br | $CH_2$ | 2-Iodo-3,5-dimethoxy-4- trifluoromethylphenyl |
| 140 | | Br | $CH_2$ | 2-Fluoro-3,5-dimethoxy-4- trifluoromethylphenyl |
| 141 | | Br | $CH_2$ | 3,5-Dibromo-4- trifluoromethoxyphenyl |
| 142 | | Br | $CH_2$ | 2-Chloro-3,5-dibromo-4-trifluoromethoxyphenyl |
| 143 | | Br | $CH_2$ | 2,3,5-Tribromo-4- trifluoromethoxyphenyl |
| 144 | | Br | $CH_2$ | 2-Iodo-3,5-dibromo-4- trifluoromethoxyphenyl |
| 145 | | Br | $CH_2$ | 2-Fluoro-3,5-dibromo-4- trifluoromethoxyphenyl |
| 146 | | Br | $CH_2$ | 3,5-Dimethyl-4-methoxyphenyl |
| 147 | | Br | $CH_2$ | 2-Chloro-3,5-dimethyl-4-methoxyphenyl |
| 148 | | Br | $CH_2$ | 2-Bromo-3,5-dimethyl-4-methoxyphenyl |
| 149 | | Br | $CH_2$ | 2-Iodo-3,5-dimethyl-4-methoxyphenyl |
| 150 | | Br | $CH_2$ | 2-Fluoro-3,5-dimethyl-4-methoxyphenyl |
| 151 | | Br | $CH_2$ | 3,5-Dimethyl-4-bromophenyl |
| 152 | | Br | $CH_2$ | 2-Chloro-3,5-dimethyl-4-bromophenyl |
| 153 | | Br | $CH_2$ | 2,4-Dibromo-3,5-dimethylphenyl |
| 154 | | Br | $CH_2$ | 2-Iodo-3,5-dimethyl-4-bromophenyl |
| 155 | | Br | $CH_2$ | 2-Fluoro-3,5-dimethyl-4-bromophenyl |
| 156 | | Br | $CH_2$ | 3,5-Dimethyl-4-chlorophenyl |
| 157 | | Br | $CH_2$ | 2,4-Dichloro-3,5-dimethylphenyl |
| 158 | | Br | $CH_2$ | 2-Bromo-3,5-dimethyl-4-chlorophenyl |
| 159 | | Br | $CH_2$ | 2-Iodo-3,5-dimethyl-4-chlorophenyl |
| 160 | 1 | Cl | $CH_2$ | 3,5-Dimethyl-4-methoxypyridin-2-yl |
| 161 | 2 | Cl | $CH_2$ | 3,5-Dimethyl-4-methoxy-1-oxypyridin-2-yl |
| 162 | | Cl | $CH_2$ | 6-Bromo-3,5-dimethyl-4-methoxypyridin-2-yl |
| 163 | | Cl | $CH_2$ | 6-Chloro-3,5-dimethyl-4-methoxypyridin-2-yl |
| 164 | | Cl | $CH_2$ | 6-Chloro-3,5-dimethyl-4-methoxy-1-oxypyridin-2-yl |
| 165 | | Cl | $CH_2$ | 6-Bromo-3,5-dimethyl-4-methoxy-1-oxypyridin-2-yl |
| 166 | | Cl | $CH_2$ | 3,5-Dimethyl-4-bromopyridin-2-yl |
| 167 | | Cl | $CH_2$ | 3,5-Dimethyl-4-bromo-1-oxypyridin-2-yl |
| 168 | | Cl | $CH_2$ | 6-Bromo-3,5-dimethyl-4-bromopyidin-2-yl |
| 169 | | Cl | $CH_2$ | 6-Chloro-3,5-dimethyl-4-bromopyridin-2-yl |
| 170 | | Cl | $CH_2$ | 6-Chloro-3,5-dimethyl-4-bromo-1-oxypyridin-2-yl |

(continued)

| No. | Ex. | $R^1$ | $R^4$ | $R^5$ |
|---|---|---|---|---|
| 171 | | Cl | $CH_2$ | 4,6-Dibromo-3,5-dimethyl-1-oxypyridin-2-yl |
| 172 | | Cl | $CH_2$ | 3,5-Dimethyl-4-chloropyridin-2-yl |
| 173 | | Cl | $CH_2$ | 3,5-Dimethyl-4-chloro-1-oxypyridin-2-yl |
| 174 | | Cl | $CH_2$ | 6-Bromo-3,5-dimethyl-4-chloropyridin-2-yl |
| 175 | | Cl | $CH_2$ | 6-Chloro-3,5-dimethyl-4-chloropyridin-2-yl |
| 176 | | Cl | $CH_2$ | 4,6-Dichloro-3,5-dimethyl-1-oxypyridin-2-yl |
| 177 | | Cl | $CH_2$ | 6-Bromo-3,5-dimethyl-4-chloro-1-oxypyridin-2-yl |
| 178 | | Cl | $CH_2$ | 3,5-Dimethyl-4-iodopyridin-2-yl |
| 179 | | Cl | $CH_2$ | 3,5-Dimethyl-4-iodo-1-oxypyridin-2-yl |
| 180 | | Cl | $CH_2$ | 6-Bromo-3,5-dimethyl-4-iodopyridin-2-yl |
| 181 | | Cl | $CH_2$ | 6-Chloro-3,5-dimethyl-4-iodopyridin-2-yl |
| 182 | | Cl | $CH_2$ | 6-Chloro-3,5-dimethyl-4-iodo-1-oxypyridin-2-yl |
| 183 | | Cl | $CH_2$ | 6-Bromo-3,5-dimethyl-4-iodo-1-oxypyridin-2-yl |
| 184 | | Cl | $CH_2$ | 3,5-Dimethyl-4-thiomethyl-pyridin-2-yl |
| 185 | | Cl | $CH_2$ | 3,5-Dimethyl-4-thiomethyl-1-oxypyridin-2-yl |
| 186 | | Cl | $CH_2$ | 6-Bromo-3,5-dimethyl-4-thiomethyl-pyridin-2-yl |
| 187 | | Cl | $CH_2$ | 6-Chloro-3,5-dimethyl-4-thiomethyl-pyridin-2-yl |
| 188 | | Cl | $CH_2$ | 6-Chloro-3,5-dimethyl-4-thiomethyl-1-oxypyridin-2-yl |
| 189 | | Cl | $CH_2$ | 6-Bromo-3,5-dimethyl-4-thiomethyl-1-oxypyridin-2-yl |
| 190 | | Cl | $CH_2$ | 3,4,5-Trimethyl-pyridin-2-yl |
| 191 | | Cl | $CH_2$ | 3,4,5-Trimethyl-1-oxypyridin-2-yl |
| 192 | | Cl | $CH_2$ | 6-Bromo-3,4,5-trimethyl-pyridin-2-yl |
| 193 | | Cl | $CH_2$ | 6-Chloro-3,4,5-trimethyl-pyridin-2-yl |
| 194 | | Cl | $CH_2$ | 6-Chloro-3,4,5-trimethyl-1-oxypyridin-2-yl |
| 195 | | Cl | $CH_2$ | 6-Bromo-3,4,5-trimethyl-1-oxypyridin-2-yl |
| 196 | | Cl | $CH_2$ | 4,5,6-Trimethoxypyridin-2-yl |
| 197 | | Cl | $CH_2$ | 4,5,6-Trimethoxy-1-oxypyridin-2-yl |
| 198 | | Cl | $CH_2$ | 3-Bromo-4,5,6-trimethoxypyridin-2-yl |
| 199 | | Cl | $CH_2$ | 3-Chloro-4,5,6-trimethoxypyridin-2-yl |
| 200 | | Cl | $CH_2$ | 3-Chloro-4,5,6-trimethoxy-1-oxypyridin-2-yl |
| 201 | | Cl | $CH_2$ | 3-Bromo-4,5,6-trimethoxy-1-oxypyridin-2-yl |
| 202 | | Cl | $CH_2$ | 3,4,5-Trimethoxy-pyridin-2-yl |
| 203 | | Cl | $CH_2$ | 3,4,5-Trimethoxy-1-oxypyridin-2-yl |
| 204 | | Cl | $CH_2$ | 3-Bromo-3,4,5-trimethoxy-pyridin-2-yl |
| 205 | | Cl | $CH_2$ | 3-Chloro-3,4,5-trimethoxy-pyridin-2-yl |
| 206 | | Cl | $CH_2$ | 3-Chloro-3,4,5-trimethoxy-1-oxypyridin-2-yl |
| 207 | | Cl | $CH_2$ | 3-Bromo-3,4,5-trimethoxy-1-oxypyridin-2-yl |
| 208 | | Cl | $CH_2$ | 4,5,6-Trimethyl-pyridin-2-yl |

(continued)

| No. | Ex. | $R^1$ | $R^4$ | $R^5$ |
|---|---|---|---|---|
| 209 | | Cl | $CH_2$ | 4,5,6-Trimethyl-1-oxypyridin-2-yl |
| 210 | | Cl | $CH_2$ | 3-Bromo-4,5,6-trimethyl-pyridin-2-yl |
| 211 | | Cl | $CH_2$ | 3-Chloro-4,5,6-trimethyl-pyridin-2-yl |
| 212 | | Cl | $CH_2$ | 3-Chloro-4,5,6-trimethyl-1-oxypyridin-2-yl |
| 213 | | Cl | $CH_2$ | 3-Bromo-4,5,6-trimethyl-1-oxypyridin-2-yl |
| 214 | | Cl | $CH_2$ | 4,6-Dimethyl-5-methoxy-pyridin-2-yl |
| 215 | | Cl | $CH_2$ | 4,6-Dimethyl-5-methoxy-1-oxypyridin-2-yl |
| 216 | | Cl | $CH_2$ | 3-Bromo-4,6-dimethyl-5-methoxy-pyridin-2-yl |
| 217 | | Cl | $CH_2$ | 3-Chloro-4,6-dimethyl-5-methoxy-pyridin-2-yl |
| 218 | | Cl | $CH_2$ | 3-Chloro-4,6-dimethyl-5-methoxy-1-oxypyridin-2-yl |
| 219 | | Cl | $CH_2$ | 3-Bromo-4,6-dimethyl-5-methoxy-1-oxypyridin-2-yl |
| 220 | | Cl | $CH_2$ | 4-Bromo-5,6-dimethoxy-pyridin-2-yl |
| 221 | | Cl | $CH_2$ | 4-Bromo-5,6-dimethoxy-1-oxypyridin-2-yl |
| 222 | | Cl | $CH_2$ | 3,4-Dibromo-5,6-dimethoxy-pyridin-2-yl |
| 223 | | Cl | $CH_2$ | 3-Chloro-4-bromo-5,6-dimethoxy-pyridin-2-yl |
| 224 | | Cl | $CH_2$ | 3-Chloro-4-bromo-5,6-dimethoxy-1-oxypyridin-2-yl |
| 225 | | Cl | $CH_2$ | 3,4-Dibromo-5,6-dimethoxy-1-oxypyridin-2-yl |
| 226 | | Cl | $CH_2$ | 4,6-Dimethyl-5-methoxypyridin-3-yl |
| 227 | | Cl | $CH_2$ | 4,6-Dimethyl-5-methoxy-1-oxypyridin-3-yl |
| 228 | | Cl | $CH_2$ | 4,6-Dimethyl-5-bromopyridin-3-yl |
| 229 | | Cl | $CH_2$ | 4,6-Dimethyl-5-chloropyridin-3-yl |
| 230 | | Cl | $CH_2$ | 5,6-Dimethyl-4-bromopyridin-3-yl |
| 231 | | Cl | $CH_2$ | 5,6-Dimethyl-4-chloropyridin-3-yl |
| 232 | | Cl | $CH_2$ | 4,6-Dimethyl-5-bromo-1-oxypyridin-pyridin-3-yl |
| 233 | | Cl | $CH_2$ | 4,6-Dimethyl-5-chloro-1-oxypyridin-pyridin-3-yl |
| 234 | | Cl | $CH_2$ | 5,6-Dimethyl-4-bromo-1-oxypyridin-pyridin-3-yl |
| 235 | | Cl | $CH_2$ | 5,6-Dimethyl-4-chloro-1-oxypyridin-pyridin-3-yl |
| 236 | | Cl | $CH_2$ | 2,6-Dimethyl-3-methoxypyridin-4-yl |
| 237 | | Cl | $CH_2$ | 2,6-Dimethyl-pyridin-4-yl |
| 238 | | Cl | $CH_2$ | 2,3,6-Trimethyl-pyridin-4-yl |
| 239 | | Cl | $CH_2$ | 2,3,6-Trimethoxy-pyridin-4-yl |
| 240 | | Cl | $CH_2$ | 2,6-Dimethyl-3-bromopyridin-4-yl |
| 241 | | Cl | $CH_2$ | 2,6-Dimethyl-3-chloxopyridin-4-yl |
| 242 | | Cl | $CH_2$ | 2,6-Dichloro-3-bromopyridin-4-yl |
| 243 | | Cl | $CH_2$ | 2,6-Dibromo-3-chloropyridin-4-yl |
| 244 | | Cl | $CH_2$ | 2,3,6-Trichloro-pyridin-4-yl |
| 245 | | Cl | $CH_2$ | 2,3,6-Tribromo-pyridin-4-yl |
| 246 | | Cl | $CH_2$ | 2,6-Dimethyl-3-methoxy-1-oxy-pyridin-4-yl |

(continued)

| No. | Ex. | $R^1$ | $R^4$ | $R^5$ |
|---|---|---|---|---|
| 247 | | Cl | $CH_2$ | 2,6-Dimethyl-1-oxy-pyridin-4-yl |
| 248 | | Cl | $CH_2$ | 2,3,6-Trimethyl-1-oxy-pyridin-4-yl |
| 249 | | Cl | $CH_2$ | 2,3,6-Trimethoxy-1-oxy-pyridin-4-yl |
| 250 | | Cl | $CH_2$ | 2,6-Dimethyl-3-bromol-oxy-pyridin-4-yl |
| 251 | | Cl | $CH_2$ | 2,6-Dimethyl-3-chlorol-oxy-pyridin-4-yl |
| 252 | | Cl | $CH_2$ | 2,6-Dichloro-3-bromo-1-oxy-pyridin-4-yl |
| 253 | | Cl | $CH_2$ | 2,6-Dibromo-3-chloro-1-oxy-pyridin-4-yl |
| 254 | | Cl | $CH_2$ | 2,3,6-Trichloro-1-oxy-pyridin-4-yl |
| 255 | | Cl | $CH_2$ | 2,3,6-Tribromo-1-oxy-pyridin-4-yl |
| 256 | | Cl | $CH_2$ | 4,6-Dimethyl-5-iodopyridin-3-yl |
| 257 | | Cl | $CH_2$ | 5,6-Dimethyl-4-iodopyridin-3-yl |
| 258 | | Cl | $CH_2$ | 4,5,6-Trichloropyridin-3-yl |
| 259 | | Cl | $CH_2$ | 4,5,6-Tribromopyridin-3-yl |
| 260 | | Br | $CH_2$ | 3,5-Dimethyl-4-methoxypyridin-2-yl |
| 261 | | Br | $CH_2$ | 3,5-Dimethyl-4-methoxy-1-oxypyridin-2-yl |
| 262 | | Br | $CH_2$ | 6-Bromo-3,5-dimethyl-4-methoxypyridin-2-yl |
| 263 | | Br | $CH_2$ | 6-Chloro-3,5-dimethyl-4-methoxypyridin-2-yl |
| 264 | | Br | $CH_2$ | 6-Chloro-3,5-dimethyl-4-methoxy-1-oxypyridin-2-yl |
| 265 | | Br | $CH_2$ | 6-Bromo-3,5-dimethyl-4-methoxy-1-oxypyridin-2-yl |
| 266 | | Br | $CH_2$ | 3,5-Dimethyl-4-bromopyridin-2-yl |
| 267 | | Br | $CH_2$ | 3,5-Dimethyl-4-bromo-1-oxypyridin-2-yl |
| 268 | | Br | $CH_2$ | 6-Bromo-3,5-dimethyl-4-bromopyridin-2-yl |
| 269 | | Br | $CH_2$ | 6-Chloro-3,5-dimethyl-4-bromopyridin-2-yl |
| 270 | | Br | $CH_2$ | 6-Chloro-3,5-dimethyl-4-bromo-1-oxypyridin-2-yl |
| 271 | | Br | $CH_2$ | 4,6-Dibromo-3,5-dimethyl-1-oxypyridin-2-yl |
| 272 | | Br | $CH_2$ | 3,5-Dimethyl-4-chloropyridin-2-yl |
| 273 | | Br | $CH_2$ | 3,5-Dimethyl-4-chloro-1-oxypyridin-2-yl |
| 274 | | Br | $CH_2$ | 6-Bromo-3,5-dimethyl-4-chloropyridin-2-yl |
| 275 | | Br | $CH_2$ | 6-Chloro-3,5-dimethyl-4-chloropyridin-2-yl |
| 276 | | Br | $CH_2$ | 4,6-Dichloro-3,5-dimethyl-1-oxypyridin-2-yl |
| 277 | | Br | $CH_2$ | 6-Bromo-3,5-dimethyl-4-chloro-1-oxypyridin-2-yl |
| 278 | | Br | $CH_2$ | 3,5-Dimethyl-4-iodopyridin-2-yl |
| 279 | | Br | $CH_2$ | 3,5-Dimethyl-4-iodo-1-oxypyridin-2-yl |
| 280 | | Br | $CH_2$ | 6-Bromo-3,5-dimethyl-4-iodopyidin-2-yl |
| 281 | | Br | $CH_2$ | 6-Chloro-3,5-dimethyl-4-iodopyridin-2-yl |
| 282 | | Br | $CH_2$ | 6-Chloro-3,5-dimethyl-4-iodo-1-oxypyridin-2-yl |
| 283 | | Br | $CH_2$ | 6-Bromo-3,5-dimethyl-4-iodo-1-oxypyridin-2-yl |
| 284 | | Br | $CH_2$ | 3,5-Dimethyl-4-thiomethyl-pyridin-2-yl |

(continued)

| No. | Ex. | $R^1$ | $R^4$ | $R^5$ |
|---|---|---|---|---|
| 285 | | Br | $CH_2$ | 3,5-Dimethyl-4-thiomethyl-1-oxypyridin-2-yl |
| 286 | | Br | $CH_2$ | 6-Bromo-3,5-dimethyl-4-thiomethyl-pyridin-2-yl |
| 287 | | Br | $CH_2$ | 6-Chloro-3,5-dimethyl-4-thiomethyl-pyridin-2-yl |
| 288 | | Br | $CH_2$ | 6-Chloro-3,5-dimethyl-4-thiomethyl-1-oxypyridin-2-yl |
| 289 | | Br | $CH_2$ | 6-Bromo-3,5-dimethyl-4-thiomethyl-1-oxypyridin-2-yl |
| 290 | | Br | $CH_2$ | 3,4,5-Trimethyl-pyridin-2-yl |
| 291 | | Br | $CH_2$ | 3,4,5-Trimethyl-1-oxypyridin-2-yl |
| 292 | | Br | $CH_2$ | 6-Bromo-3,4,5-trimethyl-pyridin-2-yl |
| 293 | | Br | $CH_2$ | 6-Chloro-3,4,5-trimethyl-pyridin-2-yl |
| 294 | | Br | $CH_2$ | 6-Chloro-3,4,5-trimethyl-1-oxypyridin-2-yl |
| 295 | | Br | $CH_2$ | 6-Bromo-3,4,5-trimethyl-1-oxypyridin-2-yl |
| 296 | | Br | $CH_2$ | 3,4,5-Trimethoxy-pyridin-2-yl |
| 297 | | Br | $CH_2$ | 3,4,5-Trimethoxy-1-oxypyridin-2-yl |
| 298 | | Br | $CH_2$ | 6-Bromo-3,4,5-trimethoxy-pyridin-2-yl |
| 299 | | Br | $CH_2$ | 6-Chloro-3,4,5-trimethoxy-pyridin-2-yl |
| 300 | | Br | $CH_2$ | 6-Chloro-3,4,5-trimethoxy-1-oxypyridin-2-yl |
| 301 | | Br | $CH_2$ | 6-Bromo-3,4,5-trimethoxy-1-oxypyridin-2-yl |
| 302 | | Br | $CH_2$ | 4,5,6-Trimethoxypyridin-2-yl |
| 303 | | Br | $CH_2$ | 4,5,6- Trimethoxy-1-oxypyridin-2-yl |
| 304 | | Br | $CH_2$ | 3-Bromo-4,5,6-trimethoxypyridin-2-yl |
| 305 | | Br | $CH_2$ | 3-Chloro-4,5,6-trimethoxypyridin-2-yl |
| 306 | | Br | $CH_2$ | 3-Chloro-4,5,6-trimethoxy-1-oxypyridin-2-yl |
| 307 | | Br | $CH_2$ | 3-Bromo-4,5,6-trimethoxy-1-oxypyridin-2-yl |
| 308 | | Br | $CH_2$ | 4,5,6-Trimethoxypyridin-2-yl |
| 309 | | Br | $CH_2$ | 4,5,6- Trimethoxy-1-oxypyridin-2-yl |
| 310 | | Br | $CH_2$ | 3-Bromo-4,5,6-trimethyl-pyridin-2-yl |
| 311 | | Br | $CH_2$ | 3-Chloro-4,5,6-trimethyl-pyridin-2-yl |
| 312 | | Br | $CH_2$ | 3-Chloro-4,5,6-trimethyl-1-oxypyridin-2-yl |
| 313 | | Br | $CH_2$ | 3-Bromo-4,5,6-trimethyl-1-oxypyridin-2-yl |
| 314 | | Br | $CH_2$ | 4,6-Dimethyl-5-methoxy-pyridin-2-yl |
| 315 | | Br | $CH_2$ | 4,6-Dimethyl-5-methoxy-1-oxypyridin-2-yl |
| 316 | | Br | $CH_2$ | 3-Bromo-4,6-dimethyl-5-methoxy-pyridin-2-yl |
| 317 | | Br | $CH_2$ | 3-Chloro-4,6-dimethyl-5-methoxy-pyridin-2-yl |
| 318 | | Br | $CH_2$ | 3-Chloro-4,6-dimethyl-5-methoxy-1-oxypyridin-2-yl |
| 319 | | Br | $CH_2$ | 3-Bromo-4,6-dimethyl-5-methoxy-1-oxypyridin-2-yl |
| 320 | | Br | $CH_2$ | 4-Bromo-5,6-dimethoxy-pyridin-2-yl |
| 321 | | Br | $CH_2$ | 4-Bromo-5,6-dimethoxy-1-oxypyridin-2-yl |
| 322 | | Br | $CH_2$ | 3,4-Dibromo-5,6-dimethoxy-pyridin-2-yl |

(continued)

| No. | Ex. | $R^1$ | $R^4$ | $R^5$ |
|---|---|---|---|---|
| 323 | | Br | $CH_2$ | 3-Chloro-4-bromo-5,6-dimethoxy-pyridin-2-yl |
| 324 | | Br | $CH_2$ | 3-Chloro-4-bromo-5,6-dimethoxy-1-oxypyridin-2-yl |
| 325 | | Br | $CH_2$ | 3,4-Dibromo-5,6-dimethoxy-1-oxypyridin-2-yl |
| 326 | | Br | $CH_2$ | 4,6-Dimethyl-5-methoxypyridin-3-yl |
| 327 | | Br | $CH_2$ | 4,6-Dimethyl-5-methoxy-1-oxypyridin-3-yl |
| 328 | | Br | $CH_2$ | 4,6-Dimethyl-5-bromopyridin-3-yl |
| 329 | | Br | $CH_2$ | 4,6-Dimethyl-5-chloropyridin-3-yl |
| 330 | | Br | $CH_2$ | 5,6-Dimethyl-4-bromopyridin-3-yl |
| 331 | | Br | $CH_2$ | 5,6-Dimethyl-4-chloropyridin-3-yl |
| 332 | | Br | $CH_2$ | 4,6-Dimethyl-5-bromo-1-oxypyridin-pyridin-3-yl |
| 333 | | Br | $CH_2$ | 4,6-Dimethyl-5-chloro-1-oxypyridin-pyridin-3-yl |
| 334 | | Br | $CH_2$ | 5,6-Dimethyl-4-bromo-1-oxypyridin-pyridin-3-yl |
| 335 | | Br | $CH_2$ | 5,6-Dimethyl-4-chloro-1-oxypyridin-pyridin-3-yl |
| 336 | | Br | $CH_2$ | 2,6-Dimethyl-3-methoxypyridin-4-yl |
| 337 | | Br | $CH_2$ | 2,6-Dimethyl-pyridin-4-yl |
| 338 | | Br | $CH_2$ | 2,3,6-Trimethyl-pyridin-4-yl |
| 339 | | Br | $CH_2$ | 2,3,6-Trimethoxy-pyridin-4-yl |
| 340 | | Br | $CH_2$ | 2,6-Dimethyl-3-bromopyridin-4-yl |
| 341 | | Br | $CH_2$ | 2,6-Dimethyl-3-chloropyridin-4-yl |
| 342 | | Br | $CH_2$ | 2,6-Dichloro-3-bromopyridin-4-yl |
| 343 | | Br | $CH_2$ | 2,6-Dibromo-3-chloropyridin4-yl |
| 344 | | Br | $CH_2$ | 2,3,6-Trichloro-pyridin-4-yl |
| 345 | | Br | $CH_2$ | 2,3,6-Tribromo-pyridin-4-yl |
| 346 | | Br | $CH_2$ | 2,6-Dimethyl-3-methoxy-1-oxy-pyridin-4-yl |
| 347 | | Br | $CH_2$ | 2,6-Dimethyl-1-oxy-pyridin-4-yl |
| 348 | | Br | $CH_2$ | 2,3,6-Trimethyl-1-oxy-pyridin-4-yl |
| 349 | | Br | $CH_2$ | 2,3,6-Trimethoxy-1-oxy-pyridin-4-yl |
| 350 | | Br | $CH_2$ | 2,6-Dimethyl-3-bromo1-oxy-pyridin-4-yl |
| 351 | | Br | $CH_2$ | 2,6-Dimethyl-3-chloro1-oxy-pyridin-4-yl |
| 352 | | Br | $CH_2$ | 2,6-Dichloro-3-bromo1-oxy-pyridin-4-yl |
| 353 | | Br | $CH_2$ | 2,6-Dibromo-3-chloro1-oxy-pyridin-4-yl |
| 354 | | Br | $CH_2$ | 2,3,6-Trichloro-1-oxy-pyridin-4-yl |
| 355 | | Br | $CH_2$ | 2,3,6-Tribromo-1-oxy-pyridin-4-yl |
| 356 | | Br | $CH_2$ | 4,6-Dimethyl-5-iodopyridin-3-yl |
| 357 | | Br | $CH_2$ | 5,6-Dimethyl-4-iodopyridin-3-yl |
| 358 | | Br | $CH_2$ | 4,5,6-Trichloropyridin-3-yl |
| 359 | | Br | $CH_2$ | 4,5,6-Tribromopyridin-3-yl |
| 360 | | Cl | $CH_2$ | 4,5,6-Trimethoxy-3-chloropyridin-2-yl |

(continued)

| No. | Ex. | $R^1$ | $R^4$ | $R^5$ |
|---|---|---|---|---|
| 361 | | Br | $CH_2$ | 4,5,6-Trimethoxy-3-chloropyridin-2-yl |
| 362 | | Cl | $CH_2$ | 4,5,6-Trimethoxy-3-bromopyridin-2-yl |
| 363 | | Br | $CH_2$ | 4,5,6-Trimethoxy-3-bromopyridin-2-yl |
| 364 | | Cl | $CH_2$ | 4,5,6-Trimethoxy-pyridin-3-yl |
| 365 | | Cl | $CH_2$ | 4,5,6-Trimethoxy-1-oxy-pyridin-3-yl |
| 366 | | Cl | $CH_2$ | 2-Bromo-4,5,6-trimethoxy-pyridin-3-yl |
| 367 | | Cl | $CH_2$ | 2-Chloro-4,5,6-trimethoxy-pyridin-3-yl |
| 368 | | Cl | $CH_2$ | 2-Chloro-4,5,6-trimethoxy-1-oxy-pyridin-3-yl |
| 369 | | Cl | $CH_2$ | 2-Bromo-4,5,6-trimethoxy-1-oxy-pyridin-3-yl |
| 370 | | Cl | $CH_2$ | 4,5,6-Trimethyl-pyridin-3-yl |
| 371 | | Cl | $CH_2$ | 4,5,6-Trimethyl-1-oxy-pyridin-3-yl |
| 372 | | Cl | $CH_2$ | 2-Bromo-4,5,6-trimethyl-pyridin-3-yl |
| 373 | | Cl | $CH_2$ | 2-Chloro-4,5,6-trimethyl -pyridin-3-yl |
| 374 | | Cl | $CH_2$ | 2-Chloro-4,5,6-trimethyl-1-oxy-pyridin-3-yl |
| 375 | | Cl | $CH_2$ | 2-Bromo-4,5,6-trimethyl-1-oxy-pyridin-3-yl |
| 376 | | Cl | $CH_2$ | 2-Iodo-4,5,6-trimethyl-pyridin-3-yl |
| 377 | | Cl | $CH_2$ | 2-Iodo-4,5,6-trimethyl-pyridin-3-yl |
| 378 | | Br | $CH_2$ | 4,5,6-Trimethoxy-pyridin-3-yl |
| 379 | | Br | $CH_2$ | 4,5,6-Trimethoxy-1-oxy-pyridin-3-yl |
| 380 | | Br | $CH_2$ | 2-Bromo-4,5,6-trimethoxy-pyridin-3-yl |
| 381 | | Br | $CH_2$ | 2-Chloro-4,5,6-trimethoxy-pyridin-3-yl |
| 382 | | Br | $CH_2$ | 2-Chloro-4,5,6-trimethoxy-1-oxy-pyridin-3-yl |
| 383 | | Br | $CH_2$ | 2-Bromo-4,5,6-trimethoxy-1-oxy-pyridin-3-yl |
| 384 | | Br | $CH_2$ | 4,5,6-Trimethyl-pyridin-3-yl |
| 385 | | Br | $CH_2$ | 4,5,6-Trimethyl-1-oxy-pyridin-3-yl |
| 386 | | Br | $CH_2$ | 2-Bromo-4,5,6-trimethyl-pyridin-3-yl |
| 387 | | Br | $CH_2$ | 2-Chloro-4,5,6-trimethyl-pyridin-3-yl |
| 388 | | Br | $CH_2$ | 2-Chloro-4,5,6-trimethyl-1-oxy-pyridin-3-yl |
| 389 | | Br | $CH_2$ | 2-Bromo-4,5,6-trimethyl-1-oxy-pyridin-3-yl |

Compounds of interest in Table 4 are: compounds 2, 3, 13, 82, 83, 162, 163, 168, 169, 174, 175, 180, 181, 186, 187, 192, 1.93, 198, 199, 204, 205, 210, 211, 228, 229, 230, 231, 232, 233, 234, 235, 236, 237, 250, 251, 262, 263, 268, 269, 274, 275, 280, 281, 286, 287, 292, 293, 298, 299, 304, 305, 3 10, 311, 316, 317, 328, 329, 338, 372, 373, 380 and 381 (with those selected being 162, 163, 168, 169, 174, 175, 180, 181, 186, 187, 192, 193, 198, 199, 204, 245, 228, 229, 262, 263, 268, 269, 274, 275, 280, 281, 286, 287, 292, 293, 316, 317, 328, and 329.)

### III. Synthesis of the Compounds of the Invention

Synthesis of compounds of the present invention may be accomplished by various methods known in the art, including those described in, for example, Montgomery, J Med. Pharm. Chem., 1962, 5, 15-24; Sircar, U.S. Patent 4,772,606, 1988; Sircar, U.S. Patent 4,748,177, 1988; Hans, U.S. Patent 5,110,818, 1992; Gillespie, PCT publication No. WO 02/055521; Matsuda, JP 10025294 A2, 1998; Hans, U.S. Patent 5,110,818, 1992 and U.S. Publication No. US

2003/0078413. The synthesis of the several embodiments of the invention is illustrated. The process is applicable to other subgenus.

A. **Synthesis of Compounds of Formula I and Related Analogs**

The compounds of Formula I and related analogs of the present invention may be synthesized by various methods known in the art. Illustratively, the strategy for the sysnthesis of pyrrolopyrimidines is outlined in Scheme 1 and consists of three parts: (1) constructing the bicyclic system, starting from either a pyridine, a pyrimidine, a pyrrole, or an acyclic precursor (2) appending the $R^5$-$R^4$- group, and (3) further elaborating the ring systems.

Importantly, one skilled in the art will recognize that the sequence of events is not necessarily (1)-(2)-(3), and that these events may be interchanged, provided there be no incompatibility between the reagents and the functional groups specific to the point in case.

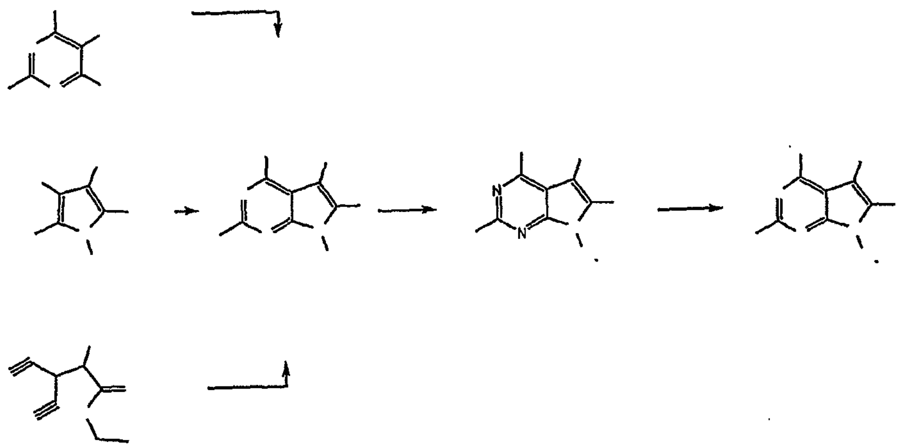

Scheme 1

Also, the starting materials or the intermediates of Formula 1, 4, 5, and I can exist in tautomeric forms as shown in Fig. 1, and both forms are indiscriminately used in this patent.

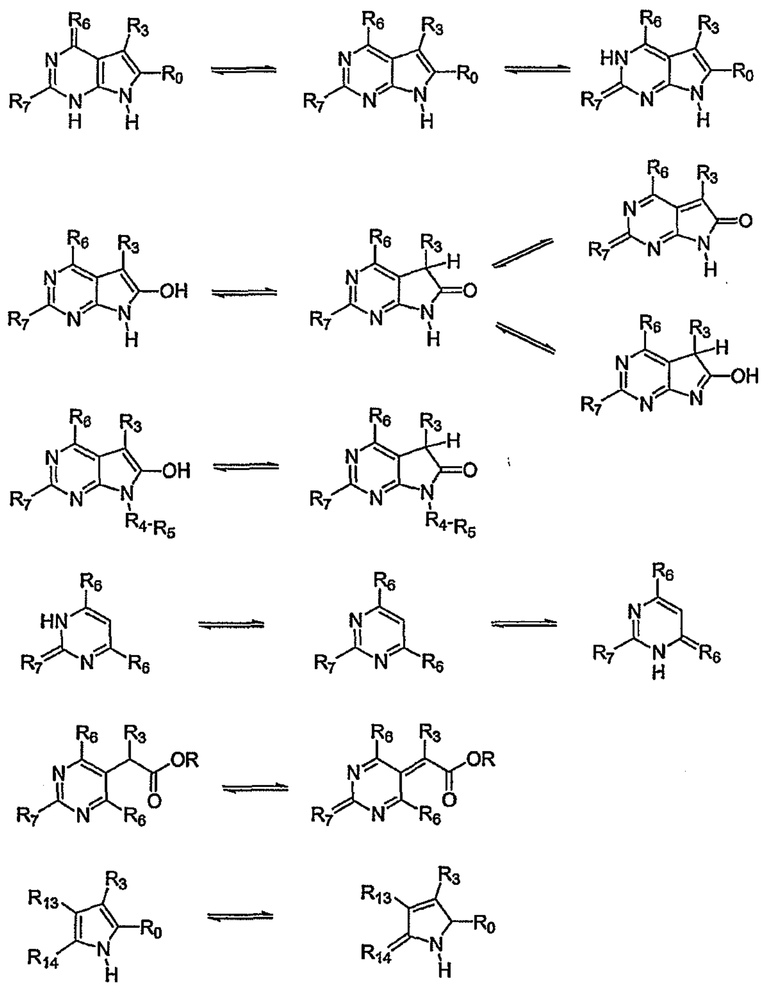

Figure 1

## 1. Assembly of the pyrolo[2,3-d]pyrimidine

1.1. Assembly of the pyrolo[2,3-d]pyrimidine starting from a pyrimidine

The compounds of Formula 4 can be prepared from pyrimidines as outlined in Scheme 2. For instance:

**STARTING FROM A PYRIMIDINE**
**METHODS TO PREPARE THE 5-MEMBERED RING OF COMPOUND (4)**

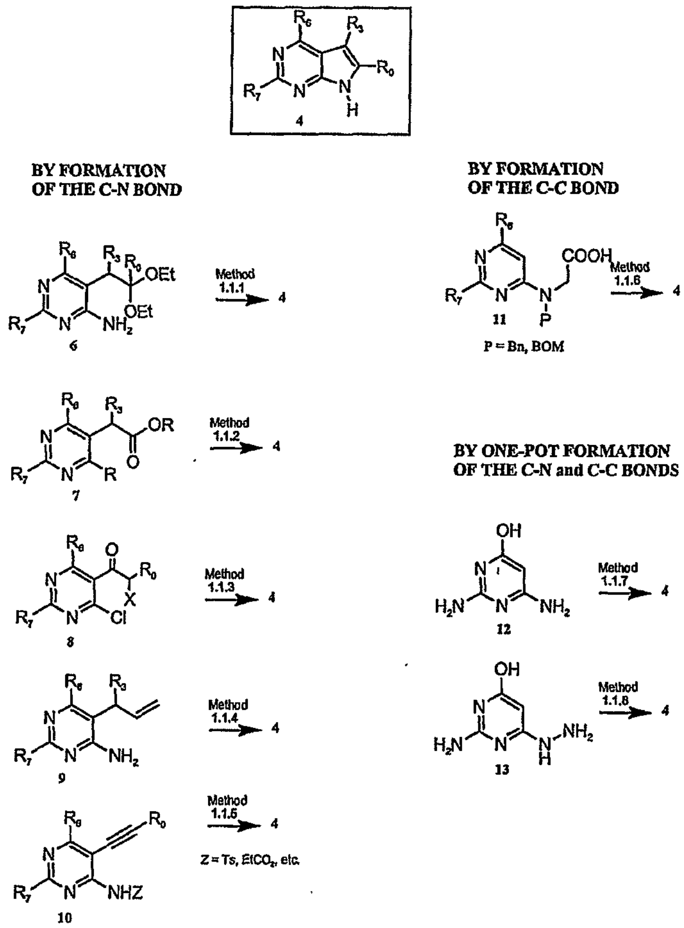


Scheme 2

<u>Method 1.1.1</u>

The compounds of Formula 4 can be made by intramolecular cyclization of an aldehyde or ketone, possibly protected, as in Formula 6. (See, J. Davoll, J. Chem. Soc. 1960, 131; J. A. Montgomery, J. Chem. Soc. 1967, 665; G. Cristalli, J. Med. Chem. 1988, 31, 390; T. Miwa, J. Org. Chem. 1993, 58, 1696; D.M. Williams, J. Chem. Soc., Perkin Trans 1, 1997, 1171).

<u>Method 1.1.2</u>

The compounds of Formula 4, wherein $R^3$ is H, $R^6$ is Cl, and $R^7$ is $NH_2$ can be prepared by treating compounds of Formula 7 wherein R is a halogen or a leaving group with ammonia. Similarly, compounds of Formula I wherein $R^3$ is H, $R^1$ is Cl, $R^2$ is $NH_2$ can be prepared by treating the compound of Formula 7 wherein R is a halogen or leaving group with $R^5$-$R^4$-$NH_2$ in butanol at reflux in presence of a base such as $K_2CO_3$, $Cs_2CO_3$ i-$PrNEt_2$. (A.B. Reitz J. Med. Chem. 1994, 37, 3561). Compounds of Formula 7 can in turn be prepared as taught by G.W. Craig J. Prakt. Chem. 2000, 342, 504 and M. Semonsky Coll. Czech. Chem. Commun. 1980, 45, 3583).

<u>Method 1.1.3</u>

The compounds of Formula 4 can be obtained by treatment of a $\alpha$-haloketone of Formula 8 wherein X is a halogen with ammonia or an synthetic equivalent thereof.

Method 1.1.4

The compounds of Formula 4 wherein $R^0$ is methyl can be obtained by a tandem Pd-mediated intramolecular cyclization/double-bond migration of alkenes of Formula 9 (S. E. Watson, Synth. Commun. 1998, 28, 3885).

Method 1.1.5

The compounds of Formula 4 wherein $R^3$ is H can be obtained by Pd-mediated intramolecular cyclization of alkynes of Formula 10, wherein Z in as electron-withdrawing group such as, *e.g.,* tosyl-, or $EtCOO_2$-.

Method 1.1.6

The compounds of Formula 4 wherein $R^3$ is AcO- can be obtained by intramolecular Friedel-Crafts acylation of precursors of Formula 11 (E. D. Edstrom, J. Org. Chem. 1993, 58, 403).

Method 1.1.7

The compound of Formula 4, wherein $R^0$ is H, $R^6$ is OH, and $R^7$ is $NH_2$, can be prepared by treating the compound of Formula 12 with an α-haloaldehydes of the formula $R^3$-CHX-CHO. See, D.M. Williams, J. Chem. Soc., Perkin Trans 1, 1997, 1171; C. J. Barnett, Org. Proc. Res. Devop. 1999, 3, 184; A. Gangjee, J. Med. Chem. 2001, 44, 1993.

Method 1.1.8

The compounds of Formula 4, wherein $R^6$ is OH and $R^7$ is $NH_2$ can be obtained by treating the compound of Formula 13 with an aldehyde of the formula $R^3$-CHO. See, A. Gangjee, J. Med. Chem. 2003, 46, 591; E. C. Taylor, Heterocycles 1996, 43, 323.

1.2: Assembly of the pyrolo[2,3-d]pyrimidine starting from a pyrrole

The compounds of Formula 4 can also be made from pyrroles of Formula 2. There is a variety of methods by which the 6-membered ring can be formed (*e.g.* R. J. Bontems, J. Med. Chem, 1990, 33, 2174 and references therein). For instance:

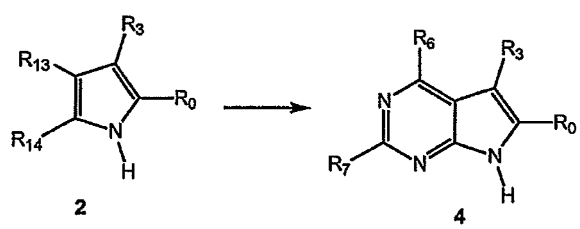

2

4

Scheme 3

Compounds of Formula 2 wherein $R^{13}$ is -CN and $R^{14}$ is R-NH-$CR^7$=N- can be cyclized and rearranged to give compounds of Formula 4 where $R^6$ is R-NH-. See, E. C. Taylor, J. Am. Chem. Soc. 1965, 87, 1995.

Compounds of Formula 2 wherein $R^{13}$ is -CN and $R^{14}$ is -$NH_2$ can be treated with thiourea, guanidine, or chloroformamidine to give compounds of Formula 4 in which $R^6$ is -$NH_2$ and $R^7$ is -$NH_2$. See, H. Kosaku, Heterocycles, 2001, 55, 2279; A. Gangjee, US Patent 5,939,420 (1999**).**

Compounds of Formula 2 wherein $R^{13}$ is -CN and $R^{14}$ is -$NH_2$ can be treated formamidine acetate to give compounds of Formula 4 wherein $R^6$ is -$NH_2$ and $R^7$ is H (J. A. Montgomery, J. Chem. Soc. 1967, 665). The same transformation can be accomplished by treatment with DMF-DMA or an orthoester such as $(EtO)_3CH$, followed by treatment with ammonia. See, E. C. Taylor, J. Am. Chem. Soc, 1965, 87, 1995.

Compounds of Formula 2 wherein $R^{13}$ is -CN and $R^{14}$ is $NH_2$ can be treated with formic acid to give compounds of Formula 4 wherein $R^6$ is -OH and $R^7$ is H (K. A. M. El-Bayould, J. Chem. Res. Miniprint, 1995, 1901).

Compounds of Formula 2 wherein $R^{13}$ is - $CO_2NH_2$ and $R^{14}$ is -$NH_2$ can be treated under Vilsmeyer-Haack conditions ($DMF/POCl_3$) to give compounds of Formula 4 wherein $R^6$ is OH or Cl and $R^7$ is H. See, K. A. M. El-Bayouki,

J. Chem. Res. Miniprint, 1995, 1901.

Compounds of Formula 2 wherein $R^{13}$ is -$CONH_2$ and $R^{14}$ is -$NH_2$ can be treated with $CS_2$ or $EtOCS_2K$ to give compounds of Formula 4 in which $R^6$ is -OH and $R^7$ is - SH. See, S. M. Bennett, J. Med. Chem. 1990, 33, 2162.

1.3. Assembly of the pyrolo[2,3-d]pyrimidine starting from an acyclic precursor

The compounds of Formula 14 can be prepared from an acyclic precursor a outlined in Scheme 4 (T. Miwa, J. Med. Chem. 1991, 34, 555).

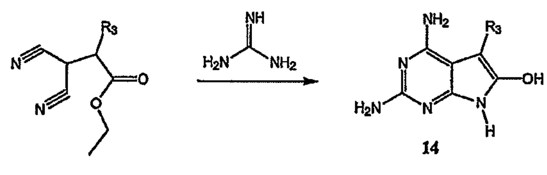

3

14

Scheme 4

**2. Incorporation of the** -$R^4$-$R^5$ **fragment**

2.1. Alkylation of compounds of Formula 4

Compounds of Formula 4 can be alkylated in the presence of a base such as $K_2CO_3$, NaH, $Cs_2CO_3$, DBU *etc.* with/without the presence of a catalyst such as NaI, KI, $(Bu)_3NI$ *etc.,* and in a polar solvent such as DMF, THF, DMSO *etc.* using electrophiles such as $L^1$-$R^4$-$R^5$ where $L^1$ is a leaving group. See Scheme 5. Leaving groups include but are not limited to, *e.g.,* halogen, triflate, tosylate, mesylate, triphenylphosphonium (generated under Mitsunobu conditions, *e.g.* $PPh_3$/DEAD) *etc.* See Kasibhatla, PCT publication number WO 03/037860.

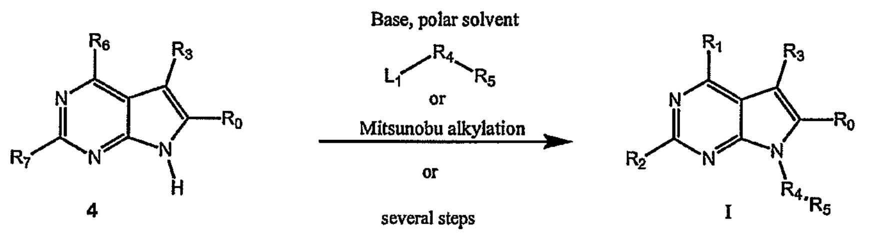


4

I

Scheme 5

2.2. Preparation of electrophiles $L^1$-$R^4$-$R^5$ wherein $L^1$ is a leaving group

2.2.1. Synthesis of benzyl type electrophile

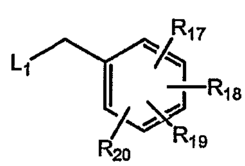

Fig 2

The electrophiles can be prepared from the substituted benzene derivatives using various methods reported

in the literature, see Jerry March, Advanced Organic Chemistry, 4th edition; Larock, Comprehensive Organic Transformations, 1989,VCH, New York. For example the compounds wherein $L^1$ is Br can be prepared by reduction of the corresponding benzoic acid or benzaldehyde, followed by halogenation. These benzyl derivatives can also be prepared by benzylic oxidation or benzylic halogenation. Further modification of the benzyl ring can be done before or after the pyrolo[2,3-d]pyrimidine alkylation step.

2.2.2. Synthesis of pyridyl methyl type electrophile

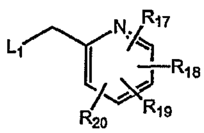
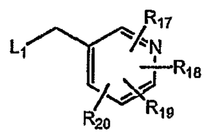
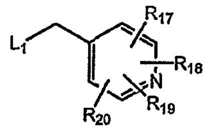

Fig 3

These compounds can be prepared from many methods reported in the literature.

Morisawa, J. Med. Chem. 1974, 17, 1083; Klaus, W., J. Med. Chem. 1992, 35, 438; Abramovitch, R.A.; Smith, E. M. "Pyridine-1-oxide in Pyridine and its Derivatives," in The Chemistry of Heterocylic Compounds; Weissberger, A., Taylor, E. C., Eds.; John Wiley, New York, 1974, Pt. 2, pp 1-261; Jeromin, G. E., Chem. Ber. 1987, 120, 649. Blanz, E. J., J. Med. Chem. 1970, 13, 1124; Smith, Kline and French, EP Application EP 0184322,1986; Abblard, J., Bull. Soc. Chim. Fr. 1972, 2466; Fisher, B. E., The Structure of Isomaltol. J. Org. Chem. 1964, 29, 776. De Cat, A., Bull. Soc. Chim. Belg. 1965, 74, 270; Looker, J. H., J. Org. Chem. 1979, 44, 3407. Ackerman, J. F. Ph.D. Dissertation, University of Notre Dame, June, 1949. These methods can be applied to the synthesis of quinoline, and isoquinolines type compounds.

2.3. Incorporation of the -$R^4$-$R^5$ fragment by nucleophilic substitution.

In some cases, the -$R^4$-$R^5$ group can be appended *before* the bicyclic pyrrolo[2,3-d]pyrimidine bicyclic ring is constructed, and this is further detailed below (paragraph 4, schemes 8 and 9). In these cases the -$R^4$-$R^5$ group can be appended by an aromatic nuclophilic substitution using $NH_2$-$R^4$-$R^5$. The compound $NH_2$-$R^4$-$R^5$ is obtained by treating $L^1$-$R^4$-$R^5$ with ammonia at temperatures of 20-160 ˚C in a pressure vessel. The corresponding amines where $L^1$ is -$NH_2$ can be prepared by a variety of methods, for instance from compounds where $L^1$ is leaving group such as chloride, bromide, tosylate, mesylate *etc.* using ammonia, or with sodium azide followed by hydrogenation.

**3. <u>Further Elaboration of the ring systems</u>**

3.1. Functional group interconversions of $R^0$

Compounds of Formula IA, wherein $R^0$ is H can be oxidized to compounds of Formula I wherein $R^0$ is -OH with pyridinium tribromide or polymer supported pyridinium tribromide in tert-butanol/acetic acid mixture followed by zinc reduction. See, C. Liang, US Patent 6,610,688 (2000); L. Sun, Bioorg. Med. Chem Lett., 2002,12, 2153.

Compounds of Formula I, wherein $R^0$ is H can be treated under Mannich conditions (HCHO + HNRR') to give compounds Formula IA wherein $R^0$ is -CH-NRR'. See F. Seela, Synthesis, 1997, 1067.

Compounds of Formula IA, wherein $R^0$ is H can be lithiated and treated with electrophiles (*e.g.,* $I_2$, ArCHO) to provide compounds of Formula IA wherein $R^0$ is, *e.g.* I or -CH(OH)Ar. See, E. Bisagni, Tetrahedron, 1983, 39, 1777; T, Sakamoto, Tetrahedron Lett. 1994, 35, 2919; T. Sakamoto, J. Chem. Soc., Perkin Trans 1, 1996, 459.

3.2. Functional group interconversions of $R^1$

Compounds of Formula IA, wherein $R^1$ is OH, can be converted to halides using standard conditions $POCl_3$, $POBr_3$ *etc.* with/without a base such as $Et_3N$, N,N-dimethylaniline, $(i\text{-}Pr)_2NEt$ *etc.* and with/without a catalyst such as $BnEt_3N^+Cl^-$, in polar solvents such as $CH_3CN$, $CH_2Cl_2$ *etc.* Related methods include, but are not limited to, $SOCl_2$/DMF (M. J. Robins, Can. J. Chem. 1973, 12, 3161), $PPh_3/CCl_4$ (L. De Napoli J. Chem. Soc. Perkin Trans 1, 1994, 923), $HMPT/CCl_4$ or HMPT/NBS (E. A. Veliz, Tetrahedron Lett, 2000, 41, 1695) or $PPh_3/I_2$ (X. Lin, Org. Letters, 2000, 2, 3497).

Compounds of Formula IA, wherein $R^1$ is $-NH_2$, can be converted to halides by a Balz-Schiemann (F) or Sandmeyer reaction (Cl, Br, I) by means of a nitrosylating agent ($NaNO_2/H^+$, $NOBF_4$, RONO) and a halogen donor ($BF_4^-$, $CuX_2$, $SbX_3$ where X is halogen).

Compounds of Formula IA, wherein $R^1$ is alkyl can be prepared from compounds of Formula 4 where $R^1$ is halogen and trialkyl aluminum or dialkyl zinc (A. Holy, J. Med. Chem. 1999, 42, 2064).

Compounds of Formula IA, wherein $R^1$ is a halide can be converted to compounds wherein $R^1$ is $-NH_2$, -OH, -SH, $-OR^8$, $-SR^8$ with standard reagents, *e.g.,* $NH_3$, NaOH, thiourea, $R^8O^-$, $R^8S^-$, with or without a catalyst (e.g. Pd, Ni, Cu, Lewis acid, $H^+$) *(e.g.,* B. G. Ugarkar, J. Med. Chem. 2000, 43, 2883-2893 and 2894-2905).

Compounds of Formula IA, wherein $R^1$ is halogen or another leaving group can be treated with ammonia to provide compounds of Formula IA wherein $R^1$ is $-NH_2$ (F. Seela, Liebigs. Ann. Chem. 1985, 315).

3.3. Functional group interconversions of $R^2$

Compounds of Formula IA, wherein $R^2$ is $-NH_2$ can be temporarily protected, *e.g*. as an amide ($Ac_2O$, PivCl), a carbamate $(tBoc)_2O$) or amidine (DMF-DMA).

Compounds of Formula IA, wherein $R^2$ is $-NH_2$ can be converted to halides by a Balz-Schiemann (F) or Sandmeyer reaction (Cl, Br, I) by means of a nitrosylating agent ($NaNO_2/H^+$, $NOBF_4$, RONO) and a halogen donor ($BF4^-$, $CuX_2$, $SbX_3$).

Compounds of Formula IA, wherein $R^2$ is a halide can be converted to compounds wherein $R^2$ is $-NH_2$, -OH, -SH, $-OR^8$, $-SR^8$ with standard reagents, *e.g.* $NH_3$, NaOH, thiourea, $R^8O^-$, $R^8S^-$, with or without a catalyst (*e.g.* Pd, Ni, Cu, Lewis acid, $H^+$).

Compounds of Formula IA, wherein $R^2$ is -SH can be converted to halides ($Br_2$). They can also be oxidized (*e.g.,* $H_2O_2$) and treated with ammonia to give a $-NH_2$ group (S. M. Bennett, J. Med. Chem. 1990, 33, 2162).

Compounds of Formula IA, wherein $R^2$ is a sulfide, *e.g.,* MeS-, can be converted to a sulfone, *e.g.* $MeSO_2^-$, and displaced with a nucleophile, *e.g.* $NH_3$ or $NH_2$-$NH_2$, $N_3$-, CN-.

3.4. Functional group interconversions of $R^3$

Compounds of Formula IA, wherein $R^3$ is H, can be halogenated (J. F. Gerster, J. Chem. Soc. 1969, 207) and further functionalized by Pd-catalyzed reactions ((a) Sonogashira coupling: E. C. Taylor et al, Tetrahedron, 1992, 48, 8089; (b) carboxylation: J. W. Pawlik, J. Heterocycl. Chem. 1992, 29, 1357; (c) Suzuki coupling: T. Y. I Wu, Org. Lett., 2003, 5, 3587) or by addition of nucleophiles (*e.g.* hydrazine, B. M. Lynch, Can. J. Chem. 1988, 66, 420).

Compounds of Formula IA wherein $R^3$ is -CHO can be sujected to a Bayer-Villiger oxidation to provide compounds of Formula IA wherein $R^3$ is -O-CHO. The latter can be hydrolyzed to $R^3$ is -OH. (A.S. Bourlot, E. Desarbre, J.Y. Mérour Synthesis 1994, 411)

Compounds of Formula IA, wherein $R^3$ is H can be treated under Mannich condition (HCHO + HNRR') to give compounds Formula IA wherein $R^3$ is -CH-NRR' (F. Seela, Synthesis, 1997, 1067)

Compounds of Formula IA, wherein $R^3$ is $-CH_2-NBn_2$ can be obtained by Mannich reaction and further treated with an aniline of Formula $NH_2$-Ar to give compounds of Formula IA wherein $R^3$ is $-CH_2$-NH-Ar (D. C. Miller, J. Med. Chem. 2002, 45, 90).

Compounds of Formula IA, wherein $R^3$ is Br can be metallated with BuLi, and treated with an electrophile such as MeI to give a compound of Formula IA, wherein $R^3$ is methyl. Compounds of Formula IA, wherein $R^1$ is -Cl and $R^3$ is -Br can undergo selective metallation at $R^3$ (J. S. Pudlo, J. Med. Chem. 1990, 33, 1984).

Compounds of Formula IA, wherein $R^0$ is -OH and $R^3$ is H can be be monalkylated or bis-alkylated to give compounds of Formula ID, wherein $R^1$ is an alkyl group. The alkylation can be effected in the presence of a base such as KHMDS, LHMDS, LDA *etc.* with/without the presence of a catalyst such as NaI, KI, $(Bu)_3NI$ *etc.,* and in a polar solvent such as THF, DMSO *etc.* using electrophiles such as $L^1$-$R^3$ where $L^1$ is a leaving group. Leaving groups include but are not limited to, *e.g.,* halogen, triflate, tosylate or mesylate.

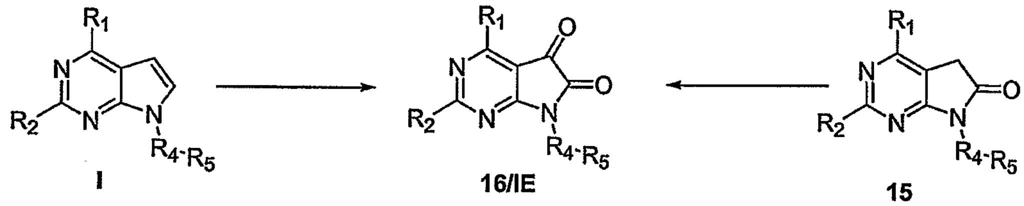

I 16/IE 15

Scheme 6

Compounds of Formula IA, wherein $R^0$ is H and $R^3$ is H can be oxidized to compounds of Formula 16/IE, with an oxidizing reagent such as ruthenium tetroxide in a binary solvent such as acetonitrile/water. (G.W. Gribble Org. Prep. Proced. Int. 2001, 33(6), 615).

Compounds of Formula IA, wherein $R^0$ is -OH and $R^3$ is H can be oxidized to compounds of Formula IC, wherein $R^3$ is an oxo group with an oxidizing reagent such as selenium dioxide or oxygen in presence of a cobalt (III) catalyst. ($SeO_2$ oxidation: Romeo Helv. Chim. Acta. 1955, 38, 463, 465. Oxygen oxidation: A. Inada Heterocycles 1982, 19, 2139).

3.5. Further elaboration of $R^5$

$R^5$, especially when it is aryl or heteroaryl, can be further modified as needed, for example by halogenation, nitration, palladium coupling of halogen, Friedel-Crafts alkylation/acylation, *etc.* or these modifications can also be done before alkylation, see Jerry March, *Advanced Organic Chemistry.* The heteroaromatic rings can also be oxidized to their corresponding N-oxides using various oxidizing agents such as $H_2O_2$, $O_3$, MCPBA *etc.* in polar solvents such as $CH_2Cl_2$, $CHCl_3$, $CF_3COOH$ *etc.* See Jerry March, Advanced Organic Chemistry, 4th edition, Chapter 19. Examples of modifications are suggested in Scheme 7.

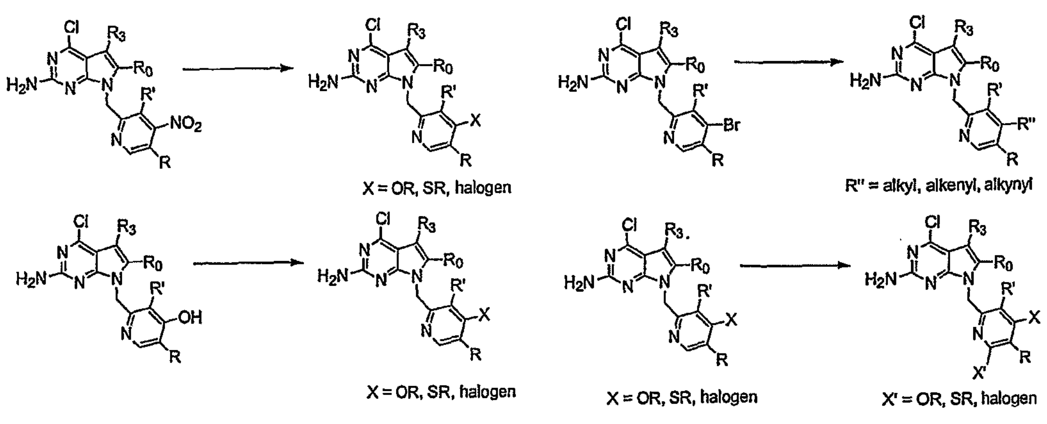


Scheme 7

**4. Permutations of the order of events**

As mentioned above, the events (1) assembly of the bicyclic system (2) appendage of the $R^5$-$R^4$- moiety, and (3) further elaboration of the ring systems do not necessarily have to be made in the sequence (1)-(2)-(3), and it may be beneficial to proceed in a different sequence.

Method 4.1.

Scheme 8 shows a synthesis in which the order of events is not (1)-(2)-(3), but is (2)-(1)-(3). First $R^5$ is appended *via* an aromatic nucleophilic substitution, then the bicyclic system is constructed, and finally it is elaborated.

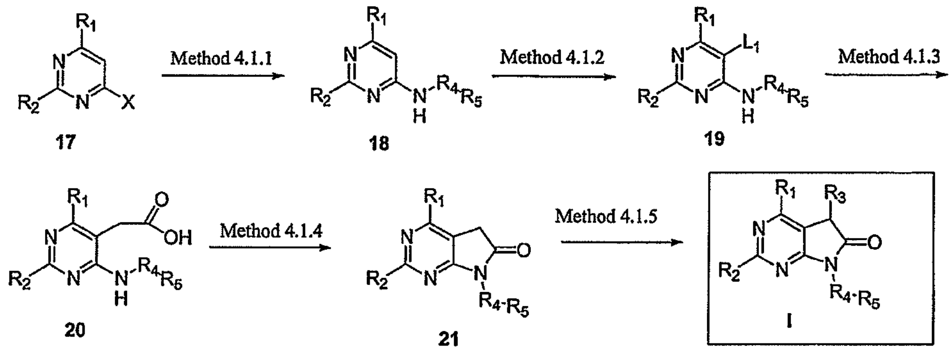


17 18 19

20 21 I

Scheme 8

Method 4.1.1

The compound of Formula 18, wherein $R^1$ is Cl and $R^2$ is $NH_2$, can be prepared by treating the compound of Formula 17 with $R^5$-$R^4$-$NH_2$ in butanol at reflux in presence of a base such as $K_2CO_3$, $Cs_2CO_3$ or $iPrNEt_2$. (A.B. Reitz J. Med. Chem. 1994, 37, 3561).

Method 4.1.2

The compound of Formula 19, wherein $R^1$ is Cl and $R^2$ is $NH_2$, can be prepared by refluxing the compound of Formula 18 in chloroform or dichloroethane in presence of an halogenating reagent such as bromine, N-bromosuccinimide, iodine or N-iodosuccinimide and an acid such as acetic acid or p-toluenesulfonic acid. (A.P. Phillips J. Am. Chem. Soc. 1952, 74, 3922).

Method 4.1.3

The compound of Formula 20, wherein $R^1$ is Cl and $R^2$ is $NH_2$, can be prepared by coupling the compound of Formula 19 with trimethylsilylacetylene under Sonogashira conditions followed by hydroboration using dichlorohexylborane and oxidation using hydrogen peroxide in presence of sodium hydroxide. (Sonogashira coupling: E. C. Taylor Tetrahedron, 1992, 48, 8089. Hydroboration/oxidation: G. Zweifel J. Am. Chem. Soc. 1976, 98, 3184).

Method 4.1.4

The compound of Formula 21, wherein $R^1$ is Cl and $R^2$ is $NH_2$, can be prepared by heating the compound of Formula 20 in a polar aprotic solvent such as THF, DME or dioxane in presence of oxalyl chloride, thionyl chloride, mesyl chloride or alkyl chloroformate and a base such as $iPrNEt_2$ or pyridine. It can also be prepared by treating the compound of Formula 20 with coupling reagents such DCC/HOBt, DCC/DMAP or EDCI/HOBt. (R.C. Larock Comprehensive Organic Transformations Second Edition, p1870).

Method 4.2

Again, as mentioned above, the events (1) assembly of the bicyclic system (2) appendage of the $R^5$-$R^4$- moiety, and (3) further elaboration of the ring systems do not necessarily have to be made in the sequence (1)-(2)-(3), and it may be beneficial to proceed in a different sequence. For illustrative purposes, Scheme 9 shows a putative synthesis in which the order of events is not (1)-(2)-(3), but is (2)-(1)-(3). First $R^5$ is appended via an aromatic nucleophilic substitution, then the bicyclic system is constructed, and finally it is elaborated.

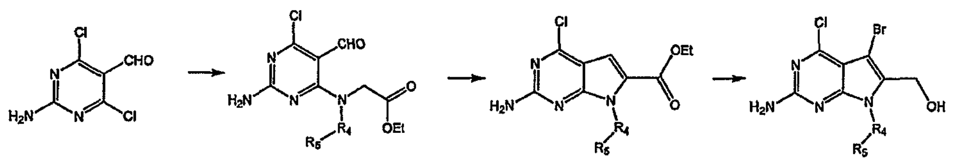

Scheme 9

Method 4.3

For illustrative purposes, Scheme 10 shows a putative synthesis in which the order of events is not (1)-(2)-(3), but is (1)-(3)-(2)-(3). First the bicyclic ring is constructed, then it is elaborated, then the $R^4$-$R^5$ moiety is appended, and finally the bicyclic ring system is further elaborated (deprotection).

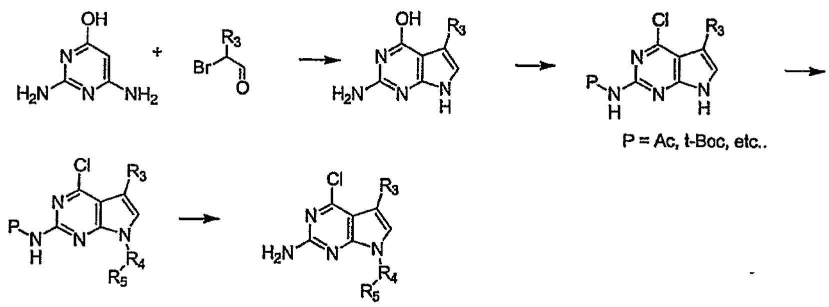


Scheme 10

Also, if $R^5$ is for instance a pyridine, it can be converted to a N-oxide either before or after alkylation.

**B. Synthesis of Compounds of Formula II (Imidazolopyridines and Aminopurines) and Related Analogs**

The compounds of Formula II may be synthesized by various methods known in the art. A general strategy for the synthesis of aminopurines (Formulae IIC & IID) is outlined in Scheme 11. Other members of compounds of Formula II may also be synthesized following this route. It should be understood that other methods can also be used.

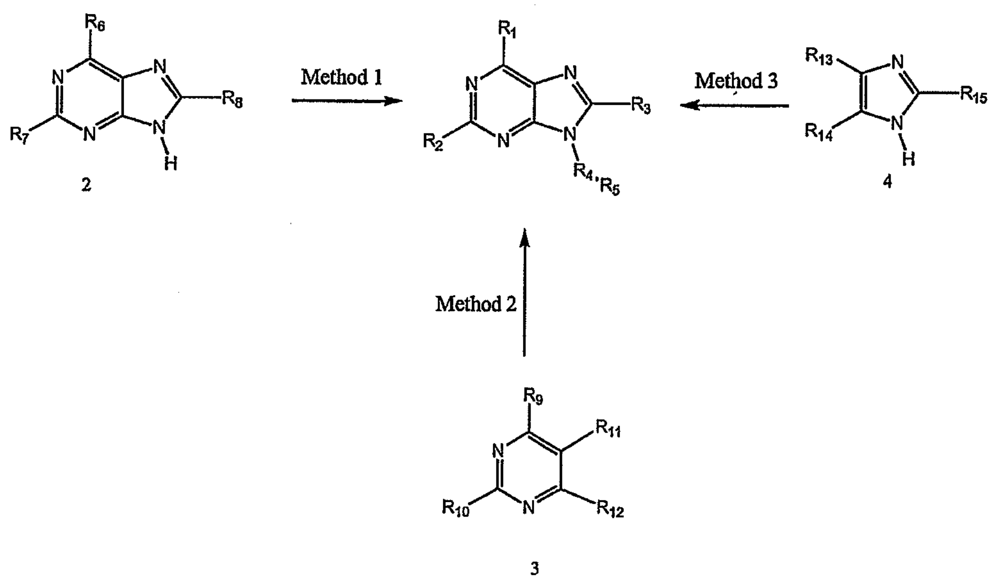


2

4

3

Scheme 11

The starting materials or the intermediates of the Formula 2, or/and 4 can exist in tautomeric forms as shown in Fig 4. Both forms are indiscriminately described in the specifications.

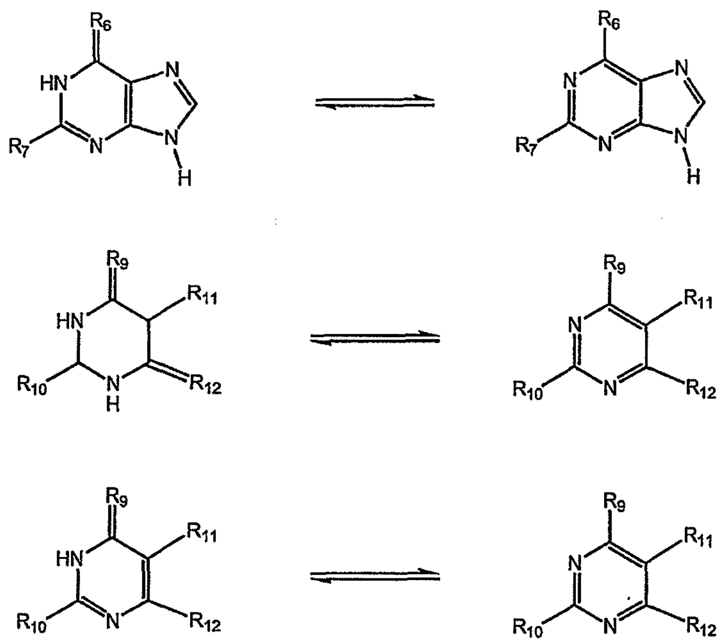

Fig 4

1. **Method 1: From purines:**

The compounds of Formula IIC (see, Scheme 12) can be synthesized from the commercially available starting heterocycle, for example compounds of Formula 2 where $R^6$ is -Cl, -Br or -OH, $R^7$ is $-NH_2$ and $R^8$ is -H are commercially available from Aldrich, AlfaAesar, *etc*. Accordingly, Formula 2 can be alkylated in the presence of a base such as $K_2CO_3$, NaH, $Cs_2CO_3$, DBU *etc.* with/without the presence of halide such as NaI, KI, $(Bu)_3NI$ *etc.*, and in a polar solvent such as DMF, THF, DMSO *etc.* using electrophiles such as $L^1$-$R^4$-$R^5$ where -$L^1$ is a leaving group. Leaving groups include

but are not

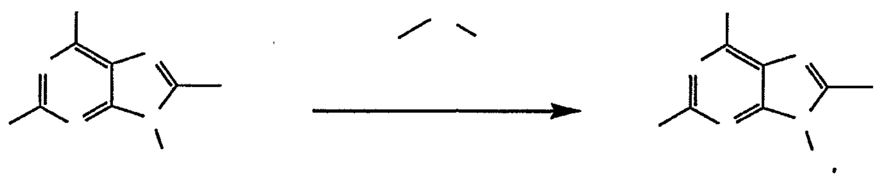

Scheme 12

limited to, e.g., halogen, triflate, tosylate , mesylate *etc.* See Kasibhatla, PCT publication number WO 03/037860. Compounds of Formula I, wherein $R^1$ is -OH can be converted to halides using standard conditions $POCl_3$, $POBr_3$ *etc.* with/without the presence of base such as $Et_3N$, N,N-diethylaniline, (i-pr)$_2$NEt *etc.* in polar solvents such as $CH_3CN$, $CH_2Cl_2$ *etc.*

The compounds of Formula IIC, wherein $R^1$ is -$OR^{11}$, -$SR^{11}$, or -$NHR^8$ where $R^{11}$ is alkyl, $R^8$ is hydrogen, lower alkyl, lower aryl, or -C(O)$R^9$, where $R^9$ is lower alkyl, lower aryl, lower heteroaryl, -$NR^{10}R^{10}$, or -$OR^{11}$, where $R^{10}$ is independently hydrogen or lower alkyl, can be prepared from compounds of Formula IIC wherein $R^1$ is halogen reacting with $HOR^{11}$, $HSR^{11}$ or $NH_2R^8$ in presence of a base such as $K_2CO_3$ or NaH and a polar solvent such as DMF or THF. Compounds of Formula IIC where $R^8$ is -C(O)$R^9$ can be prepared when $R^1$ is hydroxyl by simple acylation.

Compounds of Formula IIC where $R^1$ is alkyl can be prepared from compounds of Formula II where $R^1$ is halogen and trialkyl aluminum or dialkyl zinc. (See Holy, J. Med. Chem. 1999, 42, 2064).

$R^5$ especially when it is aryl or heteroaryl, can be further modified as needed, for example by halogenation, nitration, palladium coupling of halogen, Friedel-Crafts alkylation/acylation, *etc.* or these modifications can also be done before alkylation, see Jerry March, *Advanced Organic Chemistry.* The heteroaromatic rings can also be oxidized to their corresponding N-oxides using various oxidizing agents such as $H_2O_2$, $O_3$, MCPBA *etc.* in polar solvents such as $CH_2Cl_2$, $CHCl_3$, $CF_3COOH$ *etc.* See Jerry March, Advanced Organic Chemistry, 4th edition, Chapter 19.

Compounds of Formula IIC where $R^3$ is halogen, can be prepared from Formulae 1 or 2 using halogenating agents such as $Br_2$, NBS, NCS, NIS *etc.* in polar solvents such as DMF, water, or suitable buffer solution. See Herdewijn, J. Med. Chem. 1995, 38, 3838. Alternatively, compounds of Formula 2 where $R^8$ is iodo can also be made using procedures known in the literature, *e.g.*, Burger, J. Org. Chem. 2000, 65, 7825. These can be further modified as needed; for example, where $R^3$ is -$N_3$, or -CN by reacting with an azide such as $NaN_3$, $LiN_3$ *etc.* or cynide such as KCN or NaCN in polar solvents such as DMF, DMSO *etc.* See Halbfinger, J. Med. Chem. 1999, 42, 1625; Jacobson, J. Med. Chem. 1999, 42, 5325.

## 2. <u>Method 2: From Pyrimidines</u>

Compounds of Formula IIC can also be prepared from the substituted pyrimidines of Formula 5 (see, Scheme 13). Accordingly, reaction of commercially available compounds of Formula 5, where $R^{16}$ is hydrogen or -$NO_2$, (*see* J. Chem. Soc. 1962, 4186, for the preparation of $R^{16}$ = -$NO_2$ compound) with $NH_2$-$R^4$-$R^5$ in solvents such as EtOH, BuOH *etc.* in presence

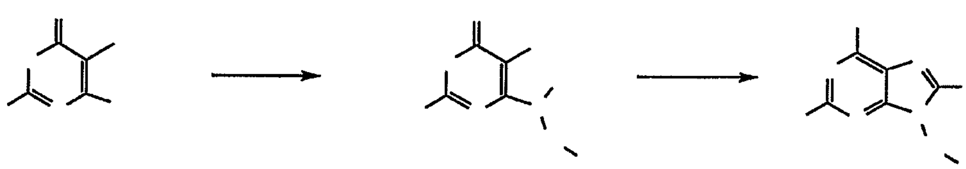

Scheme 13

of organic bases such as $Et_3N$, $(i\text{-}pr)_2NEt$ *etc.* followed by nitrosation (when $R^{16}$ = -H) using nitrous acid, then reduction with sodium dithionite or Zn/HCOOH *etc.* of Formula 6 ($R^{16}$ = -NO or $-NO_2$) to yield compounds of Formula 6 where $R^{16}$ is $-NH_2$. Condensation of Formula 6, where $R^{16}$ is $-NH_2$ using standard conditions such as triethylorthoformate, formic acid, cyanogenbromide *etc.* as described in J. Chem. Soc. 1963, 4186; Sircar, U.S. Patent 4,748,177, 1988; and Dang, WO 98/39344, followed by reaction with $POCl_3$ to give compounds of Formula II, Scheme 13. These compounds of Formula IIC can be further modified as necessary.

Similarly, compounds of Formula IIC can also be synthesized from Formula 7, 2-amino-4, 6-dichloro pyrimidine (see Scheme 14). Reaction of Formula 7 with $NH_2$-$R^4$-$R^5$ in solvents such as EtOH, BuOH *etc.* in presence of organic bases such as $Et_3N$, $(ipr)_2Net$, *etc.* followed by reaction with diazonium salt prepared from 4-chloroaniline

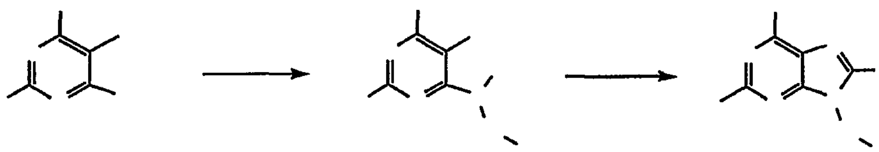

Scheme 14

and $NaNO_2$ in aq. HCl to give compound Formula 8, where $R^{16}$ is azo-(4-chlorobenzene). Reduction of the azo compound with zinc in acetic acid to give compounds of Formula 8, where $R^{16}$ is $-NH_2$. (See, Meier, U.S. Patent 5,204,353 (1993)). Condensation of these compounds using standard conditions such as triethylorthoformate, formic acid, cyanogenbromide *etc.* as described in J. Chem. Soc. 1963, 4186; Sircar, U.S. Patent 4,748,177, 1988, and Dang, WO 98/39344, followed by reaction with $POCl_3$ to give compounds of Formula IIC, Scheme 14. These compounds of Formula IIC can be further modified as necessary.

Likewise, compounds of Formula 8 where $R^{16}$ is $NH_2$ can be made from the commercially available 2,5-diamino-4,6-dihydroxy pyrimidine as described in Daluge, U.S. Patent 5,917,042 (1999), See Scheme 15.

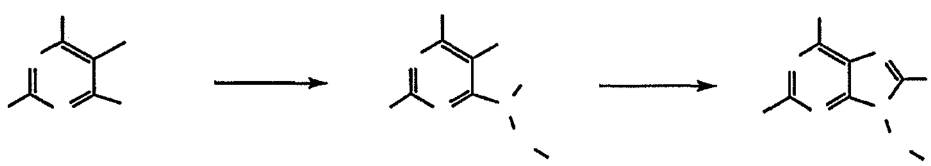

Scheme 15

### 3. <u>Method 3: From imidazoles</u>

Compounds of Formula IIC can also be prepared from the substituted imidazoles as shown in Scheme 6. Accordingly, compounds of Formula 4, wherein $R^{14}$ is $NH_2$, $R^{13}$ is $C(O)NH_2$ and $R^{15}$ is H, can be alkylated in the presence of a base such as KOH, NaOH, $K_2CO_3$, NaH, $Cs_2CO_3$, DBU *etc.* with/without the presence of halide such as NaI, KI, $(Bu)_3NI$ *etc.,* and in a polar solvent such as DMF, THF, DMSO *etc.* using electrophiles such as $L^1$-$R^4$-$R^5$ where $L^1$ is a leaving group. Leaving groups include but are not limited to, e.g., halogen, triflate, tosylate, mesylate *etc.* to give compounds of Formula 10. The ring closure can be achieved using many methods reported in the literature, Alhede, J. Org. Chem., 1991, 2139 and references cited therein to give guanines of Formula II, wherein $R^1$ is OH. These compounds can be converted to the

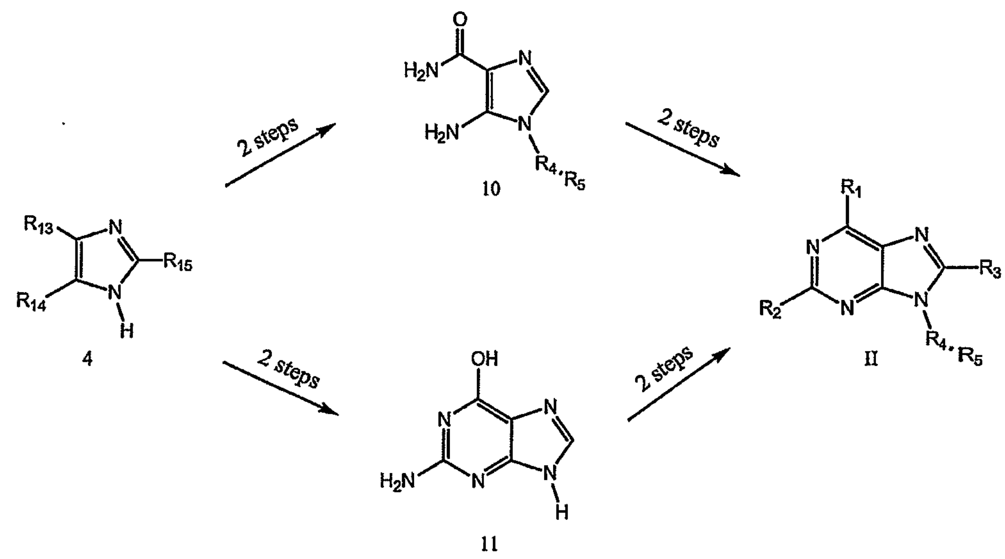


4

10

11

II

Scheme 16

compounds of Formula II, wherein $R^1$ is Cl using $POCl_3$ as described earlier. Advantageously, these steps can be reversed as shown in Scheme 16 via Formula 11. Alternately, we can also construct these 2-aminopyrimidine rings from Formula 4,

wherein $R^{14}$ is -OH or halide, $R^{13}$ is -C(O)OEt and $R^{15}$ is -H by reacting with guanidine hydrochloride as described in Chowdhury, J. Med. Chem. 1999, 42, 4300.

Preparation of electrophiles $L^1$-$R^4$-$R^5$ wherein $L^1$ is a leaving group and nucleophiles

$NH_2$-$R^4$-$R^5$.

Synthesis of benzyl type electrophile:

Benzyl type electophiles (Fig. 2 supra) can be prepared as described above in Section III.A.2.2.1 using various methods reported in the literature, see Jerry March, Advanced Organic Chemistry, 4th edition; Larock, Comprehensive Organic Transformations, 1989,VCH, New York. For example the $L_1$ is -Br can be prepared by reduction followed by halogenation of the benzoic acid or aldehyde derivatives. These benzyl derivatives can also be prepared by benzylic oxidation or benzylic halogenation. Further modification of the benzyl ring can be done before or after the corresponding amines where $L_1$ is -$NH_2$ can be prepared from the compounds where $L_1$ is leaving group such as chloride, bromide, tosylate, mesylate *etc.* using ammonia.

Synthesis of pyridyl methyl type electrophile:

Pyridyl methyl type electophiles can be prepared from many methods reported in the literature including those identified in Section III.A.2.2.2.

Further modification of the pyridyl ring can be done after the purine alkylation see Scheme 16.

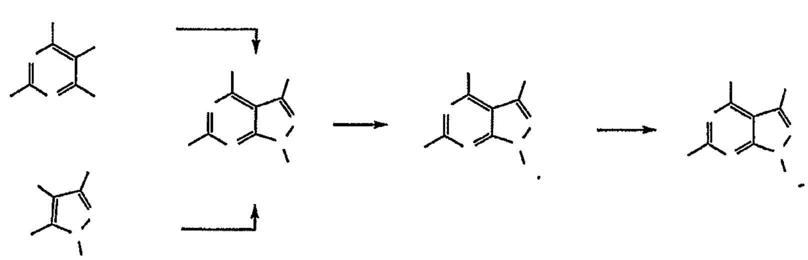

**D. Synthesis of Compounds of Formula III (Pyrazolopyrimidines and Related Analogs)**

The compounds the present invention may be synthesized by various methods known in the art, including those described in, for example, Gillespie, PCT publication No. WO 02/055082; Dempcy, US publication No. US 2003/0078413 A1. For the synthesis of compounds of Formulae III, IIIA and IIIB, a general strategy is outlined in Scheme 17 and consists of three parts: (1) constructing the bicyclic system, starting from either a pyridine or a pyrazole, (2) appending the -$R^4$-$R^5$ group, and (3) further elaborating the ring systems.

Importantly, one skilled in the art will recognize that the sequence of events is not necessarily (1)-(2)-(3), and that these events may be interchanged, provided there be no incompatibility between the reagents and the functional groups specific to the point in case.

Scheme 17

Also, the starting materials and the intermediates of the Formula 1, 2, 3, or 4 can exist in tautomeric forms as shown in Fig 5, and both forms are indiscriminately used in this patent.

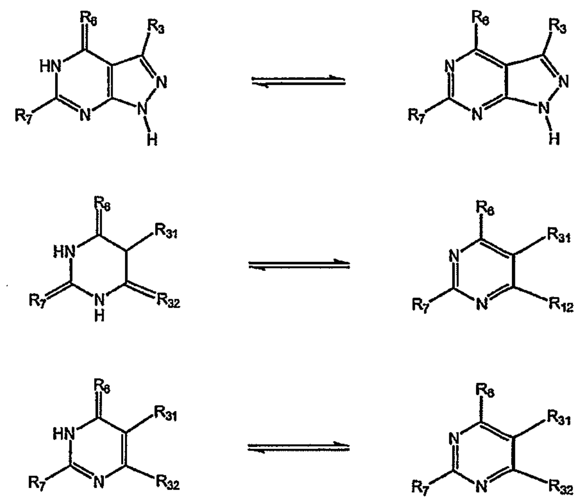

Fig 5

**1. Assembly of the pyrazolo[3,4-d]pyrimidine**

1.1 Assembly of the pyrazolo[3,4-d]pyrimidine starting from a pyrimidine

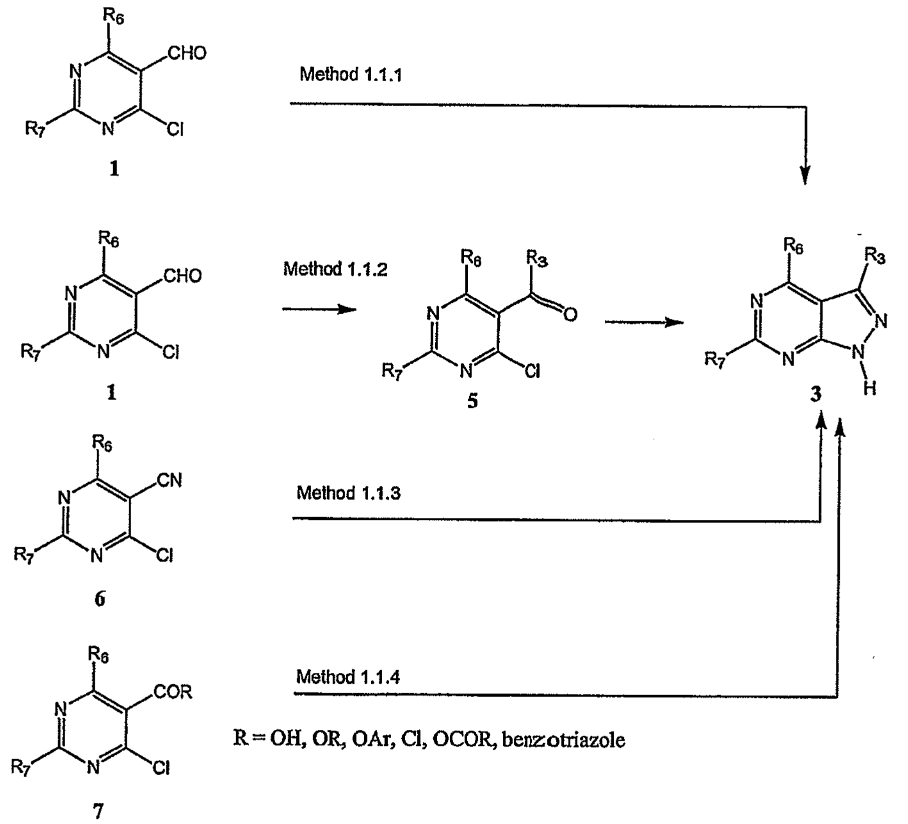


Scheme 18

The compounds of Formula 3 can be prepared from pyrimidines as outlined in Scheme 18. For instance:

Method 1.1.1

The compound of Formula 3, wherein $R^6$ is -Cl, $R^7$ is $-NH_2$, and $R^3$ is -H, is readily prepared by treating 2-amino-4,6-dichloro-pyrimidine-5-carbaldehyde (Formula 1) with hydrazine, see, F. Seela, Heterocycles 1985, 23, 2521; F. Seela, Helv. Chim. Acta 1986, 69, 1602; and R. O. Dempcy, WO 03/022859.

Method 1.1.2

The compounds of Formula 3, wherein $R^6$ is Cl, $R^7$ is $NH_2$ and $R^3$ is alkyl, aryl, or heteroaryl have not been previously reported. They can be made by converting a compound of Formula 1 to a compound of Formula 5 in two steps: i) Nucleophilic addition to the carbonyl group; and ii) Oxidation of the resulting alcohol. In a subsequent step, the compound of Formula 5 is converted to the compound of Formula 3 by reaction with hydrazine, or an equivalent thereof.

Method 1.1.3

The compounds of Formula 3 wherein $R^3$ is $NH_2$ can be obtained by treatment of a nitrile of Formula 6 with hydrazine (See A. M. El-Reedy, Phosph, Sulf, Silic, 1989, 42, 231).
The compounds of Formula 3 wherein $R^3$ is OH can be obtained by treatment of a nitrile of Formula 6 with hydrazine followed by hydrolysis (See Ciba, Patent UK 884,151 (1961)).

Method 1.1.4

The compounds of Formula 3 wherein $R^3$ is OH can be obtained by treatment of an acid, ester, or activated ester (or equivalent thereof) of Formula 7 with hydrazine (Ciba, Patent UK 884,151 (1961)).

**1.2. Assembly of the pyrazolo[3,4-d]pyimidine starting from a pyrazole**

The compounds of Formula 3 can also be made from pyrazoles of Formula 2 (Scheme 19). There are a variety of methods by which the 6-membered ring can be formed (*e.g.* R J. Bontems, J. Med. Chem, 1990, 33, 2174 and references therein). For instance:

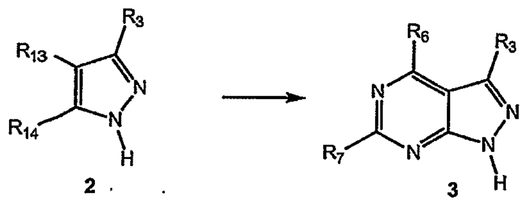

2 3

Scheme 19

Compounds of Formula 2 wherein $R^{13}$ is $-CONH_2$ and $R^{14}$ is $NH_2$ can be treated with Ph-CO-NCS to give compounds of Formula 3 in which $R^6$ is OH and $R^7$ is $NH_2$ (F. Babin, J. Heterocycl. Chem. 1983, 20, 1169.)

Compounds of Formula 2 wherein $R^{13}$ is -CN and $R^{14}$ is $NH_2$ can be treated with thiourea or guanidine to give compounds of Formula 3 in which $R^6$ is $NH_2$ and $R^7$ is $NH_2$ (H. Kosaku, Heterocycles, 2001, 55, 2279).

Compounds of Formula 2 wherein $R^{13}$ is $-CONH_2$ and $R^{14}$ is $NH_2$ can be treated with $CS_2$ or $EtOCS_2K$ to give compounds of Formula 3 in which $R^6$ is OH and $R^7$ is SH (S. M. Bennett, J. Med. Chem. 1990, 33, 2162).

**2. Incorporation of the $-R^4$-$R^4$ fragment**

2.1. Alkylation of compounds of Formula 3

Compounds of Formula 3 can be alkylated in the presence of a base such as $K_2CO_3$, NaH, $Cs_2CO_3$, DBU *etc.* with/without the presence of a catalyst such as NaI, KI, $(Bu)_3NI$ *etc.,* and in a polar solvent such as DMF, THF, DMSO *etc.* using electrophiles such as $L^1$-$R^4$-$R^5$ where $L^1$ is a leaving group (See Scheme 20). Leaving groups include but are not limited to, e.g., halogen, triflate, tosylate, mesylate, triphenylphosphonium (generated under Mitsunobu conditions, *e.g.* $PPh_3$/DEAD) *etc.* (See Kasibhatla, WO 03/037860.)

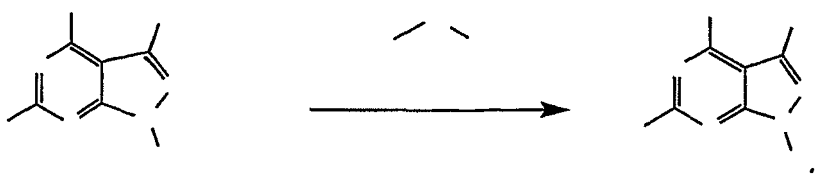

Scheme 20

2.2. Preparation of electrophiles $L_1$-$R^4$-$R^5$ wherein $L_1$ is a leaving group and of nucleophiles $NH_2$-$R^4$-$R^5$.

2.2.1. Synthesis of benzyl type electrophile:

Benzyl type electrophile, (Fig. 2, supra). The corresponding amines where $L^1$ is $NH_2$ can be prepared by a variety of methods, for instance from compounds where $L^1$ is leaving group such as chloride, bromide, tosylate, mesylate, *etc.* using ammonia, or with sodium azide followed by hydrogenation.

2.2.2. Synthesis of pyridyl methyl type electrophile:

Pyridyl methyl type electrophiles (Fig. 3 supra) can be prepared from many methods reported in the literature, including those identified in Section III.A.2.2.2.

## 3. Further Elaboration of the ring systems

3.1. Functional group interconversions of $R^1$

Compounds of Formula IIIA, wherein $R^1$ is -OH, can be converted to halides using standard conditions $POCl_3$, $POBr_3$ *etc,* with/without the presence of base such as $Et_3N$, N,N-dimethylaniline, $(i\text{-}Pr)_2NEt$ *etc.* and with/without a catalyst such as $BnEt_3N^+Cl^-$, in polar solvents such as $CH_3CN$, $CH_2Cl_2$ *etc.* Related methods include, but are not limited to, $SOCl_2$/DMF (M. J. Robins , Can. J. Chem. 1973, 12, 3161), $PPh_3/CCl_4$ (L. De Napoli, J. Chem. Soc. Perkin Trans 1, 1994, 923), HMPT/$CCl_4$ or HMPT/NBS (E. A. Veliz, Tetrahedron Lett, 2000, 41, 1695) or $PPh_3/I_2$ (X. Lin , Org. Letters, 2000, 2, 3497).

Compounds of Formula IIIA, wherein $R^1$ is $-NH_2$, can be converted to halides by a Balz-Schiemann (F) or Sandmeyer reaction (Cl, Br, I) by means of a nitrosylating agent *(e.g.* $NaNO_2/H^+$, $NOBF_4$, RONO) and a halogen donor (*e.g.* $BF_4^-$, $CuX_2$, $SbX_3$) where X is a halogen.

Compounds of Formula IIIA, wherein $R^1$ is alkyl can be prepared from compounds of Formula 3 where $R^1$ is halogen and trialkyl aluminum or dialkyl zinc (A. Holy, J. Med. Chem. 1999, 42, 2064).

Compounds of Formula IIIA, wherein $R^1$ is a halide can be converted to compounds wherein $R^1$ is $-NH_2$, -OH, -SH, -OR, -SR with standard reagents, *e.g.* $NH_3$, NaOH, thiourea, $RO^-$, $RS^-$, with or without a catalyst (*e.g.* Pd, Ni, Cu, Lewis acid, $H^+$),
wherein R is lower alkyl.

3.2. Functional group interconversions of $R^2$

Compounds of Formula IIIA, wherein $R^2$ is $-NH_2$ can be temporarily protected, *e.g.* as an amide ($Ac_2O$, PivCl, $(tBoc)_2O$) or a formamidine (DNW-DUA).

Compounds of Formula IIIA, wherein $R^2$ is $-NH_2$ can be converted to halides by a Balz-Schiemann (F) or Sandmeyer reaction (Cl, Br, I) by means of a nitrosylating agent (*e.g.* $NaNO_2/H^+$, $NOBF_4$, RONO) and a halogen donor (*e.g.* $BF_4^-$, $CuX_2$, $SbX_3$) where X is a halogen.

Compounds of Formula IIIA, wherein $R^2$ is a halide can be converted to compounds wherein $R^2$ is $-NH_2$, -OH, -SH, $-OR^8$, $-SR^8$ with standard reagents, *e.g.* $NH_3$, NaOH, thiourea, $R^8O^-$, $R^8S^-$, with or without a catalyst (*e.g.* Pd, Ni, Cu, Lewis acid, $H^+$).

Compounds of Formula I, wherein $R^2$ is -SH can be converted to halides ($Br_2$). They can also be oxidized (*e.g.* $H_2O_2$) and treated with ammonia to give a $-NH_2$ group (S. M. Bennett, J. Med. Chem. 1990, 33, 2162).

Compounds of Formula IIIA, wherein $R^2$ is a sulfide, *e.g.* MeS-, can be converted to a sulfone, eg. $MeSO_2^-$, and displaced with a nucleophile, *e.g.* $NH_3$ or $NH_2$-$NH_2$, $N_3$-, CN-.

3.3. Functional group interconversions of $R^3$:

Compounds of Formula IIIA, wherein $R^3$ is H, can be converted to compounds of Formula IIIA wherein $R^3$ is a halogen (*e.g.* NCS, NBS, NIS, $Br_2$, ICl, $I_2$/KOH) (See F. Seela et al, Helv. Chim. Acta 1999, 82, 105).

Compounds of Formula IIIA, wherein $R^3$ is a halogen, can be functionalized by Pd-calayzed reactions ((a) Sonogashira coupling: E. C. Taylor et al, Tetrahedron, 1992, 48, 8089. (b)carboxylation: J. W. Pawlik, J. Heterocycl. Chem. 1992, 29, 1357 (c) Suzuki coupling: T. Y. I Wu, Org. Lett., 2003, 5, 3587) or by addition of nucleophiles (*e.g.* hydrazine, B. M. Lynch, Can. J. Chem. 1988, 66, 420)

Compounds of Formula I, wherein $R^3$ is a halide, can be converted to compounds
wherein $R^3$ is $-NH_2$, -OH, -SH, $-OR^8$, $-SR^8$ with standard reagents, *e.g.* $NH_3$, NaOH, thiourea, $R^8O$-, $R^8S^-$, with or without a catalyst (*e.g.* Pd, Ni, Cu, Lewis acid, $H^+$)

Compounds of Formula IIIA, wherein $R^3$ is MeO can be demethylated to provide compounds of Formula IIIA, wherein $R^3$ is OH (J. D. Anderson, J. Heterocycl. Chem., 1990 27, 439).

3.4. Further elaboration of $R^5$:

$R^5$ especially when it is aryl or heteroaryl, can be further modified as needed, for example by halogenation, nitration, palladium coupling of halogen, Friedel-Crafts alkylation/acylation, *etc.* or these modifications can also be done

before alkylation, see Jerry March, *Advanced Organic Chemistry.* The heteroaromatic rings can also be oxidized to their corresponding N-oxides using various oxidizing agents such as $H_2O_2$, $O_3$, MCPBA *etc.* in polar solvents such as $CH_2Cl_2$, $CHCl_3$, $CF_3COOH$ *etc.* See Jerry March, *Advanced Organic Chemistry,* 4th edition, Chapter 19. Examples of modifications are suggested in Scheme 21.

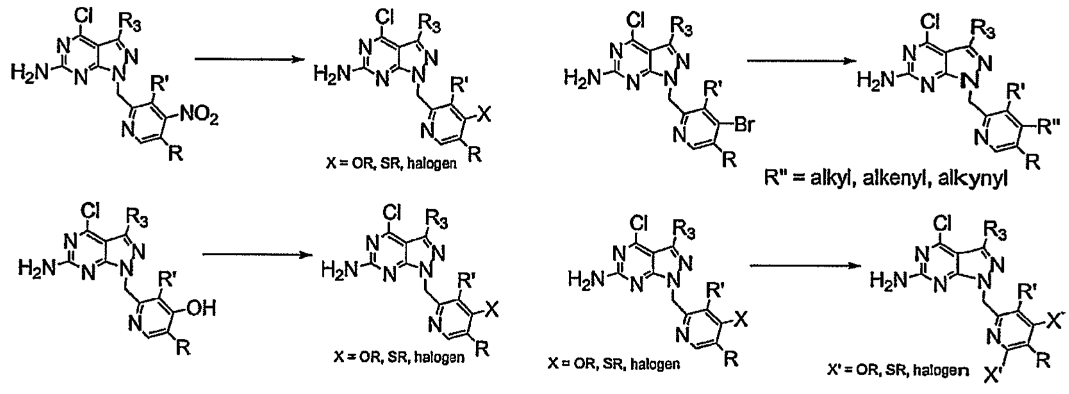


Scheme 21

## 4. Permutations of the order of events

As mentioned above, the events (1) assemby of the bicyclic system (2) appendage of the $R^5$-$R^4$- moiety, and (3) further elaboration of the ring systems do not necessarily have to be made in the sequence of (1)-(2)-(3), and it may be beneficial to proceed in a different sequence.

For illustrative purposes, Scheme 6 shows a putative synthesis in which the order of events is not (1)-(2)-(3), but is (1)-(3)-(2).

First the bicyclic system is prepared, then it is elaborated, and finally $R^5$ is appended via an alkylation.

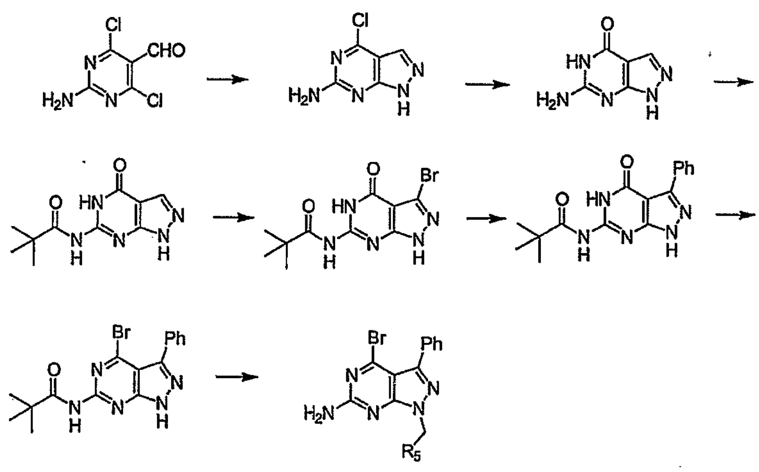

Scheme 22

For illustrative purposes, Scheme 23 shows a putative synthesis in which the order of events is not (1)-(2)-(3), but is (2)-(1)-(3). First the $R^5$ group is appended to a pyrimidine via an aromatic nucleophilic substitution, then the bicyclic ring system is constructed, and finally a series of functional group interconversions yields the compound of Formula IIIA.

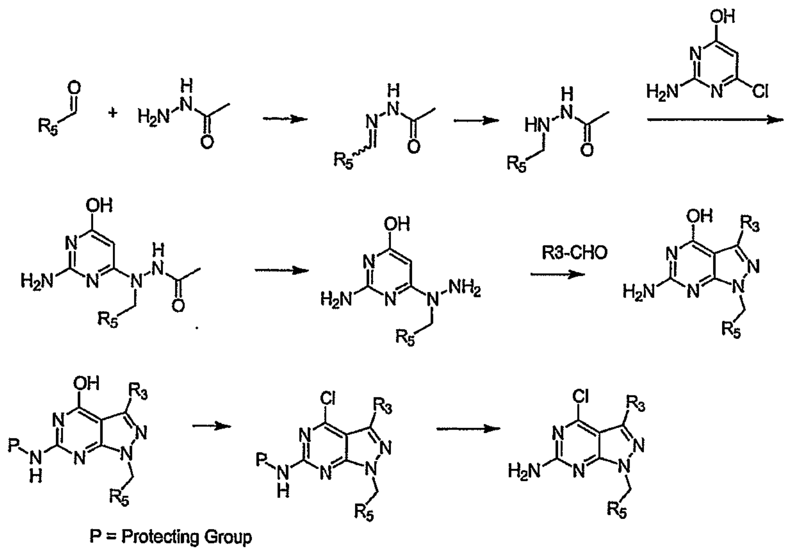


Scheme 23

Also, if $R^5$ is, for instance, a pyridine, it can be converted to a N-oxide either before or after alkylation.

E. **Synthesis of Compounds of Formula IV (Triazolopyrimidines and Related Analogs)**

The compounds of Formula IV and IVA (see Scheme 24) of the present invention may be synthesized by various methods known in the art, including those described in, for example, Parkanyi, J. Heterocyc. Chem., 1990, 27 (5), 1409-13; Beauchamp, U.S. Patent 4,714,701,1987; Meier, U.S. Patent 5,204,353, 1993. Gillespie, WO 02/055083; Peterson, J. Med. Chem., 1990, 33(4), 1214-19. The general synthetic strategy is outlined in Scheme 1 and consists of three parts: (1) constructing the bicyclic system, starting from either a pyrimidine or a 1,2,3-triazole, (2) appending the $R^5$-$R^4$- group, and (3) further elaborating the ring systems.

Importantly, one skilled in the art will recognize that the sequence of events is not necessarily (1)-(2)-(3), and that these events may be interchanged, provided there be no incompatibility between the reagents and the functional groups specific to the point in case.

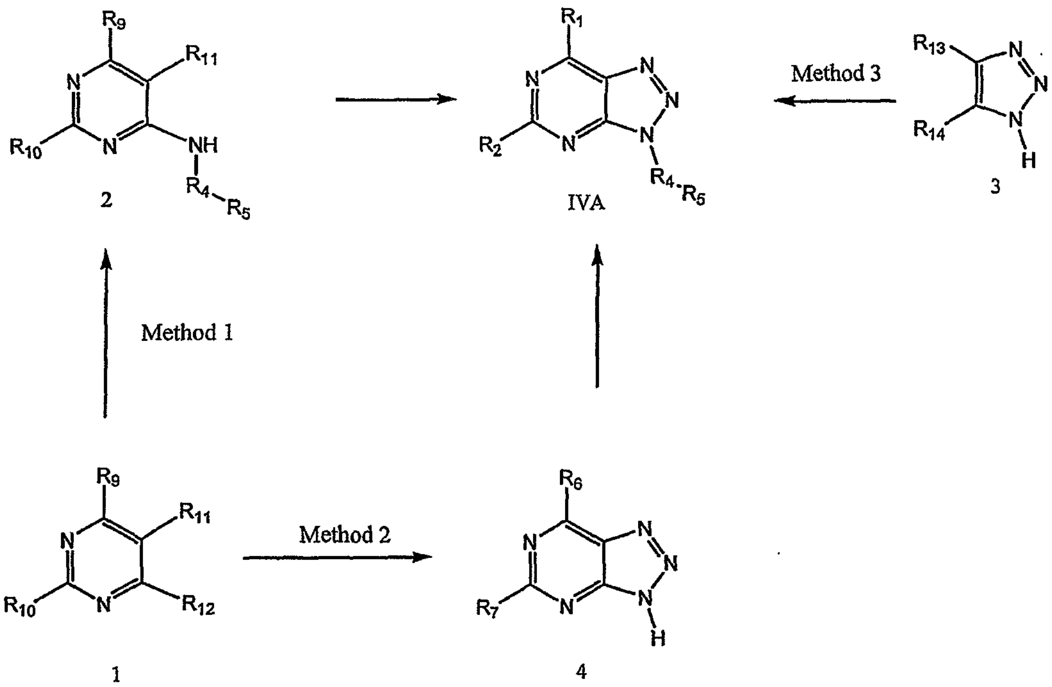


Scheme 24

The starting material and/or the intermediates of, e.g., Formulae 1, 2 or/and 4 can exist in tautomeric forms, and both forms are indiscriminately described in the specifications.

**1. <u>Synthesis of Compounds of Formula IV From Pyrimidines</u>**

1.1 <u>Method 1</u>

Compounds of Formula IVA can be prepared from the commercially available substituted pyrimidines compounds of Formula 1 where $R^9$ is -OH or halogen, $R^{10}$ is amino or protected amino or any group that can be converted to amino, such as SMe, $R^{11}$ is H or $-NO_2$, $R^{12}$ is -Cl, (see Scheme 25) by treating with an excess halogenating agent such as $POCl_3$, oxalyl chloride, or $PCl_5$, and a formulating agent such as DMF to give compounds of Formula IVA where $R^9$ is halogen, and $R^{12}$ is halogen, followed by halogen displacement with an nucleophile, such as $NH_2$-$R^4$-$R^5$, in solvents such as EtOH, tBuOH *etc.* in presence of organic bases such as $Et_3N$, $(i\text{-}pr)_2NEt$ *etc.* to yield a compound of Formula 2. Formula 2, where $R^{11}$ is $-NO_2$ may then be reduced with zinc and formic acid or sodium dithionite to give compounds of Formula 2 where $R^{11}$ is $-NH_2$, see Dempcy, U.S. Publication No. 2003/0078413 A1. Compounds of Formula IVA can then be prepared by diazotization with an alkali metal nitrite such as $NaNO_2$ in inorganic acids such as HCl, followed by *in situ* cyclization. See Beauchamp, U.S. Patent 4,714,701; Meier, U.S. Patent 5,204,353. These compounds of Formula IVA can be further modified as necessary.

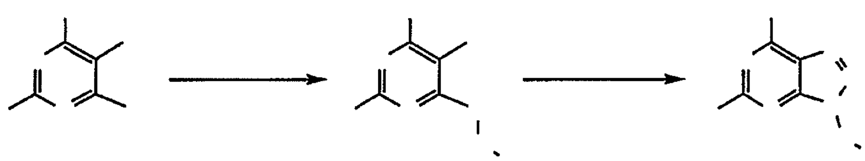

Scheme 25

Formula 2, where $R^{11}$ is H, can be treated with diazonium salts such as 4-chloroaniline diazonium salt prepared

from 4-chloroaniline and $NaNO_2$ inorganic acids such as HCl to give pyrimidine 5-azo- analog, that can be reduced with zinc dust in EtOH/AcOH (1:1) solution to give compounds of Formula 2, where $R^{11}$ is $-NH_2$, see Meier, U.S. Patent 5,204,353.

1.2 Method 2

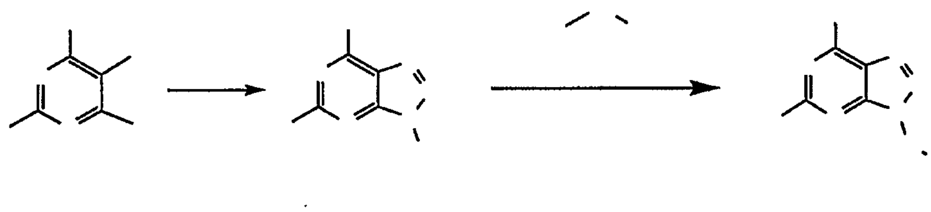

Scheme 26

Also compounds of Formula IVA can be prepared from the commercially available substituted diamino pyrimidines compounds of Formula 1 where $R^9$ is -OH or halogen, $R^{10}$ is amino or protected amino or any group that can be converted to amino, such as SMe, $R^{11}$ & $R^{12}$ are $-NH_2$, (see Scheme 26) following the diazotization method described earlier in Method 1 to give compounds of Formula 4. Formula 4 can be alkylated in the presence of a base such as $K_2CO_3$, NaH, $Cs_2CO_3$, DBU *etc.* with/without the presence of halide such as NaI, KI, $(Bu)_3NI$ *etc.,* and in a polar solvent such as DMF, THF, DMSO *etc.* using electrophiles such as $L^1$-$R^4$-$R^5$ where $L^1$ is a leaving group. Leaving groups include but are not limited to, *e.g.,* halogen, triflate, tosylate, mesylate *etc.* See Kasibhatla, PCT WO 03/037860. Compound of Formula I can also be prepared from compounds of Formula D using Mitsunobu alkylation conditions using $L^1$-$R^4$-$R^5$ where $L^1$ is hydroxyl. See Kozai, Chem. Pharm. Bull., 1999, 47(4), 574-575).

**2. Synthesis of Compounds of Formula IV From Triazole**

Compounds of Formula IVA can also be prepared from the substituted triazole as shown in Scheme 27. Accordingly, compounds of Formula 3, wherein $R^{14}$ is $NH_2$, $R^{13}$ is $-C(O)NH_2$ and $R^{15}$ is H (commercially available), can be alkylated in the presence of a base such as KOH, NaOH, $K_2CO_3$, NaH, $Cs_2CO_3$, DBU *etc.* with/without the presence of halide such as NaI, KI, $(Bu)_3NI$ *etc.,* and in a polar solvent such as DMF, THF, DMSO *etc.* using electrophiles such as $L^1$-$R^4$-$R^5$ where $L^1$ is a leaving group. Leaving groups include but are not limited to, e.g., halogen, triflate, tosylate, mesylate *etc.* to give compounds of Formula 6. The ring closure can be achieved using many methods reported in the literature, Alhede, J. Org. Chem., 1991, 2139 and references cited therein to give compounds of Formula I, wherein $R^1$ is -OH. These compounds can be converted to the compounds of Formula I, wherein $R^1$ is -Cl using $POCl_3$ as described earlier. Alternately, we can also construct from Formula 3, wherein $R^{14}$ is -OH or halide, $R^{13}$ is -C(O)OEt by reacting with guanidine hydrochloride as described in Chowdhury, J. Med. Chem. 1999, 42, 4300.

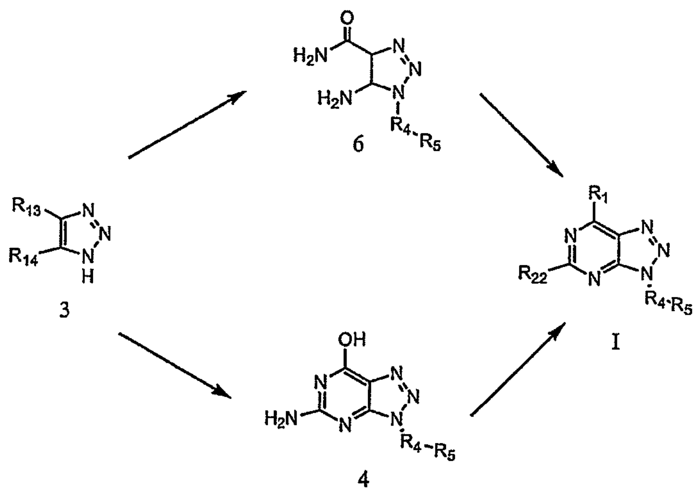

3 6 4 I

Scheme 27

### 3. Preparation of electrophiles $L^1$-$R^4$-$R^5$ wherein $L^1$ is a leaving group and nucleophiles $NH_2$-$R^4$-$R^5$

Benzyl type electophiles (Fig. 2 supra) can be prepared as described above in Section III.A.2.2.1

Synthesis of pyridyl methyl type electrophile:

Pyridine methyl type electrophile (Fig. 3 supra) can be prepared from many methods reported in the literature including those identified in Section III.A.2.2.2.

The compound $R^4$-$R^5$-$NH_2$ is obtained by treating $R^4$-$R^5$-$L^1$ with ammonia at temperatures of 20-160 ˚C in a pressure vessel, wherein $L^1$ is leaving group such as chloride, bromide, tosylate, mesylate *etc.* using ammonia, or with sodium azide followed by hydrogenation.

### 4. Further elaboration of the rime systems.

These modifications can be done at any stage depending upon the incompatibility of the functional groups present.

### 4.1 Functional group interconversions of $R^1$

Compounds of Formula IVA, wherein $R^1$ is -OH, can be converted to halides using standard conditions $POCl_3$, $POBr_3$ *etc.* with/without a base such as $Et_3N$, N,N-dimethylaniline, $(i\text{-}Pr)_2NEt$ *etc.* and with/without a catalyst such as $BnEt_3N^+Cl^-$, in polar solvents such as $CH_3CN$, $CH_2Cl_2$ *etc.* Related methods include, but are not limited to, $SOCl_2$/DMF (M. J. Robins, Can. J. Chem. 1973, 12, 3161), $PPh_3/CCl_4$ (L. De Napoli , J. Chem. Soc. Perkin Trans 1, 1994, 923), HMPT/$CCl_4$ or HMPT/NBS (E. A. Veliz, Tetrahedron Lett, 2000, 41, 1695) or $PPh_3/I_2$ (X. Lin, Org. Letters, 2000, 2, 3497).

Compounds of Formula IVA, wherein $R^1$ is $NH_2$, can be converted to halides by a Balz-Schiemann (F) or Sandmeyer reaction (Cl, Br, I) by means of a nitrosylating agent ($NaNO_2/H^+$, $NOBF_4$, RONO) and a halogen donor ($BF_4^-$, $CuX_2$, $SbX_3$).

Compounds of Formula IVA, wherein $R^1$ is alkyl can be prepared from compounds of Formula 4 where $R^1$ is halogen and trialkyl aluminum or dialkyl zinc (A. Holy, J. Med. Chem. 1999, 42, 2064).

Compounds of Formula IVA, wherein $R^1$ is a halide can be converted to compounds wherein $R^1$ is $-NH_2$, -OH, -SH, $-OR^8$, $-SR^8$ with standard reagents, *e.g.* $NH_3$, NaOH, thiourea, $R^8O^-$, $R^8S^-$, with or without a catalyst (*e.g.* Pd, Ni, Cu, Lewis acid, $H^+$) (*e.g.* B. G. Ugarkar, J. Med. Chem. 2000, 43, 2883-2893 and 2894-2905).

Compounds of Formula IVA, wherein $R^1$ is halogen or another leaving group can be treated with ammonia to provide compounds of Formula IVA wherein $R^1$ is $NH_2$ (F. Seela, Liebigs. Ann.Chem. 1985, 315)

### 4.2. Functional group interconversions of $R^2$

Compounds of Formula IVA, wherein $R^2$ is $-NH_2$ can be temporarily protected, *e.g.* as an amide ($Ac_2O$, PivCl),

a carbamate $(tBoc)_2O$) or amidine (DMF-DMA).

Compounds of Formula IVA, wherein $R^2$ is $NH_2$ can be converted to halides by a Balz-Schiemann (F) or Sandmeyer reaction (Cl, Br, I) by means of a nitrosylating agent ($NaNO_2/H^+$, $NOBF_4$, RONO) and a halogen donor ($BF4^-$, $CuX_2$, $SbX_3$ where Xis a halogen).

Compounds of Formula IVA, wherein $R^2$ is a halide can be converted to compounds wherein $R^2$ is $-NH_2$, -OH, -SH, $-OR^8$, $-SR^8$ with standard reagents, e.g. $NH_3$, NaOH, thiourea, $R^8O^-$, $R^8S^-$, with or without a catalyst (*e.g.* Pd, Ni, Cu, Lewis acid, $H^+$).

Compounds of Formula IVA, wherein $R^2$ is -SH can be converted to halides ($Br_2$). They can also be oxidized (*e.g.* $H_2O_2$) and treated with ammonia to give a $NH_2$ group (S. M. Bennett, J. Med. Chem. 1990, 33, 2162).

Compounds of Formula IVA, wherein $R^2$ is a sulfide, *e.g.* MeS-, can be converted to a sulfone, *e.g.,* $MeSO_2^-$, and displaced with a nucleophile, *e.g.* $NH_3$ or $NH_2$-$NH_2$, $N_3^-$, $CN^-$.

### 4.3 Further elaboration of $R^5$

$R^5$, especially when it is aryl or heteroaryl, can be further modified as needed, for example by halogenation, nitration, palladium coupling of halogen, Friedel-Crafts alkylation/acylation, *etc.* or these modifications can also be done before alkylation, see Jerry March, *Advanced Organic Chemistry.* The heteroaromatic rings can also be oxidized to their corresponding N-oxides using various oxidizing agents such as $H_2O_2$, $O_3$, MCPBA *etc.* in polar solvents such as $CH_2Cl_2$, $CHCl_3$, $CF_3COOH$ *etc.* See Jerry March, Advanced Organic Chemistry, 4th edition, Chapter 19. Examples of modifications are suggested in Scheme 28.

Also, if $R^5$ is for instance a pyridine, it can be converted to a N-oxide either before or after alkylation.

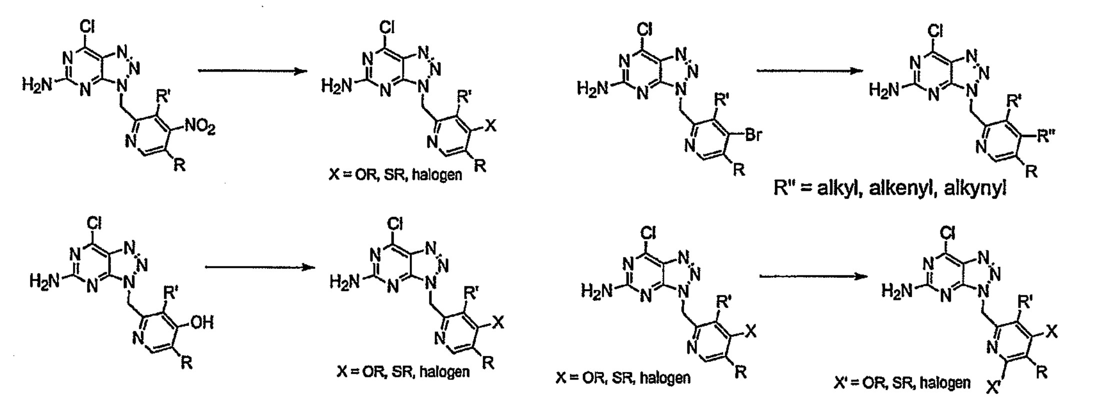


Scheme 28

## IV. PHARMACEUTICAL COMPOSITIONS, DOSAGING, AND MODES OF ADMINISTRATION

The present invention is directed to the clinical use of the heterocyclics, in particular, the pyrazolopyrimidines and their related analogs of Formulae A, I-IV, and their polymorphs, solvates, esters, tautomers, diastereomers, enantiomers, pharmaceutically acceptable salts and prodrugs thereof, for use in treatment or prevention of diseases that are HSP90-dependent. For example, a disorder such as inflammatory diseases, infections, autoimmune disorders, stroke, ischemia, cardiac disorder, neurological disorders, fibrogenetic disorders, proliferative disorders, tumors, leukemias, neoplasms, cancers, carcinomas, metabolic diseases, and malignant disease. The fibrogenetic disorders include but are not limited to scleroderma, polymyositis, systemic lupus, rheumatoid arthritis, liver cirrhosis, keloid formation, interstitial nephritis and pulmonary fibrosis.

The present invention features pharmaceutical compositions comprising the compound of Formulae A, I-IV, or a polymorph, solvate, ester, tautomer, enantiomer, diastereomer, pharmaceutically acceptable salt thereof, or prodrug thereof, of any of the preceding aspect and embodiments and one or more pharmaceutical excipients.

Those of ordinary skill in the art are familiar with formulation and administration techniques that can be employed with the compounds and methods of the invention, e.g., as discussed in Goodman and Gilman, The Pharmacological Basis of Therapeutics, (current edition), Pergamon; and Remington's, Pharmaceutical Sciences (current edition), Mack Publishing Co., Easton, PA.

The compounds utilized in the methods of the instant invention may be administered either alone or in combination with pharmaceutically acceptable carriers, excipients or diluents, in a pharmaceutical composition, according to standard pharmaceutical practices. The compounds can be administered orally or parenterally, including the intraventous, intramuscular, intraperitoneal, subcutaneous, rectal and topical routes of administration.

For example, the therapeutic or pharmaceutical compositions of the invention can be administered locally to the area in need of treatment. This may be achieved by, for example, but not limited to, local infusion during surgery, topical application, *e.g*., cream, ointment, injection, catheter, or implant, said implant made, *e.g.,* out of a porous, non-porous, or gelatinous material, including membranes, such as sialastic membranes, or fibers. The administration can also be by direct injection at the site (or former site) of a tumor or neoplastic or pre-neoplastic tissue.

Still further, the compounds or compositions of the invention can be delivered in a vesicle, *e.g*., a liposome (see, for example, Langer, Science 1990, 249, 1527-1533; Treat et al., Liposomes in the Therapy of Infectious Disease and Cancer, Lopez-Bernstein and Fidler, Ed., Liss, N.Y., pp. 353-365, 1989).

The compounds and pharmaceutical compositions used in the methods of the present invention can also be delivered in a controlled release system. In one embodiment, a pump may be used (see, Sefton, 1987, CRC Crit. Ref. Biomed. Eng. 14:201; Buchwald et al. Surgery, 1980, 88, 507; Saudek et al. N Engl. J. Med. 1989, 321, (574). Additionally, a controlled release system can be placed in proximity of the therapeutic target. (See, Goodson, Medical Applications of Controlled Release, 1984, 2, 115-138).

The pharmaceutical compositions used in the methods of the instant invention can also contain the active ingredient in a form suitable for oral use, for example, as tablets, troches, lozenges, aqueous or oily suspensions, dispersible powders or granules, emulsions, hard or soft capsules, or syrups or elixirs. Compositions intended for oral use may be prepared according to any method known to the art for the manufacture of pharmaceutical compositions, and such compositions may contain one or more agents selected from the group consisting of sweetening agents, flavoring agents, coloring agents and preserving agents in order to provide pharmaceutically elegant and palatable preparations. Tablets contain the active ingredient in admixture with non-toxic pharmaceutically acceptable excipients which are suitable for the manufacture of tablets. These excipients may be, for example, inert diluents, such as calcium carbonate, sodium carbonate, lactose, calcium phosphate or sodium phosphate; granulating and disintegrating agents, such as microcrystalline cellulose, sodium crosscarmellose, corn starch, or alginic acid; binding agents, for example starch, gelatin, polyvinyl-pyrrolidone or acacia, and lubricating agents, for example, magnesium stearate, stearic acid or talc. The tablets may be un-coated or coated by known techniques to mask the taste of the drug or delay disintegration and absorption in the gastrointestinal tract and thereby provide a sustained action over a longer period. For example, a water soluble taste masking material such as hydroxypropylmethyl-cellulose or hydroxypropylcellulose, or a time delay material such as ethyl cellulose, or cellulose acetate butyrate may be employed as appropriate.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water soluble carrier such as polyethyleneglycol or an oil medium, for example peanut oil, liquid paraffin, or olive oil.

Aqueous suspensions contain the active material in admixture with excipients suitable for the manufacture of aqueous suspensions. Such excipients are suspending agents, for example sodium carboxymethylcellulose, methylcellulose, hydroxypropylmethyl-cellulose, sodium alginate, polyvinyl-pyrrolidone, gum tragacanth and gum acacia; dispersing or wetting agents may be a naturally-occurring phosphatide, for example lecithin, or condensation products of an alkylene oxide with fatty acids, for example polyoxyethylene stearate, or condensation products of ethylene oxide with long chain aliphatic alcohols, for example heptadecaethylene-oxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl p-hydroxybenzoate, one or more coloring agents, one or more flavoring agents, and one or more sweetening agents, such as sucrose, saccharin or aspartame.

Oily suspensions may be formulated by suspending the active ingredient in a vegetable oil, for example arachisd oil, olive oil, sesame oil or coconut oil, or in mineral oil such as liquid paraffin. The oily suspensions may contain a thickening agent, for example beeswax, hard paraffin or cetyl alcohol. Sweetening agents such as those set forth above, and flavoring agents may be added to provide a palatable oral preparation. These compositions may be preserved by the addition of an anti-oxidant such as butylated hydroxyanisol or alpha-tocopherol.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example sweetening, flavoring and coloring agents, may also be present. These compositions may be preserved by the addition of an anti-oxidant such as ascorbic acid.

The compounds and pharmaceutical compositions used in the methods of the instant invention may also be in the form of an oil-in-water emulsions. The oily phase may be a vegetable oil, for example olive oil or arachis oil, or a

mineral oil, for example liquid paraffin or mixtures of these. Suitable emulsifying agents may be naturally-occurring phosphatides, for example soy bean lecithin, and esters or partial esters derived from fatty acids and hexitol anhydrides, for example sorbitan monooleate, and condensation products of the said partial esters with ethylene oxide, for example polyoxyethylene sorbitan monooleate. The emulsions may also contain sweetening agents, flavoring agents, preservatives and antioxidants.

Syrups and elixirs may be formulated with sweetening agents, for example glycerol, propylene glycol, sorbitol or sucrose. Such formulations may also contain a demulcent, a preservative, flavoring and coloring agents and antioxidant.

The pharmaceutical compositions may be in the form of a sterile injectable aqueous solution. Among the acceptable vehicles and solvents that may be employed are water, Ringer's solution and isotonic sodium chloride solution.

The sterile injectable preparation may also be a sterile injectable oil-in-water microemulsion where the active ingredient is dissolved in the oily phase. For example, the active ingredient may be first dissolved in a mixture of soybean oil and lecithin. The oil solution then introduced into a water and glycerol mixture and processed to form a microemulsion.

The injectable solutions or microemulsions may be introduced into a patient's blood-stream by local bolus injection. Alternatively, it may be advantageous to administer the solution or microemulsion in such a way as to maintain a constant circulating concentration of the instant compound. In order to maintain such a constant concentration, a continuous intravenous delivery device may be utilized. An example of such a device is the Deltec CADD-PLUS™ model 5400 intravenous pump.

The pharmaceutical compositions may be in the form of a sterile injectable aqueous or oleagenous suspension for intramuscular and subcutaneous administration. This suspension may be formulated according to the known art using those suitable dispersing or wetting agents and suspending agents which have been mentioned above. The sterile injectable preparation may also be a sterile injectable solution or suspension in a non-toxic parenterally-acceptable diluent or solvent, for example as a solution in 1,3-butane diol. In addition, sterile, fixed oils are conventionally employed as a solvent or suspending medium. For this purpose any bland fixed oil may be employed including synthetic mono- or diglycerides. In addition, fatty acids such as oleic acid find use in the preparation ofinjectables.

The compounds of the present invention used in the methods of the present invention may also be administered in the form of suppositories for rectal administration of the drug. These compositions can be prepared by mixing the inhibitors with a suitable non-irritating excipient which is solid at ordinary temperatures but liquid at the rectal temperature and will therefore melt in the rectum to release the drug. Such materials include cocoa butter, glycerinated gelatin, hydrogenated vegetable oils, mixtures of polyethylene glycols of various molecular weights and fatty acid esters of polyethylene glycol.

For topical use, creams, ointments, jellies, solutions or suspensions, *etc*., containing a compound or composition of the invention can be used. As used herein, topical application can include mouth washes and gargles.

The compounds used in the methods of the present invention can be administered in intranasal form via topical use of suitable intranasal vehicles and delivery devices, or via transdermal routes, using those forms of transdermal skin patches well known to those of ordinary skill in the art. To be administered in the form of a transdermal delivery system, the dosage administration will, of course, be continuous rather than intermittent throughout the dosage regimen.

The methods, compounds and compositions of the instant invention may also be used in conjunction with other well known therapeutic agents that are selected for their particular usefulness against the condition that is being treated. For example, the instant compounds may be useful in combination with known anti-cancer and cytotoxic agents. Further, the instant methods and compounds may also be useful in combination with other inhibitors of parts of the signaling pathway that links cell surface growth factor receptors to nuclear signals initiating cellular proliferation.

The methods of the present invention may also be useful with other agents that inhibit angiogenesis and thereby inhibit the growth and invasiveness of tumor cells, including, but not limited to VEGF receptor inhibitors, including ribozymes and antisense targeted to VEGF receptors, angiostatin and endostatin.

Examples of antineoplastic agents that can be used in combination with the compounds and methods of the present invention include, in general, and as appropriate, alkylating agents, anti-metabolites, epidophyllotoxins, antineoplastic enzymes, topoisomerase inhibitors, procarbazines, mitoxantrones, platinum coordination complexes, biological response modifiers and growth inhibitors, hormonal/anti-hormonal therapeutic agents and haematopoietic growth factors. Exemplary classes of antineoplastic include the anthracyclines, vinca drugs, mitomycins, bleomycins, cytotoxic nucleosides, epothilones, discodermolides, pteridines, diynenes and podophyllotoxins. Particularly useful members of those classes include, for example, carminomycin, daunorubicin, aminopterin, methotrexate, methopterin, dichloromethotrexate, mitomycin C, porfiromycin, 5-fluorouracil, 6-mercaptopurine, gemcitabine, cytosine arabinoside, podophyllotoxin or podo-phyllotoxin derivatives such as etoposide, etoposide phosphate or teniposide, melphalan, vinblastine, vincristine, leurosidine, vindesine, leurosine, paclitaxel and the like. Other useful antineoplastic agents include estramustine, carboplatin, cyclophosphamide, bleomycin, gemcitibine, ifosamide, melphalan, hexamethyl melamine, thiotepa, cytarabin, idatrexate, trimetrexate, dacarbazine, L-asparaginase, camptothecin, CPT-11, topotecan, ara-C, bicalutamide, flutamide, leuprolide, pyridobenzoindole derivatives, interferons and interleukins.

When a compound or composition of the invention is administered into a human subject, the daily dosage will normally be determined by the prescribing physician with the dosage generally varying according to the age, weight, and response of the individual patient, as well as the severity of the patient's symptoms.

In one exemplary application, a suitable amount of compound is administered to a mammal undergoing treatment for cancer, for example, breast cancer. Administration typically occurs in an amount of between about 0.01 mg/kg of body weight to about 100 mg/kg of body weight per day (administered in single or divided doses), more preferably at least about 0.1 mg/kg of body weight per day. A particular therapeutic dosage can include, e.g., from about 0.01 mg to about 1000 mg of compound, and preferably includes, *e.g.*, from about 1 mg to about 1000 mg. The quantity of active compound in a unit dose of preparation may be varied or adjusted from about 0.1 mg to 1000 mg, preferably from about 1 mg to 300 mg, more preferably 10 mg to 200 mg, according to the particular application. The amount administered will vary depending on the particular $IC_{50}$ value of the compound used and the judgment of the attending clinician taking into consideration factors such as health, weight, and age. In combinational applications in which the compound is not the sole active ingredient, it may be possible to administer lesser amounts of compound and still have therapeutic or prophylactic effect.

Preferably, the pharmaceutical preparation is in unit dosage form. In such form, the preparation is subdivided into unit doses containing appropriate quantities of the active component, *e.g.,* an effective amount to achieve the desired purpose.

The actual dosage employed may be varied depending upon the requirements of the patient and the severity of the condition being treated. Determination of the proper dosage for a particular situation is within the skill of the art. Generally, treatment is initiated with smaller dosages which are less than the optimum dose of the compound. Thereafter, the dosage is increased by small amounts until the optimum effect under the circumstances is reached. For convenience, the total daily dosage may be divided and administered in portions during the day if desired.

The amount and frequency of administration of the compounds and compositions of the present invention used in the methods of the present invention, and if applicable other chemotherapeutic agents and/or radiation therapy, will be regulated according to the judgment of the attending clinician (physician) considering such factors as age, condition and size of the patient as well as severity of the disease being treated.

The chemotherapeutic agent and/or radiation therapy can be administered according to therapeutic protocols well known in the art. It will be apparent to those skilled in the art that the administration of the chemotherapeutic agent and/or radiation therapy can be varied depending on the disease being treated and the known effects of the chemotherapeutic agent and/or radiation therapy on that disease. Also, in accordance with the knowledge of the skilled clinician, the therapeutic protocols (*e.g.,* dosage amounts and times of administration) can be varied in view of the observed effects of the administered therapeutic agents (*i.e.*, antineoplastic agent or radiation) on the patient, and in view of the observed responses of the disease to the administered therapeutic agents.

Also, in general, the compounds of the invention need not be administered in the same pharmaceutical composition as a chemotherapeutic agent, and may, because of different physical and chemical characteristics, be administered by a different route. For example, the compounds/compositions may be administered orally to generate and maintain good blood levels thereof, while the chemotherapeutic agent may be administered intravenously. The determination of the mode of administration and the advisability of administration, where possible, in the same pharmaceutical composition, is well within the knowledge of the skilled clinician. The initial administration can be made according to established protocols known in the art, and then, based upon the observed effects, the dosage, modes of administration and times of administration can be modified by the skilled clinician.

The particular choice of compound (and where appropriate, chemotherapeutic agent and/or radiation) will depend upon the diagnosis of the attending physicians and their judgment of the condition of the patient and the appropriate treatment protocol.

The compounds/compositions of the invention (and where appropriate chemotherapeutic agent and/or radiation) may be administered concurrently (e.g., simultaneously, essentially simultaneously or within the same treatment protocol) or sequentially, depending upon the nature of the proliferative disease, the condition of the patient, and the actual choice of chemotherapeutic agent and/or radiation to be administered in conjunction (*i.e.,* within a single treatment protocol) with the compound/composition.

In combinational applications and uses, the compound/composition and the chemotherapeutic agent and/or radiation need not be administered simultaneously or essentially simultaneously, and the initial order of administration of the compound/composition, and the chemotherapeutic agent and/or radiation, may not be important. Thus, the compounds/compositions of the invention may be administered first followed by the administration of the chemotherapeutic agent and/or radiation; or the chemotherapeutic agent and/or radiation may be administered first followed by the administration of the compounds/compositions of the invention. This alternate administration may be repeated during a single treatment protocol. The determination of the order of administration, and the number of repetitions of administration of each therapeutic agent during a treatment protocol, is well within the knowledge of the skilled physician after evaluation of the disease being treated and the condition of the patient. For example, the chemotherapeutic agent and/or radiation

may be administered first, especially if it is a cytotoxic agent, and then the treatment continued with the administration of the compounds/compositions of the invention followed, where determined advantageous, by the administration of the chemotherapeutic agent and/or radiation, and so on until the treatment protocol is complete.

Thus, in accordance with experience and knowledge, the practicing physician can modify each protocol for the administration of a compound/composition for treatment according to the individual patient's needs, as the treatment proceeds.

The attending clinician, in judging whether treatment is effective at the dosage administered, will consider the general well-being of the patient as well as more definite signs such as relief of disease-related symptoms, inhibition of tumor growth, actual shrinkage of the tumor, or inhibition of metastasis. Size of the tumor can be measured by standard methods such as radiological studies, *e.g.,* CAT or MRI scan, and successive measurements can be used to judge whether or not growth of the tumor has been retarded or even reversed. Relief of disease-related symptoms such as pain, and improvement in overall condition can also be used to help judge effectiveness of treatment.

## V. ASSAYS FOR DETERMINING HSP90 BINDING AND DOWNSTREAM EFFECT

A variety of *in vitro* and *in vivo* assays are available to test the effect of the compounds of the invention on HSP90. HSP90 competitive binding assays and functional assays can be performed as known in the art substituting in the compounds of the invention. Chiosis et al. Chemistry & Biology 2001, 8, 289-299, describe some of the known ways in which this can be done. For example, competition binding assays using, *e.g.*, geldanamycin or 17-AAG as a competitive binding inhibitor of HSP90 can be used to determine relative HSP90 affinity of the compounds of the invention by immobilizing the compound of interest or other competitive inhibitor on a gel or solid matrix, preincubating HSP90 with the other inhibitor, passing the preincubated mix over the gel or matrix, and then measuring the amount of HSP90 that retains or does not retain on the gel or matrix.

Downstream effects can also be evaluated based on the known effect of HSP90 inhibition on function and stability of various steroid receptors and signaling proteins including, *e.g.*, Raf1 and HER2. Compounds of the present invention induce dose-dependent degradation of these molecules, which can be measured using standard techniques. Inhibition of HSP90 also results in up-regulation of HSP90 and related chaperone proteins that can similarly be measured. Antiproliferative activity on various cancer cell lines can also be measured, as can morphological and functional differentiation related to HSP90 inhibition.

Many different types of methods are known in the art for determining protein concentrations and measuring or predicting the level of proteins within cells and in fluid samples. Indirect techniques include nucleic acid hybridization and amplification using, e.g., polymerase chain reaction (PCR). These techniques are known to the person of skill and are discussed, *e.g.,* in Sambrook, Fritsch & Maniatis Molecular Cloning: A Laboratory Manual, 2nd ed., Cold Spring Harbor Laboratory, Cold Spring Harbor, N.Y., 1989; Ausubel, et al. Current Protocols in Molecular Biology, John Wiley & Sons, NY, 1994, and, as specifically applied to the quantification, detection, and relative activity of HER2/Neu in patient samples, *e.g.*, in U.S. Patents 4,699,877, 4,918,162, 4,968,603, and 5,846,749. A brief discussion of two generic techniques that can be used follows.

The determination of whether cells overexpress or contain elevated levels of HER2 can be determined using well known antibody techniques such as immunoblotting, radioimmunoassays, western blotting, immunoprecipitation, enzyme-linked immunosorbant assays (ELISA), and derivative techniques that make use of antibodies directed against HER2. As an example, HER2 expression in breast cancer cells can be determined with the use of an immunohistochemical assay, such as the Dako Hercep™ test (Dako Corp., Carpinteria, CA). The Hercep™ test is an antibody staining assay designed to detect HER2 overexpression in tumor tissue specimens. This particular assay grades HER2 expression into four levels: 0, 1, 2, and 3, with level 3 representing the highest level of HER2 expression. Accurate quantitation can be enhanced by employing an Automated Cellular Imaging System (ACIS) as described, *e.g.,* by Press, M. et al. Modern Pathology 2000, 13, 225A.

Antibodies, polyclonal or monoclonal, can be purchased from a variety of commercial suppliers, or may be manufactured using well-known methods, *e.g.*, as described in Harlow et al. Antibodies: A Laboratory Manual, 2nd ed; Cold Spring Harbor Laboratory, Cold Spring Harbor, N.Y., 1988.

HER2 overexpression can also be determined at the nucleic acid level since there is a reported high correlation between overexpression of the HER2 protein and amplification of the gene that codes for it. One way to test this is by using RT-PCR. The genomic and cDNA sequences for HER2 are known. Specific DNA primers can be generated using standard, well-known techniques, and can then be used to amplify template already present in the cell. An example of this is described in Kurokawa, H. et al. Cancer Res. 2000, 60, 5887-5894. PCR can be standardized such that quantitative differences are observed as between normal and abnormal cells, *e.g.*, cancerous and noncancerous cells. Well known methods employing, *e.g.*, densitometry, can be used to quantitate and/or compare nucleic acid levels amplified using PCR.

Similarly, fluorescent in *situ* hybridization (FISH) assays and other assays can be used, *e.g.,* Northern and/or

Southern blotting. These rely on nucleic acid hybridization between the *HER2* gene or mRNA and a corresponding nucleic acid probe that can be designed in the same or a similar way as for PCR primers, above. See, *e.g.*, Mitchell MS, and Press M.F. Onco/., Suppl. 1999, 12, 108-116. For FISH, this nucleic acid probe can be conjugated to a fluorescent molecule, *e.g.*, fluorescein and/or rhodamine, that preferably does not interfere with hybridization, and which fluorescence can later be measured following hybridization. See, *e.g.*, Kurokawa, H et al, Cancer Res. 2000, 60, 5887-5894 (describing a specific nucleic acid probe having sequence 5'-FAM-NucleicAcid-TAMRA-p-3' sequence). ACIS-based approaches as described above can be employed to make the assay more quantitative (de la Torre-Bueno, J., et al. Modern Pathology 2000, 13, 221A).

Immuno and nucleic acid detection can also be directed against proteins other than HSP90 and HER2, which proteins are nevertheless affected in response to HSP90 inhibition.

The following examples are offered by way of illustration only and are not intended to be limiting of the full scope and spirit of the invention.

## EXAMPLES

## I. PREPARATION OF PYRROLOPYRINHDINES AND RELATED ANALOGS (COMPOUNDS OF FORMULA I)

### A. Materials and Methods

The chemical reagents used to create the novel products of the invention below are all available commercially, e.g., from Aldrich Chemical Co., Milwaukee, WI, USA. Otherwise their preparation is facile and known to one of ordinary skill in the art, or it is referenced or described herein.

The final compounds were usually purified by preparative TLC (silica gel 60 Å, Whatman Partisil PK6F) or flash chromatography (silica gel 60 Å, EMD Chemicals) using EtOAc/hexane or MeOH/$CH_2Cl_2$ as eluents. Rf's were measured using silica gel TLC plates (silica gel 60 Å, EMD Chemicals). Analytical HPLC chromatograms were obtained using a C18 column (Agilent Zorbax 300SB-C18; 5 microns; 4.6 mm x 150 mm). A gradient was applied between solvent A (0.1% TFA in $H_2O$) and solvent B (0.5% TFA in $CH_3CN$) increasing the proportion of A linearly from 5% (t=0) to 100% (t=7.00 min), with a constant flow rate of 1 mL/min. The samples were diluted to typically 0.1-1 mg/mL in MeOH or $CH_3CN$ and the injection volumes were typically 10 μL. The column was not heated, and UV detection was effected at 254 nm. $^1$H-NMR spectra were recorded on a Bruker Avance 400 MHz spectrometer.

The chemical names were generated using the Beilstein Autonom 2.1 software.

### B. General Procedures

### 1. General procedures to prepare and manipulate the pyrrolo[2,3-d]pyrimidine ring

General procedure 1.1: Preparation of pyrrolo[2,3-d]pyrimidines ($R^0 \neq OH$)

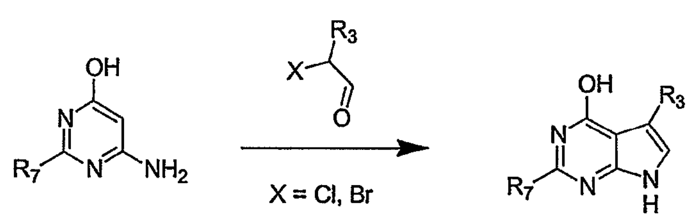


A suspension of 4-diamino-6-hydroxypyrimidine (6 mmol), AcONa (12 mmol) and α-haloaldehyde (6 mmol) in $CH_3CN$ (20 mL) and $H_2O$ (20 mL) was stirred at 22-40 °C overnight whereupon the starting materials gradually dissolved and the desired pyrrolo[2,3-d]pyrimidine precipitated. The precipitate was collected by filtration and washed (water, acetontrile, ether) and air-dried ((a) C. J. Barnett, Org. Proc. Res. Develop. 1999, 3, 184. (b) F. Seela, Liebigs Ann. Chem. 1987, 15).

## General Procedure 1.2: Preparation of pyrrolo[2,3-d]pyrimidines ($R^0$ = OH)

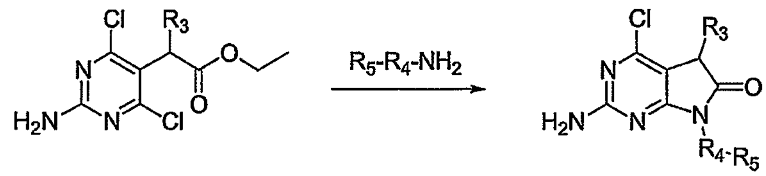


A suspension of (2-amino-4,6-dichloro-pyrimidin-5-yl)-acetic acid ethyl ester, $R^5$-$R^4$-$NH_2$ and $EtN(i\text{-}Pr)_2$ in BuOH was heated at reflux for 24h whereupon the solvent was removed under reduce pressure. The residue was then dissolved in $CH_2Cl_2$ and washed with sat. $NaHCO_3$ solution and dried with $Na_2SO_4$. The crude material was purified by preparative TLC or flash chromatography (EtOAc/hexane or MeOH/$CH_2Cl_2$) to give the pure pyrrolo[3,4-d]pyrimidin-6-one.

## General procedure 1.3: Alkylation of pyrrolo[2,3-d]pyrimidines at N-7

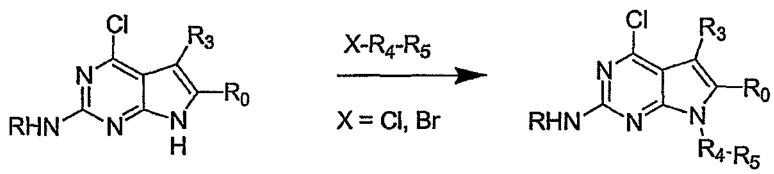


A suspension of the 4-chloro-7H-pyrrolo[2,3-d]pyrimidin-2-ylamine (1 mmol), benzyl halide (1 mmol) and $K_2CO_3$ or $Cs_2CO_3$ (1-5 mmol) in dry DMF (5 mL) was heated to 40 °C for 3 to 10 h. Work-up (EtOAc) and purification by preparative TLC or flash chromatography (EtOAc/hexane or MeOH/$CH_2Cl_2$) yielded the pure N-7 alkylated product.

## General procedure 1.4: Aminomethylation of pyrrolo[2,3-d]pyrimidines at C-5

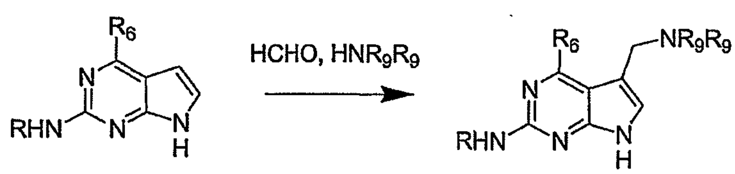


A solution of 2-amino-7H-pyrrolo[2,3-d]pyrimidin-4-ol, formaldehyde (2-5 equiv.) and $HNR^9R^9$ (2-5 equiv.) in 80% aq. acetic acid was heated in a sealed tube to 60 °C overnight, concentrated, extracted in MeOH:$CH_2Cl_2$ (1:10), washed with sat. $NaHCO_3$ and concentrated. See H. Akimoto, J. Chem. Soc. Perkin Trans 1. 1998,1637.

## General procedure 1.5: Alkylation of pyrrolo[2,3-d]pyrimidin-6-one at C-5

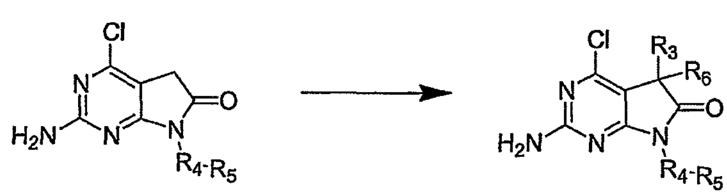

To a solution of pyrrolo[2,3-d]pyrimidin-6-one in THF at -78˚C was added a base such as LDA, LHMDS or KHMDS and after 30 min, the alkyl halide was further added to give monoalkylated and bisalkylated pyrrolo[2,3-d]pyrimidin-6-ones which were purified by preparative TLC or flash chromatography (EtOAc/hexane or McOH/$CH_2Cl_2$).

General procedure 1.6: Oxidation of pyrrolo[2,3-d]pyrimidines at C-5

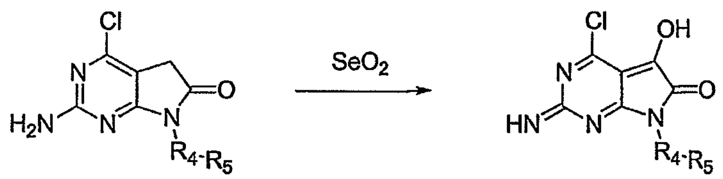


A solution of 2-amino-4-chloro-pyrrolo[3,4-d]pyrimidin-6-one and $SeO_2$ in dioxane was heated at reflux until completion (1 h) whereupon the solvent was removed under reduce pressure. The crude was purified by preparative TLC or flash chromatography (EtOAc/hexane or MeOH/$CH_2Cl_2$) to give the pure 4-chloro-5-hydroxy-2-imino-2,7-dihydro-pyrrolo[2,3-d]pyrimidin-6-one.

**2. <u>General procedures to manipulate the pyridine ring</u>**

General procedure 2.1: Preparation of pyridine N-oxides

A solution of the pyridine derivative (1 mmol) in dichloromethane or chloroform (5 mL) was cooled by means of an ice-bath, treated with m-CPBA (1.1 to 3 mmol) in three portions, and allowed to warm to r.t. The mixture was extracted with dichloromethane and washed with aqueous NaOH, followed by water. Drying ($Na_2SO_4$) and concentration afforded the pyridine N-oxide.

General procedure 2.2: Preparation of 2-(acetoxymethyl)-pyridines

A solution of the 2-methylpyridine N-oxide (1.0 mmol) in acetic anhydride (5 mL) was heated to reflux for 0.5 h. Work-up (EtOAc), drying ($MgSO_4$), evaporation and purification by preparative TLC or flash chromatography afforded the 2-(acetoxymethyl) pyridine.

General procedure 2.3: Preparation of 2-(hydroxymethyl)-pyridines

A suspension of 2-acetoxymethyl-pyridine derivative and solid $K_2CO_3$ in methanol was heated to 50 ˚C for 5-30 min. Evaporation, work-up (EtOAc), and drying ($MgSO_4$) afforded the 2-(hydroxymethyl)-pyridine.

General procedure 2.4: Preparation of 2-(bromomethyl)-pyridines

A solution of 2-(hydroxymethyl)-pyridine (1.0 mmol) and triphenyl phosphine (1.2 mmol) in dichloromethane or chloroform (5 mL) was cooled to 0 ˚C. A solution of $CBr_4$ (1.5 mmol) in dichloromethane or chloroform was added dopwise, and the resulting mixture was stirred at 0 ˚C for 0.5-1 h. Work-up followed and purification by flash chromatography afforded the 2-(bromomethyl)-pyridine.

General procedure 2.5: Preparation of 2-(aminomethyl)-pyridines

The 2-(chloromethyl)-pyridine derivative in a solution of ammonia in MeOH was heated at 100˚C overnight whereupon it was concentrated under reduce pressure and purified by flash chromatography (MeOH/$CH_2Cl_2$) to afford the 2-(aminomethyl)-pyridine derivative.

General procedure 2.6: Preparation of 2-chloropyridines

A suspension of 2-(hydroxymethyl)-pyridine (10 g) in $POCl_3$ (30 mL) was stirred at 110 ˚C for 1.5 h. The resulting viscous oil was cooled to r.t. and poured onto ice water (500 g). The pH was adjusted to 10 with solid KOH. Work-up

($CHCl_3$), drying ($MgSO_4$) and evaporation gave the 2-chloropyridine, which was used without purification.

General procedure 2.7: Preparation of pyridinium salts.

A solution of the pyridine was heated in MeOH until it dissolved. A methanolic solution of acid (1.0 equiv of e.g. HCl, MsOH) was added, and the solvent was evaporated to give the pyridinium salt.

**3. General procedure to manipulate benzene rings**

General procedure 3.1: Halogenation of benzene rings.

Variant 1: A solution of the aromatic compound in MeOH/THF/acetate buffer (1N in each AcOH and AcONa) was treated with $Br^2$ (1.3-equiv) at r.t. for 5 min. The excess bromine and solvent were removed on a rotary evaporator. Work-up ($CHCl_3$) and flash chromatography afforded the desired bromobenzene.

Variant 2: A solution of the aromatic compound (7 mmol) and n-halosuccinimide (NCS, NBS, or NIS, 1.06 equiv) in acetic acid (40 mL) was heated to 40-90 °C for 0.3-1 h. Evaporation, work-up (EtOAc) and flash chromatography afforded the desired halogenated benzene.

**C. Preparation of Intermediates**

**Example 1. 2-Chloro-1-chloromethyl-3,4,5-trimethoxy-benzene**

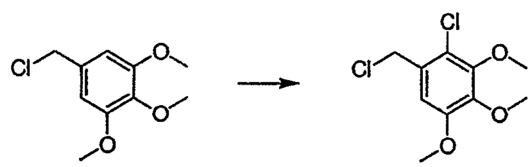

The title compound was obtained by chlorination of 5-chloromethyl-1,2,3-trimethoxy-benzene with NCS according to the general procedure 3.1.

$^1$H-NMR ($CDCl_3$): δ 6.82 (s, 1H), 4.70 (s, 1H), 3.93 (s, 3H), 3.90 (s, 3H) 3.87 (s, 3H).

**Example 2. 2-Chloro-6-chloromethyl-4-methoxy-3,5-dimethyl-pyridine**

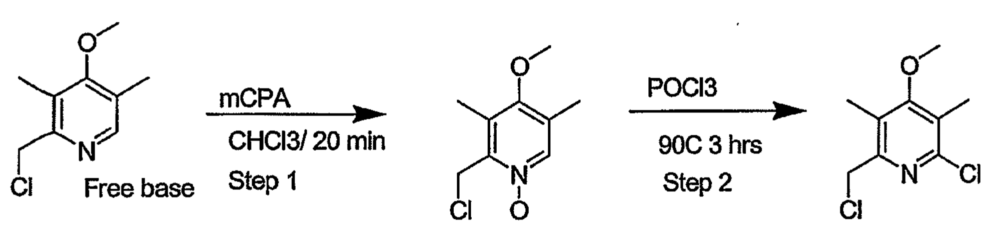


*Step 1:* 2-Chloromethyl-4-methoxy-3,5-dimethylpyridine-1-oxide

The title compound was obtained by oxidation of 2-chloromethyl-4-methoxy-3,5-dimethyl-pyridine according to the general procedure 2.1. HPLC Rt: 4.46 min. $^1$H-NMR ($CDCl_3$): δ 8.05 (s, 1H), 4.93 (s, 2H), 3.77 (s, 3H), 2.37 (s, 3H), 2.24 (s, 3H).

*Step 2:* 2-Chloro-6-chloromethyl-4-methoxy-3,5-dimethylpyridine

The title compound was obtained by treating 2-chloromethyl-4-methoxy-3,5-dimethylpyridine-1-oxide with

$POCl_3$ according to the general procedure 2.6. HPLC Rt: 6.757 min. $^1$H-NMR ($CDCl_3$): δ 4.64 (s, 2H), 3.79 (s, 3H), 2.35 (s, 3H), 2.33 (s, 3H).

**Example 3. 4-Chloro-2-chloromethyl-3,5-dimethyl-pyridine**

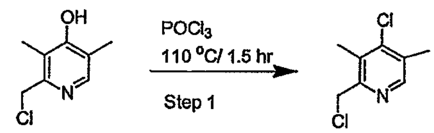


The title compound was obtained by treating 2-chloromethyl-3,5-dimethyl-pyridin-4-ol (Tarbit, et al. WO 99/10326) with $POCl_3$ in the same manner as in the general procedure 2.6 (74% yield). HPLC Rt: 5.54 min. $^1$H-NMR ($CDCl_3$): 8.24 (s, 1H), 4.71 (s, 2H), 2.48 (s, 3H), 2.36 (s, 3H).

**Example 4. 4-Bromo-2-bromomethyl-3,5-dimethyl-pyridine**

4-Bromo-2-bromomethyl-3,5-dimethyl-pyridine was prepared by any of the following three methods:

Method 1

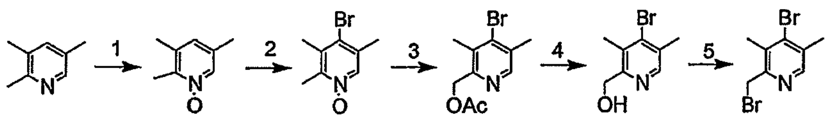


*Step 1:* 2,3,5-Collidine-N-oxide

2,3,5-Collidine-N-oxide was obtained by oxidation of 2,3,5-collidine according to the general procedure 2.1 in 70% yield. HPLC Rt: 3.96 min. $^1$H-NMR ($CDCl_3$): δ 8.03 (s, 1H), 6.90 (s, 1H), 2.47 (s, 3H), 2.31 (s, 3H), 2.24 (s, 3H). m/z (%) 138.2 (M+1, 100%). Rf (20% MeOH/EtOAc): 0.35.

*Step 2*: 4-Bromo-2,3,5-collidine-N-oxide

2,3,5-collidine-N-oxide (1.3 g, 10 mmol) and $K_2CO_3$ (2.9 g, 20 mmol) were suspended in 10 mL of $CCl_4$. Bromine (1 mL, 20 mmol) was added dropwise, and the reaction mixture was heated to reflux for 2 h. Work-up (EtOAc) and flash chromatography (10% MeOH/EtOAc) afforded the title compound as a solid (1.05 g, 51% yield). HPLC Rt: 5.24 min. $^1$H-NMR ($CDCl_3$): δ 8.06 (s, $^1$H), 2.56 (s, 3H), 2.43 (s, 3H), 2.31 (s, 3H). m/z (%) 216.2 (M+1, 100%), 218.2 (M+3, 100%). Rf (20% MeOH/EtOAc): 0.45.

*Step 3:* Acetic acid 4-bromo-3,5-dimethyl-pyridin-2-yl methyl ester

4-Bromo-2,3,5-collidine-N-oxide (0.25g, 11 mmol) was dissolved in acetic anhydride (5 mL) and the solution was heated to reflux for 30 min. Work-up and flash chromatography (50% Hexane/EtOAc) afforded the title compound (0.27 g, 96% yield). Rf (50% Hexane/EtOAc): 0.70. HPLC Rt: 4.76 min. $^1$H-NMR ($CDCl_3$): δ 8.26 (s, 1H), 5.27 (s, 2H), 2.46 (s, 3H), 2.41 (s, 3H), 2.14 (s, 3H).

*Step 4:* 4-Bromo-3,5-dimethyl-pyridin-2-yl methanol

A suspension of acetic acid 4-bromo-3,5-dimethyl-pyridin-2-yl methyl ester (0.26 g, 1.0 mmol) and $K_2CO_3$ (excess) in MeOH (5 mL) was heated to 50 ˚C for 15 min. Work-up ($CHCl_3$), evaporation, and filtration through a silica gel pad (eluent: 100% EtOAc) gave the title compound as a white solid (0.19 g, 88% yield). Rf (50% Hexane/EtOAc):

0.5. HPLC Rt: 3.80 min. $^1$H-NMR ($CDCl_3$): δ 8.23 (s, 1H), 4.70 (s, 2H), 2.46 (s, 3H), 2.30 (s, 3H).

*Step 5:* 4-Bromo-2-bromomethyl-3,5-dimethyl-pyridine

The title compound was obtained from 4-bromo-3,5-dimethyl-pyridin-2-yl methanol according to the general procedure 2.4. HPLC Rt: 6.32 min. $^1$H-NMR ($CDCl_3$): δ 8.22 (s, 1H), 4.63 (s, 2H), 2.52 (s, 3H), 2.40 (s, 3H).

Method 2:

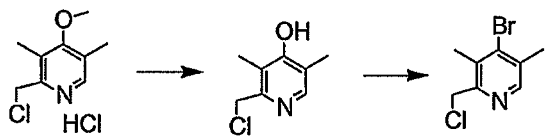

*Step 1:* 2-chloromethyl-3,5-dimethyl-pyridin-4-ol

The title compound was obtained by heating 2-chloromethyl-4-methoxy-3,5-dimethyl-pyridine hydrochloride in toluene as described in the patent by Tarbit, et al. WO 99/10326.

*Step 2:* 4-bromo-2-chloromethyl-3,5-dimethyl pyridine

A mixture of 2-chloromethyl-3,5-dimethyl-pyridin-4-ol (8.2 g, 47.8 mmol) and $POBr_3$ (60g, 209 mmol) was stirred at 130 ˚C for 3 h. The resulting viscous oil was cooled to r.t. and poured onto ice water. The pH was adjusted to 10 with solid KOH. Work-up ($CHCl_3$), drying ($MgSO_4$) and evaporation afforded the title compound as a purple solid (8.7 g, 78% yield) which was used without purification. HPLC Rt: 6.03 min. $^1$H-NMR ($CDCl_3$): 8.20 (s, 1H), 4.62 (s, 2H), 2.50 (s, 3H), 2.38 (s, 3H).

Method 3:

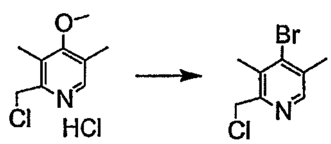

*Step 1:* 4-bromo-2-chloromethyl-3,5-dimethyl pyridine

A suspension of 2-chloromethyl-4-methoxy-3,5-dimethyl-pyridine (3.24 g, 14.6 mmol) in $PBr_3$ (8.0 mL, 85.1 mmol, 5.8 equiv.) was heated to 80 ˚C under nitrogen. A catalytic amount of DMF (0.50 mL, 6.4 mmol, 0.44 equiv.) was added, whereupon the suspension rapidly turned into an orange solution. After 40 min., the reaction was still incomplete as judged by HPLC. The temperature was raised to 110 ˚C and the reaction was prolonged for 30 min, at which point it was complete. The mixture was poured over ice, made basic with conc. aq. $NH_4OH$ and extracted into EtOAc. Washing with water, drying (brine, $MgSO_4$) and concentration gave the title compound as a pink solid (1.51 g, 44%) containing 10% of an impurity by $^1$H-NMR. The crude was used without further purification. $^1$H-NMR ($CDCl_3$) δ 8.19 (s, 1H), 4.59 (s, 2H), 2.48 (s, 3H), 2.37 (s, 3H).

**D. <u>Preparation of Final Compounds</u>**

**Example 5. 4-Chloro-7-(4-methoxy-3,5-dimethyl-pyridin-2-ylmethyl)-7H-pyrrolo[2,3-d]pyrimidin-2-ylamine**

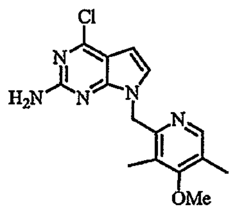

The title compound was obtained by alkylation of 4-chloro-7H-pyrrolo[2,3-d]pyrimidin-2-ylamine (F. Seela, Liebigs Ann. Chem. 1987, 15) with 2-chloromethyl-4-methoxy-3,5-dimethyl-pyridine hydrochloride according to the general procedure 1.3. HPLC Rt: 4.709 min. $^1$H-NMR ($CDCl_3$): $\delta$ 8.23 (s, 1H), 6.90 (m, 1H), 6.38 (m 1H), 5.35 (s, 2H), 4.99 (s, 2H), 3.75 (s, 3H), 2.26 (s, 3H), 2.21 (s, 3H).

**Example 6. 7-(2-Bromo-3,4,5-trimethoxy-benzyl)-4-chloro-7H-pyrrolo[2,3-d]pyrimidin-2-ylamine**

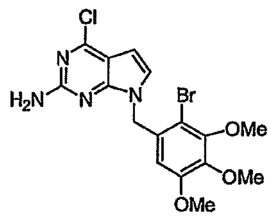

The title compound was obtained by alkylation of 4-chloro-7H-pyrrolo[2,3-d]pyrimidin-2-ylamine with 2-bromo-1-chloromethyl-3,4,5-trimethoxy-benzene according to the general procedure 1.3. HPLC Rt: 6.937 min. $^1$H-NMR (DMSO-d6): $\delta$ 7.11 (m 1H), 6.73(s, 2H), 6.42 (s, 1H), 6.37 (m 1H), 5.23 (s, 2H), 3.79 (s, 3H), 3.75 (s, 3H), 3.61 (s, 3H).

**Example 7. 4-Chloro-7-(2-iodo-3,4,5-trimethoxy-benzyl)-7H-pyrrolo [2,3-d]pyrimidin-2-ylamine**

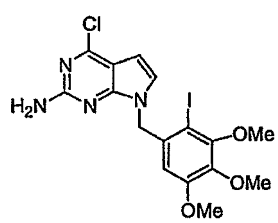

The title compound was obtained by alkylation of 4-chloro-7H-pyrrolo[2,3-d]pyrimidin-2-ylamine with 1-chloromethyl-2-iodo-3,4,5-trimethoxy-benzene according to the general procedure 1.3. HPLC Rt: 7.069 min. $^1$H-NMR (DMSO-d6): $\delta$ 7.08 (m 1H), 6.74 (s, 2H), 6.38 (m 1H), 6.36 (s, 1H), 5.19 (s, 2H), 3.80 (s, 3H), 3.77 (s, 3H), 3.60 (s, 3H).

**Example 8. 4-Chloro-7-(4-methoxy-3,5-dimethyl-1-oxy-pyridin-2-ylmethyl)-7H-pyrrolo[2,3-d]pyrimidin-2-ylamine**

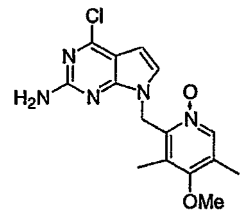

The title compound was obtained by alkylation of 4-chloro-7H-pyrrolo[2,3-d]pyrimidin-2-ylamine with 2-chloromethyl-4-methoxy-3,5-dimethyl-pyridine 1-oxide according to the general procedure 1.3. HPLC Rt: 5.079 min. $^1$H-NMR (DMSO-d6): δ 8.18 (s, 1H), 7.29 (m, 1H), 6.68(s, 2H), 6.24 (m, 1H), 5.38 (s, 2H), 3.70 (s, 3H), 2.42 (s, 3H), 2.17 (s, 3H). ESI-MS 334.2 (M+1).

**Example 9. 4-Chloro-7-(3,4,5-trimethoxy-benzyl)-7H-pyrrolo[2,3-d]pyrimidin-2-ylamine**

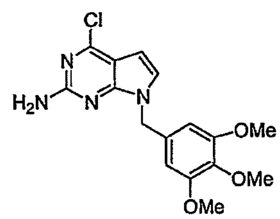

The title compound was obtained by alkylation of 4-chloro-7H-pyrrolo[2,3-d]pyrimidin-2-ylamine with 5-chloromethyl-1,2,3-trimethoxy-benzene according to the general procedure 1.3. HPLC Rt: 6.036 min. $^1$H-NMR ($CDCl_3$): δ 6.82 (m, 1H), 6.41(s, 2H), 6.40 (m, 1H), 5.36 (s, 2H), 5.16 (s, 2H), 3.81 (s, 3H), 3.78 (s, 6H).

**Example 10. 4-Chloro-7-(6-chloro-4-methoxy-3,5-dimethyl-pyridin-2-ylmethyl)-7H-pyrrolo[2,3-d]pyrimidin-2-ylamine**

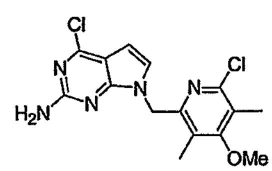

The title compound was obtained by alkylation of 4-chloro-7H-pyrrolo[2,3-d]pyrimidin-2-ylamine with 2-chloro-6-chloromethyl-4-methoary-3,5-dimethyl-pyridine according to the general procedure 1.3. HPLC Rt: 6.880 min. $^1$H-NMR (DMSO-d6): δ 7.06 (m, 1H), 6.63(s, 2H), 6.32 (m, 1H), 5.29 (s, 2H), 3.74 (s, 3H), 2.25 (s, 3H), 2.21 (s, 3H).

**Example 11. 4-Chloro-7-(4-chloro-3,5-dimethyl-pyridin-2-ylmethyl)-7H-pyrrolo[2,3-d]pyrimidin-2-ylamine**

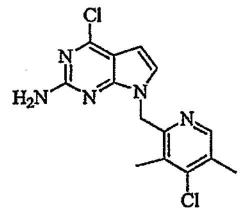

The title compound was obtained by alkylation of 4-chloro-7H-pyrrolo[2,3-d]pyrimidin-2-ylamine with 4-chloro-2-chloromethyl-3,5-dimethyl-pyridine according to the general procedure 1.3. HPLC Rt: 5.878 min. $^1$H-NMR ($CDCl_3$): δ 8.27 (s, 1H), 6.89 (m, 1H), 6.40 (m, 1H), 5.40 (s, 2H), 4.94 (s, 2H), 2.39 (s, 3H), 2.37 (s, 3H).

**Example 12. 4-Chloro-7-(2-chloro-4,5-dimethoxy-benzyl)-7H-pyrrolo[2,3-d]pyrimidin-2-ylamine**

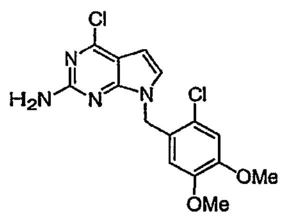

The title compound was obtained by alkylation of 4-chloro-7H-pyrrolo[2,3-d]pyrimidin-2-ylamine with 1-bromomethyl-2-chloro-4,5-dimethoxy-benzene according to the general procedure 1.3. HPLC Rt: 6.635 min. $^1$H-NMR ($CDCl_3$): δ 6.91 (m, 1H), 6.90 (s, 1H), 6.71 (s, 1H), 6.42 (m, 1H), 5.30 (s, 2H), 4.97 (s, 2H), 3.88 (s, 3H), 3.75 (s, 3H).

**Example 13. 7-(4-Bromo-3,5-dimethyl-pyridin-2-ylmethyl)4-chloro-7H-pyrrolo[2,3-d]pyrimidin-2-ylamine**

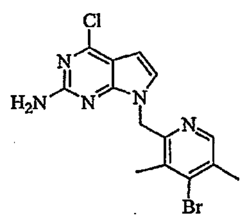

The title compound was obtained by alkylation of 4-chloro-7H-pyrrolo[2,3-d]pyrimidin-2-ylamine with 4-bromo-2-chloromethyl-3,5-dimethyl-pyridine according to the general procedure 1.3. HPLC Rt: 6.072 min. $^1$H-NMR (DMSO-d6): δ 8.15 (s, 1H), 7.10 (m, 1H), 6.60(s, 1H), 6.30 (m, 1H), 5.40(s, 2H), 2.46 (s, 3H), 2.30 (s, 3H).

**Example 14. 4-Chloro-7-(4-chloro-3,5-dimethyl-1-oxy-pyridin-2-ylmethyl)-7H-pyrrolo[2,3-d]pyrimidin-2-ylamine**

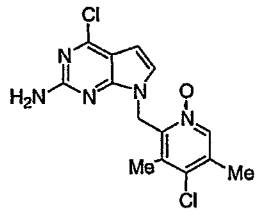

The title compound was obtained by alkylation of 4-chloro-7H-pyrrolo[2,3-d]pyrimidin-2-ylamine with 4-chloro-2-chloromethyl-3,5-dimethyl-pyridine 1-oxide according to the general procedure 1.3. HPLC Rt: 5.610 min. $^{1}$H-NMR (DMSO-d6): δ 8.36 (s, 1H), 7.26 (m, 1H), 6.69(s, 1H), 6.21 (m, 1H), 5.43(s, 2H), 2.60 (s, 3H), 2.27 (s, 3H).

**Example 15. 7-(4-Bromo-3,5-dimethyl-1-oxy-pyridin-2-ylmethyl)4-chloro-7H-pyrrolo[2,3-d]pyrimidin-2-ylamine**

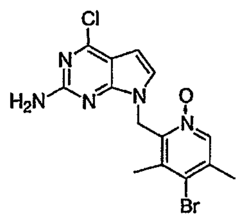

The title compound was obtained by alkylation of 4-chloro-7H-pyrrolo[2,3-d]pyrimidin-2-ylamine with 4-bromo-2-chloromethyl-3,5-dimethyl-pyridine 1-oxide according to the general procedure 1.3. HPLC Rt: 5.734 min. $^{1}$H-NMR (DMSO-d6): δ 8.33 (s, 1H), 7.24 (m, 1H), 6.69 (s, 1H), 6.25 (m, 1H), 5.47(s, 2H), 2.65 (s, 3H), 2.29 (s, 3H).

**Example 16. 4-Chloro-7-(3,5-dimethoxy-2-nitro-benzyl)-7H-pyrrolo[2,3-d]pyrimidin-2-ylamine**

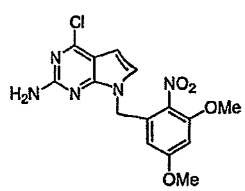

The title compound was obtained by alkylation of 4-chloro-7H-pyrrolo[2,3-d]pyrimidin-2-ylamine with 1-bromomethyl-4,5-dimethoxy-2-nitro-benzene according to the general procedure 1.3. HPLC Rt: 6.345 min. $^{1}$H-NMR (DMSO-d6): δ 7.73 (s, 1H), 7.16 (m, 1H), 6.72 (s, 2H), 6.41 (s, 1H), 6.40 (m, 1H), 5.58(s, 2H), 3.92 (s, 3H), 3.62 (s, 3H).

**Example 17. 4-Chloro-7-(3,4-dichloro-ben-zyl)-7H-pyrrolo[2,3-d]pyrimidin-2-ylamine**

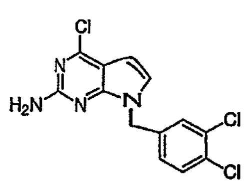

The title compound was obtained by alkylation of 4-chloro-7H-pyrrolo[2,3-d]pyrimidin-2-ylamine with 4-bromomethyl-1,2-dichloro-benzene according to the general procedure 1.3. HPLC Rt: 7.148 min. $^1$H-NMR (DMSO-d6): δ 7.60 (m, 1H), 7.59 (m, 1H), 7.25(q, 1H), 7.12(m, 1H), 6.71 (s, 2H), 6.37 (q, 1H), 5.26(s, 2H).

**Example 18. 4-Chloro-7-(3,5-dimethoxy-benzyl)-7H-pyrrolo[2,3-d]pyrimidin-2-ylamine**

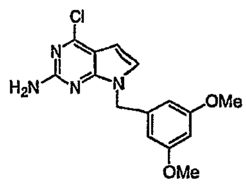

The title compound was obtained by alkylation of 4-chloro-7H-pyrrolo[2,3-d]pyrimidin-2-ylamine with 1-chloromethyl-3,5-dimethoxy-benzene according to the general procedure 1.3. HPLC Rt: 6.423 min. $^1$H-NMR (DMSO-d6): δ 7.21(m, 1H), 6.69 (s, 2H), 6.40 (m, 3H), 6.34 (m, 1H), 5.34 (s, 2H), 3.68 (s, 6H).

**Example 19. 4-Chloro-7-(2,5-dimethoxy-benzyl)-7H-pyrrolo[2,3-d]pyrimidin-2-ylamine**

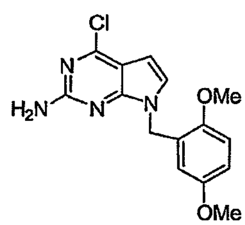

The title compound was obtained by alkylation of 4-chloro-7H-pyrrolo[2,3-d]pyrimidin-2-ylamine with 2-chloromethyl-1,4-dimethoxy-benzene according to the general procedure 1.3. HPLC Rt: 6.537 min. $^1$H-NMR (DMSO-d6): δ 7.13 (m, 1H), 6.85 (d, 1H), 6.82 (m, 1H), 6.68 (s, 2H), 6.35 (m, 1H), 6.22 (d, 1H), 3.78 (s, 3H), 3.60 (s, 3H).

**Example 20. 4-Bromo-7-(4-methoxy-3,5-dimethyl-1-oxy-pyridin-2-ylmethyl)-7H-pyrrolo[2,3-d]pyrimidin-2-ylamine**

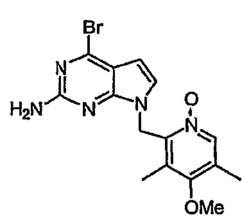

The title compound was obtained by alkylation of 4-bromo-7H-pyrrolo[2,3-d]pyrimidin-2-ylamine (obtained as described in F. Seela, Liebigs Ann. Chem. 1987, 15 for 4-chloro-7H-pyrrolo[2,3-d]pyrimidin-2-ylamine, but substituting $POCl_3$ with $POBr_3$) with 2-chloromethyl-4-methoxy-3,5-dimethyl-pyridine 1-oxide according to the general procedure 1.3. HPLC Rt: 5.158 min. $^1$H-NMR (DMSO-d6): δ 8.18 (s, 1H), 7.29 (m, 1H), 6.69 (s, 2H), 6.15 (m, 1H), 5.37(s, 2H), 3.70 (s, 3H), 2.42 (s, 3H), 2.17 (s, 3H).

**Example 21. 4-Bromo-7-(4-chloro-3,5-dimethyl-pyridin-2-ylmethyl)-7H-pyrrolo[2,3-d]pyrimidin-2-ylamine**

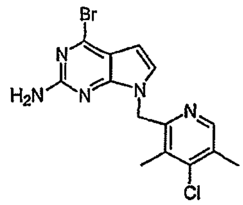

The title compound was obtained by alkylation of 4-bromo-7H-pyrrolo[2,3-d]pyrimidin-2-ylamine with 4-chloro-2-chloromethyl-3,5-dimethyl-pyridine according to the general procedure 1.3. HPLC Rt: 5.803 min. $^1$H-NMR (DMSO-d6): δ 8.20 (s, 1H), 7.04 (m, 1H), 6.61 (s, 2H), 6.21 (m, 1H), 5.38(s, 2H), 2.42 (s, 3H), 2.28 (s, 3H).

**Example 22. 4-Bromo-7-(4-chloro-3,5-dimethyl-1-oxy-pyridin-2-ylmethyl)-7H-pyrrolo[2,3-d]pyrimidin-2-ylamine**

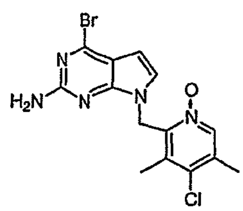

The title compound was obtained by alkylation of 4-bromo-7H-pyrrolo[2,3-d]pyrimidin-2-ylamine with 4-chloro-2-chloromethyl-3,5-dimethyl-pyridine 1-oxide according to the general procedure 1.3. HPLC Rt: 5.688 min. $^1$H-NMR (DMSO-d6): δ 8.35 (s, 1H), 7.25 (m, 1H), 6.70 (s, 2H), 6.15 (m, 1H), 5.43 (s, 2H), 2.60 (s, 3H), 2.27 (s, 3H).

**Example 23. 4-Bromo-7-(4-bromo-3,5-dimethyl-pyridin-2-ylmethyl)-7H-pyrrolo[2,3-d]pyrimidin-2-ylamine**

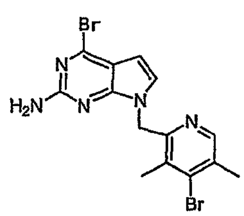

The title compound was obtained by alkylation of 4-bromo-7H-pyrrolo[2,3-d]pyrimidin-2-ylamine with 4-bromo-2-chloromethyl-3,5-dimethyl-pyridine according to the general procedure 1.3. HPLC Rt: 5.996 min. $^1$H-NMR (DMSO-d6): δ 8.15 (s, 1H), 7.05 (m, 1H), 6.61 (s, 2H), 6.21 (m, 1H), 5.43 (s, 2H), 2.46 (s, 3H), 2.30 (s, 3H).

**Example 24. 4-Bromo-7-(4-bromo-3,5-dimethyl-1-oxy-pyridin-2-ylmethyl)-7H-pyrrolo[2,3-d]pyrimidin-2-ylamine**

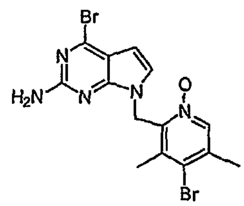

The title compound was obtained by alkylation of 4-bromo-7H-pyrrolo[2,3-d]pyrimidin-2-ylamine with 4-bromo-2-chloromethyl-3,5-dimethyl-pyridine 1-oxide according to the general procedure 1.3. HPLC Rt: 5.798 min. $^1$H-NMR (DMSO-d6): δ 8.33 (s, 1H), 7.24 (m, 1H), 6.71 (s, 2H), 6.15 (m, 1H), 5.46 (s, 2H), 2.64 (s, 3H), 2.29 (s, 3H).

**Example 25. 4-Bromo-7-(4-methoxy-3,5-dimethyl-pyridin-2-ylmethyl)-7H-pyrrolo [2,3-d]pyrimidin-2-ylamine**

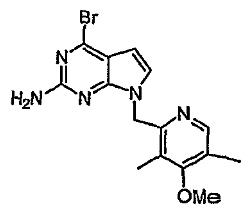

The title compound was obtained by alkylation of 4-bromo-7H-pyrrolo[2,3-d]pyrimidin-2-ylamine with 2-chloromethyl-4-methoxy-3,5-dimethyl-pyridine according to the general procedure 1.3. HPLC Rt: 4.847 min. $^1$H-NMR (DMSO-d6): δ 8.07 (s, 1H), 7.03 (m, 1H), 6.60 (s, 2H), 6.20 (m, 1H), 5.29 (s, 2H), 3.72 (s, 3H), 2.24 (s, 3H), 2.17 (s, 3H).

**Example 26. 4-Bromo-7-(3,5-dimethoxy-benzyl)-7H-pyrrolo[2,3-d]pyrimidin-2-ylamine**

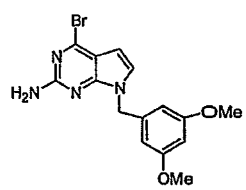

The title compound was obtained by alkylation of 4-bromo-7H-pyrrolo[2,3-d]pyrimidin-2-ylamine with 1-cloromethyl-3,5-dimethoxy-benzene according to the general procedure 1.3. HPLC Rt: 6.490 min. $^1$H-NMR ($CDCl_3$): δ 7.20 (m, 1H), 6.70 (s, 2H), 6.40 (s, 1H), 6.34 (s, 2H), 6.23 (m, 1H), 5.16 (s, 2H), 3.69 (s, 6H).

**Example 27. 4-Chloro-7-(3-methoxy-benzyl)-7H-pyrrolo[2,3-d]pyrimidin-2-ylamine**

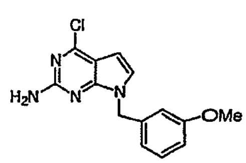

The title compound was obtained by alkylation of 4-chloro-7H-pyrrolo[2,3-d]pyrimidin-2-ylamine with 1-chloromethyl-3-methoxy-benzene according to the general procedure 1.3. HPLC Rt: 7.177 min. $^1$H-NMR (DMSO-d6): δ 7.26-7.18 (m, 2H), 6.82-6.80 (m, 1H), 6.67 (s, 1H), 6.70-6.67 (m, 3H), 6.32-6.30 (m, 1H), 5.20 (s, 2H), 3.68 (s, 3H).

**Example 28. 4-Chloro-7-(4-methoxy-benzyl)-7H-pyrrolo[2,3-d]pyrimidin-2-ylamine**

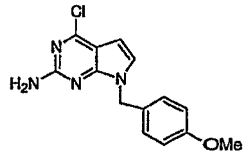

The title compound was obtained by alkylation of 4-chloro-7H-pyrrolo[2,3-d]pyrimidin-2-ylamine with 1-chloromethyl-4-methoxy-benzene according to the general procedure 1.3. HPLC Rt: 6.889 min. $^1$H-NMR (DMSO-d6): δ 7.19-7.16 (m, 3H), 6.90-6.88 (m, 2H), 6.69 (s, 2H), 6.32-6.30 (m, 1H), 5.18 (s, 2H), 3.71 (s, 3H).

**Example 29. N-[4-Chloro-5-iodo-7-(4-methoxy-3,5-dimethyl-1-oxy-pyridin-2-ylmethyl)-7H-pyrrolo[2,3-d]pyrimidin-2-yl]-2,2-dimethyl-propionamide**

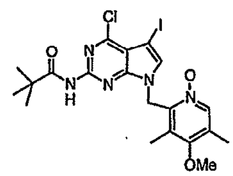

The title compound was obtained by alkylation of N-(4-chloro-5-iodo-7H-pyrrolo[2,3-d]pyrimidin-2-yl)-2,2-dimethyl-propionamide with 2-chloromethyl-4-methoxy-3,5-dimethyl-pyridine 1-oxide according to the general procedure 1.3. HPLC Rt: 6.812min. $^1$H-NMR (DMSO-d6): δ 10.20 (s, 1H), 8.13(s, 1H), 7.97 (s, 1H), 5.50 (s, 2H), 3.72 (s, 3H), 2.50 (s, 3H), 2.16 (s, 3H), 1.22 (s, 9H).

**Example 30. N-[7-(4-Bromo-3,5-dimethyl-1-oxy-pyridin-2-ylmethyl)-4-chloro-5-iodo-7H-pyrrolo[2,3-d]pyrimidin-2-yl]-2,2-dimethyl-propionamide**

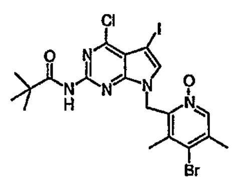

The title compound was obtained by alkylation of N-(4-chloro-5-iodo-7H-pyrrolo[2,3-d]pyrimidin-2-yl)-2,2-dimethyl-propionamide with 4-bromo-2-chloromethyl-3,5-dimethyl-pyridine 1-oxide according to the general procedure 1.3. HPLC Rt: 7.630 min. $^1$H-NMR (DMSO-d6): δ 10.21 (s, 1H), 8.30(s, 1H), 7.91 (s, 1H), 5.59 (s, 2H), 2.72 (s, 3H), 2.28 (s, 3H), 1.22 (s, 9H).

**Example 31. N-[4-Chloro-5-iodo-7-(4-methoxy-3,5-dimethyl-pyridin-2-ylmethyl)-7H-pyrrolo[2,3-d]pyrimidin-2-yl]-2,2-dimethyl-propionamide**

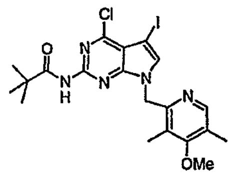

The title compound was obtained by alkylation of N-(4-chloro-5-iodo-7H-pyrrolo[2,3-d]pyrimidin-2-yl)-2,2-dimethyl-propionamide (A. Gangjee, J. Med. Chem. 2003, 46, 591) with 2-chloromethyl-4-methoxy-3,5-dimethyl-pyridine according to the general procedure 1.3. HPLC Rt: 6.627 min. $^1$H-NMR (DMSO-d6): δ 10.15 (s, 1H), 8.05(s, 1H), 7.73 (s, 1H), 5.46 (s, 2H), 3.74 (s, 3H), 2.33 (s, 3H), 2.16 (s, 3H), 1.21 (s, 9H).

**Example 32. N-[7-(-4-Bromo-3,5-dimethyl-1-oxy-pyridin-2-ylmethyl)-4-chloro-7H-pyrrolo[2,3-d]pyrimidin-2-yl]-2,2-dimethyl-propionamide**

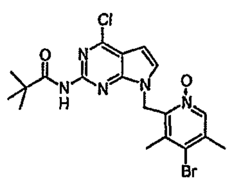

The title compound was obtained by alkylation of N-(4-chloro-7H-pyrrolo[2,3-d]pyrimidin-2-yl)-2,2-dimethyl-propionamide with 4-bromo-2-chloromethyl-3,5-dimethyl-pyridine 1-oxide according to the general procedure 1.3. HPLC Rt: 6.806 min. $^1$H-NMR (DMSO-d6): δ 10.13 (s, 1H), 8.30(s, 1H), 7.74 (m, 1H), 6.52 (m, 1 H), 5.62 (s, 2H), 2.73 (s, 3H), 2.28 (s, 3H), 1.23 (s, 9H).

**Example 33. N-[4-Chloro-7-(4-methoxy-3,5-dimethyl-pyridin-2-ylmethyl)-7H-pyrrolo[2,3-d]pyrimidin-2-yl]-2,2-dimethyl-propionamide**

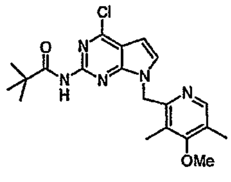

The title compound was obtained by alkylation of N-(4-chloro-7H-pyrrolo[2,3-d]pyrimidin-2-yl)-2,2-dimethyl-propionamide with 2-chloromethyl-4-methoxy-3,5-dimethyl-pyridine according to the general procedure 1.3. HPLC Rt: 6.087 min. $^1$H-NMR (CDCl3): δ 8.18 (s, 1H), 8.13(s, 1H), 7.18 (m, 1H), 6.49 (m, 1 H), 5.50 (s, 2H), 3.72 (s, 3H), 2.26 (s, 3H), 2.22 (s, 3H), 1.34 (s, 9H).

**Example 34. N-[4-Chloro-7-(4-methoxy-3,5-dimethyl-1-oxy-pyridin-2-ylmethyl)-7H-pyrrolo[2,3-d]pyrimidin-2-yl]-2,2-dimethyl-propionamide**

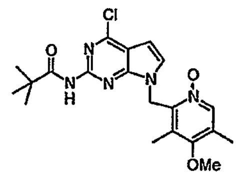

The title compound was obtained by alkylation of N-(4-chloro-7H-pyrrolo[2,3-d]pyrimidin-2-yl)-2,2-dimethyl-propionamide with 2-chloromethyl-4-methoxy-3,5-dimethyl-pyridine 1-oxide according to the general procedure 1.3. HPLC Rt: 6.115 min. $^{1}$H-NMR (CDCl3): δ 8.12 (s, 1H), 8.02(s, 1H), 7.93 (m, 1H), 6.49 (m, 1 H), 5.71 (s, 2H), 3.76 (s, 3H), 2.70 (s, 3H), 2.22 (s, 3H), 1.36 (s, 9H).

**Example 35. N-[4-Chloro-7-(4-chloro-3,5-dimethyl-pyridin-2-ylmethyl)-5-iodo-7H-pyrrolo[2,3-d]pyrimidin-2-yl]-2,2-dimethyl-propionamide**

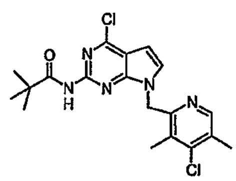

The title compound was obtained by alkylation of N-(4-chloro-7H-pyrrolo[2,3-d]pyrimidin-2-yl)-2,2-dimethyl-propionamide with 4-chloro-2-chloromethyl-3,5-dimethyl-pyridine according to the general procedure 1.3. HPLC Rt: 6.761 min. $^{1}$H-NMR (CDCl3): δ 8.23 (s, 1H), 8.11(s, 1H), 7.15 (m, 1H), 6.50 (m, 1 H), 5.71 (s, 2H), 2.43 (s, 3H), 2.33 (s, 3H), 1.35 (s, 9H).

**Example 36. N-[4-Chloro-7-(4-chloro-3,5-dimethyl-pyridin-2-ylmethyl)-5-iodo-7H-pyrrolo[2,3-d]pyrimidin-2-yl]-2,2-dimethyl-propionamide**

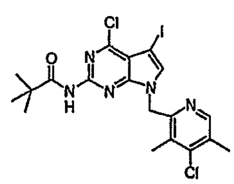

The title compound was obtained by alkylation ofN-(4-chloro-5-iodo-7H-pyrrolo[2,3-d]pyrimidin-2-yl)-2,2-dimethyl-propionamide with 4-chloro-2-chloromethyl-3,5-dimethyl-pyridine according to the general procedure 1.3. HPLC Rt: 7.508 min. $^{1}$H-NMR (CDC13): δ 8.17 (s, 1H), 8.11 (s, 1H), 8.07 (s, 1H), 5.77 (s, 2H), 2.81 (s, 3H), 2.33 (s, 3H), 1.37 (s, 9H).

**Example 37. N-[4-Chloro-7-(4-chloro-3,5-dimethyl-1-oxy-pyridin-2-ylmethyl)-7H-pyrrolo[2,3-d]pyrimidin-2-yl]-2,2-dimethyl-propionamide**

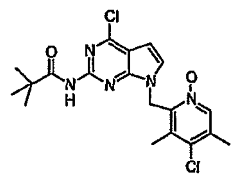

The title compound was obtained by alkylation of N-(4-chloro-7H-pyrrolo[2,3-d]pyrimidin-2-yl)-2,2-dimethyl-propionamlde with 4-chloro-2-chloromethyl-3,5-dimethyl-pyridine 1-oxide according to the general procedure 1.3. HPLC Rt: 6.688 min. $^1$H-NMR (CDCl3): δ 8.15 (s, 1H), 8.09 (s, 1H), 7.87 (m, 1H), 6.47 (m, 1H), 5.77 (s, 2H), 2.84 (s, 3H), 2.31 (s, 3H), 1.37 (s, 9H).

**Example 38. N-[4-Chloro-7-(4-chloro-3,5-dimethyl-1-oxy-pyridin-2-ylmethyl)-5-iodo-7H-pyrrolo[2,3-d]pyrimidin-2-yl]-2,2-dimethyl-propionamide**

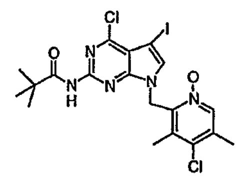

The title compound was obtained by alkylation of N-(4-chloro-5-iodo-7H-pyrrolo[2,3-d]pyrimidin-2-yl)-2,2-dimethyl-propionamide with 4-chloro-2-chloromethyl-3,5-dimethyl-pyridine 1-oxide according to the general procedure 1.3. HPLC Rt: 7.619 min. $^1$H-NMR (CDC13): δ 8.25 (s, 1H), 8.13 (s, 1H), 7.33 (s, 1H), 5.55 (s, 2H), 2.47 (s, 3H), 2.36 (s, 3H), 1.36 (s, 9H).

**Example 39. 4-Chloro-5-[(dibenzylamino)-methyl]-7-(4-methoxy-3,5-dimethyl-pyridin-2-ylmethyl)-7H-pyrrolo [2,3-d]pyrimidin-2-ylamine**

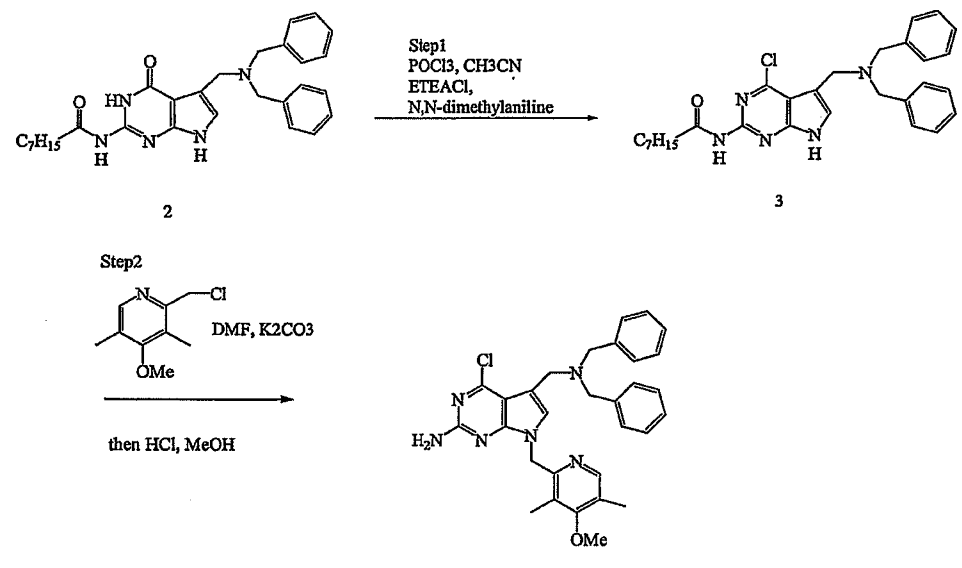


2

3

1

*Step 1.* Octanoic acid {4-chloro-5-[(dibenzylamino)-methyl]-7H-pyrrolo[2,3-d]pyrimidin-2-yl}-amide

A solution of octanoic acid {5-[(dibenzylamino)-methyl]-4-oxo-4,7-dihydro-3H-pyrrolo[2,3-d]pyrimidin-2-yl}-amide (1.0 g, 2 mmol; J. Chem. Soc. Perkin Trans. 1 1998, 1637), $BnNEt_3Cl$ (1.4 g, 4 mmol), $PhNMe_2$ (0.5 mL) and $POCl_3$ (1.73 mL, 12 mmol) in $CH_3CN$ (9.2 mL) was heated to 100 ˚C for 40 min and concentrated. The residue was poured into ice water and neutralized with 2N NaOH, extracted with EtOAc (50 mL ×3), and evaporated, to give the title compound (0.80 g, 76%). HPLC Rt: 6.868 min. $^1$H-NMR (DMSO-$d_6$): δ 12.19 (s, 1H), 10.49 (s, 1H), 1.45-7.21 (m, 11H), 3.80 (s, 2H), 3.59 (s, 4H), 2.41 (t, 2H), 1,56 (m, 2H), 1.27 (br s 8H), 0.85 (t, 3H).

*Step 2.* 4-Chloro-5-[(dibenzylamino)-methyl]-7-(4-methoxy-3,5-dimethyl-pyridin-2-ylmethyl)-7H-pyrrolo[2,3-d]pyrimidin-2-ylamine

A suspension of octanoic acid {4-chloro-5-[(dibenzylamino)-methyl]-7H-pyrrolo[2,3-d]pyrimidin-2-yl}-amide (150 mg, 0.30 mmol), 2-chloromethyl-4-methoxy-3,5-dimethyl-pyridine (56 mg, 0.30 mmol) and $K_2CO_3$ (84 mg, 0.60 mmol) in dry DMF (1 mL) was heated to 45 ˚C for overnight. After work-up (EtOAc) and evaporation, the residue was taken up in methanolic 4N HCl (1 mL), stirred at room temperature for 1 h, and neutralized to pH 7 with 2N NaOH. Extraction with EtOAc (10 mL ×3), evaporation and purification by preparative TLC (MeOH/$CH_2Cl_2$ 10:1) gave the title compound ( 70.5 mg, 45%). HPLC Rt: 5.362 min. $^1$H-NMR (DMSO-$d_6$): δ 8.05 (s, 1H), 7.30-7.22 (m, 10H), 6.99(s, 1H), 6.57s, 2H), 5.27 (s 2H), 3.70 (s, 2H), 3.65 (s, 3H), 3.54 (s, 4H), 2.17 (s, 3H), 2.15 (s, 3H).

**Example 40. 4-Chloro-7-(4-methoxy-3,5-dimethyl-pyridin-2-ylmethyl)-5-phenylaminomethyl-7H-pyrrolo[2,3-d] pyrimidin-2-ylamine**

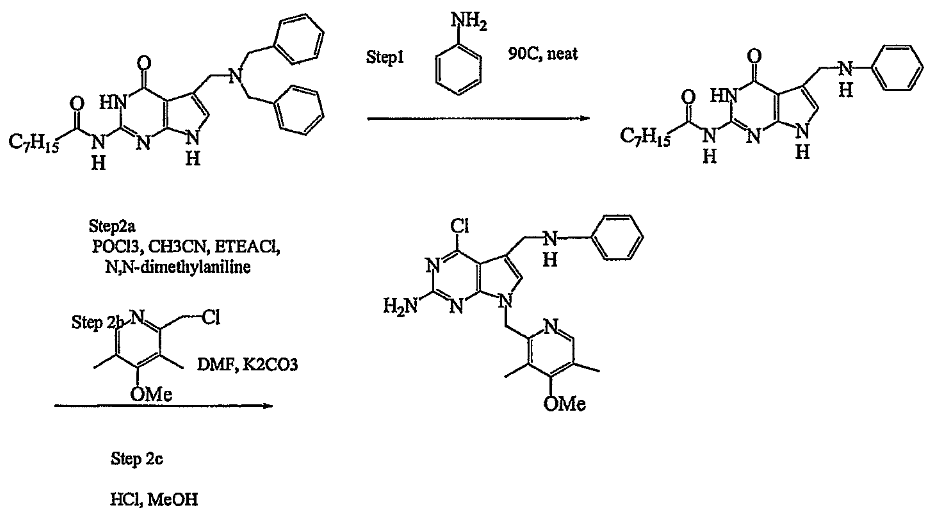


*Step 1.* Octanoic acid (4-chloro-5-phenylaminomethyl-7H-pyrrolo[2,3-d]pyrimidin-2-yl)-amide

A solution of octanoic acid {5-[(dibenzylamino)-methyl]-4-oxo-4,7-dihydro-3H-pyrrolo[2,3-d]pyrimidin-2-yl}-amide (2.42 g, 3 mmol) and aniline (10 mL) was heated to 90 ˚C in a selaed tube overnight, concentrated, filtered, and washed with MeOH (2 mL x3) to give the title compound (1.1 g, 57%). HPLC Rt: 6.327 min. $^1$H-NMR (DMSO-$d_6$): δ 11.77 (s, 1H), 11.47 (s, 1H), 11.37 (s, 1H), 7.05 (m, 2H), 6.85 (s, 1H), 6.62 (m, 2H), 6.52 (m, 1H), 5.58 (t, 1H), 4.31 (d, 2H), 2.43 (t, 2H), 1.58 (m, 2H), 1.27 (m, 8H), 0.86 (t, 3H).

*Step 2.* 4-Chloro-7-(4-methoxy-3,5-dimethyl-pyridin-2-ylmethyl)-5-phenylaminomethyl-7H-pyrrolo[2,3-d]pyrimidin-2-ylamine

A solution of octanoic acid (4-chloro-5-phenylaminomethyl-7H-pyrrolo[2,3-d]pyrimidin-2-yl)-amide (270 mg, 0.68 mmol), $BnNEt_3Cl$ (0.48 g, 1.36 mmol), $PhNMe_2$ (0.17 mL) and $POCl_3$ (0.59 mL, 4.08 mmol) in $CH_3CN$ (3 mL) was heated to 100 ˚C for 40 min and concentrated. The residue was poured into ice water and neutralized with 2N NaOH,

extracted with EtOAc (20 mL ×3), evaporated, to give 2-chloromethyl-4-methoxy-3,5-dimethyl-pyridine as a crude oil (282 mg) which was used without purification. A suspension of this crude (282 mg, 0.68 mmol), 2-chloromethyl-4-methoxy-3,5-dimethyl-pyridine (140 mg, 0.68 mmol) and $Cs_2CO_3$ (266 mg, 0.68 mmol) in dry DMF (1 mL) was heated to 45 ˚C overnight, After work-up (EtOAc) and evaporation, the residue was taken up in methanolic 4N HCl (1 mL), stirred at room temperature for 1 h, and neutralized to pH 7 with 2N NaOH. Extraction with EtOAc (10 mL ×3), evaporation and purification by preparative TLC (MeOH/$CH_2Cl_2$ 10:1) gave the title compound (4.8 mg, 1.6%). HPLC Rt: 4.785 min. $^1$H-NMR ($CDCl_3$): δ 8.12 (s, 1H), 7.18(m, 2H), 6.75(m, 2H), 6.60 (s, 1H), 5.26(s, 2H), 4.93 (s, 2H), 4.74 (s, 2H), 3.70 (s, 3H), 3.00 (s, 3H), 2.23 (s, 3H), 2.16 (s, 3H).

**Example 41. 4-Chloro-7-(4-methoxy-3,5-dimethyl-pyridin-2-ylmethyl)-5-[(methylphenyl-amino)-methyl]-7H-pyrroro[2,3-d]pyrimidin-2-ylamine**

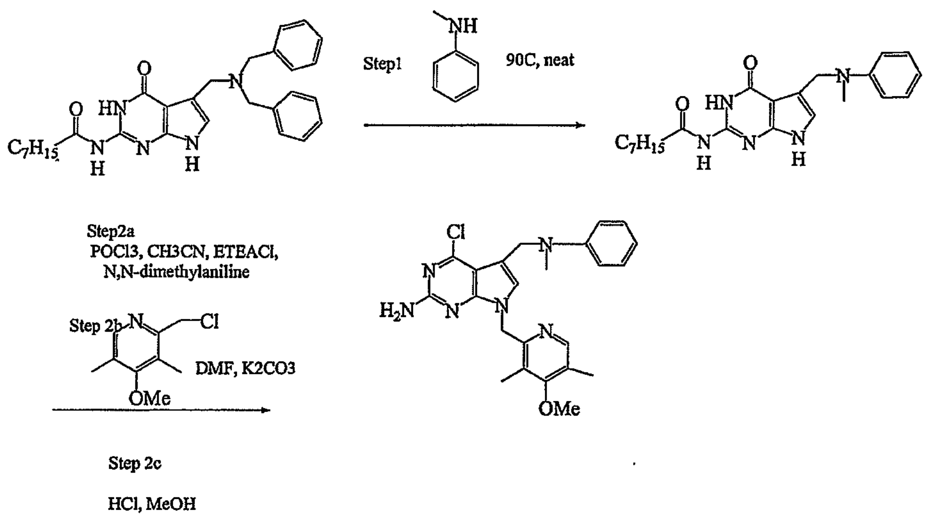


*Step 1:* Octanoic acid (4-chloro-5-[(methyl-phenyl-amino)-methyl]-7H-pyrrolo[2,3-d]pyrimidin-2-yl)-amide

The title compound was obtained by treating octanoic acid {5-[(dibenzylamino)-methyl]-4-oxo-4,7-dihydro-3H-pyrrolo[2,3-d]pyrimidin-2-yl}-amide (2.42 g, 3 mmol) and N-methylaniline (10 mL) as in step 1 of the previous example. HPLC Rt: 6.325 min. $^1$H-NMR (DMSO-$d_6$): δ 11.73 (s, 1H), 11.47 (s, 1H), 11.35 (s, 1H), 7.13 (m, 2H), 6.78 (s, 2H), 6.60 (m, 2H), 4.64(s, 2H), 2.98 (s, 3H), 2.43 (t, 2H), 1.58 (m, 2H), 1.27 (m, 8H), 0.86 (t, 3H).

*Step 2:* 4-Chloro-7-(4-methoxy-3,5-dimethyl-pyridin-2-ylmethyl)-5-[(methyl-phenyl-amino)-methyl]-7H-pyrrolo[2,3-d]pyrimidin-2-ylamine

The title compound was obtained by alkylation of octanoic acid {4-chloro-5-[(methyl-phenyl-amino)-methyl]-7H-pyrrolo[2,3-d]pyrimidin-2-yl}-amide with 2-chloromethyl-4-methoxy-3,5-dimethyl-pyridine and deprotection with 4N HCl as in step 2 of the previous example. HPLC Rt: 4.844 min. $^1$H-NMR ($CDCl_3$): δ 8.20 (s, 1H), 7.16 (m, 2H), 6.87 (s, 1H), 6.70-6.64 (m, 2H), 5.28 (s, 2H), 5.13 (s, 2H), 4.46 (s, 2H), 4.15 ( br s, 1H), 3.73 (s, 3H), 2.25 (s, 3H), 2.18 (s, 3H).

**Example 42. 4-Chloro-7-(4-methoxy-3,5-dimethyl-pyridin-2-ylmethyl)-6-pyrroHdin-1-ylmethyl-7H-pyrrolo [2,3-d]pyrimidin-2-ylamine**

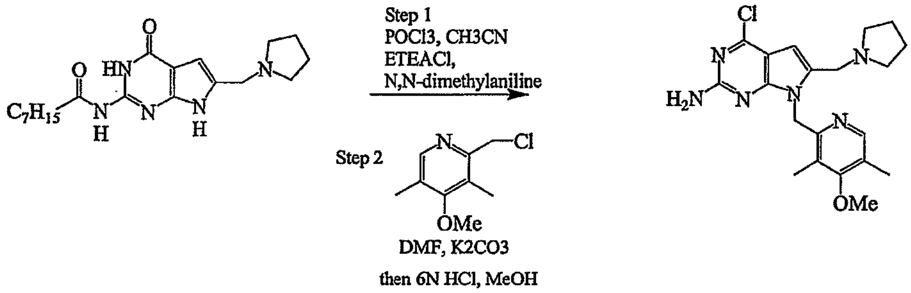


*Step 1.* Octanoic acid (4-chloro-6-pyrrolidin-1-yhnethyl-7H-pyrrolo[2,3-d]pyrimidin-2-yl)-amide

A solution of octanoic acid (4-oxo-6-pyrrolidin-1-ylmethyl-4,7-dihydro-3H-pyrrolo[2,3-d]pyrimidin-2-yl)-amide (0.36 g 1 mmol; J. Chem. Soc. Perkin Trans. 1 1998, 1637), $BnNEt_3Cl$ (0.70 g, 2 mmol), $PhNMe_2$ (0.25 mL) and $POCl_3$ (0.86 mL, 6 mmol) in $CH_3CN$ (5 mL) was heated to 100 ˚C for 40 min and concentrated. The residue was poured into ice water and neutralized with 2N NaOH, extracted with EtOAc (50 mL ×3), and evaporated to give octanoic acid (4-chloro-6-pyrrolidin-1-ylmethyl-7H-pyrrolo[2,3-d]pyrimidin-2-yl)-amide as a crude (0.33 g) which was used without purification. HPLC Rt: 6.737 min. $^1$H-NMR ($CDCl_3$): δ 11.60 (br s, 1H), 10.20 (br s, 1H), 6.38 (s, 1H), 3.86 (s, 2H), 2.90 (m, 2H), 2.70 9s, 4H), 1.86 (s, 4H), 1.78 (t, 2H), 1.32-1.29 (m, 8H), 0.90 (t, 3H).

*Step 2.* 4-Chloro-7-(4-methoxy-3,5-dimethyl-pyridin-2-ylmethyl)-6-pyrrolidin-1-ylmethyl-7H-pyrrolo[2,3-d]pyrimidin-2-ylamine

A suspension of the crude octanoic acid (4-chloro-6-pyrrolidin-1-ylmethyl-7H-pyrrolo[2,3-d]pyrimidin-2-yl)-amide (330 mg, 0.87 mmol), 2-chloromethyl-4-methoxy-3,5-dimethyl-pyridine (162 mg, 0.87 mmol) and $K_2CO_3$ (121 mg, 0.87 mmol) in dry DMF (1 mL) was heated to 45 ˚C for overnight After work-up (EtOAc) and evaporation, the residue was taken up in 6N methanolic HCl (1 mL), stirred at room temperature for 1 h, and neutralized to pH 7 with 2N NaOH. Extraction with EtOAc (10 mL ×3), evaporation and purification by preparative TLC ($MeOH/CH_2Cl_2$ 10: 1) yielded 4-chloro-7-(4-methoxy-3,5-dimethyl-pyridin-2-ylmethyl)-6-pyrrolidin-1-ylmethyl-7H-pyrrolo[2,3-d]pyrimidin-2-ylamine (4.1 mg, yield 1.0 %). HPLC Rt: 6.092 min. $^1$H-NMR ($CDCl_3$): δ 8.09 (s, 1H), 6.31(s, 1H), 5.55(s, 2H), 4.85(s, 2H), 3.77 (s, 3H), 3.52(brs, 2H), 2.43 (m, 4H), 1.76-1.71(m, 4H).

**Example 43. 4-Chloro-5-isopropyl-7-(4-methoxy-3,5-dimethyl-pyridin-2-ylmethyl)-7H-pyrrolo[2,3-d]pyrimidin-2-ylamine**

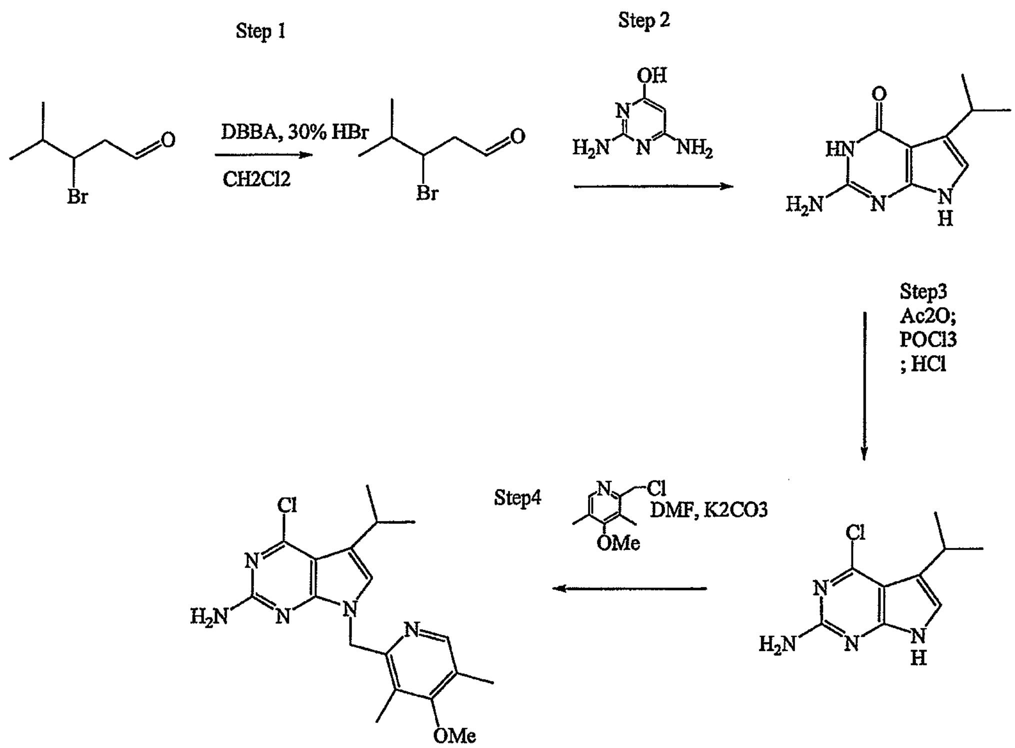


*Step1.* 3-Bromo-4-methyl-pentanal

A mixture of 4-methyl-pentanal (8.60 g, 0.10 mol), 5,5-dibromobarbituric acid (DBBA, 17.15 g, 0.06 mol), 40% HBr (2 mL) and HOAc (1 mL) in $CH_2Cl_2$ (180 mL) was stirred at 25 ˚C for 5 h. after filtration, the filtrate was washed with 1N $Na_2SO_3$, $Na_2CO_3$, and brine, dried with $Na_2SO_4$, and evaporated to give 3-bromo-4-methyl-pentanal (8.76 g, 53%). $^1$H-NMR ($CDCl_3$): δ 9.40 (s, 1H), 4.40 (t, 1H), 2.10 (m, 1H), 1.06 (s, 3H), 1.05 (s, 3H).

*Step2.* 2-Amino-5-isopropyl-3,7-dihydro-pyrrolo[2,3-d]pyrimidin-4-one

A suspension of 4-diamino-6-hydroxypyrimidine (6.68 g, 50 mmol), AcONa (8.3 g 100 mmol) and 3-bromo-4-methyl-pentanal (8.76 g, 50 mmol) in $CH_3CN$ (100 mL) and $H_2O$ (100 mL) was stirred at 25 ˚C overnight whereupon the starting materials gradually dissolved and the desired pyrrolo[2,3-d]pyrimidine precipitated. The precipitate was collected by filtration and washed with MeOH to give 2-amino-5-isopropyl-3,7-dihydro-pyrrolo[2,3-d]pyrimidin-4-one (3.80 g, 40%). HPLC Rt: 4.408 min. $^1$H-NMR (DMSO-$d_6$). δ 10.58 (s, 1H), 10.10 (s, 1H), 6.30 (s, 1H), 5.97 (s, 2H), 3.03 (7, 1H), 1.20 (s, 3H), 1.19 (s, 3H).

*Step 3.* 4-Chloro-5-isopropyl-7H-pyrrolo[2,3-d]pyrimidin-2-ylamine

A mixture of 2-amino-5-isopropyl-3,7-dihydro-pyrrolo[2,3-d]pyrimidin-4-one and acetic anhydride (20 mL) was heated to reflux for 3 h and evaporated. The residue was treated with $BnNEt_3Cl$ (8.99 g, 40 mmol), $PhNMe_2$ ( 4.9 mL) and $POCl_3$ (17 mL, 120 mmol) in $CH_3CN$ (100 mL) at 100 ˚C for 40 min and concentrated. The residue was poured into ice water and neutralized with 2N NaOH, extracted with EtOAc (80 mL x 3), and evaporated to give an oil which was digested with methanolic 4N HCl (50 mL) at 50 ˚C for 2 h. After cooling, and neutralization to pH 7 with 2N NaOH, the solid was collected by filtration and dried to give 4-chloro-5-isopropyl-7H-pyrrolo[2,3-d]pyrimidin-2-ylamine (1.88 g, 45%). HPLC Rt: 5.796 min. $^1$H-NMR (DMSO-$d_6$): δ 11.170 (s, 1H), 6.82 (s, 1H), 6.42 (s, 2H), 3.24 (7, 1H), 1.25 (s, 3H), 1.23 (s, 3H).

*Step 4.* 4-Chloro-5-isopropyl-7-(4-methoxy-3,5-dimethyl-pyridin-2-ylmethyl)-7H-pyrrolo[2,3-d]pyrimidin-2-ylamine

A suspension of 4-chloro-5-isopropyl-7H-pyrrolo[2,3-d]pyrimidin-2-ylamine (105 mg, 0.5 mmol), 2-chloromethyl-4-methoxy-3,5-dimethyl-pyridine (93 mg, 0.5 mmol) and $K_2CO_3$ (85 mg, 0.6 mmol) in dry DMF (1 mL) was heated

to 45 ˚C overnight, Work-up (EtOAc), evaporation, and purification by preparative TLC ( $MeOH/CH_2Cl_2$ 10:1) gave 4-chloro-5-isopropyl-7-(4-methoxy-3,5-dimethyl-pyridin-2-ylmethyl)-7H-pyrrolo[2,3-d]pyrimidin-2-ylamine (36 mg). HPLC Rt: 5.867 min. $^1$H-NMR (DMSO-$d_6$): δ 8.07 (s, 1H), 6.74(s, 1H), 6.51(s, 2H), 5.22 (s, 2H), 3.70 0s, 3H), 3.23 (7, 1H), 2.21 (s, 3H), 2.15 (s, 3H).

**Example 44. 4-chloro-7-(4-chloro-3-methyl-pyridin-2-ylmethyl)-5-isopropyl-7H-pyrrolo[2,3-d]pyrimidin-2-ylamine**

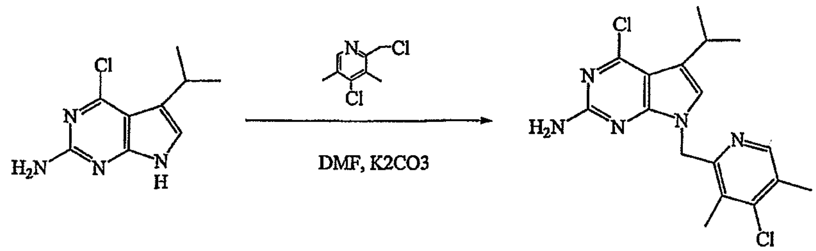


The title compound was obtained by alkylation of 4-chloro-5-isopropyl-7H-pyrrolo[2,3-d]pyrimidin-2-ylamine with 4-chloro-2-chloromethyl-3,5-dimethyl-pyridine according to the general procedure 1.3. HPLC Rt: 6.997 min. $^1$H-NMR ($CDCl_3$): δ 8.27 (s, 1H), 6.61 (s, 1H), 5.33 (s, 2H), 5.09 (s, 2H), 3.35 (7, 1H), 2.35 (s, 6H), 1.25 (s, 3H), 1.23 (s, 3H).

**Example 45. 4-Chloro-7-(4-chloro-3-methyl-1-oxy-pyridin-2-ylmethyl)-5-isopropyl-7H-pyrrolo[2,3-d]pyrimidin-2-ylamine**

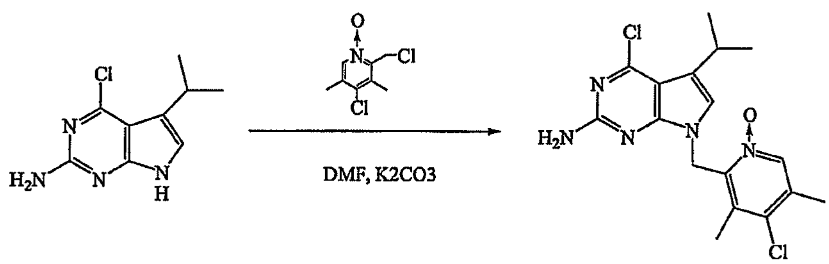


The title compound was obtained by alkylation of 4-chloro-5-isopropyl-7H-pyrrolo[2,3-d]pyrimidin-2-ylamine with 2-chloromethyl-3,5-dimethyl-pyridine 1-oxide according to the general procedure 1.3. HPLC Rt: 6.753 min. $^1$H-NMR (DMSO-$d_6$): δ 8.37 (s, 1H), 7.03 (s, 1H), 6.63 (s, 2H), 5.40 (s, 2H), 3.20 (7, 1H), 2.59 (s, 3H), 2.27 (s, 3H), 1.20 (s, 3H), 1.19 (s, 3H).

**Example 46. 4-Chloro-5-(2-isobutylamino-ethyl)-7-(4-methoxy-3,5-dimethyl-pyridin-2-ylmethyl)-7H-pyrrolo [2,3-d]pyrimidin-2-ylamine**

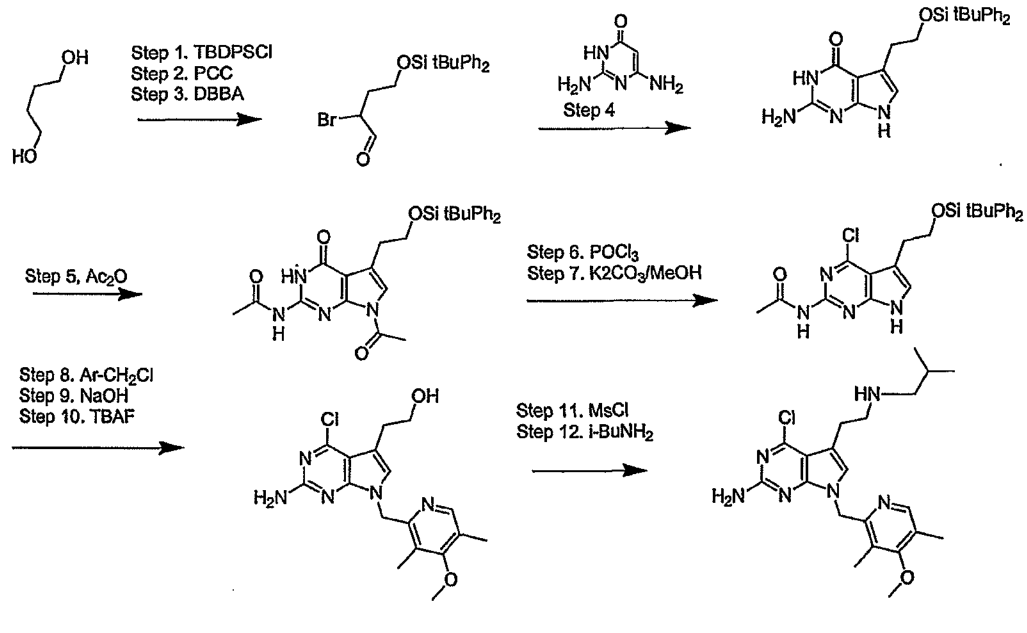


*Step 1.* 4-(tert-Butyl-diphenyl-silanyloxy)-butan-1-ol

A mixture of *t*$BuPh_2SiCl$ (25 mL, 98 mmol), 1,4-butanediol (25 mL, 281 mmol), $iPrNEt_2$ (50 mL, 303 mmol) and $CH_2Cl_2$ (50 mL) was stirred at rt for 14 h, concentrated, diluted with diethyl ether, washed with water (3 x) and brine. Drying ($Na_2SO_4$) and concentration afforded the title compound as a clear oil (29.8 g, 93%) which was used without further purification. Rf (EtOAc:hexane 1:4) 0.3. $^1$H-NMR ($CDCl_3$): δ 7.71 (dd, 4H), 7.43 (m, 6H), 3.74 (t, 2H), 3.70 (q, 2H), 2.10 (br. t, 1H), 1.69 (m, 4H), 1.08 (s, 9H).

*Step 2.* 4-(tert-Butyl-diphenyl-silanyloxy)-butyraldehyde

A solution of 4-(tert-butyl-diphenyl-silanyloxy)-butan-1-ol (29.8 g, 91 mmol) in $CH_2Cl_2$ (70 mL) was added to a slurry of PCC (21.5 g, 100 mmol), celite (50 g) and $CH_2Cl_2$ (300 mL). The mixture was stirred for 2 h at rt, and the celite was removed by filtration and washed with $CH_2Cl_2$ (300 mL). Concentration and chromatography (EtOAc/hexane 1:4) afforded the title compound as a clear oil (22.2 g, 75%). Rf (EtOAc:hexane 1:4) 0.7. $^1$H-NMR ($CDCl_3$): δ 9.82 (t, 1H), 7.68 (dd, 4H), 7.41 (m, 6H), 3.71 (t, 2H), 2.57 (t, 2H), 1.91 (q, 2H), 1.07 (s, 9H).

*Step 3.* 2-Bromo-4-(tert-butyl-diphenyl-silanyloxy)-butyraldehyde

A mixture of 4-(tert-butyl-diphenyl-silanyloxy)-butyraldehyde (22.2 g, 68 mmol), 5,5-dibromobarbituric acid (12.1 g, 43 mmol) and $CH_2Cl_2$ (80 mL) was treated with 70% aq HBr (1 mL, 14 mmol) and stirred at rt for 1 h. The by-product (barbituric acid) was removed by filtration and washed with $CH_2Cl_2$ (100 mL). The combined organic layers were washed (1N $Na_2S_2O_3$, 5% $NaHCO_3$, half-sat. brine) and dried ($Na_2SO_4$). Concentration gave the title compound as a clear oil (25.3 g, 92%) which was used without further purification. Rf (EtOAc:hexane 1:4) 0.7. $^1$H-NMR ($CDCl_3$): δ 9.55 (d, 1H), 7.68 (dd, 4H), 7.41 (m, 6H), 4.60 (ddd, 1H), 3.84 (m, 2H), 2.35 (m, 1H), 2.10 (m, 1H), 1.07 (s, 9H).

*Step 4.* 2-Amino-5-[2-(tert-butyl-diphenyl-silanyloxy)-ethyl]-3,7-dihydro-pyrrolo[2,3-d]pyrimidin-4-one

The title compound was obtained by treating 2-bromo-4-(tert-butyl-diphenyl-silanyloxy)-butyraldehyde (25. 3 g, 62 mmol) with 2,4-diamino-6-hydroxypyrimidine (10.2 g, 124 mmol) according to the general procedure 1.1 (23.4 g, 87%). HPLC Rt: 6.981 min. $^1$H-NMR ($CDCl_3$): δ 10.67 (s, 1H), 10.14 (s, 1H), 7.55 (m, 4H), 7.38 (m, 6H), 6.37 (s, 1H), 5.98 (s, 2H), 3.86 (t, 2H), 2.86 (t, 2H), 0.95 (s, 9H).

*Step 5.* N-{7-Acetyl-5-[2-(tert-butyl-diphenyl-silanyloxy)-ethyl]-4-oxo-4,7-dihydro-3H-pyrrolo[2,3-d]pyrimidin-2-yl}-acetamide

A solution of 2-amino-5-[2-(tert-butyl-diphenyl-silanyloxy)-ethyl]-3,7-dihydro-pyrrolo[2,3-d]pyrimidin-4-one

(22.7 g, 52 mmol) in $AC_2O$ (200 mL) was heated to 110 ˚C for 2.5 h, concentrated,diluted with toluene (300 mL) and concentrated again to afford the title compound to afford the title compound as a crude brown oil (27 g) which was used without further purification. An aliquot was purified by chromatography for characterization. HPLC Rt: 8.349 min. $^1$H-NMR ($CDCl_3$): δ 11.77 (s, 1H), 8.81 (s, 1H), 7.61 (dd, 4H), 7.30 (m, 7H), 6.37 (s, 1H), 4.00 (t, 2H), 3.02 (t, 2H), 2.70 (s, 3H), 2.23 (s, 3H), 1.04 (s, 9H).

*Step 6.* N-{7-Acetyl-5-[2-(tert-butyl-diphenyl-silanyloxy)-ethyl]4-chloro-7H-pyrrolo[2,3-d]pyrimidin-2-yl}-acetamide

A solution of crude N-{7-acetyl-5-[2-(tert-butyl-diphenyl-silanyloxy)-ethyl]-4-oxo-4,7-dihydro-3H-pyrrolo[2,3-d] pyrimidin-2-yl}-acetamide (26.4 g, 51 mmol), $BnNEt_3Cl$ (23.2 g, 102 mmol), $PhNMe_2$ (19.6 mL, 153 mmol) and $POCl_3$ (9.3 mL, 77 mmol) in $CH_3CN$ (100 mL) was heated to 80 ˚C for 1.5 h. The mixture was diluted with EtOAc (800 mL), washed (sat. $NaHCO_3$, brine) and concentrated to afford the title compound as an oil (46 g) which was used without further purification. An aliquot was purified by chromatography for characterization. HPLC Rt: 8.562 min. $^1$H-NMR ($CDCl_3$): δ 8.05 (s, 1H), 7.68 (s, 1H), 7.57 (dd, 4H), 7.40 (m, 6H), 3.99 (t, 2H), 3.06 (t, 2H), 2.98 (s, 3H), 2.52 (s, 3H), 1.04 (s, 9H).

*Step 7.* N-{5-[2-(tert-Butyl-diphenyl-silanyloxy)-ethyl]-4-chloro-7H-pyrrolo[2,3-d]pyrimidin-2-yl}-acetamide

A solution of crude N-{7-acetyl-5-[2-(tert-butyl-diphenyl-silanyloxy)-ethyl]-4-chloro-7H-pyrrolo[2,3-d]pyrimidin-2-yl}-acetamide (46 g) in MeOH (150 mL) was treated with $K_2CO_3$ (8.0 g, 58 mmol) at rt for 15 min. Filtration, concentration, and chromatography (EtOAc/hexane 1:1) afforded the title compound as an oil contaminated with residual PhNMe2 from step 6. The oil was diluted with EtOAc (40 mL) and treated with hexane (40 mL) to obtained the desired product as a pale yellow precipitate (4.2 g, 16% over 3 steps). HPLC Rt: 8.558 min. $^1$H-NMR ($CDCl_3$): δ 11.75 (br. s, 1H), 11.35 (br. s, 1H), 7.60 (dd, 4H), 7.37 (m, 6H), 3.97 (t, 2H), 3.10 (t, 2H), 2.57 (s, 3H), 1.06 (s, 9H).

*Step 8.* N-[5-[2-(tert-Butyl-diphenyl-silanyloxy)-ethyl]-4-chloro-7-(4-methoxy-3,5-dimethyl-pyridin-2-ylmethyl)-7H-pyrrolo[2,3-d]pyrimidin-2-yl]-acetamide

A mixture of N-{5-[2-(tert-butyl-diphenyl-silanyloxy)-ethyl]-4-chloro-7H-pyrrolo[2,3-d]pyrimidin-2-yl}-acetamide (344 mg, 0.70 mmol), 2-chloromethyl-4-methoxy-3,5-dimethyl-pyridine hydrochloride (175 mg, 0.77 mmol), $K_2CO_3$ (516 mg, 3.7 mmol) and DMF (3.0 mL) was stirred at rt overnight. Work-up (EtOAc/water; brine) afforded the title compound as an off-white solid which was used without further purification (516 mg, "115%"). HPLC Rt: 8.419 min. $^1$H-NMR ($CDCl_3$): δ 8.20 (s, 1H), 7.95 (s, 1H), 7.55 (dd, 4H), 7.32 (m, 6H), 7.04 (s, 1H), 5.37 (s, 2H), 3.91 (t, 2H), 3.73 (s, 3H), 3.06 (t, 2H), 2.57 (s, 3H), 2.26 (s, 3H), 2.25 (s, 3H), 0.97 (s, 9H).

*Step 9.* 5-[2-(tert-Butyl-diphenyl-silanyloxy)-ethyl]-4-chloro-7-(4-methoxy-3,5-dimethyl-pyridin-2-ylmethyl)-7H-pyrrolo [2,3-d]pyrimidin-2-ylamine

A solution of N-[5-[2-(tert-Butyl-diphenyl-silanyloxy)-ethyl]-4-chloro-7-(4-methoxy-3,5-dimethyl-pyridin-2-ylmethyl)-7H-pyrrolo[2,3-d]pyrimidin-2-yl]-acetamide (511 mg) in THF (3 mL) and MeOH (3 mL) was treated with NaOH 2M (3 mL) at 45 ˚C for 1.5 h. Work-up and chromatography (EtOAc/hexane 1:1) afforded the title compound as a white powder (290 mg, 69% over 2 steps). HPLC Rt: 8.198 min. $^1$H-NMR ($CDCl_3$): δ 8.20 (s, 1H), 7.57 (dd, 4H), 7.40 (m, 2H), 7.32 (m, 4H), 6.69 (s, 1H), 5.26 (s, 2H), 4.90 (s, 2H), 3.98 (t, 2H), 3.66 (s, 3H), 3.00 (t, 2H), 2.24 (s, 3H), 2.18 (s, 3H), 0.96 (s, 9H).

*Step 10.* 2-[2-Amino-4-chloro-7-(4-methoxy-3,5-dimethyl-pyridin-2-ylmethyl)-7H-pyrrolo[2,3-d]pyrimidin-5-yl]-ethanol

A solution of 5-[2-(tert-butyl-diphenyl-silanyloxy)-ethyl]-4-chloro-7-(4-methoxy-3,5-dimethyl-pyridin-2-ylmethyl)-7H-pyrrolo[2,3-d]pyrimidin-2-ylamine (246 mg, 0.41 mmol) in THF (5 mL) was treated with TBAF (1N in THF, 0.5 mL, 0.50 mmol) at rt for 1 h. Work-up (EtOAc/water, brine) gave the crude product as an oil, which was diluted with diethyl ether (15 mL) whereupon the desired product precipitated out of solution as a white powder (110 mg, 74%). HPLC Rt: 4.474 min. $^1$H-NMR ($CDCl_3$): δ 8.21 (s, 1H), 6.78 (s, 1H), 5.30 (s, 2H), 4.93 (s, 2H), 3.87 (t, 2H), 3.76 (s, 3H), 3.03 (t, 2H), 2.26 (s, 3H), 2.23 (s, 3H).

*Step 11.* Methanesulfonic acid 2-[2-amino-4-chloro-7-(4-methoxy-3,5-dimethyl-pyridin-2-ylmethyl)-7H-pyrrolo[2,3-d]pyrimidin-5-yl]-ethyl ester

A solution of 2-[2-amino-4-chloro-7-(4-methoxy-3,5-dimethyl-pyridin-2-ylmethyl)-7H-pyrrolo[2,3-d]pyrimidin-5-yl]-ethanol (11.6 mg, 0.031 mmol) and $Et_3N$ (30 ul, 0.22 mmol) in THF (2 mL) was treated with MsCl (11 uL, 0.14 mmol) at rt for 15 min to give a solution of the title compound which was used without further purification. In a separate experiment, the material was purified by preparative TLC (EtOAc 100%). HPLC Rt: 4.765 min. $^1H$-NMR ($CDCl_3$): δ 8.22 (s, 1H), 6.80 (s, 1H), 5.31 (s, 2H), 4.93 (s, 2H), 4.42 (t, 2H), 3.77 (s, 3H), 2.98 (t, 2H), 2.85 (s, 3H), 2.23 (s, 3H), 2.07 (s, 3H).

*Step 12.* 4-Chloro-5-(2-isobutylamino-ethyl)-7-(4-methoxy-3,5-dimethyl-pyridin-2-ylmethyl)-7H-pyrrolo[2,3-d]pyrimidin-2-ylamine

The solution of methanesulfonic acid 2-[2-amino-4-chloro-7-(4-methoxy-3,5-dimethyl-pyridin-2-ylmethyl)-7H-pyrrolo[2,3-d]pyrimidin-5-yl]-ethyl ester in THF obtained from step 11 was diluted with i-$BuNH_2$ (4 mL) and heated to 50 ˚C for 15 h. Concentration, work-up(EtOAc/$NaHCO_3$ sat.; brine) and preparative TLC (MeOH:$Et_3N$:$CH_2Cl_2$ 7:3:100) gave the title compound as a colorless oil (6 mg, 50 %). HPLC Rt: 4.263 min. $^1H$-NMR ($CDCl_3$): δ 8.20 (s, 1H), 6.79 (s, 1H), 5.29 (s, 2H), 4.97 (s, 2H), 3.76 (s, 3H), 3.04 (t, 2H), 2.96 (t, 2H), 2.53 (d, 2H), 2.26 (s, 3H), 2.22 (s, 3H), 1.85 (oct., 1H), 0.90 (d, 6H).

**Example 47. 2-Amino-4-chloro-7-(4-methoxy-3,5-dimethyl-pyridin-2-ylmethyl)-5,7-dihydro-pyrrolo[2,3-d]pyrimidin-6-one**

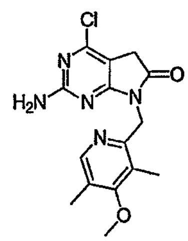

The title compound was obtained by condensation between (2-amino-4,6-dichloro-pyrimidin-5-yl)-acetic acid ethyl ester and C-(4-methoxy-3,5-dimethyl-pyridin-2-yl)-methylamine according to the general procedure 1.2. HPLC Rt: 4.893 min. $^1H$-NMR ($CDCl_3$): δ 8.07 (s, 1H), 5.03 (s, 2H), 4.92 (s, 2H), 3.77 (s, 3H), 3.57 (s, 2H), 2.31 (s, 3H), 2.20 (s, 3H).

**Example 48. 2-Amino-4-chloro-7-(4-chloro-3,5-dimethyl-pyridin-2-ylmethyl)-5,7-dihydro-pyrrolo[2,3-d]pyrimidin-6-one**

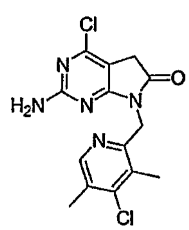

The title compound was obtained by alkylation of 4-chloro-pyrrolo[2,3-d]pyrimidin-6-one with 2-chloromethyl-4-chloro-3,5-dimethyl-pyridine according to the general procedure 1.3. HPLC Rt: 5.367 min. $^1H$-NMR ($CDCl_3$): δ 8.09 (s, 1H), 5.02 (s, 2H), 4.96 (s, 2H), 3.57 (s, 2H), 2.45 (s, 3H), 2.29 (s, 3H).

**Example 49. 2-Amino-4-chloro-7-(3,5-dimethyl-4-methoxy-1-oxy-pyridin-2-ylmethyl)-5,7-dihydro-pyrrolo[2,3-d]pyrimidin-6-one**

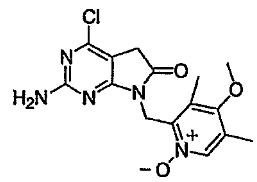

The title compound was obtained by oxidation of 2-amino-4-chloro-7-(4-methoxy-3,5-dimethyl-pyridin-2-ylmethyl)-5,7-dihydro-pyrrolo[2,3-d]pyrimidin-6-one with m-CPBA according to the general procedure 2.1. HPLC Rt: 4.763 min.$^1$H-NMR (DMSO-d6): δ 8.01 (s, 1H), 7.01 (s, 2H), 4.93 (s, 2H), 3.73 (s, 3H), 3.46 (s, 2H), 2.40 (s, 3H), 2.19 (s, 3H).

**Example 50. 2-Amino-4-chloro-7-(4-chloro-3,5-dimethyl-1-oxy-pyridin-2-ylmethyl)-5,7-dihydro-pyrrolo[2,3-d] pyrimidin-6-one**

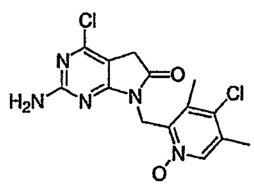

The title compound was obtained by oxidation of 2-amino-4-chloro-7-(4-chloro-3,5-dimethyl-pyridin-2-ylmethyl)-5,7-dihydro-pyrrolo[2,3-d]pyrimidin-6-one with m-CPBA according to the general procedure 2.1. HPLC Rt: 4.90 min.$^1$H-NMR ($CDCl_3$/$CD_3OD$): δ 7.96 (s, 1H), 5.12 (s, 2H), 3.38 (s, 2H), 2.47 (s, 3H), 2.27 (s, 3H).

**Example 51. 2-Amino-4-chloro-7-(3,4,5-trimethoxy-benzyl)-5,7-dihydro-pyrrolo[2,3-d]pyrimidin-6-one**

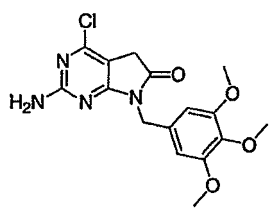

The title compound was obtained by condensation between (2-Amino-4,6-dichloro-pyrimidin-5-yl)-acetic acid ethyl ester and 3,4,5-Trimethoxy-benzylamine according to the general procedure 1.2. HPLC Rt: 6.391 min.$^1$H-NMR ($CDCl_3$): δ 6.70 (s, 2H), 5.14 (s, 2H), 4.77 (s, 2H), 3.84 (s, 6H), 3.81 (s, 3H), 3.47 (s, 2H).

**Example 52. 2-Amino-4-chloro-7-(2-bromo-3,4,5-trimethoxy-benzyl)-5,7-dihydro-pyrrolo[2,3-d]pyrimidin-6-one**

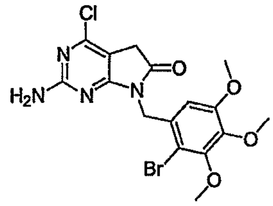

The title compound was obtained by treating 2-amino-4-chloro-7-(3,4,5-trimethoxy-benzyl)-5,7-dihydro-pyrrolo [2,3-d]pyrimidin-6-one with bromine in acetic acid according to the general procedure 3.1. HPLC Rt: 7.150 min.$^1$H-NMR ($CDCl_3$): δ 6.49 (s, 1H), 5.14 (s, 2H), 4.94 (s, 2H), 3.90 (s, 3H), 3.86 (s, 3H), 3.75 (s, 3H), 3.55 (s, 2H).

**Example 53. 2-Amino-4-chloro-7-(4-methoxy-3,5-dimethyl-pyridin-2-ylmethyl)-5-methyl-5,7-dihydro-pyrrolo [2,3-d]pyrimidin-6-one**

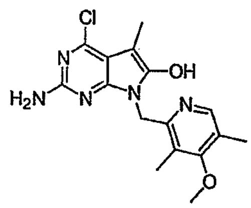

The title compound was obtained by alkylation of2-amino-4-chloro-7-(4-methoxy-3,5-dimethyl-pyridin-2-ylmethyl)-5,7-dihydro-pyrrolo[2,3-d]pyrimidin-6-one with iodomethane according to the general procedure 1.5. HPLC Rt: 4.091 min.$^1$H-NMR ($CDCl_3$): δ 8.01 (s, 1H), 5.15 (s, 2H), 4.93 (d, 1H), 4.87 (d, 1H), 3.76 (s, 3H), 3.51 (s, 1H), 2.29 (s, 3H), 2.20 (s, 3H), 1.78 (s, 3H).

**Example 54. 2-Amino-4-chloro-7-(4-methoxy-3,5-dimethyl-pyridin-2-ylmethyl)-5,5-dimethyl-5,7-dihydro-pyrrolo[2,3-d]pyrimidin-6-one**

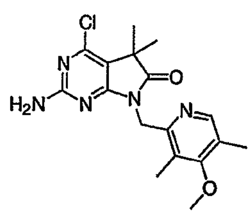

The title compound was obtained by alkylation of 2-amino-4-chloro-7-(4-methoxy-3,5-dimethyl-pyridin-2-ylmethyl)-5,7-dihydro-pyrrolo[2,3-d]pyrimidin-6-one with iodomethane according to the general procedure 1.5. HPLC Rt: 5.002 min.$^1$H-NMR ($CDCl_3$): δ 8.02 (s, 1H), 5.02 (s, 2H), 4.90 (s, 2H), 3.75 (s, 3H), 2.29 (s, 3H), 2.18 (s, 3H), 1.53 (s, 6H).

**Example 55. 2-Amino-4-chloro-7-(2-bromo-3,4,5-trimethoxy-benzyl)-5,5-dimethyl-5,7-dihydro-pyrrolo[2,3-d] pyrimidin-6-one**

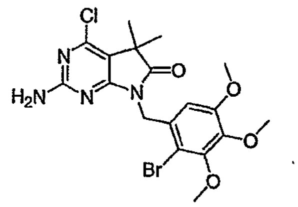

The title compound was obtained by alkylation of 2-amino-4-chloro-7-(3,4,5-trimethoxy-benzyl)-5,7-dihydro-pyrrolo[2,3-d]pyrimidin-6-one with iodomethane according to the general procedure 1.5. HPLC Rt: 6.944 min.$^1$H-NMR ($CDCl_3$): δ 6.34 (s, 1H), 5.09 (s, 2H), 4.93 (s, 2H), 3.90 (s, 3H), 3.86 (s, 3H), 3.71 (s, 3H), 1.52 (s, 6H).

**Example 56. 4-Chloro-5-hydroxy-2-imino-7-(4-methoxy-3,5-dimethyl-pyridin-2-ylmethyl)-2,7-dihydro-pyrrolo [2,3-d]pyrimidin-6-one**

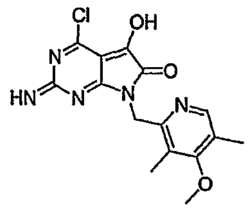

The title compound was obtained by oxidation of 2-amino-4-chloro-7-(4-methoxy-3,5-dimethyl-pyridin-2-ylmethyl)-5,7-dihydro-pyrrolo[2,3-d]pyrimidin-6-one with selenium dioxide according to the general procedure 1.6. HPLC Rt: 4.294 min.$^1$H-NMR ($CDCl_3$): δ 8.04 (s, 1H), 5.93 (s, 1H), 5.76 (s, 1H), 4.97 (s, 2H), 3.765 (s, 3H), 2.29 (s, 3H), 2.20 (s, 3H).

**Example 57. 4-Chloro-5-hydroxy-2-imino-7-(3,4,5-trimethoxy-benzyl)-2,7-dihydro-pyrrolo[2,3-d]pyrimidin-6-one**

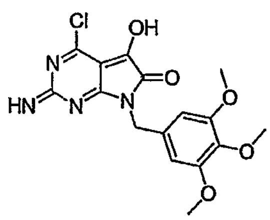

The title compound was obtained by oxidation of 2-amino-4-chloro-7-(3,4,5-trimethoxy-benzyl)-5,7-dihydro-pyrrolo[2,3-d]pyrimidin-6-one with selenium dioxide according to the general procedure 1.6. HPLC Rt: 6.156 min.$^1$H-NMR ($CDCl_3$): δ 6.68 (s, 2H), 6.12 (s, 1H), 5.93 (s, 1H), 4.84 (s, 2H), 3.86 (s, 6H), 3.83 (s, 3H).

**Example 58. 4-Chloro-5-hydroxy-2-imino-7-(2-bromo-3,4,5-trimethoxy-benzyl)-2,7-dihydro-pyrrolo[2,3-d]pyrimidin-6-one**

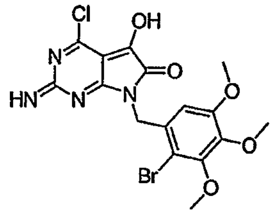

The title compound was obtained by oxidation of 4-chloro-5-hydroxy-2-imino-7-(2-bromo-3,4,5-trimethoxy-benzyl)-2,7-dihydro-pyrrolo[2,3-d]pyrimidin-6-one according to the general procedure 1.6. HPLC Rt: 6.230 min. $^1$H-NMR ($CDCl_3$): δ 6.57 (s, 1H), 6.14 (s, 1H), 5.91 (s, 1H), 5.01 (s, 2H), 3.90 (s, 3H), 3.87 (s, 3H), 3.78.(s, 3H).

BIOLOGY EXAMPLES

**Example A. rHSP90 Competitive Binding Assay**

Five microgram of purified rHSP90 protein (Stressgen, BC, Canada, #SPP-770) in phosphated buffered saline (PBS) was coated on 96 well plates by incubating overnight at 4˚C. Unbound protein was removed and the coated wells were washed twice with 200 μL PBS. DMSO controls (considered as untreated samples) or test compounds were then added at 100-30-10-3-1-0.3 μM dilutions (in PBS), the plates mixed for 30 seconds on the plate shaker, and then incubated for 60 min. at 37 ˚C. The wells were washed twice with 200 μL PBS, and 10 μM biotinylated-geldanamycin (biotin-GM) was added and incubated for 60 min. at 37 ˚C. The wells were washed again twice with 200 μL PBS, before the addition of 20 μg/mL streptavidin-phycoerythrin (streptavidin-PE) (Molecular Probes, Eugene, OR) and incubation for 60 min. at 37˚C. The wells were washed again twice with 200 μL PBS. Relative fluorescence units (RFU) was measured using a SpectraMax Gemini XS Spectrofluorometer (Molecular Devices, Sunnyvale, CA) with an excitation at 485 nm and emission at 580 nm; data was acquired using SOFTmaX®PRO software (Molecular Devices Corporation, Sunnyvale, CA). The background was defined as the RFU generated from wells that were not coated with HSP90 but were treated with the biotin-GM and streptavidin-PE. The background measurements were substrated from each sample treated with biotin-GM and streptavidin-PE measurements before other computation. Percent inhibition of binding for each sample was calculated from the background subtracted values as follows:

$$\% \text{ binding inhibition} = [\text{RFU untreated} - \text{RFU treated}]/\text{RFU untreated}] \times 100.$$

**Example B. Cell Lysate Binding Assay**

MCF7 breast carcinoma cell lysates were prepared by douncing in lysing buffer (20 mM HEPES, pH 7.3, 1 mM EDTA, 5 mM $MgCl_2$, 100 mM KCl), and then incubated with or without test compound for 30 mins at 4 ˚C, followed by incubation with biotin-GM linked to BioMag™ streptavidin magnetic beads (Qiagen) for 1 hr at 4 ˚C. The tubes were placed on a magnetic rack, and the unbound supernatant removed. The magnetic beads were washed three times in lysis buffer and boiled for 5 mins at 95 ˚C in SDS-PAGE sample buffer. Samples were analyzed on SDS protein gels, and Western blots done for rHSP90. Bands in the Western Blots were quantitated using the Bio-rad Fluor-S MultiImager, and the % inhibition of binding of rHSP90 to the biotin-GM was calculated.

The lysate binding ability of selected compounds of the invention based on the above assay is summarized in Table 5. The $IC_{50}$ reported is the concentration of test compound needed to achieve 50% inhibition of the biotin-GM binding to rHSP90 in the MCF7 cell lysates.

**Example C. HER2 Degradation Assay**

MCF7 breast carcinoma cells (ATCC) were grown in Dulbecco's modified Eagle's medium (DMEM) containing 10% fetal bovine serum (FBS) and 10 mM HEPES, and plated in 24 well plates (50% confluent). Twenty-four hrs later (cells are 65-70% confluent), test compounds were added and incubated overnight for 16 h. For the less potent compounds, the amounts added were 100 μM, 30 μM, 10 μM and 1 μM, and for more potent compounds, the amounts added were 1 μM, 0.3 μM, 0.1 μM, 0.03 μM, 0.01 μM and 0.003 μM. The wells were washed with 1 mL phosphate buffered saline (PBS), and 200 μL trypsin was added to each well. After trypsinization was complete, 50 μL of FBS was

added to each well. Then 200 μL cells was transferred to 96 well plates. The cells were pipetted up and down to obtain a single cell suspension. The plates were centrifuged at 2,500 rpm for 1 min using a Sorvall Legend RT™ tabletop centrifuge (Kendro Laboratory Products, Asheville, NC). The cells were then washed once in PBS containing 0.2% BSA and 0.2% sodium azide (BA buffer). Phycoerythrin (PE) conjugated anti HER2/Neu antibody (Becton Dickinson, #340552), or PE conjugated anti-keyhole limpet hemacyanin [KLH] (Becton Dickinson, #340761) control antibody was added at a dilution of 1:20 and 1:40 respectively (final concentration was 1 μg/mL) and the cells were pipeted up and down to form a single cell suspension, and incubated for 15 mins. The cells were washed twice with 200 μL, BA buffer, and resuspended in 200 μL BA buffer, and transferred to FACSCAN tubes with an additional 250 μL BA buffer. Samples were analyzed using a FACSCalibur™ flow cytometer (Becton Dickinson, San Jose, CA) equipped with Argon-ion laser that emits 15 mW of 488 nm light for excitation of the PE fluorochrome. 10,000 events were collected per sample. A fluorescence histogram was generated and the mean fluorescence intensity (MFI) of each sample was determined using Cellquest software. The background was defined as the MFI generated from cells incubated with control IgG-PE, and was subtracted from each sample stained with the HER2/Neu antibody. Cells incubated with DMSO was always done as untreated controls since the compounds were resuspended in DMSO. Percent degradation of HER2 was calculated as follows:

% HER2 degraded = [(MFI untreated cells - MFI treated cells)/MFI untreated cell] x 100

The HER2 degradation ability of selected compounds of the invention based on this assay is summarized in Table 5. $IC_{50}$ is defined as the concentration at which there was 50% degradation of the HER2/Neu protein.

**Example D: MTS Assay**

MTS assays measures the cytotoxicity of geldanamycin derivatives. MTS (3-(4,5-dimethylthiazol-2-yl)-5-(3-carboxymethoxyphenyl)-2-(4-sulfophenyl)-2H-tetrazolium is a tetrazolium dye that is converted to a formazan product by dehydrogenase enzymes of metabolically active cells (Corey, A. et al. "Use of an aqueous soluble tetrazolium/formazan assay for cell growth assays in culture," Cancer Commun. 1991, 3, 207-212). Cells were seeded in 96 well plates at 2000 cells/well and allowed to adhere overnight in Dulbecco's modified Eagle's medium supplemented with 10% fetal bovine serum. The final culture volume was 100 μl. Viable cell number was determined by using the Celltiter 96 $AQ_{ueous}$Non-radioactive Cell Proliferation Assay (Promega, Madison WI). The MTS /PMS (phenazine methosulfate) solution was mixed at a ratio of 20:1, and 20 μL was added per well to 100 μL of culture medium. After 2-4 hours, the formation of the formazan product was measured at 490 nm absorbance using a multiwell plate spectrophotometer. Background was determined by measuring the Abs 490 nm of cell culture medium and MTS-PMS in the absence of cells and was subtracted from all values. Percent viable cells was calculated as follows:

% viable cells = (Abs at 490 nm treated cells / Abs at 490 nm untreated cells) x 100

The effect of selected compounds of the invention on MCF7 breast carcinoma cells according to the MTS assay is summarized in Table 5. $IC_{50}$ was defined as the concentration of the compound which gave rise to 50% viable cell number.

**TABLE 5. Biological Activities of Pyrrolopyrimidines (Formula I)**

| S.No. | Ex # | Structure | Lysate binding (µM) | HER2 $IC_{50}$ (µM) | MTS $IC_{50}$ (µM) |
|---|---|---|---|---|---|
| 1 | 8 | 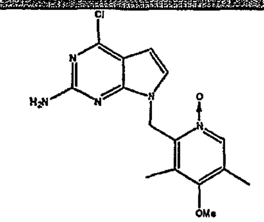 | ND | 0.023 | 0.1 |
| 2 | 10 | 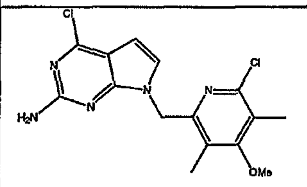 | ND | 0.25 | 0.6 |
| 3 | 11 | 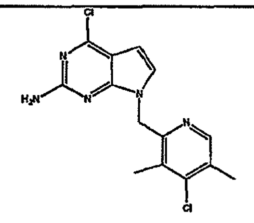 | 0.09 | 0.08 | 0.2 |
| 4 | 5 | 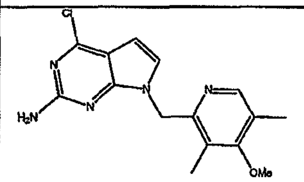 | 0.15 | 0.095 | 0.3 |
| 5 | 13 | 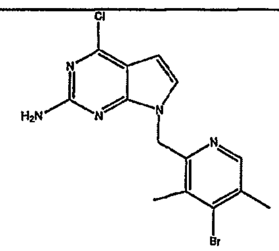 | 0.09 | 0.05 | 1.0 |
| 6 | 14 | 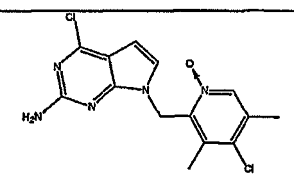 | 0.05 | 0.038 | 1.0 |

| S.No | Ex # | Structure | Lysate binding (μM) | HER2 IC$_{50}$ (μM) | MTS IC$_{50}$ (μM) |
|---|---|---|---|---|---|
| 7 | 15 | 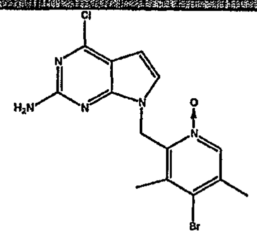 | 0.03 | 0.015 | 0.023 |
| 8 | 20 | 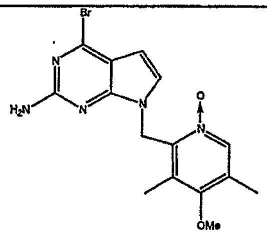 | ND | 0.042 | 1.0 |
| 9 | 21 | 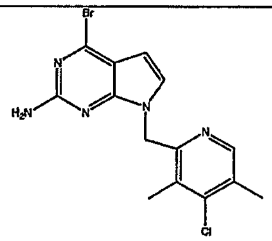 | ND | 0.17 | >10.0 |
| 10 | 22 | 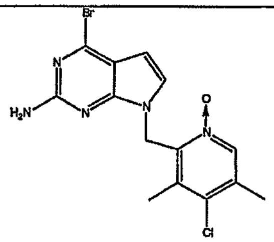 | ND | 0.065 | 1.0 |
| 11 | 23 | 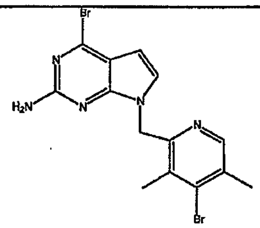 | ND | 0.13 | >10.0 |

| S.No | Ex # | Structure | Lysate binding (µM) | HER2 IC50 (µM) | MTS IC50 (µM) |
|---|---|---|---|---|---|
| 12 | 24 | 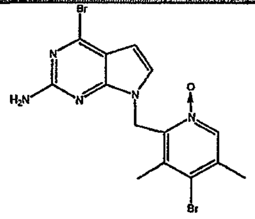 | ND | 0.025 | 0.3 |
| 13 | 25 | 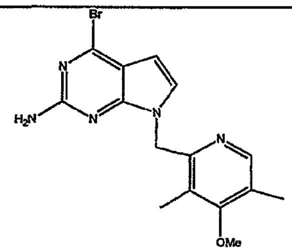 | ND | 0.15 | >10.0 |
| 14 | 46 | 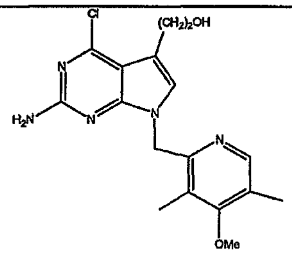 | ND | 0.07 | ND |
| 16 | 45 | 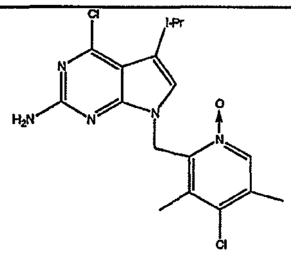 | ND | 0.02 | ND |
| 17 | 43 | 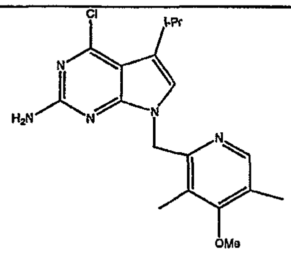 | ND | 0.13 | ND |

| S No | Ex # | Structure | Lysate binding (μM) | HER2 IC50 (μM) | MTS IC50 (μM) |
|---|---|---|---|---|---|
| 18 | 47 | 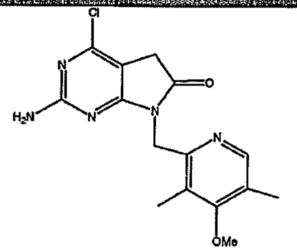 | ND | 0.45 | ND |
| 19 | 48 | 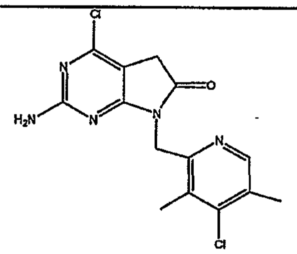 | ND | 1.5 | ND |
| 20 | 52 | 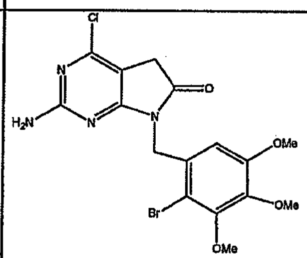 | ND | 0.4 | 10.0 |
| 21 | 49 | 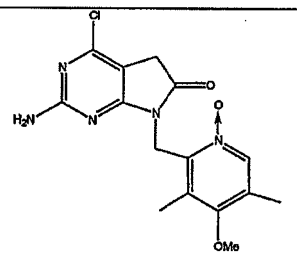 | ND | 0.18 | 0.9 |

ND, not determined.

## II. PREPARATION OF AMINOPURINES AND RELATED ANALOGS (COMPOUNDS OF FORMULA II)

### A. Materials and Methods

The chemical reagents used to create the novel products of the invention below are all available commercially, *e.g.*, from Aldrich Chemical Co., Milwaukee, WI, USA. Otherwise their preparation is facile and known to one of ordinary skill in the art, or it is referenced or described herein.

The final compounds were usually purified by preparative TLC (silica gel 60 A, Whatman Partisil PK6F) or flash chromatography (silica gel 60 Å, EMD Chemicals) using EtOAc/hexane or MeOH/$CH_2Cl_2$ as eluents. Rf's were measured using silica gel TLC plates (silica gel 60 Å, EMD Chemicals). Analytical HPLC chromatograms were obtained using a C18 column (Agilent Zorbax 300SB-C18; 5 microns; 4.6 mm x 150 mm). A gradient was applied between solvent A (0.1% TFA in $H_2O$) and solvent B (0.5% TFA in $CH_3CN$) increasing the proportion of A linearly from 5% (t=0) to 100% (t=7.00 min), with a constant flow rate of 1 mL/min. The samples were diluted to typically 0.1-1 mg/mL in MeOH or $CH_3CN$ and the injection volumes were typically 10 μL. The column was not heated, and UV detection was effected at 254 nm. $^1$H-NMR spectra were recorded on a Bruker Avance 400 MHz spectrometer.

The chemical names were generated using the Beilstein Autonom 2.1 software.

## B. General Procedure

### 1. General procedures to manipulate the purine ring

General procedure 1.1: Alkylation of purines at N-9

A suspension of the purine (3 mmol), (hetero)aryl-$CH_2$-halide (3 mmol, if needed it can be added in batches) and $K_2CO_3$ (3.3 mmol) in dry DMF (15 mL) was heated to 40-60 ˚C for 3 to 10 h. Work-up (EtOAc) and purification by prepartive TLC or flash chromatography (EtOAc/hexane or MeOH/$CH_2Cl_2$) yielded the pure N-9 alkylated product.

General procedure 1.2: Halogenation of purines at C-8

A solution of purine in MeOH/THF/acetate buffer (1N in each AcOH and AcONa) was treated with $Br_2$ (1.3-equiv. 1M in $CHCl_3$) at room termperture (r.t) for 16 h. Evaporation, work-up (EtOAc), drying ($MgSO_4$) and flash chromatography afforded the desired 8-bromopurine.

General procedure 1.3: Nucleophilic substitution of purines at C-6

For details, see J. Med. Chem.1999, 42, 2064-2086.

A) Sulfanyl derivatives: A suspension of 6-chloropurine and sodium or potassium thiolate in THF was heated to reflux for 6-24 hours. Work-up (EtOAc) and flash chromatography afforded the desired 6-sulfanylpurine.
B) Alkoxy derivatives: A suspension of 6-chloropurine and alkoxide in the appropriate alcohol was heated to reflux for 1-16 hours before quenching with water. Evaporation, work-up (EtOAc), and flash chromatography afforded the desired 6-alkoxypurine.
C) Amino derivatives: A solution of 6-chloropurine and alkylamine in MeOH was heated in a sealed tube to 100 ˚C for 16 hours. Evaporation, work-up (EtOAc), and flash chromatography afforded the desired 6-alkoxypurine.

General procedure 1.4: Methylation of purines at C-6

A suspension of 6-chloropurine (0.2 mmol) and tetrakis(triphenylphosphino)-palladium (0.02 mmol) in dry THF (3 mL) was treated with trimethylaluminum (2M in toluene, 0.45 mmol) under nitrogen. The resulting solution was heated to reflux for 3 h, cooled to r.t., diluted with toluene (5 mL), and quenched with methanol (0.5 mL) followed by ammonium chloride (1 mmol). The mixture was heated to reflux for 2 h and filtered while hot through a Celite plug. Evaporation and purification by prep TLC afforded the 6-methylpurine. See J. Med. Chem. 1999, 42, 2064-2086.

General procedure 1.5: Reductive dehalogenation of 6-halopurine

The 6-halo purine derivative was dissolved in acetic acid and a catalytic amount of 5 % Pd/C was added, and the mixture stirred under $H_2$ atmosphere (1 psi) at r.t. for 1h., evaporation, and purification afforded the dehalogenated derivatives.

General procedure 1.6: Acetylation of 2-amino-purines

The 2-amino purine derivative was dissolved in acetic anhydride, treated with a catalytic amount of concentrated sulfuric acid at r.t. for 1 hour. Work-up (EtOAc), evaporation, and purification afforded the 2-acetamido-pyridine.

### 2. General procedures to manipulate the pyridine ring

General procedure 2.1: Preparation of pyridine N-oxides

A solution of the pyridine derivative (1.0 mmol) in dichloromethane or chloroform (5 mL) was cooled by means of an ice-bath, treated with m-CPBA (1.1 to 3 mmol) in three portions, and allowed to warm to r.t. The mixture was extracted with dichloromethane and washed with aqueous NaOH, followed by water. Drying ($Na_2SO_4$) and concentration afforded the pyridine N-oxide.

General procedure 2.2: Preparation of 2-(acetoxymethyl)pyridines

A solution of the 2-methyl pyridine N-oxide (1.0 mmol) in acetic anhydride (5 mmol) was heated to reflux for 0.5 h. Work-up (EtOAc), drying ($MgSO_4$), evaporation and purification by preparative TLC or flash chromatography afforded the 2-(acetoxymethyl) pyridine.

General procedure 2.3: Preparation of 2-(hydroxymethyl)pyridines

A suspension of 2-acetoxymethyl-pyridine derivative and solid $K_2CO_3$ in methanol was heated to 50 ˚C for 5-30 min. Evaporation, work-up (EtOAc), and drying ($MgSO_4$) afforded the 2-(hydroxymethyl)pyridine.

General procedure 2.4: Preparation of 2-(chloromethyl)pyridines

A suspension of 2-hydroxymethyl-pyridine (10 g) in $POCl_3$ (30 mL) was stirred at 110 ˚C for 1.5 h. The resulting viscous oil was cooled to r.t. and poured onto ice water (500 g). The pH was adjusted to 10 with solid KOH. Work-up ($CHCl_3$), drying ($MgSO_4$) and evaporation gave the 2-(chloromethyl)pyridine, usually as a purple oil or solid, which was used without purification.

General procedure 2.5: Preparation of 2-(bromomethyl)pyridines

A solution of 2-(hydroxymethyl)pyridine (1.0 mmol) and triphenyl phosphine (1.2 mmol) in dichloromethane or chloroform (5 mL) was cooled to 0 ˚C. A solution of $CBr_4$ (1.5 mmol) in dichloromethane or chloroform was added dopwise, and the resulting mixture was stirred at 0 ˚C for 0.5-1 h. Work up followed and purification by flash chromatography afforded the 2-(bromomethyl)pyridine.

General procedure 2.6: O-alkylation of 3- or 4-hydroxypyridines

A mixture of hydroxypyridine, alkyl halide (1.1 equiv.), base ($K_2CO_3$, KOH or NaH 1.2-2 equiv.) and solvent (THF or DMF) was stirred at 23-80 ˚C for 5-30 min. Work-up (EtOAc), drying ($Na_2SO_4$) and evaporation gave the crude 4-(alkoxy)pyridine, which was purified by preparative TLC or flash chromatography.

General procedure 2.7: Preparation of salts.

Method 1: The free base (40 mmol) was heated in MeOH (1:6, 300 mL) until it dissolved. A solution of $H_3PO_4$ in MeOH (40 mmol) was added dropwise at r.t. The mixture was stirred for 10 *min*., and the solvent was evaporated to give the pyridinium phosphate as a glassy white solid. The following solvents could also be used: THF, EtOH, or i-PrOH.

Method 2: The free base (50 mmol) was heated in i-PrOH (6 L) until it dissolved. The solution was allowed to cool to r.t. and a solution of HCl in i-PrOH (75 mmol) was added slowly dropwise. The hydrochloride crystallized out of solution within a few minutes, and was collected by filtration, washed (acetone) and dried. The sulfate and mesylate salts were also prepared in this manner.

The hydrochloride salts can also be made by adding $CH_3COCl$ to the alcoholic solution of the pyridine or free base derivative.

Method 3: To a suspension of free base (5 mmol) in MeOH (50 mL) was added a solution of methane sulfonic acid in MeOH (75 mmol) was added slowly dropwise. The mixture became clear within a few minutes, upon addition of i-PrOH (50-100 mL) the salt precipitated out and was collected by filtration, washed with i-PrOH, ether and dried.

These methods can be applied to prepare all other salts.

### 3. General procedure to manipulate benzene rings

General procedure 3.1: Halogenation of benzene rings.

Variant 1: A solution of the aromatic compound in MeOH/THF/acetate buffer (1N in each AcOH and AcONa) was treated with $Br_2$ (1.3 equiv) at r.t. for 5 min. The excess bromine and solvent were removed on a rotary evaporator. Work-up ($CHCl_3$) and flash chromatography afforded the desired bromobenzene.

Variant 2: A solution of the aromatic compound (7 mmol) and N-halosuccinimide (NCS, NBS, or NIS, 1.06 equiv) in acetic acid (40 mL) was heated to 40-90 ˚C for 0.3-1 h. Evaporation, work-up (EtOAc) and flash chromatography afforded the desired halogenated benzene.

General procedure 3.2: Preparation of benzylic alcohols

Benzoic acid derivatives were reduced to the corresponding benzylic alcohols according to the procedure given by Bhaskar et al. J: Org. Chem. 1991, 56, 5964-5965.

## 4. Specific Examples

### Example 59. N-9 and N-7 alkylation of purines.

A suspension of purine (28.4 mmoles), benzyl halide (28.7 mmoles) in dry DMF (80 mls) was treated with K2C03 (31.2 mmoles) at 70 ˚C for Mrs. Extraction with EtOAc and chromatography EtOAc/Hexanes (1:1) yielded pure N9 and N-7 alkylated products. All HPLC was performed per the following parameters:

HPLC Column: Zorbax 300 SB-C 18, 5 microns, 4.6 x 150 mm.
HPLC Instrument: Agillent 1100
HPLC Reagent A: 0.1 %TFA/Water
HPLC Reagent B: 0.05% TFA/CH3CN
HPLC Method: 5%B to 100%B in 7 minutes.

The following compounds were prepared in this manner.

1.1 6-Chloro-9-(2,5-dimethoxy-benzyl)-9H-purin-2-ylamine. HPLC R.t. 5.194 min.
1.2 6-Chloro-9-(4,5-dimethoxy-2-nitro-benzyl)-9H-purin-2-ylamine. HPLC R.t. 5.194 min.
1.3 6-Chloro-9-(2,5-dichloro-benzyl)-9H-purin-2-ylamine. HPLC R.t. 5.846 min.
1.4 6-Chloro-9-(2,5-dichloro-benzyl)-9H-purin-2-ylamine. HPLC R.t. 5.982 min.
1.5 6-Bromo-9-(3,4,5-trimethoxy-benzyl)-9H-purin-2-ylamine. HPLC R.t. 4.947 min.
1.6 6-Chloro-9-(3,4,5-trimethoxy-benzyl)-9H-purine. HPLC R.t. 5.327 min
1.7 6-Chloro-9-(3,4,5-trimethoxy-benzyl)-9H-purin-2-ylamine. HPLC R.t. 4.878 min.
1.8 6-Chloro-9-(2,3,5-trifluoro-benzyl)-9H-purin-2-ylamine. HPLC R.t. 5.414 min.
1.9 6-Chloro-9-(3,5-dichloro-benzyl)-9H-purin-2-ylamine. HPLC R.t. 6.074 min.
1.10 6-Chloro-9-(3,5-dimethoxy-benzyl)-9H-purin-2-ylamine. HPLC R.t. 5.257 min.
1.11 9-(2-Bromo-3,5-dimethoxy-benzyl)-6-chloro-9H-purin-2-ylamine. HPLC R.t. 6.026 min.
1.12 6-Chloro-9-(2,6-dibromo-3,5-dimethoxy-benzyl)-9H-purin-2-ylamine. HPLC R.t. 6.022 min.
1.13 6-Chloro-9-(3-methoxy-benzyl)-9H-purin-2-ylamine. HPLC R.t. 5.136 min.
1.14 6-Chloro-9-(2-chloro-'3,4-dimethoxy-benzyl)-9H-purin-2-ylamine. HPLC R.t. 5.445 min.
1.15 6-Chloro-9-(2,4-dimethoxy-3-methyl-benzyl)-9H-purin-2-ylamine . HPLC R.t. 5.435 min.
1.16 6-Chloro-9-(6-chloro-benzo[1,3]dioxol-5-ylmethyl)-9H-purin-2-ylamine. HPLC R.t. 5.506min.
1.17 6-Chloro-9-(4-methoxy-benzyl)-9H-purin-2-ylamine. HPLC R.t. 5.067 min.
1.18 4-Chloro-1-(3,4,5-trimethoxy-benzyl)-1H-pyrazolo[3,4-d]pyrimidin-6-ylamine. HPLC R.t. 5.683 min.
1.19 6-Bromo-9-(2-chloro-3,4,5-trimethoxy-benzyl)-9H-purin-2-ylamine. HPLC R.t. 5.676 min.
1.20 6-Chloro-9-(4-methoxy-3,5-dimethyl-pyridin-2-ylmethyl)-9H-purin-2-ylamine. HPLC R.t. 3.941.
1.21 9-(2-Bromo-4,5-dimethoxy-benzyl)-6-chloro-9H-purin-2-ylamine. HPLC R.t. 5.458 min.
1.22 4-Chloro-1-(4-methoxy-3,5-dimethyl-pyridin-2-ylmethyl)-1H-pyrazolo[3,4-d]pyrimidin-6-ylamine. HPLC R.t. 4.464 min.
1.23 6-Chloro-9-(2,3-dimethoxy-benzyl)-9H-purin-2-ylamine. HPLC R.t. 5.200 min.
1.24 6-Chloro-9-(3,4-dimethoxy-benzyl)-9H-purin-2-ylamine. HPLC Rt. 4.753 min.
1.25 4-(2-Amino-6-chloro-purin-9-ylmethyl)-benzoic acid methyl ester. HPLC R.t. 5.052 min.
1.26 6-Chloro-9-(2-fluoro-4,5-dimethoxy-benzyl)-9H-purin-2-ylamine. HPLC R.t. 4.939 min.

### Example 60. Halogenation of purines.

To a solution of purine (6.6 mmoles) in acetate buffer/MeOH/THF or acetic acid or dichloromethane, bromine or N-chlorosuccinimide or N-Iodosuccinimide (8.7 mmoles) was added. While bromination can be done at room temperature, chlorination and iodination can be done at 60C to 90 C in 2 hours. Same HPLC conditions as stated in Example 59 were used.

The following compounds were prepared in this manner:

2.1 8-Bromo-6-chloro-9-(2,5-dimethoxy-benzyl)-9H-purin-2-ylamine. HPLC R.t. 6.150 min.

2.2 8-Bromo-6-chloro-9-(3,4-dimethoxy-2-nitro-benzyl)-9H-purin-2-ylamine. HPLC R.t. 6.040 min.
2.3 8-bromo-6-chloro-9-(3,4-dichloro-benzyl)-9H-purin-2-ylamine. HPLC R.t. 6.878 min.
2.4 6-Chloro-9-(2-chloro-3,4,5-trimethoxy-benzyl)-9H-purin-2-ylamine. HPLC R.t. 5.616 min.
2.5 6-Chloro-9-(2-bromo-3,4,5-trimethoxy-benzyl)-9H-purin-2-ylamine. HPLC R.t. 5.626 min.
2.6 6-Bromo-9-(2-bromo-3,4,5-trimethoxy-benzyl)-9H-purin-2-ylamine. HPLC R.t. 5.793 min.
2.7 8-Bromo-9-(2-bromo-3,5-trimethoxy-benzyl)-6-chloro-9H-purin-2-ylamine. HPLC R.t. 5.720 min.
2.8 9-(2-Bromo-3,4,5-trimethoxy-benzyl)-6-methoxy-9H-purine. HPLC R.t. 5.987 min
2.9 9-(2,6-Dibromo-3,4,5-trimethoxy-benzyl)-6-methoxy-9H-purine. HPLC R.t. 6.676 min
2.1O 9-(2-Bromo-3,4,5-trimethoxy-benzyl)-6-methoxy-9H-purine. HPLC R.t. 6.248 min
2.11 9-(2,6-Dibromo-3,4,5-trimethoxy-benzyl)-6-methoxy-9H-purine. HPLC R.t. 6.952 min
2.12 6,8-Dichloro-9-(2,6-dichloro-3,4,5-trimethoxy-benzyl)-9H-purin-2-ylamine. HPLC R.t. 6.859 min
2.13 6-Chloro-9-(2,6-dichloro-3,4,5-trimethoxy-benzyl)-9H-purin-2-ylamine. HPLC R.t. 6.100 min.
2.14 6-Chloro-9-(2-iodo-3,4,5-trimethoxy-benzyl)-9H-purin-2-ylamine. HPLC R.t. 5.887 min.

**Example 61. Displacement of 6-halogenated-purines.**

6-halogenated purines can be replaced by H, R, NHR, OR, SR using known procedures, e.g., as described in J. Med. Chem. 1999, 42, 2064-2086. Same HPLC conditions as stated in Example 59 were used.
The following compounds were prepared in this manner:

3.1 9-(3,4,5-Trimethoxy-benzyl)-9H-purin-2-ylamine. HPLC R.t. 4.123 min.
3.2 2 -Amino -9-(2 -bromo-3,4,5 -trimethoxy-benzyl)-9H-purine-6 -thiol. HPLC R.t. 4.931 min.
3.3 2-Amino-9-(2-chloro-3,4,5-trimethoxy-benzyl)-9H-purin-6-ol. HPLC R.t. 4.610 min.
3.4 9-(2-Bromo-3,4,5-trimethoxy-benzyl)-6-ethylsulfanyl-9H-purin-2-ylamine. HPLC R.t. 6.039 min.
3.5 6-Methoxy-9-(3,4,5-trimethoxy-benzyl)-9H-purine. HPLC R.t. 5.020 min.
3.6 9-(3,4,5-Trimethoxy-benzyl)-9H-purin-6-ylamine. HPLC R.t. 4.248 min.
3.7 9-(3,4,5-Trimethoxy-benzyl)-9H-purine-2,6-diamine. HPLC R.t. 4.209 min.
3.8 9-(2-Bromo-3,4,5-trimethoxy-benzyl)-6-methoxy-9H-purin-2-ylamine. HPLC R.t. 5.229 min.
3.9 9-(2-Bromo-3,4,5-trimedioxy-benzyl)-9H-purine-2,6-diamine. HPLC R.t 4.884 min.
3.10 6-Methoxy-9-(3,4,5-trimethoxy-benzyl)-9H-purin-2-ylamine. HPLC R.t. 4.489 min.
3.11 N-[9-(2-Bromo-3,4,5-trimethoxy-benzyl)-6-methoxy-9H-purin-2-yl]-N-methylacetamide. HPLC R.t. 6.178 min.

**Example 62. Acylation of 2-amino-purines**

2-amino-purine can be acylated in acetic anhydride with catalytic amount of concentrated sulfuric acid or in acetic acid and catalytic amount of fuming nitric acid at room temperature. Same HPLC conditions as stated in Example 59 were used.
The following compounds were prepared in this manner:

4.1 N-[6-Chloro-9-(3,4,5-trimethoxy-benzyl)-9H-purin-2-yl]-acetamide. HPLC R.t. 5.744 min.
4.2 N-[9-(2-Bromo-3,4,5-trimethoxy-benzyl)-6-chloro-9H-purin-2-yl]-acetamide. HPLC R.t. 5.603 min.

**Example 63. N-[9-(2-Bromo-3,4,5-trimethoxy-benzyl)-6-chloro-9H-purin-2-yl]-N-methylacetamide or Methylation of compound 4.2.**

To a suspension of compound 4.2 (Example 62 above) and iodomethane in DMF, NaH was added. Extraction with EtOAc and chromatography yield 5. HPLC R.t. 6.177 min.

**Example 64. Synthesis of 6-methyl purines**

The title compound was obtained by suspended purine (0.19 mmole) and tetrakis(triphenylphosphino)-palladium (0.019 mmole) in dry THF (3mls) before treating with trimethylaluminum (2M in toluene, 0.44 mmole) under nitrogen. The solution was refluxed for 3 hours before cooling down to room temperature. Diluted the reaction mixture with toluene (5 mls) before quenching the reaction with methanol (0.5 mls) followed by ammonium chloride (1 mmole). The mixture was refluxed for 2 hours and filtered, while hot, on Celite. See J: Med. Chem,1999, 42(12), 2064-2086.
The following compounds were prepared in this manner:

6.1 9-(2-Chloro-3,4,5-trimethoxy-benzyl)-6-methyl-9H-purin-2-ylamine. HPLC R.t. 4.800 min.

**Example 65: 9-(4-Bromo-3,5-dimethyl-pyridin-2-yl)-6-chloro-9H-purin-2-ylamine**

The title compound was obtained by alkylating 2-amino-6-chloropurine with 4-bromo-2-bromomethyl-3,5-dimethyl-pyridine according to the general procedure 1.1. HPLC Rt: 5.301 min. $^1$H-NMR ($CDCl_3$): δ 8.19 (s, 1H), 7.88 (s, 1H), 5.41 (s, 2H), 5.06 (s, 2H), 2.53 (s, 3H), 2.39 (s, 3H). m/z (%) 367.1 (M+1, 75%), 369.1 (M+3, 100%), 371.1 (M+5, 25%).

The alkylating agent, 4-bromo-2-bromomethyl-3,5-dimethyl-pyridine, could itself be prepared by any of the following three methods:

<u>Method 1</u>

*Step 1*: 2,3,5-Collidine-N-oxide

Oxidation of 2,3,5-collidine according to the general procedure 2.1 gave 2,3,5-collidine-N-oxide. Yield: 70%. HPLC Rt: 3.96 min. $^1$H-NMR ($CDCl_3$): δ 8.03 (s, 1H), 6.90 (s, 1H), 2.47 (s, 3H), 2.31 (s, 3H), 2.24 (s, 3H). m/z (%) 138.2 (M+1, 100%). Rf (20% MeOH/EtOAc): 0.35.

*Step 2*: 4-Bromo-2,3,5-collidine-N-oxide

2,3,5-collidine-N-oxide (1.3 g, 10 mmol) and $K_2CO_3$ (2.9 g, 20 mmol) were suspended in 10 mL of $CCl_4$. Bromine (1 mL, 20 mmol) was added dropwise, and the reaction mixture was heated to reflux for 2 h. Work-up (EtOAc) and flash chromatography (10% MeOH/EtOAc) afforded the product as a solid (1.05 g, 51% yield). HPLC Rt: 5.239 min. $^1$H-NMR ($CDCl_3$): δ 8.06 (s, $^1$H), 2.56 (s, 3H), 2.43 (s, 3H), 2.31 (s, 3H). m/z (%) 216.2 (M+1, 100%), 218.2 (M+3, 100%). Rf (20% MeOH/EtOAc): 0.45.

*Step 3:* Acetic acid 4-bromo-3,5-dimethyl-pyridin-2-yl methyl ester

4-Bromo-2,3,5-collidine-N-oxide (0.25g, 11 mmol) was dissolved in acetic anhydride (5 mL) and the solution was heated to reflux for 30 min. Work-up and flash chromatography (50% Hexane/EtOAc) afforded the product (0.27 g, 96% yield). Rf (50% Hexane/EtOAc): 0.70. HPLC Rt: 4.759 min. $^1$H-NMR ($CDCl_3$): δ 8.26 (s, 1H), 5.27 (s, 2H), 2.46 (s, 3H), 2.41 (s, 3H), 2.14 (s, 3H).

*Step 4:* 4-Bromo-3,5-dimethyl-pyridin-2-yl methanol

A suspension of acetic acid 4-bromo-3,5-dimethyl-pyridin-2-yl methyl ester (0.26 g, 1.0 mmol) and $K_2CO_3$ (excess) in MeOH (5 mL) was heated to 50˚C for 15 min. Work-up ($CHCl_3$), filtration through a silica gel pad (eluent: 100% EtOAc) and evaporation gave the title compound as a white solid (0.19 g, 88% yield). Rf(50% Hexane/EtOAc): 0.5. HPLC Rt: 3.80 min. $^1$H-NMR ($CDCl_3$): δ 8.23 (s, 1H), 4.70 (s, 2H), 2.46 (s, 3H), 2.30 (s, 3H).

*Step 5:* 4-Bromo-2-bromomethyl-3,5-dimethyl-pyridine

The title compound was obtained from 4-bromo-3,5-dimethyl-pyridin-2-yl methanol according to the general procedure 2.5. HPLC Rt: 6.323 min. $^1$H-NMR ($CDCl_3$): δ 8.22 (s, 1H), 4.63 (s, 2H), 2.52 (s, 3H), 2.40 (s, 3H).

<u>Method 2:</u>

*Step 1:* 2-chloromethyl-3,5-dimethyl-pyridin-4-ol

The title compound was obtained by following the procedure described in the patent by Tarbit, et al. WO 99/10326.

*Step 2:* 4-bromo-2-chloromethyl-3,5-dimethyl pyridine

A neat mixture of 2-chloromethyl-3,5-dimethyl-pyridin-4-ol (8.2 g, 47.8 mmol) and $POBr_3$ (60g, 209 mmol) was stirred at 130˚C for 3 h. The resulting viscous oil was cooled to r.t. and poured onto ice water. The pH was adjusted to 10 with solid KOH. Work-up ($CHCl_3$), drying ($MgSO_4$) and evaporation afforded the title compound as a purple solid (8.7 g, 78% yield) which was used without purification. HPLC Rt: 6.028 min. $^1$H-NMR ($CDCl_3$): 8.20 (s, 1H), 4.62 (s, 2H), 2.50 (s, 3H), 2.38 (s, 3H).

Method 3:

*Step 1:* 4-bromo-2-chloromethyl-3,5-dimethyl pyridine

A suspension of 2-chloromethyl-4-methoxy-3,5-dimethyl-pyridine (3.24 g, 14.6 mmol) in $PBr_3$ (8.0 ml, 85.1 mmol, 5.8 equiv.) was heated to 80 ˚C under nitrogen. A catalytic amount of DMF (0.50 ml, 6.4 mmol, 0.44 equiv.) was added, whereupon the suspension rapidly turned into an orange solution. After 40 min., the reaction was still incomplete as judged by HPLC. The temperature was raised to 110 ˚C and the reaction was prolonged for 30 min, at which point it was complete. The mixture was poured over ice, made basic with conc. aq. $NH_4OH$ and extracted into EtOAc. Washing with water, drying (brine, $MgSO_4$) and concentration gave the title compound as a pink solid (1.51 g, 44%) containing 10% of an impurity by $^1$H-NMR. The crude was used without further purification. $^1$H-NMR ($CDCl_3$) δ 8.19 (s, 1H), 4.59 (s, 2H), 2.48 (s, 3H), 2.37 (s, 3H).

**Example 66: 9-(4-bromo-3,5-dimethyl-pyridin-2-yl methyl)-6-chloro-9H-purin-2-ylamine, phosphate salt**

The title compound was obtained by treating 9-(4-bromo-3,5-dimethyl-pyridin-2-yl methyl)-6-chloro-9H-purin-2-ylamine with $H_3PO_4$ according to the general procedure 2.7. HPLC Rt: 5.294 min. $^1$H-NMR ($d_6$-DMSO): δ 8.12 (s, 1H), 8.09 (s, 1H), 6.83 (s, 2H), 5.47 (s, 2H), 2.49 (s, 3H), 2.29 (s, 3H)

**Example 67: 9-(4-bromo-3,5-dimethyl-pyridin-2-yl methyl)-6-chloro-9H-purin-2-ylamine, hydrochloric acid salt**

The title compound was obtained by treating 9-(4-bromo-3,5-dimethyl-pyridin-2-yl methyl)-6-chloro-9H-purin-2-ylamine with HCl according to the general procedure 2.7. HPLC Rt: 5.294 min. $^1$H-NMR ($d_6$-DMSO): δ 8.13 (s, 1H), 8.12 (s, 1H), 5.47 (s, 2H), 5.47 (s, 2H), 2.49 (s, 3H), 2.30 (s, 3H).

**Example 68: 9-(4-bromo-3,5-dimethyl-1-oxy-pyridin-2-yl methyl)-6-chloro-9H-purin-2-ylamine**

The title compound was obtained by oxidation of 9-(4-bromo-3,5-dimethyl-pyridin-2-yl)-6-chloro-9H-purin-2-ylamine according to the general procedure 2.1. HPLC Rt: 4.916 min. $^1$H-NMR ($CDCl_3$): δ 8.46 (s, 1H), 8.07 (s, 1H), 5.57 (s, 2H), 5.03 (s, 2H), 2.81 (s, 3H), 2.35 (s, 3H).

**Example 69: 6-bromo-9-(4-bromo-3,5-dimethyl-pyridin-2-yl methyl) -9H-purin-2-ylamine**

A mixture of 6-bromo-9H-purin-2-ylamine (2.4 g, 11 mmol), 4-bromo-2-chloromethyl-3,5-dimethyl pyridine (3.5 g, 15 mmol), $K_2CO_3$ (2.07 g, 15 mmol) and DMF (50 mL) was stirred at 50 ˚C for 2 h. Work-up and flash chromatography gave the title compound as a white solid (2.6 g, 56 %). HPLC Rt: 5.415 min. $^1$H-NMR ($CDCl_3$): δ 8.17 (s, 1H), 7.88 (s, 1H), 5.38 (s, 2H), 5.05 (s, 2H), 2.51(s, 3H), 2.37 (s, 3H).

**Example 70: 6-bromo-9-(4-bromo-3,5-dimethyl-1-oxy-pyridin-2-yl methyl) -9H-purin-2-ylamine**

The title compound was obtained by oxidation of 6-bromo-9-(4-bromo-3,5-dimethyl-pyridin-2-yl methyl) according to the general procedure 2.1 (52% yield). Rf (100% EtOAc): 0.1. HPLC Rt: 4.978 min. $^1$H-NMR ($CDCl_3$): δ 8.47 (s, 1H), 8.07 (s, 1H), 5.56 (s, 2H), 5.06 (s, 2H), 2.81(s, 3H), 2.35 (s, 3H).

**Example 71: 2-(2-Amino-6-chloro-purin-9-ylmethyl)-3,5-dimethyl-pyridin-4-ol**

The title compound was obtained by alkylating 6-chloro-9H-purin-2-ylamine with 2-chloromethyl-3,5-dimethyl-pyridin-4-ol according to the general procedure 1.1. HPLC Rt: 3.624 min. $^1$H-NMR ($d_6$-DMSO): δ 8.07 (s, 1H), 7.47 (s, 1H), 6.90 (s, 2H), 5.20(s, 2H), 2.00 (s, 3H), 1.86 (s, 3H).

**Example 72: 6-Chloro-9-(4-ethoxy-3,5-dimethyl-pyridin-2-ylmethyl)-9H-purin-2-**ylamine

The title compound was obtained by O-alkylation of 2-(2-amino-6-chloro-purin-9-ylmethyl)-3,5-dimethyl-pyridin-4-ol with ethyl iodide using the general procedure 2.6. HPLC Rt: 4.321 min. $^1$H-NMR ($CDCl_3$): δ 8.21 (s, 1H), 7.90 (s, 1H), 5.34 (s, 2H), 5.12 (s, 2H), 3.90 (q, 2H), 2.31 (s, 3H) 2.26 (s, 3H), 1.44 (t, 3H).

**Example 73: 9-(4-Allyloxy-3,5-dimethyl-pyridin-2-ylmethyl)-6-chloro-9H-purin-2-ylamine**

The title compound was obtained by O-alkylation of 2-(2-amino-6-chloro-purin-9-ylmethyl)-3,5-dimethyl-pyridin-4-ol with allyll chloride using the general procedure 2.6. HPLC Rt: 4.417 min. $^1$H-NMR ($CDCl_3$): δ 8.20 (s, 1H), 7.90 (s, 1H), 6.03-6.10 (m, 1H), 5.40-5.44 (dd, 1H), 5.34 (s, 2H), 5.29-5.32 (dd, 1H), 5.19 (s, 2H), 4.34-4.36 (m, 2H), 2.30 (s, 3H) 2.25 (s, 3H).

**Example 74: 6-Chloro-9-[4-(2-ethoxy-ethoxy)-3,5-dimethyl-pyridin-2-ylmethyl]-9H-purin-2-ylamine**

The title compound was obtained by O-alkylation of 2-(2-amino-6-chloro-purin-9-ylmethyl)-3,5-dimethyl-pyridin-4-ol with ethoxyethyl chloride, using the general procedure 2.6. HPLC Rt: 4.388 min. $^1$H-NMR ($CDCl_3$): δ 8.19 (s, 1H), 7.89 (s, 1H), 5.33 (s, 2H), 5.14 (s, 2H), 3.97-4.00 (t, 2H), 3.73-3.76 (t, 2H), 3.56-3.61 (q, 2H), 2.33 (s, 3H), 2.26 (s, 3H), 1.22-1.26 (t, 3H).

**Example 75: 6-Chloro-9-(4-isopropoxy-3,5-dimethyl-pyridin-2-ylmethyl)-9H-purin-2-ylamine**

The title compound was obtained by O-alkylation of 2-(2-amino-6-chloro-purin-9-ylmethyl)-3,5-dimethyl-pyridin-4-ol with isopropyl iodide using the general procedure 2.6. HPLC Rt: 4.571 min. $^1$H-NMR ($CDCl_3$): δ 8.17 (s, 1H), 7.89 (s, 1H), 5.32 (s, 2H), 5.06 (s, 2H), 4.20 (m, 1H), 2.26 (s, 3H) 2.22 (s, 3H), 1.28-1.30 (d, 3H).

**Example 76: 6-Chloro-9-(4-cyclopropylmethoxy-3,5-dimethyl-pyridin-2-ylmethyl)-9H-purin-2-ylamine**

The title compound was obtained by O-alkylation of 2-(2-amino-6-chloro-purin-9-ylmethyl)-3,5-dimethyl-pyridin-4-ol with cyclopropylmethyl iodide using the general procedure 2.6. HPLC Rt: 4.709 min. $^1$H-NMR ($CDCl_3$): δ 8.20 (s, 1H), 7.90 (s, 1H), 5.34 (s, 2H), 5.09 (s, 2H), 3.68-3.70 (d, 2H), 2.32(s, 3H) 2.27 (s, 3H), 1.23-1.31 (m, 1H), 0.63-0.68 (m, 2H), 0.30-0.33 (m, 2H).

**Example 77: 6-Chloro-9-[3,5-dimethyl-4-(3-methyl-butoxy)-pyridin-2-ylmethyl]-9H-purin-2-ylamine**

The title compound was obtained by O-alkylation of 2-(2-amino-6-chloro-purin-9-ylmethyl)-3,5-dimethyl-pyridin-4-ol with 3-methylbutyl bromide using the general procedure 2.6. HPLC Rt: 5.425 min. $^1$H-NMR ($CDCl_3$): δ 8.21 (s, 1H), 7.90 (s, 1H), 5.34 (s, 2H), 5.07 (s, 2H), 3.82-3.86 (t, 2H), 2.31 (s, 3H) 2.26 (s, 3H), 1.84-1.91 (m, 1H), 1.71-1.74 (q, 2H), 1.00 (s, 3H), 0.98 (s, 3H).

**Example 78: 6-Chloro-9-(4-isobutoxy-3,5-dimethyl-pyridin-2-ylmethyl)-9H-purin-2-ylamine**

The title compound was obtained by O-alkylation of 2-(2-amino-6-chloro-purin-9-ylmethyl)-3,5-dimethyl-pyridin-4-ol with isobutyl bromide using the general procedure 2.6. HPLC Rt: 4.321 min. $^1$H-NMR ($CDCl_3$): δ 8.21 (s, 1H), 7.90 (s, 1H), 5.34 (s, 2H), 5.12 (s, 2H), 3.58-3.56 (d, 2H), 2.30 (s, 3H) 2.26 (s, 3H), 1.09 (s, 3H), 1.08 (s, 3H).

**Example 79: Acetic acid 2-[2-(2-amino-6-chloro-purin-9-ylmethyl)-3,5-dimethyl-pyridin-4-yloxy]-ethyl ester**

The title compound was obtained by O-alkylation of 2-(2-amino-6-chloro-purin-9-ylmethyl)-3,5-dimethyl-pyridin-4-ol with 2-bromoethyl acetate using the general procedure 2.6. HPLC Rt: 4.103 min. $^1$H-NMR ($CDCl_3$): δ 8.21 (s, 1H), 7.90 (s, 1H), 5.35 (s, 2H), 5.10 (s, 2H), 4.42 (t, 2H), 4.05(t, 2H), 2.34 (s, 3H) 2.27 (s, 3H), 2.13 (s, 3H).

**Example 80: Acetic acid 3-[2-(2-amino-6-chloro-purin-9-ylmethyl)-3,5-dimethyl-pyridin-4-yloxy]-propyl ester**

The title compound was obtained by O-alkylation of 2-(2-amino-6-chloro-purin-9-ylmethyl)-3,5-dimethyl-pyridin-4-ol with 2-chloropropyl acetate the general procedure 2.6. HPLC Rt: 4.414 min. $^1$H-NMR ($CDCl_3$): δ 8.21 (s, 1H), 7.91 (s, 1H), 5.34 (s, 2H), 5.12 (s, 2H), 4.34 (t, 2H), 3.90 (t, 2H), 2.31 (s, 3H), 2.25 (s, 3H), 2.15 (m, 1H), 2.09 (s, 3H).

**Example 81: 6-Chloro-9-(3,5-dimethyl-4-propoxy-pyridin-2-ylmethyl)-9H-purin-2-ylamine**

The title compound was obtained by O-alkylation of 2-(2-amino-6-chloro-purin-9-ylmethyl)-3,5-dimethyl-pyridin-4-ol according to the general procedure 2.6. HPLC Rt: 4.644 min. $^1$H-NMR ($CDCl_3$): δ 8.21 (s, 1H), 7.91 (s, 1H), 5.34 (s, 2H), 5.12 (s, 2H), 3.80-3.76 (t, 2H), 2.31 (s, 3H), 2.26 (s, 3H), 1.85 (m, 2H), 1.07-1.10 (t, 3H).

**Example 82: 6-Chloro-9-(4-chloro-3,5-dimethyl-pyridin-2-ylmethyl)-9H-purin-2-ylamine**

*Step 1:* 4-Chloro-2-chloromethyl-3,5-dimethyl pyridine

The title compound was obtained by treating 2-chloromethyl-3,5-dimethyl-pyridin-4-ol with $POCl_3$ according to the general procedure 2.4 (74% yield). HPLC Rt: 5.543 min. $^1$H-NMR ($CDCl_3$): 8.24 (s, 1H), 4.71 (s, 2H), 2.48 (s, 3H), 2.36 (s, 3H).

*Step 2:* 6-chloro-9-(4-chloro-3,5-dimethyl-1-oxy-pyridin-2-yl methyl) -9H-purin-2-ylamine

A mixture of 6-chloro-9H-purin-2-ylamine (7 g, 41 mmol), 4-chloro-2-chloromethyl-3,5-dimethyl pyridine (8.2 g, 43 mmol)), $K_2CO_3$ (10 g, 72 mmol) and DMF (200 mL) was heated to 50 °C for 2 h. The reaction mixture was diluted with water (200 mL) and the resulting precipitate was collected by filtration, washed with water, and dried to give the title compound as a beige solid (11.7 g, 88% yield, 90% purity). HPLC Rt: 5.167 min. $^1$H-NMR ($CDCl_3$): δ 8.24 (s, 1H), 7.90 (s, 1H), 5.40 (s, 2H), 5.07 (s, 2H), 2.49 (s, 3H), 2.37 (s, 3H).

**Example 83: 6-Chloro-9-(4-chloro-3,5-dimethyl-1-oxy-pyridin-2-yl methyl) -9H-purin-2-ylamine**

The title compound was obtained by oxidation of 6-chloro-9-(4-chloro-3,5-dimethyl-pyridin-2-ylmethyl)-9H-purin-2-ylamine according to the general procedure 2.1 (56% yield). HPLC Rt: 4.813 min. $^1$H-NMR ($d_6$-DMSO): δ 8.31 (s, 1H), 8.20 (s, 1H), 6.91 (s, 2H), 5.41 (s, 2H), 2.73 (s, 3H), 2.26 (s, 3H).

**Examples 84: 6-Chloro-9-(3,5-dimethyl-pyridin-2-yl methyl) -9H-purin-2-ylamine**

*Step 1:* Acetic acid 3,5-dimethyl-pyridin-2-yl methyl ester

The title compound was prepared from 2,3,5-collidine-N-oxide (see example 1) according to the general procedure 2.2. HPLC Rt: 2.916 min. $^1$H-NMR ($CDCl_3$): δ 8.30 (s, 1H), 7.33 (s, 1H), 5.22 (s, 2H), 2.34 (s, 3H), 2.32 (s, 3H), 2.13 (s, 3H).

*Step 2:* 3,5-Dimethyl-pyridin-2-yl methanol

The title compound was obtained by deacetylation of acetic acid 3,5-dimethyl-pyridin-2-yl methyl ester according to the general procedure 2.3. HPLC Rt: 2.909 min. $^1$H-NMR ($CDCl_3$): δ 8.24 (s, 1H), 7.30 (s, 1H), 4.85 (broad s, 1H), 4.67 (s, 2H), 2.33 (s, 3H), 2.20 (s, 3H).

*Step 3:* 2-Bromomethyl-3,5-dimethyl pyridine

The title compound was obtained from 3,5-dimethyl-pyridin-2-yl methanol according to the general procedure 2.5. HPLC Rt: 3.895 min. $^1$H-NMR ($CDCl_3$): δ 8.3 (s, 1H), 7.3 (s, 1H), 4.61 (s, 2H), 2.41(s, 3H), 2.33 (s, 3H).

*Step 4:* 6-Chloro-9-(3,5-dimethyl-pyridin-2-ylmethyl)-9H-purin-2-ylamine

The title compound was obtained by alkylation of 2-amino-6-chloropurine with 2-bromomethyl-3,5-dimethyl pyridine according to the general procedure 1.1. HPLC Rt: 3.760 min. $^1$H-NMR ($CDCl_3$): δ 8.21 (s, 1H), 7.89 (s, 1H), 7.30 (s, 1H), 5.32 (s, 2H), 5.05 (s, 2H), 2.36 (s, 3H), 2.29 (s, 3H).

**Example 85: 6-Bromo-9-(4-methoxy-3,5-dimethyl-pyridin-2-ylmethyl)-9H-purin-2-ylamine**

The title compound was obtained by alkylation of 6-bromo-9H-purin-2-ylamine with 2-chloromethyl-4-methoxy-3,5-dimethylpyridine according to the general procedure 1.1. PLC Rt: 4.138. $^1$H-NMR ($CDCl_3$): δ 8.21 (s, 1H), 7.91 (s, 1H), 5.34 (s, 2H), 5.12 (s, 2H), 3.77 (s, 3H), 2.32 (s, 3H), 2.27 (s, 3H).). m/z (%) 363.2(M+1, 100%), 365.2 (M+3, 100%).

**Example 86: 6-Bromo-9-(4-methoxy-3,5-dimethyl-pyridin-2-ylmethyl)-9H-purin-2-ylamine, phosphate salt**

The title compound was obtained by treating 6-bromo-9-(4-methoxy-3,5-dimethyl-pyridin-2-ylmethyl)-9H-purin-2-ylamine with $H_3PO_4$ according to the general procedure 2.7. HPLC Rt: 4.138. $^1$H-NMR ($d_6$-DMSO): δ 8.07 (s, 1H), 8.02 (s, 1H), 6.84 (s, 2H), 5.35(s, 2H), 3.73 (s, 3H), 2.29 (s, 3H), 2.15 (s, 3H).). m/z (%) 363.2(M+1, 100%), 365.2 (M+3,

100%).

**Example 87: 6-Bromo-9-(4-methoxy-3,5-dimethyl-pyridin-2-ylmethyl)-9H-purin-2-ylamine, hydrochloride salt**

The title compound was obtained by treating 6-bromo-9-(4-methoxy-3,5-dimethyl-pyridin-2-ylmethyl)-9H-purin-2-ylamine with HCl according to the general procedure 2.7. HPLC Rt: 4.138. $^1$H-NMR ($d_6$-DMSO): δ 8.44 (s, 1H), 8.26 (s, 1H), 5.57 (s, 2H), 3.96 (s, 3H), 2.35 (s, 3H), 2.34 (s, 3H).

**Example 88: 6-Bromo-9-(4-methoxy-3,5-dimethyl-1-oxy-pyridin-2-ylmethyl)-9H-purin-2-ylamine**

The title compound was obtained by oxidation of 6-bromo-9-(4-methoxy-3,5-dimethyl-pyridin-2-yhnethyl)-9H-purin-2-ylamine according to the general procedure 2.1. HPLC Rt: 4.439 min. $^1$H-NMR ($CDCl_3$): δ 8.55 (s, 1H), 8.06 (s, 1H), 5.50 (s, 2H), 5.12 (s, 2H), 3.76 (s, 3H), 2.60 (s, 3H), 2.25 (s, 3H). m/z (%) 379.1 (M+1, 100%), 381.1 (M+3, 100%).

**Example 89: 6-Chloro-9-(4-methoxy-3,5-dimethyl-pyridin-2-ylmethyl)-9H-purin-2-ylamine**

Method 1

The title compound was obtained by alkylating 6-chloro-9H-purin-2-ylamine with 2-chloromethyl-4-methoxy-3,5-dimethylpyridine (or its HCl salt) according to the general procedure 1.1.

Method 2

The title compound could also be obtained by O-methylation of 6-chloro-9-(4-hydroxy-3,5-dimethyl-pyridin-2-ylmethyl)-9H-purin-2-ylamine according to the procedure 2.6 (KOH, MeI, DMF, 80 ˚C, 5 min). HPLC Rt: 3.980 min. $^1$H-NMR ($CDCl_3$): δ 8.19 (s, 1H), 7.88 (s, 1H), 5.32 (s, 2H), 5.07 (s, 2H), 3.75 (s, 3H), 2.29 (s, 3H), 2.24 (s, 3H).

Method 3

*Step 1:* (4-Methoxy-3,5-dimethyl-pyridin-2-yl)-methylamine

A solution of 2-chloromethyl-4-methoxy-3,5-dimethyl-pyridine hydrochloride in 7N methanolic ammonia was placed in a pressure vessel and to 100 ˚C for 16 h. Concentration and flash chromatography gave the title compound as a greenish solid (76% yield). HPLC Rt: 3.773 min. $^1$H-NMR ($CDCl_3$): δ 8.19 (s, 1H), 4.35 (s, 2H), 3.76 (s, 3H), 2.24 (s, 3H), 2.18 (s, 3H).

*Step 2:* 6-Chloro-4-(4-methoxy-3,5-dimethyl-pyridin-2-ylmethyl)-pyrimidine-2,4,5-triamine

A solution of 4,6-dichloro-pyrimidine-2,5-diamine (see Seela et al. Helv. Chim. Acta. 1986, 69, 1602-1613 and US Patent No. 5,917,042), 4-methoxy-3,5-dimethyl-pyridin-2-yl)-methylamine, and $Et_3N$ in butanol was heated to reflux for 1 h to give the title compound. HPLC Rt: 3.761 min. $^1$H-NMR ($CDCl_3$): δ 8.24 (s, 1H), 7.15 (s, 1H), 4.60 (s, 2H), 4.56-4.55 (d, 2H), 3.80 (s, 3H), 3.00 (s, 2H), 2.28 (s, 3H), 2.27 (s, 3H).

*Step 3:* Synthesis of 6-chloro-9-(4-methoxy-3,5-dimethyl-pyridin-2-ylmethyl)-9H-purin-2-ylamine

The cyclization with trimethylorthoformate in presence of acid to prepare purines can be done. See similar reaction, example 48.

**Example 90: 6-chloro-9-(4-methoxy-3,5-dimethyl-pyridin-2-ylmethyl)-9H-purin-2-ylamine, phosphate salt**

The title compound was obtained treating 6-chloro-9-(4-methoxy-3,5-dimethyl-pyridin-2-ylmethyl)-9H-purin-2-ylamine with $H_3PO_4$ according to the general procedure 2.7. HPLC Rt: 4.003 min. $^1$H-NMR ($d_6$-DMSO): δ 8.06 (s, 1H), 8.03 (s, 1H), 6.83 (s, 2H), 5.36 (s, 2H), 3.74 (s, 3H), 2.29 (s, 3H), 2.16 (s, 3H).

**Example 91: 6-Chloro-9-(4-methoxy-3,5-dimethyl-pyridin-2-ylmethyl)-9H-purin-2-ylamine, sulphate salt**

The title compound was obtained treating 6-chloro-9-(4-methoxy-3,5-dimethyl-pyridin-2-ylmethyl)-9H-purin-2-ylamine with $H_2SO_4$ according to the general procedure 2.7. HPLC Rt: 3.999 min. $^1$H-NMR ($d_6$-DMSO): δ 8.36 (s, 1H),

8.15 (s, 1H), 5.52 (s, 2H), 3.93 (s, 3H), 2.31 (s, 3H), 2.30 (s, 3H).

**Example 92: 6-Chloro-9-(4-methoxy-3,5-dimethyl-pyridin-2-ylmethyl)-9H-purin-2-ylamine, hydrochloride salt**

The title compound was obtained treating 6-chloro-9-(4-methoxy-3,5-dimethyl-pyridin-2-yhnethyl)-9H-purin-2-ylamine with HCl according to the general procedure 2.7. HPLC Rt: 4.093 min. $^1$H-NMR ($d_6$-DMSO): δ 8.38 (s, 1H), 8.23 (s, 1H), 5.55 (s, 2H), 3.93 (s, 3H), 2.34 (s, 3H), 2.31 (s, 3H).

**Example 93: 6-Chloro-9-(4-methoxy-3,5-dimethyl-pyridin-2-ylmethyl)-9H-purin-2-ylamine, mesylate salt**

The title compound was obtained treating 6-chloro-9-(4-methoxy-3,5-dimethyl-pyridin-2-ylmethyl)-9H-purin-2-ylamine with $MeSO_3H$ according to the general procedure 2.7. HPLC Rt: 4.093 min. $^1$H-NMR ($d_6$-DMSO): δ 8.38 (s, 1H), 8.17 (s, 1H), 5.54 (s, 2H), 3.95 (s, 3H), 2.36 (s, 3H), 2.34 (s, 3H), 2.33 (s, 3H).

**Example 94: N-[6-Chloro-9-(4-methoxy-3,5-dimethyl-pyridin-2-ylmethyl)-9H-purin-2-yl]-acetamide**

A suspension of 6-chloro-9-(4-methoxy-3,5-dimethyl-pyridin-2-ylmethyl)-9H-purin-2-ylamine (80 mg, 0.25 mmol) in acetic acid anhydride (2.2 g) was treated with one drop of concentrated $H_2SO_4$ and stirred at r.t. for 5 min. Work-up (EtOAc), drying ($MgSO_4$) and evaporation gave the title compound as a white solid. HPLC Rt: 4.093 min. $^1$H-NMR ($CDCl_3$): δ 8.20 (s, 1H), 8.10 (s, 1H), 5.46 (s, 2H), 3.78 (s, 3H), 2.54 (s, 3H), 2.38 (s, 3H), 2.27 (s, 3H).

**Example 95: 6-Chloro-9-(4-methoxy-3,5-dimethyl-1-oxy-pyridin-2-ylmethyl)-9H-purin-2-ylamine**

The title compound was obtained by oxidation of 6-chloro-9-(4-methoxy-3,5-dimethyl-pyridin-2-ylmethyl)-9H-purin-2-ylamine according to the general procedure 2.1. HPLC Rt: 4.435 min. $^1$H-NMR ($CDCl_3$): δ 8.55 (s, 1H), 8.06 (s, 1H), 5.52 (s, 2H), 5.07(s, 2H), 3.76 (s, 3H), 2.61 (s, 3H), 2.25 (s, 3H).). m/z (%) 335.1(M+1, 100%), 337.1 (M+3, 34%).

**Example 96: 6-Chloro-9-(4-methoxy-3,5-dimethyl-1-methoxy-pyridinium-2-methyl)-9H-purin-2-ylamine methyl sulfate salt**

A suspension of 6-chloro-9-(4-methoxy-3,5-dimethyl-1-oxy-pyridin-2-ylmethyl)-9H-purin-2-ylamine (0.2 g, 0.56 mmol) in DCM (10 ml) was heated to reflux. Dimethyl sulfate (1.12 mmol) was added dropwise (Tarbit WO 99/10326) and heating was continued for 3h. Filtration and washing (hot acetone) gave the title compound as a beige solid. HPLC Rt: 3.379 min. $^1$H-NMR ($d_6$-DMSO): δ 9.68 (s, 1H), 9.40 (s, 1H), 5.85 (s, 2H), 4.42 (s, 3H), 4.15 (s, 3H), 4.12 (s, 3H), 2.70 (s, 3H), 2.47 (s, 3H).

**Example 97: 6-Chloro-9-(6-chloro-4-methoxy-3,5-dimethyl-pyridin-2-ylmethyl)-9H-purin-2-ylamine**

Method 1

6-Chloro-9-(4-methoxy-3,5-dimethyl-1-oxy-pyridin-2-ylmethyl)-9H-purin-2-ylamine was treated with $POCl_3$ according to the general procedure 2.4. Flash chromatography gave the title compound as a white solid. HPLC Rt: 5.741 min. $^1$H-NMR ($CDCl_3$): δ 7.94 (s, 1H), 5.29 (s, 2H), 5.05 (s, 2H), 3.74 (s, 3H), 2.30 (s, 3H), 2.28 (s, 3H).

Method 2

*Step 1:* 2-Chloromethyl-4-methoxy-3,5-dimethylpyridine-1-oxide

The title compound was obtained by oxidation of 2-chloromethyl-4-methoxy-3,5-dimethylpyridine according to the general procedure 2.1. HPLC Rt: 4.462 min. $^1$H-NMR ($CDCl_3$): δ 8.05 (s, 1H), 4.93 (s, 2H), 3.77 (s, 3H), 2.37 (s, 3H), 2.24 (s, 3H).

*Step 2:* 2-Chloro-6-chloromethyl-4-methoxy-3,5-dimethylpyridine

The title compound was obtained by treating 2-chloromethyl-4-methoxy-3,5-dimethylpyridine-1-oxide with $POCl_3$ according to the general procedure 2.4. HPLC Rt: 6.757 min. $^1$H-NMR ($CDCl_3$): δ 4.64 (s, 2H), 3.79 (s, 3H), 2.35 (s, 3H), 2.33 (s, 3H).

*Step 3:* 6-Chloro-9-(6-chloro-4-methoxy-3,5-dimethyl-pyridin-2-ylmethyl)-9H-purin-2-ylamine

The title compound was obtained by alkylation of 6-chloro-9H-purin-2-ylamine with 2-chloro-6-chloromethyl-4-methoxy-3,5-dimethylpyridine according to the general procedure 1.1.

**Example 98: 6-Chloro-9-(5-methoxy-4-methoxymethyl-6-methyl-pyridin-3-ylmethyl)-9H-purin-2-ylamine**

*Step 1:* Acetic acid 3-acetoxy-5-hydroxymethyl-2-methyl-pyridin-4-ylmethyl ester

The title compound was obtained by following the procedure reported by Morisawa et al, J. Med. Chem. 1974, 17, 1083-1086. HPLC Rt: 3.08 min. $^1$H-NMR ($CDCl_3$): δ 8.41 (s, 1H), 5.20 (s, 2H), 4.80 (s, 2H), 2.40 (s, 3H), 2.38 (s, 3H), 2.03 (s, 3H).

*Step 2:* Acetic acid 3-acetoxy-5-bromomethyl-2-methyl-pyridin-4-ylmethyl ester

The title compound was obtained from Acetic acid 3-acetoxy-5-hydroxymethyl-2-methyl-pyridin-4-ylmethyl ester according to the general procedure 2.5. HPLC Rt: 5.332 min. $^1$H-NMR ($CDCl_3$): δ 8.43 (s, 1H), 5.22 (s, 2H), 4.70 (s, 2H), 2.43 (s, 3H), 2.41 (s, 3H), 2.06 (s, 3H).

*Step 3:* 6-Chloro-9-(5-acetoxy-4-acetoxymethyl-6-methyl-pyridin-3-ylmethyl)-9H-purin-2-ylamine

The title compound was obtained by alkylation of 2-amino-6-chloropurine with acetic acid 3-acetoxy-5-bromomethyl-2-methyl-pyridin-4-ylmethyl ester according to the general procedure 1.1. HPLC Rt: 4.498 min. $^1$H-NMR ($CDCl_3$): δ 8.42 (s, 1H), 7.74 (s, 1H), 5.43 (s, 2H), 5.10 (s, 2H), 5.07 (s, 2H), 2.42 (s, 3H), 2.39 (s, 3H), 1.96 (s, 3H).

*Step 4:* 6-Chloro-9-(5-hydroxy-4-hydroxymethyl-6-methyl-pyridin-3-ylmethyl)-9H-purin-2-ylamine

A suspension of 6-chloro-9-(5-acetoxy-4-acetoxymethyl-6-methyl-pyridin-3-ylmethyl)-9H-purin-2-ylamine and $K_2CO_3$ (excess) in MeOH was heated to 50 ˚C for 15 min. Filtration, work-up (EtOAc) and purification by preparative TLC gave the title compound. HPLC Rt: 4.498 min. $^1$H-NMR ($d_6$-DMSO) δ 8.08 (s, 1H), 7.74 (s, 1H), 6.95 (s, 1H), 5.31 (s, 2H), 4.74 (s, 2H), 2.31 (s, 3H).

*Step 5:* 6-Chloro-9-(5-methoxy-4-methoxymethyl-6-methyl-pyrldin-3-ylmethyl)-9H-purin-2-ylamine

A mixture of 6-chloro-9-(5-hydroxy-4-hydroxymethyl-6-methyl-pyridin-3-ylmethyl)-9H-purin-2-ylamine, MeI, $K_2CO_3$ (excess) and DMF was heated to 50 ˚C for 15 min. Work-up (EtOAc), drying ($MgSO_4$), evaporation, and purification by preparative TLC gave the title compound. HPLC Rt: 5.446 min. $^1$H-NMR ($CDCl_3$) δ 8.35 (s, 1H), 7.70 (s, 1H), 5.35 (s, 2H), 5.25 (s, 2H), 5.15 (s, 2H), 3.80 (s, 3H) 2.59 (s, 3H), 1.970 (s, 3H).

**Example 99: Synthesis of 6-Chloro-9-(5-ethoxy-4-hydroxymethyl-6-methyl-pyridin-3-ylmethyl)-9H-purin-2-ylamine**

A mixture of 6-chloro-9-(5-hydroxy-4-hydroxymethyl-6-methyl-pyridin-3-ylmethyl)-9H-purin-2-ylamine (see previous example), EtI (excess), $K_2CO_3$ (excess) and DMF was heated to 50 ˚C for 15 min. Work-up (EtOAc), drying ($MgSO_4$), evaporation, and purification by preparative TLC gave the title compound. HPLC Rt: 3.720 min. $^1$H-NMR ($CDCl_3$) δ 8.27 (s, 1H), 7.90 (s, 1H), 5.40 (s, 2H), 5.11 (s, 2H), 4.85 (d, 2H), 4.50 (t, 1H), 3.95 (q, 2H), 2.51 (s, 3H) 1.45(t, 3H).

**Example 100: 6-Chloro-9-(3,5-dimethyl-4-amino-pyridin-2-ylmethyl)-9H-purin-2-ylamine**

*Step 1:* 2-Bromomethyl-3,5-dimethyl-4-nitro-pyridine

The title compound was obtained from (3,5-dimethyl-4-nitro-pyridin-2-yl)-methanol according to the general procedure 2.5. HPLC Rt: 6.206 min. $^1$H-NMR ($CDCl_3$) δ 8.46 (s, 1H), 4.64 (s, 2H), 2.38 (s, 3H), 2.33 (s, 3H).

*Step 2:* 6-Chloro-9-(3,5-dimethyl-4-nitro-pyridin-2-ylmethyl)-9H-purin-2-ylamine

The title compound was obtained by alkylation of 2-amino-6-chloropurine with 2-bromomethyl-3,5-dimethyl-4-nitro-pyridine according to the general procedure 1.1 HPLC Rt: 6.206 min. $^1$H-NMR ($CDCl_3$) δ 8.40 (s, 1H), 7.94 (s, 1H),

5.40 (s, 2H), 5.05 (s, 2H), 2.40 (s, 3H), 2.27 (s, 3H).

*Step* 3: 6-Chloro-9-(3,5-dimethyl-4-amino-pyridin-2-ylmethyl)-9H-purin-2-ylamine

A suspension of 6-chloro-9-(4,6-dimethyl-5-nitro-pyridin-3-ylmethyl)-9H-purin-2-ylamine and excess of sodium hydrosulfite ($Na_2S_2O_4$) in methanol was stirred for 2 days at r.t. The MeOH was evaporated before extracting with EtOAc. Evaporation and purification by preparative TLC (100% EtOAc) gave the title compound. HPLC Rt: 3.544 min. $^1$H-NMR ($CDCl_3$) δ 8.05 (s, 1H), 7.83 (s, 1H), 5.31 (s, 2H), 5.05 (s, 2H), 4.08 (s, 2H), 2.12 (s, 6H).

**Example 101: 6-Chloro-9-(3-methoxy-5-methoxymethyl-4-methyl-pyridin-2-ylmethyl)-9H-purin-2-ylamine**

*Step 1:* 3-Methoxy-5-methoxymethyl-2,4-dimethyl-pyridine

The title compound was obtained by treating a solution of 5-hydroxymethyl-2,4-dimethyl-pyridin-3-ol hydrochloride (1 g, 5.2 mmol) in DMF with MeI (2.28 g, 15 mmol) and NaH (0.6 g, 50 mmol) for 1 h at 0 ˚C. HPLC Rt: 2.835 min. $^1$H-NMR ($CDCl_3$) δ 8.16 (s, 1H), 4.44 (s, 2H), 3.75 (s, 3H), 3.41 (s, 3H), 2.53 (s, 3H), 2.32 (s, 3H).

*Step 2:* 3-Methoxy-5-methoxymethyl-2,4-dimethyl-pyridine 1-oxide

The title compound was obtained by oxidation of 3-methoxy-5-methoxymethyl-2,4-dimethyl-pyridine according to the general procedure 2.1. HPLC Rt: 4.181 min. $^1$H-NMR ($CDCl_3$) δ 8.18 (s, 1H), 4.39 (s, 2H), 3.76 (s, 3H), 3.43 (s, 3H), 2.52 (s, 3H), 2.46 (s, 3H).

*Step* 3: Acetic acid 3-methoxy-5-methoxymethyl-4-methyl-pyridin-2-ylmethyl ester

The title compound was obtained by treating 3-methoxy-5-methoxymethyl-2,4-dimethyl-pyridine 1-oxide with $Ac_2O$ according to the general procedure 2.2. HPLC Rt: 4.062 min. $^1$H-NMR ($CDCl_3$) δ 8.32(s, 1H), 5.27 (s, 2H), 4.47 (s, 2H), 3.80 (s, 3H), 3.43 (s, 3H), 2.34 (s, 3H), 2.25 (s, 3H).

*Step 4:* (3-Methoxy-5-methoxymethyl-4-methyl-pyridin-2-yl)-methanol

The title compound was obtained from acetic acid 3-methoxy-5-methoxymethyl-4-methyl-pyridin-2-ylmethyl ester according to the general procedure 2.3. HPLC Rt: 3.465 min. $^1$H-NMR ($CDCl_3$) δ 8.22(s, 1H), 4.75 (d, 2H), 4.47 (s, 2H), 4.20 (t, 1H), 3.77 (s, 3H), 3.43 (s, 3H), 2.34 (s, 3H).

*Step 5:* 2-Bromomethyl-3-methoxy-5-methoxymethyl-4-methyl-pyridine

The title compound was obtained from (3-methoxy-5-methoxymethyl-4-methyl-pyridin-2-yl)-methanol according to the general procedure 2.5. HPLC Rt: 4.498 min. $^1$H-NMR ($CDCl_3$) δ 8.22(s, 1H), 4.695 (s, 2H), 4.42 (s, 2H), 3.86 (s, 3R), 3.40 (s, 3H), 2.31 (s, 3R).

*Step 6:* 6-Chloro-9-(3-methoxy-5-methoxymethyl-4-methyl-pyridin-2-ylmethyl)-9H-purin-2-ylamine

The title compound was obtained by alkylation of 2-amino-6-chloropurine with 2-bromomethyl-3-methoxy-5-methoxymethyl-4-methyl-pyridine according to the general procedure 1.1. HPLC Rt: 4.254 min. $^1$H-NMR ($CDCl_3$) δ 8.210 (s, 1H), 7.96 (s, 1H), 5.40 (s, 2H), 5.05 (s, 2H), 4.42 (s, 2H), 3.78 (s, 3H), 3.40 (s, 3H), 2.31 (s, 3H).

**Example 102: 6-Chloro-9-(5-chloro-6-methoxy-pyridin-3-ylmethyl)-9H-purin-2-ylamine**

*Step 1:* (5-Chloro-6-methoxy-pyridin-3-yl)-methanol

(5,6-Dichloro-pyridin-3-yl)-methanol was dissolved in a saturated solution of NaOMe in MeOH and heated to reflux overnight. Evaporation of MeOH, work-up (EtOAc) and evaporation gave the title compound. $^1$H-NMR ($CDCl_3$) δ 8.03 (d, 1H), 7.72 (d, 1H), 4.65 (s, 2H), 4.04 (s, 3H).

*Step 2:* 5-Bromomethyl-3-chloro-2-methoxy-pyridine

The title compound was obtained from (5-chloro-6-methoxy-pyridin-3-yl)-methanol

according to the general procedure 2.5. $^1H$-NMR ($CDCl_3$) δ 8.05 (d, 1H), 7.70 (d, 1H), 4.42 (s, 2H), 4.02 (s, 3H).

*Step 3:* 6-Chloro-9-(5-chloro-6-methoxy-pyridin-3-ylmethyl)-9H-purin-2-ylamine

The title compound was obtained by alkylation of 2-amino-6-chloropurine with 5-bromomethyl-3-chloro-2-methoxy-pyridine according to the general procedure 1.1. HPLC Rt: 5.256 min. $^1H$-NMR ($CDCl_3$) δ 8.11(d, 1H), 7.78 (s, 1H), 7.63 (d, 1H), 5.19 (s, 2H), 5.16 (s, 2H), 4.04 (s, 3H ).

**Example 103: 6-chloro-9-(3,4-dimethoxy-pyridin-2-ylmethyl)-9H-purin-2-ylamine**

The title compound was obtained by alkylation of 2-amino-6-chloropurine with 2-chloromethyl-3,4-dimethoxylpyridine hydrochloride according to the general procedure 1.1. HPLC Rt: 3.777 min. $^1H$-NMR ($CDCl_3$): δ 8.19 (d, 1H), 7.95 (s, 1H), 6.82 (d, 1H), 5.39 (s, 2H), 5.09 (s, 2H), 3.92 (s, 3H), 3.87 (s, 3H).

**Example 104: 6-chloro-9-(3-methoxy-6-methyl-pyridin-2-ylmethyl)-9H-purin-2-ylamine**

*Step 1:* (3-Methoxy-6-methyl-pyridin-2-yl)-methanol

The title compound was obtained by O-methylation of 2-hydroxymethyl-6-methyl-pyridin-3-ol according to the general procedure 2.6. HPLC Rt: 2.304 min. $^1H$-NMR ($CDCl_3$) δ 7.05-7.11(m, 2H), 4.72-4.71 (d, 2H), 4.47-4.49 (t, 1H), 3.84 (s, 3H), 2.51 (s, 3H).

*Step 2:* 2-Bromomethyl-3-methoxy-6-methyl-pyridine

The title compound was obtained from (3-Methoxy-6-methyl-pyridin-2-yl)-methanol according to the general procedure 2.5. HPLC Rt: 4.361. $^1H$-NMR ($CDCl_3$) δ 7.06-7.12 (m, 2H), 4.61 (s, 2H), 3.89 (s, 3H), 2.49 (s, 3H).

*Step* 3: 6-chloro-9-(3-methoxy-6-methyl-pyridin-2-ylmethyl)-9H-purin-2-ylamine

The title compound was obtained by alkylation of 2-amino-6-chloropurine with 2-bromomethyl-3- mothoxy-6-methylpyridine according to the general procedure 1.1. HPLC Rt: 3.777 min. $^1H$-NMR ($CDCl_3$): δ 7.92 (s, 1H), 7.11 (m, 2H), 5.39 (s, 2H), 5.15 (s, 2H), 3.85 (s, 3H), 2.45 (s, 3H).

**Example 105: 6-Chloro-9-(5-methoxy-4,6-dimethyl-pyridin-3-ylmethyl)-9H-purin-2-ylamine**

*Step 1:* (5-Methoxy-4,6-dimethyl-pyridin-3-yl)-methanol

The title compound was obtained by O-methylation of 5-hydroxymethyl-2,4-dimethyl-pyridin-3-ol according to the general procedure 2.6. HPLC Rt: 3.114 min. $^1H$-NMR ($CDCl_3$) δ 8.08 (s, 1H), 4.67 (s, 2H), 3.74 (s, 3H), 2.49 (s, 3H), 2.33 (s, 3H).

*Step 2:* 5-Bromomethyl-3-methoxy-2,4-dimethyl-pyridine

The title compound was obtained from (5-methoxy-4,6-dimethyl-pyridin-3-yl)-methanol according to the general procedure 2.5. HPLC Rt: 2.873. $^1H$-NNM ($CDCl_3$) δ 8.22 (s, 1H), 4.50 (s, 2H), 3.764(s, 3H), 2.54 (s, 3H), 2.37 (s, 3H).

*Step 3:* 6-Chloro-9-(5-methoxy-4,6-dimethyl-pyridin-3-ylmethyl)-9H-purin-2-ylamine

The title compound was obtained by alkylation of 2-amino-6-chloropurine with 5-bromomethyl-3- methoxy-2,4-methylpyridine according to the general procedure 1.1. HPLC Rt: 3.7387 min. $^1H$-NMR ($CDCl_3$): δ 8.25 (s, 1H), 7.63 (s, 1H), 5.39 (s, 2H), 5.11 (s, 2H), 3.73 (s, 3H), 2.55 (s, 3H), 2.22 (s, 3H).

**Example 106: 9-(4-Methoxy-3,5-dimethyl-pyridin-2-ylmethyl)-9H-purin-2-ylamine**

The title compound was obtained by alkylation of 2-amino-6-chloropurine with 2-chloromethyl-4-methoxy-3,5-dimethylpyridine hydrochloride according to the general procedure 1.1. HPLC Rt: 3.434 min. $^1H$-NMR ($CDCl_3$): δ 8.75 (s, 1H), 8.25 (s, 1H), 7.90 (s, 1H), 5.37(s, 2H), 5.07 (s, 2H), 3.77 (s, 3H), 2.32 (s, 3H), 2.26 (s, 3H).

**Example 107: 6-Chloro-9-(3,5-dimethoxy-4-methyl-benzyl)-9H-purin-2-ylamlite**

*Step 1:* (3,5-Dimethoxy-4-methyl-phenyl)-methanol

The title compound was obtained by reducing 3,5-dimethoxy-4-methyl-benzoic acid as described by Bhaskar et al. J. Org. Chem. 1991, 56, 5964-5965. HPLC Rt: 5.352. $^1$H-NMR ($CDCl_3$): δ 6.58(s, 2H), 4.68 (s, 2H), 3.85 (s, 6H), 2.10 (s, 3H).

*Step 2:* 5-Bromomethyl-1,3-dimethoxy-2-methyl-benzene

The title compound was obtained from (3,5-dimethoxy-4-methyl-phenyl)-methanol according to the general procedure 2.5. HPLC Rt: 7.200. $^1$H-NMR ($CDCl_3$): δ 6.59 (s, 2H), 4.51 (s, 2H), 3.86 (s, 3H),3.85 (s, 3H), 2.22 (s, 3H).

*Step 3:* 6-Chloro-9-(3,5-dimethoxy-4-methyl-benzyl)-9H-purin-2-ylamine

The title compound was obtained by alkylation of 2-amino-6-chloropurine with 5-bromomethyl-1,3-dimethoxy-4-methyl benzene according to the general procedure 1.1. HPLC Rt: 5.841 min. $^1$H-NMR ($CDCl_3$): δ 7.75 (s, 1H), 6.46 (s, 1H), 5.21 (s, 2H), 5.07(s, 2H), 3.80 (s, 6H), 2.09 (s, 3H).

**Example 108: 9-(2-Bromo-3,5-dimethoxy-4-methyl-benzyl)-6-chloro-9H-purin-2-ylamine**

The title compound was obtained by brominating 6-chloro-9-(3,5-dimethoxy-4-methyl-benzyl)-9H-purin-2-ylamine with bromine according to the general procedure 3.1. HPLC Rt: 6.222 min. $^1$H-NMR ($CDCl_3$): δ 7.85 (s, 1H), 6.60 (s, 1H), 5.36 (s, 2H), 5.07(s, 2H), 3.80 (s, 3H), 3.74 (s, 3H), 2.20 (s, 3H).

**Example 109: 8-Bromo-9-(2-bromo-3,5-dimethoxy-4-methyl-benzyl)-6-chloro-9H-purin-2-ylamine**

The title compound was obtained by brominating 6-chloro-9-(3,5-dimethoxy-4-methyl-benzyl)-9H-purin-2-ylamine with excess bromine according to the general procedure 3.1. HPLC Rt: 7.040 min. $^1$H-NMR ($CDCl_3$): δ 5.88 (s, 1H), 5.38 (s, 2H), 5.14(s, 2H), 3.83 (s, 3 H), 3.57 (s, 3H), 2.19 (s, 3H).

**Example 110: 2,6-Dichloro-9-(4-methoxy-3,5-dimethyl-pyridin-2-ylmethyl)-9H-purine**

The title compound was obtained by alkylation of 2-amino-6-chloropurine with 2-chloromethyl-4-methoxy-3,5-dimethylpyridine (or its HCl salt) according to the general procedure 1.1. HPLC Rt: 5.081 min. $^1$H-NMR ($CDCl_3$): δ 8.30 (s, 1H), 8.17 (s, 1H), 5.50 (s, 2H), 3.80 (s, 3H), 2.38 (s, 3H), 2.27 (s, 3H).

**Example 111: 8-butyl-6-chloro-9-(3,4,5-trimethoxy-benzyl)-9H-purin-2-ylamine**

*Step 1:* 6-Chloro-N4-(3,4,5-trimethoxy-benzyl)-pyrimidine-2,4,5-triamine

The title compound was obtained by refluxing 4,6-dichloro-pyrimidine-2,5-diamine (see Seela et al. Helv. Chim. Acta. 1986, 69, 1602-1613 and US Patent No. 5,917,042), 3,4,-5-trimethoxybenzylamine, and $Et_3N$ in butanol or ethanol for 1 to 14 h. HPLC Rt: 4.327 min. $^1$H-NMR ($CDCl_3$): δ 6.57 (s, 2H), 5.65 (t, 1H), 4.75 (s, 2H), 4.54 (d, 2H), 3.86-3.87 (d, 9H).

*Step* 2: 8-Butyl-6-chloro-9-(3,4,5-trimethoxy-benzyl)-9H-purin-2-ylamine

The title compound was obtained by following the procedure given in WO 98/39344. HPLC Rt: 5.971 min. $^1$H-NMR ($CDCl_3$): δ 6.34(s, 2H), 5.19 (s, 2H), 5.04 (s, 2H), 3.81 (s, 3H), 3.77 (s, 6H), 2.71-2.75 (t, 2H), 1.68-1.74 (m, 2H), 1.35-1.41 (m, 2H), 0.88-0.92 (t, 2H).

**Example 112: 6-Chloro-9-(3,4,5-trimethoxy-benzyl)-9H-purin-2-ylamine**

The title compound was obtained by alkylation of 2-amino-6-chloropurine with 3,4,5-trimethoxybenzyl according to the general procedure 1.1. The title compound was also obtained by treating a solution of 6-chloro-N-4-(3,4,5-trimethoxy-benzyl)-pyrimidine-2,4,5-triamine in triethyl orthoformate with a catalytic amount of conc. HCl at r.t. for 20 min. HPLC Rt: 4.906 min. $^1$H-NMR ($CDCl_3$): δ 7.76 (s, 1H), 6.51 (s, 2H), 5.18 (s, 2H), 5.12 (s, 2H), 3.85 (s, 3H), 3.84 (s, 6H).

**Example 113: Acetic acid 4-(2-amino-6-chloro-purin-9-ylmethyl)-3-bromo-2,6-dimethoxy-phenyl ester**

*Step 1:* Acetic acid 4-hydroxymethyl-2,6-dimethoxy-phenyl ester

A solution of acetic acid 4-formyl-2,6-dimethoxy-phenyl ester (25 mmol) in MeOH (100 mL) was treated with $NaBH_4$ (1 equiv.) at 0 °C for 15 min. After quenching with acetone and evaporating the solvent, work-up ($CH_2Cl_2$) and evaporation gave the title compound as a white solid (85% yield). Rf (in EtOAc/Hexane 1:1): 0.5. $^1$H-NMR ($CDCl_3$): δ 6.66 (s, 1H), 4.68-4.70 (d, 2H), 3.85 (s, 3H), 2.36 (s, 3H), 1.74 (t, 1H).

*Step 2:* Acetic acid 3-bromo-4-hydroxymethyl-2,6-dimethoxy-phenyl ester

The title compound was obtained by bromination of acetic acid 4-hydroxymethyl-2,6-dimethoxy-phenyl ester in AcOH/AcONa buffer according to the general procedure 3.1. Rf(EtOAc/Hexane 1:3): 0.2. $^1$H-NMR ($CDCl_3$): δ 7.01 (s, 1H), 4.75-4.76 (d, 2H), 3.86 (s, 3H), 3.85 (s, 3H), 2.38 (s, 3H), 2.05 (t, 1H).

*Step 3:* Acetic acid 3-bromo-4-bromomethyl-2,6-dimethoxy-phenyl ester

The title compound was obtained from Acetic acid 3-bromo-4-hydroxymethyl-2,6-dimethoxy-phenyl ester according to the general procedure 2.5. Rf (EtOAc/Hexane 1:3): 0.8. $^1$H-NMR ($CDCl_3$): δ 6.87 (s, 1H), 4.60 (s, 2H), 3.84 (s, 3H), 3.83 (s, 3H ), 2.36 (s, 3H).

*Step 4:* Acetic acid 4-(2-amino-6-chloro-purin-9-ylmethyl)-3-bromo-2,6-dimethoxy-phenyl ester

The title compound was obtained by alkylation of 2-amino-6-chloropurine with acetic acid 3-bromo-4-bromomethyl-2,6-dimethoxy-phenyl ester according to the general procedure 1.1. HPLC Rt: 5.081 min. $^1$H-NMR ($CDCl_3$): δ 8.30 (s, 1H), 8.17 (s, 1H), 5.50 (s, 2H), 3.80 (s, 3H), 2.38 (s, 3H), 2.27 (s, 3H).

**Example 114: 4-(2-amino-6-chloro-purin-9-ylmethyl)-3-bromo-2,6-dimethoxy-phenol**

The title compound was obtained by deacetylation of Acetic acid 4-(2-amino-6-chloro-purin-9-ylmethyl)-3-bromo-2,6-dimethoxy-phenyl ester (see previous example) in $NH_3$/MeOH at r.t. for 0.5 h or $K_2CO_3$ in methanol according to the general procedure 2.3. HPLC Rt: 4.912 min. $^1$H-NMR ($CDCl_3$): δ 7.85 (s, 1H), 6.72 (s, 1H), 5.70 (s, 1H), 5.33 (s, 1H), 5.07 (s, 2H), 3.94 (s, 3H), 3.82 (s, 3H).

**Example 115: 9-(2-bromo-3,4,5-trimethoxy-benzyl)-6-chloro-9H-purin-2-ylamine**

The title compound was obtained by O-methylation of 4-(2-amino-6-chloro-purin-9-ylmethyl)-3-bromo-2,6-dimethoxy-phenol (see previous example) according to the general procedure 2.6. The title compound could also be obtained by bromination of 6-chloro-9-(3,4,5-trimethoxy-benzyl)-9H-purin-2-ylamine (see example 48) in acetate buffer according to the general procedure 3.1. HPLC Rt: 5.742 min. $^1$H-NMR ($CDCl_3$): δ 7.8 (s, 1H), 6.66 (s, 1H), 5.32 (s, 2H), 5.11 (s, 2H), 3.90 (s, 3H), 3.87 (s, 3H), 3.76 (s, 3H).

**Example 116: 9-(4-allyloxy-2-bromo-3,5-dimethoxy-benzyl)-6-chloro-9H-purin-2-ylamine**

The title compound was obtained by alkylation of 4-(2-amino-6-chloro-purin-9-ylmethyl)-3-bromo-2,6-dimethoxy-phenol (see example 50) with 3-bromo-propene in DMF in the presence of $K_2CO_3$ at 70 °C for 0.25-1 h. HPLC Rt: 6.309 min. $^1$H-NMR ($CDCl_3$): δ 7.84 (s, 1H), 6.64 (s, 1H), 6.00-6.10 (m, 2H), 5.36-5.36 (m, 1H), 5.31 (s, 2H), 5.20-5.21 (m, 2H), 4.52-4.54 (m, 2H), 3.90 (s, 3H), 3.74 (s, 2H).

**Example 117: 9-(2-bromo-4-chloromethoxy-3,5-dimethoxy-benzyl)-6-chloro-9H-purin-2-ylamine**

The title compound was obtained by alkylation of 4-(2-amino-6-chloro-purin-9-ylmethyl)-3-bromo-2,6-dimethoxy-phenol (see example 50) with chloroiodomethane in DMF in the presence of $K_2CO_3$ at 70 °C for 0.25-1 h. HPLC Rt: 6.109 min. $^1$H-NMR ($CDCl_3$): δ 7.87 (s, 1H), 6.66 (s, 1H), 5.91 (s, 2H), 5.34 (s, 2H), 5.08 (s, 2H), 3.90 (s, 3H), 3.76 (s, 3H).

**Example 118: 9-[2-bromo-4-(2-chloro-ethoxy)-3,5-dimethoxy-benzyl]-6-chloro-9H-purin-2-ylamine**

The title compound was obtained by alkylation of 4-(2-amino-6-chloro-purin-9-ylmethyl)-3-bromo-2,6-dimethoxy-phenol (see example 50) with 1-bromo-2-chloroethane in DMF in the presence of $K_2CO_3$ at 70 ˚C for 0.25-1 h. HPLC Rt: 6.285 min. $^1$H-NMR ($CDCl_3$): δ 7.86 (s, 1H), 6.67 (s, 1H), 5.37 (s, 2H), 5.32 (s, 2H), 4.22-4.25 (t, 2H), 3.91 (s, 3H), 3.77-3.78 (t, 2H) 3.75 (s, 3H).

**Example 119: 9-(2-bromo-4-cyclopropylmethoxy-3,5-dimethoxy-benzyl)-6-chloro-9H-purin-2-ylamine**

The title compound was obtained by alkylation of 4-(2-amino-6-chloro-purin-9-ylmethyl)-3-bromo-2,6-dimethoxy-phenol (see example 50) with bromomethyl-cyclopropane in DMF in the presence of $K_2CO_3$ at 70 ˚C for 0.25-1 h. HPLC Rt: 6.512 min. $^1$H-NMR ($CDCl_3$): δ 7.86 (s, 1H), 6.67 (s, 1H), 5.34 (s, 2H), 5.17 (s, 2H), 3.95 (s, 3H), 3.83-3.84 (d, 2H), 3.77 (s, 3H) 1.27 (m, 1H), 0.58-0.62 (m, 2H), 0.28-0.32 (m, 2H).

**Example 120: 9-(2-bromo-4-ethoxy-3,5-dimethoxy-benzyl)-6-chloro-9H-purin-2-ylamine**

The title compound was obtained by alkylation of 4-(2-amino-6-chloro-purin-9-ylmethyl)-3-bromo-2,6-dimethoxy-phenol (see example 50) with EtI in DMF in the presence of $K_2CO_3$ at 70 ˚C for 0.25-1 h. HPLC Rt: 6.112 min. $^1$H-NMR ($CDCl_3$): δ 7.84 (s, 1H), 6.65 (s, 1H), 5.31 (s, 2H), 5.13 (s, 2H), 4.04-4.09 (q, 2H), 3.90(s, 3H), 3.82 (s, 3H), 1.32-1.38 (t, 3H).

**Example 121: 9-(2-bromo-3,5-dimethoxy-4-propoxy-benzyl)-6-chloro-9H-purin-2-ylamine**

The title compound was obtained by alkylation of 4-(2-amino-6-chloro-purin-9-ylmethyl)-3-bromo-2,6-dimethoxy-phenol (see example 50) with PrI in DMF in the presence of $K_2CO_3$ at 70 ˚C for 0.25-1 h. HPLC Rt: 6.594 min. $^1$H-NMR ($CDCl_3$): δ 7.84 (s, 1H), 6.65 (s, 1H), 5.30 (s, 2H), 5.14 (s, 2H), 3.93-3.97 (t, 2H), 3.89 (s, 3H), 3.74 (s, 3H), 1.72-1.81 (m, 2H), 1.00-1.04 (t, 3H).

**Example 122: 9-(2-bromo-4-butoxy-3,5-dimethoxy-bemyl)-6-chloro-9H-purin-2-ylamine**

The title compound was obtained by alkylation of 4-(2-amino-6-chloro-purin-9-ylmethyl)-3-bromo-2,6-dimethoxy-phenol (see example 50) with BuI in DMF in the presence of $K_2CO_3$ at 70 ˚C for 0.25-1 h. HPLC Rt: 6.594 min. $^1$H-NMR ($CDCl_3$): δ 7.84 (s, 1H), 6.65 (s, 1H), 5.30 (s, 2H), 5.14 (s, 2H), 3.97-4.00 (t, 2H), 3.89 (s, 3H), 3.74 (s, 3H), 1.68-1.76 (m, 2H), 1.44-1.53 (m, 2H), 0.94-0.97 (t, 3H).

**Example 123: 6-chloro-9-(3-methoxymethoxy-6-methyl-pyridin-2-ylmethyl)-9H-purin-2-ylamine**

*Step 1:* (3-methoxymethoxy-6-methyl-pyridin-2-yl)-methanol

The title compound was obtained by O-alkylation of 2-hydroxymethyl-6-methyl-pyridin-3-ol with chloromethyl methyl ether according to the general procedure 2.6. $^1$H-NMR ($CDCl_3$) δ 7.28-7.30 (d, 1H), 6.98-7.00 (d, 1H), 5.17 (s, 2H), 4.71 (s, 2H), 4.50 (s, 2H), 3.45 (s, 3H), 2.49 (s, 3H).

*Step 2:* 2-Bromomethyl-3-methoxymethoxy-6-methyl-pyridine

The title compound was obtained from (3-methoxymethoxy-6-methyl-pyridin-2-yl)-methanol according to the general procedure 2.5. $^1$H-NMR ($CDCl_3$) δ 7.32-7.40 (d 1H), 7.08-7.10 (d, 1H),5.30 (s, 2H), 4.67 (s, 2H), 3.55 (s, 3H), 2.54 (s, 3H).

*Step 3:* 6-Chloro-9-(3-methoxymethoxy-6-methyl-pyridin-2-ylmethyl)-9H-purin-2-ylamine

The title compound was obtained by alkylation of 2-amino-6-chloropurine with 2-bromomethyl-3-methoxymethoxy-6-methyl-pyridine according to the general procedure 1.1. HPLC Rt: 3.884 min. $^1$H-NMR ($CDCl_3$): δ 7.92 (s, 1H), 7.34-7.36 (d, 1H), 7.05-7.07 (d, 1H), 5.39 (s, 2H), 5.17 (s, 2H), 5.06 (s, 2H), 3.40 (s, 3H), 2.44 (s, 3H).

**Example 124: 3-(2-Amino-6-chloro-purin-9-ylmethyl)-3H-benzothiazole-2-thione**

The title compound was obtained by alkylation of 2-amino-6-chloropurine with 3-chloromethyl-3H-benzothiazole-2-thione according to the general procedure 1.1. HPLC Rt: 5.982 min. $^1$H-NMR ($d_6$-DMSO): δ 8.37 (s, 1H), 8.25-8.28 (d, 1H) 7.79-7.81 (d, 1H), 7.54-7.56 (t, 1H), 7.39-7.43 (m 1H), 7.20 (s, 2H), 6.62 (s, 2H), 3.34 (s, 3H).

**Example 125: 6-Chloro-9-(2,5-dimethyl-benzyl)-9H-purin-2-ylamine**

The title compound was obtained by alkylation of 2-amino-6-chloropurine with 2-chloromethyl-1,4-dimethylbenzene according to the general procedure 1.1. HPLC Rt: 5.920 min. $^1$H-NMR ($d_6$-DMSO): δ 8.10 (s, 1H), 7.10 (d, 1H) 7.04 (d, 1H), 6.95 (s, 2H), 6.65(s, 1H), 5.23, (s, 2H), 3.34 (s, 3H), 2.30 (s, 3H), 2.17 (s, 3H).

**Example 126: 6-Chloro-9-isoquinolin-1-ylmethyl-9H-purin-2-ylamine**

The title compound was obtained by alkylation of 2-amino-6-chloropurine with 1-bromomethyl-isoquinoline according to the general procedure 1.1. HPLC Rt: 4.306 min. $^1$H-NMR ($d_6$-DMSO): δ 8.43-8.45 (d, 1H) 8.27-8.28 (d, 1H), 8.20 (s, 1H), 8.03-8.05 (d 1H), 7.85-7.89 (m, 1H), 7.77-7.82 (m, 2H), 6.83 (s, 2H), 6.04 (s, 2H).

**Example 127: 9-benzo[1,2,5]thiadiazol-5-ylmethyl-6-chloro-9H-purin-2-ylamine**

The title compound was obtained by alkylation of 2-amino-6-chloropurine with 5-bromomethyl-benzo[1,2,5] thiadiazole according to the general procedure 1.1. HPLC Rt: 4.931 min. $^1$H-NMR ($d_6$-DMSO): δ 8.31 (s, 1H), 8.11 (d, 1H), 7.87 (br. s, 1H), 7.70-7.67 (dd, 1H), 6.98 (br, s, 2H), 5.52 (s, 2H).

**Example 128: 9-(1-methyl-1H-benzotriazol-5-ylmethyl)-6-chloro-9H-purin-2-ylamine**

The title compound was obtained by alkylation of 2-amino-6-chloropurine with 6-bromomethyl-1-methyl-1H-benzotriazole according to the general procedure 1.1. HPLC Rt: 4.295 min. $^1$H-NMR ($d_6$-DMSO): δ 8.29 (s, 1H), 7.95 (s, 1H), 7.86-7.84 (d, 1H), 7.55-7.53 (dd, 1H), 6.97 (s, 2H), 5.45 (s, 2H), 4.3 (s, 3H).

**Example 129: 6-chloro-9-(6-chloro-benzo[1,2,5]thiadiazol-5-ylmethyl)-9H-purin-2-ylamine**

The title compound was obtained by alkylation of 2-amino-6-chloropurine with 5-bromomethyl-6-chloro-benzo [1,2,5]thiadiazole according to the general procedure 1.1. HPLC Rt: 5.400 min. $^1$H-NMR ($d_6$-DMSO): δ 8.45 (s, 1H), 8.20 (s, 1H), 7.64 (s, 1H), 6.97 (s, 2H), 5.55 (s, 2H).

**Example 130: 9-benzo[1,2,5]thiadiazol-4-ylmethyl-6-chloro-9H-purin-2-ylamine**

The title compound was obtained by alkylation of 2-amino-6-chloropurine with 4-bromomethyl-benzo[1,2,5] thiadiazole according to the general procedure 1.1. HPLC Rt: 5.027 min. $^1$H-NMR ($d_6$-DMSO): δ 8.26 (s, 1H), 8.04-8.07 (d, 1H), 7.68-7.64 (dd, 1H), 7.22-7.20 (dd, 1H), 6.93 (s, 2H), 5.79 (s, 2H).

**Example 131: 6-chloro-9-(6-fluoro-4a,8a-dihydro-4H-benzo[1,3]dioxin-8-ylmethyl)-9H-purin-2-ylamine**

The title compound was obtained by alkylation of 2-amino-6-chloropurine with 8-chloromethyl-6-fluoro-4a,8a-dihydro-4H-benzo[1,3]dioxine according to the general procedure 1.1. HPLC Rt: 5.172 min. $^1$H-NMR ($CDCl_3$): δ 7.84 (s, 1H), 6.92-6.89 (dd, 1H), 6.70-6.67 (dd, 1H), 5.31 (s, 2H), 5.22 (s, 2H), 5.07 (s, 2H), 4.90 (s, 2H).

**Example 132: 1-[3-(2-Amino-6-chloro-purin-9-ylmethyl)-4-methoxy-phenyl]-ethanone**

The title compound was obtained by alkylation of 2-amino-6-chloropurine with 1-(3-chloromethyl-4-methoxyphenyl)-ethanone according to the general procedure 1.1. HPLC Rt: 4.887 min. $^1$H-NMR ($CDCl_3$): δ 8.03-8.02 (d, 1H), 7.97-7.95 (dd, 1H), 7.81 (s, 1H), 6.96-6.93 (d, 1H), 5.25 (s, 2H), 5.08 (s, 2H), 3.93 (s, 3H), 2.54 (s, 3H).

**Example 133: 6-chloro-9-(3-trifluoromethoxy-benzyl)-9H-purin-2-ylamine**

The title compound was obtained by alkylation of 2-amino-6-chloropurine with 1-chloromethyl-3-trifluoromethoxy-benzene according to the general procedure 1.1. HPLC Rt: 5.965 min. $^1$H-NMR ($CDCl_3$): δ 7.76 (s, 1H), 7.39-7.37 (t, 1H), 7.21-7.15 (m, 3H), 5.27 (s, 2H), 5.12 (s, 2H).

**Example 134: 6-chloro-9-(2-fluoro-3-trifluoromethyl-benzyl)-9H-purin-2-ylamine**

The title compound was obtained by alkylation of 2-amino-6-chloropurine with 1-bromomediyl-2-fluoro-3-trifluoromethyl-benzene according to the general procedure 1.1. HPLC Rt: 4.841 min. $^1$H-NMR ($CDCl_3$): δ 7.83 (s, 1H), 7.63-7.59 (t, 1H), 7.48-7.45(t, 1H), 7.25-7.22 (t, 1H), 5.36 (s, 2H), 5.12 (s, 2H).

**Example 135: 6-Chloro-9-(2-fluoro-4,5-dimethoxy-benzyl)-9H-purin-2-ylamine**

*Step 1:* (2-Fluoro-4,5-dimethoxy-phenyl)-methanol

A solution of 2-fluoro-4,5-dimethoxy benzaldehyde (6.0 mmol) in MeOH (10 mL) was treated with $NaBH_4$ (1.2 equiv.) at 0-23 °C for 0.5 h. After quenching with acetone and evaporating the solvent, work-up ($CH_2Cl_2$), drying ($MgSO_4$) and evaporation gave the title compound as a crude oily product (94% yield). $^1$H-NMR ($CDCl_3$): δ 6.90-6.88 (d, 1H), 6.61-6.64 (d, 1H), 4.65-4.63 (d, 2H), 3.86 (s, 3H), 3.84 (s, 3H), 2.00-1.94 (t, 1H).

*Step 2:* 1-Bromomethyl-2-fluoro-4,5-dimethoxy-benzene

The title compound was obtained from (2-fluoro-4,5-dimethoxy-phenyl)-methanol according to the general procedure 2.5. $^1$H-NMR ($CDCl_3$): δ 6.84-6.81 (d, 1H), 6.64-6.61 (d, 1H), 4.52 (s, 2H), 3.89 (s, 3H), 3.84 (s, 3H).

*Step 3:* 6-Chloro-9-(2-fluoro-4,5-dimethoxy-benzyl)-9H-purin-2-ylamine

The title compound was obtained by alkylation of 2-amino-6-chloropurine with 1-bromomethyl-2-fluoro-4,5-dimethoxy-benzene according to the general procedure 1.1. HPLC Rt: 4.939 min. $^1$H-NMR ($CDCl_3$): δ 7.79 (s, 1H), 6.88-6.85 (d, 1H), 6.69-6.66 (d, 1H), 5.22 (s, 2H), 5.12 (s, 2H), 3.89 (s, 3H), 3.82 (s, 3H).

**Example 136: 6-chloro-9-(2,3-dimethoxy-benzyl)-9H-purin-2-ylamine**

*Step 1:* 1-Bromornethyl-2,3-dimethoxy-benzene

The title compound was obtained from (2,3-dimethoxy-phenyl)-methanol according to the general procedure 2.5. $^1$H-NMR ($CDCl_3$): δ 7.02-7.08 (t, 1H), 6.95-6.98 (dd, 1H), 6.87-6.91 (dd, 1H), 4.57 (s, 2H), 3.92 (s, 3H), 3.86 (s, 3H).

*Step 2:* 6-Chloro-9-(2,3-dimethoxy-benzyl)-9H-purin-2-ylamine

The title compound was obtained by alkylation of 2-amino-6-chloropurine with 1-bromomethyl-2,3-dimethoxy-benzene according to the general procedure 1.1. HPLC Rt: 5.200. $^1$H-NMR ($CDCl_3$): δ 7.81 (s, 1H), 7.02-7.08 (t, 1H), 6.95-6.98(dd, 1H), 6.87-6.91 (dd, 1H), 5.28 (s, 2H), 5.12 (s, 2H), 3.92 (s, 3H), 3.87 (s, 3H).

**Example 137: 6-chloro-9-(3,4-dimethoxy-benzyl)-9H-purin-2-ylamine**

*Step 1:* 4-Bromomethyl-1,2-diinethoxy-benzene

The title compound was obtained from (3,4-dimethoxy-phenyl)-methanol according to the general procedure 2.5. $^1$H-NMR ($CDCl_3$): δ 6.95-6.98 (dd, 1H), 6.92-6.94 (d, 1H), 6.78-6.81 (d, 1H), 4.51 (s, 2H), 3.92 (s, 3H), 3.86 (s, 3H).

*Step 2:* 6-Chloro-9-(3,4-dimethoxy-benzyl)-9H-purin-2-ylamine

The title compound was obtained by alkylation of 2-amino-6-chloropurine with 4-bromomethyl-1,2-dimethoxy-benzene according to the general procedure 1.1. HPLC Rt: 4.753.

$^1$H-NMR ($CDCl_3$): δ 7.71 (s, 1H), 6.87 (s, 2H), 6.82 (s, 1H), 5.20 (s, 2H), 5.12 (s, 2H), 3.92 (s, 3H), 3.87 (s, 3H).

**Example 138: 9-(2-chloro-3,4,5-trimethoxy-benzyl)-6-methyl-9H-purin-2-ylamine**

A suspension of 6-chloro-9-(2-chloro-3,4,5-trimethoxy-benzyl)-9H-purin-2-ylamine, see example 97) (0.2 mmol) and tetrakis(triphenylphosphino)-palladium (0.02 mmol) in dry THF (3 mL) was treated with trimethylaluminum (2M in toluene, 0.45 mmol) under nitrogen and heated to reflux for 3 h. The reaction mixture was cooled to r.t., diluted with toluene (5 mL) and quenched with methanol (0.5 mL) followed by ammonium chloride (1 mmol). The mixture was

heated to reflux for 2 h and filtered, while hot, on Celite. See J. Med. Chem. 1999, 42, 2064-2086. TLC (100% EtOAc) Rf was 0.2. HPLC Rt: 4.800 min.

**Example 139: 6-chloro-9-(2-chloro-4,5-dimethoxy-benzyl)-9H-purin-2-ylamine**

*Step 1:* (2-Chloro-4,5-dimethoxy-phenyl)-methanol

The title compound was obtained by chlorination of (3,4-dimethoxy-phenyl)-methanol according to the general procedure 3.1. $^1$H-NMR ($CDCl_3$): δ 7.01 (s, 1H), 6.88 (s, 1H), 4.75-4.73 (d, 2H), 3.91 (s, 3H), 3.90 (s, 3H), 1.95-1.92 (t, 1H).

*Step 2:* 1-Bromomethyl-2-chloro-4,5-dimethoxy-benzene

The title compound was obtained from (2-chloro-4,5-dimethoxy-phenyl)-methanol according to the general procedure 2.5. $^1$H-NMR ($CDCl_3$): δ 6.92 (s, 1H), 6.88 (s, 1H), 4.60 (s, 2H), 3.91 (s, 3H), 3.90 (s, 3H).

*Step 3:* 6-Chloro-9-(2-chloro-4,5-dimethoxy-benzyl)-9H-purin-2-ylamine

The title compound was obtained by alkylation of 2-amino-6-chloropurine with 1-bromomethyl-2-chloro-4,5-dimethoxy-benzene according to the general procedure 1.1. HPLC Rt: 5.366 min. $^1$H-NMR ($CDCl_3$): δ 7.81 (s, 1H), 7.28 (s, 1H), 6.92 (s, 1H), 5.30 (s, 2H), 5.15 (s, 2H), 3.90 (s, 3H), 3.84 (s, 3H).

**Example 140: 6-chloro-9-(2-iodo-4,5-dimethoxy-benzyl)-9H-purin-2-ylamine**

*Step 1:* 4-Bromomethyl-1,2-dimethoxy-benzene

The title compound was obtained from (3,4-dimethoxy-phenyl)-methanol according to the general procedure 2.5. $^1$H-NMR ($CDCl_3$): δ 6.95-6.98 (dd, 1H), 6.92-6.94 (d, 1H), 6.78-6.81 (d, 1H), 4.51 (s, 2H), 3.92 (s, 3H), 3.86 (s, 3H).

*Step 2:* 1-Bromomethyl-2-iodo-4,5-dimethoxy-benzene

The title compound was obtained by iodination of 4-bromomethyl-1,2-dimethoxy-benzene according to the general procedure 3.1. $^1$H-NMR ($CDCl_3$): δ 7.04 (s, 1H), 6.95 (s, 1H), 4.61 (s, 2H), 3.91 (s, 3H), 3.90 (s, 3H).

*Step 3:* 6-Chloro-9-(2-iodo-4,5-dimethoxy-benzyl)-9H-purin-2-ylamine

The title compound was obtained by alkylation of 2-amino-6-chloropurine with 1-bromomethyl-2-iodo-4,5-dimethoxy-benzene according to the general procedure 1.1. HPLC Rt: 5.470 min. $^1$H-NMR ($CDCl_3$): δ 7.84 (s, 1H), 7.08 (s, 1H), 6.93 (s, 1H), 5.30 (s, 2H), 5.15 (s, 2H), 3.91 (s, 3H), 3.82 (s, 3H).

**Example 141: 6-Bromo-9-(2-chloro-3,4,5-trimethoxy-benzyl)-9H-purin-2-ylamine**

*Step 1:* 2-Chloro-1-chloromethyl-3,4,5-trimethoxy-benzene

The title compound was obtained by chlorination of 5-chloromethyl-1,2,3-trimethoxy-benzene according to the general procedure 3.1. $^1$H-NMR ($CDCl_3$): δ 6.82 (s, 1H), 4.70 (s, 1H), 3.93 (s, 3H), 3.90 (s, 3H) 3.87 (s, 3H).

*Step 2:* Synthesis of 6-chloro-9-(2-chloro-3,4,5-trimethoxy-benzyl)-9H-purin-2-ylamine

The title compound was obtained by alkylation of 6-bromoguanine with 2-chloro-1-chloromethyl-3,4,5-trimethoxy-benzene according to the general procedure 1.1. HPLC Rt: 5.676 min. $^1$H-NMR ($CDCl_3$): δ 7.82 (s, 1H), 6.70 (s, 1H), 5.32 (s, 2H), 5.15 (s, 2H), 3.93 (s, 3H), 3.91 (s, 3H) 3.79 (s, 3H).

**Example 142: 6-chloro-9-(6-chloro-benzo[1,3]dioxol-5-ylmethyl)-9H-purin-2-ylamine**

The title compound was obtained by alkylation of 2-amino-6-chloropurine with 5-chloro-6-chloromethyl-benzo [1,3]dioxole according to the general procedure 1.1. HPLC Rt: 5.506 min. $^1$H-NMR ($CDCl_3$): δ 7.81 (s, 1H), 6.88 (s, 1H), 6.79 (s, 1H), 5.98 (s, 2H), 5.25 (s, 2H), 5.13 (s, 2H).

**Example 143: 6-chloro-9-(2,4-dimethoxy-3-methyl-benzyl)-9H-purin-2-ylamine**

*Step 1:* 1-Bromomethyl-2,4-dimethoxy-3-methyl-benzene

The title compound was obtained from (2,4-dimethoxy-3-methyl-phenyl)-methanol according to the general procedure 2.5.

*Step 2:* 6-Chloro-9-(2,4-dimethoxy-3-methyl-benzyl)-9H-purin-2-ylamine

The title compound was obtained by alkylation of 2-amino-6-chloropurine with 1-bromomethyl-2,4-dimethoxy-3-methyl-benzene according to the general procedure 1.1. HPLC Rt: 5.433 min. $^1$H-NMR ($CDCl_3$): δ 7.76 (s, 1H), 7.08-7.06 (d, 1H), 6.60-6.62 (d, 1H), 5.20 (s, 2H), 5.07 (s, 2H), 3.82 (s, 3H), 3.72 (s, 3H), 2.17 (s, 3H).

**Example 144: 6-Chloro-9-(2-chloro-3,4-dimethoxy-benzyl)-9H-purin-2-ylamine**

*Step 1:* 1-Bromomethyl-2-chloro-3,4-dimethoxy-benzene

The title compound was obtained from (2-chloro-3,4-dimethoxy-phenyl)-methanol according to the general procedure 2.5.

*Step 2:* 6-Chloro-9-(2-chloro-3,4-dimethoxy-benzyl)-9H-purin-2-ylamine

The title compound was obtained by alkylation of 2-amino-6-chloropurine with 1-bromomethyl-2-chloro-3,4-dimethoxy-benzene according to the general procedure 1.1. HPLC Rt: 5.633 min. $^1$H-NMR ($CDCl_3$): δ 7.80 (s, 1M, 7.00-6.98 (d, 1H), 6.82-6.79 (d, 1H), 5.31 (s, 2H), 5.08 (s, 2H), 3.88 (s, 3H), 3.88 (s, 3H).

**Example 145: 6-chloro-9-(3-methoxy-benzyl)-9H-purin-2-ylamine**

The title compound was obtained by alkylation of 2-amino-6-chloropurine with 1-bromomethyl-3-methoxybenzene according to the general procedure 1.1. HPLC Rt: 5.136 min. $^1$H-NMR ($CDCl_3$): δ 7.75 (s, 1H), 7.30-7.28 (m. 1H), 6.88-6.85 (dd, 1H), 6.84-6.82 (dd, 1H), 6.80-6.79 (m, 1H), 5.28 (s, 2H), 5.15 (s, 2H), 3.78 (s, 3H).

**Example 146: 6-Chloro-9-(2,6-dibromo-3,5-dimethoxy-benzyl)-9H-purin-2-ylamine**

*Step 1:* 2-Bromo-1-chloromethyl-3,5-dimethoxy-benzene and 2,4-dibromo-3-chloromethyl-1,5-dimethoxy-benzene

Bromination of 1-chloromethyl-3,5-dimethoxy-benzene according to the general procedure 3.1 gave a mixture of the two title compounds, which were separated by flash chromatography. $^1$H-NMR of 2,4-dibromo-3-chloromethyl-1,5-dimethoxy-benzene (CDCl3): δ 6.52 (s, 1H), 5.02 (s, 2H), 3.93 (s, 6H). 2-bromo-1-chloromethyl-3,5-dimethoxy-benzene $^1$H-NMR ($CDCl_3$): δ 6.67-6.67 (d, 1H), 6.47-6.46 (d, 1H), 4.80 (s, 2H), 3.85 (s, 3H), 3.82 (s, 3H).

*Step 2:* 6-Chloro-9-(2,6-dibromo-3,5-dimethoxy-benzyl)-9H-purin-2-ylamine

The title compound was obtained by alkylation of 2-amino-6-chloropurine with 2,4-dibromo-3-chloromethyl-1,5-dimethoxy-benzene according to the general procedure 1.1. HPLC Rt: 6.022 min. $^1$H-NMR ($CDCl_3$): δ 7.46 (s, 1H), 6.64 (s, 1H), 5.64 (s, 2H), 5.14 (s, 2H), 3.99 (s, 6H).

**Example 147: 9-(2-Bromo-3,5-dimethoxy-benzyl)-6-chloro-9H-purin-2-ylamine**

The title compound was obtained by alkylation of 2-amino-6-chloropurine with 2-bromo-1-chloromethyl-3,5-dimethoxy-benzene (see previous example, step 1) according to the general procedure 1.1. HPLC Rt: 6.026 min. $^1$H-NMR ($CDCl_3$): δ 7.82 (s, 1H), 6.48-6.47 (d, 1H), 6.32-6.32 (d, 1H), 5.35 (s, 2H), 5.09 (s, 2H), 3.90 (s, 3H), 3.73 (s, 3H).

**Example 148: 6-chloro-9-(3,5-dimethoxy-benzyl)-9H-purin-2-ylamine**

The title compound was obtained by alkylation of 2-amino-6-chloropurine with 1-chloromethyl-3,5-dimethoxybenzene according to the general procedure 1.1. HPLC Rt: 5.257 min. $^1$H-NMR ($CDCl_3$): δ 7.79 (s, 1H), 6.44-6.42 (t, 1H), 6.41-6.39 (d, 2H), 5.22 (s, 2H), 5.15 (s, 2H), 3.80 (s, 6H).

**Example 149: N-[6-chloro-9-(3,4,5-trimethoxy-benzyl)-9H-purin-2-yl]-acetamide**

A solution of 6-chloro-9-(3,4,5-trimethoxy-benzyl)-9H-purin-2-ylamine in acetic acid was treated with fuming $HNO_3$ at 0 ˚C for 15 min. Work-up and preparative TLC (EtOAc:hexane 1:1) gave N-[6-chloro-9-(3,4,5-trimethoxy-benzyl)-9H-purin-2-yl]-acetamide. HPLC Rt: 5.744 min. $^1$H-NMR ($CDCl_3$): δ 8.09 (s, 1H), 6.58 (s, 2H), 5.33 (s, 2H), 3.85 (s, 3H), 3.85 (s, 6H), 2.43 (s, 3H).

**Example 150: 6-chloro-9-(2,5-dimethoxy-benzyl)-9H-purin-2-ylamine**

The title compound was obtained by alkylation of 2-amino-6-chloropurine with 1-chloromethyl-2,5-dimethoxy-benzcne according to the general procedure 1.1. HPLC Rt: 5.291 min. $^1$H-NMR ($CDCl_3$): δ 7.82 (s, 1H), 6.85-6.84 (d, 1H), 6.82-6.82 (d, 2H), 5.18 (s, 2H), 5.16 (s, 2H), 3.80 (s, 3H), 3.75 (s, 3H).

**Example 151: 8-bromo-6-chloro-9-(2,5-dimethoxy-benzyl)-9H-purin-2-ylamine**

The title compound was obtained by bromination of 6-chloro-9-(2,5-dimethoxybenzyl)-9H-purin-2-ylamine according to the general procedure 1.2. HPLC Rt: 6.150 min. $^1$H-NMR ($CDCl_3$): δ 6.83-6.78 (m, 2H), 6.37-6.36 (d, 1H), 5.31 (s, 2H), 5.13 (s, 2H), 3.83 (s, 3H), 3.70 (s, 3H).

**Example 152: 6-chloro-9-(4,5-dimethoxy-2-nitro-benzyl)-9H-purin-2-ylamine**

The title compound was obtained by alkylation of 2-amino-6-chloropurine with 1-bromomethyl-4,5-dimethoxy-2-nitro-benzene according to the general procedure. 1.1. HPLC Rt: 5.194 min. $^1$H-NMR ($CDCl_3$): δ 7.98 (s, 1H), 7.74 (s, 1H), 6.79 (s, 1H), 5.67 (s, 2H), 5.15 (s, 2H), 3.98 (s, 3H), 3.85 (s, 3H).

**Example 153: 8-bromo-6-chloro-9-(4,5-dimethoxy-2-nitro-benzyl)-9H-purin-2-ylamine**

The title compound was obtained by brominating 6-chloro-9-(4,5-dimethoxy-2-nitro-benzyl)-9H-purin-2-ylamine (see previous example) according to the general procedure 1.2. HPLC Rt: 6.040 min. $^1$H-NMR ($CDCl_3$): δ 7.74 (s, 1H), 6.13 (s, 1H), 5.78 (s, 2H), 5.16 (s, 2H), 3.99 (s, 3H), 3.71 (s, 3H).

**Example 154: 6-chloro-9-(2,5-dichloro-benzyl)-9H-purin-2-ylamine**

The title compound was obtained by alkylation of 2-amino-6-chloropurine with 2-bromomethyl-1,4-dichloro-benzene according to the general procedure 1.1. HPLC Rt: 5.846 min. $^1$H-NMR ($CDCl_3$): δ 7.82 (s, 1H), 7.38-7.36 (d, 1H), 7.28-7.26 (dd, 1H), 7.18-7.18 (d, 1H), 5.32 (s, 2H), 5.17 (s, 2H).

**Example 155: 6-chloro-9-(2,3,5-trifluoro-benzyl)-9H-purin-2-ylamine**

The title compound was obtained by alkylation of 2-amino-6-chloropurine with 1-bromomethyl-2,3,5-trifluoro-benzene according to the general procedure 1.1. HPLC Rt: 5.414 min. $^1$H-NMR ($CDCl_3$): δ 7.82 (s, 1H), 6.98-6.89 (m, 1H), 6.82-6.75 (m. 1H), 5.30 (s, 2H), 5.13 (s, 2H).

**Example 156: (2-amino-6-chloro-purin-9-yl)-(3,4,5-trimethoxy-phenyl)-methanone**

A solution of 6-chloro-9H-purin-2-ylamine in pyridine was treated with 3,4,5-trimethoxybenzoyl chloride at r.t. for 2 h. Work-up and purification by preparative TLC (EtOAc:hexane 1:1) gave the title compound. HPLC Rt: 5.305 min. $^1$H-NMR ($CDCl_3$): δ 8.24 (s, 1H), 7.13 (s, 2H), 5.36 (s, 2H), 3.99 (s, 3H), 3.88 (s, 6H).

**Example 157: N-[9-(2-Bromo-3,4,5-trimethoxy-benzyl)-6-chloro-9H-purin-2-yl]-acetamide**

A suspension of 9-(2-bromo-3,4,5-trimethoxy-benzyl)-6-chloro-9H-purin-2-ylamine (example 51) in acetic anhydride was treated with a catalytic amount of conc. $H_2SO_4$ at r.t. for 3 h. Work-up and purification by preparative TLC (EtOAc:hexane 9:1) gave the title compound. HPLC Rt: 5.603 min. $^1$H-NMR ($CDCl_3$): δ 8.20 (s, 1H), 8.10 (s, 1H), 7.00 (s, 1H), 5.47 (s, 2H), 3.92 (s, 3H), 3.90 (s, 3H), 3.86 (s, 3H), 2.51 (s, 3H).

**Example 158: N-[9-(2-bromo-3,4,5-trimethoxy-benzyl)-6-chloro-9H-purin-2-yl]-N-methyl-acetamide**

A mixture of N-[9-(2-bromo-3,4,5-trimethoxy-benzyl)-6-chloro-9H-purin-2-yl]-acetamide and NaH in DMF was stirred at r.t. for 15 min, before adding MeI. Stirring was prolonged for 2 h at 50 ˚C. Work-up and purification by preparative TLC (EtOAc:hexane 1:1) gave the title compound. HPLC Rt: 6.422 min. $^1$H-NMR ($CDCl_3$): δ 8.20 (s, 1H), 6.80 (s, 1H), 5.45 (s, 2H), 3.93 (s, 3H), 3.90 (s, 3H), 3.82 (s, 3H), 3.57 (s, 3H), 2.51 (s, 3H).

**Example 159: 6-chloro-9-(3,5-dichloro-benzyl)-9H-purin-2-ylamine**

*Step 1:* 1-Bromomethyl-3,5-dichloro-benzene

The title compound was obtained from (3,5-dichloro-phenyl)-methanol according to the general procedure 2.5.

*Step 2:* 6-chloro-9-(3,5-dichloro-benzyl)-9H-purin-2-ylamine

The title compound was obtained by alkylation of 2-amino-6-chloropurine with 1-bromomethyl-3,5-dichloro-benzene. HPLC Rt: 6.074 min. $^1$H-NMR (Acetone-$d_6$): δ 8.12 (s, 1H), 7.45-7.43 (t, 1H), 7.72-7.42 (d, 2H), 6.30 (s, 2H), 5.40 (s, 2H).

**Example 160: 6-chloro-9-(3,4-dichloro-benzyl)-9H-purin-2-ylamine**

The title compound was obtained by alkylation of 2-amino-6-chloropurine with 4-bromomethyl-1,2-dichloro-benzene according to the general procedure 1.1. HPLC Rt: 5.982 min. $^1$H-NMR ($CDCl_3$): δ 7.76 (s, 1H), 7.48-7.45 (d, 1H), 7.40-7.39 (d, 1H), 7.12-7.10 (dd, 1H), 5.23 (s, 2H), 5.12 (s, 2H).

**Example 161: 8-bromo-6-chloro-9-(3,4-dichloro-benzyl)-9H-purin-2-ylamine**

The title compound was obtained by bromination of 6-chloro-9-(3,4-dichlorobenzyl)-9H-purin-2-ylamine (see previous example) according to the general procedure 1.2. HPLC Rt: 6.878 min. $^1$H-NMR ($CDCl_3$): δ 7.45-7.43 (m, 2H), 7.17-7.14 (dd, 1H), 5.23 (s, 2H), 5.12 (s, 2H).

**Example 162: 6-chloro-9-(2-chloro-3,4,5-trimethoxy-benzyl)-9H-purin-2-ylamine**

Chlorination of 6-chloro-9-(3,4,5-trimethoxy-benzyl)-9H-purin-2-ylamine according to the general procedure 3.1 gave a mixture of the title compound and of 6-chloro-9-(2,6-dichloro-3,4,5-trimethoxy-benzyl)-9H-purin-2-ylamine. The two compounds were isolated by preparative TLC. HPLC Rt: 5.626 min. $^1$H-NMR ($CDCl_3$): δ 7.83 (s, 1H), 6.68 (s, 1H), 5.51 (s, 2H), 5.23 (s, 2H), 3.94 (s, 3H), 3.90 (s, 3H), 3.80 (s, 3H).

**Example 163: 6-chloro-9-(2,6-dichloro-3,4,5-trimethoxy-benzyl)-9H-purin-2-**ylamine

See the previous example. HPLC Rt: 6.099 min. $^1$H-NMR ($CDCl_3$): δ 7.57 (s, 1H), 5.48 (s, 2H), 5.12 (s, 2H), 3.99 (s, 3H), 3.92 (s, 6H).

**Example 164: 2-amino-9-(2-chloro-3,4,5-trimethoxy-benzyl)-9H-purin-6-ol**

The title compound was obtained by heating a solution of 6-chloro-9-(2-chloro-3,4,5-trimethoxy-benzyl)-9H-purin-2-ylamine (see example 98) in 1N HCl for 4 h. Solvent was evaporated and the residue was washed with EtOAc to give the title compound. HPLC Rt: 4.603 min. $^1$H-NMR (DMSO-$d_6$): δ 7.82 (s, 1H), 6.65 (s, 1H), 6.60 (s, 2H), 5.15 (s, 2H), 3.81 (s, 3H), 3.77 (s, 3H), 3.69 (s, 3H).

**Example 165: 6-bromo-9-(3,4,5-trimethoxy-benzyl)-9H-purin-2-ylamine**

The title compound was obtained by alkylation of 6-bromoguanine with 3-chloromethyl-3,4,5-trimethoxy benzene according to the general procedure 1.1. HPLC. Rt: 4.947 min. $^1$H-NMR ($CDCl_3$): δ 7.80 (s, 1H), 6.50 (s, 2H), 5.20 (s, 2H), 5.18 (s, 2H), 3.85 (s, 3H), 3.84 (s, 6H).

**Example 166: 6-bromo-9-(2-bromo-3,4,5-trimethoxy-benzyl)-9H-purin-2-ylamine**

The title compound was obtained by bromination of 6-bromo-9-(3,4,5-trimethoxy-benzyl)-9H-purin-2-ylamine (see previous example) according to the general procedure 3.1. HPLC Rt: 5.793 min. $^1$H-NMR ($CDCl_3$): δ 7.88 (s, 1H), 6.68 (s, 1H), 5.33 (s, 2H), 5.19 (s, 2H), 3.93 (s, 3H), 3.90 (s, 3H), 3.79 (s, 3H).

**Example 167: 9-(2-bromo-3,4,5-trimethoxy-benzyl)-6-ethylsulfanyl-9H-purin-2-ylamine**

A mixture of 6-bromo-9-(2-bromo-3,4,5-trimethoxy-benzyl)-9H-purin-2-ylamine (see previous example), EtSH, $K_2CO_3$ and THF was placed in a pressure vessel and heated to 70 ˚C for 6 h. Work-up and purification by preparative TLC (EtOAc:hexane 1:1) gave the title compound. HPLC Rt: 6.039 min. $^1$H-NMR ($CDCl_3$): δ 7.72 (s, 1H), 6.62 (s, 1H), 5.30 (s, 2H), 4.98 (s, 2H), 3.91 (s, 3H), 3.88 (s, 3H), 3.74 (s, 3H), 3.35-3.29 (q, 2H), 1.44-1.41 (t, 3H).

**Example 168: 9-(2-bromo-3,4,5-trimethoxy-benzyl)-6-methoxy-9H-purin-2-ylamine**

A solution of 9-(2-bromo-3,4,5-trimethoxy-benzyl)-6-chloro-9H-purin-2-ylamine (see example 51) in MeOH was treated with MeONa at reflux for 1 h. Work-up and purification by preparative TLC (EtOAc:hexane 1:1) gave the title compound. HPLC Rt: 5.229 min. $^1$H-NMR ($CDCl_3$): δ 7.69 (s, 1H), 6.58 (s, 1H), 5.33 (s, 2H), 4.88 (s, 2H), 4.11 (s, 3H), 3.93 (s, 3H), 3.89 (s, 3H), 3.74 (s, 3H).

**Example 169: 9-(2-bromo-3,4,5-trimethoxy-benzyl)-9H-purine-2,6-diamine**

A solution of 9-(2-bromo-3,4,5-trimethoxy-benzyl)-6-chloro-9H-purin-2-ylamine (see example 51) in MeOH was treated with $NH_3$ (7N in MeOH) in a pressure vessel at 90 ˚C for 16 h. Work-up and purification by preparative TLC (EtOAc:hexane 3:1) gave the title compound. HPLC Rt: 4.884 min. $^1$H-NMR (DMSO-$d_6$): δ 7.67 (s, 1H), 6.71 (s, 2H), 6.60 (s, 1H), 5.84 (s, 2H), 5.17 (s, 2H), 3.80 (s, 3H), 3.76 (s, 3H), 3.66 (s, 3H).

**Example 170: 6-chloro-9-(2-iodo-3,4,5-trimethoxy-benzyl)-9H-purin-2-ylamine**

The title compound was obtained by iodination of 6-chloro-9-(3,4,5-trimethoxybenzyl)-9H-purin-2-ylamine (see example 48) according to the general procedure 3.1. HPLC Rt: 5.887 min. $^1$H-NMR ($CDCl_3$): δ 7.87 (s, 1H), 6.67 (s, 1H), 5.33 (s, 2H), 5.22 (s, 2H), 3.91 (s, 3H), 3.88 (s, 3H), 3.77 (s, 3H).

**Example 171: 9-(2-bromo-3,5-dimethoxy-4-methoxymethoxy-benzyl)-6-chloro-9H-purin-2-ylamine**

The title compound was obtained by alkylation of 4-(2-amino-6-chloro-purin-9-ylmethyl)-3-bromo-2,6-dimethoxy-phenol (see example 50) chloromethyl methyl ether according to the general procedure 2.6 (NaOH, THF, r.t.) HPLC Rt: 5.817 min. $^1$H-NMR ($CDCl_3$): δ 7.90 (s, 1H), 6.70 (s, 1H), 5.35 (s, 2H), 5.32 (s, 2H), 5.17 (s, 2H), 3.93 (s, 3H), 3.78 (s, 3H), 3.62 (s, 3H).

**Example 172: 9-benzothiazol-2-ylmethyl-6-chloro-9H-purin-2-ylamine**

The title compound was obtained by alkylation of 2-amino-6-chloropurine with 2-bromomethyl-benzothiazole according to the general procedure 1.1. HPLC Rt: 5.055 min. $^1$H-NMR (DMSO-$d_6$): δ 8.34 (s, 1H), 8.09-8.07 (dd, 1H), 7.98-7.96 (d, 1H), 7.53-7.50 (m, 1H), 7.47-7.43 (m, 1H), 7.01 (s, 2H), 5.81 (s, 2H).

**Example 173: 6-chloro-9-(4-methoxy-benzyl)-9H-purin-2-ylamine**

The title compound was obtained by alkylation of 2-amino-6-chloropurine with 1-chloromethyl-4-methoxy-benzene according to the general procedure 1.1. HPLC Rt: 5.067 min. $^1$H-NMR ($CDCl_3$): δ 7.69 (s, 1H), 7.22-7.20 (d, 2H), 6.88-6.86 (d, 2H), 5.22 (s, 2H), 5.17 (s, 2H), 3.79 (s, 3H).

**Example 174: 9-(2-bromo-4,5-dimethoxy-benzyl)-6-chloro-9H-purin-2-ylamine**

*Step 1:* (2-Bromo-4,5-dimethoxy-phenyl)-methanol

The title compound was obtained by bromination of (3,4-dimethoxy-phenyl)-methanol according to the general procedure 3.1. $^1$H-NMR ($CDCl_3$): δ 7.03 (s, 1H), 7.03 (s, 1H), 4.70 (s, 2H), 3.91 (s, 3H), 3.89 (s, 3H)

*Step 2:* 1-Bromomethyl-2-chloro-4,5-dimethoxy-benzene

The title compound was obtained from (2-bromo-4,5-dimethoxy-phenyl)-methanol according to the general procedure 2.5. $^1$H-NMR ($CDCl_3$): δ 7.03 (s, 1H), 6.94 (s, 1H), 4.60 (s, 2H), 3.89 (s, 3H), 3.86 (s, 3H)

*Step 3:* 9-(2-Bromo-4,5-dimethoxy-benzyl)-6-chloro-9H-purin-2-ylamine

The title compound was obtained by alkylation of 2-amino-6-chloropurine with 1-bromomethyl-2-chloro-4,5-dimethoxy-benzene according to the general procedure 1.1. HPLC Rt: 5.458 min. $^1$H-NMR ($CDCl_3$): δ 7.84 (s, 1H), 7.08 (s, 1H), 6.93 (s, 1H), 5.30 (s, 2H), 5.15 (s, 2H), 3.90 (s, 3H), 3.82 (s, 3H).

**Example 175: 6-Chloro-9-(4-iodo-3,5-dimethyl-pyridin-2-ylmethyl)-9H-purin-2-**ylamine

*Step 1:* 2,3,5-Collidine-N-oxide

The title compound was obtained by oxidation of 2,3,5-collidine according to the general procedure 2.1 (yield 70%). HPLC Rt: 3.964 min. $^1$H-NMR ($CDCl_3$): δ 8.03 (s, 1H), 6.90 (s, 1H), 2.47 (s, 3H), 2.31 (s, 3H), 2.24 (s, 3H). m/z (%) 138.2 (M+1, 100%). Rf(20% MeOH/EtOAc) was 0.35.

*Step 2:* 2,3,5-Trimethyl-4-nitro-pyridine 1-oxide

A suspension of 2,3,5-collidine-N-oxide (3.77 g, 28 mmol) in conc. $H_2SO_4$ (8 mL) was cooled to 0 ˚C and fuming $HNO_3$ (5 mL, 100 mmol) was added dropwise. The resulting transparent solution was stirred at 100 ˚C for 24 h, cooled to r.t., poured onto ice, and the pH was adjusted to 10. Work-up ($CHCl_3$), drying ($MgSO_4$), and evaporation gave the title compound, (97% yield, 97% purity). Rf (MeOH/EtOAc 1:9): 0.7. HPLC Rt: 4.756 min. $^1$H NMR ($CDCl_3$): δ 8.08 (s, 1H), 2.50 (s, 3H), 2.27 (s, 3H), 2.23 (s, 3H). m/z (%) 183.1 (M+1, 100%).

*Step 3:* 2,3,5-Trimethyl-pyridin-4-ylamine-1-oxide hydrochloride

A suspension of 2,3,5-trimethyl-4-nitro-pyridine 1-oxide (4.2g, 23 mmol) and 10% Pd/C (0.42 g) in conc. aq. HC1/EtOH (1:11) was treated with $H_2$ (60 psi) at r.t. for 3 h. Filtration and evaporation gave the title compound as a slightly yellow solid. HPLC Rt: 4.756 min. $^1$H-NMR (DMSO-$d_6$): δ 8.28 (s, 1H), 7.24 (s, 2H), 2.50 (s, 3H), 2.12 (s, 3H), 2.11 (s, 3H). m/z (%) 153.2 (M+1, 100%).

*Step 4:* 4-Iodo-2,3,5-trimethyl-pyridine-1-oxide

A solution of 2,3,5-Trimethyl-pyridin-4-ylamine-1-oxide hydrochloride (1.9 g, 10 mmol) $HBF_4$ (20 mmol) in water (50 mL) was cooled to 0˚C. A solution of $NaNO_2$ (0.76 g, 11 mmol) in water (5 mL) was added dropwise to give a dark yellow solution which gradually gave a precipitate within 15 min. Potassium iodide (2.3 g, 1.39 x $10^{-2}$ mol) was added slowly in several portion to give a dark brown precipitate. The reaction mixture was stirred at r.t. for 5 min, and then heated to 60 ˚C for 10 min. The mixture was cooled to r.t. and the pH was adjusted to 10. Work-up ($CHCl_3$), drying ($MgSO_4$), evaporation, and flash chromatography gave the title compound. HPLC Rt: 5.579 min. $^1$H-NMR ($CDCl_3$):δ 8.07 (s, 1H), 2.62 (s, 3H), 2.56 (s, 3H), 2.38 (s, 3H). m/z (%) 264.1 (M+1, 100%).

*Step 5:* Acetic acid 4-iodo-3,5-dimethyl-pyridin-2-ylmethyl ester

The title compound was obtained by treating 4-iodo-3,5-dimethyl-pyridine 1-oxide was with $Ac_2O$ according to the general procedure 2.2. HPLC Rt: 2.913 min. $^1$H-NMR ($CDCl_3$): δ 8.26 (s, 1H), 5.32 (s, 2H), 2.47 (s, 3H), 2.41 (s, 3H), 2.24 (s, 3H). m/z (%) 306.0 (M+1, 100%).

*Step 6:* (4-Iodo-3,5-dimethyl-pyridin-2-yl)-methanol

The title compound was obtained by deacetylation of 4-iodo-3,5-dimethyl-pyridin-2-ylmethyl ester according to the general procedure 2.3. HPLC Rt: 3.773 min. $^1$H-NMR ($CDCl_3$): δ 8.15 (s, 1H),4.70 (s, 2H), 2.46 (s, 3H), 2.40 (s, 3H). m/z (%) 264.1 (M+1, 100%).

*Step 7:* 2-Bromomethyl-4-iodo-3,5-dimethyl-pyridine

The title compound was obtained from (4-Iodo-3,5-dimethyl-pyridin-2-yl)-methanol according to the general procedure 2.5. HPLC Rt: 5.957 min. $^1$H-NMR ($CDCl_3$): δ 8.14 (s, 1H), 4.67 (s, 2H), 2.59 (s, 3H), 2.45 (s, 3H). m/z (%) 326.07 (M+1, 100%), 328.07 (M+1, 100%).

*Step* 8: 6-Chloro-9-(4-iodo-3,5-dimethyl-pyridiun-2-ylmethyl)-9H-purin-2-ylamine

The title compound was obtained by alkylation of 2-amino-6-chloropurine with 2-bromomethyl-4-iodo-3,5-dimethyl-pyridine according to the general procedure 1.1. HPLC Rt: 5.361 min. $^1$H-NMR ($CDCl_3$): δ 8.08 (s, 1H), 7.86 (s, 1H), 5.41 (s, 2H), 5.04 (s, 2H), 2.57 (s, 3H), 2.41 (s, 3H).

**Example 176: 6-chloro-9-(4-methyl-quinolin-2-ylmethyl)-9H-purin-2-ylamine**

*Step 1*: 2,4-Dimethyl-quinoline 1-oxide:

The title compound was obtained by oxidation of 2,4-dimethyl-quinoline according to the general procedure 2.1. HPLC Rt: 4.489 min. $^1$H-NMR ($CDCl_3$): δ, 8.89-8.07 (dd, 1H), 8.00-7.97 (dd, 1H), 7.82-7.79 (m, 1H), 7.69-7.65 (m, 1H), 7.20 (s, 1H), 2.73 (s, 3H), 2.69 (s, 3H).

*Step 2*: Acetic acid 4-methyl-quinolin-2-ylmethyl ester

The title compound was obtained by treating 2,4-dimethyl-quinoline-1-oxide with $Ac_2O$ according to the general procedure 2.2. HPLC Rt: 3.158 min. Rf (EtOAc/Hexane 1:1): 0.8.

*Step 3:* (4-Methyl-quinolin-2-yl)-methanol :

The title compound was obtained by deacetylation of acetic acid 4-methyl-quinolin-2-ylmethyl ester according to the general procedure 2.3. HPLC Rt: 3.715 min. $^1$H-NMR ($CDCl_3$): δ, 8.08-8.06 (dd, 1H), 8.00-7.97 (dd, 1H), 7.73-7.69 (m, 1H), 7.57-7.54 (m, 1H), 7.12 (s, 1H), 4.87 (s, 2H), 4.52 (s, 1H), 2.70 (s, 3H).

*Step 4:* 2-bromomethyl-4-methyl-quinoline:

The title compound was obtained from (4-methyl-quinolin-2-yl)-methanol according to the general procedure 2.5. HPLC Rt: 4.516 min. $^1$H-NMR ($CDCl_3$): δ, 8.07-8.05 (dd, 1H), 7.98-7.96 (dd, 1H), 7.73-7.69 (m, 1H), 7.58-7.54 (m, 1H), 7.40 (d, 1H), 4.66 (s, 2H), 2.70 (s, 3H).

*Step 5:* 6-chloro-9-(4-methyl-quinolin-2-ylmethyl)-9H-purin-2-ylamine

The title compound was obtained by alkylation of 2-amino-6-chloropurine with 2-bromomethyl-4-methyl-quinoline according to the general procedure 1.1. HPLC Rt: 4.387 min. $^1$H-NMR (DMSO-d6): δ 8.31 (s, 1H), 8.11-8.09 (dd, 1H), 7.90-7.88 (dd, 1H), 7.77-7.74 (m, 1H), 7.66-7.62 (m, 1H), 7.27 (s, 1H), 6.91 (s, 2H) 5.58 (s, 2H), 2.68 (s, 3H).

**Example 177: 6-bromo-9-(4-methyl-quinolin-2-ylmethyl)-9H-purin-2-ylamine**

The title compound was obtained by alkylation of 6-bromoguanine with 2-bromomethyl-4-methyl-quinoline (see previous example) according to the general procedure 1.1. HPLC Rt: 4.489 min. $^1$H-NMR (DMSO-d6): δ 8.27 (s, 1H), 8.06-8.04 (dd, 1H), 7.85-7.83 (dd, 1H), 7.73-7.69 (m, 1H), 7.61-7.57 (m, 1H), 7.22 (s, 1H), 6.89 (s, 2H), 5.52 (s, 2H), 2.64 (s, 3H)

**Example 178: 6-bromo-9-(4-methyl-1-oxy-quinolin-2-ylmethyl)-9H-purin-2-ylamine**

The title compound was obtained by oxidation of 6-bromo-9-(4-methyl-quinolin-2-ylmethyl)-9H-purin-2-ylamine (see previous example) according to the general procedure 2.1. HPLC Rt: 4.698 min. $^1$H-NMR (DMSO-d6): δ 8.62-8.60 (dd, 1H), 8.27 (s, 1H), 8.12-8.10 (dd, 1H), 7.88-7.85 (m, 1H), 7.79-7.75 (m, 1H), 6.93 (s, 2H), 6.89 (s, 1H) 5.57 (s, 2H), 2.64 (s, 3H).

**Example 179: 6-chloro-9-(3,5-dimethyl-4-methylsulfanyl-pyridin-2-ylmethyl)-9H-purin-2-ylamine**

*Step 1*: 2,3,5-Trimethyl-4-methylsulfanyl-pyridine 1-oxide

A solution of 4-bromo-2,3,5-trimethyl-pyridine 1-oxide in THF was treated in a pressure vessel with NaSMe at 110˚C for 16 h. HPLC Rt: 5.303 min. $^1$H-NMR ($CDCl_3$): δ 8.07 (s, 1H), 2.57 (s, 3H), 2.52 (s, 3H), 2.42 (s, 3H), 2.23 (s, 3H).

*Step 2*: Acetic acid 3,5-dimethyl-4-methylsulfanyl-pyridin-2-ylmethyl ester

The title compound was obtained by treating 2,3,5-trimethyl-4-methylsulfanyl-pyridine 1-oxide with $Ac_2O$ according to the general procedure 2.2. HPLC Rt: 4.341 min. $^1$H-NMR ($CDCl_3$): δ 8.27 (s, 1H), 5.20 (s, 2H), 2.57 (s, 3H), 2.46 (s, 3H), 2.25 (s, 3H), 2.10 (s, 3H).

*Step 3*: (3,5-Dimethyl-4-methylsulfanyl-pyridin-2-yl)-methanol:

The title compound was obtained by deacetylation of acetic acid 3,5-dimethyl-4-methylsulfanyl-pyridin-2-ylmethyl ester according to the general procedure 2.3. HPLC Rt: 3.921 min.

*Step 4:* 2-Bromomethyl-3,5-dimethyl-4-methylsulfanyl-pyridine:

The compound was obtained from (3,5-dimethyl-4-methylsulfanyl-pyridin-2-yl)-methanol according to the general procedure 2.5. HPLC Rt: 4.905 min. $^1$HNMR ($CDCl_3$): δ 8.26 (s, 1H), 4.59 (s, 2H), 2.62 (s, 3H), 2.47 (s, 3H), 2.27 (s, 3H), 2.10 (s, 3H). m/z (%): 246.13 (M+1, 96%), 248.09 (M+3, 100%).

*Step 5:* 6-Chloro-9-(3,5-dimethyl-4-methylsulfanyl-pyridin-2-ylmethyl)-9H-purin-2-ylamine

The title compound was obtained by alkylation of 2-amino-6-chloropurine with 2-bromomethyl-3,5-dimethyl-4-methylsulfanyl-pyridine according to the general procedure 1.1. HPLC Rt: 4.611 min. $^1$H-NMR ($CDCl_3$): δ 8.24 (s, 1H), 7.87 (s, 1H), 5.36 (s, 2H), 5.00 (s, 2H), 2.61 (s, 3H), 2.47 (s, 3H), 2.26 (s, 3H).

**Example 180: 6-chloro-9-(7-chloro-benzothiazol-2-ylmethyl)-9H-purin-2-ylamine**

*Step 1:* (2,3-dichloro-phenyl)-acetamide

A solution of 2,3-dichloroaniline (5.00g, 30.86 mmol) in pyridine (20 ml) was cooled to 0˚C and treated with AcCl (4.85 g, 62 mmol). The reaction mixture was stirred at r.t. for 1 h and then concentrated under reduced pressure. The residue was dissolved in ethyl acetate, washed with 1% aqueous hydrochloric acid, water and brine, and dried over $MgSO_4$. The solution was concentrated and recrystallized from EtOAc/hexane to give (2,3-dichloro-phenyl)-acetamide (4.50 g, 22 mmol). HPLC Rt: 5.52 min. $^1$H-NMR ($CDCl_3$): δ 8.35 (br. s, 1H), 7.7 (1H), 7.24 (1H), 7.23 (1H), 2.28 (s, 3H).

*Step 2*: (2,3-dichloro-phenyl)-thioacetamide

A solution of (2,3-dichloro-phenyl)-acetamide (4.50 g, 22 mmol,) in toluene (50 ml) was treated at r.t. with $P_2S_5$ (9.80 g, 22 mmol). The reaction mixture was heated to 90 ˚C for 1.5 h, cooled to r.t., and filtered. The solid was washed with ether and the washings were combined with the filtrate. The combined solutions were extracted twice with 10% aq. NaOH. The combined aqueous extracts were acidified at 0˚C with HCl. The precipitate was collected and recrystallized from ethyl acetate/hexane to afford (2,3-dichloro-phenyl)-thioacetamide (3.40 g, 16 mmol). HPLC Rt: 5.91 min. $^1$H-NMR ($CDCl_3$):δ 8.80 (br. s, 1H, NH), 8.5 (d, 1H), 7.42 (d, 1H), 7.30 (t, 1H), 2.82 (s, 3H).

*Step 3:* 7-Chloro-2-methyl-benzothiazole

A solution of (2,3-dichloro-phenyl)-thioacetamide (3.4 g, 15 mmol) in N-methyl-2-pyrrolidinone (25 ml) was treated with NaH (60% oil suspension, 0.74 g, 19 mmol) at r.t. The reaction mixture was heated to 150˚C for 30 min. Work-up (EtOAc), drying (brine, $MgSO_4$), evaporation, and purification by flash chromatography afforded the title compound (2.4 g, 13 mmol). HPLC Rt: 6.65 min. $^1$H-NMR (CDCl3): δ 7.87 (d, 1H, J = 7.9 Hz, ph-H), 7.44 (t, 1H, J = 7.9 Hz, ph-H), 7.35 (d, 1H, J = 8.0 Hz, ph-H), 2.87 (s, 3H, $CH_3$).

*Step 4*: 2-Bromomethyl-7-chloro-benzothiazole

A mixture of 7-chloro-2-methyl-benzothiazole (1.00 g, 5.45 mmol), N-bromosuccinimide (1.26 g, 7.08 mmol), benzoyl peroxide (0.1 g), and $CCl_4$ (10 mL was heated to reflux under irradiation of a UV lamp for 14 h. The reaction mixture was cooled and filtered to remove the succinimide formed in the reaction, and the filtrate was evaporated to dryness. The resulting solid was purified by flash chromatography to give the title lamp (400 mg, 1.5 mmol). $^1$H-NMR ($CDCl_3$): δ 7.94 (d, 1H), 7.47 (t, 1H), 7.42 (d, 1H), 4.82 (s, 2H, $CH_2$). HPLC Rt: 7.19 min.

*Step 5:* 6-Chloro-9-(7-chloro-benzothiazol-2-ylmethyl)-9H-purin-2-ylamine

A mixture of 2-bromomethyl-7-chloro-benzothiazole (60 mg, 0.2286 mmol), 2-amino-6-chloropurine (32 mg, 0.19 mmol), $Cs_2CO_3$ (67.86 mg, 0.208 mmol), and DMF (2 ml) was heated to 40°C for 1 h. The reaction was cooled to r.t. and the solvent was removed on a rotary evaporator. The resulting solid was purified by preparative TLC to give the title compound (50 mg, 0.14 mmol). HPLC Rt: 5.81 min. $^1$H- NMR ($CDCl_3$): δ 8.0 (s, 1H), 7.95 (d, 1H), 7.48 (t, 1H), 7.43 (d, 1H), 5.69 (s, 2H), 5.17 (s, 2H).

**Example 181: 6-Chloro-9-(3,4,5-trimethyl-pyridin-2-ylmethyl)-9H-purin-2-ylamine**

*Step 1*: 2,3,5-Collidine-N-oxide

See Example 1, Method 1, step 1.

*Step 2:* 4-Bromo-2,3,5-collidine-N-oxide

See Example 1, Method 1, step 2.

*Step 3*: 2,3,4,5-Tetramethyl-pyridine 1-oxide

4-Bromo-2,3,5-trimethyl-pyridine 1-oxide (2g, 9.2 mmole) and catalytic tetrakis(triphenylphosphino)-palladium (80 mg, 4% by wt) in 20 mL of dry THF before treating with trimethylaluminum (2M in toluene, 15.2 mmole) under nitrogen. The solution was heated to reflux for 3 h, diluted with toluene (20 mL) before quenching the reaction with 4 mL of methanol followed by ammonium chloride (15 mmole). The mixture was refluxed for 2 h and filtered, while hot, on Celite. See J. Med. Chem. 1999, 42(12), 2064-2086. HPLC Rt: 4.183 min. $^1$H-NMR ($CDCl_3$): δ 8.05 (s, 1H), 2.55 (s, 3H), 2.27 (s, 3H), 2.22 (s, 3H), 2.20 (s, 3H).

*Step 4:* Acetic acid 3,4,5-trimethyl-pyridin-2-ylmethyl ester

The compound was obtained by dissolving in acetic anhydride before bringing the reaction to reflux for 0.5 h as described in the general procedure 2.2. Quenching the reaction with water and extracting with chloroform yielded the titled product. HPLC Rt: 3.843 min. $^1$H-NMR ($CDCl_3$): δ 8.20 (s, 1H), 5.22 (s, 2H), 2.26 (s, 3H), 2.25 (s, 3H), 2.21 (s, 3H), 2.11 (s, 3H).

*Step 5:* (3,4,5-Trimethyl-pyridin-2-yl)-methanol

The compound was obtained by hydrolysis of acetic acid 3,4,5-trimethyl-pyridin-2-ylmethyl ester in MeOH and $K_2CO_3$ at 50°C for 0.5 h as described in the general procedure 2.3. After removing MeOH, the residue was dissolved in water and extracted with chloroform. HPLC Rt: 3.405 min. $^1$H-NMR ($CDCl_3$): δ 8.18 (s, 1H), 5.00 (s, 1H), 4.67 (s, 2H), 2.28 (s, 3H), 2.23 (s, 3H), 2.12 (s, 3H).

*Step 6:* 2-bromomethyl-3,4,5-trimethyl-pyridine

The compound was obtained from reacting (3,4,5-trimethyl-pyridin-2-yl)-methanol with triphenyl phosphine and carbon tetrabromide in dichloromethane as described in the general procedure 2.5. HPLC Rt: 3.979 min. $^1$H-NMR ($CDCl_3$): δ 8.18 (s, 1H), 4.63 (s, 2H), 2.35 (s, 3H), 2.48 (s, 3H), 2.24 (s, 3H).

*Step 7*: 6-Chloro-9-(3,4,5-trimethyl-pyridin-2-ylmethyl)-9H-purin-2-ylamine

The compound was obtained by reacting 2-bromomethyl-3,4,5-trimethyl-pyridine with 6-chloro-9H-purin-2-ylamine in the presence of $K_2CO_3$ at 50 °C for 0.5 h as described in the general procedure 1.1. HPLC Rt: 3.903 min. $^1H$-NMR ($CDCl_3$): δ 8.18 (s, 1H), 7.84 (s, 1H), 5.38 (s, 2H), 5.08 (s, 2H), 2.29 (s, 3H), 2.27 (s, 3H), 2.22 (s, 3H).

**Example 182: 6-Bromo-9-(3,4,5-trimethyl-pyridin-2-ylmethyl)-9H-purin-2-ylamine**

The compound was obtained by reacting 2-bromomethyl-3,4,5-trimethyl-pyridine (see example 117) with 6-bromo-9H-purin-2-ylamine in the presence of $K_2CO_3$ in DMF for 0.5 h at 50 °C as described in the general procedure 1.1. HPLC Rt6: 4.045 min. $^1H$-NMR ($CDCl_3$): δ 8.18 (s, 1H), 7.85 (s, 1H), 5.37 (s, 2H), 5.10 (s, 2H), 2.29 (s, 3H), 2.27 (s, 3H), 2.22 (s, 3H).

**Example 183: 6-Bromo-9-(3,4,5-trimethyl-1-oxy-pyridin-2-ylmethyl)-9H-purin-2-ylamine**

The compound was obtained by the oxidation of 6-bromo-9-(3,4,5-trimethyl-pyridin-2-ylmethyl)-9H-purin-2-ylamine with m-CPBA in dichloromethane as described in the general procedure 2.1. HPLC Rt: 5.611 min. $^1H$-NMR ($CDCl_3$): δ 9.13 (s, 1H), 8.08 (s, 1H), 5.91 (s, 2H), 2.70 (s, 3H), 2.27 (s, 3H), 2.22 (s, 3H).

BIOLOGY EXAMPLES

The biological activites of selected aminopurines were determined using four assays: the inhibition of binding of biotinylated geldamamycin (biotin-GM) to rHSP90, the lysate binding ability, the HER2 degradation ability and the cytotoxity measurement. These assays have been described in Examples A, B, C and D in the previous sections. The biological activites are summarized in Table 6.

**TABLE 6. Biological Activities of Selected Aminopurines of Formula II, Where $R_3$=H**

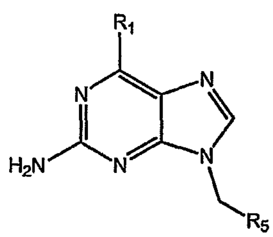

II

| S.No | Ex. # | $R^5$ | $R^1$ | Recombinent Binding assay (µM) | Lysate binding (µM) | HER2 $IC_{50}$ (µM) | MTS $IC_{50}$ (µM) |
|---|---|---|---|---|---|---|---|
| 1 | 51 | 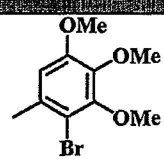 | Cl | 0.7 | 0.025 | 0.055 | 0.2 |
| 2 | 98 | 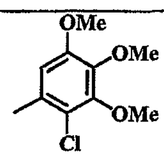 | Cl | 0.7 | 0.03 | 0.025 | 0.1 |

| S.No | Ex. # | $R^5$ | $R^1$ | Recombinent Binding assay (µM) | Lysate binding (µM) | HER2 $IC_{50}$ (µM) | MTS $IC_{50}$ (µM) |
|---|---|---|---|---|---|---|---|
| 11 | 18 | 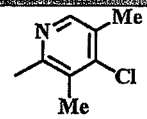 | Cl | ND | ND | 0.055 | 0.1 |
| 12 | 1 | 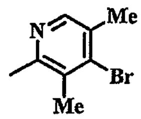 | Cl | ND | ND | 0.015 | 0.02 |
| 13 | 4 | 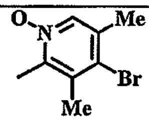 | Cl | ND | ND | 0.020 | 0.13 |
| 14 | 24 | 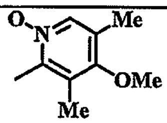 | Br | ND | ND | 0.060 | 0.4 |
| 15 | 19 | 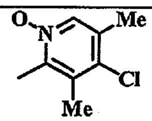 | Cl | ND | ND | 0.080 | 0.4 |
| 16 | 5 | 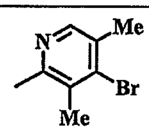 | Br | ND | 0.008 | 0.014 | 0.02 |
| 17 | 6 | 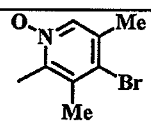 | Br | ND | ND | 0.060 | 0.05 |
| 18 | 111 | 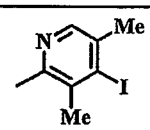 | Cl | ND | 0.003 | 0.009 | 0.02 |
| 19 | 115 | 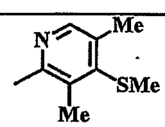 | Cl | ND | ND | 0.060 | ND |

| S.No | Ex. # | $R^5$ | $R^1$ | Recombinent Binding assay (µM) | Lysate binding (µM) | HER2 $IC_{50}$ (µM) | MTS $IC_{50}$ (µM) |
|---|---|---|---|---|---|---|---|
| 20 | 1.11 | 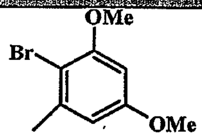 | Cl | ND | ND | 0.3 | ND |
| 21 | 1.12 | 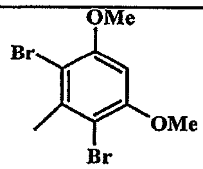 | Cl | ND | ND | 0.75 | ND |
| 22 | 1.13 | 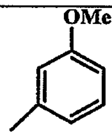 | Cl | ND | ND | 7.0 | ND |
| 23 | 1.14 | 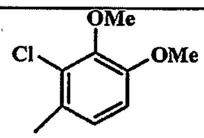 | Cl | ND | ND | 1.9 | ND |
| 24 | 1.15 | 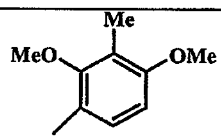 | Cl | ND | ND | 5.0 | ND |
| 25 | 1.18 | 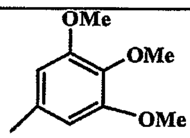 | Cl | ND | ND | 2.8 | ND |

ND = not determined

### III. PREPARATION OF PYRAZOLOPYRIMIDINES (FORMULA III)

#### A. Materials and Methods

The chemical reagents used to create the novel products of the invention below are all available commercially, *e.g.*, from Aldrich Chemical Co., Milwaukee, WI, USA. Otherwise their preparation is facile and known to one of ordinary skill in the art, or it is referenced or described herein.

The final compounds were usually purified by preparative TLC (silica gel 60 A, Whatman Partisil PK6F) or flash chromatography (silica gel 60 A, EMD Chemicals) using EtOAc/hexane or $MeOH/CH_2Cl_2$ as eluents. Rfs were measured using silica gel TLC plates (silica gel 60 A, EMD Chemicals). Analytical HPLC chromatograms were obtained using a C18 column (Agilent Zorbax 300SB-C18; 5 microns; 4.6 mm x 150 mm). A gradient was applied between solvent A (0.1 % TFA in $H_2O$) and solvent B (0.5% TFA in $CH_3CN$) increasing the proportion of A linearly from 5% (t=0) to 100% (t=7.00 min), with a constant flow rate of 1 mL/min. The samples were diluted to typically 0.1-1 mg/mL in MeOH or $CH_3CN$ and the injection volumes were typically 10 µL. The column was not heated, and UV detection was effected at 254 nm. $^1H$-NMR spectra were recorded on a Bruker Avance 400 MHz spectrometer.

The chemical names were generated using the Beilstein Autonom 2.1 software.

## B. General Procedures

### 1. General procedures to prepare and manipulate the pyrazolo[3,4-d]pyrimidine ring

General procedure 1.1: Alkylation of pyrazolo[3,4-d]pyrimidines at N-1

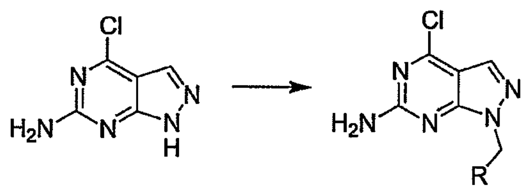

4-Chloro-1H-pyrazolo[3,4-d]pyrimidin-6-ylamine was prepared as described in Seela, F.; Stecker, H. Helv. Chim. Acta 1986, 69, 1602-1613. A suspension of the 4-Chloro-1H-pyrazolo[3,4-d]pyrimidin-6-ylamine (1 mmol), benzyl halide (1 mmol) and $K_2CO_3$ (1-3 mmol) in dry DMF (5 mL) was stirred at 22-70 ˚C for 0.5-16 h. Work-up (EtOAc) and purification by preparative TLC or flash chromatography (EtOAc/hexane or MeOH/$CH_2Cl_2$) yielded the pure N-1 alkylated product.

General procedure 1.2: Preparation of 3-alkyl pyrazolo[3,4-d]pyrimidines

*Step 1:* 1-(2-Amino-4,6-dichloro-pyrimidin-5-yl)-ethanol

A fine suspension of 2-amino-4,6-dichloro-pyrimidine-5-carbaldehyde (3.0 g, 15 mmol); (Seela, F.; Stecker, H. Helv. Chim. Acta 1986, 69, 1602) in THF was cooled to - 78 ˚C. A 3M solution of MeMgBr in THF (25 mL, 75 mmol, 5 equiv.) was added over 3 h, keeping the internal temperature at -78 ˚C. The mixture was stirred for a further 0.5 h, quenched with 100 ml $H_2O$ and neutralized with aw. HCl. Extraction (EtOAc) gave 1-(2-amino-4,6-dichloro-pyrimidin-5-yl)-ethanol as a pale yellow solid (2.5 g, 76%) which was used without further purification.

*Step 2:* 1-(2-Amino-4,6-dichloro-pyrimidin-5-yl)-ethanone

1-(2-Amino-4,6-dichloro-pyrimidin-5-yl)-ethanol (2.0 g, 9.6 mmol) was treated with $MnO_2$ (20 g, 229 mmol, 24 equiv.) in 1,2-dichloroethane for 16 h at 70˚C. Filtration over celite and concentration gave 1-(2-Amino-4,6-dichloro-pyrimidin-5-yl)-ethanone as a pale orange solid (1.4 g, 6.7 mmol, 71%), which was used without further purification.

*Step 3:* 4-Chloro-3-methyl-1H-pyrazolo[3,4-d]pyrimidin-6-ylamine

1-(2-Amino-4,6-dichloro-pyrimidin-5-yl)-ethanone (200 mg, 0.97 mmol) was dissolved in $CH_2Cl_2$ and treated with anhydrous hydrazine (31 mg, 0.97 mmol, 1 equiv.) at r.t. overnight. The precipitate was collected by filtration, washed with $CH_2Cl_2$, dissolved in DMSO (0.5 mL), and partitioned between EtOAc (100 mL) and water (25 mL). The organic layer was dried (brine, $Na_2SO_4$) and concentrated to afford the title compound as a white solid (95 mg, 0.52 mmol, 53%).

### 2. General procedures to manipulate the pyridine ring

General procedure 2.1: Preparation of pyridine N-oxides

A solution of the pyridine derivative (1.0 mmol) in dichloromethane or chloroform (5 mL) was cooled by means of an ice-bath, treated with m-CPBA (1.1 to 3 mmol) in three portions, and allowed to warm to r.t. The mixture was extracted with dichloromethane and washed with aqueous NaOH, followed by water. Drying ($Na_2SO_4$) and concentration afforded the pyridine N-oxide.

General procedure 2.2: Preparation of 2-(acetoxymethyl)-pyridines

A solution of the 2-methyl pyridine N-oxide (1.0 mmol) in acetic anhydride (5 mL) was heated to reflux for 0.5

h. Work-up (EtOAc), drying ($MgSO_4$), evaporation and purification by preparative TLC or flash chromatography afforded the 2-(acetoxymethyl) pyridine.

General procedure 2.3: Preparation of2-(hydroxymethyl)-pyridines

A suspension of 2-acetoxymethyl-pyridine derivative and solid $K_2CO_3$ in methanol was heated to 50 °C for 5-30 min. Evaporation, work-up (EtOAc), and drying ($MgSO_4$) afforded the 2-hydroxymethyl pyridine.

General procedure 2.4: Preparation of 2-(bromomethyl)-pyridines

A solution of 2-(hydroxymethyl)-pyridine (1.0 mmol) and triphenyl phosphine (1.2 mmol) in dichloromethane or chloroform (5 mL) was cooled to 0°C. A solution of $CBr_4$ (1.5 mmol) in dichloromethane or chloroform was added dopwise, and the resulting mixture was stirred at 0°C for 0.5-1 h. Work-up followed and purification by flash chromatography afforded the 2-(bromomethyl)-pyridine.

General procedure 2.5: Preparation of 2-chloropyridines

A suspension of 2-(hydroxymethyl)-pyridine (10 g) in $POCl_3$ (30 mL) was stirred at 110 °C for 1.5 h. The resulting viscous oil was cooled to r.t. and poured onto ice water (500 g). The pH was adjusted to 10 with solid KOH. Work-up ($CHCl_3$), drying ($MgSO_4$) and evaporation gave the 2-(chloromethyl)-pyridine, usually as a purple oil or solid, which was used without purification.

General procedure 2.6: Preparation of pyridinium salts

A solution of pyridine was heated in MeOH until it dissolved. A methanolic solution of acid (1.0 equiv of *e.g.* HCl, MeOH) was added, and the solvent was evaporated to give the pyridinium salt.

**3. General Procedure to manipulate benzene rings**

General procedure 3.1: Halogenation of benzene rings.

Variant 1: A solution of the aromatic compound in MeOH/THF/acetate buffer (1N in each AcOH and AcONa) was treated with $Br_2$ (1.3 equiv) at r.t. for 5 min. The excess bromine and solvent were removed on a rotary evaporator. Work-up ($CHCl_3$) and flash chromatography afforded the desired bromobenzene.

Variant 2: A solution of the aromatic compound (7 mmol) and n-halosuccinimide (NCS, NBS, or NIS, 1.06 equiv) in acetic acid (40 mL) was heated to 40-90°C for 0.3-1 h. Evaporation, work-up (EtOAc) and flash chromatography afforded the desired halogenated benzene.

**C. Preparation of intermediates**

Example 184. 2-Chloro-1-chloromethyl-3,4,5-trimethoxy-benzene

The title compound was obtained by chlorination of 5-chloromethyl-1,2,3-trimethoxy-benzene with NCS according to the general procedure 3.1. $^1$H-NMR ($CDCl_3$) δ 6.82 (s, 1H), 4.70 (s, 1H), 3.93 (s, 3H), 3.90 (s, 3H) 3.87 (s, 3H).

**Example 185. 2-Chloro-6-chloromethyl-4-methoxy-3,5-dimethyl-pyridine**

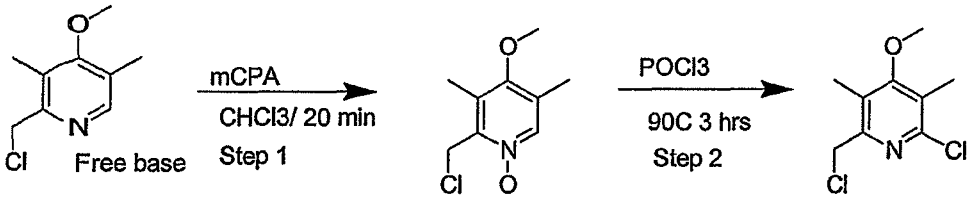

*Step* 1: 2-Chloromethyl-4-methoxy-3,5-dimethylpyridine-1-oxide

The title compound was obtained by oxidation of 2-chloromethyl-4-methoxy-3,5-dimethyl-pyridine according to the general procedure 2.1. R.t.: 4.46 min. $^1$H-NMR ($CDCl_3$): δ 8.05 (s, 1H), 4.93 (s, 2H), 3.77 (s, 3H), 2.37 (s, 3H), 2.24 (s, 3H).

*Step 2:* 2-Chloro-6-chloromethyl-4-methoxy-3,5-dimethylpyridine

The title compound was obtained by treating 2-chloromethyl-4-methoxy-3,5-dimethylpyridine-1-oxide with $POCl_3$ according to the general procedure 2.5. R.t.: 6.757 min. $^1$H-NMR ($CDCl_3$): δ 4.64 (s, 2H), 3.79 (s, 3H), 2.35 (s, 3H), 2.33 (s, 3H).

**Example 186. 4-Chloro-2-chloromethyl-3,5-dimethyl-pyridine**

The title compound was obtained by treating 2-chloromethyl-3,5-dimethyl-pyridin-4-ol (Tarbit, et al. WO 99/10326) with $POCl_3$ according in the same manner as the general procedure 2.5 (74% yield). R.t.: 5.54 min. $^1$H-NMR ($CDCl_3$): 8.24 (s, 1H), 4.71 (s, 2H), 2.48 (s, 3H), 2.36 (s, 3H).

**Example 187. 4-Bromo-2-bromomethyl-3,5-dimethyl-pyridine**

4-Bromo-2-bromomethyl-3,5-dimethyl-pyridine was prepared by any of the following three methods:

Method 1

*Step 1:* 2,3,5-Collidine-N-oxide

2,3,5-Collidine-N-oxide was obtained by oxidation of 2,3,5-collidine according to the general procedure 2.1 in 70% yield. R.t.: 3.96 min. $^1$H-NMR ($CDCl_3$): δ 8.03 (s, 1H), 6.90 (s, 1H), 2.47 (s, 3H), 2.31 (s, 3H), 2.24 (s, 3H). m/z (%) 138.2 (M+1, 100%). Rf(20% MeOH/EtOAc): 0.35.

*Step 2:* 4-Bromo-2,3,5-collidine-N-oxide

2,3,5-collidine-N-oxide (1.3 g, 10 mmol) and $K_2CO_3$ (2.9 g, 20 mmol) were suspended in 10 mL of $CCl_4$. Bromine (1 mL, 20 mmol) was added dropwise, and the reaction mixture was heated to reflux for 2 h. Work-up (EtOAc) and flash chromatography (10% MeOH/EtOAc) afforded the title compound as a solid (1.05 g, 51% yield). R.t.: 5.24 min. $^1$H-NMR ($CDCl_3$): δ 8.06 (s, 1H), 2.56 (s, 3H), 2.43 (s, 3H), 2.31 (s, 3H). m/z (%) 216.2 (M+1, 100%), 218.2 (M+3, 100%). Rf (20% MeOH/EtOAc): 0.45.

*Step 3:* Acetic acid 4-bromo-3,5-dimethyl-pyridin-2-yl methyl ester

4-Bromo-2,3,5-collidine-N-oxide (0.25g, 11 mmol) was dissolved in acetic anhydride (5 mL) and the solution was heated to reflux for 30 min. Work-up and flash chromatography (50% Hexane/EtOAc) afforded the title compound (0.27 g, 96% yield). Rf(50% Hexane/EtOAc): 0.70. R.t.: 4.76 min. $^1$H-NMR ($CDCl_3$): δ 8.26 (s, 1H), 5.27 (s, 2H), 2.46 (s, 3H), 2.41 (s, 3H), 2.14 (s, 3H).

*Step* 4: 4-Bromo-3,5-dimethyl-pyridin-2-yl methanol

A suspension of acetic acid 4-bromo-3,5-dimethyl-pyridin-2-yl methyl ester (0.26 g, 1.0 mmol) and $K_2CO_3$ (excess) in MeOH (5 mL) was heated to 50 ˚C for 15 min. Work-up ($CHCl_3$), evaporation, and filtration through a silica gel pad (eluent: 100% EtOAc) gave the title compound as a white solid (0.19 g, 88% yield). Rf(50% Hexane/EtOAc): 0.5. R.t.: 3.80 min. $^1$H-NMR ($CDCl_3$): δ 8.23 (s, 1H), 4.70 (s, 2H), 2.46 (s, 3H), 2.30 (s, 3H).

*Step 5:* 4-Bromo-2-bromomethyl-3,5-dimethyl-pyridine

The title compound was obtained from 4-bromo-3,5-dimethyl-pyridin-2-yl methanol according to the general procedure 2.4. R.t.: 6.32 min. $^1$H-NMR ($CDCl_3$): δ 8.22 (s, 1H), 4.63 (s, 2H), 2.52 (s, 3H), 2.40 (s, 3H).

Method 2:

*Step 1*: 2-chloromethyl-3,5-dimetllyl-pyridin-4-ol

The title compound was obtained by heating 2-chloromethyl-4-methoxy-3,5-dimethyl-pyridine hydrochloride in toluene as described in the patent by Tarbit, et al. WO 99/10326.

*Step 2:* 4-bromo-2-chloromethyl-3,5-dimethyl pyridine

A mixture of 2-chloromethyl-3,5-dimethyl-pyridin-4-ol (8.2 g, 47.8 mmol) and $POBr_3$ (60g, 209 mmol) was stirred at 130˚C for 3 h. The resulting viscous oil was cooled to r.t. and poured onto ice water. The pH was adjusted to 10 with solid KOH. Work-up ($CHCl_3$), drying ($MgSO_4$) and evaporation afforded the title compound as a purple solid (8.7 g, 78% yield) which was used without purification. R.t.: 6.03 min. $^1$H-NMR ($CDCl_3$): 8.20 (s, 1H), 4.62 (s, 2H), 2.50 (s, 3H), 2.38 (s, 3H).

Method 3:

4-bromo-2-chloromethyl-3,5-dimethyl pyridine

A suspension of 2-chloromethyl-4-methoxy-3,5-dimethyl-pyridine (3.24 g, 14.6 mmol) in $PBr_3$ (8.0 ml, 85.1 mmol, 5.8 equiv.) was heated to 80˚C under nitrogen. A catalytic amount of DMF (0.50 ml, 6.4 mmol, 0.44 equiv.) was added, whereupon the suspension rapidly turned into an orange solution. After 40 min., the reaction was still incomplete as judged by HPLC. The temperature was raised to 110 ˚C and the reaction was prolonged for 30 min, at which point it was complete. The mixture was poured over ice, made basic with conc. aq. $NH_4OH$ and extracted into EtOAc. Washing with water, drying (brine, $MgSO_4$) and concentration gave the title compound as a pink solid (1.51 g, 44%) containing 10% of an impurity by $^1$H-NMR. The crude was used without further purification. $^1$H-NMR ($CDCl_3$) $\delta$ 8.19 (s, 1H), 4.59 (s, 2H), 2.48 (s, 3H), 2.37 (s, 3H).

**D. <u>Preparation of final compounds</u>**

**Example 188. 4-Chloro-1-(3,4,5-trimethoxy-benzyl)-1H-pyrazolo[3,4-d]pyrimidin-6-ylamine**

The title compound was obtained by alkylation of 4-chloro-1H-pyrazolo[3,4-d]pyrimidin-6-ylamine (See, F. Seela, Heterocycles 1985, 23, 2521; F. Seela, Helv. Chim. Acta 1986, 69, 1602; R. O. Dempcy, PCT Publication No. WO 03/022859) with 5-chloromethyl-1,2,3-trimethoxy-benzene according to the general procedure 1.1. R.t. 5.68 min. $^1$H-NMR ($CDCl_3$) $\delta$ 7.93 (s, 1H), 6.59 (s, 2H), 5.37 (br. s., 4H), 3.84 (s, 6H), 3.82 (s, 3H).

**Example 189. 4-Chloro-1-(2-chloro-3,4,5-trimethoxy-benzyl)-1H-pyrazolo[3,4-d]pyrimidin-6-ylamine**

The title compound was obtained by alkylation of 4-chloro-1H-pyrazolo[3,4-d]pyrimidin-6-ylamine with 2-chloro-1-chloromethyl-3,4,5-trimethoxy-benzene according to the general procedure 1.1. R.t. 6.44 min. $^1$H-NMR ($CDCl_3$) $\delta$ 7.95 (s, 1H), 6.36 (s, 1H), 5.51 (s, 2H), 5.24 (br. s, 2H), 3.90 (s, 3H), 3.86 (s, 3H), 3.70 (s, 3H).

**Example 190. 4-Chloro-1-(4-methoxy-3,5-dimethyl-pyridin-2-ylmethyl)-1H-pyrazolo [3,4-d]pyrimidin-6-ylamine**

A mixture of 4-chloro-1H-pyrazolo[3,4-d]pyrimidin-6-ylamine (1.76 g), 2-chloromethyl-4-methoxy-3,5-dimethyl-pyridine hydrochloride (3.70 g), $K_2CO_3$ (5.17 g), and DMF (20 ml) was heated to 80 ˚C for 30 min, diluted with EtOAc, washed with water and brine, concentrated, and purified by flash chromatography to give the title compound as a white solid (0.57 g). R.t. 4.46 min. $^1$H-NMR ($CDCl_3$) $\delta$ 8.10 (s, 1H), 7.89 (s, 1H), 5.53 (2H), 5.24 (br. s, 2H), 3.74 (s, 3H), 2.27 (s, 3H), 2.22 (s, 3H).

**Example 191. 4-Chloro-1-(6-chloro-4-methoxy-3,5-dimethyl-pyridin-2-ylmethyl)-1H-pyrazolo[3,4-d]pyrimidin-6-ylamine**

A mixture of 4-chloro-1H-pyrazolo[3,4-d]pyrimidin-6-ylamine (124 mg), $Cs_2CO_3$ (392 mg) and crude 2-chloro-6-chloromethyl-4-methoxy-3,5-dimethyl-pyridine (200 mg) in DMF (2 ml) was heated to 80 ˚C for 1 h, diluted with EtOAc and washed with water. Concentration and purification by preparative TLC (EtOAc) gave the title compound. R.t. 6.43 min. $^1$H-NMR ($CDCl_3$) $\delta$ 7.86 (s, 1H), 5.48 (s, 2H), 5.37 (s, 2H), 3.71 (s, 3H), 2.27 (s, 3H), 2.15 (s, 3H).

**Example 192. 4-Chloro-1-(4-chloro-3,5-dimethyl-pyridin-2-ylmethyl)-1H-pyrazolo[3,4-d]pyrimidin-6-ylamine**

A mixture of 4-chloro-1H-pyrazolo[3,4-d]pyrimidin-6-ylamine (158 mg), crude 4-chloro-2-chloromethyl-3,5-dimethyl-pyridine (204 mg), $Cs_2CO_3$ (660 mg) and DMF was heated to 80 ˚C for 1.5 h, diluted with EtOAc and washed with water. The crude material was concentrated and suspended in MeOH/DCM. Filtration gave a 2:1 mixture of regioisomers which was further purified by preparative silica gel plate (EtoAc 100%). The major (less polar) isomer corresponded to the title compound. R.t. 5.45 min. $^1$H-NMR ($CDCl_3$) δ 8.22 (s, 1H), 7.90 (s, 1H), 5.57 (s, 2H), 5.28 (s, 2H), 2.43 (s, 3H), 2.31 (s, 3H).

**Example 193. 4-Chloro-1-(4-methoxy-3,5-dimethyl-1-oxy-pyridin-2-ylmethyl)-1H-pyrazolo[3,4-d]pyrimidin-6-ylamine**

A solution of 4-chloro-1-(4-methoxy-3,5-dimethyl-pyridin-2-ylmethyl)-1H-pyrazolo[3,4-d]pyrimidin-6-ylamine (50 mg) in $CH_2Cl_2$ (2 ml) was treated with m-CPBA (90 mg) for 10 min, diluted with $CH_2Cl_2$, washed with sat. aq. $NaHCO_3$, concentrated and re-crystallized from $CHCl_3$/MoOH to give the title compound as a white solid. R.t. 4.87 min. $^1$H-NMR (DMSO-$d_6$) δ 8.06 (s, 1H), 7.89 (s, 1H), 7.36 (s, 2H), 5.55 (s, 2H), 3.72 (s, 3H), 2.30 (s, 3H), 2.18 (s, 3H).

**Example 194. 4-Chloro-1-(3,4-dichloro-benzyl)-1H-pyrazolo[3,4-d]pyrimidin-6-ylamine**

The title compound was obtained by alkylation of 4-chloro-1H-pyrazolo[3,4-d]pyrimidin-6-ylamine with 4-bromomethyl-1,2-dichloro-benzene according to general procedure 1.1. R.t. 6.89 min. $^1$H-NMR ($CDCl_3$) δ 7.90 (s, 1H), 7.39-7.37 (m, 2H), 7.26 (dd, 1H), 5.37 (s, 2H), 5.20 (br. s, 2H).

**Example 195. 4-Chloro-1-(2,5-dimethoxy-benzyl)-1H-pyrazolo[3,4-d]pyrimidin-6-ylamine**

The title compound was obtained by alkylation of 4-chloro-1H-pyrazolo[3,4-d]pyrimidin-6-ylamine with 2-chloromethyl-1,4-dimethoxy-benzene according to general procedure 1.1. R.t. 6.06 min. $^1$H-NMR ($CDCl_3$) δ 7.94 (s, 1H), 6.85 (d, 1H), 6.75 (dd, 1H), 6.42 (dd, 1H), 5.48 (s, 2H), 5.24 (s, 2H), 3.82 (s, 3H), 3.70 (s, 3H).

**Example 196. 4-Chloro-1-(4,5-dimethoxy-2-nitro-benzyl)-1H-pyrazolo[3,4-d]pyrimidin-6-ylamine**

The title compound was obtained by alkylation of 4-chloro-1H-pyrazolo[3,4-d]pyrimidin-6-ylamine with 1-bromomethyl-4,5-dimethoxy-2-nitro-benzene according togeneral procedure 1.1. R.t 5.99 min. $^1$H-NMR (DMSO-$d_6$) δ 8.06 (s, 1H), 7.71 (s, 1H), 7.38 (br. s, 2H), 6.57 (s, 1H), 5.71 (s, 2H), 3.86 (s, 3H), 3.68 (s, 3H).

**Example 197. 1-(4-Bromo-3,5-dimethyl-pyridin-2-ylmethyl)-4-chloro-1H-pyrazolo[3,4-d]pyrimidin-6-ylamine**

The title compound was obtained by alkylation of 4-chloro-1H-pyrazolo[3,4-d]pyrimidin-6-ylamine with 4-bromo-2-chloromethyl-3,5-dimethyl-pyridine according to general procedure 1.1. R.t. 5.64 min. $^1$H-NMR ($CDCl_3$) δ 8.20 (s, 1H), 7.92 (s, 1H), 5.61 (s, 2H), 5.21 (br. s, 2H), 2.50 (s, 3H), 2.37 (s, 3H).

**Example 198. 1-(4-Bromo-3,5-dimethyl-1-oxy-pyridin-2-ylmethyl)-4-choro-1H-pyrazolo[3,4-d]pyrimidin-6-ylamine**

The title compound was obtained by oxidation of 1-(4-bromo-3,5-dimethyl-pyridin-2-ylmethyl)-4-chloro-1H-pyrazolo[3,4-d]pyrimidin-6-ylamine with m-CPBA according to general procedure 2.1. R.t. 5.57 min. $^1$H-NMR ($CDCl_3$) δ 8.23 (s, 1H), 7.90 (s, 1H), 7.38 (s, 2H), 5.64 (s, 2H), 2.50 (s, 3H), 2.30 (s, 3H).

**Example 199. 4-Chloro-1-(2,3,6-trifluoro-benzyl)-1H-pyrazolo[3,4-d]pyrimidin-6-ylamine**

The title compound was obtained by alkylation of 4-chloro-1H-pyrazolo[3,4-d]pyrimidin-6-ylamine with 2-bromomethyl-1,3,4-trifluoro-benzene according to general procedure 1.1. R.t. 7.12 min. $^1$H-NMR ($CDCl_3$) δ 7.89(s, 1H), 7.25-7.05 (m, 1H), 6.95-6.85 (m, 1H), 5.53 (s, 2H), 5.49 (br. s, 2H).

**Example 200. 1-(2-Amino-4,6-dichloro-pyrimidin-5-yl)-ethanol**

The title compound was obtained by treatment of 2-Amino-4-chloro-pyrimidine-5-carbaldehyde with MeMgBr according to general procedure 1.2. R.t. 4.19 min. $^1$H-NMR (DMSO-$d_6$) δ 7.38 (s, 1H), 5.18 (bs, 2H), 5.15 (m, 1H), 3.56

(d, 3H).

**Example 201. 1-(2-Amino-4,6-dichloro-pyrimidin-5-yl)-ethanone**

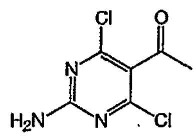

The title compound was obtained by treatment of 1-(2-Amino-4,6-dichloro-pyrimidin-5-yl)-ethanol with $MnO_2$ according to general procedure 1.2. R.t. 5.23 min. $^1$H-NMR (DMSO-$d_6$) δ 7.90 (s, 2H), 2.52 (s, 3H).

**Example 202. 4-Chloro-3-methyl-1H-pyrazolo[3,4-d]pyrimidin-6-ylamine**

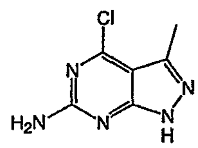

The title compound was obtained by treatment of 1-(2-Amino-4,6-dichloro-pyrimidin-5-yl)-ethanone with hydrazine according to general procedure 1.2. R.t. 4.61 min. $^1$H-NMR (DMSO-$d_6$) δ 11.82 (s, 1H), 8.16 (bs, 2H), 2.46 (s, 3H).

**Example 203. 4-Chloro-3-ethyl-1H-pyrazolo[3,4-d]pyrimidin-6-ylamine**

The title compound was obtained by treating 2-amino-4,6-dichloro-pyrimidine-5-carbaldehyde by sequentially with EtMgCl, $MnO_2$, and hydrazine according to general procedure 1.2. R.t. 4.55 min. $^1$H-NMR (DMSO-$d_6$) δ 12.84 (s, 1H), 7.07 (s, 2H), 2.85 (m, 2H), 1.27-1.23 (m, 3H).

**Example 204. 4-Chloro-3-isopropyl-1H-pyrazolo [3,4-d]pyrimidin-6-ylamine**

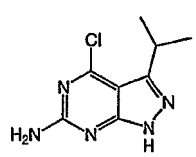

The title compound was obtained by treating 2-amino-4,6-dichloro-pyrimidine-5-carbaldehyde sequentially with i-PrMgCl, $MnO_2$ and hydrazine according to general procedure 1.2. R.t. 6.10 min. $^1$H-NMR (DMSO-$d_6$) δ 12.86 (s, 1H), 7.06 (s, 2H), 1.29 (d, 6H).

**Example 205. 4-Chloro-3-phenyl-1H-pyrazolo[3,4-d]pyrimidin-6-ylamine**

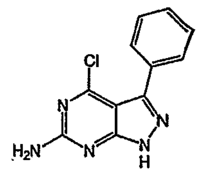

The title compound was obtained by treating 2-amino-4,6-dichloro-pyrimidine-5-carbaldehyde sequentially with PhMgCl, $MnO_2$ and hydrazine according to general procedure 1.2. R.t. 6.04 min. $^1$H-NMR (DMSO-$d_6$) δ 13.04 (s, 1H), 7.70 (m, 2H), 7.46 (m, 3H), 7.19 (bs, 2H).

**Example 206. 4-Chloro-1-(4-methoxy-3,5-dimethyl-pyridin-2-ylmethyl)-3-methyl-1H-pyrazolo[3,4-d]pyrimidin-6-ylamine**

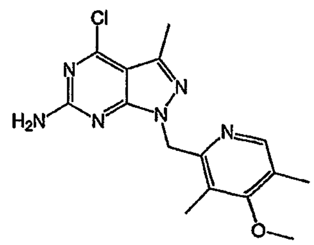

The title compound was obtained by alkylation of 4-chloro-3-methyl-1H-pyrazolo[3,4-d]pyrimidin-6-ylamine with 2-chloromethyl-4-methoxy-3,5-dimethylpyridine according to general procedure 1.1. R.t 6.72 min. $^1$H-NMR ($CDCl_3$) δ 8.20 (s, 1H), 5.47 (s, 2H), 5.26 (s, 2H), 3.76 (s, 2H), 2.58 (s, 3H), 2.30 (s, 3H), 2.23 (s, 3H).

**Example 207. 1-(4-Bromo-3,5-dimethyl-1-oxy-pyridin-2-ylmethyl)-4-chloro-3-methyl-1H-pyrazolo[3,4-d]pyrimidin-6-ylamine**

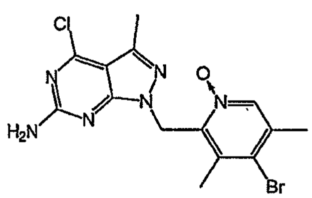

The title compound was obtained by alkylation of 4-chloro-3-methyl-1H-pyrazolo[3,4-d]pyrimidin-6-ylamine with 4-bromo-2-chloromethyl-3,5-dimethylpyridine 1-oxide according to general procedure 1.1. R.t. 5.90 min. $^1$H-NMR (DMSO-$d_6$) δ 8.25 (s, 1H), 7.29 (s, 2H), 5.53 (s, 2H), 2.45 (s, 3H), 2.36 (s, 3H), 2.28 (s, 3H).

**Example 208. 4-Chloro-1-(4-chloro-3,5-dimethyl-1-oxy-pyridin-2-ylmethyl)-3-methyl-1H-pyrazolo[3,4-d]pyrimidin-6-ylamine**

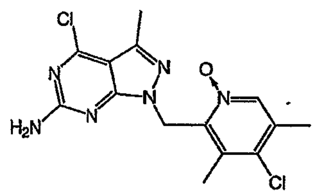

The title compound was obtained by alkylation of 4-chloro-3-methyl-1H-pyrazolo[3,4-d]pyrimidin-6-ylamine with 4-chloro-2-chloromethyl-3,5-dimethylpyridine 1-oxide according to general procedure 1.1. R.t. 5.90 min. $^{1}$H-NMR (DMSO-$d_6$) δ 8.25 (s, 1H), 7.30 (s, 2H), 5.54 (s, 2H), 2.45 (s, 3H), 2.36 (s, 3H), 2.28 (s, 3H).

**Example 209. 4-Chloro-1-(4-chloro-3,5-dimethyl-pyridin-2-ylmethyl)-3-methyl-1H-pyrazolo[3,4-d]pyrimidin-6-ylamine**

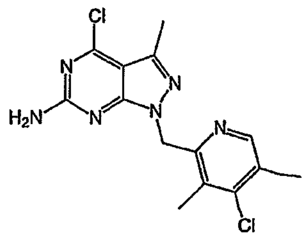

The title compound was obtained by alkylation of 4-chloro-3-methyl-1H-pyrazolo[3,4-d]pyrimidin-6-ylamine with 4-chloro-2-chloromethyl-3,5-dimethylpyridine according to general procedure 1.1. R.t. 5.63 min. $^{1}$H-NMR ($CDCl_3$) δ 8.23 (s, 1H), 5.51 (s, 2H), 5.28 (br.s 2H), 2.57 (s, 3H), 2.45 (s, 3H), 2.33 (s, 3H).

**Example 210. 4-Chloro-3-methyl-1-(3,4,5-trimethoxy-benzyl)-1H-pyrazolo[3,4-d]pyrimidin-6-ylamine**

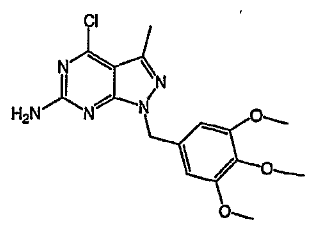

The title compound was obtained by alkylation of 4-chloro-3-methyl-1H-pyrazolo[3,4-d]pyrimidin-6-ylamine with 5-chloromethyl-1,2,3-trimethoxy-benzene according to general procedure 1.1. R.t. 6.72 min. $^{1}$H-NMR (DMSO-$d_6$) δ 7.30 (s, 2H), 6.57 (s, 2H), 5.22 (s, 2H), 3.71 (s, 6H), 3.62 (s, 3H), 2.47(s, 3H), 2.29 (s, 3H).

**Example 211. 1-(4-Bromo-3,5-dimethyl-pyridin-2-ylmethyl)-4-chloro-3-methyl-1H-pyrazolo[3,4-d]pyrimidin-6-ylamine**

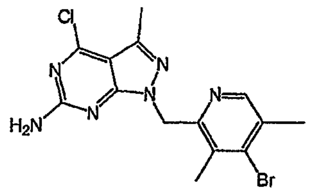

The title compound was obtained by alkylation of 4-chloro-3-methyl-1H-pyrazolo[3,4-d]pyrimidin-6-ylamine with 4-bromo-2-chloromethyl-3,5-dimethylpyridine according to general procedure 1.1. R.t. 5.90 min. $^1$H-NMR (DMSO-$d_6$) δ 8.15 (s, 1H), 7.22 (s, 1H), 5.46 (s, 2H), 2.42 (s, 6H), 2.30 (s, 3H).

**Example 212. 4-Chloro-3-ethyl-1-(4-methoxy-3,5-dimethyl-pyridin-2-ylmethyl)-1H-pyrazolo[3,4-d]pyrimidin-6-ylamine**

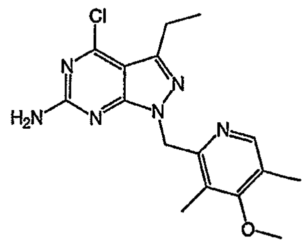

The title compound was obtained by alkylation of 4-chloro-3-ethyl-1H-pyrazolo[3,4-d]pyrimidin-6-ylamine with 2-chloromethyl-4-methoxy-3,5-dimethylpyridine according to general procedure 1.1. Rt. 6.02 min. $^1$H-NMR (DMSO) δ 8.04 (s,1H), 7.19 (br. s, 2H), 5.39 (s, 2H), 3.71(s, 3H), 2.87-2.81 (m, 2H), 2.22 (s, 3H), 2.16 (s, 3H), 1.21(m, 3H).

**Example 213. 4-Chloro-1-(4-chloro-3,5-dimethyl-pyridin-2-ylmethyl)-3-ethyl-1H-pyrazolo[3,4-d]pyrimidin-6-ylamine**

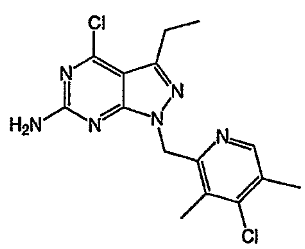

The title compound was obtained by alkylation of 4-chloro-3-ethyl-1H-pyrazolo[3,4-d]pyrimidin-6-ylamine with 4-chloro-2-chloromethyl-3,5-dimethylpyridine according to general procedure 1.1. R.t. 6.90 min. $^1$H-NMR (DMSO-$d_6$) δ 8.18 (s 1H), 7.21 (s, 1H), 5.47 (s, 2H), 2.87-2.81 (m, 2H), 2.39 (s, 3H), 2.27 (s, 3H), 1.21 (m, 3H).

**Example 214. 4-Chloro-3-isopropyl-1-(4-methoxy-3,5-dimethyl-pyridin-2-ylmethyl)-1H-pyrazolo[3,4-d]pyrimidin-6-ylamine**

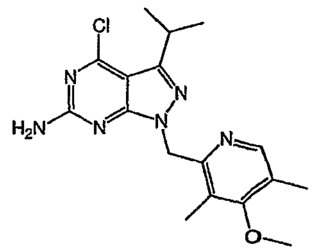

The title compound was obtained by alkylation of 4-chloro-3-isopropyl-1-(4-methoxy-3,5-dimethyl-pyridin-2-ylmethyl)-1H-pyrazolo[3,4-d]pyrimidin-6-ylamine with 2-chloromethyl-4-methoxy-3,5-dimethyl-pyridine according to the general procedure 1.1. R.t. 5.75 min. $^1$H-NMR (DMSO-$d_6$) δ 8.02 (s, 1H), 7.17 (br. s, 1H), 5.40 (s, 2H), 3.71 (s, 3H), 2.23 (s, 3H), 2.16 (s, 3H), 1.26 (d, 6H).

**Example 215. 4-Chloro-1-(4-methosy-3,5-dimethyl-pyridin-2-ylmethyl)-3-phenyl-1H-pyrazolo[3,4-d]pyrimidin-6-ylamine**

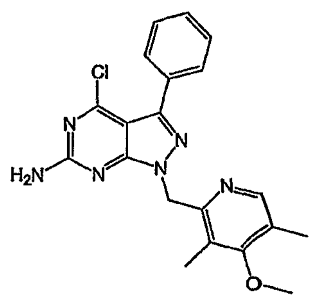

The title compound was obtained by alkylation of 4-chloro-3-phenyl-1H-pyrazolo[3,4-d]pyrimidin-6-ylamine with 2-chloromethyl-4-methoxy-3,5-dimethylpyridine according to general procedure 1.1. R.t 5.89 min. $^1$H-NMR (DMSO-$d_6$) δ 8.06 (s, 1H), 7.68-7.66 (m, 2H), 7.47-7.45 (m, 3H), 7.32 (br.s, 2H), 5.52 (s, 2H), 3.72 (s, 3H), 2.27 (s, 3H), 2.16 (s, 3H).

**Example 216. 4-Chloro-1-(4-chloro-3,5-dimethyl-pyridin-2-ylmethyl)-3-phenyl-1H-pyrazolo[3,4-d]pyrimidin-6-ylamine**

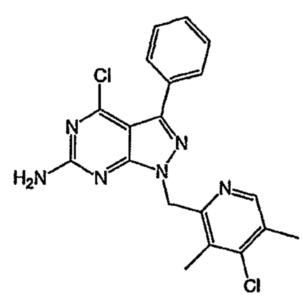

The title compound was obtained by alkylation of 4-chloro-3-phenyl-1H-pyrazolo[3,4-d]pyrimidin-6-ylamine with 2-chloromethyl-4-methoxy-3,5-dimethylpyridine according to general procedure 1.1. R.t. 6.80 min. $^1$H-NMR (DMSO-$d_6$) δ 8.20 (s, 1H), 7.67-7.65 (m, 2H), 7.47-7.45 (m, 3H), 7.34 (br.s, 2H), 5.61 (s, 2H), 2.43 (s, 3H), 2.27 (s, 3H).

**Example 217. 1-(4-Bromo-3,5-dimethyl-pyridin-2-ylmethyl)-4-chloro-3-phenyl-1H-pyrazolo [3,4-d]pyrimidin-6-ylamine**

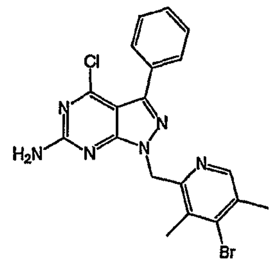

The title compound was obtained by alkylation of 4-chloro-3-phenyl-1H-pyrazolo[3,4-d]pyrimidin-6-ylamine with 4-bromo-2-chloromethyl-3,5-dimethylpyridine according to general procedure 1.1. R.t. 7.41 min. $^{1}$H-NMR (DMSO-$d_6$) δ 8.15 (s, 1H), 7.67 (m, 2H), 7.46 (m, 3H), 7.34 (br. s, 2H), 5.62 (s, 2H), 2.4 (s, 3H), 2.3 (s, 3H).

**Example 218. 4-Chloro-1-(4-chloro-3,5-dimethyl-1-oxy-pyridin-2-ylmethyl)-3-phenyl-1H-pyrazolo[3,4-d]pyrimidin-6-ylamine**

The title compound was obtained by alkylation of 4-chloro-3-phenyl-1H-pyrazolo[3,4-d]pyrimidin-6-ylamine with 4-chloro-2-chloromethyl-3,5-dimethylpyridine 1-oxide according to general procedure 1.1. R.t. 7.50 min. $^{1}$H-NMR (DMSO-$d_6$) δ 8.25 (s, 1H), 7.57 (s, 2H), 7.42 (m, 5H), 5.67 (s, 2H), 2.49 (s, 3H), 2.26 (s, 3H).

BIOLOGY EXAMPLES

The biological activites of selected pyrazolopyrimidines were determined using four assays: the inhibition of binding of biotinylated geldamamycin (biotin-GM) to rHSP90, the lysate binding ability, the HER2 degradation ability and the cytotoxity measurement. These assays have been described in Examples A, B, C and D in the previous sections. The biological activites are summarized in Table 7.

**TABLE 7. Biological Activities of Pyrazolopurimidine**

| S.No. | Ex # | Structure | Lysate binding (µM) | HER2 $IC_{50}$ (µM) | MTS $IC_{50}$ (µM) |
|---|---|---|---|---|---|
| 1 | 7 | 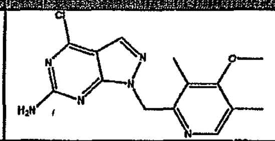 | 0.14 | 0.05 | 0.13 |
| 2 | 8 | 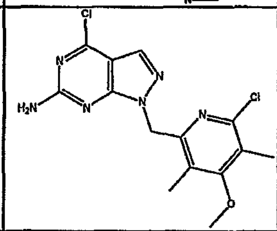 | ND | 0.14 | 0.5 |
| 3 | 9 | 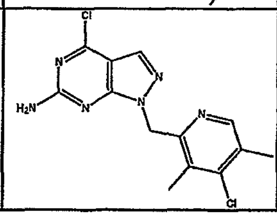 | ND | 0.09 | 0.3 |

| S/No | Ex # | Structure | Lysate binding (μM) | HER2 $IC_{50}$ (μM) | MTS $IC_{50}$ (μM) |
|---|---|---|---|---|---|
| 4 | 31 | 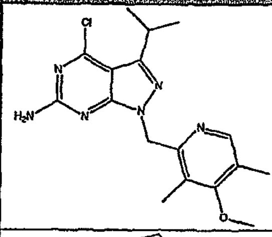 | ND | 0.05 | 0.3 |
| 5 | 32 | 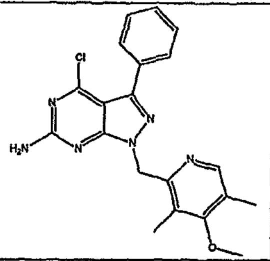 | ND | 0.04 | 0.08 |
| 6 | 33 | 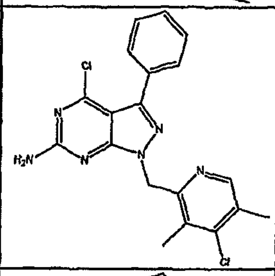 | ND | 0.16 | 0.6 |
| 7 | 34 | 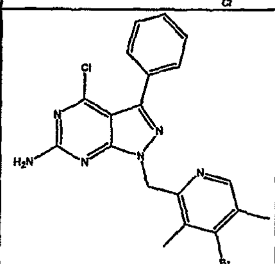 | ND | 0.12 | 1.0 |
| 8 | 30 | 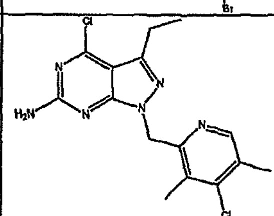 | ND | 0.11 | 1.0 |
| 9 | 29 | 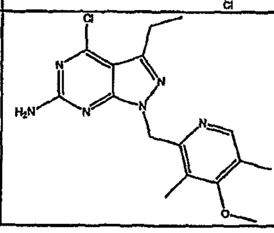 | 0.1 | 0.85 | 1.0 |

| S.No | Ex # | Structure | Lysate binding (μM) | HER2 $IC_{50}$ (μM) | MTS $IC_{50}$ (μM) |
|---|---|---|---|---|---|
| 10 | 35 | 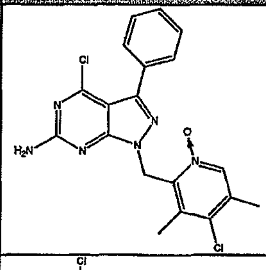 | 0.08 | 0.02 | 1.0 |
| 11 | 10 | 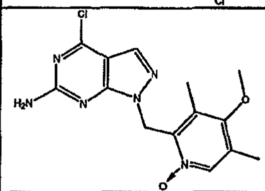 | 0.06 | 0.03 | 0.7 |
| 12 | 23 | 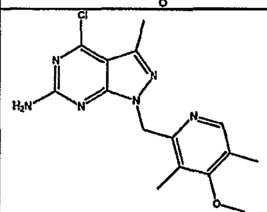 | ND | 0.04 | 0.1 |
| 13 | 14 | 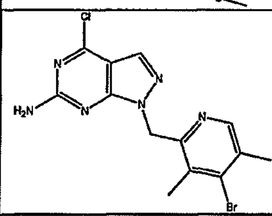 | 0.11 | 0.09 | 1.0 |
| 14 | 26 | 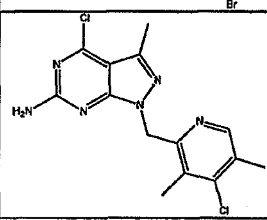 | 0.09 | 0.05 | ND |
| 15 | 15 | 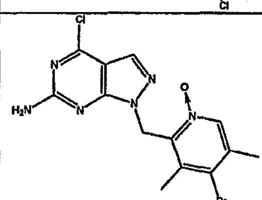 | ND | 0.9 | ND |
| 16 | 25 | 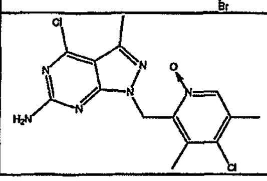 | ND | 0.03 | 0.3 |

| S.No | Ex # | Structure | Lysate binding (μM) | HER2 $IC_{50}$ (μM) | MTS $IC_{50}$ (μM) |
|---|---|---|---|---|---|
| 17 | 28 | 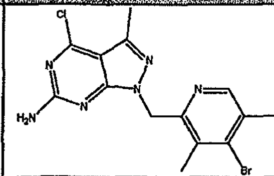 | ND | 0.04 | 1.0 |
| 18 | 24 | 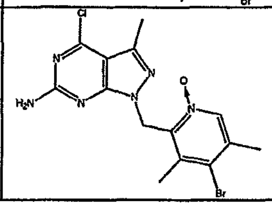 | ND | 0.03 | 1.0 |

ND = not determined

**IV. PREPARATION OF TRIAZOLOPYRIMIDINES OF FORMULA IV**

**A. Materials and Methods**

The chemical reagents used to create the novel products of the invention below are all available commercially, e.g., from Aldrich Chemical Co., Milwaukee, WI, USA. Otherwise their preparation is facile and known to one of ordinary skill in the art, or it is referenced or described herein.

The final compounds were usually purified by preparative TLC (silica gel 60 Å, Whatman Partisil PK6F) or flash chromatography (silica gel 60 Å, EMD Chemicals) using EtOAc/hexane or MeOH/$CH_2Cl_2$ as eluents. Rfs were measured using silica gel TLC plates (silica gel 60 Å, EMD Chemicals). Analytical HPLC chromatograms were obtained using a C18 column (Agilent Zorbax 300SB-C18; 5 microns; 4.6 mm x 150 mm). A gradient was applied between solvent A (0.1 % TFA in $H_2O$) and solvent B (0.5% TFA in $CH_3CN$) increasing the proportion of A linearly from 5% (t=0) to 100% (t=7.00 min), with a constant flow rate of 1 mL/min. The samples were diluted to typically 0.1-1 mg/mL in MeOH or $CH_3CN$ and the injection volumes were typically μL. The column was not heated, and UV detection was effected at 254 nm. $^1$H-NMR spectra were recorded on a Bruker Avance 400 MHz spectrometer.

The chemical names were generated using the Beilstein Autonom 2.1 software.

**B. General Procedures**

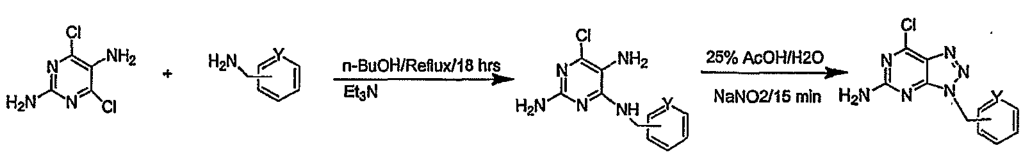


**General Procedure 1: Displacement of chlorine with amines**

Ref: Helv. Chim Acta.1986, 69, 1602-1613; US patent 5,917,042)

A mixture of benzylamine derivative or aminomethyl pyridine derivative (5.88 mmole, 2.1 equivalents), triethylamine (1ml, 7.2 mmole) and 4,6-dichloro-pyrimidine-2,5-diamine (0.5 g, 2.8 mmole), was refluxed in n-BuOH or ethanol (10 mls) for 3 to 18 hours. The mixture was cooled to room temperature and was extracted with $CH_2Cl_2$. The organic layer was washed with water and dried with $MgSO_4$ to afford the crude product. The pyridinyl derivatives were purified by chromatography (100% EtOAc-10% MeOH/ EtOAc), whereas the benzylderivatives were used without further purifycation.

General Procedure 2: Cyclization to form triazolopyrimidine ring system

To a solution of 6-chloto-$N^4$-benzyl-pyrimidine-2,4,5-triamine derivatives or 6-chloro-$N^4$-pyridin-2-ylmethyl-pyrimidine-2,4,5-triamine derivatives (0.57 mmole) in 25%HOAc/$H_2O$, an aqueous solution of $NaNO_2$ (1.2 equivalents, 1ml) was added dropwise at 0˚C. The reaction mixture was stirred for 15 minutes at room temperature and filtered the crude product and purified by column chromatography using 75%EtOAc/Hexanes-100% EtOAc.

General Procedure 3: Aromatic ring halogenation

A mixture of 7-chloro-3-benzyl-3H-[1,2,3]triazolo[4,5-d]pyrimidin-5-ylamine derivatives (0.57 mmole) and NCS (N-chlorosuccinimide) or NBS (N-bromosuccinimide) or NIS (N-iodosuccinimide) (1.5 equivalents) in 10 mls HOAc was stirred at 50˚C for 1 to 15 hours to afford the crude corresponding halogenated product which was purified by chromatography (50-75% EtOAc/Hexanes).

General Procedure 4: N-oxide formation

A solution of the pyridine derivative (1 mmol) in dichloromethane or chloroform (5 mL) was cooled by means of an ice-bath, treated with m-CPBA (1.1 to 3 mmol) in three portions, and allowed to warm to r.t The mixture was extracted with dichloromethane and washed with aqueous NaOH, followed by water. Drying ($Na_2SO_4$) and concentration afforded the pyridine N-oxide.

**Example 219. 7-Chloro-3-(4-methoxy-3,5-dimethylpyridin-2-yl)-3H-[1,2,3]triazolo[4,5-d]pyrimidin-5-ylamine**

*Step 1:* Synthesis of 2-aminomethyl-4-methoxy-3,5-dimethylpyridine:

A solution of 2-chloromethyl-4-methoxy-3,5-dimethyl-pyridine HCl (Aldrich 3.7g, 16.6 mmole) in 7N $NH_3$/MeOH (Aldrich, 200mls) was refluxed in a steel bomb for 15 hours. Removed the solvent under reduced pressure, the residue was taken into 5% MeOH/$CH_2Cl_2$ and filtering it through a thin layer of silica gel afforded the product at 76% yield. HPLC RT was 2.850 min. $^1$HNMR ($CDCl_3$) δ 8.18 (s, 1H), 4.32 (s, 2H), 3.76 (s, 3H), 2.23 (s, 3H), 2.18 (s, 3H).

*Step 2:* Synthesis of 6-chloro-$N^4$-(4-methoxy-3,5-dimethyl-pyridin-2-ylmethyl)-pyrimidine-2,4,5-triamine

A mixture of 4,6-dichloro-pyrimidine-2,5-diamine and 2-aminomethyl-4-methoxy-3,5-dimethyl-pyridine was heated to reflux in n-BuOH for 3 h, following the general procedure 1. HPLC RT was 3.597 min. $^1$HNMR ($CDCl_3$) δ 8.22 (s, 1H), 7.12 (br. t, 1H), 4.61 (s, 2H), 4.56-4.55 (d, 2H), 3.80 (s, 3H), 3.00 (s, 2H), 2.29 (s, 3H), 2.27 (s, 3H).

*Step 3:* Synthesis of 7-chloro-3-(4-methoxy-3,5-dimethyl-pyridin-2-ylmethyl)-3H-[1,2,3]triazolo[4,5-d]pyrimidin-5-ylamine

A solution of 6-chloro-$N^4$-(4-methoxy-3,5-dimethyl-pyridin-2-ylmethyl)-pyrimidine-2,4,5-triamine was treated with a cold aqueous solution of $NaNO_2$, following the general procedure 2. HPLC RT was 3.597 min. $^1$HNMR ($CDCl_3$): δ 8.22 (s, 1H), 7.12 (broad t, 1H), 4.61 (s, 2H), 4.56-4.55 (d, 2H), 3.80 (s, 3H), 3.00 (s, 2H), 2.29 (s, 3H), 2.27 (s, 3H).

**Example 220. 7-Chloro-3-(4-methoxy-3,5-dimethyl-1-oxy-pyridin-2-yl)-3H-[1,2,3]triazolo[4,5-d]pyrimidin-5-ylamine**

The compound was obtained by oxidation of 7-Chloro-3-(4-methoxy-3,5-dimethyl-pyridin-2-ylmethyl)-3H-[1,2,3]triazolo[4,5-d]pyrimidin-5-ylamine (see Example 1) with m-CPBA (m-chloroperoxybenzoic acid) in methylene chloride, following the general procedure 4. HPLC RT was 4.780 min. $^1$HNMR ($CDCl_3$) δ 8.02 (s, 1H), 5.90 (s, 2H), 5.61 (s, 2H), 3.81 (s, 3H), 2.54 (s, 3H), 2.25(s, 3H).

**Example 221. 7-Chloro-3-(4-methoxy-benzyl)-3H-[1,2,3]triazolo[4,5-d]pyrimidin-5-ylamine**

*Step 1:* Synthesis of 6-chloro-$N^4$-(4-methoxy-benzyl)-pyrimidine-2,4,5-triamine:

A mixture of 4,6-dichloro-pyrimidine-2,5-diamine and 1-aminomethyl-4-methoxybenzene was refluxed in n-BuOH for 15 h, following the general procedure 1. HPLC RT was 4.675 min. $^1$HNMR ($CDCl_3$) δ 7.29-7.27 (d, 2H), 6.91-6.89 (d, 2H), 5.62 (br. t, 1H) 4.67 (s, 2H), 4.56-4.54 (d, 2H), 3.84 (s, 3H), 2.74 (s, 2H).

*Step 2:* Synthesis of 7-chloro-3-(4-methoxy-benzyl)-3H-[1,2,3]triazolo[4,5-d]pyrimidin-5-ylamine:

A solution of 6-chloro-$N^4$-(4-methoxy-phenyl)-pyrimidine-2,4,5-triamine was treated with a cold aqueous solution of $NaNO_2$, following the general procedure 2. HPLC RT was 5.784min. $^1$HNMR ($CDCl_3$): δ 7.37-7.35 (d, 2H), 6.86-6.84 (d, 2H), 5.57 (s, 2H), 5.39 (s, 2H), 3.78 (s, 3H).

**Example 222. Synthesis of 7-chloro-3-pyridin-2-ylmethyl-3H-[1,2,3]triazolo[4,5-d]pyrimidin-5-ylamine**

*Step* 1: 6-chloro-$N^4$-pyridin-2-ylmethyl-pyrimidine-2,4,5-triamine

A mixture of 4,6-dichloro-pyrimidine-2,5-diamine and 2-aminomethyl-pyridine was refluxed in n-BuOH for 15 h, following the general procedure 1. HPLC RT was 2.573 min. $^1$HNMR ($CDCl_3$) δ 8.60-8.59 (m, 1H), 7.69-7.66 (m, 1H), 7.31-7.29 (m, 1H), 7.25-7.20 (m, 1H), 6.55 (br. t, 1H) 4.63 (s, 2H), 4.73-4.71 (d, 2H), 1.84 (s, 2H).

*Step 2:* Synthesis of 7-chloro-3-pyridin-2-ylmethyl-3H-[1,2,3]triazolo[4,5-d]pyrimidin-5-ylamine:

A solution of 6-chloro-$N^4$-pyridin-2-ylmethyl-pyrimidine-2,4,5-triamine was treated with a cold aqueous solution of $NaNO_2$, following the general procedure 2. $^1$HNMR ($CDCl_3$): δ 8.60-8.59 (m, 1H), 7.71-7.67(m, 1H), 7.29-7.25 (m, 1H), 7.22-7.20 (m, 1H), 5.81 (s, 2H), 5.48 (s, 2H).

**Example 223. Synthesis of 7-chloro-3-(3,4,5-trimethoxy-benzyl)-3H-[1,2,3]triazolo[4,5-d]pyrimidin-5-ylamine**

*Step 1:* 6-chloro-$N^4$-(3,4,5-trimethoxy-benzyl)-pyrimidine-2,4,5-triamine

A mixture of 4,6-dichloro-pyrimidine-2,5-diamine and 1-aminonlethyl-3,4-5-trimethoxybenzene in n-BuOH for 15 h, following the general procedure 1. HPLC RT was 4.458 min. $^1$HNMR ($CDCl_3$) δ 6.58 (s, 2H), 5.62 (br. t, 1H) 4.72 (s, 2H), 4.56-4.54 (d, 2H), 3.88 (s, 6H), 3.86 (s, 3H), 2.77 (s, 2H).

*Step 2:* Synthesis of 7-chloro-3-(3,4,5-trimethoxy-benzyl)-3H-[1,2,3]triazolo[4,5-d]pyrimidin-5-ylamine):

A solution of 6-chloro-$N^4$-(3,4,5-trimethoxy-benzyl)-pyrimidine-2,4,5-triamine was treated with a cold aqueous solution of $NaNO_2$, following the general procedure 2. HPLC RT was 5.755min. $^1$HNMR ($CDCl_3$): δ 6.66 (s, 2H), 5.55 (s, 2H), 5.42 (s, 2H), 3.83 (s, 3H), 3.80 (s, 6H).

**Example 224. Synthesis of 7-chloro-3-(2-chloro-3,4,5-trimethoxy-benzyl)-3H-[1,2,3]triazolo[4,5-d]pyrimidin-5-ylamine**

Chlorination of 7-chloro-3-(3,4,5-trimethoxy-benzyl)-3H-[1,2,3]triazolo[4,5-d]pyrimidin-5-ylamine) (CF 2137) with NCS (1.5 equivalents) was done following the general procedure 3 to give 7-chloro-3-(2-chloro-3,4,5-trimethoxy-benzyll)-3H-[1,2,3]triazolo[4,5-d]pyrimidin-5-ylamine: HPLC RT was 6.244min. $^1$HNMR ($CDCl_3$): δ 6.53 (s, 1H), 5.70 (s, 2H), 5.48 (s, 2H), 3.89 (s, 3H), 3.87 (s, 3H), 3.75 (s,3H).

**Example 225. 7-Chloro-3-(2,6-dichloro-3,4,5-trimethoxy-benzyl)-3H-[1,2,3]triazolo[4,5-d]pyrimidin-5-ylamine**

Chlorination of 7-chloro-3-(3,4,5-trimethoxy-benzyl)-3H-[1,2,3]triazolo[4,5-d]pyrimidin-5-ylamine) with NCS (1.5 equivalents) was done following the general procedure 3 to give 7-Chloro-3-(2,6-dichloro-3,4,5-trimethoxy-benzyl)-3H-[1,2,3]triazolo[4,5-d]pyrimidin-5-ylamine: HPLC RT was 6.616min. $^1$HNMR ($CDCl_3$): δ 5.81 (s, 2H), 5.47 (s, 2H), 3.97 (s, 3H), 3.90(s, 6H).

**Example 226. Synthesis of 7-chloro-3-(2-bromo-3,4,5-trimethoxy-benzyl)-3H-[1,2,3]triazolo[4,5-d]pyrimidin-5-ylamine**

Bromination of 7-chloro-3-(3,4,5-trimethoxy-benzyl)-3H-[1,2,3]triazolo[4,5-d]pyrimidin-5-ylamine) with NBS (1.5 equivalents) was done following the general procedure 3 to give 7-chloro-3-(2-bromo-3,4,5-trimethoxy-benzyl)-3H-[1,2,3]triazolo[4,5-d]pyrimidin-5-ylamine HPLC RT was 6.541min. $^1$HNMR ($CDCl_3$): δ 6.52 (s, 1H), 5.74(s, 2H), 5.46

(s, 2H), 3.93 (s, 3H), 3.89 (s, 3H), 3.76 (s,3H).

**Example 227. 7-Chloro-3-(2,6-dibromo-3,4,5-trimethoxy-benzyl)-3H-[1,2,3]triazolo[4,5-d]pyrimidin-5-ylamine** (CF 2564)

Bromination of 7-chloro-3-(3,4,5-trimethoxy-benzyl)-3H-[1,2,3]triazolo[4,5-d]pyrimidin-5-ylamine) with NBS (1.5 equivalents) was done following the general procedure 3 to give 7-Chloro-3-(2,6-dibromo-3,4,5-trimethoxy-benzyl)-3H-[1,2,3]triazolo[4,5-d]pyrimidin-5-ylamine: HPLC RT was 6.923min. $^1$HNMR ($CDCl_3$): δ 5.91 (s, 2H), 5.51 (s, 2H), 3.99 (s, 3H), 3.93(s, 6H).

**Example 228. Synthesis of 7-chloro-3-(2-iodo-3,4,5-trimethoxy-benzyl)-3H-[1,2,3]triazolo[4,5-d]pyrimidin-5-ylamine**

The titled compound was obtained from iodination of 7-chloro-3-(3,4,5-trimethoxy-benzyl)-3H-[1,2,3]triazolo [4,5-d]pyrimidin-5-ylamine) with NIS (1.5 equivalents) following the general procedure 3. HPLC RT was 6.497min. $^1$HNMR ($CDCl_3$): δ 6.47 (s, 1H), 5.73(s, 2H), 5.44 (s, 2H), 3.92 (s, 3H), 3.88 (s, 3H), 3.73 (s,3H).

**Example 229. Synthesis of 7-chloro-3-(3,5-dimethoxy-benzyl)-3H-[1,2,3]triazolo[4,5-d]pyrimidin-5-ylamine**

*Step 1:* 6-chloro-$N^4$-(3,5-dimethoxy-benzyl)-pyrimidine-2,4,5-triamine

A mixture of 4,6-dichloro-pyrimidine-2,5-diamine and 1-aminomethyl-3, 5-dimethoxybenzene in n-BuOH for 15 h, following the general procedure 1. HPLC RT was 4.835 min. $^1$HNMR ($CDCl_3$) δ 6.46-6.47 (d, 2H), 6.38-6.37(d, 1H), 5.67 (br. t, 1H) 4.63 (s, 2H), 4.53-4.52 (d, 2H), 3.81 (s, 6H), 2.72 (s, 2H).

*Step 2:* Synthesis of 7-chloro-3-(3,5-dimethoxy-benzyl)-3H-[1,2,3]triazolo[4,5-d]pyrimidin-5-ylamin:

A solution of 6-chloro-$N^4$-(3,5-dimethoxy-benzyl)-pyrimidine-2,4,5-triamine was treated with a cold aqueous solution of $NaNO_2$, following the general procedure 2. HPLC RT was 6.185min. $^1$HNMR ($CDCl_3$): δ 6.54-6.53 (d, 2H), 6.41-6.40 (d, 1H), 5.58 (s, 2H), 5.54 (s, 2H), 3.78 (s, 6H).

**Example 230. Synthesis of 7-chloro-3-(2-chloro-3,5-dimethoxy-benzyl)-3H-[1,2,3]triazolo[4,5-d]pyrimidin-5-ylamine**

The titled compound was obtained by chlorination of 7-chloro-3-(3,5-dimethoxybenzyl)-3H-[1,2,3]triazolo[4,5-d]pyrimidin-5-ylamine) with NCS (1.5 equivalents) in acetic acid at 50 ˚C for 1 h, following the general procedure 3. HPLC RT was 6.467min. $^1$HNMR ($CDCl_3$): δ 6.50-6.49 (d, 1H), 6.18-6.17 (d, 1H), 5.77(s, 2H), 5.44 (s, 2H), 3.91 (s, 3H), 3.72 (s, 3H).

**Example 231. Synthesis of 7-chloro-3-(2-bromo-3,5-dimethoxy-benzyl)-3H-[1,2,3]triazolo[4,5-d]pyrimidin-5-ylamine**

The titled compound was obtained by bromination of 7-chloro-3-(3,5-dimethoxybenzyl)-3H-[1,2,3]triazolo[4,5-d]pyrimidin-5-ylamine) with.NBS (1.5 equivalents) in acetic acid at 50 ˚C during 1 h, following the general procedure 3. HPLC RT was 6.573min. $^1$HNMR ($d_6$-DMSO): δ 7.74 (s, 2H), 6.70-6.69 (d, 1H), 6.23-6.22 (d,1H), 5.63(s, 2H), 3.87 (s, 3H), 3.71 (s, 3H).

**Example 232. Synthesis of 7-chloro-3-(2-iodo-3,5-dimethoxy-benzyl)-3H-[1,2,3]triazolo[4,5-d]pyrimidin-5-ylamine**

The titled compound was obtained by iodination of 7-chloro-3-(3,5-dimethoxybenzyl)-3H-[1,2,3]triazolo[4,5-d] pyrimidin-5-ylamine) with NIS (1.5 equivalents) in acetic acid at 50 ˚C during 1 h, following the general procedure 3. HPLC RT was 6.739min. $^1$HNMR ($d_6$-DMSO): δ 7.75 (s, 2H), 6.61-6.60 (d, 1H), 6.15-6.14 (d, 1H), 5.58(s, 2H), 3.86 (s, 3H), 3.70 (s, 3H).

**Example 233. Synthesis of 7-Chloro-3-(2,5-dimethoxy-benzyl)-3H-[1,2,3]triazolo[4,5-d]pyrimidin-5-ylamine**

*Step 1:* 6-chloro-$N^4$-(2,5-dimethoxy-benzyl)-pyrimidine-2,4,5-triamine

A mixture of 4,6-dichloro-pyrimidine-2,5-diamine and 1-aminomethyl-2,5-dimethoxybenzene in n-BuOH for 15 h, following the general procedure 1. HPLC RT was 4.601min. $^1$HNMR ($CDCl_3$) δ 6.91-6.90 (d, 1H), 6.82-6.80(m, 2H), 5.82 (br. t, 1H) 4.62 (s, 2H), 4.59-4.58 (d, 2H), 3.85 (s, 3H), 2.78 (s, 3H), 2.75 (s, 2H).

***Step 2:* Synthesis of 7-chloro-3-(2,5-dimethoxy-benzyl)-3H-[1,2,3]triazolo[4,5-d]pyrimidin-5-ylamine**

A solution of 6-chloro-$N^4$-(2,5-dimethoxy-benzyl)-pyrimidine-2,4,5-triamine was treated with a cold aqueous solution of $NaNO_2$, following the general procedure 2. HPLC RT was 6.130min. $^1$HNMR ($CDCl_3$): δ 6.84-6.83 (m, 2H), 6.64-6.63 (d,1H), 5.67 (s, 2H), 5.54 (s, 2H), 3.83 (s, 3H), 3.73 (s, 3H).

**Exapmle 234. Synthesis of 7-chloro-3-(4-bromo-2,5-dimethoxy-benzyl)-3H-[1,2,3]triazolo[4,5-d]pyrimidin-5-ylamine**

The titled compound was obtained by bromination of 7-chloro-3-(2,5-dimethoxybenzyl)-3H-[1,2,3]triazolo[4,5-d]pyrimidin-5-ylamine) with NBS (1.5 equivalents) in acetic acid at 50 ˚C during 1 h, following the general procedure 3. HPLC RT was 6.438min. $^1$HNMR ($CDCl_3$): δ 7.11 (s, 1H), 6.80 (s, 1H), 5.63 (s, 2H), 5.57 (s, 2H), 3.82 (s, 3H), 3.79 (s, 3H).

**Example 235. Synthesis of 7-chloro-3-(3-chloro-2,5-dimethoxy-benzyl)-3H-[1,2,3]triazolo[4,5-d]pyrimidin-5-ylamine**

The titled compound was obtained by chlorination of 7-chloro-3-(2,5-dimethoxybenzyl)-3H-[1,2,3]triazolo[4,5-d]pyrimidin-5-ylamine) with NCS (1.5 equivalents) in acetic acid at 50˚C during 1 h, following the general procedure 3. HPLC RT was 6.392min. $^1$HNMR ($CDCl_3$): δ 6.91-6.90 (d, 1H), 6.67-6.66 (d, 1H), 5.70 (s, 2H), 5.43(s, 2H), 3.92 (s, 3H), 3.73 (s, 3H).

BIOLOGY EXAMPLES

The biological activites of selected triazolopyrimidines were determined using four assays: the inhibition of binding of biotinylated geldamamycin (biotin-GM) to rHSP90, the lysate binding ability, the HER2 degradation ability and the cytotoxity measurement. These assays have been described in Examples A, B, C and D in the previous sections. The biological activites are summarized in Table 8.

**TABLE 8. Biological Activities of Selected Triazolopyrimidines of Formula IV**

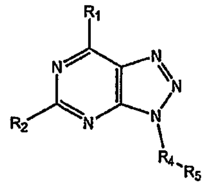

IV

| S.No | Ex # | Structure | HER2 IC$_{50}$ (μM) | MTS IC$_{50}$ (μM) |
|---|---|---|---|---|
| 1 | 5 | 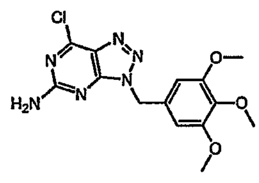 | 15.0 | ND |

| S.No | Ex # | Structure | HER2 $IC_{50}$ (uM) | MTS $IC_{50}$ (uM) |
|---|---|---|---|---|
| 2 | 1 | 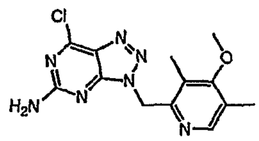 | 0.4 | 8.0 |
| 3 | 7 | 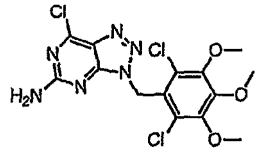 | 4.0 | ND |
| 4 | 6 | 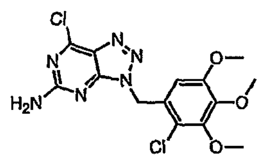 | 12.0 | ND |
| 5 | 9 | 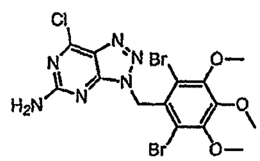 | 6.3 | ND |
| 6 | 8 | 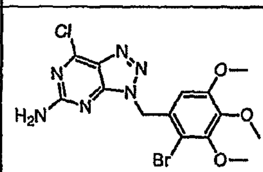 | 6.5 | ND |
| 7 | 10 | 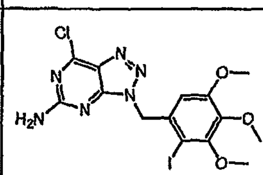 | 10.0 | ND |
| 8 | 12 | 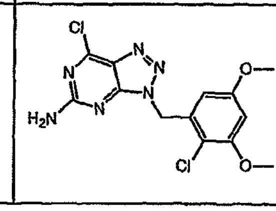 | 8.5 | ND |

| S.No | Ex # | Structure | HER2 $IC_{50}$ (µM) | MTS $IC_{50}$ (µM) |
|---|---|---|---|---|
| 9 | 13 | 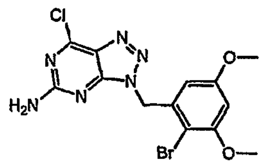 | 16.0 | ND |
| 10 | 16 | 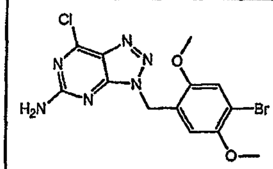 | 22.0 | ND |
| 11 | 17 | 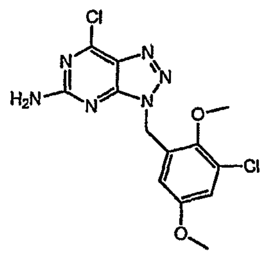 | 19.0 | ND |
| 12 | 2 | 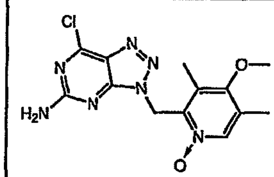 | 0.5 | ND |

ND = not determined

The foregoing examples are not limiting and are merely illustrative of various aspects and embodiments of the present invention. All documents cited herein are indicative of the levels of skill in the art to which the invention pertains and are incorporated by reference herein in their entireties. None, however, is admitted to be prior art.

One skilled in the art will readily appreciate that the present invention is well adapted to carry out the objects and obtain the ends and advantages mentioned, as well as those inherent therein. The methods and compositions described illustrate preferred embodiments, are exemplary, and are not intended as limitations on the scope of the invention. Certain modifications and other uses will occur to those skilled in the art, and are encompassed within the spirit of the invention, as defined by the scope of the claims.

The invention illustratively described herein suitably may be practiced in the absence of any element or elements, limitation or limitations which is not specifically disclosed herein. The terms and expressions which have been employed are used as terms of description and not of limitation, and there is no intention in the use of such terms and expressions of excluding any equivalents of the features shown and described, or portions thereof. It is recognized that various modifications are possible within the scope of the invention claimed. Thus, it should be understood that although the present invention has been specifically disclosed by preferred embodiments, optional features, modifications and variations of the concepts herein disclosed may be resorted to by those skilled in the art, and that such modifications and variations are considered to be within the scope of this invention as defined by the description and the appended claims.

In addition, where features or aspects of the invention are described in terms of Markush groups or other grouping of alternatives, e.g., genuses, those skilled in the art will recognize that the invention is also thereby described

in terms of any individual member or subgroup of members of the Markush group or subgenus, and exclusions of individual members as appropriate, *e.g.,* by proviso.

The invention is also characterized by the following items:

1. A compound of Formula A

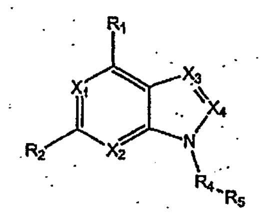

A

or tautomer or pharmaceutically acceptable salt or prodrug thereof, wherein
$X_1$ and $X_2$ are the same or different and each is nitrogen or $-CR^6$;
$X_3$ is nitrogen or $-CR^3$ wherein $R^3$ is hydrogen, OH, a keto tautomer, $-OR^8$, -CN, halogen, lower alkyl, or $-C(O)R^9$;
$X_4$ is nitrogen or a group $CR^6$ when $X_3$ is nitrogen, and $X_4$ is $-CR^6R^7$ when $X_3$ is $- CR^3$;
$R^1$ is halogen, $-OR^8$, $-SR^8$, or lower alkyl;
$R^2$ is $-NR^8R^{10}$;
$R^4$ is $-(CH_2)_n-$ where n = 0-3, -C(O), -C(S), $-SO_2-$, or $-SO_2N-$; and
$R^5$ is alkyl, aryl, heteroaryl, alicyclic, heterocyclic, all optionally bi-or tricyclic, and optionally substituted with H, halogen, lower alkyl, lower alkenyl, lower alkynyl, lower aryl, lower alicyclic, araalkyl, aryloxyalkyl, alkoxyalkyl, perhaloalkyl, perhaloalkyloxy, perhaloacyl, $-N_3$, $-SR^8$, $-OR^8$, -CN, $-CO_2R^9$, $-NO_2$, or $-NR^8R^{10}$;
with the provisos that:

said compound is not one found or described in one or more of JP 10025294; US Patent 4,748,177; US Patent 4,748,177; US Patent 6,369,092; WO 00/06573; WO 02/055521; WO 02/055082; WO 02/055083; European Journal of Medicinal Chemistry, 1994, 29(1), 3-9; and J. Het. Chem. 1990, 27(5), 1409;
$-R^4R^5$ is not a ribose or derivative thereof, or a sugar or derivative thereof;
$-R^4R^5$ is not a phosphonate or phosphonic acid, or substituted with phosphonate or phosphonic acid; and
when $R^4$ is $(CH_2)_n$ where n= 0 or 1, then $R^4$ and $R^5$ are not connected with 'O'.

2. The compound, tautomer, pharmaceutically acceptable salt thereof, or prodrug of item 1 having Formula IIA:

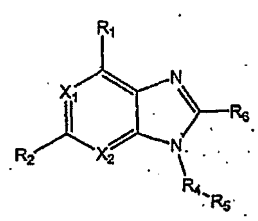

IIA

wherein
$X_1$ and $X_2$ are the same or different and each is nitrogen or $-CR^6$;
$R^1$ is halogen, $OR^8$, $SR^8$, or lower alkyl;
$R^2$ is $-NR^8R^{10}$;
$R^4$ is $-(CH_2)_n-$ where n = 0-3, -C(O), -C(S), $-SO_2-$, or $-SO_2N-$;
$R^6$ is hydrogen, halogen, lower alkyl, $-SR^8$, $-OR^8$, $-NR^8R^{10}$, $-N_3$, -CN, or $- C(O)R^9$;
$R^5$ is alkyl, aromatic, heteroaromatic, alicyclic, or heterocyclic, all optionally bi-or tri-cyclic, and all optionally substituted with H, halogen, lower alkyl, $-SR^8$, $-OR^8$, - CN, $- CO_2R^9$, $-NO_2$, or $-NR^8R^{10}$;
$R^8$ is hydrogen, lower alkyl, lower aryl, or $-(CO)R^9$;
$R^9$ is lower alkyl, lower aryl, lower heteroaryl, $NR^8R^{10}$ or $-OR^{11}$;
$R^{11}$ is lower alkyl or lower aryl; and

$R^{10}$ is hydrogen or lower alkyl.

3. The compound, tautomer, pharmaceutically acceptable salt thereof, or prodrug thereof of item1 having Formula IV:

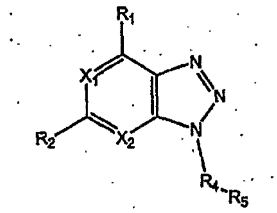

IV

wherein
$X_1$ and $X_2$ are the same or different and each is nitrogen or $CR^6$;
$R^1$ is halogen, $-OR^8$, $-SR^8$, or lower alkyl;
$R^2$ is $-NR^8R^{10}$;
$R^4$ is $-(CH_2)_n$,- where n = 0-3, -C(O), -C(S), $-SO_2-$, or $-SO_2N-$;
$R^5$ is alkyl, aryl, heteroaryl, alicyclic, heterocyclic, all optionally bi-or tricyclic, and all optionally substituted with H, halogen, lower alkyl, $-SR^8$, $-OR^8$, -CN, - $CO_2R^9$, - $NO_2$, or $-NR^8R^{10}$;
$R^8$ is hydrogen, lower alkyl, lower aryl or $-(CO)R^9$;
$R^9$ is lower alkyl, lower aryl, lower heteroaryl, $-NR^8R^{10}$ or $-OR^{11}$;
$R^{11}$ is lower alkyl or lower aryl; and
$R^{10}$ is hydrogen or lower alkyl.

4. The compound, tautomer, pharmaceutically acceptable salt thereof, or prodrug thereof of item 1 having Formula III

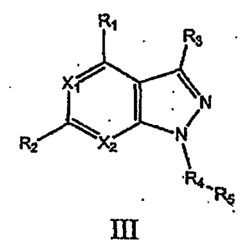

III

wherein
$X_1$ and $X_2$ are the same or different and each is nitrogen or $CR^6$;
$R_1$ is halogen, $-OR^8$, $-SR^8$ or lower alkyl;
$R^2$ is $-NR^8R^{10}$;
$R^3$ is hydrogen, OH or keto tautomer, $-OR^8$, halogen, -CN, lower alkyl or - $C(O)R^9$;
$R^4$ is $-(CH_2)_n-$ where n = 0-3, -C(O), -C(S), $-SO_2-$ or $-SO_2N-$;
$R^5$ is alkyl, aryl, heteroaryl, alicyclic, heterocyclic, all optionally bi- or tricyclic, and all optionally substituted with H, halogen, lower alkyl, $-SR^8$, $-OR^8$, -CN, $-CO_2R^9$, - $NO_2$ or $-NR^8R^{10}$;
$R^8$ is hydrogen, lower alkyl, lower aryl or $-(CO)R^9$;
$R^9$ is lower alkyl, lower aryl, lower heteroaryl, $-NR^8R^{10}$ or $OR^{11}$;
$R^{11}$ is lower alkyl or lower aryl; and
$R^{10}$ is hydrogen or lower alkyl.

5. The compound, tautomer, pharmaceutically acceptable salt thereof, or prodrug thereof of item 1 having Formula I

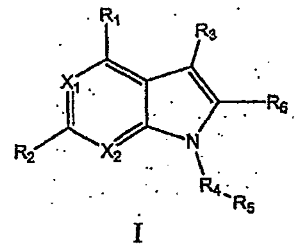

I

wherein
$X_1$ and $X_2$ are the same or different and each is nitrogen or $-CR^6$;
$R_1$ is halogen, $-OR^8$, $-SR^8$ or lower alkyl;
$R^2$ is $-NR^8R^{10}$;
$R^3$ is hydrogen, OH or a keto tautomer, $-OR^8$, halogen, -CN, lower alkyl or $-C(O)R^9$;
$R^4$ is $-(CH_2)_n-$ where n = 0-3, -C(O), -C(S), $-SO_2-$ or $-SO_2N-$;
$R^6$ is hydrogen, halogen, lower alkyl, $-SR^8$, $-OR^8$, $-NR^8R^{10}$, $-N_3$, or $-C(O)R^9$;
$R^5$ is alkyl, aromatic, heteroaromatic, alicyclic, heterocyclic, all optionally bi- or tricyclic, and all optionally substituted with H, halogen, lower alkyl, $-SR^8$, $-OR^8$, -CN, - $CO_2R^9$, $-NO_2$, or $-NR^8R^{10}$;
$R^8$ is hydrogen, lower alkyl, lower aryl, or $-(CO)R^9$;
$R^9$ is lower alkyl, lower aryl, lower heteroaryl, $-NR^8R^{10}$ or $-OR^{11}$;
$R^{11}$ is lower alkyl or lower aryl; and
$R^{10}$ is hydrogen or lower alkyl;

6. The compound, tautomer, pharmaceutically acceptable salt thereof, or prodrug thereof of item 1 having Formula IIA:

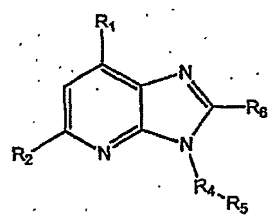

IIA

wherein
$R_1$ is halogen, $-OR^8$, $-SR^8$ or lower alkyl;
$R^2$ is $-NR^8R^{10}$;
$R^4$ is $-(CH_2)_n-$ where n = 0-3, -C(O), -C(S), $-SO_2-$ or $-SO_2N-$;
$R^6$ is hydrogen, halogen, lower alkyl, $-SR^8$, $-OR^8$, $-NR^8R^{10}$, $-N_3$, -CN or $C(O)R^9$;
$R^5$ is alkyl, aromatic, heteroaromatic, alicyclic, heterocyclic, all optionally bi- or tricyclic, and all optionally substituted with H, halogen, lower alkyl, $-SR^8$, $-OR^8$, -CN, - $CO_2R^9$, $-NO_2$, or-$NR^8R^{10}$;
$R^8$ is hydrogen, lower alkyl, lower aryl, or $-(CO)R^9$;
$R^9$ is lower alkyl, lower aryl, lower heteroaryl, $NR^8R^{10}$ or $OR^{11}$;
$R^{11}$ is lower alkyl or lower aryl; and
$R^{10}$ is hydrogen or lower alkyl.

7. The compound, tautomer, pharmaceutically acceptable salt thereof, or prodrug thereof of item 1 having Formula IIB

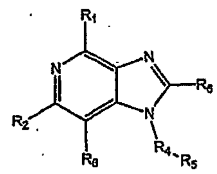

VII

IIB

wherein
$R^1$ is halogen, $-OR^8$, $-SR^8$ or lower alkyl;
$R^2$ is $-NR^8R^{10}$;
$R^4$ is $-(CH_2)_n-$, where n = 0-3, -C(O), -C(S), $-SO_2-$ or $-SO_2N-$;
$R^6$ is hydrogen, halogen, lower alkyl, $-SR^8$, $-OR^8$, $-NR^8R^{10}$, $-N_3$, -CN or $-C(O)R^9$;
$R^5$ is alkyl, aromatic, heteroaromatic, alicyclic, heterocyclic, all optionally bi- or tricyclic, and all optionally substituted with H, halogen, lower alkyl, $-SR^8$, $-OR^8$, -CN, - $CO_2R^9$, $-NO_2$, or $-NR^8R^{10}$;
$R^8$ is hydrogen, lower alkyl, lower aryl, or $-(CO)R^9$;
$R^9$ is lower alkyl, lower aryl, lower heteroaryl, $-NR^8R^{10}$ or$-OR^{11}$;
$R^{11}$ is lower alkyl or lower aryl; and
$R^{10}$ is hydrogen or lower alkyl.

8. The compound, tautomer, pharmaceutically acceptable salt thereof, or prodrug thereof of item 1 having Formula IA:

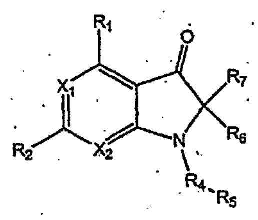

IA

wherein
$X_1$ and $X_2$ are the same or different and each is nitrogen or a group $-CR^6$;
$R^1$ is halogen, $-OR^8$, $-SR^8$, or lower alkyl;
$R^2$ is $-NR^8R^{10}$;
$R^4$ is $-(CH_2)_n-$ where n = 0-3, -C(O), -C(S), $-SO_2-$ or $-SO_2N-$;
$R^5$ is alkyl, aromatic, heteroaromatic, alicyclic, heterocyclic, all optionally bi- or tricyclic, and all optionally substituted with H, halogen, lower alkyl, $-SR^8$, $-OR^8$, -CN, - $CO_2R^9$, $-NO_2$, or $-NR^8R^{10}$;
$R^6$ is hydrogen, halogen, lower alkyl, $-SR^8$, $-OR^8$, $-NR^8R^{10}$, $N_3$, -CN, $-C(O)R^9$, or together with $R^7$ is carbonyl (C=O);
$R^7$ is independently selected from hydrogen, lower alkyl or together with $R^6$ is carbonyl (C=O);
$R^8$ is hydrogen, lower alkyl, lower aryl, or $-(CO)R^9$;
$R^9$ is lower alkyl, lower aryl, lower heteroaryl, $-NR^8R^{10}$ or $-OR^{11}$; $R^{11}$ is lower alkyl or lower aryl; and
$R^{10}$ is hydrogen or lower alkyl.

9. The compound, tautomer, pharmaceutically acceptable salt thereof, or prodrug thereof of item 2 wherein $R^1$ is halogen, $R^2$ is $NH_2$, $R^4$ is $-CH_2-$, $R^6$ is H or halogen, and wherein $R^5$ is phenyl optionally substituted with H, halogen, C1-4 alkyl, C1-4 alkoxy, C1-4 alkylthio, perhaloalkyl, perhaloalkyloxy, -CN, $-NO_2$, $-NH_2$ or - $CO_2R^{11}$.

10. The compound, tautomer, pharmaceutically acceptable salt thereof, or prodrug thereof of item 9 wherein $R^1$ is halogen, $R^2$ is $-NH_2$, $R^4$ is $-CH_2-$, $R^6$ is H, and
wherein $R^5$ is 2-halo-3,5 dimethoxyphenyl optionally substituted with H, halogen, C1-4 alkyl, C1-4 alkoxy, C1-4 alkylthio, perhaloalkyl, perhaloalkyloxy, -CN, $-NO_2$, $-NH_2$, or $-CO_2R^{11}$ at the para (4-) position.

11. The compound, tautomer, pharmaceutically acceptable salt thereof, or prodrug thereof of item 9 wherein $R^1$ is chloro, $R^2$ is $-NH_2$, $R^4$ is $-CH_2-$, $R^6$ is H and $R^5$ is 2-chloro-3, 4, 5 trimethoxyphenyl.

12. The compound, tautomer, pharmaceutically acceptable salt thereof, or prodrug thereof of item 9 wherein $R^1$ is chloro, $R^2$ is $-NH_2$, $R^4$ is $-CH_2-$, $R^6$ is H and $R^5$ is 2-bromo-3, 4, 5 trimethoxyphenyl.

13. The compound, tautomer, pharmaceutically acceptable salt thereof, or prodrug thereof of item 9 wherein $R_1$ is chloro, $R^2$ is $-NH_2$, $R^4$ is $-CH_2-$, $R^6$ is H and $R^5$ is 2-iodo-3, 4, 5 trimethoxyphenyl.

14. The compound, tautomer, pharmaceutically acceptable salt thereof, or prodrug thereof of item 9 wherein $R_1$ is chloro, $R^2$ is $-NH_2$, $R^4$ is $-CH_2-$, $R^6$ is H and $R^5$ is 2-fluoro-3, 4, 5 trimethoxyphenyl.

15. The compound, tautomer, pharmaceutically acceptable salt thereof, or prodrug thereof of item 9 wherein $R_1$ is bromo, $R^2$ is $-NH_2$, $R^4$ is $-CH_2-$, $R^6$ is H, and $R^5$ is 2-bromo-3, 4, 5 trimethoxyphenyl.

16. The compound, tautomer, pharmaceutically acceptable salt thereof, or prodrug thereof of item 9 wherein $R_1$ is bromo, $R_2$ is $-NH_2$, $R_4$ is $-CH_2-$, $R^6$ is H, and $R^5$ is 2-iodo-3, 4, 5 trimethoxyphenyl.

17. The compound, tautomer, pharmaceutically acceptable salt thereof, or prodrug thereof of item 9 wherein $R_1$ is bromo, $R^2$ is $-NH_2$, $R^4$ is $-CH_2-$, $R^6$ is H and $R^5$ is 2-iodo-3, 4, 5 trimethoxyphenyl.

18. The compound, tautomer, pharmaceutically acceptable salt thereof, or prodrug thereof of item 9 wherein $R^1$ is bromo, $R^2$ is $-NH_2$, $R^4$ is $-CH_2-$, $R^6$ is H and $R^5$ is 2-fluoro-3, 4, 5 trimethoxyphenyl.

19. The compound, tautomer, pharmaceutically acceptable salt thereof, or prodrug thereof of item 9 wherein $R^1$ is chloro, $R^2$ is $-NH_2$, $R^4$ is $-CH_2-$, $R^6$ is halo and $R^5$ is 2-chloro-3, 4, 5 trimethoxyphenyl.

20. The compound, tautomer, pharmaceutically acceptable salt thereof, or prodrug thereof of item 9 wherein $R^1$ is chloro, $R^2$ is $NH_2$, $R^4$ is $-CH_2-$, $R^6$ is halo, and $R^5$ is 2-bromo-3, 4, 5 trimethoxyphenyl.

21. The compound, tautomer, pharmaceutically acceptable salt thereof, or prodrug thereof of item 9 wherein $R^1$ is bromo, $R^2$ is $-NH_2$, $R^4$ is $-CH_2-$, $R^6$ is halo and $R^5$ is 2-chloro-3, 4, 5 trimethoxyphenyl.

22. The compound, tautomer, pharmaceutically acceptable salt thereof, or prodrug thereof of item 9 wherein $R^1$ is bromo, $R^2$ is $-NH_2$, $R^4$ is $-CH_2-$, $R^6$ is halo and $R^5$ is 2-bromo-3, 4, 5 trimethoxyphenyl.

23. A pharmaceutical composition comprising the compound, tautomer, pharmaceutically acceptable salt thereof, or prodrug thereof of item 2 and one or more pharmaceutical excipients.

24. A method of inhibiting an HSP90, comprising:

contacting a cell having an HSP90 with a compound, tautomer pharmaceutically acceptable salt thereof, prodrug thereof or pharmaceutical composition according to item 2.

25. A compound represented by Formula I, or a polymorph, solvate, ester, tautomer, enantiomer, pharmaceutically acceptable salt or prodrug thereof:

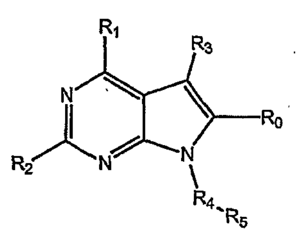

I

wherein:

$R^0$ is selected from hydrogen, halogen, lower alkyl, $-SR^8$, $-OR^8$, -CN, and $-NHR^8$,

$R^1$ is halogen, -$OR^{11}$, -$SR^{11}$ or lower alkyl;
$R^2$ is -$NHR^8$;
$R^3$ is selected from the group consisting of hydrogen, halogen, -$SR^8$, -$OR^8$, -CN, -C(O)$R^9$, -C(O)OH, $NO_2$, -$NR^8R^{10}$, lower alkyl, lower alkenyl, lower alkynyl, lower perhaloalkyl, aryl, heteroaryl, alicyclic and heterocyclic, all optionally substituted, wherein:

the aryl, heteroaryl, alicyclic and heterocyclic groups are optionally mono-, bi- or tri-cyclic,
$R^8$ and $R^{10}$ taken together optionally form a ring of 3-7 ring atoms and optionally 1-3 of the ring atoms are heteroatoms selected from the group of O, S and N, and
the optional substituents on $R^3$ are selected from the group consisting of halogen, lower alkyl, lower alkenyl, lower alkynyl, -$SR^8$, -$OR^8$, -CN, -C(O)OH, -C(O)$R^9$, -$NO_2$, -$NR^8R^{10}$, lower aryl, heteroaryl, alicyclic, lower heterocyclic, arylalkyl, heteroarylalkyl, amino, alkylamino, dialkylamino, diarylalkylamino, oxo, oxa, perhaloalkyl, perhaloalkoxy, perhaloacyl, guanidine, pyridinyl, thiophene, furanyl, indole, indazole, phosphonates, phosphates, phosphoramides, sulfonates, sulfones, sulfates, sulphonamides, carbamates, ureas, thioureas and thioamides, wherein $R^8$ and $R^{10}$ taken together optionally form a ring of 3-7 ring atoms and optionally 1-3 of the ring atoms are heteroatoms selected from the group of O, S and N;

$R^0$ or $R^3$ is -OH or -SH, the compound may exist as the corresponding (thio)keto tautomer or a mixture of keto-enol tautomers;
$R^4$ is -$CHR^{12}$-, -C(O)-, -C(S)-, -S(O)- or -$SO_2$-;
$R^5$ is aryl, heteroaryl, alicyclic, or heterocyclic, wherein
the aryl group is substituted with 3 to 5 substituents,
the heteroaryl group is substituted with 2 to 5 substituents,
the alicyclic group is substituted with 3 to 5 substituents,
the heterocyclic group is substituted with 3 to 5 substituents, and
the substituents are selected from the group consisting of halogen, lower alkyl, lower alkenyl, lower alkynyl, -$SR^8$, -$OR^8$, -CN, -C(O)OH, -C(O)$R^9$, $NO_2$, -$NR^8R^{10}$, lower aryl, heteroaryl, alicyclic, lower heterocyclic, arylalkyl, heteroarylalkyl, amino, alkylamino, dialkylamino, diarylalkylamino, oxo, oxa, perhaloalkyl, perhaloalkoxy, perhaloacyl, guanidine, pyridinyl, thiophene, furanyl, indole, indazole, phosphonates, phosphates, phosphoramides, sulfonates, sulfones, sulfates, sulphonamides, carbamates, ureas, thioureas and thioamides, wherein $R^8$ and $R^{10}$ taken together optionally form a ring of 3-7 ring atoms and optionally 1-3 of the ring atoms are heteroatoms selected from the group of O, S and N;
$R^8$ is hydrogen, lower alkyl, lower alkenyl, or lower alkynyl, lower aryl, lower heteroaryl, or -C(O)$R^9$;
$R^9$ is H, lower alkyl, lower alkenyl, lower alkynyl, lower aryl, lower heteroaryl, -$NR^{10}R^{10}$, or -$OR^{11}$, wherein $R^{10}$ and $R^{10}$ taken together optionally form a ring of 3-7 ring atoms and optionally 1-3 of the ring atoms are heteroatoms selected from the group of O, S and N;
$R^{10}$ is hydrogen, lower alkyl, lower alkenyl, lower alkynyl, lower aryl or lower heteroaryl;
$R^{11}$ is lower alkyl, lower alkenyl, lower alkynyl, lower heteroaryl or lower aryl; and
$R^{12}$ is hydrogen or lower alkyl.

26. The compound of item 25, or a polymorph, solvate, ester, tautomer, enantiomer, pharmaceutically acceptable salt or prodrug thereof, wherein each of said aryl, heteroaryl, alicyclic or heterocyclic group is monocyclic or bicyclic.

27. The compound of item 25 or a polymorph, solvate, ester, tautomer, enantiomer, pharmaceutically acceptable salt or prodrug thereof, wherein:

$R^0$ is hydrogen, halogen, -SH, -OH, or -CN;
$R^1$ is halogen; and
$R^2$ is -$NHR^8$, where $R^8$ is hydrogen or -C(O)$R^9$,

28. The compound of item 25, or a polymorph, solvate, ester, tautomer, enantiomer, pharmaceutically acceptable salt or prodrug thereof, wherein:

$R^1$ is chloro or bromo,
$R^2$ is -$NHR^8$, where $R^8$ is hydrogen or -C(O)$R^9$; and
$R^3$ is hydrogen, halogen, -$OR^8$, -$SR^8$, -$NR^8R^{10}$, lower alkyl, lower alkenyl, or lower alkynyl, lower perhaloalkyl, lower aryl, or lower heteroaryl.

29. The compound of item 25, or a polymorph, solvate, ester, tautomer, enantiomer, pharmaceutically acceptable salt or prodrug thereof, wherein:

$R^0$ is hydrogen, halogen or-CN;
$R^2$ is $-NHR^8$, where $R^8$ is hydrogen or $-C(O)R^9$; and
$R^4$ is $-CH_2-$.

30. The compound of item 25, or a polymorph, solvate, ester, tautomer, enantiomer, pharmaceutically acceptable salt or prodrug thereof, wherein:

$R^0$ is hydrogen, halogen, -SH, -OH or -CN;
$R^1$ is halogen;
$R^2$ is $NH_2$;
$R^3$ is hydrogen, halogen, $-OR^8$, $-SR^8$, $-NR^8R^{10}$, lower alkyl, lower alkenyl, lower alkynyl, lower perhaloalkyl, lower aryl, or lower heteroaryl, wherein $R^8$ is hydrogen, lower alkyl, lower aryl, or $-C(O)R^9$;
$R^4$ is $-CH_2-$; and
$R^5$ is aryl or heteroaryl, wherein each of said aryl and heteroaryl is monocyclic or bicyclic and is substituted with 3 to 5 substituents.

31. The compound of item 30, or a polymorph, solvate, ester, tautomer, enantiomer, pharmaceutically acceptable salt or prodrug thereof, wherein $R^1$ is chloro or bromo, $R^2$ is $-NH_2$, and $R^5$ is a phenyl having at least three substituents.

32. The compound of item 30, or a polymorph, solvate, ester, tautomer, enantiomer, pharmaceutically acceptable salt or prodrug thereof, wherein $R^1$ is chloro or bromo, $R^2$ is $-NH_2$ and $R^5$ is a pyridyl having at least two substituents.

33. The compound of item 30, or a polymorph, solvate, ester, tautomer, enantiomer, pharmaceutically acceptable salt or prodrug thereof, wherein $R^1$ is chloro or bromo, $R^2$ is $-NH_2$, and $R^5$ is 1-oxy-pyridyl (N-oxy-pyridyl) having at least two substituents.

34. The compound of item 30, wherein said compound is a member selected from the group below, or a polymorph, solvate, ester, tautomer, enantiomer, pharmaceutically acceptable salt or prodrug thereof:

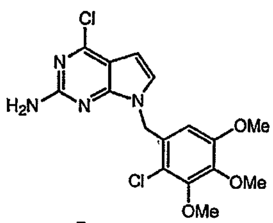 , 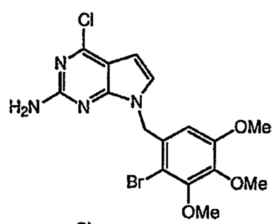 , 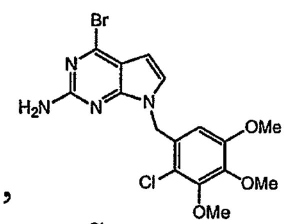 ,

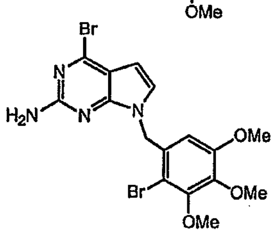 , 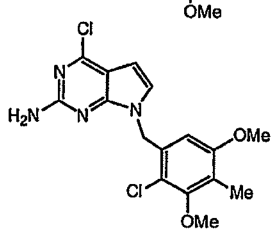 , 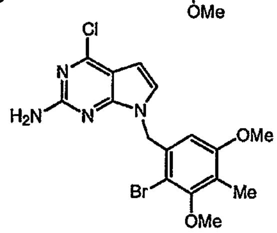 ,

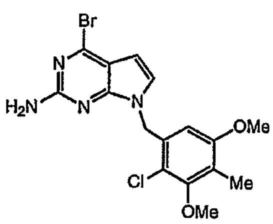 , 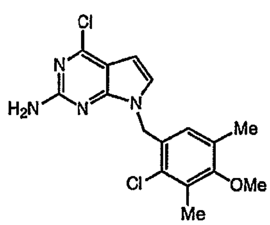 and 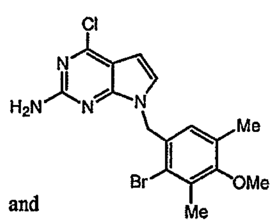

35. The compound of item 30, wherein said compound is a member selected from the group below, or a polymorph, solvate, ester, tautomer, enantiomer, pharmaceutically acceptable salt or prodrug thereof:

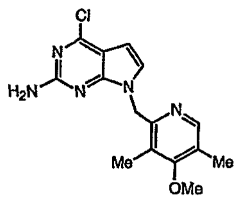,
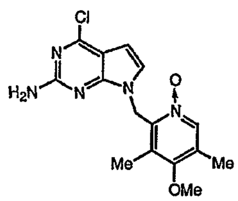,
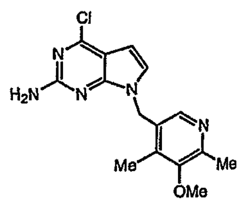,
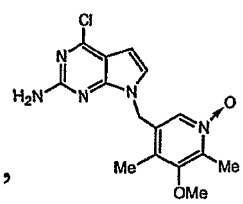,
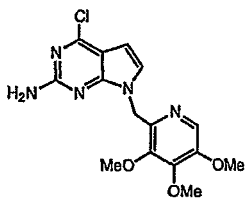,
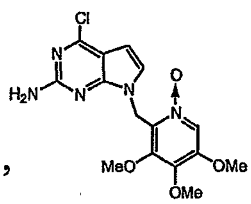,
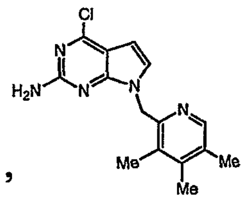,
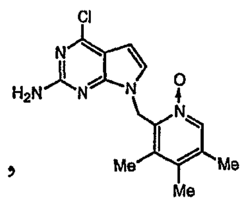,
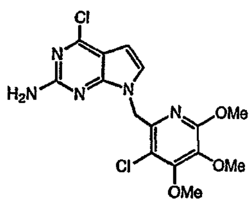,
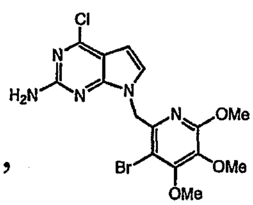,
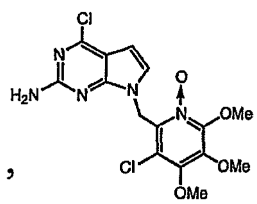
and
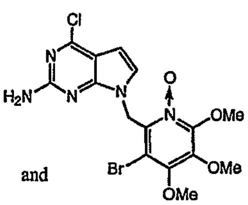

36. The compound of (item 30, wherein said compound is a member selected from the group below, or a polymorph, solvate, ester, tautomer, enantiomer, pharmaceutically acceptable salt or prodrug thereof:

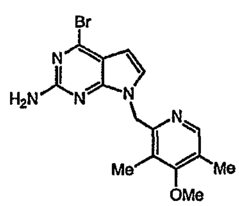,
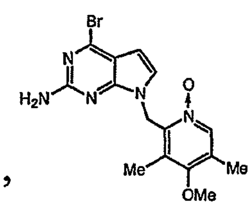,
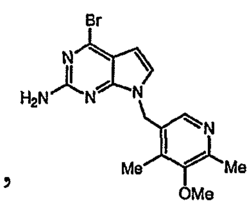,
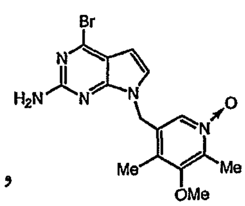,
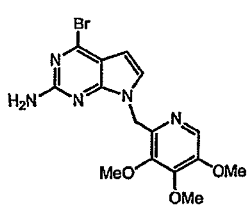,
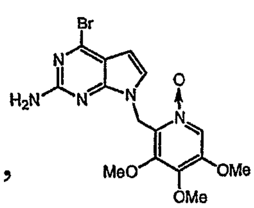,
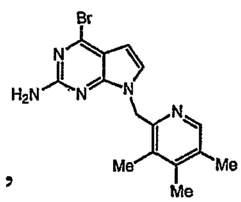,
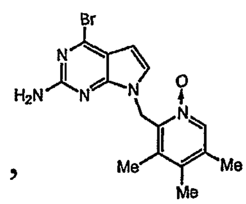,

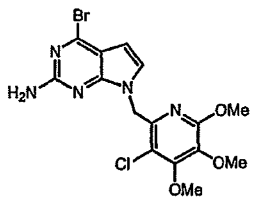,
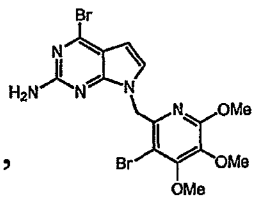,
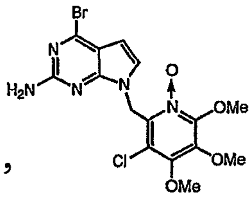
and
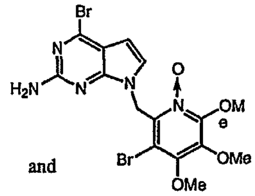

37. The compound of item 30, wherein the compound is a member selected from the group of compounds below, or a polymorph, solvate, ester, tautomer, enantiomer, pharmaceutically acceptable salt or prodrug thereof:

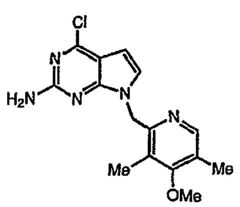,
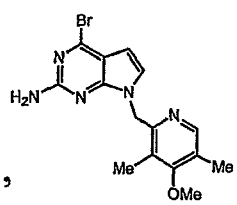,
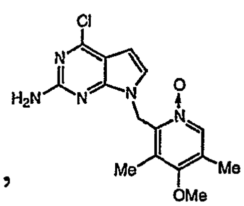,
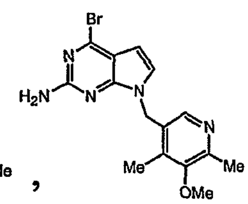,

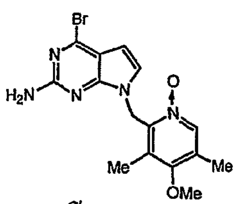,
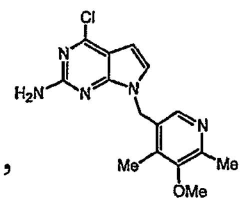,
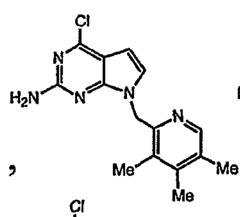,
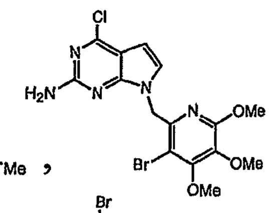,

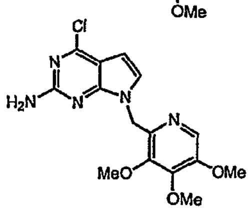,
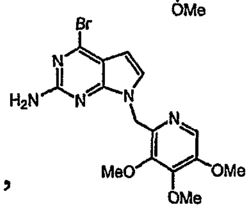,
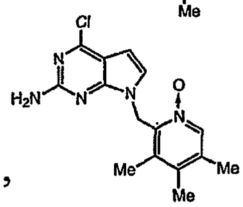,
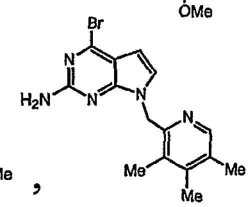,

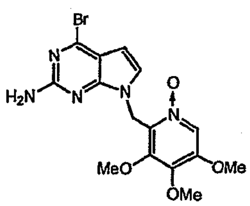,
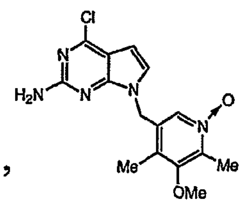,
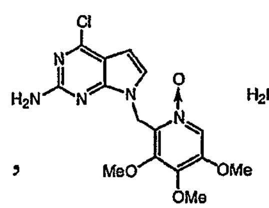
and
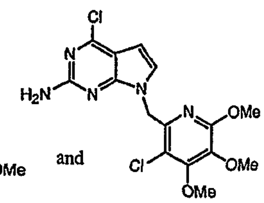

38. The compound of item 30, wherein said compound is a member selected from the group below, or a polymorph, solvate, ester, tautomer, enantiomer, pharmaceutically acceptable salt or prodrug thereof:

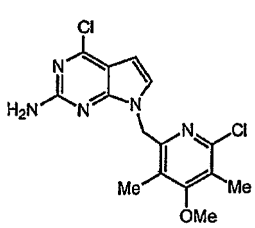,
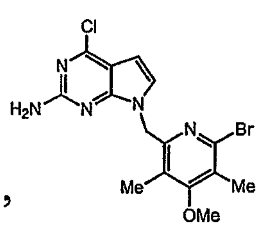,
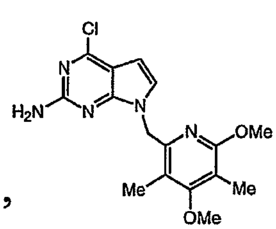,

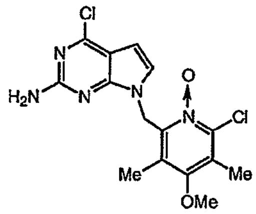 , 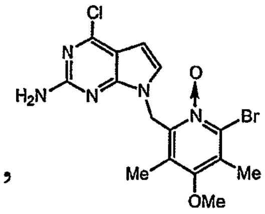 and 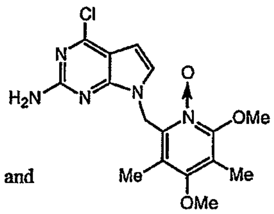

39. The compound of item30, wherein said compound is a member selected from the group below, or a polymorph, solvate, ester, tautomer, enantiomers, pharmaceutically acceptable salt or prodrug thereof:

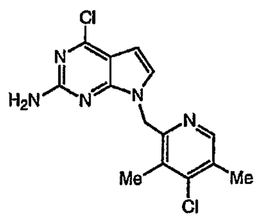 , 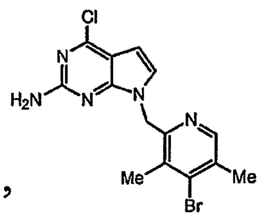 , 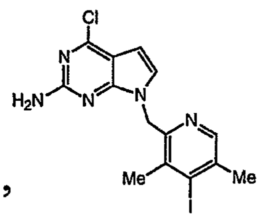 ,

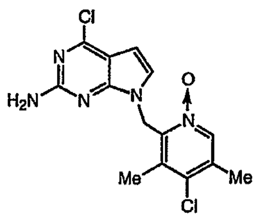 , 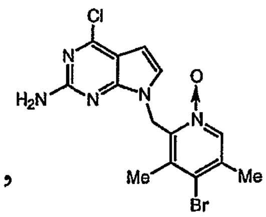 and 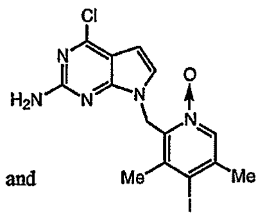

40. The compound of item 30, wherein said compound is a member selected from the group below, or a polymorph, solvate, ester, tautomer, enantiomers, pharmaceutically acceptable salt or prodrug thereof:

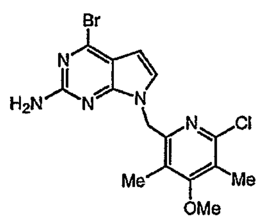 , 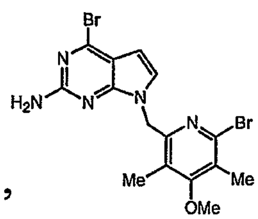 , 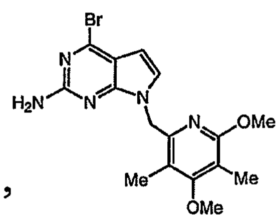 ,

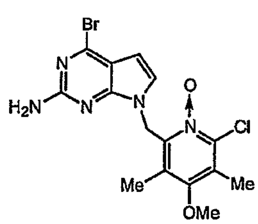 , 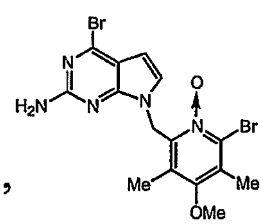 and 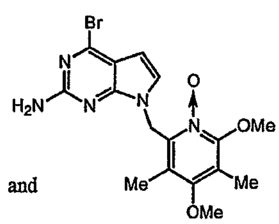

41. The compound of item 30, wherein said compound is a member selected from the group below, or a polymorph, solvate, ester, tautomer, enantiomers, pharmaceutically acceptable salt or prodrug thereof:

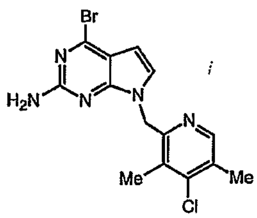, 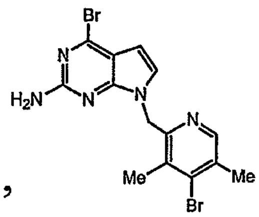, 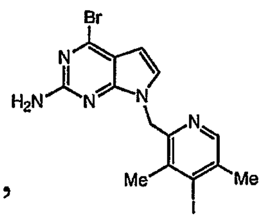,

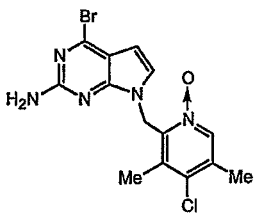, 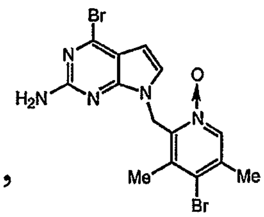 and 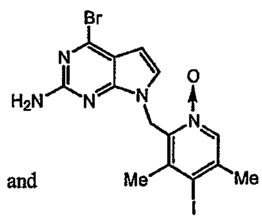

42. The compound of item 30, wherein said compound is a member selected from the group below, or a polymorph, solvate, ester, tautomer, enantiomers, pharmaceutically acceptable salt or prodrug thereof:

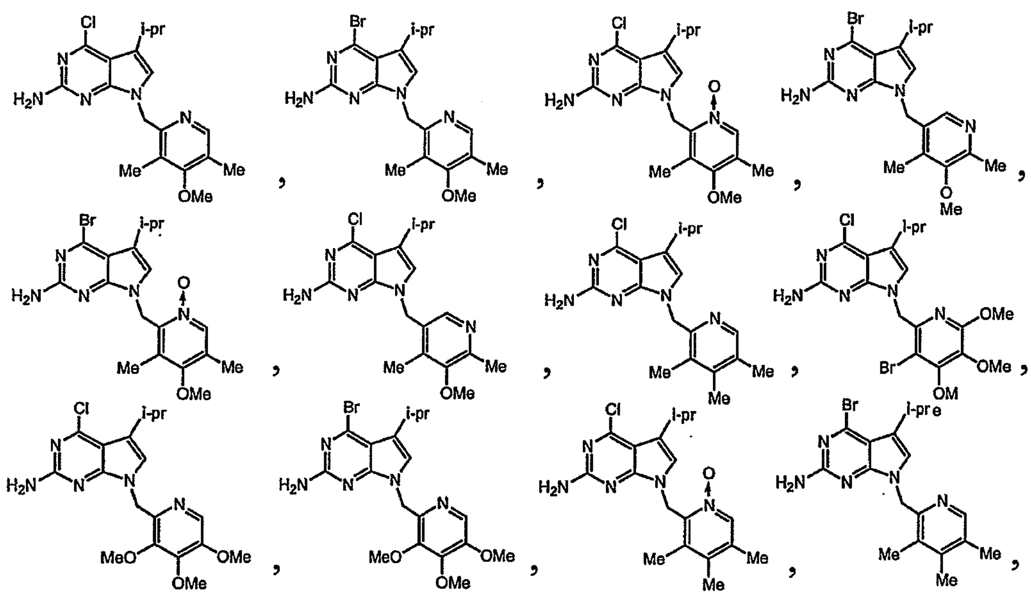

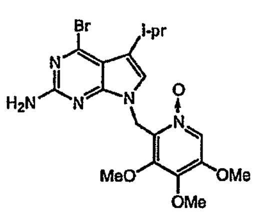, 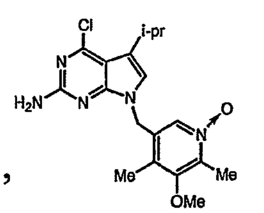, 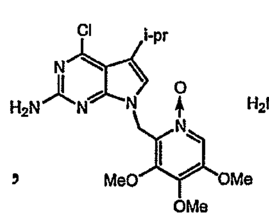 and 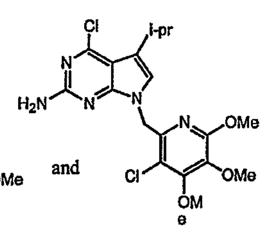

43. The compound of item 30, wherein said compound is a member selected from the group below, or a polymorph, solvate, ester, tautomer, enantiomers, pharmaceutically acceptable salt or prodrug thereof:

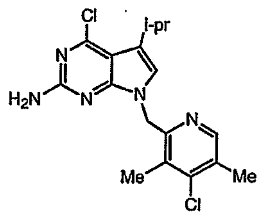 , 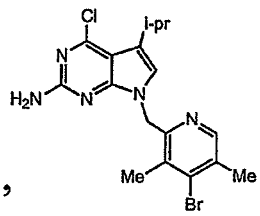 , 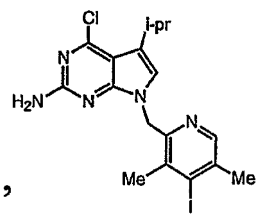 ,

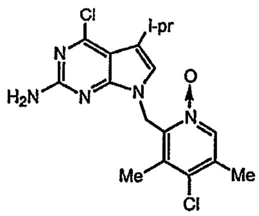 , 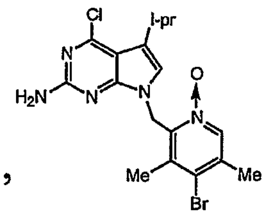 and 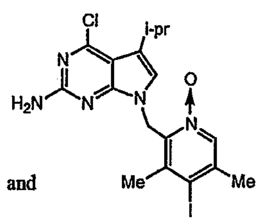

44. The compound of item 30, wherein said compound is a member selected from the group below, or a polymorph, solvate, ester, tautomer, enantiomers, pharmaceutically acceptable salt or prodrug thereof:

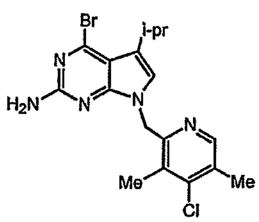 , 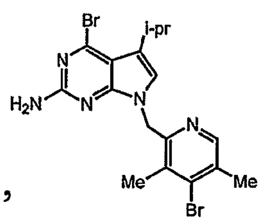 , 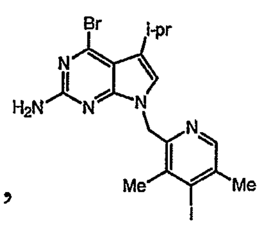 ,

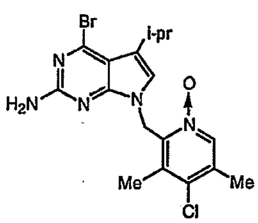 , 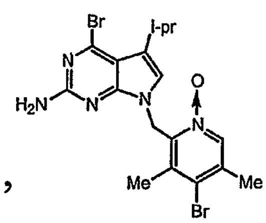 and 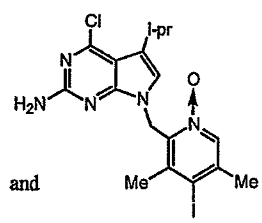

45. The compound of item 30, wherein said compound is a member selected from the group below, or a polymorph, solvate, ester, tautomer, enantiomers, pharmaceutically acceptable salt or prodrug thereof:

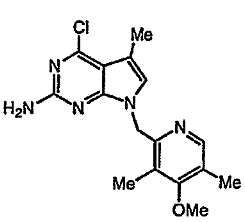 , 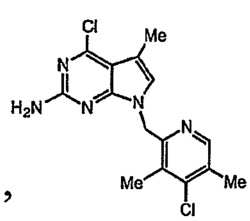 , 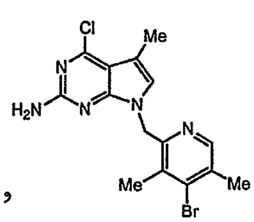 , 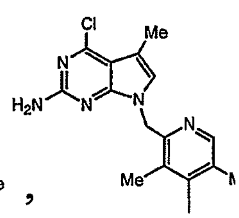

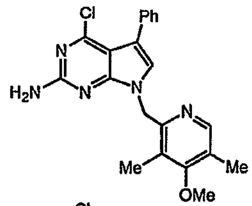 , 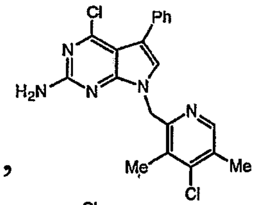 , 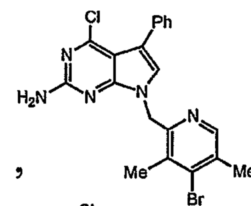 , 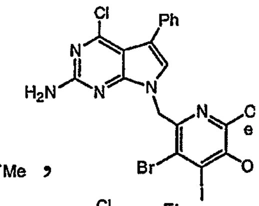

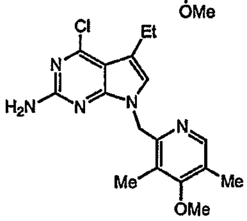 , 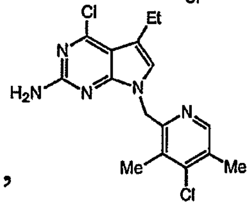 , 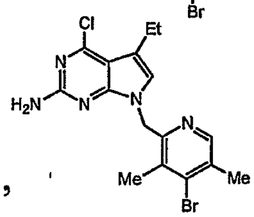 , 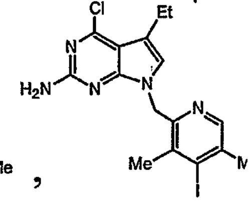

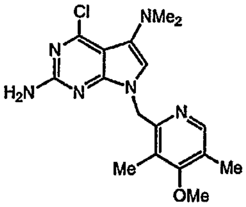 , 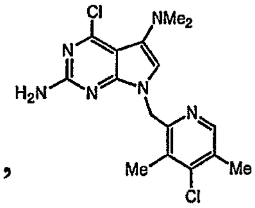 , 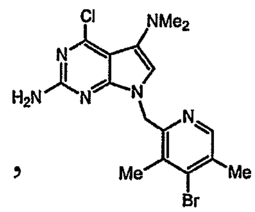 and 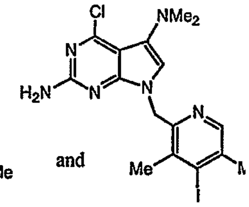

46. The compound of item 30, wherein said compound is a member selected from the group below, or a polymorph, solvate, ester, tautomer, enantiomers, pharmaceutically acceptable salt or prodrug thereof:

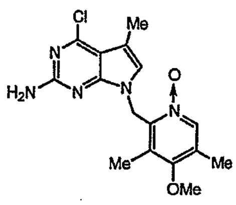 , 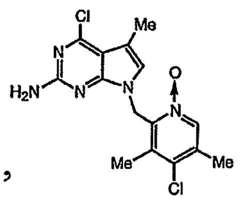 , 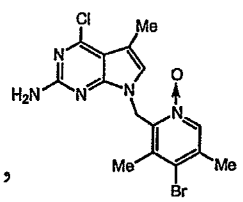 , 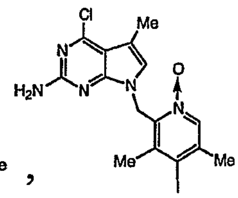 ,

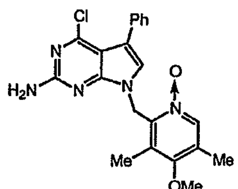 , 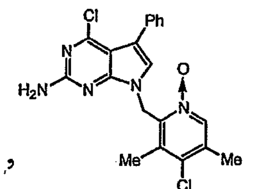 , 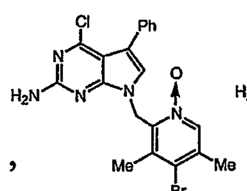 , 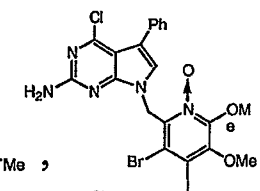 ,

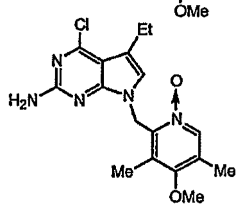 , 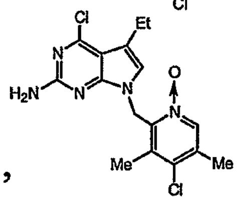 , 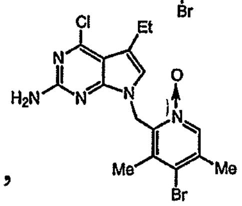 , 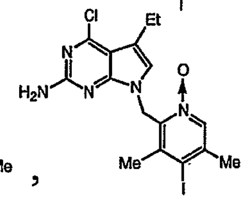 ,

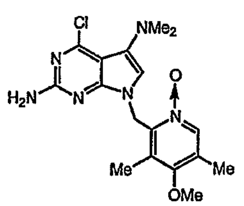 , 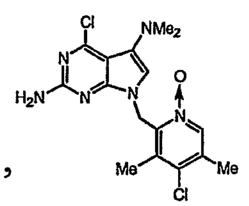 , 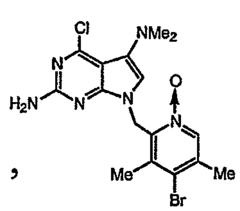 and 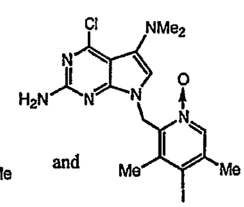

47. The compound of item 30, wherein said compound is a member selected from the group below, or a polymorph, solvate, ester, tautomer, enantiomers, pharmaceutically acceptable salt or prodrug thereof:

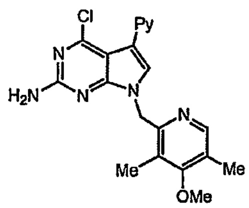 , 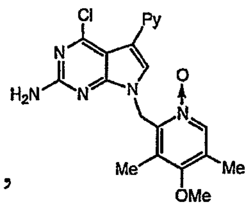 , 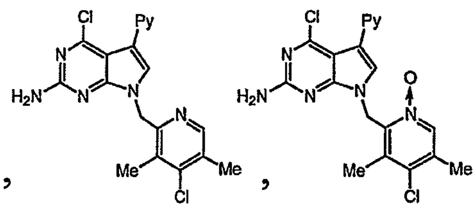

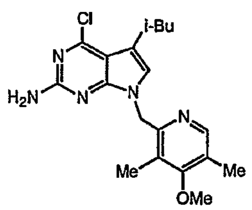 , 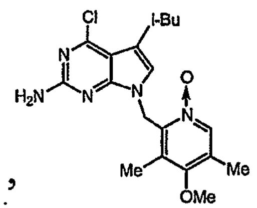 , 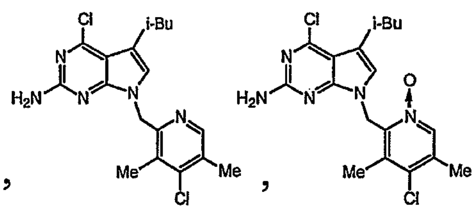

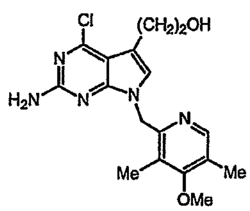 , 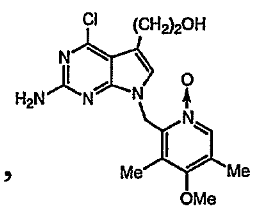 , 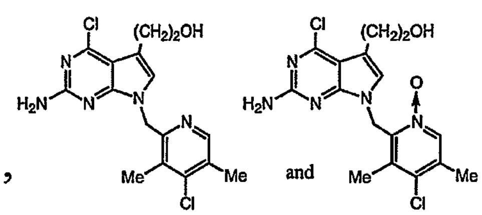

48. The compound of item 30, wherein said compound is a member selected from the group below, or a polymorph, solvate, ester, tautomer, enantiomers, pharmaceutically acceptable salt or prodrug thereof:

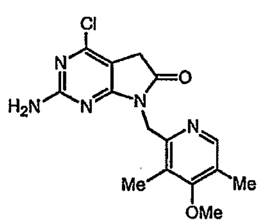 , 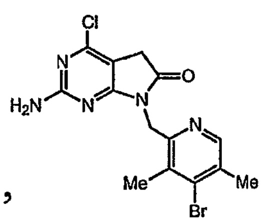 , 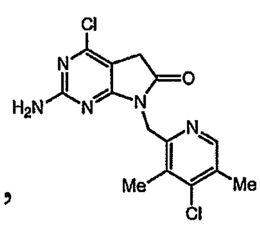 ,

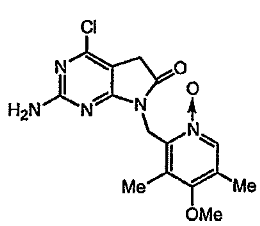 , 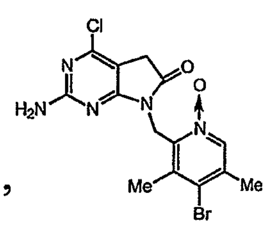 and 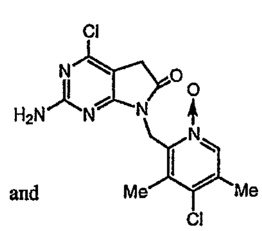

49. The compound of item 30, wherein said compound is represented by the formula below, or a polymorph, solvate, ester, tautomer, pharmaceutically acceptable salt or prodrug thereof:

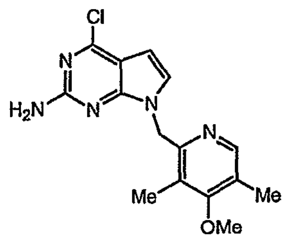

50. The compound of item 30, wherein said compound is represented by the formula below, or a polymorph, solvate, ester, tautomer, pharmaceutically acceptable salt or prodrug thereof:

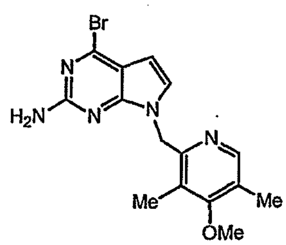

51. The compound of item 30, wherein said compound is represented by the formula below, or a polymorph, solvate, ester, tautomer, pharmaceutically acceptable salt or prodrug thereof:

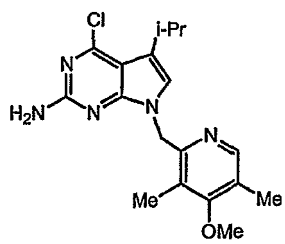

52. The compound of item 30, wherein said compound is represented by the formula below, or a polymorph, solvate, ester, tautomer, pharmaceutically acceptable salt or prodrug thereof:

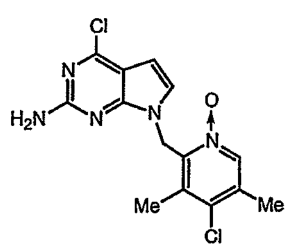

53. The compound of item 30, wherein said compound is represented by the formula below, or a polymorph, solvate, ester, tautomer, pharmaceutically acceptable salt or prodrug thereof:

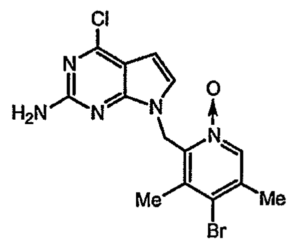

54. The compound of item 30, wherein said compound is represented by the formula below, or a polymorph, solvate, ester, tautomer, pharmaceutically acceptable salt or prodrug thereof:

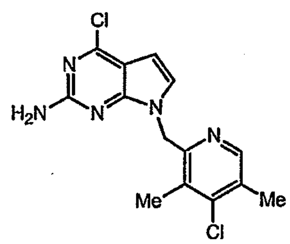

55. The compound of item 30, wherein said compound is represented by the formula below, or a polymorph, solvate, ester, tautomer, pharmaceutically acceptable salt or prodrug thereof:

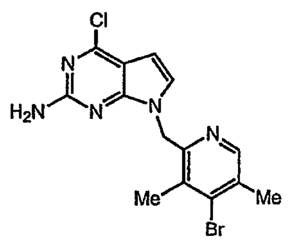

56. The compound of item 30, wherein said compound is represented by the formula below, or a polymorph, solvate, ester, tautomer, pharmaceutically acceptable salt or prodrug thereof:

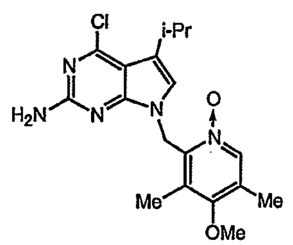

57. The compound of item 30, wherein said compound is represented by the formula below, or a polymorph, solvate, ester, tautomer, pharmaceutically acceptable salt or prodrug thereof:

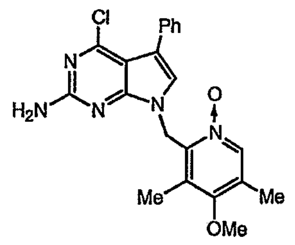

58. The compound of item 30, wherein said compound is represented by the formula below, or a polymorph, solvate, ester, tautomer, pharmaceutically acceptable salt or prodrug thereof:

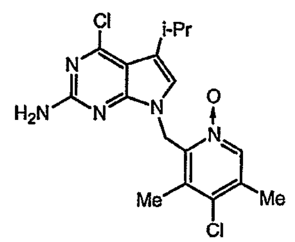

59. The compound of item 30, wherein said compound is represented by the formula below, or a polymorph, solvate, ester, tautomer, pharmaceutically acceptable salt or prodrug thereof:

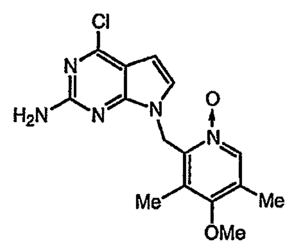

60. The compound of item 30, wherein said compound is represented by the formula below, or a polymorph, solvate, ester, tautomer, pharmaceutically acceptable salt or prodrug thereof:

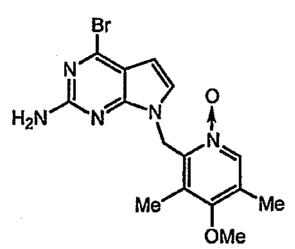

61. The compound of item 30, wherein said compound is represented by the formula below, or a polymorph, solvate, ester, tautomer, pharmaceutically acceptable salt or prodrug thereof:

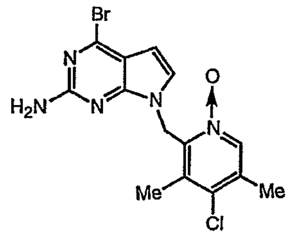

62. The compound of item 30, wherein said compound is represented by the formula below, or a polymorph, solvate, ester, tautomer, pharmaceutically acceptable salt or prodrug thereof:

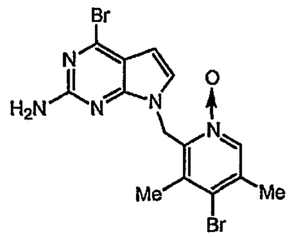

63. The compound of item 30, wherein said compound is represented by the formula below, or a polymorph, solvate, ester, tautomer, pharmaceutically acceptable salt or prodrug thereof:

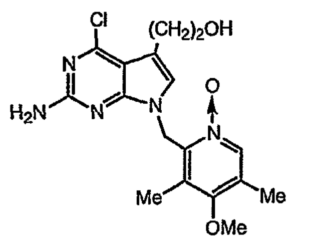

64. The compound of item 30, wherein said compound is represented by the formula below, or a polymorph, solvate, ester, tautomer, pharmaceutically acceptable salt or prodrug thereof:

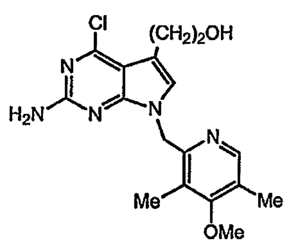

65. The compound of item 30, wherein said compound is represented by the formula below, or a polymorph, solvate, ester, tautomer, pharmaceutically acceptable salt or prodrug thereof:

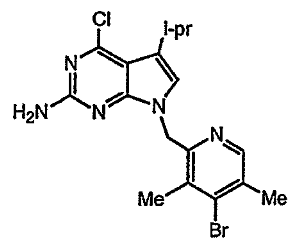

66. The compound of item 30, wherein said compound is represented by the formula below, or a polymorph, solvate, ester, tautomer, pharmaceutically acceptable salt or prodrug thereof:

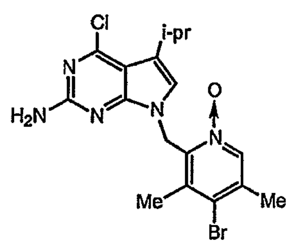

67. The compound of item 30, wherein said compound is represented by the formula below, or a polymorph, solvate, ester, tautomer, pharmaceutically acceptable salt or prodrug thereof:

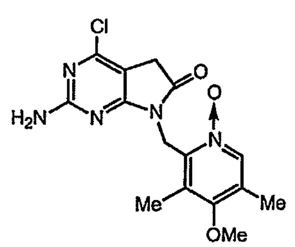

68. A pharmaceutical composition comprising one or more pharmaceutical acceptable excipients and at least one compound represented by Formula IB below, or a polymorph, solvate, ester, tautomer, enantiomer, pharmaceutically acceptable salt or prodrug thereof:

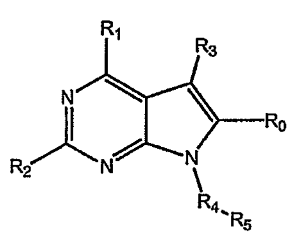

IB

wherein:

$R^0$ is selected from hydrogen, halogen, lower alkyl, $-SR^8$, $-OR^8$, -CN, and $-NHR^8$,
$R^1$ is halogen, $-OR^{11}$, $-SR^{11}$ or lower alkyl;

$R^2$ is -NHR$^8$;
$R^3$ is selected from the group consisting of hydrogen, halogen, -SR$^8$, -OR$^8$, -CN, - C(O)R$^9$, -C(O)OH, -$NO_2$, -NR$^8$R$^{10}$, lower alkyl, lower alkenyl, lower alkynyl, lower perhaloalkyl, aryl, heteroaryl, alicyclic and heterocyclic, all optionally substituted, wherein:
the aryl, heteroaryl, alicyclic and heterocyclic groups are optionally mono-, bi- or tri-cyclic,
$R^8$ and $R^{10}$ taken together optionally form a ring of 3-7 ring atoms and optionally 1-3 of the ring atoms are heteroatoms selected from the group of O, S and N, and
the optional substituents on $R^3$ are selected from the group consisting of halogen, lower alkyl, lower alkenyl, lower alkynyl, -SR$^8$, -OR$^8$, -CN, -C(O)OH, -C(O)R$^9$, -$NO_2$, -NR$^8$R$^{10}$, lower aryl, heteroaryl, alicyclic, lower heterocyclic, arylalkyl, heteroarylalkyl, amino, alkylamino, dialkylamino, diarylalkylamino, oxo, oxa, perhaloalkyl, perhaloalkoxy, perhaloacyl, guanidine, pyridinyl, thiophene, furanyl, indole, indazole, phosphonates, phosphates, phosphoramides, sulfonates, sulfones, sulfates, sulphonamides, carbamates, ureas, thioureas and thioamides, wherein $R^8$ and $R^{10}$ taken together optionally form a ring of 3-7 ring atoms and optionally 1-3 of the ring atoms are heteroatoms selected from the group of 0, S and N;
$R^0$ or $R^3$ is -OH or -SH, the compound may exist as the corresponding (thio)keto tautomer or a mixture of keto-enol tautomers;
$R^4$ is -CHR$^{12}$-, -C(O)-, -C(S)-, -S(O)- or -$SO_2$-;
$R^5$ is aryl, heteroaryl, alicyclic, or heterocyclic, wherein the aryl group is substituted with 3 to 5 substituents, the heteroaryl group is substituted with 2 to 5 substituents, the alicyclic group is substituted with 3 to 5 substituents, the heterocyclic group is substituted with 3 to 5 substituents, and the substituents are selected from the group consisting of halogen, lower alkyl, lower alkenyl, lower alkynyl, -SR$^8$, -OR$^8$, -CN, -C(O)OH, -C(O)R$^9$, -$NO_2$, -NR$^8$R$^{10}$, lower aryl, heteroaryl, alicyclic, lower heterocyclic, arylalkyl, heteroarylalkyl, amino, alkylamino, dialkylamino, diarylalkylamino, oxo, oxa, perhaloalkyl, perhaloalkoxy, perhaloacyl, guanidine, pyridinyl, thiophene, furanyl, indole, indazole, phosphonates, phosphates, phosphoramides, sulfonates, sulfones, sulfates, sulphonamides, carbamates, ureas, thioureas and thioamides, wherein $R^8$ and $R^{10}$ taken together optionally form a ring of 3-7 ring atoms and optionally 1-3 of the ring atoms are heteroatoms selected from the group of O, S and N;
$R^8$ is hydrogen, lower alkyl, lower alkenyl, or lower alkynyl, lower aryl, lower heteroaryl, or -C(O)R$^9$;
$R^9$ is H, lower alkyl, lower alkenyl, lower alkynyl, lower aryl, lower heteroaryl, -NR$^{10}$R$^{10}$, or -OR$^{11}$ , wherein $R^{10}$ and $R^{10}$ taken together optionally form a ring of 3-7 ring atoms and optionally 1-3 of the ring atoms are heteroatoms selected from the group of O, S and N;
$R^{10}$ is hydrogen, lower alkyl, lower alkenyl, lower alkynyl, lower aryl or lower heteroaryl;
$R^{11}$ is lower alkyl, lower alkenyl, lower alkynyl, lower heteroaryl or lower aryl; and
$R^{12}$ is hydrogen or lower alkyl.

69. The pharmaceutical composition of item 68, wherein:

$R^0$ is hydrogen, halogen, -SH, -OH, or -CN,
$R^1$ is halogen; and
$R^2$ is -NHR$^8$, where $R^8$ is hydrogen or -C(O)R$^9$.

70. The pharmaceutical composition of item 68, wherein:

$R^0$ is hydrogen, halogen or -CN,
$R^2$ is -NHR$^8$, where $R^8$ is hydrogen or -C(O)R$^9$; and
$R^4$ is -$CH_2$-.

71. The pharmaceutical composition of item 68, wherein:

$R^0$ is hydrogen, halogen, -SH, -OH or -CN;
$R^1$ is halogen;
$R^2$ is $NH_2$;
$R^3$ is hydrogen, halogen, OR$^8$, SR$^8$, NR$^8$R$^{10}$, lower alkyl, lower alkenyl, lower alkynyl, lower perhaloalkyl, lower aryl, or lower heteroaryl, wherein $R^8$ is hydrogen, lower alkyl, lower aryl, or -C (O)R$^9$;
$R^4$ is -$CH_2$-; and
$R^5$ is aryl or heteroaryl, wherein each of said aryl and heteroaryl is monocyclic or bicyclic and is substituted with 3 to 5 substituents.

72. The pharmaceutical composition of item 68, or a polymorph, wherein:

$R^1$ is chloro or bromo;
$R^2$ is $-NH_2$; and
$R^5$ is a phenyl having at least three substituents, a pyridyl having at least two substituents, or 1-oxy-pyridyl (N-oxy-pyridyl) having at least two substituents.

73. A method of treating an individual having an HSP90 mediated disorder comprising administering to said individual a pharmaceutical composition comprising a pharmaceutically effective amount of a compound of Formula IB:

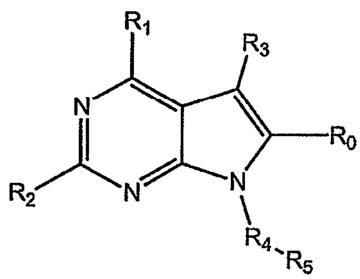

IB

or a polymorph, solvate, ester, tautomer, enantiomer, pharmaceutically acceptable salt or prodrug thereof, wherein:

$R^0$ is selected from hydrogen, halogen, lower alkyl, $-SR^8$, $-OR^8$, -CN, and $-NHR^8$,
$R^1$ is halogen, $-OR^{11}$, $-SR^{11}$ or lower alkyl;
$R^2$ is $-NHR^8$;
$R^3$ is selected from the group consisting of hydrogen, halogen, $-SR^8$, $-OR^8$, -CN,-C(O)$R^9$, -C(O)OH, $-NO_2$, $-NR^8R^{10}$, lower alkyl, lower alkenyl, lower alkynyl, lower perhaloalkyl, aryl, heteroaryl, alicyclic and heterocyclic, all optionally substituted, wherein:
the aryl, heteroaryl, alicyclic and heterocyclic groups are optionally mono-, bi- or tri-cyclic,
$R^8$ and $R^{10}$ taken together optionally form a ring of 3-7 ring atoms and optionally 1-3 of the ring atoms are heteroatoms selected from the group of O, S and N, and
the optional substituents on $R^3$ are selected from the group consisting of halogen, lower alkyl, lower alkenyl, lower alkynyl, $-SR^8$, $-OR^8$, -CN, -C(O)OH, -C(O)$R^9$, $-NO_2$, $-NR^8R^{10}$, lower aryl, heteroaryl, alicyclic, lower heterocyclic, arylalkyl, heteroarylalkyl, amino, alkylamino, dialkylamino, diarylalkylamino, oxo, oxa, perhaloalkyl, perhaloalkoxy, perhaloacyl, guanidine, pyridinyl, thiophene, furanyl, indole, indazole, phosphonates, phosphates, phosphoramides, sulfonates, sulfones, sulfates, sulphonamides, carbamates, ureas, thioureas and thioamides, wherein $R^8$ and $R^{10}$ taken together optionally form a ring of 3-7 ring atoms and optionally 1-3 of the ring atoms are heteroatoms selected from the group of O, S and N;
$R^0$ or $R^3$ is -OH or -SH, the compound may exist as the corresponding (thio)keto tautomer or a mixture of keto-enol tautomers;
$R^4$ is $-CHR^{12}$-, -C(O)-, -C(S)-, -S(O)- or $-SO_2$-;
$R^5$ is aryl, heteroaryl, alicyclic, or heterocyclic, wherein
the aryl group is substituted with 3 to 5 substituents,
the heteroaryl group is substituted with 2 to 5 substituents,
the alicyclic group is substituted with 3 to 5 substituents,
the heterocyclic group is substituted with 3 to 5 substituents, and
the substituents are selected from the group consisting of halogen, lower alkyl, lower alkenyl, lower alkynyl, $-SR^8$, $-OR^8$, -CN, -C(O)OH, -C(O)$R^9$, $-NO_2$, $-NR^8R^{10}$, lower aryl, heteroaryl, alicyclic, lower heterocyclic, arylalkyl, heteroarylalkyl, amino, alkylamino, dialkylamino, diarylalkylamino, oxo, oxa, perhaloalkyl, perhaloalkoxy, perhaloacyl, guanidine, pyridinyl, thiophene, furanyl, indole, indazole, phosphonates, phosphates, phosphoramides, sulfonates, sulfones, sulfates, sulphonamides, carbamates, ureas, thioureas and thioamides, wherein $R^8$ and $R^{10}$ taken together optionally form a ring of 3-7 ring atoms and optionally 1-3 of the ring atoms are heteroatoms selected from the group of O, S and N;
$R^8$ is hydrogen, lower alkyl, lower alkenyl, or lower alkynyl, lower aryl, lower heteroaryl, or -C(O)$R^9$;
$R^9$ is H, lower alkyl, lower alkenyl, lower alkynyl, lower aryl, lower heteroaryl, $NR^{10}R^{10}$, or $-OR^{11}$, wherein $R^{10}$ and $R^{10}$ taken together optionally form a ring of 3-7 ring atoms and optionally 1-3 of the ring atoms are heteroatoms selected from the group of O, S and N;

$R^{10}$ is hydrogen, lower alkyl, lower alkenyl, lower alkynyl, lower aryl or lower heteroaryl;
$R^{11}$ is lower alkyl, lower alkenyl, lower alkynyl, lower heteroaryl or lower aryl; and
$R^{12}$ is hydrogen or lower alkyl.

74. The method of item 73, wherein:

$R^0$ is hydrogen, halogen, -SH, -OH, or -CN,
$R^1$ is halogen; and
$R^2$ is -NHR$^8$, where $R^8$ is hydrogen or -C(O)R$^9$,

75. The method of item 73, wherein:

$R^1$ is chloro or bromo,
$R^2$ is -NHR$^8$, where $R^8$ is hydrogen or -C(O)R$^9$,
$R^3$ is hydrogen, halogen, OR$^8$, SR$^8$, NR$^8$R$^{10}$, lower alkyl, lower alkenyl, lower alkynyl, lower perhaloalkyl, lower aryl, or lower heteroaryl.

76. The method of item 73, wherein:

$R^0$ is hydrogen, halogen or -CN,
$R^2$ is -NHR$^8$, where $R^8$ is hydrogen or -C(O)R$^9$; and
$R^4$ is -CH$_2$-.

77. The method of item 73, wherein:

$R^0$ is hydrogen, halogen, -SH, -OH or -CN;
$R^1$ is halogen;
$R^2$ is $NH_2$;
$R^3$ is hydrogen, halogen, -OR$^8$, -SR$^8$, -NR$^8$R$^8$, lower alkyl, lower alkenyl, lower alkynyl, lower perhaloalkyl, lower aryl, or lower heteroaryl, wherein $R^8$ is hydrogen, lower alkyl, lower aryl, or -C(O)R$^9$;
$R^4$ is -CH$_2$-; and
$R^5$ is aryl or heteroaryl, wherein each of said aryl and heteroaryl is monocyclic or bicyclic and is substituted with 3 to 5 substituents.

78. The method of item 77, wherein:

$R^1$ is chloro or bromo;
$R^2$ is -NH$_2$; and
$R^5$ is a phenyl having at least three substituent, a pyridyl having at least two substituent, or 1-oxy-pyridyl (N-oxy-pyridyl) having at least two substituents.

79. The method of item 73, wherein the HSP90 mediated disorder is selected from the group of inflammatory diseases, infections, autoimmune disorders, stroke, ischemia, cardiac disorder, neurological disorders, fibrogenetic disorders, proliferative disorders, tumors, leukemias, neoplasms, cancers, carcinomas, metabolic diseases, and malignant disease.

80. The method of item 79 wherein the fibrogenetic disorder is further selected from the group of scleroderma, polymyositis, systemic lupus, rheumatoid arthritis, liver cirrhosis, keloid formation, interstitial nephritis and pulmonary fibrosis.

81. The method of item 73, further comprising administering at least one therapeutic agent selected from the group of cytotoxic agents, anti-angiogenesis agents and anti-neoplastic agents.

82. The method of item 81, wherein the at least one anti-neoplastic agent is selected from the group of alkylating agents, anti-metabolites, epidophyllotoxins; antineoplastic enzymes, topoisomerase inhibitors, procarbazines, mitoxantrones, platinum coordination complexes, biological response modifiers and growth inhibitors, hormonal/anti-hormonal therapeutic agents, and haematopoietic growth factors.

83. A compound represented by Formula IC, or a polymorph, solvate, ester, tautomer, enantiomer, pharmaceutically acceptable salt or prodrug thereof:

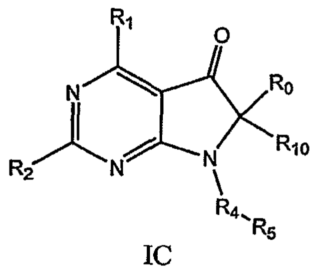

IC

wherein:

$R^0$ is hydrogen, halogen, lower alkyl, $-SR^8$, $-OR^8$, -CN or $-NHR^8$;
$R^1$ is halogen, $-OR^{11}$, $-SR^{11}$ or lower alkyl;
$R^2$ is $-NH_2$;
$R^4$ is $-CHR^{12}-$, -C(O)-, -C(S)-, -S(O)- or $-SO_2-$;
$R^5$ is aryl, heteroaryl, alicyclic, or heterocyclic, wherein:

the aryl group is substituted with 3 to 5 substituents,
the heteroaryl group is substituted with 2 to 5 substituents,
the alicyclic group is substituted with 3 to 5 substituents,
the heterocyclic group is substituted with 3 to 5 substituents, and
the substituents on $R^5$ are selected from the group consisting of halogen, lower alkyl, lower alkenyl, lower alkynyl, $-SR^8$, $-OR^8$, -CN, -C(O)OH, $-C(O)R^9$, $-NO_2$, $-NR^8R^{10}$, lower aryl, heteroaryl, alicyclic, lower heterocyclic, arylalkyl, heteroarylalkyl, amino, alkylamino, dialkylamino, diarylalkylamino, oxo, oxa, perhaloalkyl, perhaloalkoxy, perhaloacyl, guanidine, pyridinyl, thiophene, furanyl, indole, indazole, phosphonates, phosphates, phosphoramides, sulfonates, sulfones, sulfates, sulphonamides, carbamates, ureas, thioureas and thioamides, wherein when $R^8$ and $R^{10}$ taken together optionally form a ring of 3-7 ring atoms and optionally 1-3 of the ring atoms are heteroatoms selected from the group of O, S and N;

$R^8$ is hydrogen, lower alkyl, lower alkenyl, lower alkynyl, lower aryl, lower heteroaryl, or $-C(O)R^9$;
$R^9$ is H, lower alkyl, lower aryl, lower heteroaryl, $-NR^{10}R^{10}$, or $-OR^{11}$, wherein
$R^{10}$ and $R^{10}$ taken together optionally form a ring of 3-7 ring atoms and optionally 1-3 of the ring atoms are heteroatoms selected from the group of O, S and N;
$R^{10}$ is hydrogen, lower alkyl, lower heteroaryl, lower aryl, lower alkenyl, or lower alkynyl;
$R^{11}$ is lower alkyl, lower alkenyl, lower alkynyl, lower heteroaryl or lower aryl;
$R^{12}$ is hydrogen or lower alkyl; and
$R^0$ and $R^{10}$ taken together optionally form an exocyclic double bond which is optionally substituted, or optionally form a ring of 3-7 ring atoms and optionally 1-3 of the ring atoms are heteroatoms selected from the group of O, S and N.

84. The compound of item 83, or a polymorph, solvate, ester, tautomer, enantiomer, pharmaceutically acceptable salt or prodrug thereof, wherein:

$R^1$ is halogen or lower alkyl;
$R^4$ is $-CHR^{12}-$; and
$R^5$ is aryl or heteroaryl, wherein each of said aryl and heteroaryl is monocyclic or bicyclic and is substituted with 3 to 5 substituents.

85. The compound of item 84, or a polymorph, solvate, ester, tautomer, enantiomer, pharmaceutically acceptable salt or prodrug thereof, wherein

$R^0$ is hydrogen or $-NHR_8$;
$R^1$ is halogen, $-OR^{11}$, $-SR^{11}$ or lower alkyl;

$R^{10}$ is hydrogen or lower alkyl.

86. The compound of item 84, or a polymorph, solvate, ester, tautomer, enantiomer, pharmaceutically acceptable salt or prodrug thereof, wherein:

$R^0$ is hydrogen;
$R^1$ is halogen;
$R^4$ is $-CH_2-$;
$R^5$ is aryl or heteroaryl, wherein each of said aryl and heteroaryl is monocyclic or bicyclic and is substituted with 3 to 5 substituents; and
$R^{10}$ is hydrogen.

87. The compound of item 86, or a polymorph, solvate, ester, tautomer, enantiomer, pharmaceutically acceptable salt or prodrug thereof, wherein: $R^1$ is chloro or bromo; $R^5$ is phenyl, pyridyl or 1-oxy-pyridyl (N-oxy-pyridyl), each of which has at least two substituents.

88. The compound of item 86, wherein said compound is a member selected from the group below, or a polymorph, solvate, ester, tautomer, enantiomers, pharmaceutically acceptable salt or prodrug thereof:

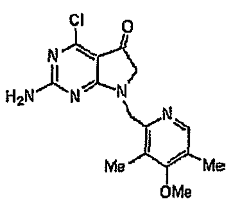 , 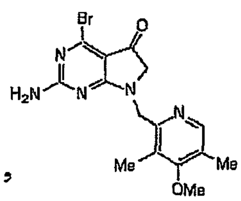 , 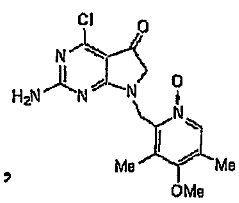 , 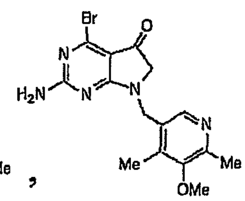 ,

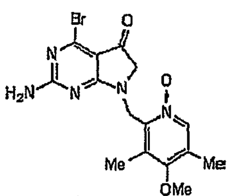 , 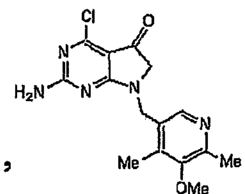 , 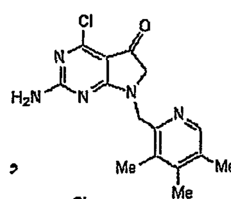 , 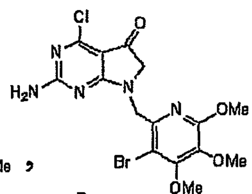 ,

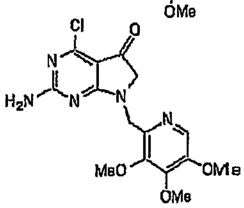 , 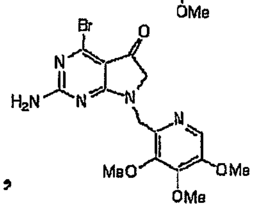 , 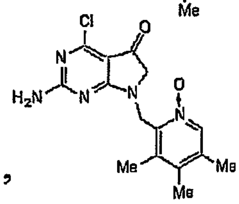 , 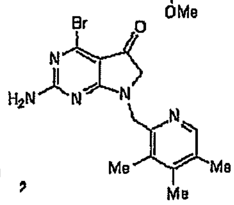 ,

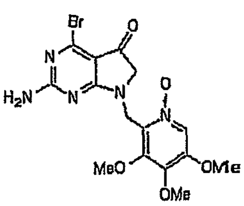 , 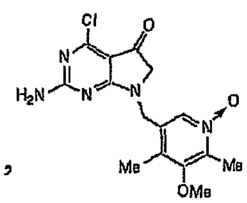 , 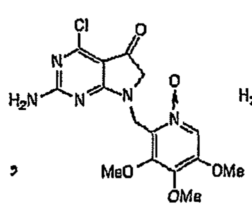 and 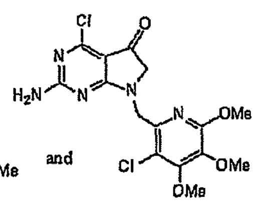

89. The compound of item 86, wherein said compound is a member selected from the group below, or a polymorph, solvate, ester, tautomer, enantiomers, pharmaceutically acceptable salt or prodrug thereof:

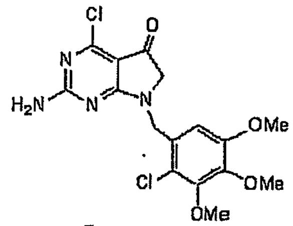, 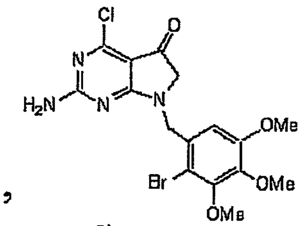, 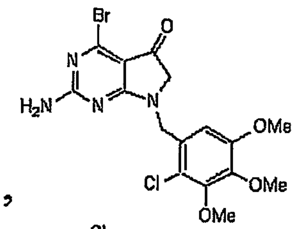, 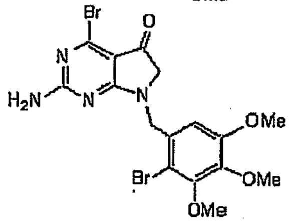, 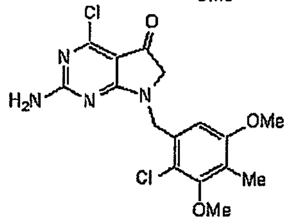, 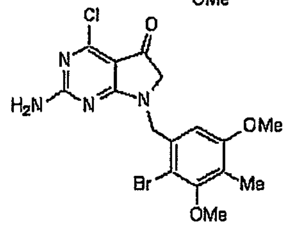, 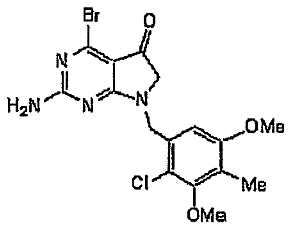, 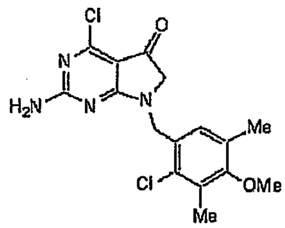 and 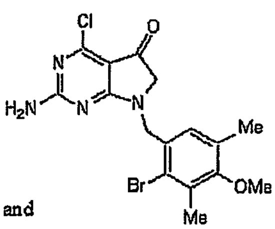

90. The compound of item 86, wherein said compound is a member selected from the group below, or a polymorph, solvate, ester, tautomer, enantiomers, pharmaceutically acceptable salt or prodrug thereof:

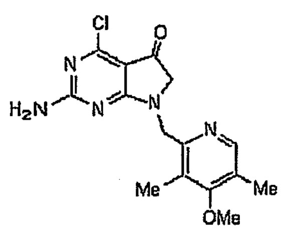 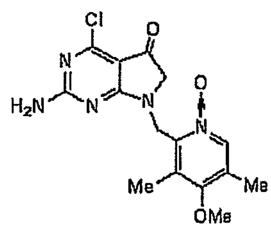

91. The compound of item 86, wherein said compound is a member selected from the group below, or a polymorph, solvate, ester, tautomer, enantiomers, pharmaceutically acceptable salt or prodrug thereof:

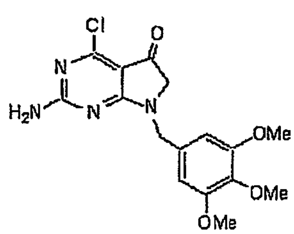 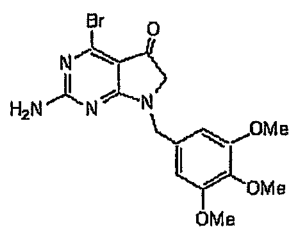

92. A pharmaceutical composition comprising one or more pharmaceutical acceptable excipients and at least one compound represented by Formula IC below, or a polymorph, solvate, ester, tautomer, enantiomer, pharmaceutically acceptable salt or prodrug thereof:

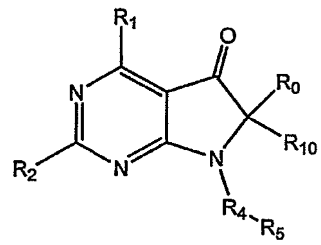

IC

wherein:

$R^0$ is hydrogen, halogen, lower alkyl, $-SR^8$, $-OR^8$, -CN or $-NHR^8$;
$R^1$ is halogen, $-OR^{11}$, $-SR^{11}$ or lower alkyl;
$R^2$ is $-NH_2$;
$R^4$ is $-CHR^{12}$-, -C(O)-, -C(S)-, -S(O)- or $-SO_2$-;
$R^5$ is aryl, heteroaryl, alicyclic, or heterocyclic, wherein:

the aryl group is substituted with 3 to 5 substituents,
the heteroaryl group is substituted with 2 to 5 substituents,
the alicyclic group is substituted with 3 to 5 substituents,
the heterocyclic group is substituted with 3 to 5 substituents, and
the substituents on $R^5$ are selected from the group consisting of halogen, lower alkyl, lower alkenyl, lower alkynyl, $-SR^8$, $-OR^8$, -CN, -C(O)OH,- $C(O)R^9$, $-NO_2$, $-NR^8R^{10}$, lower aryl, heteroaryl, alicyclic, lower heterocyclic, arylalkyl, heteroarylalkyl, amino, alkylamino, dialkylamino, diarylalkylamino, oxo, oxa, perhaloalkyl, perhaloalkoxy, perhaloacyl, guanidine, pyridinyl, thiophene, furanyl, indole, indazole, phosphonates, phosphates, phosphoramides, sulfonates, sulfones, sulfates, sulphonamides, carbamates, ureas, thioureas and thioamides, wherein when $R^8$ and $R^{10}$ taken together optionally form a ring of 3-7 ring atoms and optionally 1-3 of the ring atoms are heteroatoms selected from the group of O, S and N;

$R^8$ is hydrogen, lower alkyl, lower alkenyl, lower alkynyl, lower aryl, lower heteroaryl, or $-C(O)R^9$;
$R^9$ is H, lower alkyl, lower aryl, lower heteroaryl, $-NR^{10}R^{10}$, or $-OR^{11}$, wherein
$R^{10}$ and $R^{10}$ taken together optionally form a ring of 3-7 ring atoms and optionally 1-3 of the ring atoms are heteroatoms selected from the group of O, S and N;
$R^{10}$ is hydrogen, lower alkyl, lower heteroaryl, lower aryl, lower alkenyl, or lower alkynyl,
$R^{11}$ is lower alkyl, lower alkenyl, lower alkynyl, lower heteroaryl or lower aryl;
$R^{12}$ is hydrogen or lower alkyl; and
$R^0$ and $R^{10}$ when taken together optionally form an exocyclic double bond which is optionally substituted, or optionally form a ring of 3-7 ring atoms and ptionally 1-3 of the ring atoms are heteroatoms selected from the group of O, S and N.

93. A method of treating an individual having an HSP90 mediated disorder comprising administering to said individual a pharmaceutical composition comprising a pharmaceutically effective amount of a compound of Formula IC:

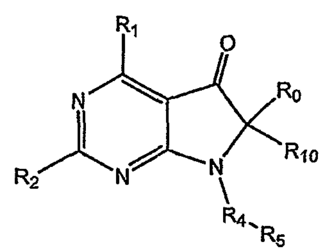

IC

wherein:

$R^0$ is hydrogen, halogen, lower alkyl, $-SR^8$, $-OR^8$,-CN or-$NHR^8$;
$R^1$ is halogen, $-OR^{11}$, $-SR^{11}$ or lower alkyl;
$R^2$ is $NH_2$;
$R^4$ is $-CHR^{12}$-, -C(O)-, -C(S)-, -S(O)- or $-SO_2$-;
$R^5$ is aryl, heteroaryl, alicyclic, or heterocyclic, wherein:

the aryl group is substituted with 3 to 5 substituents,
the heteroaryl group is substituted with 2 to 5 substituents,
the alicyclic group is substituted with 3 to 5 substituents,
the heterocyclic group is substituted with 3 to 5 substituents, and
the substituents on $R^5$ are selected from the group consisting of halogen, lower alkyl, lower alkenyl, lower alkynyl, $-SR^8$, $-OR^8$, -CN, -C(O)OH, $-C(O)R^9$, $-NO_2$ and $-NR^8R^{10}$, lower aryl, heteroaryl, alicyclic, lower heterocyclic, arylalkyl, heteroarylalkyl, amino, alkylamino, dialkylamino, diarylalkylamino, oxo, oxa, perhaloalkyl, perhaloalkoxy, perhaloacyl, guanidine, pyridinyl, thiophene, furanyl, indole, indazole, phosphonates, phosphates, phosphoramides, sulfonates, sulfones, sulfates, sulphonamides, carbamates, ureas, thioureas and thioamides, wherein when $R^8$ and $R^{10}$ taken together optionally form a ring of 3-7 ring atoms and optionally 1-3 of the ring atoms are heteroatoms selected from the group of O, S and N;

$R^8$ is hydrogen, lower alkyl, lower alkenyl, lower alkynyl, lower aryl, lower heteroaryl, or $-C(O)R^9$;
$R^9$ is H, lower alkyl, lower aryl, lower heteroaryl, $-NR^{10}R^{10}$, or $-OR^{11}$, wherein
$R^{10}$ and $R^{10}$ taken together optionally form a ring of 3-7 ring atoms and optionally 1-3 of the ring atoms are heteroatoms selected from the group of O, S and N;
$R^{10}$ is hydrogen, lower alkyl, lower heteroaryl, lower aryl, lower alkenyl, or lower alkynyl,
$R^{11}$ is lower alkyl, lower alkenyl, lower alkynyl, lower heteroaryl or lower aryl;
$R^{12}$ is hydrogen or lower alkyl; and
$R^0$ and $R^{10}$ taken together optionally form an exocyclic double bond which is optionally substituted, or optionally form a ring of 3-7 ring atoms and optionally 1-3 of the ring atoms are heteroatoms selected from the group of O, S and N.

94. A compound represented by Formula ID, or a polymorph, solvate, ester, tautomer, enantiomer, pharmaceutically acceptable salt or prodrug thereof:

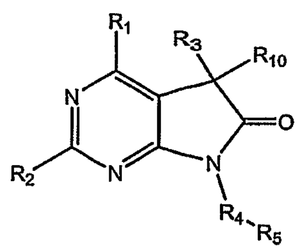

ID

wherein:

$R^1$ is halogen, $-OR^{11}$, $-SR^{11}$ or lower alkyl;
$R^2$ is $-NH_2$;
$R^3$ is selected from the group consisting of hydrogen, halogen, $-SR^8$, $-OR^8$, -CN, $-C(O)R^9$, -C(O)OH, $-NO_2$, $-NR^8R^{10}$, lower alkyl, lower alkenyl, lower alkynyl, lower perhaloalkyl, aryl, heteroaryl, alicyclic, heterocyclic, all optionally substituted, wherein:

the aryl, heteroaryl, alicyclic and heterocyclic groups are optionally mono-, bi- or tri-cyclic, $R^8$ and $R^{10}$ taken together optionally form a ring of 3-7 ring atoms and optionally 1-3 of the ring atoms are heteroatoms selected from the group of O, S and N, and
the optional substituents on $R^3$ are selected from the group consisting of halogen, lower alkyl, lower alkenyl,

lower alkynyl, $-SR^8$, $-OR^8$, -CN, -C(O)OH, $-C(O)R^9$, $-NO_2$, $-NR^8R^{10}$, lower aryl, heteroaryl, alicyclic, lower heterocyclic, arylalkyl, heteroarylalkyl, amino, alkylamino, dialkylamino, diarylalkylamino, oxo, oxa, perhaloalkyl, perhaloalkoxy, perhaloacyl, guanidine, pyridinyl, thiophene, furanyl, indole, indazole, phosphonates, phosphates, phosphoramides, sulfonates, sulfones, sulfates, sulphonamides, carbamates, ureas, thioureas and thioamides, wherein $R^8$ and $R^{10}$ taken together optionally form a ring of 3-7 ring atoms and optionally 1-3 of the ring atoms are heteroatoms selected from the group of O, S and N;
$R^4$ is $-CHR^{12}-$, -C(O)-, -C(S)-, -S(O)- or $-SO_2-$;
$R^5$ is aryl, heteroaryl, alicyclic, or heterocyclic, wherein
the aryl group is substituted with 3 to 5 substituents,
the heteroaryl group is substituted with 2 to 5 substituents,
the alicyclic group is substituted with 3 to 5 substituents,
the heterocyclic group is substituted with 3 to 5 substituents, and
the substituents on $R^5$ are selected from the group consisting of halogen, lower alkyl, lower alkenyl, lower alkynyl, $-SR^8$, $-OR^8$, -CN, -C(O)OH, $-C(O)R^9$, $-NO_2$, $-NR^8R^{10}$, lower aryl, heteroaryl, alicyclic, lower heterocyclic, arylalkyl, heteroarylalkyl, amino, alkylamino, dialkylamino, diarylalkylamino, oxo, oxa, perhaloalkyl, perhaloalkoxy, perhaloacyl, guanidine, pyridinyl, thiophene, furanyl, indole, indazole, phosphonates, phosphates, phosphoramides, sulfonates, sulfones, sulfates, sulphonamides, carbamates, ureas, thioureas and thioamides, wherein $R^8$ and $R^{10}$ taken together optionally form a ring of 3-7 ring atoms and optionally 1-3 of the ring atoms are heteroatoms selected from the group of O, S and N;
$R^8$ is hydrogen, lower alkyl, lower alkenyl, lower alkynyl, lower aryl, lower heteroaryl, or $-C(O)R^9$;
$R^9$ is H, lower alkyl, lower aryl, lower heteroaryl, $-NR^{10}R^{10}$, or $-OR^{11}$, wherein when $R^{10}$ and $R^{10}$ taken together optionally form a ring of 3-7 ring atoms and optionally 1-3 of the ring atoms are heteroatoms selected from the group of O, S and N;
$R^{10}$ is hydrogen, lower alkyl, lower heteroaryl, lower aryl, lower alkenyl, or lower alkynyl,
$R^{11}$ is lower alkyl, lower alkenyl, lower alkynyl, lower heteroaryl or lower aryl;
$R^{12}$ is hydrogen or lower alkyl; and
$R^3$ and $R^{10}$ taken together optionally form an exocyclic double bond which is optionally substituted, or optionally form a ring of 3-7 ring atoms and optionally 1-3 of the ring atoms are heteroatoms selected from the group of O, S and N.

95. The compound of item 94, or a polymorph, solvate, ester, tautomer, enantiomers, pharmaceutically acceptable salt or prodrug thereof, wherein:

$R^1$ is halogen;
$R^3$ is hydrogen, halogen, $-OR^8$, $-SR^8$, $-NR^8R^{10}$, lower alkyl, lower alkenyl, lower alkynyl, lower perhaloalkyl, lower aryl, or lower heteroaryl, wherein $R^8$ is hydrogen, lower alkyl, lower alkenyl, or lower alkynyl, lower aryl, lower heteroaryl, or $-C(O)R^9$;
$R^4$ is $-CH^2-$;
$R^5$ is aryl or heteroaryl, wherein each of said aryl and heteroaryl is monocyclic or bicyclic and is substituted with 3 to 5 substituents, and
$R^{10}$ is hydrogen or lower alkyl.

96. The compound of item 94, or a polymorph, solvate, ester, tautomer, enantiomer, pharmaceutically acceptable salt or prodrug thereof, wherein:

$R^1$ is halogen;
$R^4$ is $-CH_2-$;
$R^5$ is aryl or heteroaryl, wherein each of said aryl and heteroaryl is monocyclic or bicyclic and is substituted with 3 to 5 substituents; and
$R^{10}$ is hydrogen.

97. The compound of item 94, or a polymorph, solvate, ester, tautomer, enantiomer, pharmaceutically acceptable salt or prodrug thereof, wherein:

$R^1$ is halogen;
$R^3$ is hydrogen;
$R^4$ is $-CH_2-$;
$R^5$ is aryl or heteroaryl, wherein each of said aryl and heteroaryl is monocyclic or bicyclic and is substituted

with 3 to 5 substituents; and
$R^{10}$ is hydrogen.

98. The compound of item 97, or a polymorph, solvate, ester, tautomer, enantiomer, pharmaceutically acceptable salt or prodrug thereof, wherein: $R^1$ is chloro or bromo; and $R^5$ is phenyl, pyridyl or 1-oxy-pyridyl (N-oxy-pyridyl), each of which has at least two substituents.

99. A pharmaceutical composition comprising one or more pharmaceutical acceptable excipients and at least one compound represented by Formula ID below, or a polymorph, solvate, ester, tautomer, enantiomer, pharmaceutically acceptable salt or prodrug thereof:

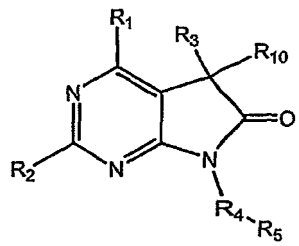

ID

wherein:

$R^1$ is halogen, $-OR^{11}$, $-SR^{11}$ or lower alkyl;
$R^2$ is $-NH_2$;
$R^3$ is selected from the group consisting of hydrogen, halogen, $-SR^8$, $-OR^8$, -CN, $-C(O)R^9$, -C(O)OH, $-NO_2$, $-NR^8R^{10}$, lower alkyl, lower alkenyl, lower alkynyl, lower perhaloalkyl, aryl, heteroaryl, alicyclic, heterocyclic, all optionally substituted, wherein:

the aryl, heteroaryl, alicyclic and heterocyclic groups are optionally mono-, bi- or tri-cyclic,
$R^8$ and $R^{10}$ taken together optionally form a ring of 3-7 ring atoms and optionally 1-3 of the ring atoms are heteroatoms selected from the group of O, S and N, and
the optional substituents on $R^3$ are selected from the group consisting of halogen, lower alkyl, lower alkenyl, lower alkynyl, $-SR^8$, $-OR^8$,-CN, -C(O)OH,
$-C(O)R^9$, $-NO_2$, $-NR^8R^{10}$, lower aryl, heteroaryl, alicyclic, lower heterocyclic, arylalkyl, heteroarylalkyl, amino, alkylamino, dialkylamino, diarylalkylamino, oxo, oxa, perhaloalkyl, perhaloalkoxy, perhaloacyl, guanidine, pyridinyl, thiophene, furanyl, indole, indazole, phosphonates, phosphates, phosphoramides, sulfonates, sulfones, sulfates, sulphonamides, carbamates, ureas, thioureas and thioamides, wherein $R^8$ and $R^{10}$ taken together optionally form a ring of 3-7 ring atoms and optionally 1-3 of the ring atoms are heteroatoms selected from the group of O, S and N;
$R^4$ is $-CHR^{12}-$, -C(O)-, -C(S)-, -S(O)- or $-SO_2-$;
$R^5$ is aryl, heteroaryl, alicyclic, or heterocyclic, wherein
the aryl group is substituted with 3 to 5 substituents,
the heteroaryl group is substituted with 2 to 5 substituents,
the alicyclic group is substituted with 3 to 5 substituents,
the heterocyclic group is substituted with 3 to 5 substituents, and
the substituents on $R^5$ are selected from the group consisting of halogen, lower alkyl, lower alkenyl, lower alkynyl, $-SR^8$, $-OR^8$, -CN, - C(O)OH, $-C(O)R^9$,
$-NO_2$ and $-NR^8R^{10}$, lower aryl, heteroaryl, alicyclic, lower heterocyclic, arylalkyl, heteroarylalkyl, amino, alkylamino, dialkylamino, diarylalkylamino, oxo, oxa, perhaloalkyl, perhaloalkoxy, perhaloacyl, guanidine, pyridinyl, thiophene, furanyl, indole, indazole, phosphonates, phosphates, phosphoramides, sulfonates, sulfones, sulfates, sulphonamides, carbamates, ureas, thioureas and thioamides, wherein $R^8$ and $R^{10}$ taken together optionally form a ring of 3-7 ring atoms and optionally 1-3 of the ring atoms are heteroatoms selected from the group of O, S and N;
$R^8$ is hydrogen, lower alkyl, lower alkenyl, lower alkynyl, lower aryl, lower heteroaryl, or $-C(O)R^9$;

$R^9$ is H, lower alkyl, lower aryl, lower heteroaryl, $-NR^{10}R^{10}$, or $-OR^{11}$, wherein $R^{10}$ and $R^{10}$ taken together optionally form a ring of 3-7 ring atoms and optionally 1-3 of the ring atoms are heteroatoms selected from the group of O, S and N;
$R^{10}$ is hydrogen, lower alkyl, lower heteroaryl, lower aryl, lower alkenyl, or lower alkynyl,
$R^{11}$ is lower alkyl, lower alkenyl, lower alkynyl, lower heteroaryl or lower aryl;
$R^{12}$ is hydrogen or lower alkyl; and
$R^3$ and $R^{10}$ taken together optionally form an exocyclic double bond which is optionally substituted, or optionally form a ring of 3-7 ring atoms and optionally 1-3 of the ring atoms are heteroatoms selected from the group of O, S and N.

100. A method of treating an individual having an HSP90 mediated disorder comprising administering to said individual a pharmaceutical composition comprising a pharmaceutically effective amount of a compound of Formula ID:

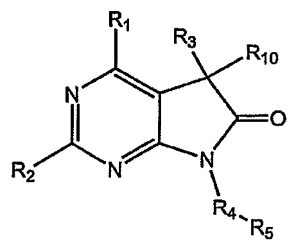

ID

wherein:

$R^1$ is halogen, $-OR^{11}$, $-SR^{11}$ or lower alkyl;
$R^2$ is $NH_{2;}$
$R^3$ is selected from the group consisting of hydrogen, halogen, $-SR^8$, $-OR^8$, -CN, - C(O)$R^9$,
-C(O)OH, $-NO_2$, $-NR^8R^{10}$, lower alkyl, lower alkenyl, lower alkynyl, lower perhaloalkyl, aryl, heteroaryl, alicyclic, heterocyclic, all optionally substituted, wherein:

the aryl, heteroaryl, alicyclic and heterocyclic groups are optionally mono-, bi- or tri-cyclic;
$R^8$ and $R^{10}$ taken together optionally form a ring of 3-7 ring atoms and optionally 1-3 of the ring atoms are heteroatoms selected from the group of O, S and N, and
the optional substituents on $R^3$ are selected from the group consisting of halogen, lower alkyl, lower alkenyl, lower alkynyl, $-SR^8$, $-OR^8$,-CN, -C(O)OH,
-C(O)$R^9$, $-NO_2$, $-NR^8R^{10}$, lower aryl, heteroaryl, alicyclic, lower heterocyclic, arylalkyl, heteroarylalkyl, amino, alkylamino, dialkylamino, diarylalkylamino, oxo, oxa, perhaloalkyl, perhaloalkoxy, perhaloacyl, guanidine, pyridinyl, thiophene, furanyl, indole, indazole, phosphonates, phosphates, phosphoramides, sulfonates, sulfones, sulfates, sulphonamides, carbamates, ureas, thioureas and thioamides, wherein $R^8$ and $R^{10}$ taken together optionally form a ring of 3-7 ring atoms and optionally 1-3 of the ring atoms are heteroatoms selected from the group of O, S and N;

$R^4$ is $-CHR^{12}-$, -C(O)-, -C(S)-, -S(O)- or $-SO_2-$;
$R^5$ is aryl, heteroaryl, alicyclic, or heterocyclic, wherein
the aryl group is substituted with 3 to 5 substituents,
the heteroaryl group is substituted with 2 to 5 substituents,
the alicyclic group is substituted with 3 to 5 substituents,
the heterocyclic group is substituted with 3 to 5 substituents, and
the substituents on $R^5$ are selected from the group consisting of halogen, lower alkyl, lower alkenyl, lower alkynyl, $-SR^8$ $-OR^8$, -CN, - C(O)OH, -C(O)$R^9$,
$-NO_2$ and $-NR^8R^{10}$, lower aryl, heteroaryl, alicyclic, lower heterocyclic, arylalkyl, heteroarylalkyl, amino, alkylamino, diallcylamino, diarylallcylamino, oxo, oxa, perhaloalkyl, perhaloalkoxy, perhaloacyl, guanidine, pyridinyl, thiophene, furanyl, indole, indazole, phosphonates, phosphates, phosphoramides, sulfonates, sulfones, sulfates, sulphonamides, carbamates, ureas, thioureas and thioamides, wherein $R^8$ and $R^{10}$ taken together optionally form a ring of 3-7 ring atoms and optionally 1-3 of the ring atoms are heteroatoms selected from the

group of O, S and N;
$R^8$ is hydrogen, lower alkyl, lower alkenyl, lower alkynyl, lower aryl, lower heteroaryl, or -C(O)$R^9$;
$R^9$ is H, lower alkyl, lower aryl, lower heteroaryl, -$NR^{10}R^{10}$, or -$OR^{11}$, wherein $R^{10}$ and $R^{10}$ taken together optionally form a ring of 3-7 ring atoms and optionally 1-3 of the ring atoms are heteroatoms selected from the group of O, S and N;
$R^{10}$ is hydrogen, lower alkyl, lower heteroaryl, lower aryl, lower alkenyl, or lower alkynyl,
$R^{11}$ is lower alkyl, lower alkenyl, lower alkynyl, lower heteroaryl or lower aryl;
$R^{12}$ is hydrogen or lower alkyl; and
$R^3$ and $R^{10}$ taken together optionally form an exocyclic double bond which is optionally substituted, or optionally form a ring of 3-7 ring atoms and optionally 1-3 of the ring atoms are heteroatoms selected from the group of O, S and N.

101. A compound represented by Formula IE, or a polymorph, solvate, ester, tautomer, enantiomer, pharmaceutically acceptable salt or prodrug thereof:

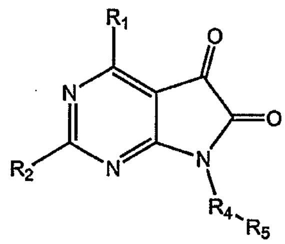

IE

wherein:

$R^1$ is halogen, -$OR^{11}$, -$SR^{11}$ or lower alkyl;
$R^2$ is -$NH_2$;
$R^4$ is -$CHR^{12}$-, -C(O)-, -C(S)-, -S(O)- or -$SO_2$-;
$R^5$ is aryl, heteroaryl, alicyclic, or heterocyclic, wherein
the aryl group is substituted with 3 to 5 substituents,
the heteroaryl group is substituted with 2 to 5 substituents,
the alicyclic group is substituted with 3 to 5 substituents,
the heterocyclic group is substituted with 3 to 5 substituents, and
the substituents on $R^5$ are selected from the group consisting of halogen, lower alkyl, lower alkenyl, lower alkynyl, -$SR^8$, -$OR^8$, -CN, - C(O)OH, -C(O)$R^9$, -$NO_2$, -$NR^8R^{10}$, lower aryl, heteroaryl, alicyclic, lower heterocyclic, arylalkyl, heteroarylalkyl, amino, alkylamino, dialkylamino, diarylalkylamino, oxo, oxa, perhaloalkyl, perhaloalkoxy, perhaloacyl, guanidine, pyridinyl, thiophene, furanyl, indole, indazole, phosphonates, phosphates, phosphoramides, sulfonates, sulfones, sulfates, sulphonamides, carbonate, ureas, thioureas and thioamides, wherein $R^8$ and $R^{10}$ taken together optionally form a ring of 3-7 ring atoms and optionally 1-3 of the ring atoms are heteroatoms selected from the group of O, S and N;
$R^8$ is hydrogen, lower alkyl, lower alkenyl, lower alkynyl, lower aryl, lower heteroaryl, or -C(O)$R^9$;
$R^9$ is H, lower alkyl, lower aryl, lower heteroaryl, -$NR^{10}R^{10}$, or -$OR^{11}$, wherein $R^{10}$ and $R^{10}$ taken together optionally form a ring of 3-7 ring atoms and optionally 1-3 of the ring atoms are heteroatoms selected from the group of O, S and N;
$R^{10}$ is hydrogen, lower alkyl, lower heteroaryl, lower aryl, lower alkenyl, or lower alkynyl,
$R^{11}$ is lower alkyl, lower alkenyl, lower alkynyl, lower heteroaryl or lower aryl; and
$R^{12}$ is hydrogen or lower alkyl.

102. The compound of item 101, or a polymorph, solvate, ester, tautomer, enantiomer, pharmaceutically acceptable salt or prodrug thereof, wherein:

$R^1$ is halogen;
$R^4$ is -$CH_2$-; and

$R^5$ is aryl or heteroaryl, wherein each of said aryl and heteroaryl is monocyclic or bicyclic and is substituted with 3 to 5 substituents.

103. The compound of item 102, or a polymorph, solvate, ester, tautomer, enantiomers, pharmaceutically acceptable salt or prodrug thereof, wherein $R^1$ is chloro or bromo, $R^5$ is phenyl, pyridyl or 1-oxy-pyridyl (N-oxy-pyridyl), each of which has at least two substituents.

104. A pharmaceutical composition comprising one or more pharmaceutical acceptable excipients and at least one compound represented by Formula IE below, or a polymorph, solvate, ester, tautomer, enantiomer, pharmaceutically acceptable salt or prodrug thereof:

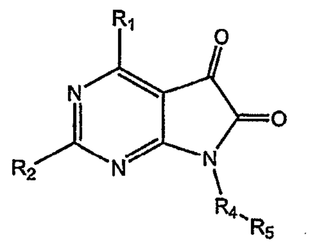

IE

wherein:

$R^1$ is halogen, $-OR^{11}$, $-SR^{11}$ or lower alkyl;
$R^2$ is $-NH_2$;
$R^4$ is $-CHR^{12}-$, -C(O)-, -C(S)-, -S(O)- or $-SO_2-$;
$R^5$ is aryl, heteroaryl, alicyclic, or heterocyclic, wherein the aryl group is substituted with 3 to 5 substituents, the heteroaryl group is substituted with 2 to 5 substituents, the alicyclic group is substituted with 3 to 5 substituents, the heterocyclic group is substituted with 3 to 5 substituents, and the substituents on $R^5$ are selected from the group consisting of halogen, lower alkyl, lower alkenyl, lower alkynyl, $-SR^8$, $-OR^8$, -CN, - C(O)OH, $-C(O)R^9$, $-NO_2$ and $-NR^8R^{10}$, lower aryl, heteroaryl, alicyclic, lower heterocyclic, arylalkyl, heteroarylalkyl, amino, alkylamino, dialkylamino, diarylalkylamino, oxo, oxa, perhaloalkyl, perhaloalkoxy, perhaloacyl, guanidine, pyridinyl, thiophene, furanyl, indole, indazole, phosphonates, phosphates, phosphoramides, sulfonates, sulfones, sulfates, sulphonamides, carbamates, ureas, thioureas and thioamides, wherein $R^8$ and $R^{10}$ taken together optionally form a ring of 3-7 ring atoms and optionally 1-3 of the ring atoms are heteroatoms selected from the group of O, S and N;
$R^8$ is hydrogen, lower alkyl, lower alkenyl, lower alkynyl, lower aryl, lower heteroaryl, or $-C(O)R^9$;
$R^9$ is H, lower alkyl, lower aryl, lower heteroaryl, $-NR^{10}R^{10}$, or $-OR^{11}$, wherein $R^{10}$ and $R^{10}$ taken together optionally form a ring of 3-7 ring atoms and optionally 1-3 of the ring atoms are heteroatoms selected from the group of O, S and N;
$R^{10}$ is hydrogen, lower alkyl, lower heteroaryl, lower aryl, lower alkenyl, or lower alkynyl,
$R^{11}$ is lower alkyl, lower alkenyl, lower alkynyl, lower heteroaryl or lower aryl; and
$R^{12}$ is hydrogen or lower alkyl.

105. A method of treating an individual having an HSP90 mediated disorder comprising administering to said individual a pharmaceutical composition comprising a pharmaceutically effective amount of a compound of Formula IE:

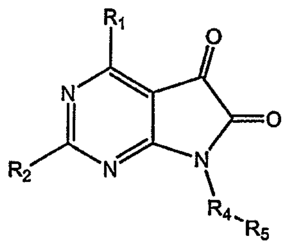

IE

wherein:

$R^1$ is halogen, $-OR^{11}$, $-SR^{11}$ or lower alkyl;
$R^2$ is $-NH_2$;
$R^4$ is $-CHR^{12}-$, -C(O)-, -C(S)-, -S(O)- or $-SO_2-$;
$R^5$ is aryl, heteroaryl, alicyclic, or heterocyclic, wherein
the aryl group is substituted with 3 to 5 substituents,
the heteroaryl group is substituted with 2 to 5 substituents,
the alicyclic group is substituted with 3 to 5 substituents,
the heterocyclic group is substituted with 3 to 5 substituents, and
the substituents on $R^5$ are selected from the group consisting of halogen,
lower alkyl, lower alkenyl, lower alkynyl, $-SR^8$, $-OR^8$, -CN, - C(O)OH, $-C(O)R^9$,
$-NO_2$ and $-NR^8R^{10}$, lower aryl, heteroaryl, alicyclic, lower heterocyclic, arylalkyl, heteroarylalkyl, amino, alkylamino, dialkylamino, diarylalkylamino, oxo, oxa, perhaloalkyl, perhaloalkoxy, perhaloacyl, guanidine, pyridinyl, thiophene, furanyl, indole, indazole, phosphonates, phosphates, phosphoramides, sulfonates, sulfones, sulfates, sulphonamides, carbamates, ureas, thioureas and thioamides, wherein $R^8$ and $R^{10}$ taken together optionally form a ring of 3-7 ring atoms and optionally 1-3 of the ring atoms are heteroatoms selected from the group of O, S and N;
$R^8$ is hydrogen, lower alkyl, lower alkenyl, lower alkynyl, lower aryl, lower heteroaryl, or $-C(O)R^9$;
$R^9$ is H, lower alkyl, lower aryl, lower heteroaryl, $-NR^{10}R^{10}$, or $-OR^{11}$, wherein $R^{10}$ and $R^{10}$ taken together optionally form a ring of 3-7 ring atoms and optionally 1-3 of the ring atoms are heteroatoms selected from the group of O, S and N;
$R^{10}$ is hydrogen, lower alkyl, lower heteroaryl, lower aryl, lower alkenyl, or lower alkynyl;
$R^{11}$ is lower alkyl, lower alkenyl, lower alkynyl, lower heteroaryl or lower aryl;
and
$R^{12}$ is hydrogen or lower alkyl.

106. A compound represented by Formula IIC, or a polymorph, solvate, ester, tautomer, pharmaceutically acceptable salt or prodrug thereof:

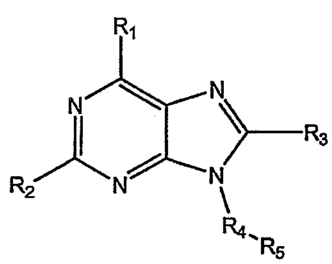

IIC

wherein:

$R^1$ is halogen or lower alkyl;
$R^2$ is $-NR^8R^{10}$;
$R^4$ is $-CHR^{12}-$;

$R^3$ is hydrogen, halogen, or -CN;
$R^5$ is aryl, heteroaryl, alicyclic, or heterocyclic, wherein:

the aryl group is substituted with 3 to 5 substituents,
the heteroaryl group is substituted with 2 to 5 substituents, wherein when the heteroaryl is substituted with only two substituents, the two substituents must form part of an optionally substituted fused ring,
the alicyclic group is substituted with 3 to 5 substituents,
the heterocyclic group is substituted with 3 to 5 substituents, and
the subsituents are selected from the group of halogen, lower alkyl, lower alkenyl, lower alkynyl, lower aryl, lower alicyclic, arylalkyl, aryloxy, aryloxyalkyl, alkoxyalkyl, perhaloalkyl, perhaloalkyloxy, perhaloacyl, $-N_3$, $-SR^8$, $-OR^8$, -CN, $-C(O)R^9$, $-NO_2$, $-NR^8R^{10}$, phosphonate and phosphonic acid;

$R^8$ is hydrogen, lower alkyl, lower aryl, or $-C(O)R^9$;
$R^9$ is lower alkyl, lower aryl, lower heteroaryl, $-NR^{10}R^{10}$ or $-OR^{11}$;
$R^{10}$ is independently hydrogen or lower alkyl;
$R^{11}$ is lower alkyl, lower aryl or lower heteroaryl;
$R^{12}$ is hydrogen or lower alkyl;
provided that
when $R^5$ is aryl, $R^5$ is not an organo-metallic cyclopentadiene;
when $R^5$ is phenyl, the substituents are not 3,5 di-halo;
when $R^5$ is alicyclic, the ring system does not contain any tetra-substituted $sp^3$ ring carbons; and
when $R^5$ is heterocyclic, the ring system does not contain any tetra-substituted $sp^3$ ring carbons or the ring system is not a tetra-substituted pyrrolidine.

107. The compound of item 106, or a polymorph, solvate, ester, tautomer, pharmaceutically acceptable salt or prodrug thereof, wherein:

$R^1$ is halogen or methyl; and
$R^2$ is $-NHR^8$, where $R^8$ is hydrogen or $-C(O)R^9$.

108. The compound of item 106, or a polymorph, solvate, ester, tautomer, pharmaceutically acceptable salt or prodrug thereof, wherein:

$R^1$ is halogen;
$R^2$ is $-NH_2$;
$R^3$ is hydrogen; and
$R^5$ is aryl or heteroaryl, wherein
each of said aryl and heteroaryl groups is monocyclic or bicyclic,
the aryl group is substituted with 4 to 5 substituents,
the heteroaryl group is substituted with 2 to 5 substituents, wherein when the heteroaryl is substituted with only two substituents, the two substituents must form part of an optionally substituted fused ring.

109. The compound of item 106, or a polymorph, solvate, ester, tautomer, pharmaceutically acceptable salt or prodrug thereof, wherein:

$R^1$ is halogen;
$R^2$ is $-NH_2$;
$R^4$ is lower alkyl;
$R^3$ is hydrogen; and
$R^5$ is aryl or heteroaryl, wherein
each of said aryl and heteroaryl groups is monocyclic or bicyclic,
the aryl group is substituted with 4 to 5 substituents,
the heteroaryl group is substituted with 2 to 5 substituents, wherein when the heteroaryl is substituted with only two substituents, the two substituents must form part of an optionally substituted fused ring.

110. The compound of item 106, or a polymorph, solvate, ester, tautomer, pharmaceutically acceptable salt or prodrug thereof, wherein

$R^1$ is chloro or bromo,
$R^2$ is $-NH_2$, and
$R^5$ is a phenyl having 3 to 5 substituents, a pyridyl having 3 to 5 substituents or an 1-oxy- pyridyl (N-oxy-pyridyl), each of which has 3 to 5 substituents.

111. A compound represented by Formula IID, or a polymorph, solvate, ester, tautomer, pharmaceutically acceptable salt or prodrug thereof:

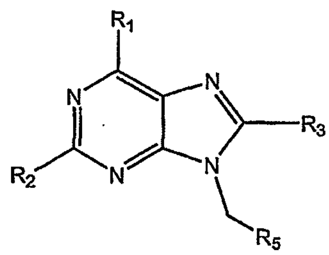

IID

wherein:

$R^1$ is halogen or lower alkyl;
$R^2$ is $-NR^8R^{10}$;
$R^3$ is hydrogen, halogen, or -CN;
$R^5$ is aryl, heteroaryl, alicyclic, or heterocyclic, wherein
the aryl group is substituted with 3 to 5 substituents,
the heteroaryl group is substituted with 2 to 5 substituents, wherein when the heteroaryl is substituted with only two substituents, the two substituents must form part of an optionally substituted fused ring,
the alicyclic group is substituted with 3 to 5 substituents,
the heterocyclic group is substituted with 3 to 5 substituents, and
the subsituents are selected from the group of halogen, lower alkyl, lower alkenyl, lower alkynyl, lower aryl, lower alicyclic, arylalkyl, aryloxy, aryloxyalkyl, alkoxyalkyl, perhaloalkyl, perhaloalkyloxy, perhaloacyl, $-N_3$, $-SR^8$, $-OR^8$, -CN, $-C(O)R^9$, $-NO_2$, $-NR^8R^{10}$, phosphonate and phosphonic acid;
$R^8$ is hydrogen, lower alkyl, lower aryl, or $-C(O)R^9$;
$R^9$ is lower alkyl, lower aryl, lower heteroaryl, $-NR^{10}R^{10}$ or $-OR^{11}$;
$R^{10}$ is independently hydrogen or lower alkyl; and
$R^{11}$ is lower alkyl, lower aryl or lower heteroaryl;
provided that
when $R^5$ is aryl, $R^5$ is not an organo-metallic cyclopentadiene;
when $R^5$ is phenyl, the substituents are not 3,5 di-halo;
when $R^5$ is alicyclic, the ring system does not contain any tetra-substituted $sp^3$ ring carbons;
when $R^5$ is heterocyclic, the ring system does not contain any tetra-substituted $sp^3$ ring carbons or the ring system is not a tetra-substituted pyrrolidine.

112. The compound of item 111, or a polymorph, solvate, ester, tautomer, pharmaceutically acceptable salt or prodrug thereof, wherein each of said aryl, heteroaryl, alicyclic or heterocyclic group is monocyclic or bicyclic.

113. The compound of item 111, or a polymorph, solvate, ester, tautomer, pharmaceutically acceptable salt or prodrug thereof, wherein:

$R^1$ is halogen or methyl; and
$R^2$ is $-NHR^8$, where $R^8$ is hydrogen or $-C(O)R^9$,

114. The compound of item 113, or a polymorph, solvate, ester, tautomer, pharmaceutically acceptable salt or prodrug thereof, wherein $R^1$ is halogen.

115. The compound of item 114, or a polymorph, solvate, ester, tautomer, pharmaceutically acceptable salt or

prodrug thereof, wherein $R^2$ is $NH_2$ and $R^3$ is hydrogen.

116. The compound of item 111, or a polymorph, solvate, ester, tautomer, pharmaceutically acceptable salt or prodrug thereof, wherein:

$R^1$ is halogen;
$R^2$ is $-NH_2$;
$R^3$ is hydrogen; and
$R^5$ is aryl or heteroaryl, wherein
each of the aryl and heteroaryl groups is monocyclic or bicyclic,
the aryl group is substituted with 4 to 5 substituents,
the heteroaryl group is substituted with 2 to 5 substituents, wherein when the heteroaryl is substituted with only two substituents, the two substituents must form part of an optionally substituted fused ring.

117. The compound of item 111, or a polymorph, solvate, ester, tautomer, pharmaceutically acceptable salt or prodrug thereof, wherein $R^1$ is chloro or bromo, $R^2$ is $-NH_2$, and $R^5$ is a phenyl having 3 to 5 substituents.

118. The compound of item 111, or a polymorph, solvate, ester, tautomer, pharmaceutically acceptable salt or prodrug thereof, wherein $R^1$ is chloro or bromo, $R^2$ is $-NH_2$, and $R^5$ is a pyridyl having 3 to 5 substituents.

119. The compound of item 111, or a polymorph, solvate, ester, tautomer, pharmaceutically acceptable salt or prodrug thereof, wherein $R^1$ is chloro or bromo, $R^2$ is $-NH_2$, and $R^5$ is an 1-oxy-pyzidyl (N-oxy-pyridyl) having 3 to 5 substituents.

120. The compound of item 116, wherein said compound is a member selected from the group below, or a polymorph, solvate, ester, tautomer, pharmaceutically acceptable salt or prodrug
thereof:

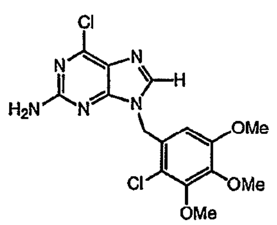 , 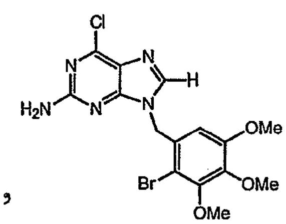 , 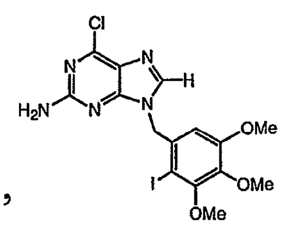 ,

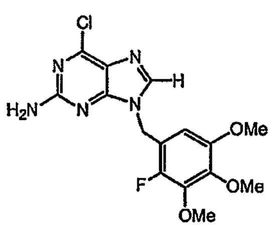 , 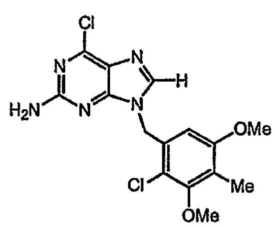 , 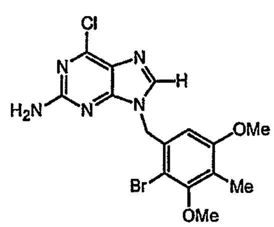 ,

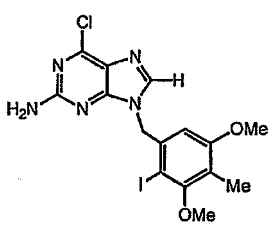 , 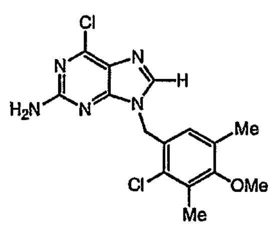 and 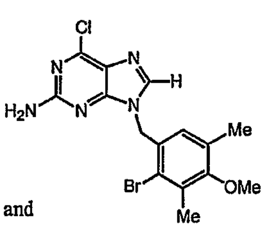

121. The compound of item 116, wherein said compound is a member selected from the group below, or a polymorph, solvate, ester, tautomer, pharmaceutically acceptable salt or prodrug thereof:

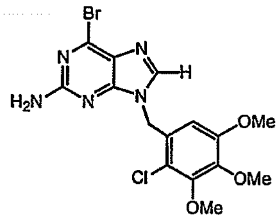,
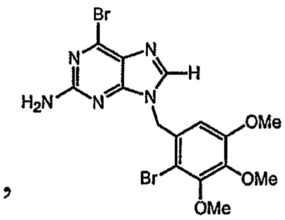,
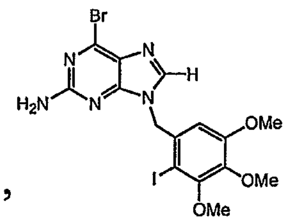,

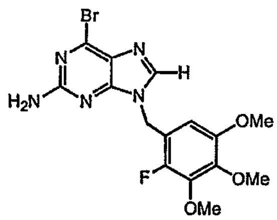,
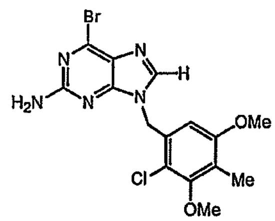,
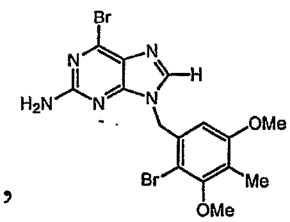,

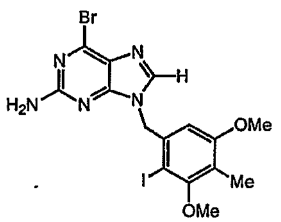,
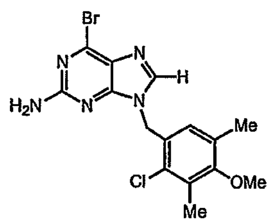
and
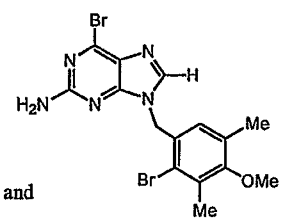

122. The compound of item 116, wherein said compound is a member selected from the group below, or a polymorph, solvate, ester, tautomer, pharmaceutically acceptable salt or prodrug thereof:

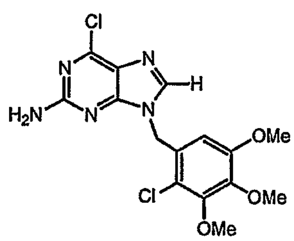,
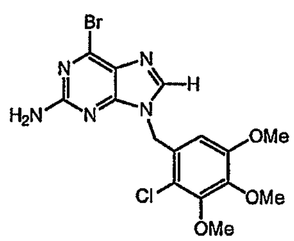,

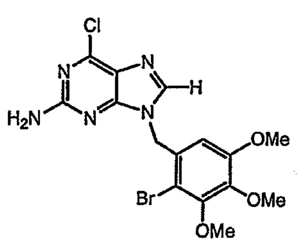
and
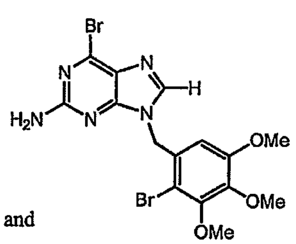

123. The compound of item 116, wherein said compound is represented by the formula below, or a polymorph, solvate, ester, tautomer, pharmaceutically acceptable salt or prodrug thereof:

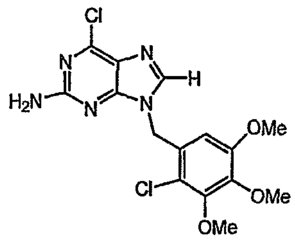

124. The compound of item 116, wherein said compound is represented by the formula below, or a polymorph, solvate, ester, tautomer, pharmaceutically acceptable salt or prodrug thereof:

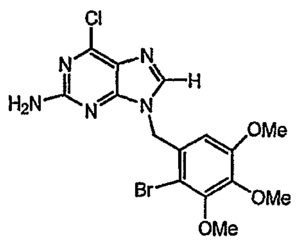

125. The compound of item 116, wherein said compound is represented by the formula below, or a polymorph, solvate, ester, tautomer, pharmaceutically acceptable salt or prodrug thereof:

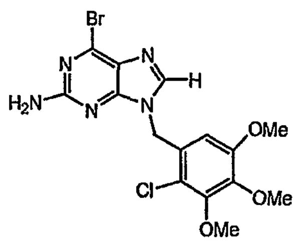

126. The compound of item 116, wherein said compound is represented by the formula below, or a polymorph, solvate, ester, tautomer, pharmaceutically acceptable salt or prodrug thereof:

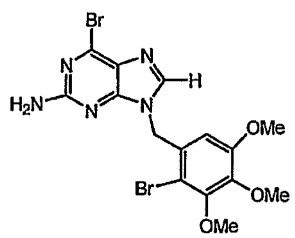

127. The compound of item 116, wherein the compound is a member selected from the group of compounds below, or a polymorph, solvate, ester, tautomer, pharmaceutically acceptable salt or prodrug thereof:

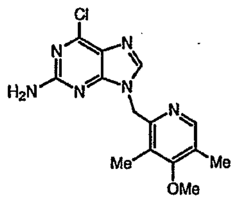, 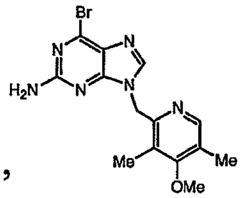, 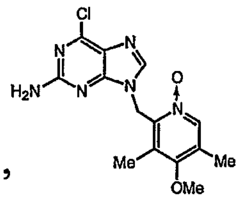, 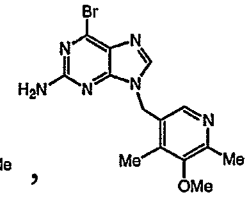,

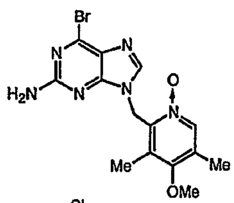, 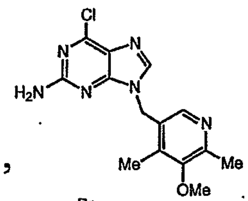, 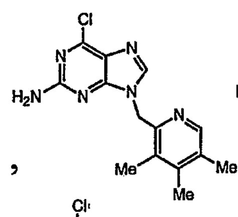, 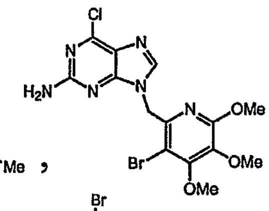,

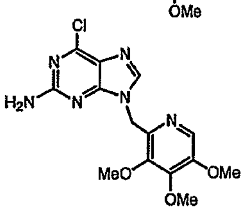, 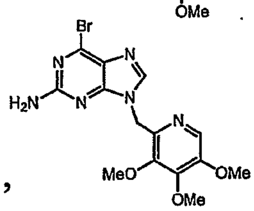, 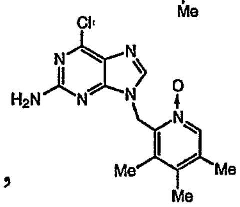, 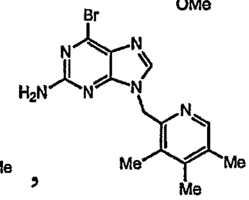,

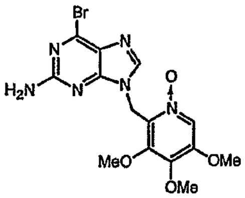, 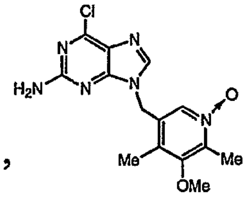, 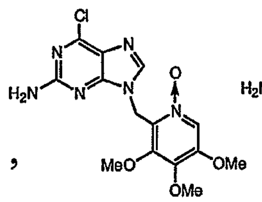 and 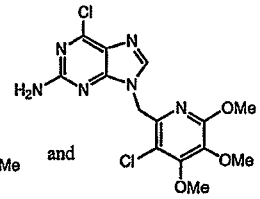

128. The compound of item 116, wherein said compound is a member selected from the group below, or a polymorph, solvate, ester, tautomer, pharmaceutically acceptable salt or prodrug thereof:

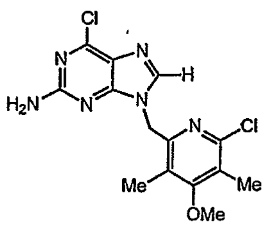, 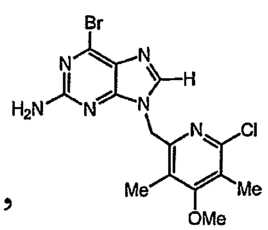, 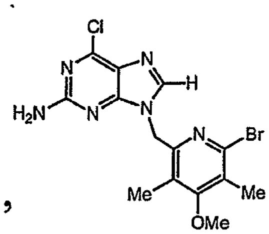,

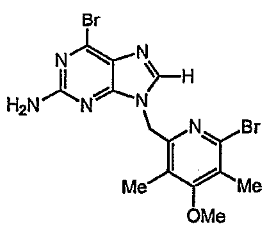, 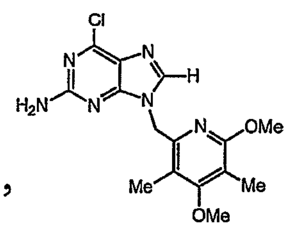 and 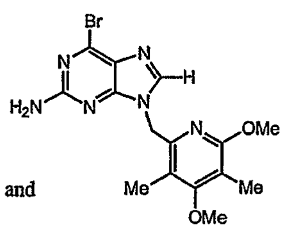

129. The compound of item 116, wherein said compound is a member selected from the group below, or a polymorph, solvate, ester, tautomer, pharmaceutically acceptable salt or prodrug thereof:

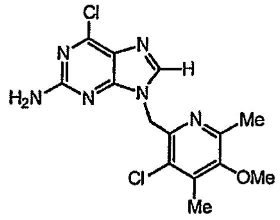 , 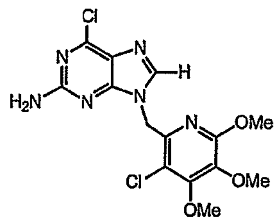 , 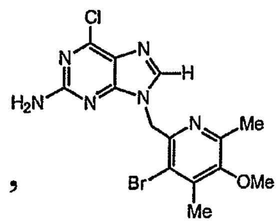 ,

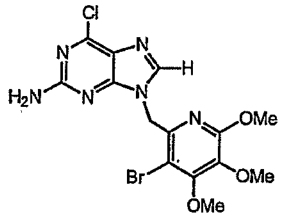 , 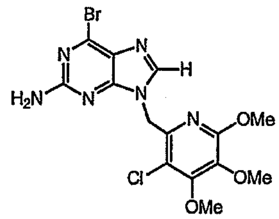 and 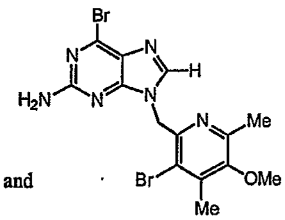

130. The compound of item 116, wherein said compound is a member selected from the group below, or a polymorph, solvate, ester, tautomer, pharmaceutically acceptable salt or prodrug thereof:

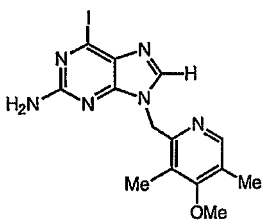 , 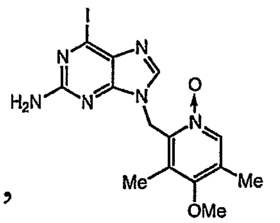 , 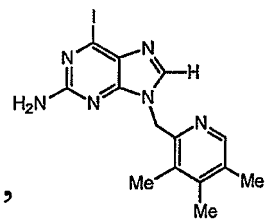 ,

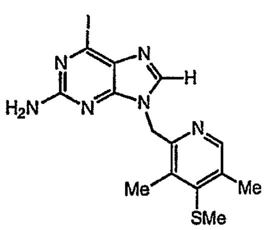 , 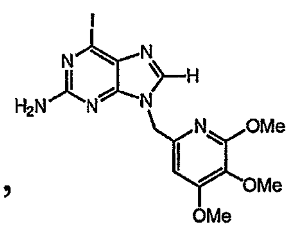 and 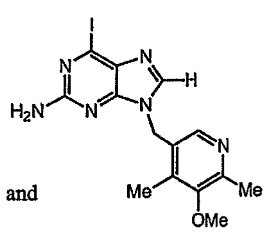

131. The compound of item 116, wherein said compound is a member selected from the group below, or a polymorph, solvate, ester, tautomer, pharmaceutically acceptable salt or prodrug thereof:

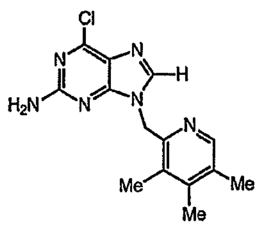 and 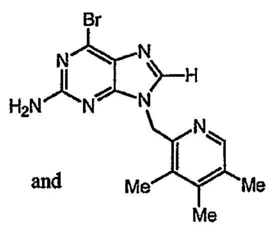

132. The compound of item 116, wherein said compound is a member selected from the group below, or a polymorph, solvate, ester, tautomer, pharmaceutically acceptable salt or prodrug thereof:

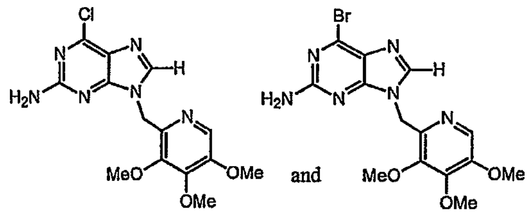

and

133. The compound of item 116, wherein said compound is a member selected from the group below, or a polymorph, solvate, ester, tautomer, pharmaceutically acceptable salt or prodrug thereof:

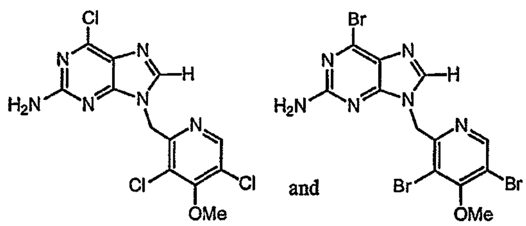

and

134. The compound of item 116, wherein said compound is represented by the formula below, or a polymorph, solvate, ester, tautomer, pharmaceutically acceptable salt or prodrug thereof:

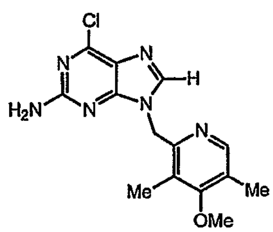

135. The compound of item 116, wherein said compound is represented by the formula below, or a polymorph, solvate, ester, tautomer, pharmaceutically acceptable salt or prodrug thereof:

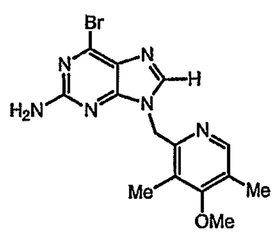

136. The compound of item 116, wherein said compound is represented by the formula below, or a polymorph, solvate, ester, tautomer, pharmaceutically acceptable salt or prodrug thereof:

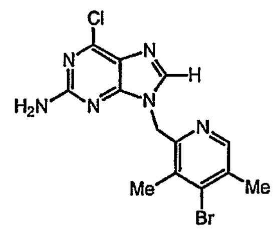

137. The compound of item 116, wherein said compound is represented by the formula below, or a polymorph, solvate, ester, tautomer, pharmaceutically acceptable salt or prodrug thereof:

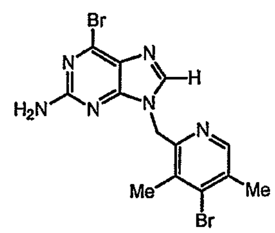

138. The compound of item 116, wherein said compound is represented by the formula below, or a polymorph, solvate, ester, tautomer, pharmaceutically acceptable salt or prodrug thereof:

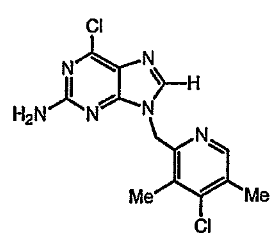

139. The compound of item 116, wherein said compound is represented by the formula below, or a polymorph, solvate, ester, tautomer, pharmaceutically acceptable salt or prodrug thereof:

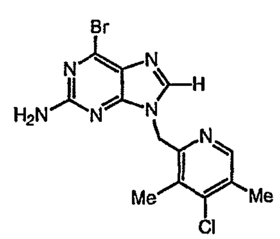

140. The compound of item 116, wherein said compound is represented by the formula below, or a polymorph, solvate, ester, tautomer, pharmaceutically acceptable salt or prodrug thereof:

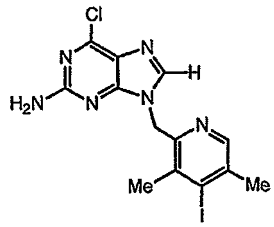

141. The compound of item 116, wherein said compound is represented by the formula below, or a polymorph, solvate, ester, tautomer, pharmaceutically acceptable salt or prodrug thereof:

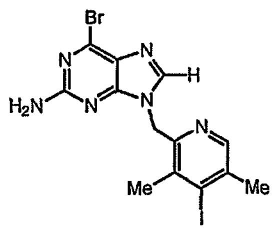

142. The compound of item 116, wherein said compound is represented by the formula below, or a polymorph, solvate, ester, tautomer, pharmaceutically acceptable salt or prodrug thereof:

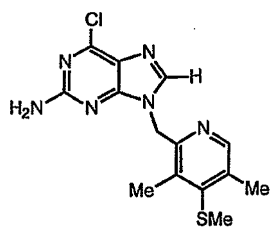

143. The compound of item 116, wherein said compound is represented by the formula below, or a polymorph, solvate, ester, tautomer, pharmaceutically acceptable salt or prodrug thereof:

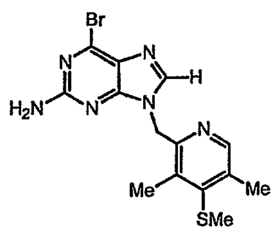

144. The compound of item 116, wherein said compound is represented by the formula below, or a polymorph, solvate, ester, tautomer, pharmaceutically acceptable salt or prodrug thereof:

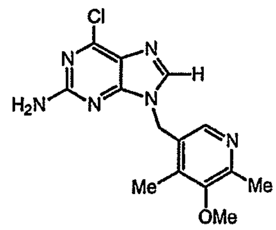

145. The compound of item 116, wherein said compound is represented by the formula below, or a polymorph, solvate, ester, tautomer, pharmaceutically acceptable salt or prodrug thereof:

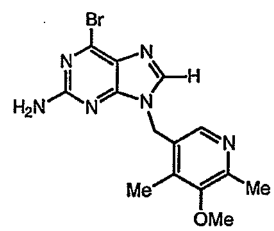

146. The compound of item 116, wherein said compound is a member selected from the group below, or a polymorph, solvate, ester, tautomer, pharmaceutically acceptable salt or prodrug thereof:

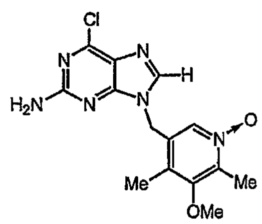

and

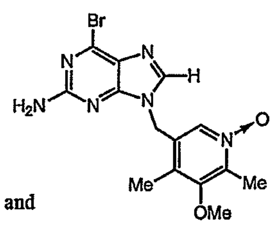

147. The compound of item 116, wherein said compound is a member selected from the group below, or a polymorph, solvate, ester, tautomer, pharmaceutically acceptable salt or prodrug thereof:

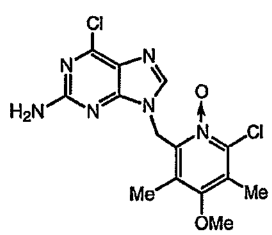

and

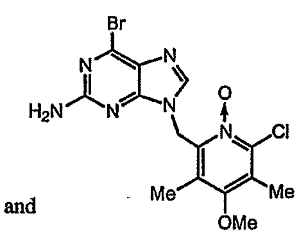

148. The compound of item 116, wherein said compound is a member selected from the group below, or a polymorph, solvate, ester, tautomer, pharmaceutically acceptable salt or prodrug thereof:

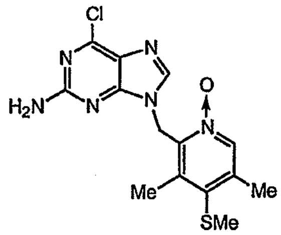

and

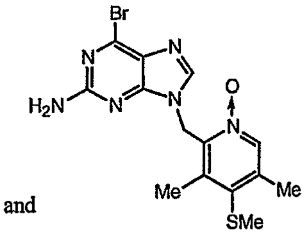

149. The compound of item 116, wherein said compound is represented by the formula below, or a polymorph, solvate, ester, tautomer, pharmaceutically acceptable salt or prodrug thereof:

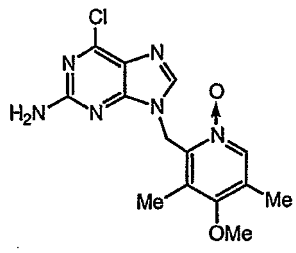

150. The compound of item 116, wherein said compound is represented by the formula below, or a polymorph, solvate, ester, tautomer, pharmaceutically acceptable salt or prodrug thereof:

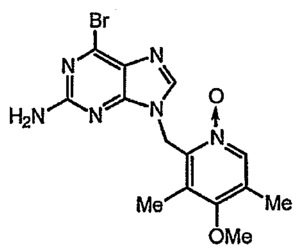

151. The compound of item 16, wherein said compound is represented by the formula below, or a polymorph, solvate, ester, tautomer, pharmaceutically acceptable salt or prodrug thereof:

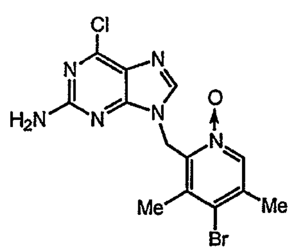

152. The compound of item 116, wherein said compound is represented by the formula below, or a polymorph, solvate, ester, tautomer, pharmaceutically acceptable salt or prodrug thereof:

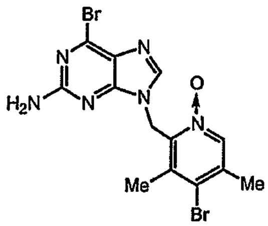

153. The compound of item 116, wherein said compound is represented by the formula below, or a polymorph, solvate, ester, tautomer, pharmaceutically acceptable salt or prodrug thereof:

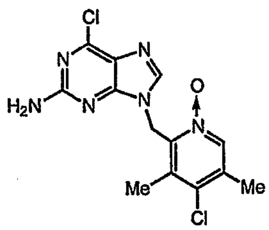

154. The compound of item 116, wherein said compound is represented by the formula below, or a polymorph, solvate, ester, tautomer, pharmaceutically acceptable salt or prodrug thereof:

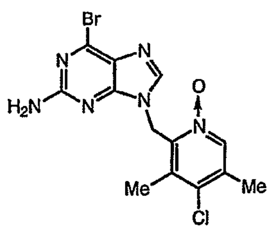

155. The compound of item 116, wherein said compound is represented by the formula below, or a polymorph, solvate, ester, tautomer, pharmaceutically acceptable salt or prodrug thereof:

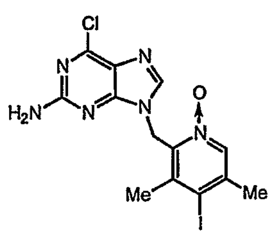

156. The compound of item 116, wherein said compound is represented by the formula below, or a polymorph, solvate, ester, tautomer, pharmaceutically acceptable salt or prodrug thereof:

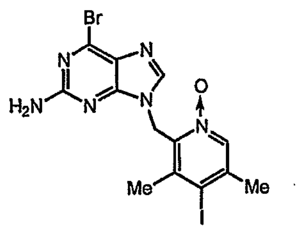

157. The compound of item 116, wherein said compound is a member selected from the group below, or a polymorph, solvate, ester, tautomer, pharmaceutically acceptable salt or prodrug thereof:

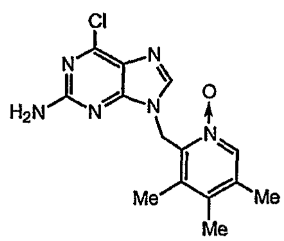

and

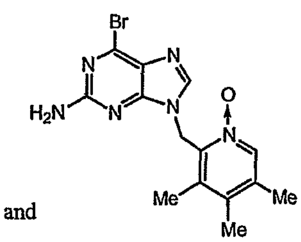

158. The compound of item 116, wherein said compound is a member selected from the group below, or a polymorph, solvate, ester, tautomer, pharmaceutically acceptable salt or prodrug thereof:

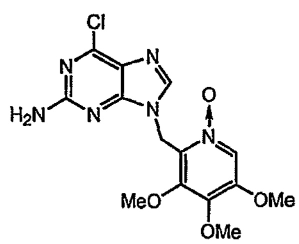

and

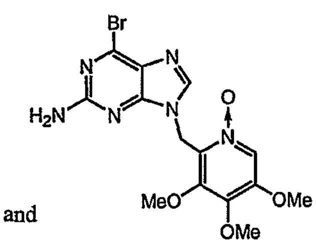

159. The compound of item 116, wherein said compound is a member selected from the group below, or a polymorph, solvate, ester, tautomer, pharmaceutically acceptable salt or prodrug thereof:

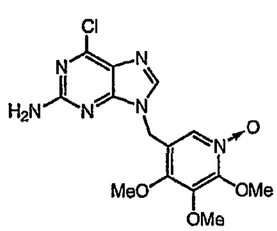

,

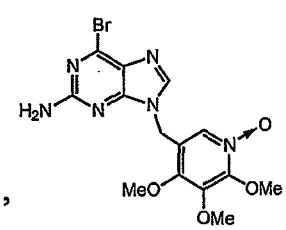

,

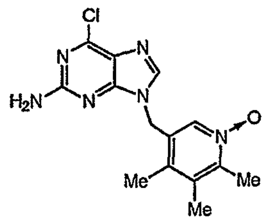

and

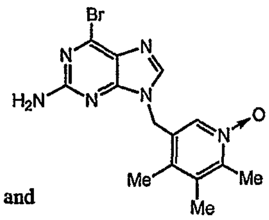

160. A pharmaceutical composition comprising one or more pharmaceutical acceptable excipients and at least one compound represented by Formula IIC below, or a polymorph, solvate, ester, tautomer, pharmaceutically acceptable salt or prodrug thereof:

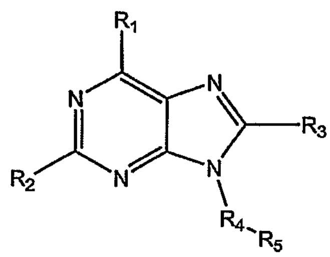

IIC

wherein:

$R^1$ is halogen or lower alkyl;
$R^2$ is $-NR^8R^{10}$;
$R^4$ is $-CHR^{12}-$;
$R^3$ is hydrogen, halogen, or -CN;
$R^5$ is aryl, heteroaryl, alicyclic, or heterocyclic, wherein
the aryl group is substituted with 3 to 5 substituents,
the heteroaryl group is substituted with 2 to 5 substituents, wherein when the heteroaryl is substituted with only two substituents, the two substituents must form part of an optionally substituted fused ring,
the alicyclic group is substituted with 3 to 5 substituents,
the heterocyclic group is substituted with 3 to 5 substituents, and
the subsituents are selected from the group of halogen, lower alkyl, lower alkenyl, lower alkynyl, lower aryl, lower alicyclic, arylalkyl, aryloxy, aryloxyalkyl, alkoxyalkyl, perhaloalkyl, perhaloalkyloxy, perhaloacyl, $-N_3$, $-SR^8$, $-OR^8$, -CN, $-C(O)R^9$, $-NO_2$, $-NR^8R^{10}$, phosphonate and phosphonic acid;
$R^8$ is hydrogen, lower alkyl, lower aryl, or $-C(O)R^9$;
$R^9$ is lower alkyl, lower aryl, lower heteroaryl, $-NR^{10}R^{10}$ or $-OR^{11}$;
$R^{10}$ is independently hydrogen or lower alkyl;
$R^{11}$ is lower alkyl, lower aryl or lower heteroaryl;
$R^{12}$ is hydrogen or lower alkyl; and
provided that
when $R^5$ is aryl, $R^5$ is not an organo-metallic cyclopentadiene;
when $R^5$ is phenyl, the substituents are not 3,5 di-halo;
when $R^5$ is alicyclic, the ring system does not contain any tetra-substituted $sp^3$ ring carbons; and
when $R^5$ is heterocyclic, the ring system does not contain any tetra-substituted $sp^3$ ring carbons or the ring system is not a tetra-substituted pyrrolidine.

161. The pharmaceutical composition of item 160 wherein $R^4$ is $-CH_2-$.

162. The pharmaceutical composition of item 160, wherein:

$R^1$ is halogen;

$R^2$ is $-NH_2$;
$R^3$ is hydrogen; and
$R^5$ is aryl or heteroaryl, wherein
each of the aryl and heteroaryl groups is monocyclic or bicyclic,
the aryl group is substituted with 4 to 5 substituents,
the heteroaryl group is substituted with 2 to 5 substituents, wherein when the heteroaryl is substituted with only two substituents, the two substituents must form part of an optionally substituted fused ring.

163. The pharmaceutical composition of item 161 wherein, wherein
$R^1$ is chloro or bromo;
$R^2$ is $-NH_2$; and
$R^5$ is selected from a phenyl having 3 to 5 substituents, a pyridyl having 3 to 5 substituents and an 1-oxy-pyridyl (N-oxy-pyridyl), each of which has 3 to 5 substituents.

164. A method of treating an individual having an HSP90 mediated disorder comprising administering to said individual a pharmaceutical composition comprising a pharmaceutically effective amount of a compound of Formula IIC:

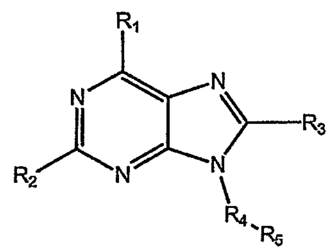

IIC

or a polymorph, solvate, ester, tautomer, pharmaceutically acceptable salt or prodrug thereof, wherein:

$R^1$ is halogen, $-OR^{11}$, $-SR^{11}$, $-NHR^8$, hydrogen, or lower alkyl;
$R^2$ is $-NR^8R^{10}$;
$R^3$ is hydrogen, halogen, $-N_3$, or -CN;
$R^4$ is $-(CHR^{12})_n-$ where n = 0, 1 or 2, -C(O)-, -C(S)-, or -S(O)-;
$R^5$ is alkyl, aryl, heteroaryl, alicyclic, or heterocyclic, all optionally substituted with halogen, lower alkyl, lower alkenyl, lower alkynyl, lower aryl, lower alicyclic, arylalkyl, aryloxy, aryloxyalkyl, alkoxyalkyl, perhaloalkyl, perhaloalkyloxy, perhaloacyl, $-N_3$, $-SR^8$, $-OR^8$, -CN, $-C(O)R^9$, $-NO_2$, or - $NR^8R^{10}$;
$R^8$ is hydrogen, lower alkyl, lower aryl, or $-C(O)R^9$;
$R^9$ is lower alkyl, lower aryl, lower heteroaryl, $-NR^{10}R^{10}$, or $-OR^{11}$;
$R^{10}$ is independently hydrogen or lower alkyl;
$R^{11}$ is lower alkyl, lower aryl or lower heteroaryl;
$R^{12}$ is hydrogen or lower alkyl; and
provided that:
$-R^4R^5$ is not a ribose or derivative thereof, or a sugar or derivative thereof;
$-R^4R^5$ is not a phosphonate or phosphonic acid, or substituted with phosphonate or phosphonic acid; and
when $R^4$ is $-(CH_2)_n-$ where n=1 or 2, then $R^4$ and $R^5$ are not connected through an ether linkage.

165. The method of item 164, wherein:

$R^3$ is hydrogen, halogen or -CN; and
$R^5$ is aryl, heteroaryl, alicyclic, or heterocyclic, all optionally substituted with halogen, lower alkyl, lower alkenyl, lower alkynyl, lower aryl, lower alicyclic, arylalkyl, aryloxy, aryloxyalkyl, alkoxyalkyl, perhaloalkyl, perhaloalkyloxy, perhaloacyl, $-N_3$, $-SR^8$, $-OR^8$, -CN, $-C(O)R^9$, $-NO_2$, $-NR^8R^{10}$, phosphonate, or phosphonic acid.

166. The method of item 164, wherein:

$R^1$ is halogen;
$R^2$ is $-NH_2$;

$R^3$ is hydrogen;
$R^4$ is $-CH_2-$; and
$R^5$ is aryl or heteroaryl, wherein:

the aryl and heteroaryl groups are monocyclic or bicyclic,
the aryl group is substituted with 4 to 5 substituents,
the heteroaryl group is substituted with 2 to 5 substituents, wherein when the heteroaryl is substituted with two substituents, the two substituents must form part of an optionally substituted fused ring.

167. The method of item 165, wherein $R^1$ is chloro or bromo, $R^2$ is $-NH_2$, and $R^5$ is a phenyl having 3 to 5 substituents, a pyridyl having 3 to 5 substituents or an 1-oxy-pyridyl (N-oxy-pyridyl) having 3 to 5 substituents.

168. The method of item 165, wherein the HSP90 mediated disorder is selected from the group of inflammatory diseases, infections, autoimmune disorders, stroke, ischemia, cardiac disorders, neurological disorders, fibrogenetic disorders, proliferative disorders, tumors, leukemias, neoplasms, cancers, carcinomas, metabolic diseases, and malignant disease.

169. The method of item 168 wherein the fibrogenetic disorder is further selected from the group of scleroderma, polymyositis, systemic lupus, rheumatoid arthritis, liver cirrhosis, keloid formation, interstitial nephritis and pulmonary fibrosis.

170. The method of item 165, further comprising administering at least one therapeutic agent selected from the group of cytotoxic agents, anti-angiogenesis agents and anti-neoplastic agents.

171. The method of item 170, wherein the at least one anti-neoplastic agent is selected from the group of alkylating agents, anti-metabolites, epidophyllotoxins; antineoplastic enzymes, topoisomerase inhibitors, procarbazines, mitoxantrones, platinum coordination complexes, biological response modifiers and growth inhibitors, hormonal/anti-hormonal therapeutic agents, and haematopoietic growth factors.

172. A compound, or a polymorph, solvate, ester, tautomer, pharmaceutically acceptable salt or prodrug thereof, prepared by the process comprising:
reacting a compound of formula Y and a compound of formula Z, wherein:
Y is represented by any of the following formulae, respectively:

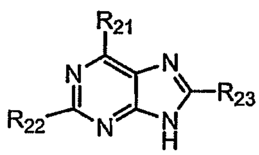 , 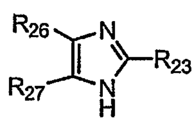 and 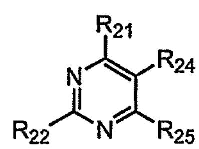 ,

and
Z is $L^1$-$R^4$-$R^5$; wherein:

$L^1$ is halogen, $NR^8R^{10}$, triflate, tosylate, or mesylate;
$R^4$ is $-CHR^{12}-$,
$R^5$ is aryl, heteroaryl, alicyclic, or heterocyclic, wherein:

the aryl group is substituted with 3 to 5 substituents,
the heteroaryl group is substituted with 2 to 5 substituents,
wherein when the heteroaryl is substituted with only two substituents, the two substituents must form part of an optionally substituted fused ring,
the alicyclic group is substituted with 3 to 5 substituents,
the heterocyclic group is substituted with 3 to 5 substituents, and
the subsituents are selected from the group of halogen, lower alkyl, lower alkenyl, lower alkynyl, lower aryl, lower alicyclic, arylalkyl, aryloxy, aryloxyalkyl, alkoxyalkyl, perhaloalkyl, perhaloalkyloxy, perhaloacyl, $-N_3$, $-SR^8$, $-OR^8$, -CN, $-C(O)R^9$, $-NO_2$, $-NR^8R^{10}$, phosphonate and phosphonic acid;

$R^8$ is hydrogen, lower alkyl, lower aryl, or -C(O)$R^9$;
$R^9$ is lower alkyl, lower aryl, lower heteroaryl, -N$R^{10}R^{10}$ or -O$R^{11}$;
$R^{10}$ is independently hydrogen or lower alkyl;
$R^{11}$ is lower alkyl, lower aryl or lower heteroaryl;
$R^{12}$ is hydrogen or lower alkyl;
$R^{21}$ is halogen, lower alkyl or -OH;
$R^{22}$ is -N$R^8R^{10}$;
$R^{23}$ is hydrogen, halogen, or -CN;
$R^{24}$ is -$NH_2$, -$NO_2$ or -NO;
$R^{25}$ is halogen or -OH;
$R^{26}$ is -C(O)$NH_2$ or C(O)OEt; and
$R^{27}$ is -NH2, -OH or halogen;
provided that:

when $R^5$ is aryl, $R^5$ is not an organo-metallic cyclopentadiene;
when $R^5$ is phenyl, the substituents are not 3, 5 di-halo;
when $R^5$ is alicyclic, the ring system does not contain any tetra-substituted $sp^3$ ring carbons; or
when $R^5$ is heterocyclic, the ring system does not contain any tetra-substituted $sp^3$ ring carbons or the ring system is not a tetra-substituted pyrrolidine.

173. The compound of item 172 wherein $R^4$ is -$CH_2$-.

174. The compound of item 173, wherein:

$L^1$ is -Cl, -Br or $NH_2$; and
$R^5$ is aryl or heteroaryl, wherein the aryl group is substituted with 4 to 5 substituents, the heteroaryl group is substituted with 2 to 5 substituents,

wherein when the heteroaryl is substituted with two substituents, the two substituents must form part of an optionally substituted fused ring..

175. The compound of item 174, or a polymorph, solvate, ester, tautomer, pharmaceutically acceptable salt or prodrug thereof, wherein Y is a substituted purine.

176. The compound of item 173, or a polymorph, solvate, ester, tautomer, pharmaceutically acceptable salt or prodrug thereof, wherein said reaction is performed in a solvent comprising a member selected from the group of DMF, THF and DMSO.

177. The compound of item 175, or a polymorph, solvate, ester, tautomer, pharmaceutically acceptable salt or prodrug thereof, wherein said reaction is performed in a solvent that comprises DMF.

178. A compound represented by Formula IIIA, or a polymorph, solvate, ester, tautomer, enantiomer, pharmaceutically acceptable salt or prodrug thereof:

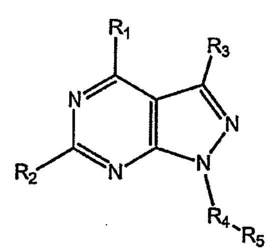

IIIA

wherein:

$R^1$ is halogen, -O$R^{11}$, -S$R^{11}$ or lower alkyl;

$R^2$ is $-NHR^8$;
$R^3$ is selected from the group consisting of hydrogen, halogen, $-SR^8$, $-OR^8$, -CN, - $C(O)R^9$, $-CO_2H$, $-NO_2$, $-NR^8R^{10}$, lower alkyl, lower alkenyl, lower alkynyl, lower perhaloalkyl, aryl, heteroaryl, alicyclic and heterocyclic, all optionally substituted, wherein:

the aryl, heteroaryl, alicyclic and heterocyclic groups are optionally mono-, bi- or tri-cyclic,
$R^8$ and $R^{10}$ taken together optionally form a ring of 3-7 ring atoms and optionally 1-2 of the ring atoms are heteroatoms selected from the group of O, S and N, and
the optional substituents on $R^3$ are selected from the group consisting of halogen, lower alkyl, lower alkenyl, lower alkynyl, $-SR^8$, $-OR^8$, - CN,- $C(O)R^9$, -C(O)OH, $-NO_2$, $-NR^8R^{10}$, lower aryl, lower heteroaryl, lower alicyclic, lower heterocyclic, arylalkyl, heteroarylalkyl, amino, alkylamino, dialkylamino, diarylalkylatnino, oxo, oxa, perhaloalkyl, perhaloalkoxy, perhaloacyl, guanidine, pyridinyl, thiophene, furanyl, indole, indazole, phosphonates, phosphates, phosphoramides, sulfonates, sulfones, sulfates, sulphonamides, carbamates, ureas, thioureas and thioamides, wherein $R^8$ and $R^{10}$ taken together optionally form a ring of 3-7 ring atoms and optionally 1-3 of the ring atoms are heteroatoms selected from the group of O, S and N;

$R^4$ is $-CHR^{12}$-, -C(O), -C(S), -S(O)-, or $-SO_2$-;
$R^5$ is aryl, heteroaryl, alicyclic, or heterocyclic, wherein
the aryl group is substituted with 3 to 5 substituents,
the heteroaryl group is substituted with 2 to 5 substituents,
the alicyclic group is substituted with 3 to 5 substituents,
the heterocyclic group is substituted with 3 to 5 substituents, and
the substituents on $R^5$ are selected from the group consisting of halogen,
lower alkyl, lower alkenyl, lower alkynyl, $-SR^8$, $-OR^8$, -CN, - C(O)OH, $-C(O)R^9$,
$-NO_2$ and $-NR^8R^{10}$, lower aryl, heteroaryl, alicyclic, lower heterocyclic, arylalkyl, heteroarylalkyl, amino, alkylamino, dialkylamino, diarylalkylamino, oxo, oxa, perhaloalkyl, perhaloalkoxy, perhaloacyl, guanidine, pyridinyl, thiophene, furanyl, indole, indazole, phosphonates, phosphates, phosphoramides, sulfonates, sulfones, sulfates, sulphonamides, carbamates, ureas, thioureas and thioamides, wherein $R^8$ and $R^{10}$ taken together optionally form a ring of 3-7 ring atoms and optionally 1-3 of the ring atoms are heteroatoms selected from the group of O, S and N;
$R^8$ is hydrogen, lower alkyl, lower alkenyl, lower alkynyl, lower aryl, lower heteroaryl or $-C(O)R^9$;
$R^9$ is hydrogen, lower alkyl, lower alkenyl, lower alkynyl, lower aryl, lower heteroaryl, $-NR^{10}R^{10}$ or $-OR^{11}$, $R^{10}$ and $R^{10}$ taken together optionally form a ring of 3-7 ring atoms and optionally 1-3 of the ring atoms are heteroatoms selected from the group of O, S and N;
$R^{10}$ is hydrogen, lower alkyl, lower alkenyl, lower alkynyl, lower aryl or lower heteroaryl;
$R^{11}$ is lower alkyl, lower alkenyl, lower alkynyl, lower aryl or lower heteroaryl;
and
$R^{12}$ is hydrogen or lower alkyl.

179. The compound of item 178 or a polymorph, solvate, ester, tautomer, enantiomer, pharmaceutically acceptable salt or prodrug thereof, wherein:

$R^1$ is halogen or lower alkyl;
$R^2$ is $-NHR^8$, where $R^8$ is hydrogen or $-C(O)R^9$; and
$R^5$ is aryl or heteroaryl, wherein each of said aryl and heteroaryl groups is monocyclic or bicyclic.

180. The compound of item 178, or a polymorph, solvate, ester, tautomer, enantiomer, pharmaceutically acceptable salt or prodrug thereof, wherein:

$R^2$ is $-NH_2$;
$R^3$ is selected from hydrogen, halogen, $-SR^8$, $-OR^8$, -CN, $-NR^8R^{10}$, lower alkyl, lower alkenyl, lower alkynyl, lower perhaloalkyl, lower aryl, lower heteroaryl, lower alicyclic, lower heterocyclic, wherein $R^8$ is hydrogen, lower alkyl, lower alkenyl, lower alkynyl, lower aryl or lower heteroaryl, and wherein $R^8$ and $R^{10}$ taken together optionally form a ring of 3-7 ring atoms and optionally 1-3 of the ring atoms are heteroatoms selected from the group of O, S and N; and
$R^5$ is aryl or heteroaryl, wherein each of said aryl and heteroaryl groups is monocyclic or bicyclic.

181. The compound of item 178, or a polymorph, solvate, ester, tautomer, enantiomer, pharmaceutically acceptable salt or prodrug thereof, wherein:

$R^1$ is halogen or lower alkyl;
$R^2$ is $NH_2$;
$R^4$ is $-(CH_2)-$; and
$R^5$ is aryl, heteroaryl, alicyclic or heterocyclic, wherein each of said aryl, heteroaryl alicyclic or heterocyclic groups is monocyclic or bicyclic.

182. The compound of item 178, or a polymorph, solvate, ester, tautomer, enantiomer, pharmaceutically acceptable salt or prodrug thereof, wherein

$R^1$ is halogen;
$R^2$ is $-NH_2$;
$R^3$ is hydrogen, halogen, $-SR^8$, $-OR^8$, lower alkyl, lower aryl, lower heteroaryl, or $-NR^8R^{10}$ wherein $R^8$ and $R^{10}$ taken together optionally form a ring of 3-7 ring atoms and optionally 1-3 of the ring atoms are heteroatoms selected from the group of O, S and N;
$R^4$ is $-CH_2-$; and
$R^5$ is aryl or heteroaryl, wherein each of said aryl and heteroaryl groups is monocyclic or bicyclic.

183. The compound of item 182, or a polymorph, solvate, ester, tautomer, enantiomer, pharmaceutically acceptable salt or prodrug thereof, wherein $R^1$ is chloro or bromo, $R^5$ is a phenyl having 3 to 5 substituents.

184. The compound of item 182, or a polymorph, solvate, ester, tautomer, enantiomer, pharmaceutically acceptable salt or prodrug thereof, wherein $R^1$ is chloro or bromo, $R^5$ is a pyridyl having 3 to 5 substituents.

185. The compound of item 182, or a polymorph, solvate, ester, tautomer, enantiomer, pharmaceutically acceptable salt or prodrug thereof, wherein $R^1$ is chloro or bromo, $R^5$ is an 1-oxy-pyridyl (N-oxy-pyridyl) having 3 to 5 substituents.

186. The compound of item 182, wherein said compound is a member selected from the group below, or a polymorph, solvate, ester, tautomer, enantiomer, pharmaceutically acceptable salt or prodrug thereof:

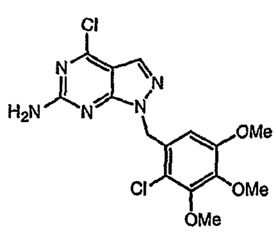 , 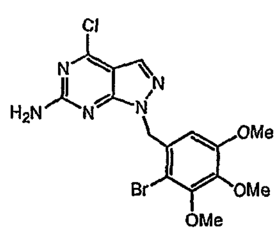 , 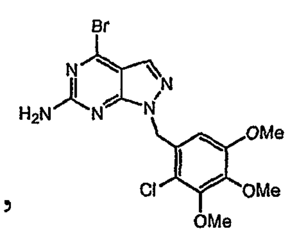 ,

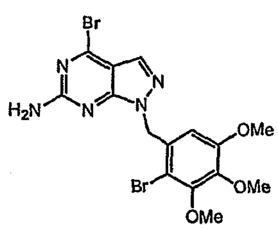 , 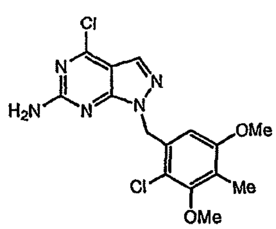 , 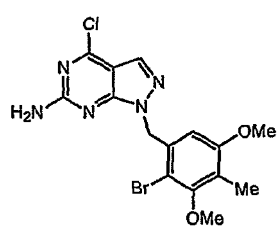 ,

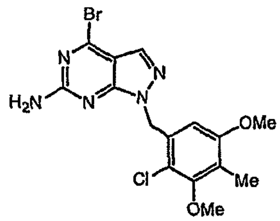,
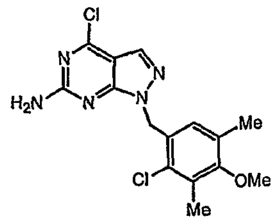
and
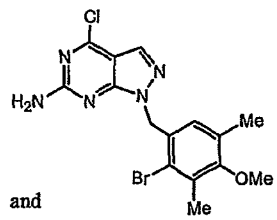

187. The compound of item 182, wherein said compound is a member selected from the group below, or a polymorph, solvate, ester, tautomer, enantiomers, pharmaceutically acceptable salt or prodrug thereof:

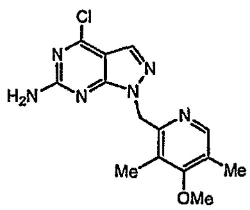,
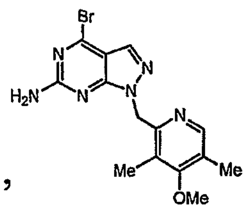,
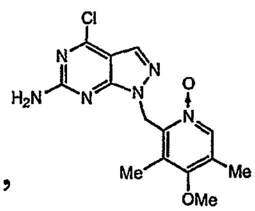,
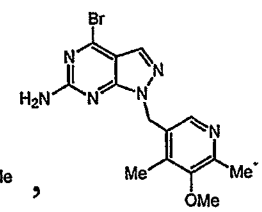;

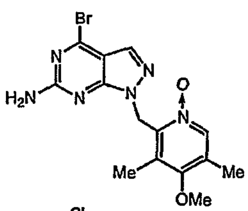,
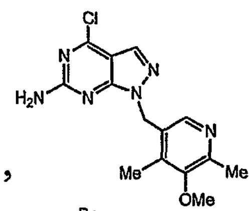,
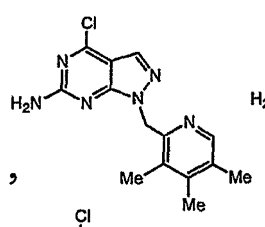,
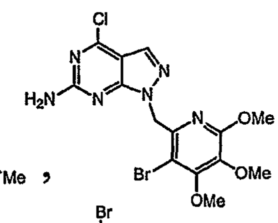

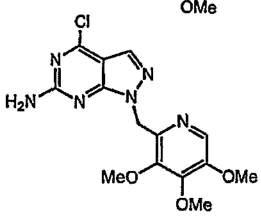,
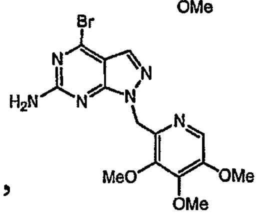,
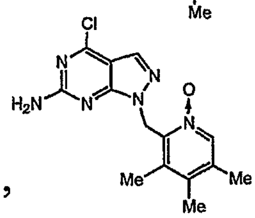,
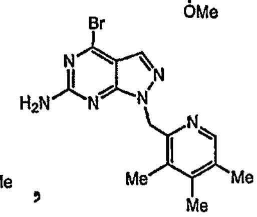;

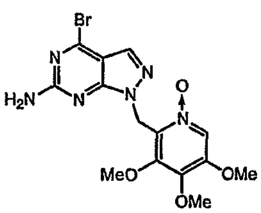,
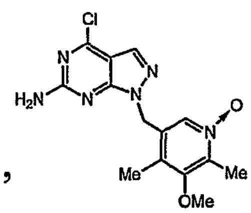,
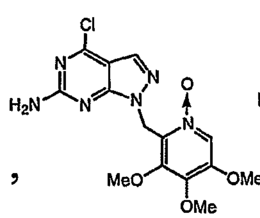
and
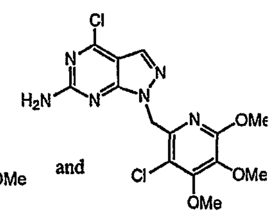

188. The compound of item 182, wherein said compound is a member selected from the group below, or a polymorph, solvate, ester, tautomer, enantiomers, pharmaceutically acceptable salt or prodrug thereof:

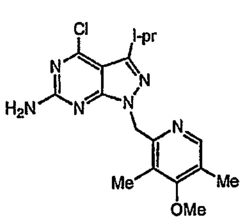,
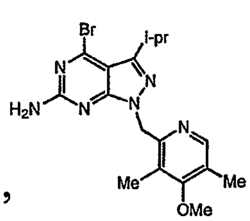,
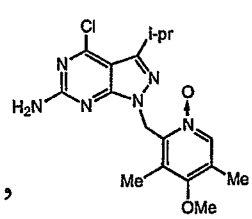,
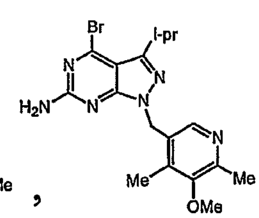

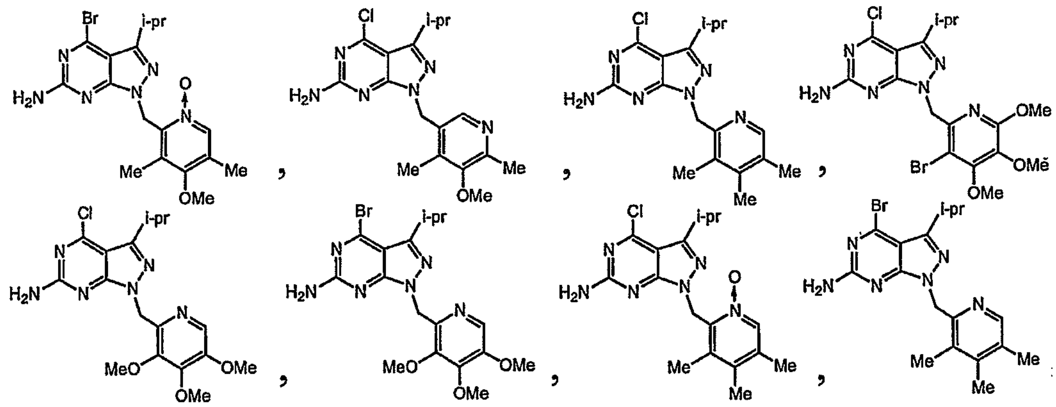

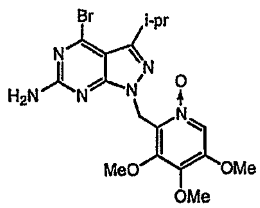,

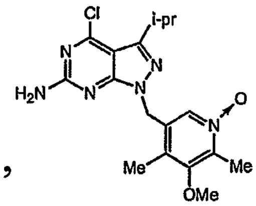,

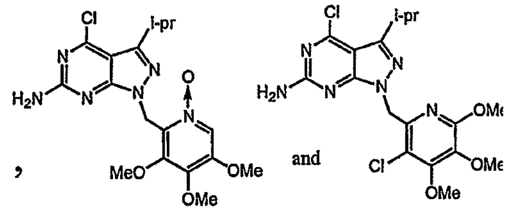

189. The compound of item 182, wherein said compound is a member selected from the group below, or a polymorph, solvate, ester, tautomer, enantiomers, pharmaceutically acceptable salt or prodrug thereof:

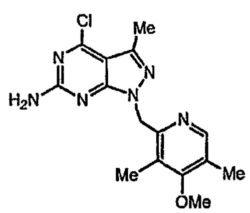,

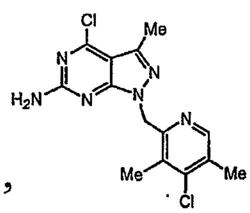,

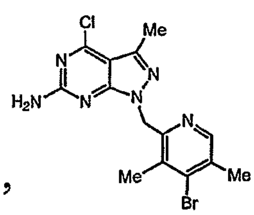,

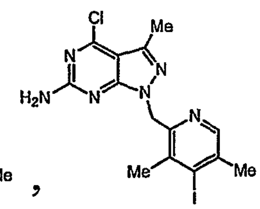;

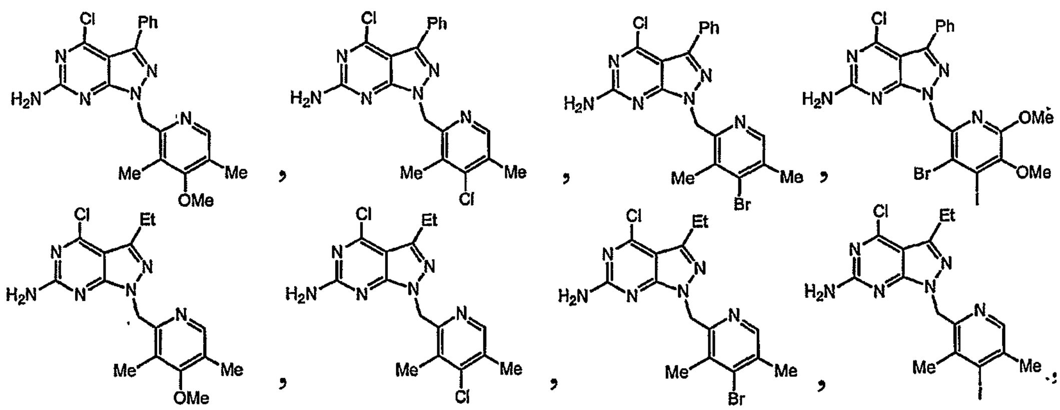

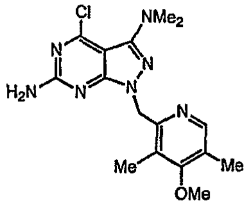, 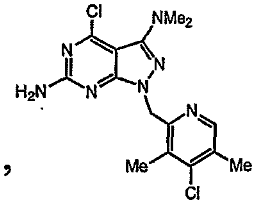, 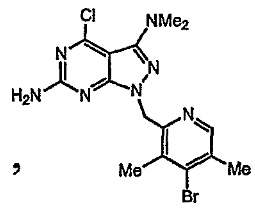 and 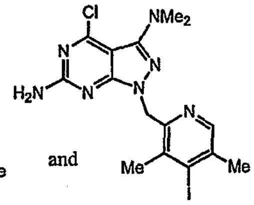

190. The compound of item 182, wherein said compound is a member selected from the group below, or a polymorph, solvate, ester, tautomer, enantiomers, pharmaceutically acceptable salt or prodrug thereof:

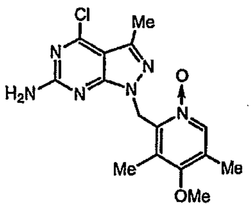, 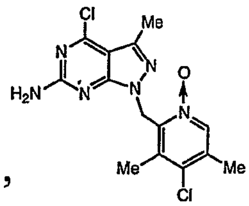, 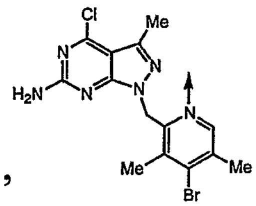, 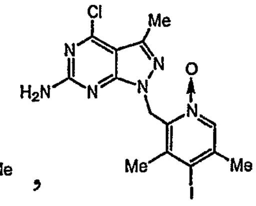

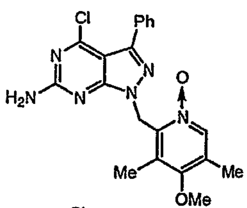, 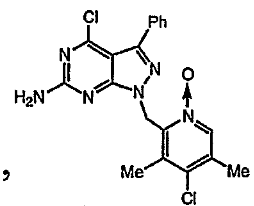, 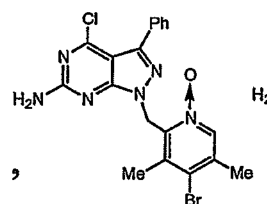, 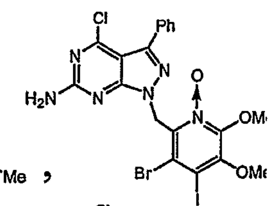

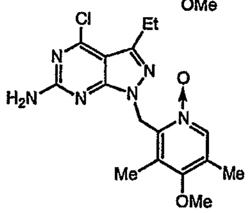, 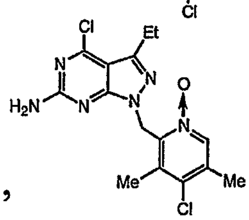, 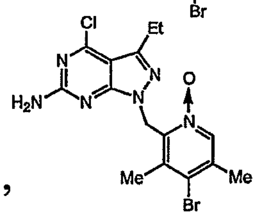, 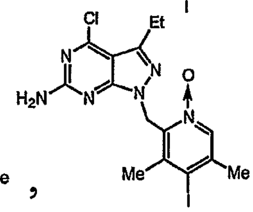

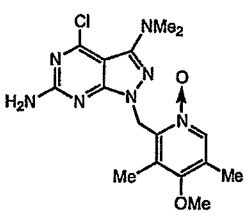, 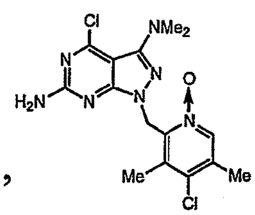, 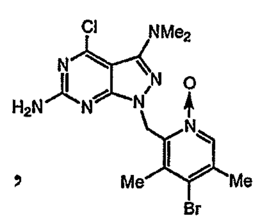 and 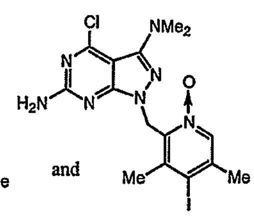

191. The compound of item 182, wherein said compound is a member selected from the group below, or a polymorph, solvate, ester, tautomer, enantiomers, pharmaceutically acceptable salt or prodrug thereof:

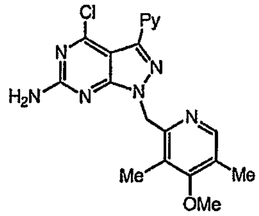, 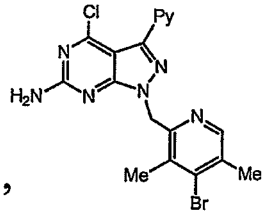, 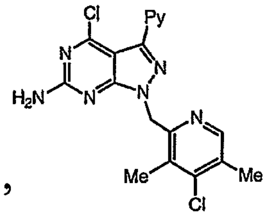,

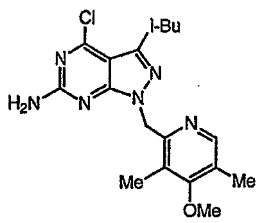 , 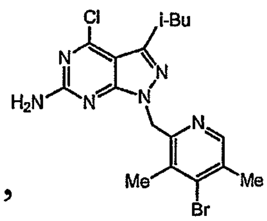 , 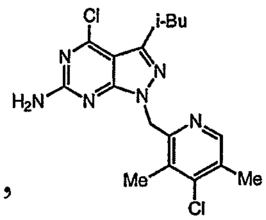 ,

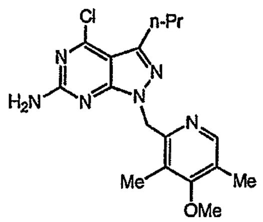 , 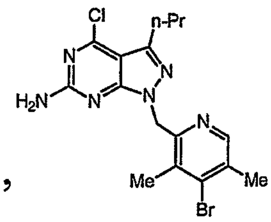 and 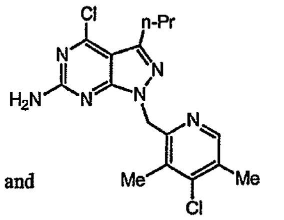

192. The compound of item 182, wherein said compound is a member selected from the group below, or a polymorph, solvate, ester, tautomer, enantiomers, pharmaceutically acceptable salt or prodrug thereof:

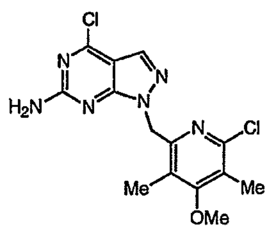 , 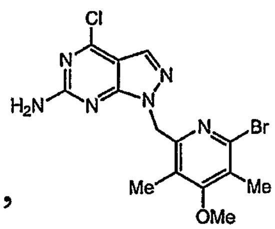 , 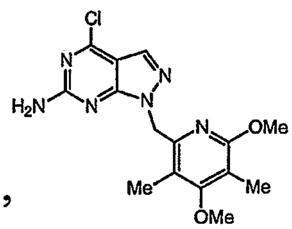 ,

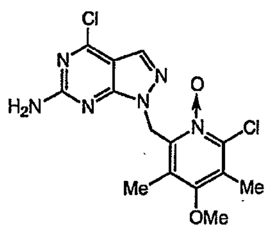 , 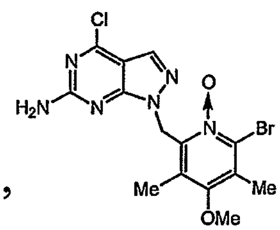 and 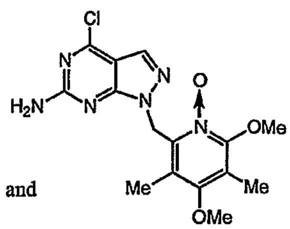

193. The compound of item 182, wherein said compound is a member selected from the group below, or a polymorph, solvate, ester, tautomer, enantiomers, pharmaceutically acceptable salt or prodrug thereof:

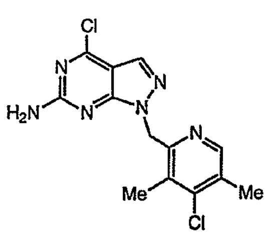 , 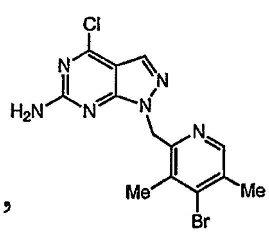 , 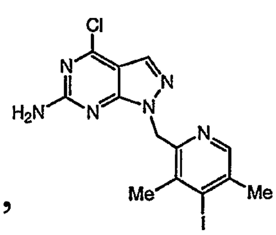 ,

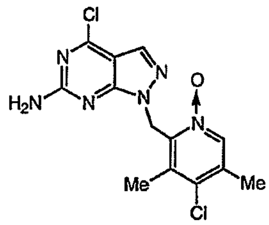, 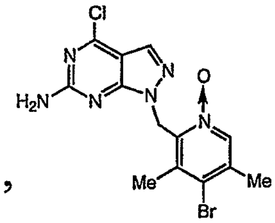 and 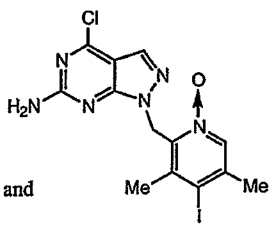

194. The compound of item 182, wherein said compound is a member selected from the group below, or a polymorph, solvate, ester, tautomer, enantiomers, pharmaceutically acceptable salt or prodrug thereof:

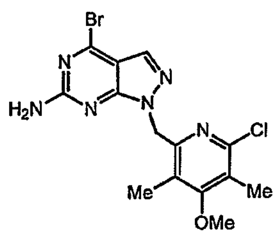, 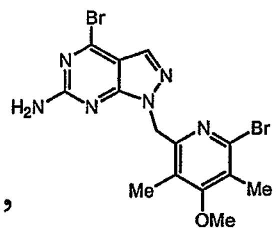, 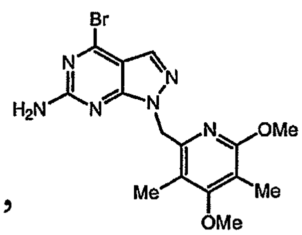,

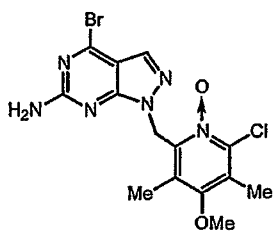, 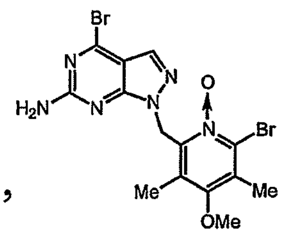 and 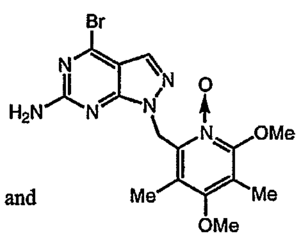

195. The compound of item 182, wherein said compound is a member selected from the group below, or a polymorph, solvate, ester, tautomer, enantiomers, pharmaceutically acceptable salt or prodrug thereof:

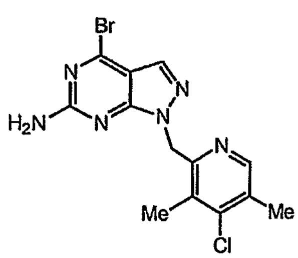, 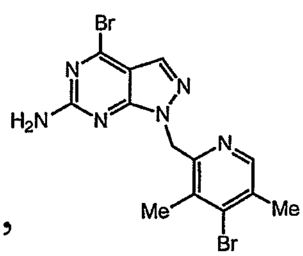, 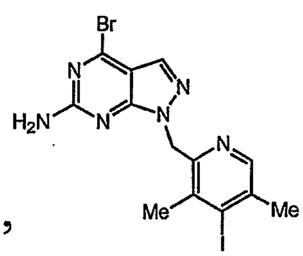,

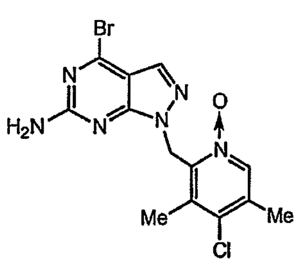, 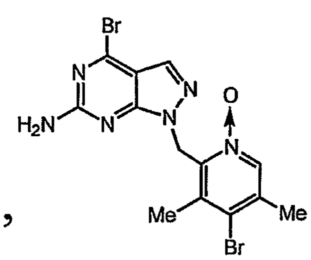 and 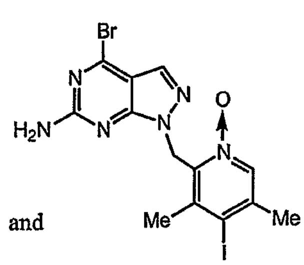

196. The compound of item 182, wherein said compound is represented by the formula below, or a polymorph, solvate, ester, tautomer, pharmaceutically acceptable salt or prodrug thereof:

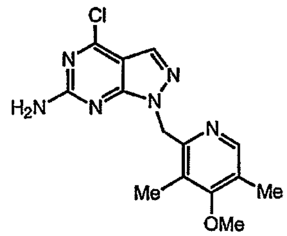

197. The compound of item 182, wherein said compound is represented by the formula below, or a polymorph, solvate, ester, tautomer, pharmaceutically acceptable salt or prodrug thereof:

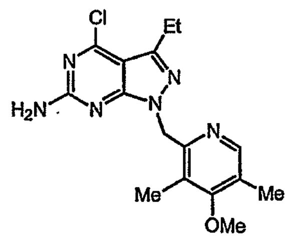

198. The compound of item 182, wherein said compound is represented by the formula below, or a polymorph, solvate, ester, tautomer, pharmaceutically acceptable salt or prodrug thereof:

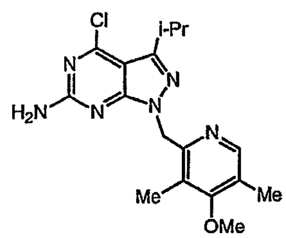

199. The compound of item 182, wherein said compound is represented by the formula below, or a polymorph, solvate, ester, tautomer, pharmaceutically acceptable salt or prodrug thereof:

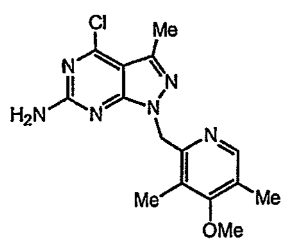

200. The compound of item 182, wherein said compound is represented by the formula below, or a polymorph, solvate, ester, tautomer, pharmaceutically acceptable salt or prodrug thereof:

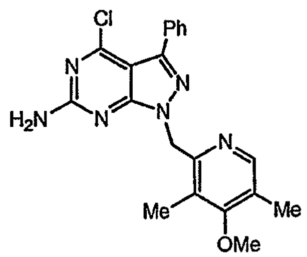

201. The compound of item 182, wherein said compound is represented by the formula below, or a polymorph, solvate, ester, tautomer, pharmaceutically acceptable salt or prodrug thereof:

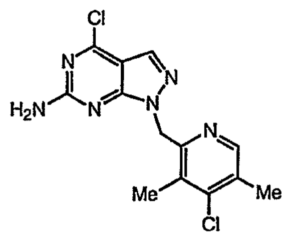

202. The compound of item 182, wherein said compound is represented by the formula below, or a polymorph, solvate, ester, tautomer, pharmaceutically acceptable salt or prodrug thereof:

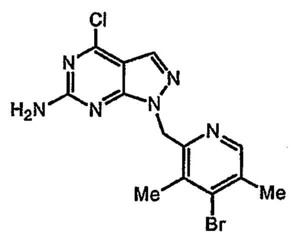

203. The compound of item 182, wherein said compound is represented by the formula below, or a polymorph, solvate, ester, tautomer, pharmaceutically acceptable salt or prodrug thereof:

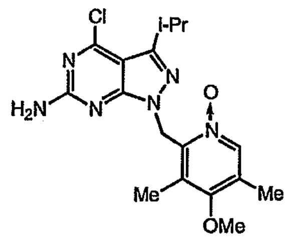

204. The compound of item 182, wherein said compound is represented by the formula below, or a polymorph, solvate, ester, tautomer, pharmaceutically acceptable salt or prodrug thereof:

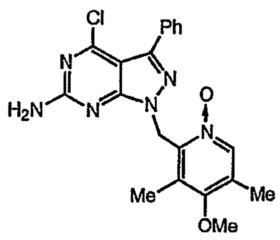

205. The compound of item 182, wherein said compound is represented by the formula below, or a polymorph, solvate, ester, tautomer, pharmaceutically acceptable salt or prodrug thereof:

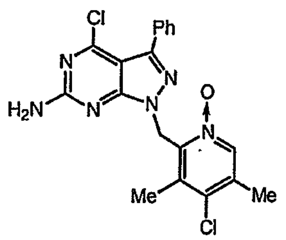

206. The compound of item 182, wherein said compound is represented by the formula below, or a polymorph, solvate, ester, tautomer, pharmaceutically acceptable salt or prodrug thereof:

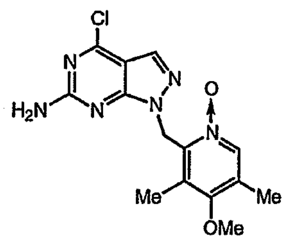

207. The compound of item 182, wherein said compound is represented by the formula below, or a polymorph, solvate, ester, tautomer, pharmaceutically acceptable salt or prodrug thereof:

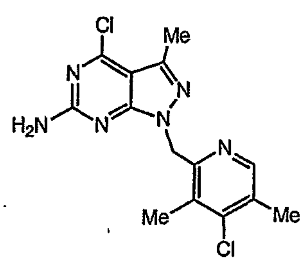

208. The compound of item 182, wherein said compound is represented by the formula below, or a polymorph, solvate, ester, tautomer, pharmaceutically acceptable salt or prodrug thereof:

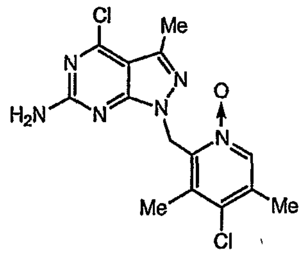

209. The compound of item 182, wherein said compound is represented by the formula below, or a polymorph, solvate, ester, tautomer, pharmaceutically acceptable salt or prodrug thereof:

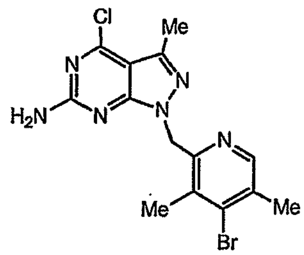

210. The compound of item 182, wherein said compound is represented by the formula below, or a polymorph, solvate, ester, tautomer, pharmaceutically acceptable salt or prodrug thereof:

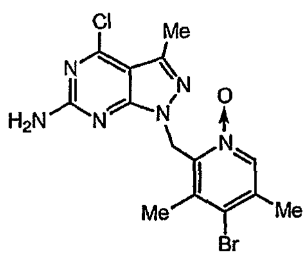

211. The compound of item 182, wherein said compound is represented by the formula below, or a polymorph, solvate, ester, tautomer, pharmaceutically acceptable salt or prodrug thereof:

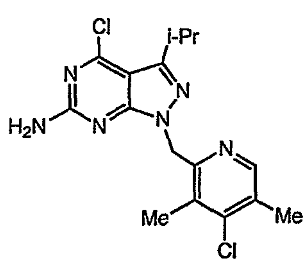

212. The compound of item 182, wherein said compound is represented by the formula below, or a polymorph, solvate, ester, tautomer, pharmaceutically acceptable salt or prodrug thereof:

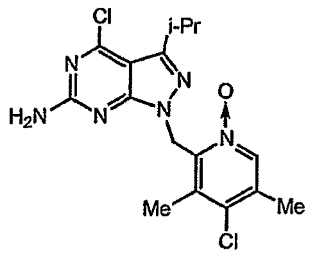

213. The compound of item 182, wherein said compound is represented by the formula below, or a polymorph, solvate, ester, tautomer, pharmaceutically acceptable salt or prodrug thereof:

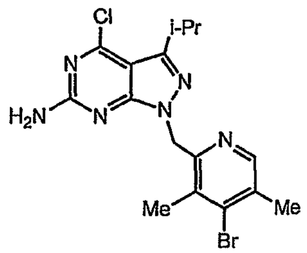

214. The compound of item 182, wherein said compound is represented by the formula below, or a polymorph, solvate, ester, tautomer, pharmaceutically acceptable salt or prodrug thereof:

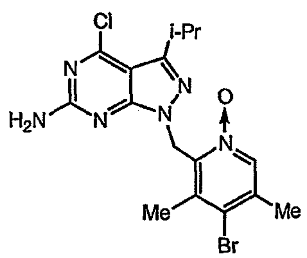

215. A pharmaceutical composition comprising one or more pharmaceutical acceptable excipients and at least one compound represented by Formula IIIA below, or a polymorph, solvate, ester, tautomer, pharmaceutically acceptable salt or prodrug thereof:

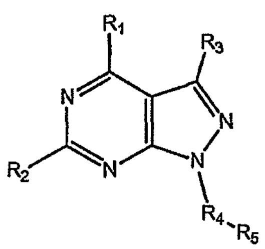

IIIA

wherein:

$R^1$ is halogen, $-OR^{11}$,$-SR^{11}$ or lower alkyl;
$R^2$ is $-NHR^8$;
$R^3$ is selected from the group consisting of hydrogen, halogen, $-SR^8$, $-OR^8$, -CN, - $C(O)R^9$,
$-CO_2H$, $-NO_2$, $-NR^8R^{10}$, lower alkyl, lower alkenyl, lower alkynyl, lower perhaloalkyl, aryl, heteroaryl, alicyclic and heterocyclic, all optionally substituted, wherein:

the aryl, heteroaryl, alicyclic and heterocyclic groups are optionally mono-, bi- or tri-cyclic,
$R^8$ and $R^{10}$ taken together optionally form a ring of 3-7 ring atoms and optionally
1-2 of the ring atoms are heteroatoms selected from the group of O, S and N, and
the optional substituents on $R^3$ are selected from the group consisting of halogen, lower alkyl, lower alkenyl, lower alkynyl, $-SR^8$, $-OR^8$, - CN, $-C(O)R^9$,
$-C(O)OH$, $-NO_2$, $-NR^8R^{10}$, lower aryl, lower heteroaryl, lower alicyclic, lower heterocyclic, arylalkyl, heteroarylalkyl, amino, alkylamino, dialkylamino, diarylalkylamino, oxo, oxa, perhaloalkyl, perhaloalkoxy, perhaloacyl, guanidine, pyridinyl, thiophene, furanyl, indole, indazole, phosphonates, phosphates, phosphoramides, sulfonates, sulfones, sulfates, sulphonamides, carbamates, ureas, thioureas and thioamides, wherein $R^8$ and $R^{10}$ taken together optionally form a ring of 3-7 ring atoms and optionally
1-3 of the ring atoms are heteroatoms selected from the group of O, S and N;

$R^4$ is $-CHR^{12}$-, -C(O), -C(S), -S(O)-, or $-SO_2$-;
$R^5$ is aryl, heteroaryl, alicyclic, or heterocyclic, wherein
the aryl group is substituted with 3 to 5 substituents,
the heteroaryl group is substituted with 2 to 5 substituents,
the alicyclic group is substituted with 3 to 5 substituents,
the heterocyclic group is substituted with 3 to 5 substituents, and
the substituents on $R^5$ are selected from the group consisting of halogen, lower alkyl, lower alkenyl, lower alkynyl, $-SR^8$, $-OR^8$, -CN, - C(O)OH, $-C(O)R^9$, $-NO_2$, $-NR^8R^{10}$, lower aryl, heteroaryl, alicyclic, lower heterocyclic, arylalkyl, heteroarylalkyl, amino, allcylamino, dialkylamino, diarylalkylamino, oxo, oxa, perhaloalkyl, perhaloalkoxy, perhaloacyl, guanidine, pyridinyl, thiophene, furanyl, indole, indazole, phosphonates, phosphates, phosphoramides, sulfonates, sulfones, sulfates, sulphonamides, carbamates, ureas, thioureas and thioamides, wherein $R^8$ and $R^{10}$ taken together optionally form a ring of 3-7 ring atoms and optionally 1-3 of the ring atoms are heteroatoms selected from the group of O, S and N;
$R^8$ is hydrogen, lower alkyl, lower alkenyl, lower alkynyl, lower aryl, lower heteroaryl or
$-C(O)R^9$;
$R^9$ is hydrogen, lower alkyl, lower alkenyl, lower alkynyl, lower aryl, lower heteroaryl, $NR^{10}R^{10}$ or $-OR^{11}$, $R^{10}$ and $R^{10}$ taken together optionally form a ring of 3-7 ring atoms and optionally 1-3 of the ring atoms are heteroatoms selected from the group of O, S and N;
$R^{10}$ is hydrogen, lower alkyl, lower alkenyl, lower alkynyl, lower aryl or lower heteroaryl;
$R^{11}$ is lower alkyl, lower alkenyl, lower alkynyl, lower aryl or lower heteroaryl;
and
$R^{12}$ is hydrogen or lower alkyl.

216. The pharmaceutical composition of claim 215, wherein:

$R^1$ is halogen or lower alkyl;
$R^2$ is $-NHR^8$, where $R^8$ is hydrogen or $-C(O)R^9$; and
$R^5$ is aryl or heteroaryl, wherein each of said aryl and heteroaryl groups is monocyclic or bicyclic.

217. The pharmaceutical composition of claim 215, wherein:

$R^1$ is halogen;
$R^2$ is $-NH_2$;
$R^3$ is hydrogen, halogen, $-SR^8$, $-OR^8$, lower alkyl, lower alkenyl, lower alkynyl, lower perhaloalkyl, lower aryl, lower heteroaryl, or $-NR^8R^{10}$ wherein $R^8$ and $R^{10}$ taken together optionally form a ring of 3-7 ring atoms and optionally 1-3 of the ring atoms are heteroatoms selected from the group of O, S and N;
$R^4$ is $-CH_2$-; and
$R^5$ is aryl or heteroaryl, wherein each of said aryl and heteroaryl groups is monocyclic or bicyclic.

218. The pharmaceutical composition of claim 217, wherein $R^1$ is chloro or bromo, $R^5$ is a phenyl having 3 to 5

substituents.

219. The pharmaceutical composition of claim 217, wherein $R^1$ is chloro or bromo, $R^5$ is a pyridyl having 3 to 5 substituents.

220. The pharmaceutical composition of claim 217, wherein $R^1$ is chloro or bromo, $R^5$ is an 1-oxy-pyridyl (N-oxy-pyridyl) having 3 to 5 substituents.

221. A method of treating an individual having an HSP90 mediated disorder comprising dministering to said individual a pharmaceutical composition comprising a pharmaceutically effective amount of a compound of Formula IIA:

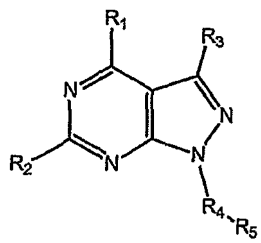

IIA

or a polymorph, solvate, ester, tautomer, pharmaceutically acceptable salt or prodrug thereof, wherein:

$R^1$ is halogen, $-OR^{11}$, $-SR^{11}$ or lower alkyl;
$R^2$ is $-NHR^8$;
$R^3$ is selected from the group consisting of hydrogen, halogen, $-SR^8$, $-OR^8$, -CN, - $C(O)R^9$, $-CO_2H$ $-NO_2$, $-NR^8R^{10}$, lower alkyl, lower alkenyl, lower alkynyl, lower perhaloalkyl, aryl, heteroaryl, alicyclic and heterocyclic, all optionally substituted, wherein:

the aryl, heteroaryl, alicyclic and heterocyclic groups are optionally mono-, bi- or tri-cyclic;
when $R^8$ and $R^{10}$ taken together optionally form a ring of 3-7 ring atoms and optionally 1-2 of the ring atoms are heteroatoms selected from the group of O, S and N, and
the optional substituents on $R^3$ are selected from the group consisting of halogen, lower alkyl, lower alkenyl, lower alkynyl, $-SR^8$, $-OR^8$,-CN,
$-C(O)R^9$, -C(O)OH, $-NO_2$, $-NR^8R^{10}$, lower aryl, lower heteroaryl, lower alicyclic, lower heterocyclic, arylalkyl, heteroarylalkyl, amino, alkylamino, dialkylamino, diarylalkylamino, oxo, oxa, perhaloalkyl, perhaloalkoxy, perhaloacyl, guanidine, pyridinyl, thiophene, furanyl, indole, indazole, phosphonates, phosphates, phosphoramides, sulfonates, sulfones, sulfates, sulphonamides, carbamates, ureas, thioureas and thioamides, wherein $R^8$ and $R^{10}$ taken together optionally form a ring of 3-7 ring atoms and optionally 1-3 of the ring atoms are heteroatoms selected from the group of O, S and N;

$R^4$ is $-CHR^{12}-$, -C(O), -C(S), -S(O)-, or $-SO_2-$;
$R^5$ is aryl, heteroaryl, alicyclic, or heterocyclic, wherein
the aryl group is substituted with 3 to 5 substituents,
the heteroaryl group is substituted with 2 to 5 substituents,
the alicyclic group is substituted with 3 to 5 substituents,
the heterocyclic group is substituted with 3 to 5 substituents, and
the substituents on $R^5$ are selected from the group consisting of halogen, lower alkyl, lower alkenyl, lower alkynyl, $-SR^8$, $-OR^8$, -CN, - C(O)OH, $-C(O)R^9$, $-NO_2$ and $-NR^8R^{10}$, lower aryl, heteroaryl, alicyclic, lower heterocyclic, arylalkyl, heteroarylalkyl, amino, alkylamino, dialkylamino, diarylalkylamino, oxo, oxa, perhaloalkyl, perhaloalkoxy, perhaloacyl, guanidine, pyridinyl, thiophene, furanyl, indole, indazole, phosphonates, phosphates, phosphoramides, sulfonates, sulfones, sulfates, sulphonamides, carbamates, ureas, thioureas and thioamides, wherein $R^8$ and $R^{10}$ taken together optionally form a ring of 3-7 ring atoms and optionally 1-3 of the ring atoms are heteroatoms selected from the group of O, S and N;
$R^8$ is hydrogen, lower alkyl, lower alkenyl, lower alkynyl, lower aryl, lower heteroaryl or

-C(O)$R^9$;
$R^9$ is hydrogen, lower alkyl, lower alkenyl, lower alkynyl, lower aryl, lower heteroaryl, -$NR^{10}R^{10}$ or -$OR^{11}$, $R^{10}$ and $R^{10}$ taken together optionally form a ring of 3-7 ring atoms and optionally 1-3 of the ring atoms are heteroatoms selected from the group of O, S and N;
$R^{10}$ is hydrogen, lower alkyl, lower alkenyl, lower alkynyl, lower aryl or lower heteroaryl;
$R^{11}$ is lower alkyl, lower alkenyl, lower alkynyl, lower aryl or lower heteroaryl;
and
$R^{12}$ is hydrogen or lower alkyl.

222. The method of claim 221, wherein:

$R^1$ is halogen or lower alkyl;
$R^2$ is -$NHR^8$, where $R^8$ is hydrogen or -C(O)$R^9$; and
$R^5$ is aryl or heteroaryl, wherein each of said aryl and heteroaryl groups is monocyclic or bicyclic.

223. The method of item 221, wherein:

$R^2$ is -$NH_2$;
$R^3$ is selected from hydrogen, halogen, -$SR^8$, -$OR^8$, -CN, -$NR^8R^{10}$, lower alkyl, lower alkenyl, lower alkynyl, lower perhaloalkyl, lower aryl, lower heteroaryl, lower alicyclic, lower heterocyclic, wherein $R^8$ is hydrogen, lower alkyl, lower alkenyl, lower alkynyl, lower aryl or lower heteroaryl, and wherein $R^8$ and $R^{10}$ taken together optionally form a ring of 3-7 ring atoms and optionally 1-3 of the ring atoms are heteroatoms selected from the group of O, S and N; and
$R^5$ is aryl or heteroaryl, wherein each of said aryl and heteroaryl groups is monocyclic or bicyclic.

224. The method of item 221, wherein:

$R^1$ is halogen or lower alkyl;
$R^2$ is -$NH_2$;
$R^4$ is -($CH_2$)-; and
$R^5$ is aryl, heteroaryl, alicyclic or heterocyclic, wherein each of said aryl, heteroaryl alicyclic or heterocyclic groups is monocyclic or bicyclic.

225. The method of item 221, wherein:

$R^1$ is halogen;
$R^2$ is -$NH_2$;
$R^3$ is hydrogen, halogen, -$SR^8$, -$OR^8$, lower alkyl, lower alkenyl, lower alkynyl, lower perhaloalkyl, lower aryl, lower heteroaryl, or -$NR^8R^{10}$ wherein $R^8$ and $R^{10}$ when taken together optionally form a ring of 3-7 ring atoms and optionally 1-3 of the ring atoms are heteroatoms selected from the group of O, S and N;
$R^4$ is -$CH_2$-; and
$R^5$ is aryl or heteroaryl, wherein each of said aryl and heteroaryl groups is monocyclic or bicyclic.

226. The method of item 225, wherein $R^1$ is chloro or bromo, $R^5$ is a phenyl having 3 to 5 substituents.

227. The method of item 225, wherein $R^1$ is chloro or bromo, $R^5$ is a pyridyl having 3 to 5 substituents.

228. The method of item 225, wherein $R^1$ is chloro or bromo, $R^5$ is an 1-oxy-pyridyl (N-oxy-pyridyl) having 3 to 5 substituents.

229. The method of item 221, wherein the HSP90-mediated disorder is selected from the group of inflammatory diseases, infections, autoimmune disorders, stroke, ischemia, cardiac disorders, neurological disorders, fibrogenetic disorders, proliferative disorders, tumors, leukemias, neoplasms, cancers, carcinomas, metabolic diseases, and malignant disease.

230. The method of item 229 wherein the fibrogenetic disorder is further selected from the group of scleroderma, polymyositis, systemic lupus, rheumatoid arthritis, liver cirrhosis, keloid formation, interstitial nephritis and pulmonary fibrosis.

231. The method of item 221 further comprising administering at least one therapeutic agent selected from the group of cytotoxic agents, anti-angiogenesis agents and anti-neoplastic agents.

232. The method of item 231 wherein the at least one anti-neoplastic agent is selected from the group of alkylating agents, anti-metabolites, epidophyllotoxins; antineoplastic enzymes, topoisomerase inhibitors, procarbazines, mitoxantrones, platinum coordination complexes, biological response modifiers and growth inhibitors, hormonal/anti-hormonal therapeutic agents, and haematopoietic growth factors.

233. A compound represented by Formula IIB, or a polymorph, solvate, ester, tautomer, pharmaceutically acceptable salt or prodrug thereof:

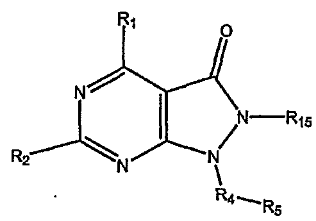

IIB

wherein:

$R^1$ is halogen, $-OR^{11}$, $-SR^{11}$ or lower alkyl;
$R^2$ is $NHR^8$;
$R^4$ is $-CHR^{12}-$, -C(O), -C(S), -S(O)-, or $-SO_2-$;
$R^5$ is aryl, heteroaryl, alicyclic, or heterocyclic, wherein
the aryl group is substituted with 3 to 5 substituents,
the heteroaryl group is substituted with 2 to 5 substituents,
the alicyclic group is substituted with 3 to 5 substituents,
the heterocyclic group is substituted with 3 to 5 substituents, and
the substituents on $R^5$ are selected from the group consisting of halogen, lower alkyl, lower alkenyl, lower alkynyl, $-SR^8$, $-OR^8$, -CN, - C(O)OH, $-C(O)R^9$, $-NO_2$ and $-NR^8R^{10}$, lower aryl, heteroaryl, alicyclic, lower heterocyclic, arylalkyl, heteroarylalkyl, amino, alkylamino, dialkylamino, diarylalkylamino, oxo, oxa, perhaloalkyl, perhaloalkoxy, perhaloacyl, guanidine; pyridinyl, thiophene, furanyl, indole, indazole, phosphonates, phosphates, phosphoramides, sulfonates, sulfones, sulfates, sulphonamides, carbamates, ureas, thioureas and thioamides, wherein $R^8$ and $R^{10}$ taken together optionally form a ring of 3-7 ring atoms and optionally 1-3 of the ring atoms are heteroatoms selected from the group of O, S and N;
$R^8$ is hydrogen, lower alkyl, lower alkenyl, lower alkynyl, lower aryl, lower heteroaryl or $-C(O)R^9$;
$R^9$ is hydrogen, lower alkyl, lower alkenyl, lower alkynyl, lower aryl, lower heteroaryl, $-NR^{10}R^{10}$ or $-OR^{11}$, $R^{10}$ and $R^{10}$ taken together optionally form a ring of 3-7 ring atoms and optionally 1-3 of the ring atoms are heteroatoms selected from the group of O, S and N;
$R^{10}$ is hydrogen, lower alkyl, lower alkenyl, lower alkynyl, lower aryl or lower heteroaryl;
$R^{11}$ is lower alkyl, lower alkenyl, lower alkynyl, lower aryl or lower heteroaryl;
$R^{12}$ is hydrogen or lower alkyl; and
$R^{15}$ is hydrogen, lower alkyl, lower alkenyl or lower alknyl.

234. The compound of item 233, or a polymorph, solvate, ester, tautomer, pharmaceutically acceptable salt or prodrug thereof:

$R^2$ is $-NHR^8$, where $R^8$ is hydrogen or $-C(O)R^9$;
$R^5$ is aryl, heteroaryl, alicyclic, or heterocyclic, all optionally mono-, bi- or tri-cyclic; and
$R^9$ is lower alkyl, lower alkenyl, lower alkynyl, lower aryl or lower heteroaryl.

235. The compound of item 233, or a polymorph, solvate, ester, tautomer, pharmaceutically acceptable salt or

prodrug thereof:

$R^1$ is halogen or lower alkyl;
$R^2$ is -NHR$^8$, where $R^8$ is hydrogen or -C(O)$R^9$;
$R^4$ is -$CH_2$-; and
$R^5$ is aryl, heteroaryl, alicyclic, or heterocyclic, all optionally mono-, bi- or tri-cyclic.

236. The compound of item 235, or a polymorph, solvate, ester, tautomer, enantiomer, pharmaceutically acceptable salt or prodrug thereof, wherein $R^1$ is chloro or bromo, $R^2$ is -$NH_2$, $R^5$ is a phenyl having 3 to 5 substituents.

237. The compound of item 235, or a polymorph, solvate, ester, tautomer, enantiomer, pharmaceutically acceptable salt or prodrug thereof, wherein $R^1$ is chloro or bromo, $R^2$ is -$NH_2$, $R^5$ is a pyridyl having 3 to 5 substituents.

238. The compound of item 235, or a polymorph, solvate, ester, tautomer, enantiomer, pharmaceutically acceptable salt or prodrug thereof, wherein $R^1$ is chloro or bromo, $R^2$ is -$NH_2$, $R^5$ is an 1-oxy-pyridyl (N-oxy-pyridyl) having 3 to 5 substituents.

239. A pharmaceutical composition comprising one or more pharmaceutical acceptable excipients and at least one compound represented by Formula IIB below, or a polymorph, solvate, ester, tautomer, pharmaceutically acceptable salt or prodrug thereof:

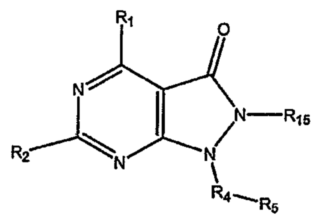

IIB

wherein

$R^1$ is halogen, -OR$^{11}$, -SR$^{11}$ or lower alkyl;
$R^2$ is -NHR$^8$;
$R^4$ is -CHR$^{12}$-, -C(O), -C(S), -S(O)-, or -$SO_2$-;
$R^5$ is aryl, heteroaryl, alicyclic, or heterocyclic, wherein
the aryl group is substituted with 3 to 5 substituents,
the heteroaryl group is substituted with 2 to 5 substituents,
the alicyclic group is substituted with 3 to 5 substituents,
the heterocyclic group is substituted with 3 to 5 substituents, and
the substituents on $R^5$ are selected from the group consisting of halogen, lower alkyl, lower alkenyl, lower alkynyl, -SR$^8$, -OR$^8$, -CN, - C(O)OH, -C(O)R$^9$, -$NO_2$ and -NR$^8$R$^{10}$, lower aryl, heteroaryl, alicyclic, lower heterocyclic, arylalkyl, heteroarylalkyl, amino, alkylamino, dialkylamino, diarylalkylamino, oxo, oxa, perhaloalkyl, perhaloalkoxy, perhaloacyl, guanidine, pyridinyl, thiophene, furanyl, indole, indazole, phosphonates, phosphates, phosphoramides, sulfonates, sulfones, sulfates, sulphonamides, carbamates, ureas, thioureas and thioamides, wherein $R^8$ and $R^{10}$ taken together optionally form a ring of 3-7 ring atoms and optionally 1-3 of the ring atoms are heteroatoms selected from the group of O, S and N;
$R^8$ is hydrogen, lower alkyl, lower alkenyl, lower alkynyl, lower aryl, lower heteroaryl or -C(O)$R^9$;
$R^9$ is hydrogen, lower alkyl, lower alkenyl, lower alkynyl, lower aryl, lower heteroaryl, -NR$^{10}$R$^{10}$ or -OR$^{11}$, $R^{10}$ and $R^{10}$ taken together optionally form a ring of 3-7 ring atoms and optionally 1-3 of the ring atoms are heteroatoms selected from the group of O, S and N;
$R^{10}$ is hydrogen, lower alkyl, lower alkenyl, lower alkynyl, lower aryl or lower heteroaryl;
$R^{11}$ is lower alkyl, lower alkenyl, lower alkynyl, lower aryl or lower heteroaryl;
$R^{12}$ is hydrogen or lower alkyl; and
$R^{15}$ is hydrogen, lower alkyl, lower alkenyl or lower alkyl.

63. A pharmaceutical composition of claim 62, wherein:

$R^1$is halogen or lower alkyl;
$R^2$ is -$NHR^8$, where $R^8$ is hydrogen or -C(O)$R^9$;
$R^4$ is -($CH_2$)-; and
$R^5$ is aryl, heteroaryl, alicyclic, or heterocyclic, all optionally mono-, bi- or tri-cyclic.

240. A method of treating an individual having an HSP90 mediated disorder comprising administering to said individual a pharmaceutical composition comprising a pharmaceutically effective amount of a compound of Formula IIB:

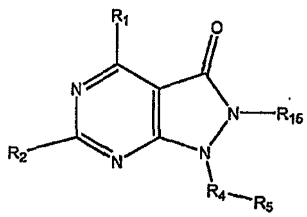

IIB

or a polymorph, solvate, ester, tautomer, pharmaceutically acceptable salt or prodrug thereof, wherein:

$R^1$ is halogen, -$OR^{11}$, -$SR^{11}$ or lower alkyl;
$R^2$ is -$NHR^8$;
$R^4$ is -$CHR^{12}$-, -C(O), -C(S), -S(O)-, or -SO2-;
$R^5$ is aryl, heteroaryl, alicyclic, or heterocyclic, wherein
the aryl group is substituted with 3 to 5 substituents,
the heteroaryl group is substituted with 2 to 5 substituents,
the alicyclic group is substituted with 3 to 5 substituents,
the heterocyclic group is substituted with 3 to 5 substituents, and
the substituents on $R^5$ are selected from the group consisting of halogen, lower alkyl, lower alkenyl, lower alkynyl, -$SR^8$, -$OR^8$, -CN, - C(O)OH, -C(O)$R^9$, -$NO_2$ and -$NR^8R^{10}$, lower aryl, heteroaryl, alicyclic, lower heterocyclic, arylalkyl, heteroarylalkyl, amino, alkylamino, dialkylamino, diarylalkylamino, oxo, oxa, perhaloalkyl, perhaloalkoxy, perhaloacyl, guanidine, pyridinyl, thiophene, furanyl, indole, indazole, phosphonates, phosphates, phosphoramides, sulfonates, sulfones, sulfates, sulphonamides, carbamates, ureas, thioureas and thioamides, wherein $R^8$ and $R^{10}$ taken together optionally form a ring of 3-7 ring atoms and optionally 1-3 of the ring atoms are heteroatoms selected from the group of O, S and N;
$R^8$ is hydrogen, lower alkyl, lower alkenyl, lower alkynyl, lower aryl, lower heteroaryl or -C(O)$R^9$;
$R^9$ is hydrogen, lower alkyl, lower alkenyl, lower alkynyl, lower aryl, lower heteroaryl, -$NR^{10}R^{10}$ or -$OR^{11}$, $R^{10}$ and $R^{10}$ taken together optionally form a ring of 3-7 ring atoms and optionally 1-3 of the ring atoms are heteroatoms selected from the group of O, S and N;
$R^{10}$ is hydrogen, lower alkyl, lower alkenyl, lower alkynyl, lower aryl or lower heteroaryl;
$R^{11}$ is lower alkyl, lower alkenyl, lower alkynyl, lower aryl or lower heteroaryl;
$R^{12}$ is hydrogen or lower alkyl; and
$R^{15}$ is hydrogen, lower alkyl, lower alkenyl or lower alknyl.

65. The method of claim 64, wherein:

5 $R^1$ is halogen or lower alkyl;
$R^2$ is -$NHR^8$, where $R^8$ is hydrogen or -C(O)$R^9$;
$R^4$ is -($CH_2$)-; and
$R^5$ is aryl, heteroaryl, alicyclic, or heterocyclic, all optionally mono-, bi- or tri-cyclic.

241. A compound, or a polymorph, solvate, ester, tautomer, pharmaceutically acceptable salt or prodrug thereof, prepared by the process comprising:

reacting a compound of Formula Y and a compound of Formula Z, wherein:
Y is represented by the following formulae:

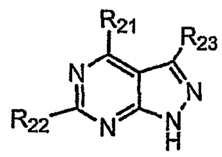 , 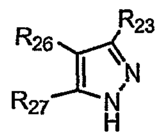 and 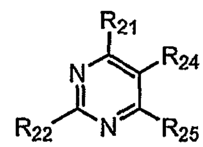 , and

Z is $L^1$-$R^4$-$R^5$; wherein:

$L^1$ is halogen, $NR^8R^{10}$, triflate, tosylate, or mesylate;
$R^4$ is -($CHR^{12}$)-, -C(O), -C(S), -S(O)-, or -$SO_2$-;
$R^5$ is aryl, heteroaryl, alicyclic, or heterocyclic, wherein
the aryl group is substituted with 3 to 5 substituents,
the heteroaryl group is substituted with 2 to 5 substituents,
the alicyclic group is substituted with 3 to 5 substituents,
the heterocyclic group is substituted with 3 to 5 substituents, and
the substituents on $R^5$ are selected from the group consisting of
halogen, lower alkyl, lower alkenyl, lower alkynyl, -$SR^8$, - $OR^8$, -CN, -C(O)OH, -C(O)$R^9$, -$NO_2$ and -$NR^8R^{10}$, lower aryl, heteroaryl, alicyclic, lower heterocyclic, arylalkyl, heteroarylalkyl, amino, alkylamino, dialkylamino, diarylalkylamino, oxo, oxa, perhaloalkyl, perhaloalkoxy, perhaloacyl, guanidine, pyridinyl, thiophene, furanyl, indole, indazole, phosphonates, phosphates, phosphoramides, sulfonates, sulfones, sulfates, sulphonamides, carbamates, ureas, thioureas and thioamides, wherein $R^8$ and $R^{10}$ taken together optionally form a ring of 3-7 ring atoms and optionally 1-3 of the ring atoms are heteroatoms selected from the group of O, S and N;
$R^8$ is hydrogen, lower alkyl, lower alkenyl, lower alkynyl, lower aryl, lower heteroaryl or -C(O)$R^9$;
$R^9$ is hydrogen, lower alkyl, lower alkenyl, lower alkynyl, lower aryl, lower heteroaryl, -$NR^{10}R^{10}$ or -$OR^{11}$, $R^{10}$ and $R^{10}$ taken together optionally form a ring of 3-7 ring atoms and optionally 1-3 of the ring atoms are heteroatoms selected from the group of O, S and N;
$R^{10}$ is hydrogen, lower alkyl, lower alkenyl, lower alkynyl, lower aryl or lower heteroaryl;
$R^{11}$ is lower alkyl, lower alkenyl, lower alkynyl, lower aryl or lower heteroaryl;
$R^{12}$ is hydrogen or lower alkyl;
$R^{21}$ is halogen, -$OR^8$, -$SR^8$ or lower alkyl;
$R^{22}$ is -$NR^8R^{10}$;
$R^{23}$ is hydrogen, -OH or its keto tautomer, -$OR^8$, halogen, -CN, lower alkyl, lower aryl or -C(O)$R^9$;
$R^{24}$ is -CHO, -$NH_2$, -$NO_2$ or -NO;
$R^{25}$ is halogen or -OH;
$R^{26}$ is -C(O)$NH_2$ or C(O)OEt; and
$R^{27}$ is -$NH_2$, -OH or halogen.

242. The compound of item 241, or a polymorph, solvate, ester, tautomer, pharmaceutically acceptable salt or prodrug thereof, wherein $R^5$ is aryl, heteroaryl, alicyclic, or heterocyclic, optionally mono- or bicyclic.

243. The compound of item 242, or a polymorph, solvate, ester, tautomer, pharmaceutically acceptable salt or prodrug thereof, wherein $L^1$ is -Cl, -Br or -$NH_2$; $R^4$ is -$CH_2$-; $R^5$ is aryl or heteroaryl.

244. The compound of item 242, or a polymorph, solvate, ester, tautomer, pharmaceutically acceptable salt or prodrug thereof, wherein Y is a pyrazolopyrimidine.

245. The compound of item 242, or a polymorph, solvate, ester, tautomer, pharmaceutically acceptable salt or prodrug thereof, wherein said reaction is performed in a solvent comprising a member selected from the group of DMF, THF and DMSO.

246. The compound of item 242, or a polymorph, solvate, ester, tautomer, pharmaceutically acceptable salt or prodrug thereof, wherein said reaction is performed in a solvent that comprises DMF.

247. A compound represented by Formula IVA, or a polymorph, solvate, ester, tautomer, enantiomer, diastereomer, pharmaceutically acceptable salt or prodrug thereof,

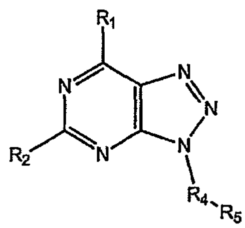

IVA

wherein:

$R^1$ is halogen, $-OR^{11}$, $-SR^{11}$ or lower alkyl;
$R^2$ is $-NHR^8$;
$R^4$ is $-CHR^{12}-$, -C(O)-, -C(S)-, -S(O)- or $-SO_2-$;
$R^5$ is aryl, heteroaryl, alicyclic, or heterocyclic, wherein:

the aryl group is substituted with 3 to 5 substituents,
the heteroaryl group is substituted with 2 to 5 substituents,
the alicyclic group is substituted with 3 to 5 substituents,
the heterocyclic group is substituted with 3 to 5 substituents, and
the substituents are selected from the group consisting of halogen, lower alkyl, lower alkenyl, lower alkynyl, $-SR^8$, $-OR^8$, -CN, -C(O)OH, - $C(O)R^9$, $-NO_2$ and $-NR^8R^{10}$, lower aryl, heteroaryl, alicyclic, lower heterocyclic, arylalkyl, heteroarylalkyl, amino, alkylamino, dialkylamino, diarylalkylamino, oxo, oxa, perhaloalkyl, perhaloalkoxy, perhaloacyl, guanidine, pyridinyl, thiophene, furanyl, indole, indazol, phosphonates, phosphates, phosphoramides, sulfonates, sulfones, sulfates, sulphonamides, carbamates, ureas, thioureas, thioamides, wherein $R^8$ and $R^{10}$ taken together optionally form a ring of 3-7 ring atoms and optionally 1-3 of the ring atoms are heteroatoms selected from the group of O, S and N;

$R^8$ is hydrogen, lower alkyl, lower alkenyl, or lower alkynyl, lower aryl, lower heteroaryl, or $-C(O)R^9$;
$R^9$ is H, lower alkyl, lower alkenyl, or lower alkynyl, lower aryl, lower heteroaryl,
$-NR^{10}R^{10}$, or $-OR^{11}$, wherein $R^{10}$ and $R^{10}$ taken together optionally form a ring of 3-7 ring atoms and optionally 1-3 of the ring atoms are heteroatoms selected from the group of O, S and N;
$R^{10}$ is hydrogen, lower alkyl, lower heteroaryl, lower aryl, lower alkenyl, or lower alkynyl,
$R^{11}$ is lower alkyl, lower alkenyl, lower alkynyl, lower heteroaryl or lower aryl; and $R^4$ is $-CHR^{12}-$, -C(O)-, -C(S)-, -S(O)- or $-SO_2-$; and
$R^{12}$ is hydrogen or lower alkyl;
provided that when $R^5$ is alicyclic, the ring system does not contain any tetra-substituted $sp^3$ ring carbons.

248. The compound of item 247, or a polymorph, solvate, ester, tautomer, enantiomer, diastereomer, pharmaceutically acceptable salt or prodrug thereof, wherein each of said aryl, heteroaryl, alicyclic or heterocyclic group is monocyclic or bicyclic.

249. The compound of item 247, or a polymorph, solvate, ester, tautomer, enantiomer, diastereomer, pharmaceutically acceptable salt or prodrug thereof, wherein:

$R^1$ is halogen; and
$R^2$ is $-NHR^8$, where $R^8$ is hydrogen or $-C(O)R^9$.

250. The compound of item 247, or a polymorph, solvate, ester, tautomer, enantiomer, pharmaceutically acceptable

salt or prodrug thereof, wherein:

$R^1$ is chloro or bromo,
$R^2$ is $-NHR^8$, where $R^8$ is hydrogen or $-C(O)R^9$; and
$R^4$ is $-CHR^{12}-$,

251. The compound of item 247, or a polymorph, solvate, ester, tautomer, enantiomer, pharmaceutically acceptable salt or prodrug thereof, wherein:

$R^2$ is $-NHR^8$, where $R^8$ is hydrogen or $-C(O)R^9$; and
$R^4$ is $-CH_2-$.

252. The compound of item 247, or a polymorph, solvate, ester, tautomer, enantiomer, pharmaceutically acceptable salt or prodrug thereof, wherein:

$R^1$ is chloro or bromo;
$R^2$ is $-NH_2$,
$R^4$ is $-CH_2-$; and
$R^5$ is aryl or heteroaryl, wherein each of the aryl and heteroaryl is monocyclic or bicyclic and is substituted with 3 to 5 substituents.

253. The compound of item 252, or a polymorph, solvate, ester, tautomer, enantiomer, pharmaceutically acceptable salt or prodrug thereof, wherein $R^1$ is chloro or bromo, $R^2$ is $-NH_2$, and $R^5$ is a phenyl having at least three substituent.

254. The compound of item 252, or a polymorph, solvate, ester, tautomer, enantiomer, pharmaceutically acceptable salt or prodrug thereof, wherein $R^1$ is chloro or bromo, $R^2$ is $-NH_2$ and $R^5$ is a pyridyl having at least two substituent.

255. The compound of item 252, or a polymorph, solvate, ester, tautomer, enantiomer, pharmaceutically acceptable salt or prodrug thereof, wherein $R^1$ is chloro or bromo, $R^2$ is $-NH_2$, and $R^5$ is 1-oxy-pyridyl (N-oxy-pyridyl) having at least two substituent.

256. The compound of item 252, wherein the compound is a member selected from the group below, or a polymorph, solvate, ester, tautomer, enantiomer, pharmaceutically acceptable salt or prodrug thereof:

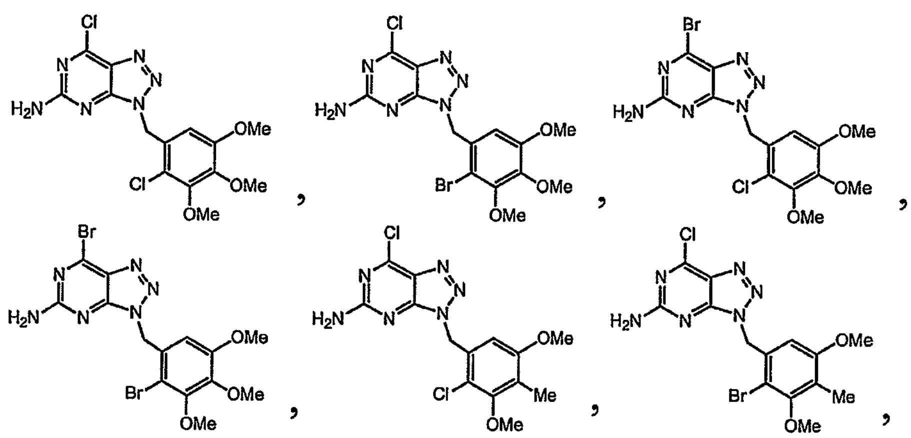
, , , , , ,

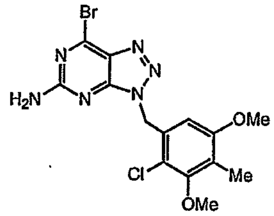, 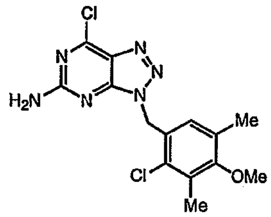 and 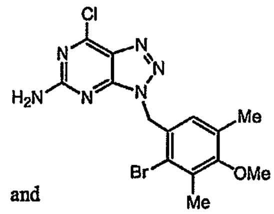

257. The compound of item 252, wherein the compound is a member selected from the group below, or a polymorph, solvate, ester, tautomer, enantiomer, pharmaceutically acceptable salt or prodrug thereof:

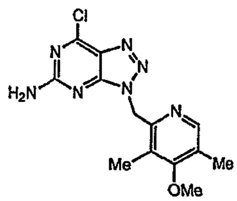, 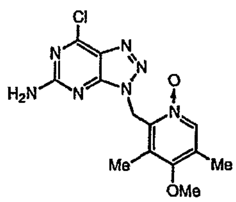, 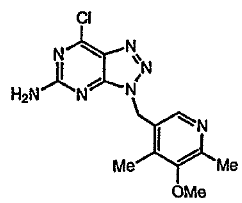, 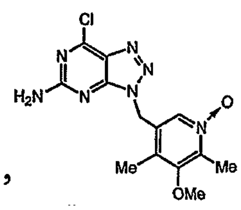,

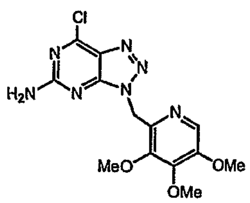, 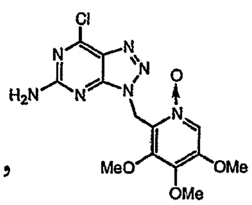, 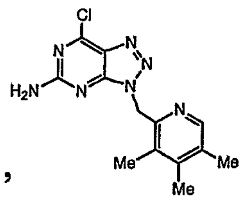, 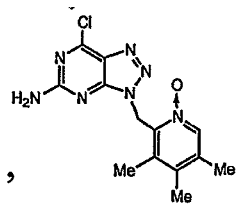,

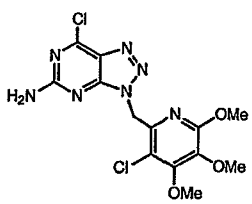, 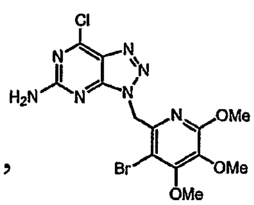, 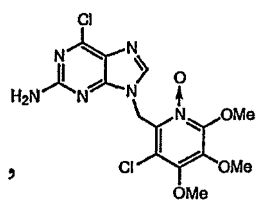 and 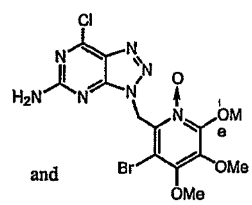

258. The compound of item 252, wherein the compound is a member selected from the group below, or a polymorph, solvate, ester, tautomer, enantiomer, pharmaceutically acceptable salt or prodrug thereof:

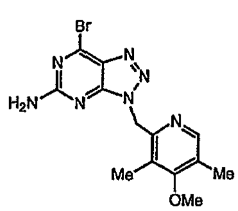, 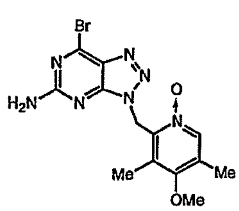, 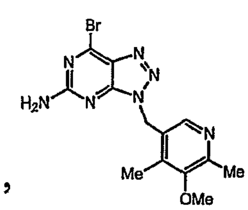, 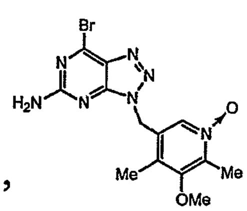,

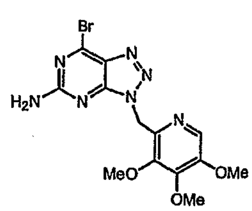, 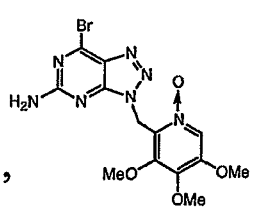, 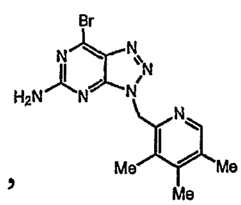, 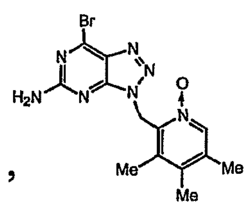,

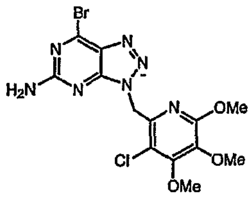, 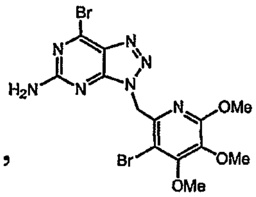, 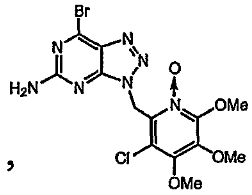 and 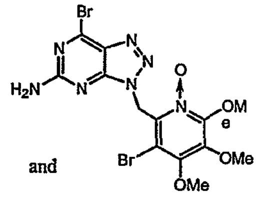.

259. The compound of item 252, wherein the compound is a member selected from the group below, or a polymorph, solvate, ester, tautomer, enantiomers, pharmaceutically acceptable salt or prodrug thereof:

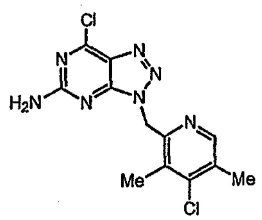, 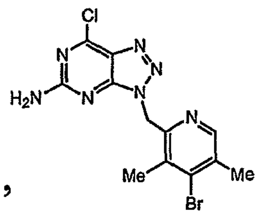, 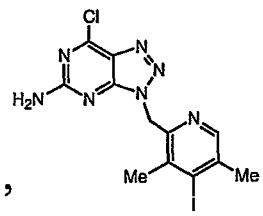,

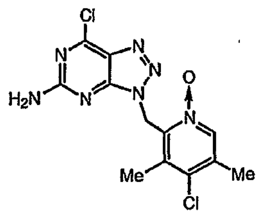, 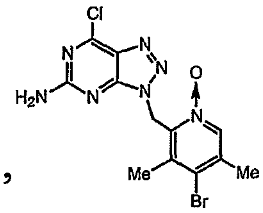 and 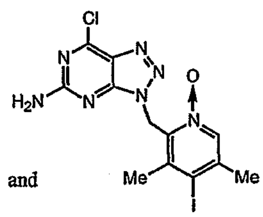

260. The compound of item 252, wherein the compound is a member selected from the group below, or a polymorph, solvate, ester, tautomer, enantiomers, pharmaceutically acceptable salt or prodrug thereof:

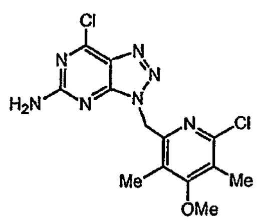, 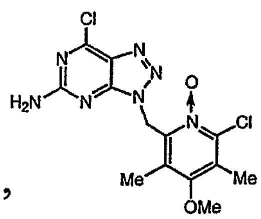, 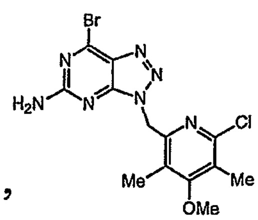,

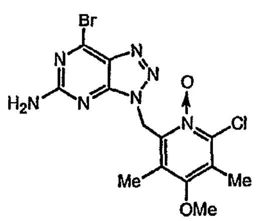, 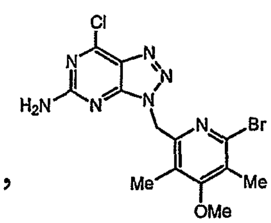 and 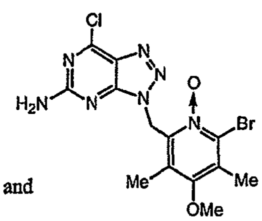

261. The compound of item 252, wherein the compound is a member selected from the group below, or a polymorph, solvate, ester, tautomer, enantiomers, pharmaceutically acceptable salt or prodrug thereof:

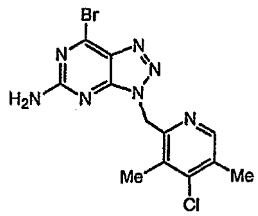 , 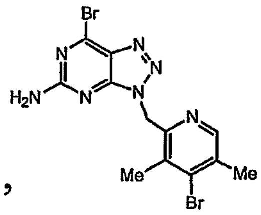 , 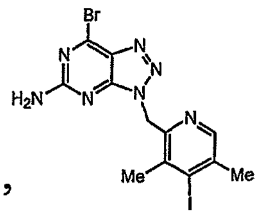 ,

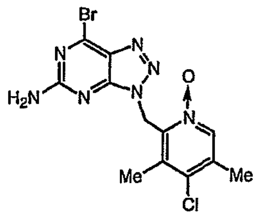 , 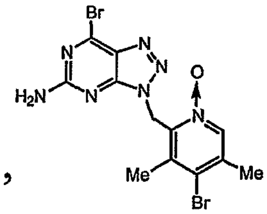 and 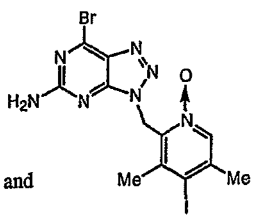

262. The compound of item 252, wherein the compound is represented by the formula below, or a polymorph, solvate, ester, tautomer, pharmaceutically acceptable salt or prodrug thereof:

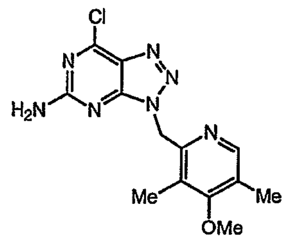

263. The compound of item 252, wherein the compound is represented by the formula below, or a polymorph, solvate, ester, tautomer, pharmaceutically acceptable salt or prodrug thereof:

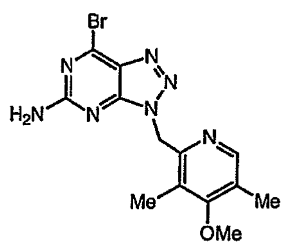

264. The compound of item 252, wherein the compound is represented by the formula below, or a polymorph, solvate, ester, tautomer, pharmaceutically acceptable salt or prodrug thereof:

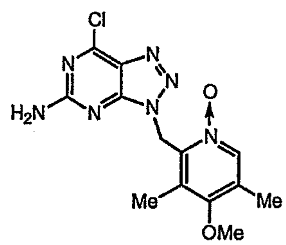

265. The compound of item 252, wherein the compound is represented by the formula below, or a polymorph, solvate, ester, tautomer, pharmaceutically acceptable salt or prodrug thereof:

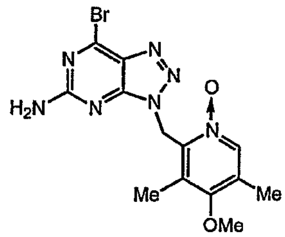

266. A pharmaceutical composition comprising one or more pharmaceutical acceptable excipient and at least one compound represented by Formula IVA below, or a polymorph, solvate, ester, tautomer, enantiomer, pharmaceutically acceptable salt or prodrug thereof,

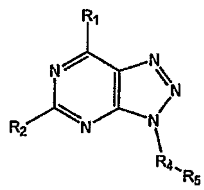

Formula IVA

wherein:

$R^1$ is halogen, $-OR^{11}$, $-SR^{11}$ or lower alkyl;
$R^2$ is $-NHR^8$;
$R^4$ is $-CHR^{12}-$, -C(O)-, -C(S)-, -S(O)- or $-SO_2-$;
$R^5$ is aryl, heteroaryl, alicyclic, or heterocyclic, wherein:

the aryl group is substituted with 3 to 5 substituents,
the heteroaryl group is substituted with 2 to 5 substituents,
the alicyclic group is substituted with 3 to 5 substituents,
the heterocyclic group is substituted with 3 to 5 substituents, and
the substituents are selected from the group consisting of halogen, lower alkyl, lower alkenyl, lower alkynyl, $-SR^8$, $-OR^8$, -CN, -C(O)OH, - $C(O)R^9$, $-NO_2$ and $-NR^8R^{10}$, lower aryl, heteroaryl, alicyclic, lower heterocyclic, arylalkyl, heteroarylalkyl, amino, alkylamino, dialkylamino, diarylalkylamino, oxo, oxa, perhaloalkyl, perhaloalkoxy, perhaloacyl, guanidine, pyridinyl, thiophene, furanyl, indole, indazol, phosphonates, phosphates, phosphoramides, sulfonates, sulfones, sulfates, sulphonamides, carbamates, ureas, thioureas, thioamides, wherein $R^8$ and $R^{10}$ taken together optionally form a ring of 3-7 ring atoms and optionally 1-3 of the ring atoms are heteroatoms selected from the group of O, S and N;

$R^8$ is hydrogen, lower alkyl, lower alkenyl, or lower alkynyl, lower aryl, lower heteroaryl, or $-C(O)R^9$;
$R^9$ is H, lower alkyl, lower alkenyl, or lower alkynyl, lower aryl, lower heteroaryl, $-NR^{10}R^{10}$ or $-OR^{11}$, wherein $R^{10}$ and $R^{10}$ taken together optionally form a ring of 3-7 ring atoms and optionally 1-3 of the ring atoms are heteroatoms selected from the group of O, S and N;
$R^{10}$ is hydrogen, lower alkyl, lower heteroaryl, lower aryl, lower alkenyl, or lower alkynyl,
$R^{11}$ is lower alkyl, lower alkenyl, lower alkynyl, lower heteroaryl or lower aryl; and $R_4$ is $-CHR^{12}-$, -C(O)-, -C(S)-, -S(O)- or $-SO_2-$; and
$R^{12}$ is hydrogen or lower alkyl;

provided that when $R^5$ is alicyclic, the ring system does not contain any tetra-substituted $sp^3$ ring carbons.

267. The pharmaceutical composition of item 266, wherein:

$R^1$ is halogen;
$R^2$ is $-NH_2$,
$R^4$ is $-CH_2-$; and
$R^5$ is aryl or heteroaryl, wherein each of the aryl and heteroaryl is monocyclic or bicyclic and is substituted with 3 to 5 substituents.

268. A method of treating an individual having an HSP90 mediated disorder comprising administering to said individual a pharmaceutical composition comprising a pharmaceutically effective amount of a compound of Formula IVA:

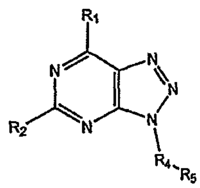

Formula IVA

or a polymorph, solvate, ester, tautomer, enantiomer, pharmaceutically acceptable salt or prodrug thereof, wherein:

$R^1$ is halogen, $-OR^{11}$, $-SR^{11}$ or lower alkyl;
$R^2$ is $-NHR^8$;
$R^4$ is $-CHR^{12}-$, -C(O)-, -C(S)-, -S(O)- or $-SO_2-$;
$R^5$ is aryl, heteroaryl, alicyclic, or heterocyclic, wherein:

the aryl group is substituted with 3 to 5 substituents,
the heteroaryl group is substituted with 2 to 5 substituents,
the alicyclic group is substituted with 3 to 5 substituents,
the heterocyclic group is substituted with 3 to 5 substituents, and
the substituents are selected from the group consisting of halogen, lower
alkyl, lower alkenyl, lower alkynyl, $-SR^8$, $-OR^8$, -CN, -C(O)OH, - $C(O)R^9$, $-NO_2$ and $-NR^8R^{10}$, lower aryl, heteroaryl, alicyclic, lower heterocyclic, arylalkyl, heteroarylalkyl, amino, alkylamino, dialkylamino, diarylalkylamino, oxo, oxa, perhaloalkyl, perhaloalkoxy, perhaloacyl, guanidine, pyridinyl, thiophene, furanyl, indole, indazol, phosphonates, phosphates, phosphoramides, sulfonates, sulfones, sulfates, sulphonamides, carbamates, ureas, thioureas, thioamides, wherein $R^8$ and $R^{10}$ taken together optionally form a ring of 3-7 ring atoms and optionally 1-3 of the ring atoms are heteroatoms selected from the group of O, S and N;

$R^8$ is hydrogen, lower alkyl, lower alkenyl, or lower alkynyl, lower aryl, lower heteroaryl, or $-C(O)R^9$;
$R^9$ is H, lower alkyl, lower alkenyl, or lower alkynyl, lower aryl, lower heteroaryl,
$-NR^{10}R^{10}$, or $-OR^{11}$, wherein $R^{10}$ and $R^{10}$ taken together optionally form a ring of 3-7 ring atoms and optionally 1-3 of the ring atoms are heteroatoms selected from the group of O, S and N;
$R^{10}$ is hydrogen, lower alkyl, lower heteroaryl, lower aryl, lower alkenyl, or lower alkynyl,
$R^{11}$ is lower alkyl, lower alkenyl, or lower alkynyl, lower heteroaryl or lower aryl; and $R_4$ is $-CHR^{12}-$, -C(O)-, -C(S)-, -S(O)- or $-SO_2-$; and
$R^{12}$ is hydrogen or lower alkyl;
provided that when $R^5$ is alicyclic, the ring system does not contain any tetra-substituted $sp^3$ ring carbons.

269. The method of item 268, wherein:

$R^1$ is halogen;
$R^2$ is $-NH_2$,
$R^4$ is $-CH_2-$; and
$R^5$ is aryl or heteroaryl, wherein each of the aryl and heteroaryl is monocyclic or bicyclic and is substituted with 3 to 5 substituents.

270. The method of item 268, wherein $R^1$ is chloro or bromo, $R^2$ is $-NH_2$, and $R^5$ is a phenyl having at least three substituent.

271. The method of item 268, wherein $R^1$ is chloro or bromo, $R^2$ is $-NH_2$ and $R^5$ is a pyridyl having at least two

substituent.

272. The method of item 268, wherein $R^1$ is chloro or bromo, $R^2$ is $-NH_2$, and $R^5$ is an 1-oxy-pyridyl (N-oxy-pyridyl) having at least two substituent.

273. The method of item 268, wherein the HSP90 mediated disorder is selected from the group of inflammatory diseases, infections, autoimmune disorders, stroke, ischemia, cardiac disorder, neurological disorders, fibrogenetic disorders, proliferative disorders, tumors, leukemias, neoplasms, cancers, carcinomas, metabolic diseases, and malignant disease.

274. The method of item 273 wherein the fibrogenetic disorder is further selected from the group of scleroderma, polymyositis, systemic lupus, rheumatoid arthritis, liver cirrhosis, keloid formation, interstitial nephritis and pulmonary fibrosis.

275. The method of item 268, further comprising administering at least one therapeutic agent selected from the group of cytotoxic agents, anti-angiogenesis agents and anti-neoplastic agents.

276. The method of item 275, wherein the at least one anti-neoplastic agent is selected from the group of alkylating agents, anti-metabolites, epidophyllotoxins; antineoplastic enzymes, topoisomerase inhibitors, procarbazines, mitoxantrones, platinum coordination complexes, biological response modifiers and growth inhibitors, hormonal/anti-hormonal therapeutic agents, and haematopoietic growth factors.

277. A compound, or a polymorph, solvate, ester, tautomer, enantiomer, pharmaceutically acceptable salt or prodrug thereof, prepared by the process comprising:
reacting a compound of formula Y and a compound of formula Z, wherein:
Y is a represented by any one of the following formulae:

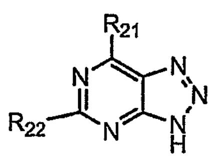 , 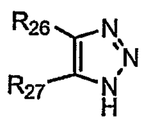 and 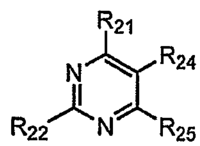 ,

Z is $L^1$-$R^4$-$R^5$; wherein:

$L^1$ is halogen, $NR^8R^{10}$, triflate, tosylate, or mesylate;
$R^4$ is $-CHR^{12}-$, -C(O)-, -C(S)-, -S(O)- or $-SO_2-$;
$R^5$ is aryl, heteroaryl, alicyclic, or heterocyclic, wherein:

the aryl group is substituted with 3 to 5 substituents,
the heteroaryl group is substituted with 2 to 5 substituents,
the alicyclic group is substituted with 3 to 5 substituents,
the heterocyclic group is substituted with 3 to 5 substituents, and
the substituents are selected from the group consisting of halogen, lower alkyl, lower alkenyl, lower alkynyl, $-SR^8$, $-OR^8$,-CN, -C(O)OH, $-C(O)R^9$, $-NO_2$ and $-NR^8R^{10}$, lower aryl, heteroaryl, alicyclic, lower heterocyclic, arylalkyl, heteroarylalkyl, amino, alkylamino, dialkylamino, diarylalkylamino, oxo, oxa, perhaloalkyl, perhaloalkoxy, perhaloacyl, guanidine, pyridinyl, thiophene, furanyl, indole, indazol, phosphonates, phosphates, phosphoramides, sulfonates, sulfones, sulfates, sulphonamides, carbamates, ureas, thioureas, thioamides, wherein $R^8$ and $R^{10}$ taken together optionally form a ring of 3-7 ring atoms and optionally 1-3 of the ring atoms are heteroatoms selected from the group of O, S and N;

$R^8$ is hydrogen, lower alkyl, lower alkenyl, or lower alkynyl, lower aryl, lower heteroaryl, or $-C(O)R^9$;
$R^9$ is H, lower alkyl, lower alkenyl, or lower alkynyl, lower aryl, lower heteroaryl,
$-NR^{10}R^{10}$, or $-OR^{11}$, wherein $R^{10}$ and $R^{10}$ taken together optionally form a ring of 3-7 ring atoms and optionally 1-3 of the ring atoms are heteroatoms selected from the group of O, S and N;
$R^{10}$ is hydrogen, lower alkyl, lower heteroaryl, lower aryl, lower alkenyl, or lower alkynyl,
$R^{11}$ is lower alkyl, lower alkenyl, or lower alkynyl, lower heteroaryl or lower aryl;

$R^{12}$ is hydrogen or lower alkyl,
$R^{21}$ is halogen, $-OR^8$, $-SR^8$ or lower alkyl;
$R^{22}$ is $-NR^8R^{10}$;
$R^{24}$ is $-NH_2$, $-NO_2$ or -NO;
$R^{25}$ is halogen or -OH;
$R^{26}$ is $-C(O)NH_2$ or C(O)OEt; and
$R^{27}$ is $-_{NH2}$, -OH or halogen;
provided that when $R^5$ is alicyclic, the ring system does not contain any tetra-substituted $sp^3$ ring carbons.

278. The compound of item 276, or a polymorph, solvate, ester, tautomer, enantiomer, pharmaceutically acceptable salt or prodrug thereof, wherein $L^1$ is -Cl, -Br or $-NH_2$; $R^5$ is aryl or heteroaryl.

279. The compound of item 278, or a polymorph, solvate, ester, tautomer, enantiomer, pharmaceutically acceptable salt or prodrug thereof, $R^4$ is $-CH_2-$.

280. The compound of item 278, or a polymorph, solvate, ester, tautomer, enantiomer, pharmaceutically acceptable salt or prodrug thereof, wherein said reaction is performed in a solvent comprising a member selected from the group of DMF, THF and DMSO.

281. The compound of item 278, or a polymorph, solvate, ester, tautomer, enantiomer, pharmaceutically acceptable salt or prodrug thereof, wherein said reaction is performed in a solvent that comprises DMF.

## Claims

**1.** A compound, tautomer, pharmaceutically acceptable salt thereof, or prodrug thereof having Formula I wherein

$X_1$ and $X_2$ are each nitrogen;
$R^1$ is halogen, $-OR^8$, $-SR^8$, or lower alkyl;
$R^2$ is $-NR^8R^{10}$;
$R^4$ is $-(CH_2)_n-$ where n = 0-3;
$R^6$ is hydrogen, halogen, lower alkyl, $-SR^8$, $-OR^8$, $-NR^8R^{10}$, $-N_3$, -CN, $-C(O)R^9$,
$R^5$ is alkyl, aromatic, heteroaromatic, alicyclic, heterocyclic, all optionally bi- or tricyclic, and all optionally substituted with H, halogen, lower alkyl, $-SR^8$, $-OR^8$, -CN, $-CO_2R^9$, $-NO_2$, or $-NR^8R^{10}$;
$R^8$ is hydrogen, lower alkyl, lower aryl, or $-(CO)R^9$;
$R^9$ is lower alkyl, lower aryl, lower heteroaryl, $-NR^8R^{10}$ or $-OR^{11}$;
$R^{11}$ is lower alkyl or lower aryl; and
$R^{10}$ is hydrogen or lower alkyl.

**2.** A compound, tautomer, pharmaceutically acceptable salt thereof, or prodrug thereof having Formula IA:

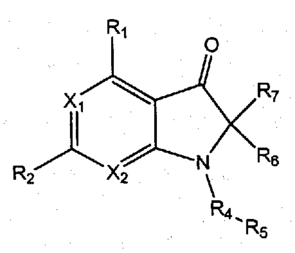

IA

wherein:

$X_1$ and $X_2$ are each nitrogen;
$R^1$ is halogen, $-OR^8$, $-SR^8$, or lower alkyl;

$R^2$ is $-NR^8R^{10}$;
$R^4$ is $-(CH_2)_n-$ where n = 0-3;
$R^5$ is alkyl, aromatic, heteroaromatic, alicyclic, heterocyclic, all optionally bi- or tricyclic, and all optionally substituted with H, halogen, lower alkyl, $-SR^8$, $-OR^8$, -CN, $-CO_2R^9$, $-NO_2$, or $-NR^8R^{10}$;
$R^6$ is hydrogen, halogen, lower alkyl, $-SR^8$, $-OR^8$, $-NR^8R^{10}$, $-N_3$, -CN, $-C(O)R^9$, or together with $R^7$ is carbonyl (C=O);
$R^7$ is independently selected from hydrogen, lower alkyl or together with $R^6$ is carbonyl (C=O);
$R^8$ is hydrogen, lower alkyl, lower aryl, or $-(CO)R^9$;
$R^9$ is lower alkyl, lower aryl, lower heteroaryl, $-NR^8R^{10}$ or $-OR^{11}$; $R^{11}$ is lower alkyl or lower aryl; and
$R^{10}$ is hydrogen or lower alkyl.

**3.** A compound represented by Formula I, or a polymorph, solvate, ester, tautomer, enantiomer, pharmaceutically acceptable salt or prodrug thereof:

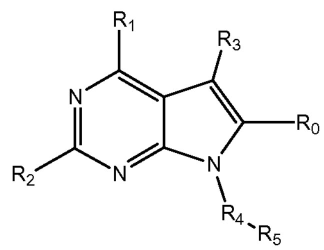

I

wherein:

$R^0$ is selected from hydrogen, halogen, lower alkyl, $-SR^8$, $-OR^8$, -CN, and $-NHR^8$, $R^1$ is halogen, $-OR^{11}$, $-SR^{11}$ or lower alkyl;
$R^2$ is $-NHR^8$;
$R^3$ is selected from the group consisting of hydrogen, halogen, $-SR^8$, $-OR^8$, -CN, $-C(O)R^9$, -C(O)OH, $-NO_2$, $-NR^8R^{10}$, lower alkyl, lower alkenyl, lower alkynyl, lower perhaloalkyl, aryl, heteroaryl, alicyclic and heterocyclic, all optionally substituted, wherein:

the aryl, heteroaryl, alicyclic and heterocyclic groups are optionally mono-, bi- or tri-cyclic,
$R^8$ and $R^{10}$ taken together optionally form a ring of 3-7 ring atoms and
optionally 1-3 of the ring atoms are heteroatoms selected from the group of O, S and N, and
the optional substituents on $R^3$ are selected from the group consisting of halogen, lower alkyl, lower alkenyl, lower alkynyl, $-SR^8$, $-OR^8$, -CN, -C(O)OH, $-C(O)R^9$, $-NO_2$, $-NR^8R^{10}$, lower aryl, heteroaryl, alicyclic, lower heterocyclic, arylalkyl, heteroarylalkyl, amino, alkylamino, dialkylamino, diarylalkylamino, oxo, oxa, perhaloalkyl, perhaloalkoxy, perhaloacyl, guanidine, pyridinyl, thiophene, furanyl, indole, indazole, phosphonates, phosphates, phosphoramides, sulfonates, sulfones, sulfates,
sulphonamides, carbamates, ureas, thioureas and thioamides, wherein
$R^8$ and $R^{10}$ taken together optionally form a ring of 3-7 ring atoms and
optionally 1-3 of the ring atoms are heteroatoms selected from the group of O, S and N;

$R^0$ or $R^3$ is -OH or -SH, the compound may exist as the corresponding (thio)keto tautomer or a mixture of keto-enol tautomers;
$R^4$ is $-CHR^{12}-$;
$R^5$ is aryl, heteroaryl, alicyclic, or heterocyclic, wherein
the aryl group is substituted with 3 to 5 substituents,
the heteroaryl group is substituted with 2 to 5 substituents,
the alicyclic group is substituted with 3 to 5 substituents,
the heterocyclic group is substituted with 3 to 5 substituents, and
the substituents are selected from the group consisting of halogen, lower alkyl, lower alkenyl, lower alkynyl, $-SR^8$, $-OR^8$, -CN, -C(O)OH, $-C(O)R^9$, $-NO_2$, $-NR^8R^{10}$, lower aryl, heteroaryl, alicyclic, lower heterocyclic, arylalkyl, heteroarylalkyl, amino, alkylamino, dialkylamino, diarylalkylamino, oxo, oxa, perhaloalkyl, perhaloalkoxy,

perhaloacyl, guanidine, pyridinyl, thiophene, furanyl, indole, indazole, phosphonates, phosphates, phosphoramides, sulfonates, sulfones, sulfates, sulphonamides, carbamates, ureas, thioureas and thioamides, wherein $R^8$ and $R^{10}$ taken together optionally form a ring of 3-7 ring atoms and
optionally 1-3 of the ring atoms are heteroatoms selected from the group of O, S and N;
$R^8$ is hydrogen, lower alkyl, lower alkenyl, or lower alkynyl, lower aryl, lower heteroaryl, or -C(O)$R^9$;
$R^9$ is H, lower alkyl, lower alkenyl, lower alkynyl, lower aryl, lower heteroaryl, -$NR^{10}R^{10}$, or -$OR^{11}$, wherein $R^{10}$ and $R^{10}$ taken together optionally form a ring of 3-7 ring atoms and optionally 1-3 of the ring atoms are heteroatoms selected
from the group of O, S and N;
$R^{10}$ is hydrogen, lower alkyl, lower alkenyl, lower alkynyl, lower aryl or lower heteroaryl;
$R^{11}$ is lower alkyl, lower alkenyl, lower alkynyl, lower heteroaryl or lower aryl; and
$R^{12}$ is hydrogen or lower alkyl.

**4.** The compound of claim 3, or a polymorph, solvate, ester, tautomer, enantiomer, pharmaceutically acceptable salt or prodrug thereof, wherein:

$R^0$ is hydrogen, halogen, -SH, -OH or-CN;
$R^1$ is halogen;
$R^2$ is -$NH_2$;
$R^3$ is hydrogen, halogen, -$OR^8$, -$SR^8$, -$NR^8R^{10}$, lower alkyl, lower alkenyl, lower alkynyl, lower perhaloalkyl, lower aryl, or lower heteroaryl, wherein $R^8$ is hydrogen, lower alkyl, lower aryl, or -C(O)$R^9$;
$R^4$ is -$CH_2$-; and
$R^5$ is aryl or heteroaryl, wherein each of said aryl and heteroaryl is monocyclic or bicyclic and is substituted with 3 to 5 substituents.

**5.** The compound of claim 4, or a polymorph, solvate, ester, tautomer, enantiomer, pharmaceutically acceptable salt or prodrug thereof, wherein $R^1$ is chloro or bromo, $R^2$ is -$NH_2$, and $R^5$ is a phenyl having at least three substituents, a pyridyl having at least two substituents; or 1-oxy-pyridyl (N-oxy-pyridyl) having at least two substituents.

**6.** A compound selected from the group below, or a polymorph, solvate, ester, tautomer, enantiomer, pharmaceutically acceptable salt or prodrug thereof:

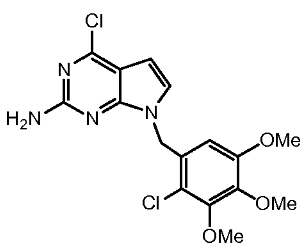
,
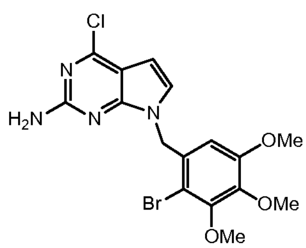
,
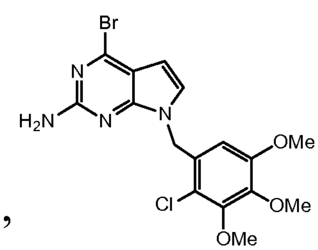
,

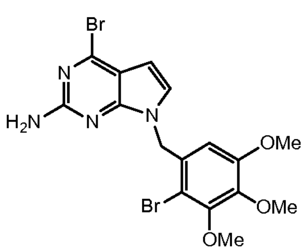
,
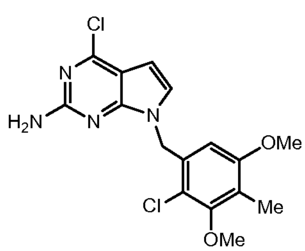
,
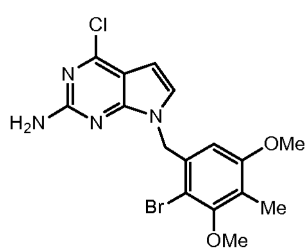
,

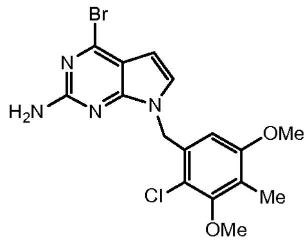,
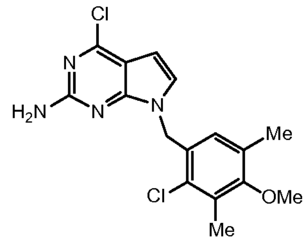
and
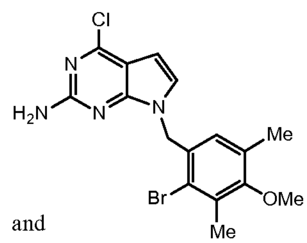

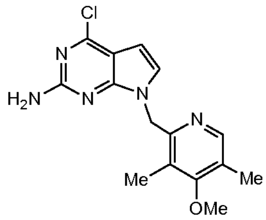,
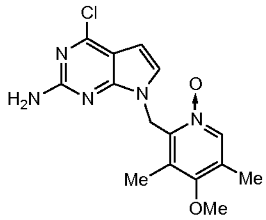,
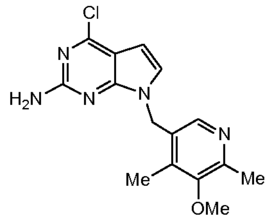,
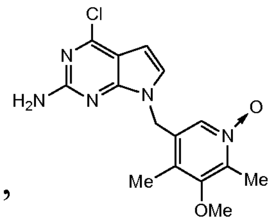,

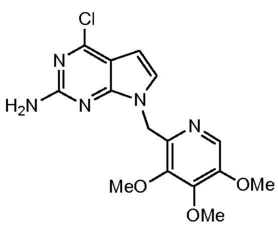,
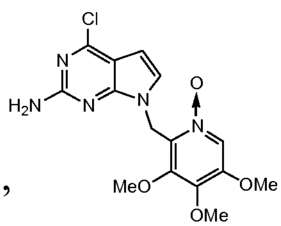,
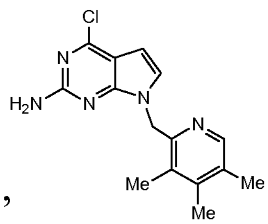,
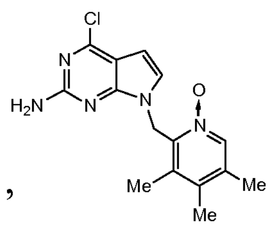,

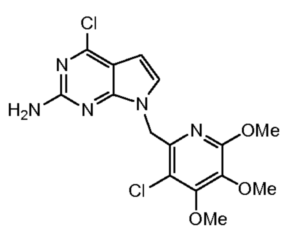,
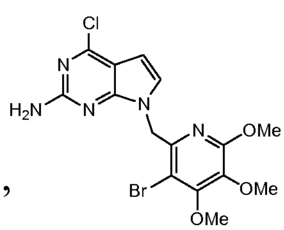,
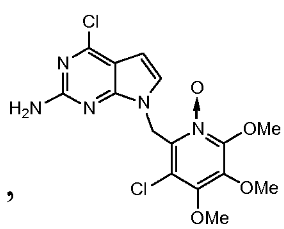
and
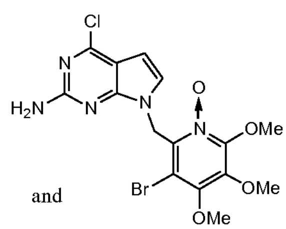

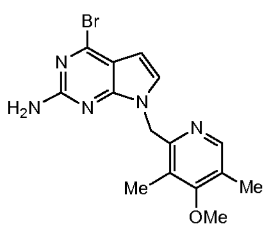,
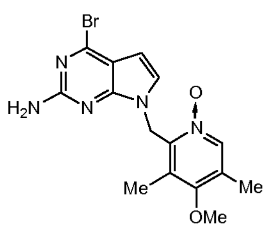,
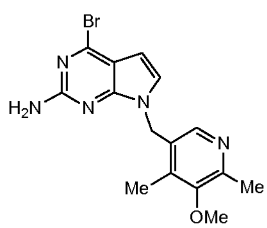,
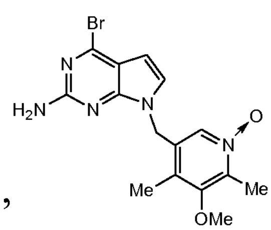,

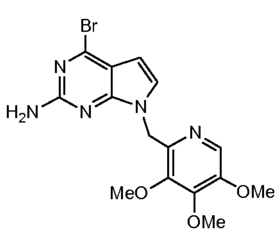,
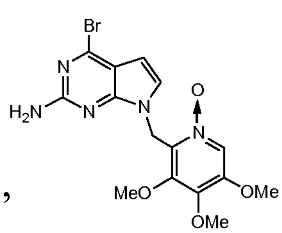,
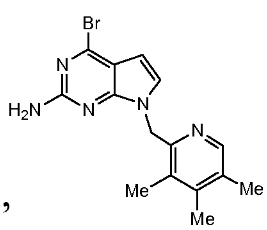,
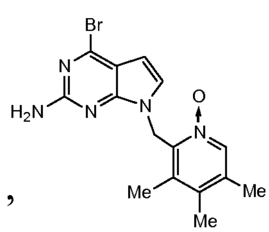,

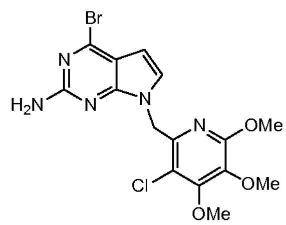, 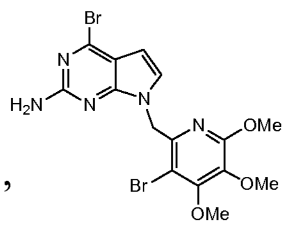, 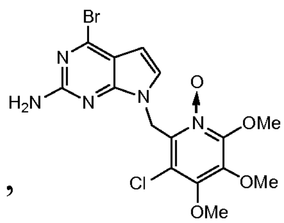 and 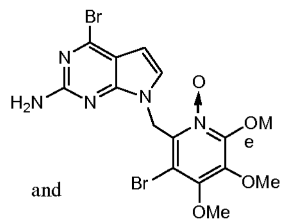

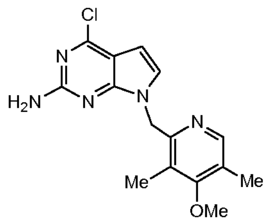, 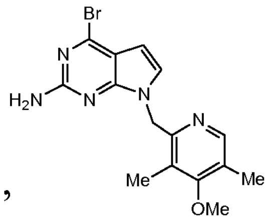, 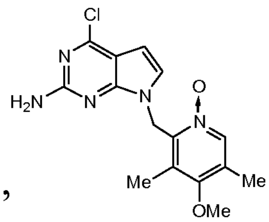, 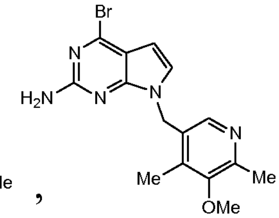,

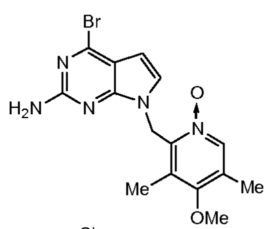, 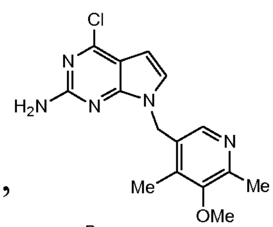, 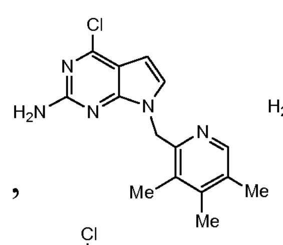, 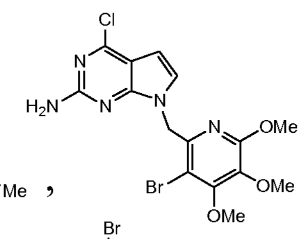,

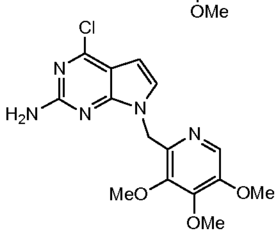, 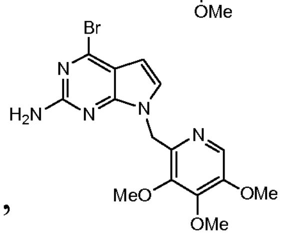, 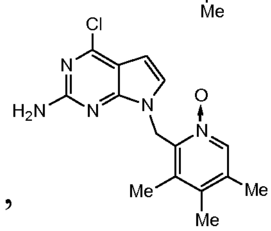, 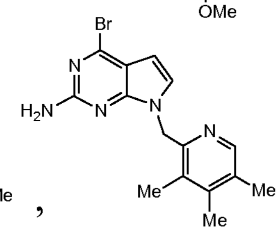,

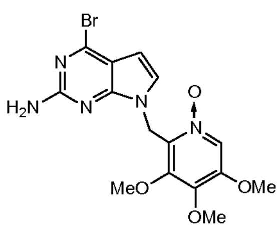, 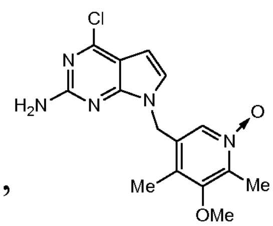, 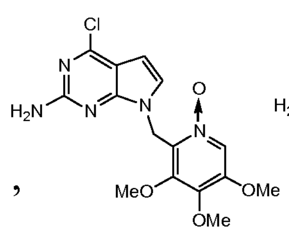 and 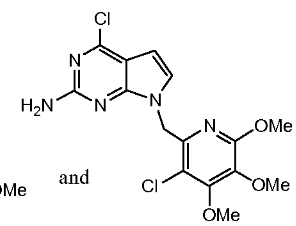

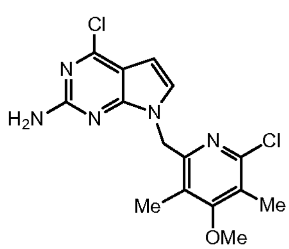, 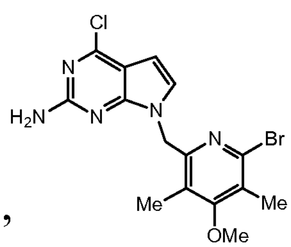, 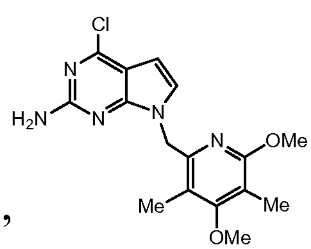,

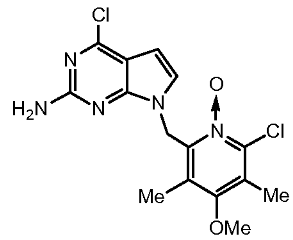 , 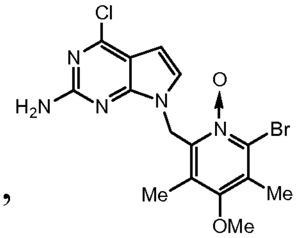 and 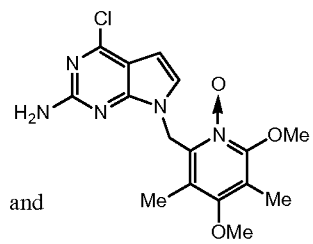

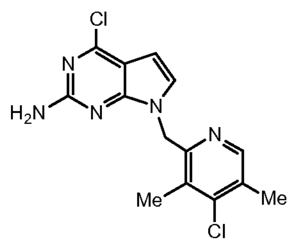 , 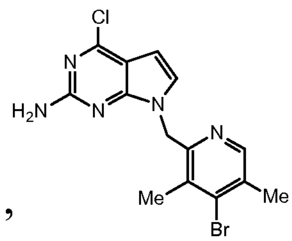 , 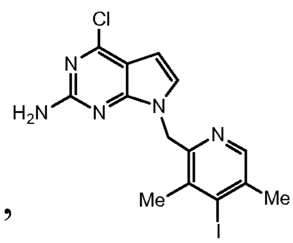 ,

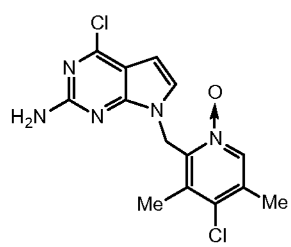 , 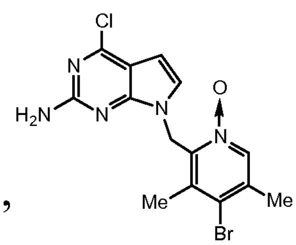 and 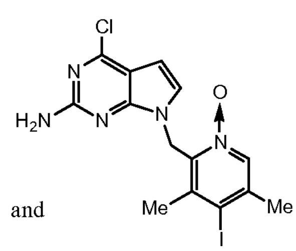

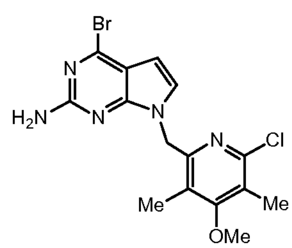 , 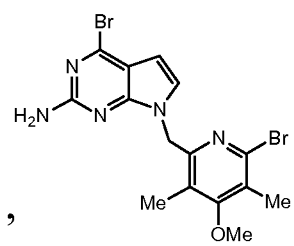 , 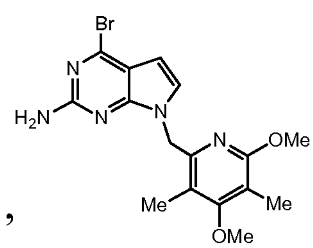 ,

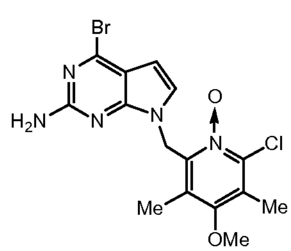 , 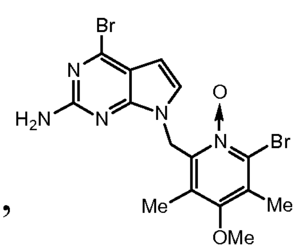 and 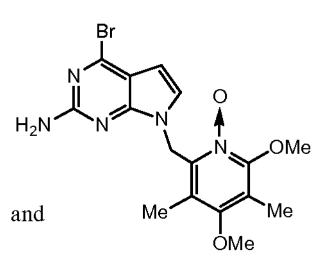

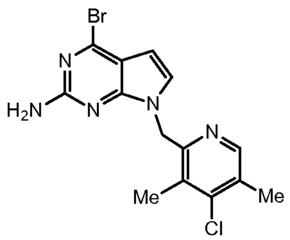 , 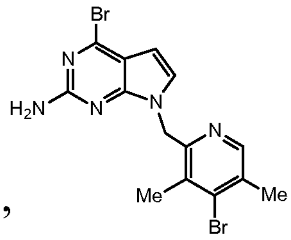 , 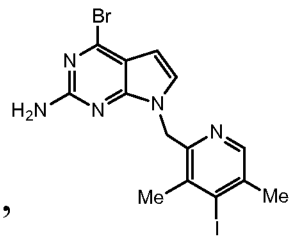 ,

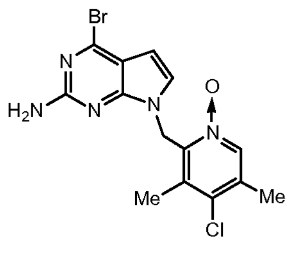 , 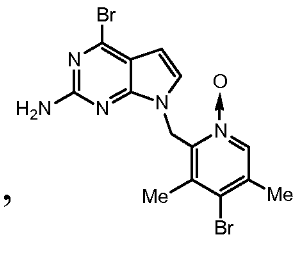 and 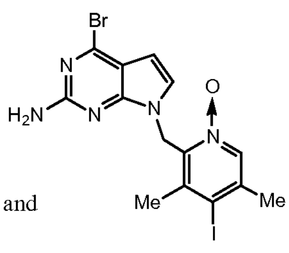

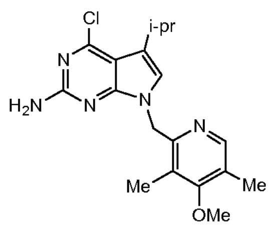 , 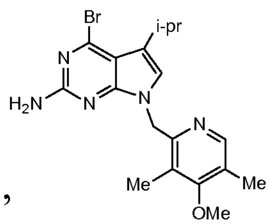 , 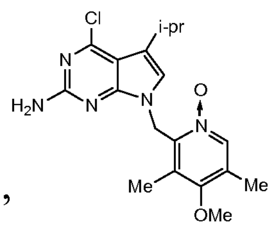 , 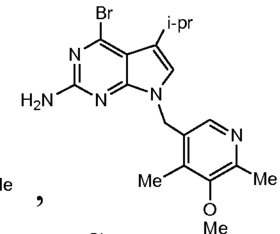 ,

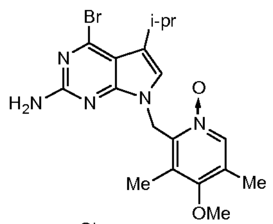 , 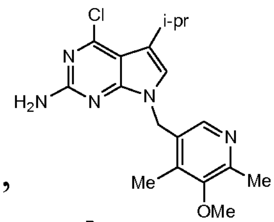 , 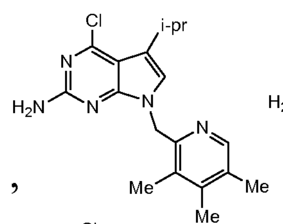 , 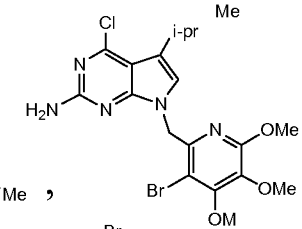 ,

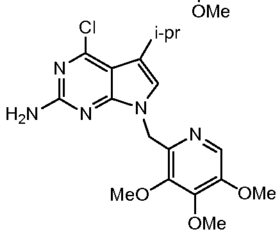 , 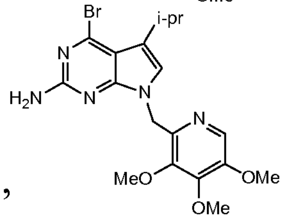 , 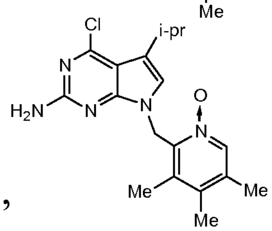 , 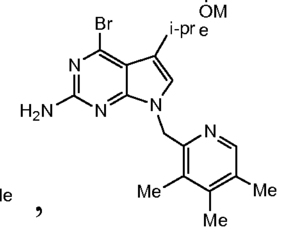 ,

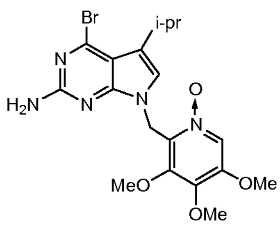 , 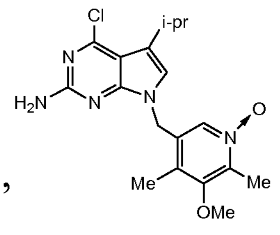 , 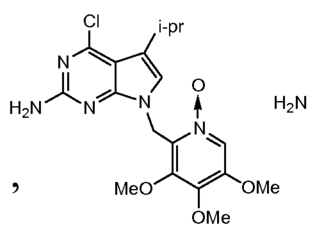 and 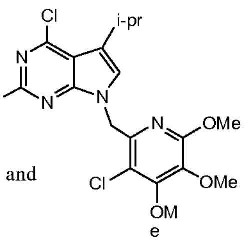

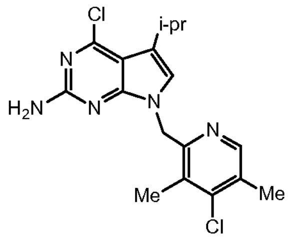
,
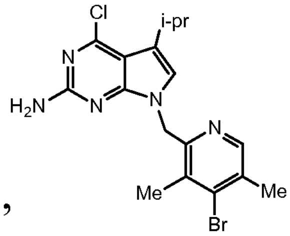
,
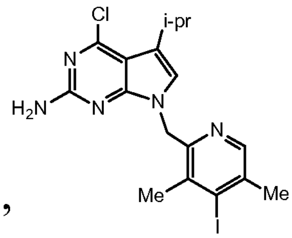
,

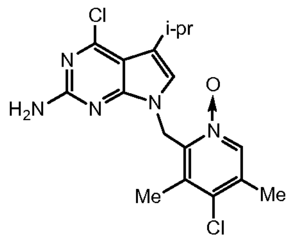
,
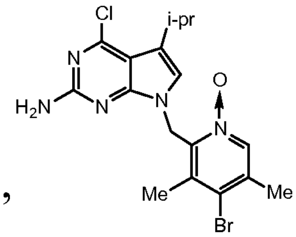
and
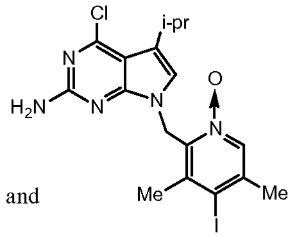

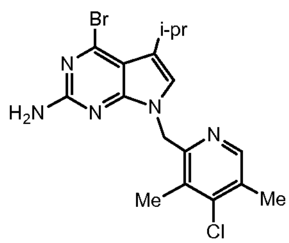
,
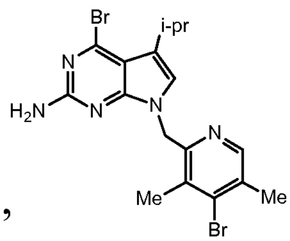
,
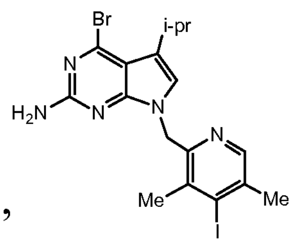
,

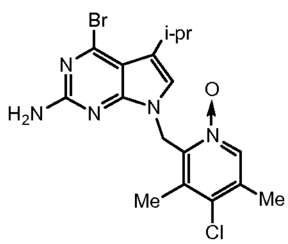
,
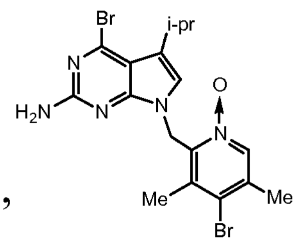
and
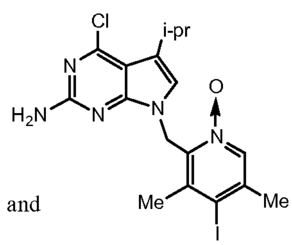

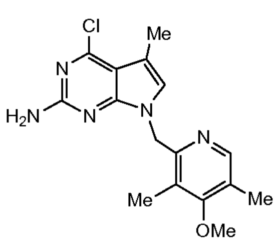
,
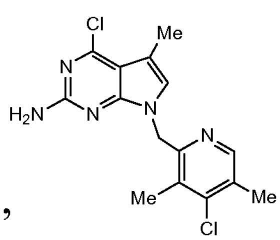
,
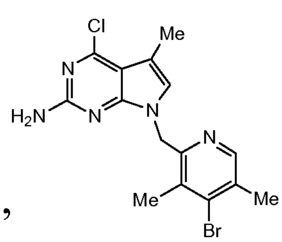
,
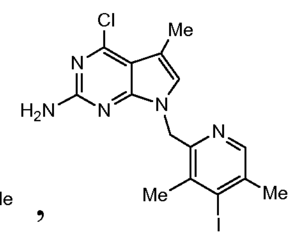
,

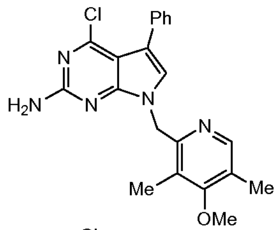 , 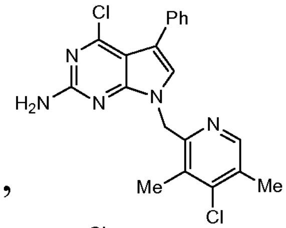 , 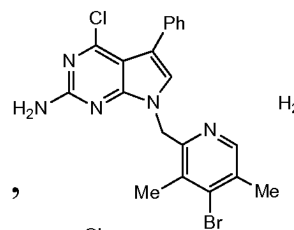 , 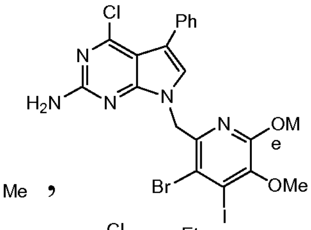 ,

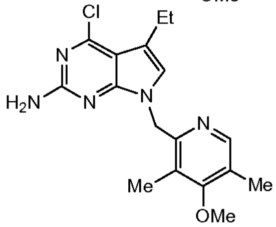 , 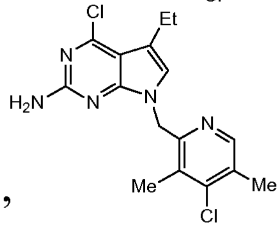 , 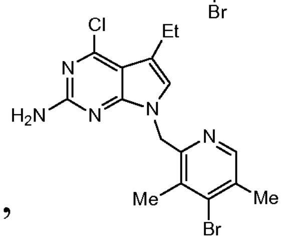 , 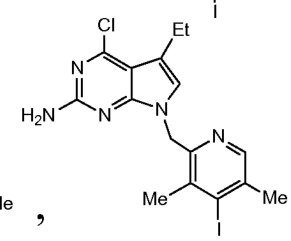 ,

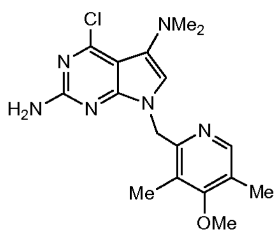 , 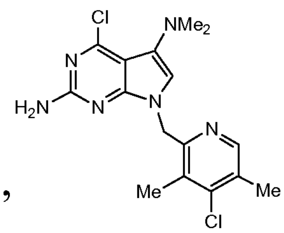 , 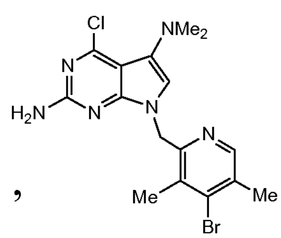 and 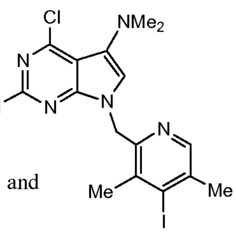

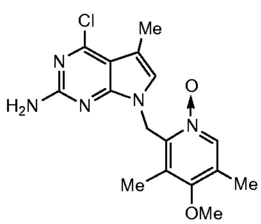 , 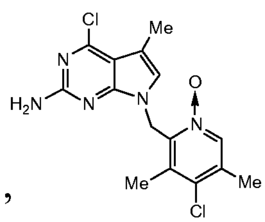 , 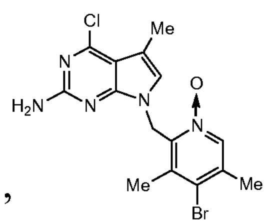 , 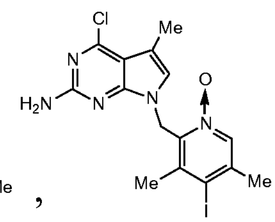 ,

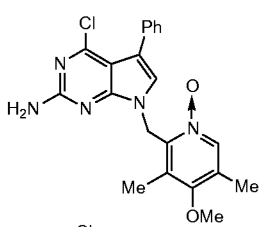 , 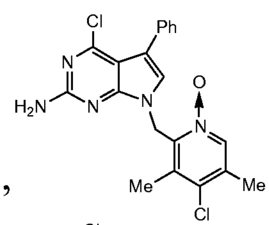 , 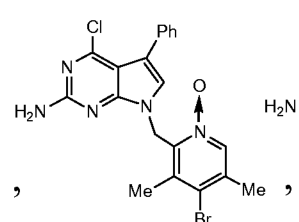 , 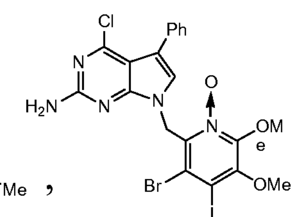 ,

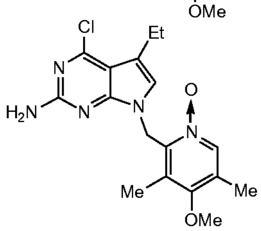 , 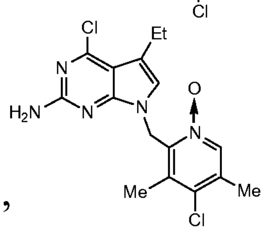 , 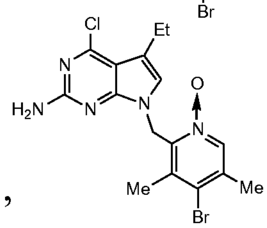 , 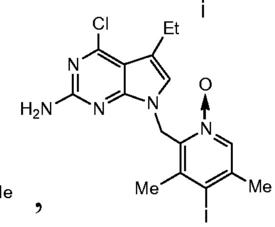 ,

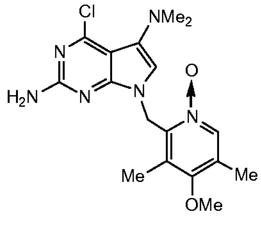 , 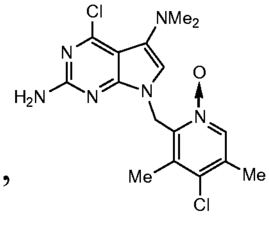 , 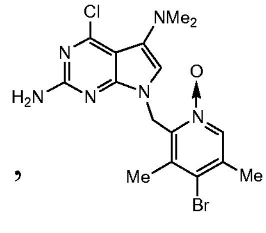 and 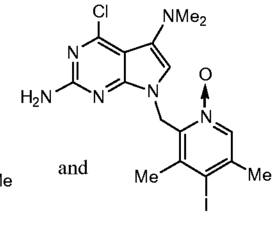

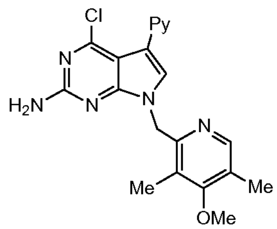 , 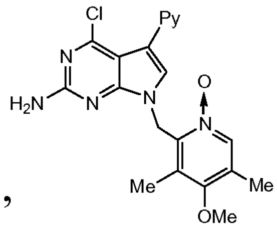 , 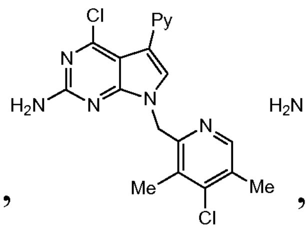 , 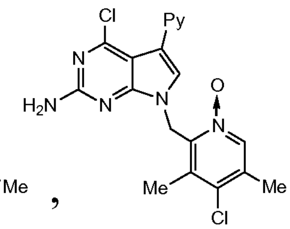 ,

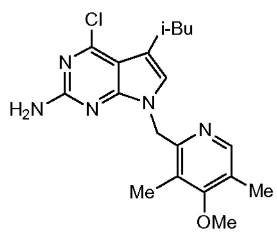 , 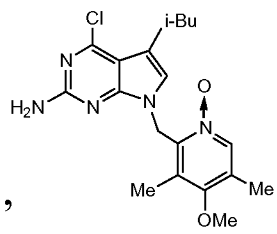 , 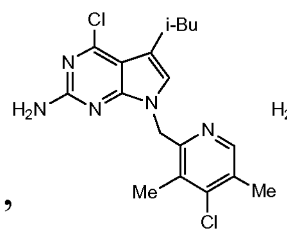 , 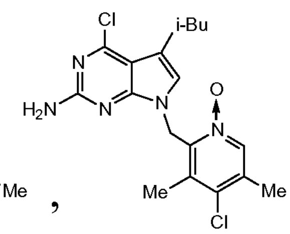 ,

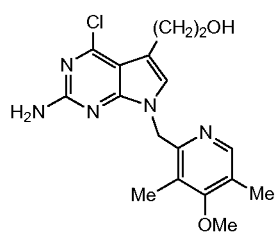 , 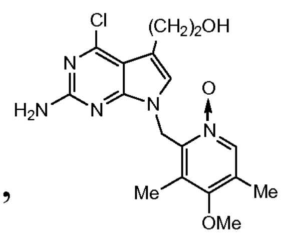 , 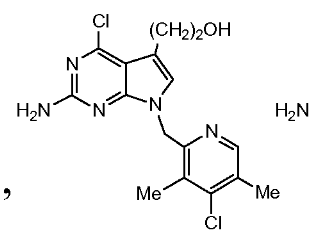 and 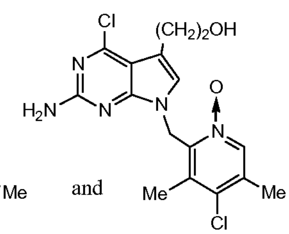

7. A compound selected from the group below, or a polymorph, solvate, ester, tautomer, enantiomers, pharmaceutically acceptable salt or prodrug thereof:

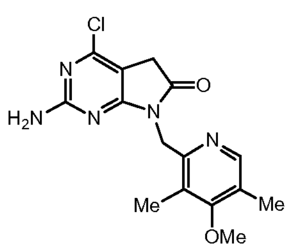 , 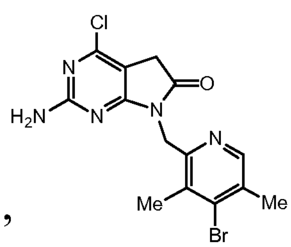 , 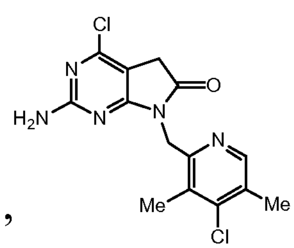 ,

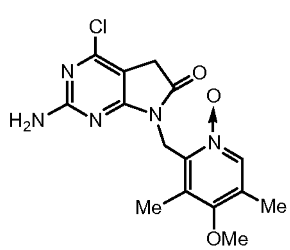 , 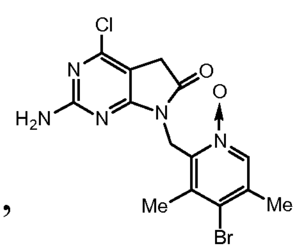 and 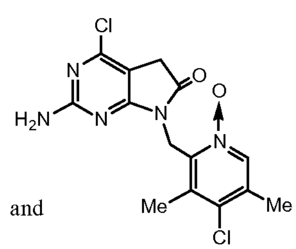

8. A compound represented by one of the formulae below, or a polymorph, solvate, ester, tautomer, pharmaceutically acceptable salt or prodrug thereof:

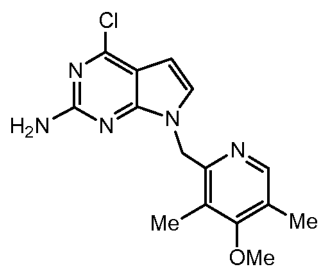

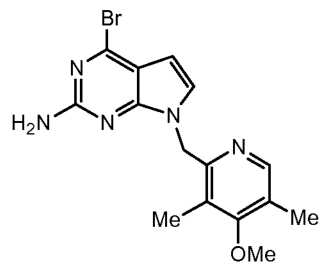

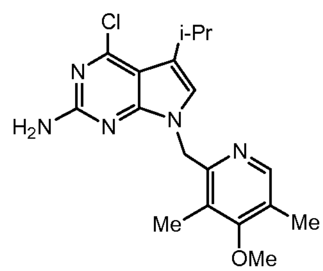

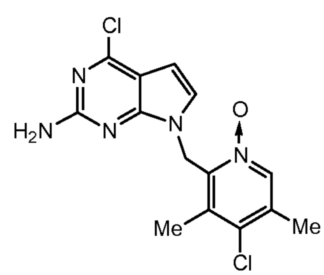

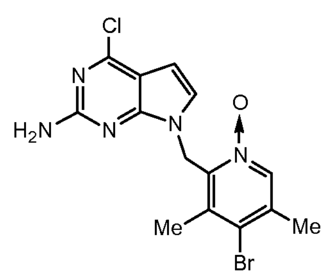

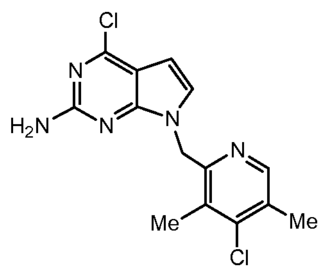

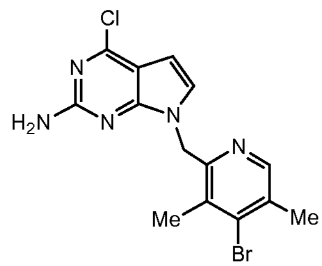

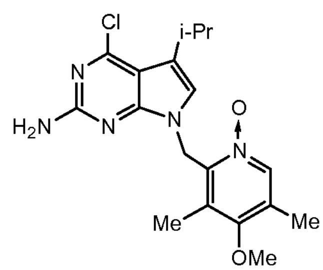

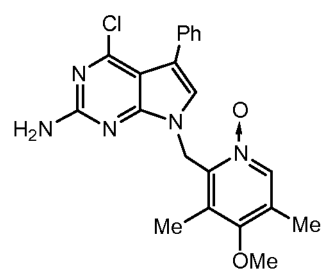

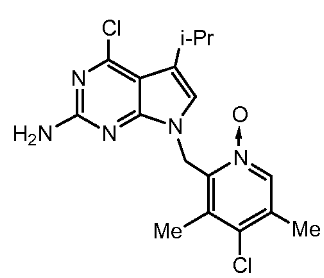

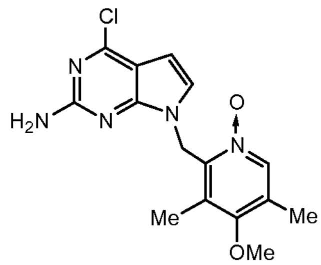

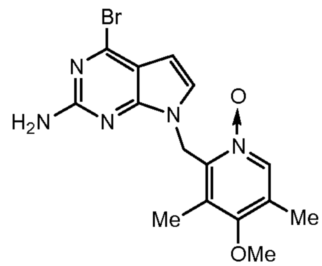

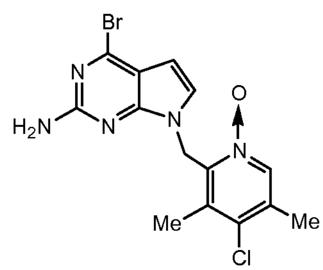

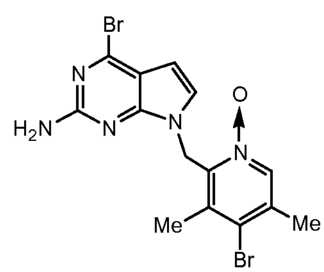

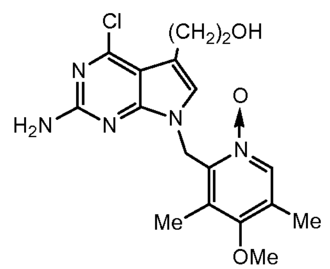

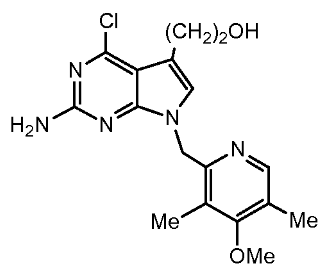

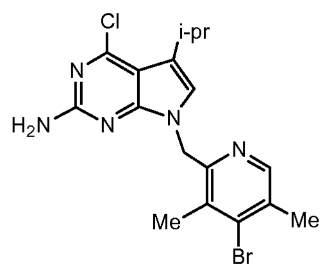

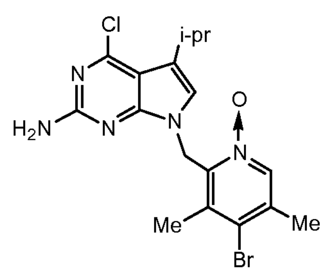

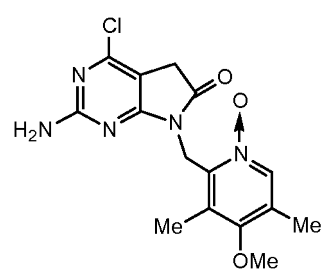

**9.** A compound represented by Formula IC, or a polymorph, solvate, ester, tautomer, enantiomer, pharmaceutically acceptable salt or prodrug thereof:

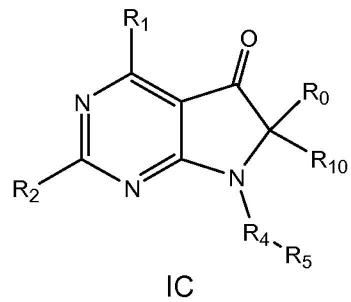

IC

wherein:

$R^0$ is hydrogen, halogen, lower alkyl, $-SR^8$, $-OR^8$, -CN or $-NHR^8$;
$R^1$ is halogen, $-OR^{11}$, $-SR^{11}$ or lower alkyl;
$R^2$ is $-NH_2$;
$R^4$ is $-CHR^{12}$-;
$R^5$ is aryl, heteroaryl, alicyclic, or heterocyclic, wherein:

the aryl group is substituted with 3 to 5 substituents,
the heteroaryl group is substituted with 2 to 5 substituents,
the alicyclic group is substituted with 3 to 5 substituents,
the heterocyclic group is substituted with 3 to 5 substituents, and
the substituents on $R^5$ are selected from the group consisting of halogen, lower alkyl, lower alkenyl, lower alkynyl, $-SR^8$, $-OR^8$, -CN, -C(O)OH, $-C(O)R^9$, $-NO_2$, $-NR^8R^{10}$, lower aryl, heteroaryl, alicyclic, lower heterocyclic, arylalkyl, heteroarylalkyl, amino, alkylamino, dialkylamino, diarylalkylamino, oxo, oxa, perhaloalkyl, perhaloalkoxy, perhaloacyl, guanidine, pyridinyl, thiophene, furanyl, indole, indazole, phosphonates, phosphates, phosphoramides, sulfonates, sulfones, sulfates, sulphonamides, carbamates, ureas, thioureas and thioamides, wherein when $R^3$ and $R^{10}$ taken together optionally form a ring of 3-7 ring atoms and optionally 1-3 of the ring atoms are heteroatoms selected from the group of O, S and N;

$R^8$ is hydrogen, lower alkyl, lower alkenyl, lower alkynyl, lower aryl, lower heteroaryl, or $-C(O)R^9$;
$R^9$ is H, lower alkyl, lower aryl, lower heteroaryl, $-NR^{10}R^{10}$, or $-OR^{11}$, wherein
$R^{10}$ and $R^{10}$ taken together optionally form a ring of 3-7 ring atoms and optionally 1-3 of the ring atoms are heteroatoms selected from the group of O, S and N; $R^{10}$ is hydrogen, lower alkyl, lower heteroaryl, lower aryl, lower alkenyl, or lower alkynyl;
$R^{11}$ is lower alkyl, lower alkenyl, lower alkynyl, lower heteroaryl or lower aryl;
$R^{12}$ is hydrogen or lower alkyl; and
$R^0$ and $R^{10}$ taken together optionally form an exocyclic double bond which is optionally substituted, or optionally form a ring of 3-7 ring atoms and optionally 1-3 of the ring atoms are heteroatoms selected from the group of O, S and N.

**10.** The compound of claim 9, or a polymorph, solvate, ester, tautomer, enantiomer, pharmaceutically acceptable salt or prodrug thereof, wherein:

$R^0$ is hydrogen;
$R^1$ is halogen;
$R^4$ is $-CH_2$-;
$R^5$ is aryl or heteroaryl, wherein each of said aryl and heteroaryl is monocyclic or bicyclic and is substituted with 3 to 5 substituents; and
$R^{10}$ is hydrogen.

**11.** The compound of claim 10, or a polymorph, solvate, ester, tautomer, enantiomer, pharmaceutically acceptable salt or prodrug thereof, wherein: $R^1$ is chloro or bromo; $R^5$ is phenyl, pyridyl or 1-oxy-pyridyl (N-oxy-pyridyl), each of which has at least two substituents.

**12.** A compound selected from the group below, or a polymorph, solvate, ester, tautomer, enantiomers, pharmaceutically

acceptable salt or prodrug thereof:

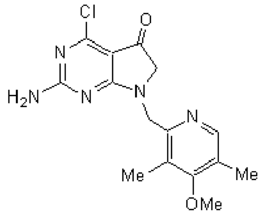 , 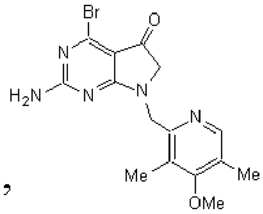 , 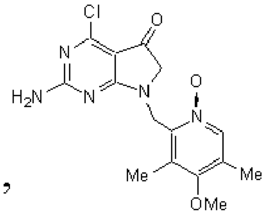 , 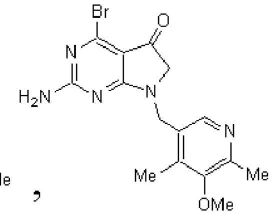 ,

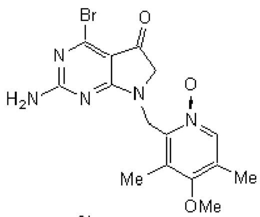 , 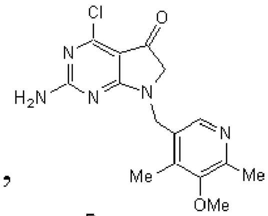 , 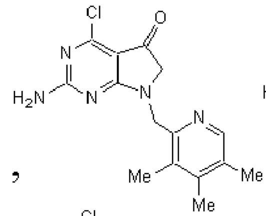 , 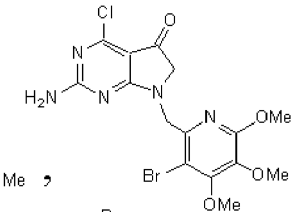 ,

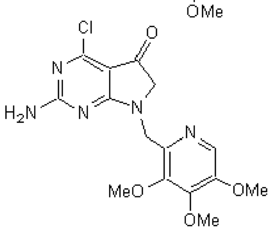 , 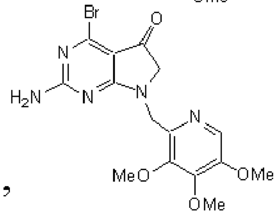 , 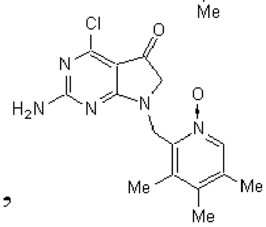 , 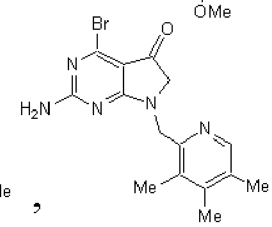 ,

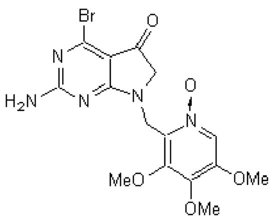 , 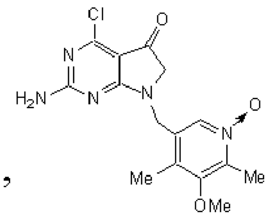 , 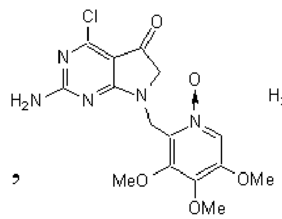 and 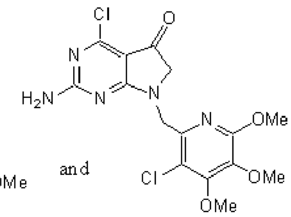

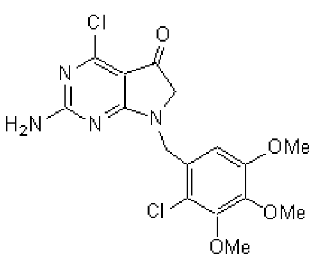 , 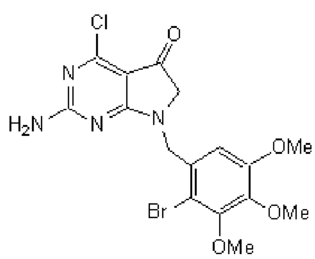 , 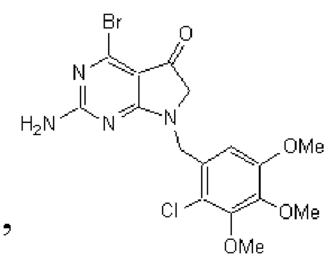 ,

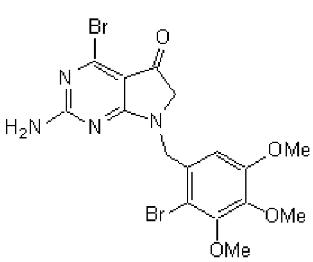 , 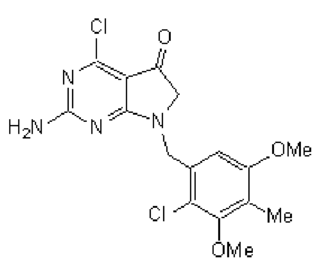 , 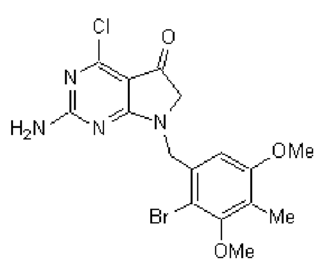 ,

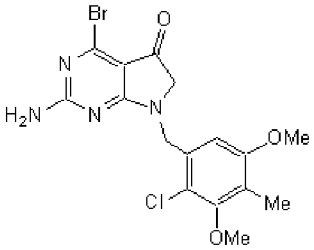,
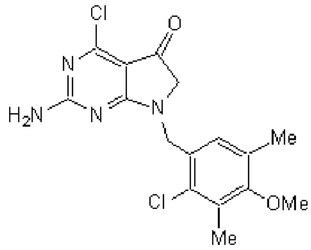
and
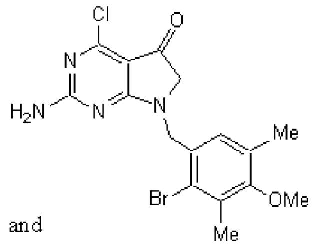

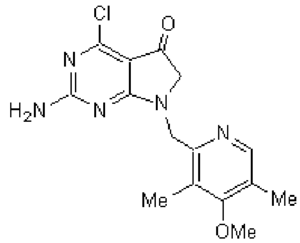
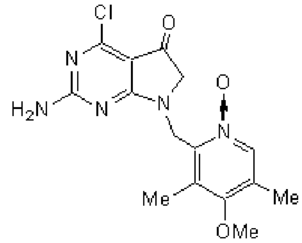

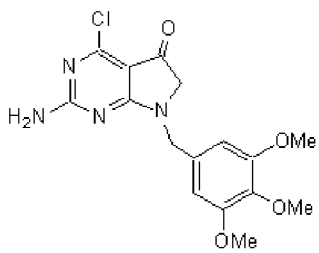
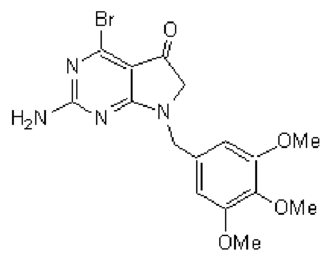

**13.** A compound represented by Formula ID, or a polymorph, solvate, ester, tautomer, enantiomer, pharmaceutically acceptable salt or prodrug thereof:

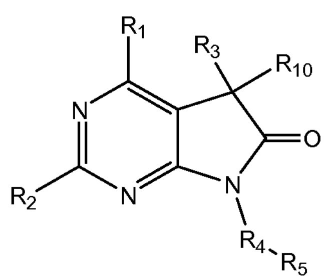

ID

wherein:

$R^1$ is halogen, $-OR^{11}$, $-SR^{11}$ or lower alkyl;
$R^2$ is $-NH_2$;
$R^3$ is selected from the group consisting of hydrogen, halogen, $-SR^8$, $-OR^8$, -CN, $-C(O)R^9$, -C(O)OH, $-NO_2$, $-NR^8R^{10}$, lower alkyl, lower alkenyl, lower alkynyl, lower perhaloalkyl, aryl, heteroaryl, alicyclic, heterocyclic, all optionally substituted, wherein:

the aryl, heteroaryl, alicyclic and heterocyclic groups are optionally mono-, bi- or tri-cyclic, $R^3$ and $R^{10}$ taken

together optionally form a ring of 3-7 ring atoms and optionally 1-3 of the ring atoms are heteroatoms selected from the group of O, S and N, and
the optional substituents on $R^3$ are selected from the group consisting of halogen, lower alkyl, lower alkenyl, lower alkynyl, $-SR^8$, $-OR^8$, -CN, -C(O)OH, $-C(O)R^9$, $-NO_2$, $-NR^8R^{10}$, lower aryl, heteroaryl, alicyclic, lower heterocyclic, arylalkyl, heteroarylalkyl, amino, alkylamino, dialkylamino, diarylalkylamino, oxo, oxa, perhaloalkyl, perhaloalkoxy, perhaloacyl, guanidine, pyridinyl, thiophene, furanyl, indole, indazole, phosphonates, phosphates, phosphoramides, sulfonates, sulfones, sulfates, sulphonamides, carbamates, ureas, thioureas and thioamides, wherein $R^8$ and $R^{10}$ taken together optionally form a ring of 3-7 ring atoms and optionally 1-3 of the ring atoms are heteroatoms selected from the group of O, S and N;

$R^4$ is $-CHR^{12}-$;
$R^5$ is aryl, heteroaryl, alicyclic, or heterocyclic, wherein
the aryl group is substituted with 3 to 5 substituents,
the heteroaryl group is substituted with 2 to 5 substituents,
the alicyclic group is substituted with 3 to 5 substituents,
the heterocyclic group is substituted with 3 to 5 substituents, and
the substituents on $R^5$ are selected from the group consisting of halogen, lower alkyl, lower alkenyl, lower alkynyl, $-SR^8$, $-OR^8$, -CN, -C(O)OH, $-C(O)R^9$, $-NO_2$, $-NR^8R^{10}$, lower aryl, heteroaryl, alicyclic, lower heterocyclic, arylalkyl, heteroarylalkyl, amino, alkylamino, dialkylamino, diarylalkylamino, oxo, oxa, perhaloalkyl, perhaloalkoxy, perhaloacyl, guanidine, pyridinyl, thiophene, furanyl, indole, indazole, phosphonates, phosphates, phosphoramides, sulfonates, sulfones, sulfates, sulphonamides, carbamates, ureas, thioureas and thioamides, wherein
$R^8$ and $R^{10}$ taken together optionally form a ring of 3-7 ring atoms and optionally 1-3 of the ring atoms are heteroatoms selected from the group of O, S and N;
$R^8$ is hydrogen, lower alkyl, lower alkenyl, lower alkynyl, lower aryl, lower heteroaryl, or $-C(O)R^9$;
$R^9$ is H, lower alkyl, lower aryl, lower heteroaryl, $-NR^{10}R^{10}$, or $-OR^{11}$, wherein when $R^{10}$ and $R^{10}$ taken together optionally form a ring of 3-7 ring atoms and optionally 1-3 of the ring atoms are heteroatoms selected from the group of O, S and N;
$R^{10}$ is hydrogen, lower alkyl, lower heteroaryl, lower aryl, lower alkenyl, or lower alkynyl,
$R^{11}$ is lower alkyl, lower alkenyl, lower alkynyl, lower heteroaryl or lower aryl;
$R^{12}$ is hydrogen or lower alkyl; and
$R^3$ and $R^{10}$ taken together optionally form an exocyclic double bond which is optionally substituted, or optionally form a ring of 3-7 ring atoms and optionally 1-3 of the ring atoms are heteroatoms selected from the group of O, S and N.

**14.** The compound of claim 13, or a polymorph, solvate, ester, tautomer, enantiomers, pharmaceutically acceptable salt or prodrug thereof, wherein:

$R^1$ is halogen;
$R^3$ is hydrogen, halogen, $-OR^8$, $-SR^3$, $-NR^8R^{10}$, lower alkyl, lower alkenyl, lower alkynyl, lower perhaloalkyl, lower aryl, or lower heteroaryl, wherein $R^8$ is hydrogen, lower alkyl, lower alkenyl, or lower alkynyl, lower aryl, lower heteroaryl, or -C (O)$R^9$;
$R^4$ is $-CH_2-$;
$R^5$ is aryl or heteroaryl, wherein each of said aryl and heteroaryl is monocyclic or bicyclic and is substituted with 3 to 5 substituents, and
$R^{10}$ is hydrogen or lower alkyl.

**15.** The compound of claim 14, or a polymorph, solvate, ester, tautomer, enantiomer, pharmaceutically acceptable salt or prodrug thereof, wherein: $R^1$ is chloro or bromo; and $R^5$ is phenyl, pyridyl or 1-oxy-pyridyl (N-oxy-pyridyl), each of which has at least two substituents.

**16.** A compound represented by Formula IE, or a polymorph, solvate, ester, tautomer, enantiomer, pharmaceutically acceptable salt or prodrug thereof:

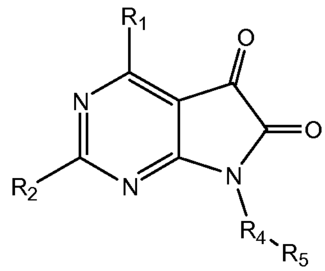

IE

wherein:

$R^1$ is halogen, $-OR^{11}$, $-SR^{11}$ or lower alkyl;
$R^2$ is $-NH_2$;
$R^4$ is $-CHR^{12}-$;
$R^5$ is aryl, heteroaryl, alicyclic, or heterocyclic, wherein
the aryl group is substituted with 3 to 5 substituents,
the heteroaryl group is substituted with 2 to 5 substituents,
the alicyclic group is substituted with 3 to 5 substituents,
the heterocyclic group is substituted with 3 to 5 substituents, and
the substituents on $R^5$ are selected from the group consisting of halogen,
lower alkyl, lower alkenyl, lower alkynyl, $-SR^8$, $-OR^8$, -CN, - C(O)OH, $-C(O)R^9$, $-NO_2$, $-NR^8R^{10}$, lower aryl, heteroaryl, alicyclic, lower heterocyclic, arylalkyl, heteroarylalkyl, amino, alkylamino, dialkylamino, diarylalkylamino, oxo, oxa, perhaloalkyl, perhaloalkoxy, perhaloacyl, guanidine, pyridinyl, thiophene, furanyl, indole, indazole, phosphonates, phosphates, phosphoramides, sulfonates, sulfones, sulfates, sulphonamides, carbamates, ureas, thioureas and thioamides, wherein $R^8$ and $R^{10}$ taken together optionally form a ring of 3-7 ring atoms and optionally 1-3 of the ring atoms are heteroatoms selected from the group of O, S and N;
$R^8$ is hydrogen, lower alkyl, lower alkenyl, lower alkynyl, lower aryl, lower heteroaryl, or $-C(O)R^9$;
$R^9$ is H, lower alkyl, lower aryl, lower heteroaryl, $-NR^{10}R^{10}$, or $-OR^{11}$, wherein $R^{10}$ and $R^{10}$ taken together optionally form a ring of 3-7 ring atoms and optionally 1-3 of the ring atoms are heteroatoms selected from the group of O, S and N;
$R^{10}$ is hydrogen, lower alkyl, lower heteroaryl, lower aryl, lower alkenyl, or lower alkynyl,
$R^{11}$ is lower alkyl, lower alkenyl, lower alkynyl, lower heteroaryl or lower aryl; and
$R^{12}$ is hydrogen or lower alkyl.

**17.** A pharmaceutical composition comprising one or more pharmaceutically acceptable excipients and at least one compound according to any of claims 1-16.

**18.** An *in vitro* method of inhibiting an HSP90, comprising:

contacting a cell having an HSP90 with a compound according to any of claims 1-16.

**19.** A compound according to any of claims 1-16 for use in therapy or diagnosis.

**20.** A compound according to any of claims 1-16 for use in treating an HSP90 mediated disorder.

**21.** Use of a compound according to any of claims 1-16 for the preparation of a pharmaceutical composition for treating an HSP90 mediated disorder.

**22.** The use of claims 20 or 21, wherein the HSP90 mediated disorder is selected from the group of inflammatory diseases, infections, autoimmune disorders, stroke, ischemia, cardiac disorder, neurological disorders, fibrogenetic disorders selected from the group of scleroderma, polymyositis, systemic lupus, rheumatoid arthritis, liver cirrhosis, keloid formation, interstitial nephritis and pulmonary fibrosis, proliferative disorders, tumors, leukemias, neoplasms, cancers, carcinomas, metabolic diseases, and malignant disease.

**23.** The use of any one of claims 18-22, further comprising administering at least one therapeutic agent selected from the group of cytotoxic agents, anti-angiogenesis agents and anti-neoplastic agents wherein the at least one anti-neoplastic agent is selected from the group of alkylating agents, anti-metabolites, epidophyllotoxins; antineoplastic enzymes, topoisomerase inhibitors, procarbazines, mitoxantrones, platinum coordination complexes, biological response modifiers and growth inhibitors, hormonal/anti-hormonal therapeutic agents, and haematopoietic growth factors.

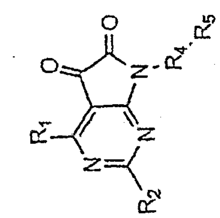
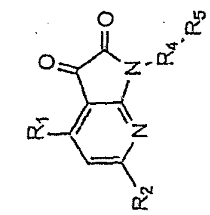
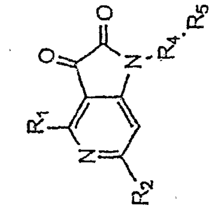
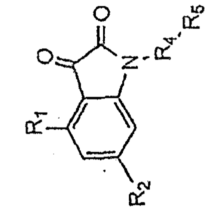
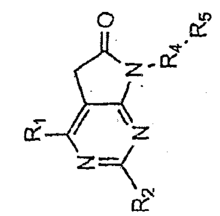
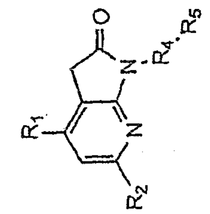
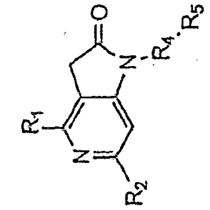
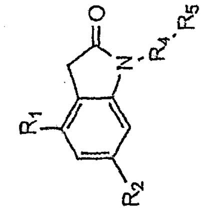
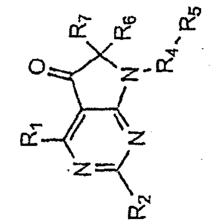
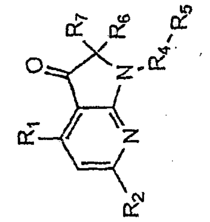
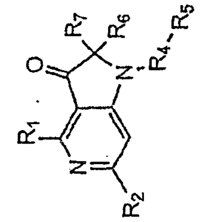
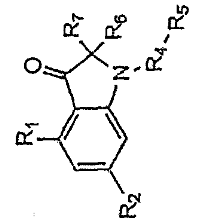
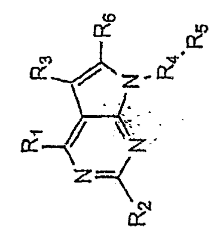
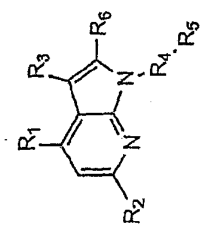
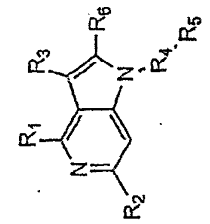
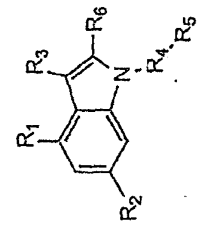

Fig 1 (Contin)

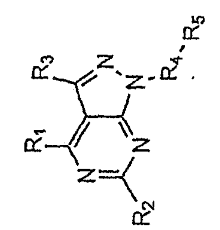
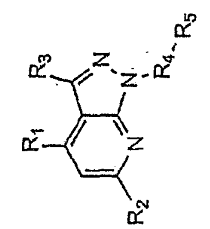
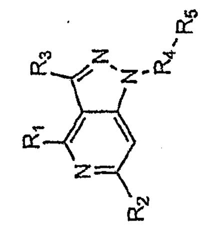
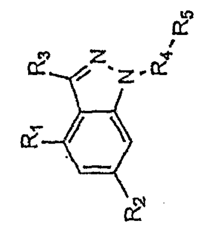
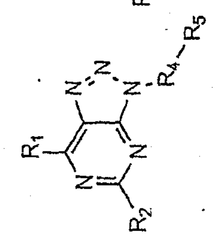
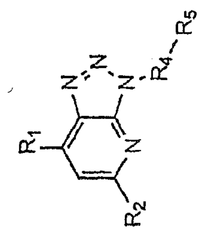
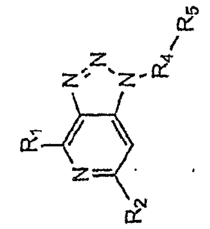
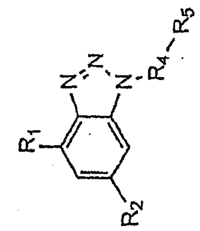
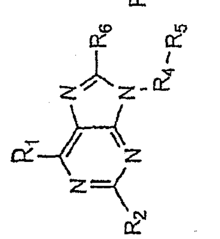
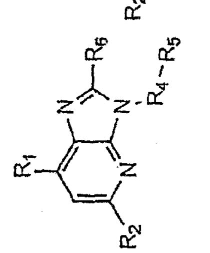
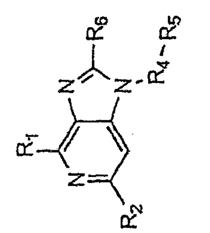
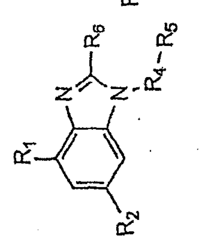

Europäisches Patentamt
European Patent Office
Office européen des brevets

# EUROPEAN SEARCH REPORT

Application Number
EP 09 15 7018

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HOOPS ET AL.: "The Synthesis and Determination of Acidic Ionization Constants of Certain 5-Substituted 2-Aminopyttolo[2,3-d]pyrimidin-4-ones and Methylated Analogs" JOURNAL OF HETEROCYCLIC CHEMISTRY, vol. 33, 1996, pages 767-781, XP002546738 * compounds 27,28 * ----- | 1,3-5,17 | INV. C07D487/04 A61K31/519 |
| D,A | WO 02/36075 A (SLOAN KETTERING INST CANCER [US]; CHIOSIS GABRIELA [US]; ROSEN NEAL [U) 10 May 2002 (2002-05-10) * the whole document * ----- | 1-23 | |
| A | US 5 002 950 A (MALONE THOMAS C [US] ET AL) 26 March 1991 (1991-03-26) * the whole document * ----- | 1-23 | |

TECHNICAL FIELDS SEARCHED (IPC)

C07D
A61K

The present search report has been drawn up for all claims

| Place of search | Date of completion of the search | Examiner |
|---|---|---|
| The Hague | 1 October 2009 | Fritz, Martin |

3

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..........................................................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 09 15 7018

This annex lists the patent family members relating to the patent documents cited in the above-mentioned European search report.
The members are as contained in the European Patent Office EDP file on
The European Patent Office is in no way liable for these particulars which are merely given for the purpose of information.

01-10-2009

| Patent document cited in search report | Publication date | Patent family member(s) | Publication date |
|---|---|---|---|
| WO 0236075 A | 10-05-2002 | AU 2877102 A | 15-05-2002 |
| | | CA 2426952 A1 | 10-05-2002 |
| | | CN 1501928 A | 02-06-2004 |
| | | EP 1335920 A2 | 20-08-2003 |
| | | JP 2004514660 T | 20-05-2004 |
| | | JP 2005519848 T | 07-07-2005 |
| US 5002950 A | 26-03-1991 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0209696 A, Rosen
- US 0123640 W
- WO 9951223 A, Degranco
- US 9907242 W
- US 6210974 B1, Gold
- US 6174875 B, DeFranco
- WO 0202123 A, Strehlow
- US 0120578 W
- US 0304283 W
- US 0235938 W
- US 0216287 W
- US 0206518 W
- US 9809805 W
- US 0009512 W
- US 0109512 W
- US 01L23640 W
- US 0146303 W
- US 0146304 W
- US 0229715 W
- US 0235069 W
- US 0239993 W
- US 60293246 W
- US 60371668 W
- US 60335391 W
- US 60128593 W
- US 60337919 W
- US 60340762 W
- US 60359484 W
- US 60331893 W
- US 0236075 W
- JP 10025294 B
- US 4748177 A
- US 4772606 A
- US 6369092 B
- WO 0006573 A
- WO 02055521 A
- WO 02055082 A
- WO 02055083 A
- EP 0178178 A
- US 1988 A
- US 5110818 A, Hans
- US 1992 A
- JP 10025294 A, Matsuda
- JP 1998 A
- US 20030078413 A
- US 5939420 A, A. Gangjee
- WO 03037860 A, Kasibhatla
- EP 0184322 A, Smith, Kline and French
- EP 1986 A
- US 6610688 B, C. Liang
- WO 9839344 A, Dang
- US 5204353 A, Meier
- US 5917042 A, Daluge
- WO 02055082 PCT
- US 20030078413 A1
- WO 03022859 A, R. O. Dempcy
- GB 884151 A
- US 4714701 A, Beauchamp
- US 1987 A
- US 1993 A
- US 4699877 A
- US 4918162 A
- US 4968603 A
- US 5846749 A
- WO 9910326 A, Tarbit
- WO 03022859 PCT


### Non-patent literature cited in the description


- **Buchner.** *J. TIBS,* 1999, vol. 24, 136-141
- **Stepanova, L. et al.** *Genes Dev.,* 1996, vol. 10, 1491-502
- **Dai, K. et al.** *J. Biol. Chem.,* 1996, vol. 271, 22030-4
- **Caplan, A.** *Trends in Cell Biol.,* 1999, vol. 9, 262-68
- **Stebbins, C. et al.** *Cell,* 1997, vol. 89, 239-250
- **Grenert, J.P et al.** *J. Biol. Chem.,* 1997, vol. 272, 23843-50
- **Proromou, C. et al.** *Cell,* 1997, vol. 90, 65-75
- **Panaretou, B. et al.** *EMBO J.,* 1998, vol. 17, 4829-36
- **Scheibel, T.H. et al.** *Proc. Natl. Acad. Sci. USA,* 1999, vol. 96, 1297-302
- **Schulte, T. W. et al.** *J. Biol. Chem.,* 1995, vol. 270, 24585-8

- **Whitesell, L. et al.** *Proc. Natl. Acad. Sci. USA,* 1994, vol. 91, 8324-8328
- **Schneider, C.L. et al.** *Proc. Natl. Acad. Sci., USA,* 1996, vol. 93, 14536-41
- **Sepp-Lorenzino et al.** *J Biol. Chem.,* 1995, vol. 270, 16580-16587
- **Sepp-Lorenzino, L. et al.** *J. Biol. Chem.,* 1995, vol. 270, 16580-16587
- **Schulte, T. W. et al.** *Biochem. Biophys. Res. Commun.,* 1997, vol. 239, 655-9
- **Segnitz, B. ; U. Gehring.** *J. Biol. Chem.,* 1997, vol. 272, 18694-18701
- **Smith, D. F. et al.** *Mol. Cell. Biol.,* 1995, vol. 15, 6804-12
- **Sepp-Lorenzino, L. et al.** *J. Biol.Chem.,* 1995, vol. 270, 16580-16587
- **Hartmann, F. et al.** *Int. J. Cancer,* 1997, vol. 70, 221-9
- **Miller, P. et al.** *Cancer Res.,* 1994, vol. 54, 2724-2730
- **Mimnaugh, E. G. et al.** *J. Biol. Chem.,* 1996, vol. 271, 22796-801
- **Schnur, R. et al.** *J. Med. Chem.,* 1995, vol. 38, 3806-3812
- **Erlichman et al.** *Proc. AACR,* 2001, vol. 42, 4474
- **Muise-Heimericks, R. C. et al.** *J. Biol. Chem.,* 1998, vol. 273, 29864-72
- **Vasilevskaya, A. et al.** *Cancer Res.,* 1999, vol. 59, 3935-40
- *Eur. J Med. Chem,* 1994, vol. 29 (1), 3-9
- *J. Het. Chem.,* 1990, vol. 27 (5), 1409
- **Berge et al.** Pharmaceutical Salts. *J. Pharm. Sci.,* 1977, vol. 66, 1-19
- Design of Prodrugs. Elseview, 1985
- Method in Enzymology. Academic, 1985, vol. 42, 309-396
- Design and Application of Prodrugs. **Bundgaard, H.** A Textbook of Drug Design and Development. 1991, 113-191
- **Bundgaard, H.** *Advanced Drug Delivery Review,* 1992, vol. 8, 1-38
- *Eur. J. Med. Chem.,* 1994, vol. 29 (1), 3-9
- **Montgomery.** *J Med. Pharm. Chem.,* 1962, vol. 5, 15-24
- **J. Davoll.** *J. Chem. Soc.,* 1960, 131
- **J. A. Montgomery.** *J. Chem. Soc.,* 1967, 665
- **G. Cristalli.** *J. Med. Chem.,* 1988, vol. 31, 390
- **T. Miwa.** *J. Org. Chem.,* 1993, vol. 58, 1696
- **D.M. Williams.** *J. Chem. Soc., Perkin Trans,* 1997, vol. 1, 1171
- **A.B. Reitz.** *J. Med. Chem.,* 1994, vol. 37, 3561
- **G.W. Craig.** *J. Prakt. Chem.,* 2000, vol. 342, 504
- **M. Semonsky.** *Coll. Czech. Chem. Commun.,* 1980, vol. 45, 3583
- **S. E. Watson.** *Synth. Commun.,* 1998, vol. 28, 3885
- **E. D. Edstrom.** *J. Org. Chem.,* 1993, vol. 58, 403
- **C. J. Barnett.** *Org. Proc. Res. Devop.,* 1999, vol. 3, 184
- **A. Gangjee.** *J. Med. Chem.,* 2001, vol. 44, 1993
- **A. Gangjee.** *J. Med. Chem.,* 2003, vol. 46, 591
- **E. C. Taylor.** *Heterocycles,* 1996, vol. 43, 323
- **R. J. Bontems.** *J. Med. Chem,* 1990, vol. 33, 2174
- **E. C. Taylor.** *J. Am. Chem. Soc.,* 1965, vol. 87, 1995
- **H. Kosaku.** *Heterocycles,* 2001, vol. 55, 2279
- **E. C. Taylor.** *J. Am. Chem. Soc,* 1965, vol. 87, 1995
- **K. A. M. El-Bayould.** *J. Chem. Res. Miniprint,* 1995, 1901
- **K. A. M. El-Bayouki.** *J. Chem. Res. Miniprint,* 1995, 1901
- **S. M. Bennett.** *J. Med. Chem.,* 1990, vol. 33, 2162
- **T. Miwa.** *J. Med. Chem.,* 1991, vol. 34, 555
- **Jerry March.** Advanced Organic Chemistry
- **Larock.** Comprehensive Organic Transformations. VCH, 1989
- **Morisawa.** *J. Med. Chem.,* 1974, vol. 17, 1083
- **Klaus, W.** *J. Med. Chem.,* 1992, vol. 35, 438
- Pyridine-1-oxide in Pyridine and its Derivatives. **Abramovitch, R.A. ; Smith, E. M.** The Chemistry of Heterocylic Compounds. John Wiley, 1974, vol. 2, 1-261
- **Jeromin, G. E.** *Chem. Ber.,* 1987, vol. 120, 649
- **Blanz, E. J.** *J. Med. Chem.,* 1970, vol. 13, 1124
- **Abblard, J.** *Bull. Soc. Chim. Fr.,* 1972, 2466
- **Fisher, B. E.** *The Structure of Isomaltol. J. Org. Chem.,* 1964, vol. 29, 776
- **De Cat, A.** *Bull. Soc. Chim. Belg.,* 1965, vol. 74, 270
- **Looker, J. H.** *J. Org. Chem.,* 1979, vol. 44, 3407
- **Ackerman, J. F.** Ph.D. Dissertation. June 1949
- **L. Sun.** *Bioorg. Med. Chem Lett.,* 2002, vol. 12, 2153
- **F. Seela.** *Synthesis,* 1997, 1067
- **E. Bisagni.** *Tetrahedron,* 1983, vol. 39, 1777
- **T, Sakamoto.** *Tetrahedron Lett.,* 1994, vol. 35, 2919

- **T. Sakamoto.** *J. Chem. Soc., Perkin Trans,* 1996, vol. 1, 459
- **M. J. Robins.** *Can. J. Chem.,* 1973, vol. 12, 3161
- **L. De Napoli J.** *Chem. Soc. Perkin Trans,* 1994, vol. 1, 923
- **E. A. Veliz.** *Tetrahedron Lett,* 2000, vol. 41, 1695
- **X. Lin.** *Org. Letters,* 2000, vol. 2, 3497
- **A. Holy.** *J. Med. Chem.,* 1999, vol. 42, 2064
- **B. G. Ugarkar.** *J. Med. Chem.,* 2000, vol. 43, 2883-28932894-2905
- **F. Seela.** *Liebigs. Ann. Chem.,* 1985, 315
- **J. F. Gerster.** *J. Chem. Soc.,* 1969, 207
- **E. C. Taylor et al.** *Tetrahedron,* 1992, vol. 48, 8089
- **J. W. Pawlik.** *J. Heterocycl. Chem.,* 1992, vol. 29, 1357
- **T. Y. I Wu.** *Org. Lett.,* 2003, vol. 5, 3587
- **B. M. Lynch.** *Can. J. Chem.,* 1988, vol. 66, 420
- **A.S. Bourlot ; E. Desarbre ; J.Y. Mérour.** *Synthesis,* 1994, 411
- **D. C. Miller.** *J. Med. Chem.,* 2002, vol. 45, 90
- **J. S. Pudlo.** *J. Med. Chem.,* 1990, vol. 33, 1984
- **G.W. Gribble.** *Org. Prep. Proced. Int.,* 2001, vol. 33 (6), 615
- **Romeo.** *Helv. Chim. Acta.,* 1955, vol. 38, 463, 465
- *Oxygen oxidation: A. Inada Heterocycles,* 1982, vol. 19, 2139
- **A.P. Phillips.** *J. Am. Chem. Soc.,* 1952, vol. 74, 3922
- **E. C. Taylor.** *Tetrahedron,* 1992, vol. 48, 8089
- **G. Zweifel.** *J. Am. Chem. Soc.,* 1976, vol. 98, 3184
- **R.C. Larock.** Comprehensive Organic Transformations. 1870
- **Holy.** *J. Med. Chem.,* 1999, vol. 42, 2064
- **Herdewijn.** *J. Med. Chem.,* vol. 38, 3838
- **Burger.** *J. Org. Chem.,* 2000, vol. 65, 7825
- **Halbfinger.** *J. Med. Chem.,* 1999, vol. 42, 1625
- **Jacobson.** *J. Med. Chem.,* 1999, vol. 42, 5325
- *J. Chem. Soc.,* 1962, 4186
- *J. Chem. Soc.,* 1963, 4186
- **Alhede.** *J. Org. Chem.,* 1991, 2139
- **Chowdhury.** *J. Med. Chem.,* 1999, vol. 42, 4300
- **F. Seela.** *Heterocycles,* 1985, vol. 23, 2521
- **F. Seela.** *Helv. Chim. Acta,* 1986, vol. 69, 1602
- **A. M. El-Reedy.** *Phosph, Sulf, Silic,* 1989, vol. 42, 231
- **R J. Bontems.** *J. Med. Chem,* 1990, vol. 33, 2174
- **F. Babin.** *J. Heterocycl. Chem.,* 1983, vol. 20, 1169
- **L. De Napoli.** *J. Chem. Soc. Perkin Trans,* 1994, vol. 1, 923
- **F. Seela et al.** *Helv. Chim. Acta,* 1999, vol. 82, 105
- **J. D. Anderson.** *J. Heterocycl. Chem.,* 1990, vol. 27, 439
- **Parkanyi.** *J. Heterocyc. Chem.,* 1990, vol. 27 (5), 1409-13
- **Peterson.** *J. Med. Chem.,* 1990, vol. 33 (4), 1214-19
- **Kozai.** *Chem. Pharm. Bull.,* 1999, vol. 47 (4), 574-575
- **F. Seela.** *Liebigs. Ann.Chem.,* 1985, 315
- **Goodman ; Gilman.** The Pharmacological Basis of Therapeutics. Pergamon
- **Remington's.** Pharmaceutical Sciences. Mack Publishing Co,
- **Langer.** *Science,* 1990, vol. 249, 1527-1533
- Therapy of Infectious Disease and Cancer. **Treat et al.** Liposomes. Liss, 1989, 353-365
- **Sefton.** *CRC Crit. Ref. Biomed. Eng.,* 1987, vol. 14, 201
- **Buchwald et al.** *Surgery,* 1980, vol. 88, 507
- **Saudek et al.** *N Engl. J. Med.,* 1989, vol. 321, 574
- **Goodson.** *Medical Applications of Controlled Release,* 1984, vol. 2, 115-138
- **Chiosis et al.** *Chemistry & Biology,* 2001, vol. 8, 289-299
- **Sambrook ; Fritsch ; Maniatis.** Molecular Cloning: A Laboratory Manual. Cold Spring Harbor Laboratory, 1989
- **Ausubel et al.** Current Protocols in Molecular Biology. John Wiley & Sons, 1994
- **Press, M. et al.** *Modern Pathology,* 2000, vol. 13, 225A
- **Harlow et al.** Antibodies: A Laboratory Manual. Cold Spring Harbor Laboratory, 1988
- **Kurokawa, H. et al.** *Cancer Res.,* 2000, vol. 60, 5887-5894
- **Mitchell MS ; Press M.F.** *Oncol.,* 1999, vol. 12, 108-116
- **Kurokawa, H et al.** *Cancer Res.,* 2000, vol. 60, 5887-5894
- **de la Torre-Bueno, J. et al.** *Modern Pathology,* 2000, vol. 13, 221A
- **C. J. Barnett.** *Org. Proc. Res. Develop.,* 1999, vol. 3, 184
- **F. Seela.** *Liebigs Ann. Chem.,* 1987, 15
- **H. Akimoto.** *J. Chem. Soc. Perkin Trans,* 1998, vol. 1, 1637

- *J. Chem. Soc. Perkin Trans.,* 1998, vol. 1, 1637
- **Corey, A. et al.** Use of an aqueous soluble tetrazolium/formazan assay for cell growth assays in culture. *Cancer Commun.,* 1991, vol. 3, 207-212
- *J. Med. Chem.,* 1999, vol. 42, 2064-2086
- **Bhaskar et al.** *J: Org. Chem.,* 1991, vol. 56, 5964-5965
- *J: Med. Chem,* 1999, vol. 42 (12), 2064-2086
- **Seela et al.** *Helv. Chim. Acta.,* 1986, vol. 69, 1602-1613
- **Morisawa et al.** *J. Med. Chem.,* 1974, vol. 17, 1083-1086
- **Bhaskar et al.** *J. Org. Chem.,* 1991, vol. 56, 5964-5965
- *J. Med. Chem.,* 1999, vol. 42 (12), 2064-2086
- **Seela, F. ; Stecker, H.** *Helv. Chim. Acta,* 1986, vol. 69, 1602-1613
- **Seela, F. ; Stecker, H.** *Helv. Chim. Acta,* 1986, vol. 69, 1602
- *Helv. Chim Acta,* 1986, vol. 69, 1602-1613
- *European Journal of Medicinal Chemistry,* 1994, vol. 29 (1), 3-9